# EUROPEAN PATENT APPLICATION

(11) **EP 2 327 418 A1**
(43) Date of publication of application: **01.06.2011**
(21) Application number: 10011015.4
(22) Date of filing: 21.02.2003
(51) Int. Cl.: A61K 39/00, C07K 14/11, C07K 14/115, C07K 14/135, G01N 33/50, C12N 15/86

(54) **Recombinant parainfluenza virus expression systems and vaccines comprising heterologous antigens derived from other viruses**

(30) Priority: 21.02.2002 US 358934 P
(62) Divisional of application: 03716118.9
(71) Applicant: MedImmune, LLC, Gaithersburg, MD 20878 (US); Vironovative BV, 3000 DR Rotterdam (NL)
(72) Inventor: Haller, Aurelia, Redwood City, CA 94062 (US); Coelingh, Kathleen, Seattle, WA 98101 (US)
(74) Representative: Chapman, Paul Gilmour

(57) **Abstract**

The present invention relates to recombinant bovine parainfluenza virus (BPIV) cDNA or RNA which may be used to express heterologous gene products in appropriate host cell systems and/or to rescue negative strand RNA recombinant viruses that express, package, and/or present the heterologous gene product. In particular, the heterologous gene products include gene product of another species of PIV or from another negative strand RNA virus, including but not limited to, influenza virus, respiratory syncytial virus, human metapneumovirus and avian pneumovirus. The chimeric viruses and expression products may advantageously be used in vaccine formulations including vaccines against a broad range of pathogens and antigens.

SEQUENCE LISTING

<110> MedImmune Vaccines, Inc.
      ViroNovative BV

<120> RECOMBINANT PARAINFLUENZA VIRUS
  EXPRESSION SYSTEMS AND VACCINES
  COMPRISING HETEROLOGOUS ANTIGENS
  DERIVED FROM METAPNEUMOVIRUS

<130> 7682-061-228

<140> To be assigned
<141> Herewith

<150> 60/358,934
<151> 2002-02-21

<160> 327

<170> FastSEQ for Windows Version 4.0

<210> 1
<211> 2507
<212> DNA
<213> metapneumovirus

<220>
<221> CDS
<222> (1)...(2507)
<223> Human metapneumovirus isolate 00-1 matrix protein
      (M) and fusion protein (F) genes

<400> 1
atggagtcct acctagtaga cacctatcaa ggcattcctt acacagcagc tgttcaagtt 60
gatctaatag aaaaggacct gttacctgca agcctaacaa tatggttccc tttgtttcag 120
gccaacacac caccagcagt gctgctcgat cagctaaaaa ccctgacaat aaccactctg 180
tatgctgcat cacaaaatgg tccaatactc aaagtgaatg catcagccca aggtgcagca 240
atgtctgtac ttcccaaaaa atttgaagtc aatgcgactg tagcactcga tgaatatagc 300
aaactggaat ttgacaaact cacagtctgt gaagtaaaaa cagtttactt aacaaccatg 360
aaaccatacg ggatggtatc aaaatttgtg agctcagcca aatcagttgg caaaaaaaca 420
catgatctaa tcgcactatg tgattttatg gatctagaaa agaacacacc tgttacaata 480
ccagcattca tcaaatcagt ttcaatcaaa gagagtgagt cagctactgt tgaagctgct 540
ataagcagtg aagcagacca agctctaaca caggccaaaa ttgcacctta tgcgggatta 600
attatgatca tgactatgaa caatcccaaa ggcatattca aaaagcttgg agctgggact 660
caagtcatag tagaactagg agcatatgtc caggctgaaa gcataagcaa aatatgcaag 720
acttggagcc atcaagggac aagatatgtc ttgaagtcca gataacaacc aagcaccttg 780
gccaagagct actaacccta tctcatagat cataaagtca ccattctagt tatataaaaa 840
tcaagttaga acaagaatta aatcaatcaa gaacgggaca aataaaaatg tcttggaaag 900
tggtgatcat tttttcattg ttaataacac ctcaacacgg tcttaaagag agctacttag 960
aagagtcatg tagcactata actgaaggat atctcagtgt tctgaggaca ggttggtaca 1020
ccaatgtttt tacactggag gtaggcgatg tagagaacct tacatgtgcc gatggaccca 1080
gcttaataaa aacagaatta gacctgacca aaagtgcact aagagagctc agaacagttt 1140
ctgctgatca actggcaaga gaggagcaaa ttgaaaatcc cagacaatct agattcgttc 1200
taggagcaat agcactcggt gttgcaactg cagctgcagt tacagcaggt gttgcaattg 1260
ccaaaaccat ccggcttgaa agtgaagtaa cagcaattaa gaatgccctc aaaaagacca 1320
atgaagcagt atctacattg gggaatggag ttcgtgtgtt ggcaactgca gtgagagagc 1380
tgaaagattt tgtgagcaag aatctaacac gtgcaatcaa caaaaacaag tgcgacattg 1440
ctgacctgaa aatggccgtt agcttcagtc aattcaacag aaggttccta aatgttgtgc 1500
ggcaattttc agacaacgct ggaataacac cagcaatatc tttggactta atgacagatg 1560
ctgaactagc cagagctgtt tccaacatgc caacatctgc aggacaaata aaactgatgt 1620

tggagaaccg tgcaatggta agaagaaaag ggttcggatt cctgatagga gtttacggaa 1680
gctccgtaat ttacatggtg caactgccaa tctttggggt tatagacacg ccttgctgga 1740
tagtaaaagc agccccttct tgttcaggaa aaaagggaaa ctatgcttgc ctcttaagag 1800
aagaccaagg atggtattgt caaaatgcag ggtcaactgt ttactaccca aatgaaaaag 1860
actgtgaaac aagaggagac catgtctttt gcgacacagc agcaggaatc aatgttgctg 1920
agcagtcaaa ggagtgcaac ataaacatat ctactactaa ttacccatgc aaagttagca 1980
caggaagaca tcctatcagt atggttgcac tatctcctct tggggctttg gttgcttgct 2040
acaagggagt gagctgttcc attggcagca acagagtagg gatcatcaag caactgaaca 2100
aaggctgctc ttatataacc aaccaagacg cagacacagt gacaatagac aacactgtat 2160
accagctaag caaagttgaa ggcgaacagc atgttataaa aggaaggcca gtgtcaagca 2220
gctttgaccc agtcaagttt cctgaagatc aattcaatgt tgcacttgac caagttttcg 2280
agagcattga gaacagtcag gccttggtgg atcaatcaaa cagaatccta agcagtgcag 2340
agaaaggaaa cactggcttc atcattgtaa taattctaat tgctgtcctt ggctctacca 2400
tgatcctagt gagtgttttt atcataataa agaaaacaaa gagacccaca ggagcacctc 2460
cagagctgag tggtgtcaca aacaatggct tcataccaca taattag 2507

<210> 2
<211> 1596
<212> DNA
<213> pneumvirus

<220>
<221> CDS
<222> (1)...(1596)
<223> Avian pneumovirus fusion protein gene, partial cds

<400> 2
atgtcttgga aagtggtact gctattggta ttgctagcta ccccaacggg ggggctagaa 60
gaaagttatc tagaggagtc atgcagtact gttactagag gatacctgag tgttttgagg 120
acaggatggt atacaaatgt gttcacactt ggggttggag atgtgaaaaa tctcacatgt 180
accgacgggc ccagcttaat aagaacagaa cttgaactga caaaaaatgc acttgaggaa 240
ctcaagacag tatcagcaga tcaattggca aaggaagcta ggataatgtc accaagaaaa 300
gcccggtttg ttctgggtgc catagcatta ggtgtggcaa ctgctgctgc tgtgacggct 360
ggtgtagcga tagccaagac aattaggcta gaaggagaag tggctgcaat caaaggtgcg 420
ctcaggaaaa caaatgaggc tgtatctaca ttaggaaatg gcgtgagggt acttgcaaca 480
gctgtgaatg atctcaagga ctttataagt aaaaaattga cacctgcaat aaacaggaac 540
aagtgtgaca tctcagacct taagatggca gtgagctttg gacaatacaa tcggaggttc 600
ctcaatgtgg taagacagtt ttctgacaat gcaggtatta cgcctgcaat atctctagat 660
ttaatgactg acgctgagct tgtaagagct gtaagcaaca tgcccacatc ttcaggacag 720
atcaatctga tgcttgagaa tcgggcaatg gtcagaagga aaggatttgg gattttgatt 780
ggagtttatg gtagctctgt ggtctatata gtgcagcttc ctattttcgg tgtgatagat 840
acaccgtgtt ggagggtgaa ggctgctcca ttatgttcag ggaaagacgg gaattatgca 900
tgtctcttgc gagaggacca aggttggtat tgtcaaaatg ctggatccac agtttattat 960
ccaaatgagg aggactgtga agtaagaagt gatcatgtgt tttgtgacac agcagctggg 1020
ataaatgtag caaaggagtc agaagagtgc aacaggaata tctcaacaac aaagtaccct 1080
tgcaaggtaa gtacagggcg tcacccaata agcatggtgg ccttatcacc actgggtgct 1140
ttggtagcct gttatgacgg tatgagttgt tccattggaa gcaacaaggt tggaataatc 1200
agacctttgg ggaaagggtg ttcatacatc agcaatcaag atgctgacac tgttacaatt 1260
gacaacacag tgtaccaatt gagcaaagtt gaaggagaac aacacacaat taaagggaag 1320
ccagtatcta gcaattttga ccctatagag ttccctgaag atcagttcaa cgtagccctg 1380
gatcaggtgt ttgaaagtgt tgagaagagt cagaatctga tagaccagtc aaacaagata 1440
ttggatagca ttgaaaaggg gaatgcagga tttgtcatag tgatagtcct cattgtcctg 1500
ctcatgctgg cagcagttgg tgtgggtgtc ttctttgtgg ttaagaagag aaaagctgct 1560
cccaaattcc caatggaaat gaatggtgtg aacaac 1596

<210> 3
<211> 1666
<212> DNA
<213> pneumovirus

<220>
<221> CDS

<222> (14)...(1627)
<223> Avian pneumovirus isolate 1b fusion protein mRNA,
      complete cds

<400> 3
gggacaagtg aaaatgtctt ggaaagtggt actgctattg gtattgctag ctaccccaac 60
gggggggcta gaagaaagtt atctagagga gtcatgcagt actgttacta gaggatacct 120
gagtgttttg aggacaggat ggtatacaaa tgtgttcaca cttgaggttg gagatgtgga 180
aaatctcaca tgtaccgacg gcccagcctt aataagaaca gaacttgaac tgacaaaaaa 240
tgcacttgag gaactcaaga cagtatcagc agatcaattg gcaaaggaag ctaggataat 300
gtcaccaaga aaagcccggt ttgttctggg tgccatagca ttaggtgtgg caactgctgc 360
tgctgtgacg gctggtgtag cgatagccaa gacaattagg ctagaaggag aagtggctgc 420
aatcaagggt gcgctcagga aaacaaatga ggctgtatct acattaggaa atggcgtgag 480
ggtacttgca acagctgtga atgatctcaa ggactttata agtaaaaaat tgacacctgc 540
aataaacagg aacaagtgtg acatctcaga ccttaagatg gcagtgagct ttggacaata 600
caatcggagg ttcctcaatg tggtaagaca gttttctgac aatgcaggta ttacgcctgc 660
aatatctcta gatttaatga ctgacgctga gcttgtaaga gctgtaagca acatgcccac 720
atcttcagga cagatcaatc tgatgcttga gaatcgggca atggtcagaa ggaaaggatt 780
tgggattttg attggagttt atggtagctc tgtggtctat atagtgcagc ttcctatttt 840
cggtgtgata gatacaccgt gttggaaggt gaaggctgct ccattatgtt cagggaaaga 900
cgggaattat gcatgtctct tgcgagagga ccaaggttgg tattgtcaaa atgctggatc 960
cacagtttat tatccaaatg aggaggactg tgaagtaaga agtgatcatg tgttttgtga 1020
cacagcagct gggataaatg tagcaaagga gtcagaagag tgcaacagga atatctcaac 1080
aacaaagtac ccttgcaagg taagtacagg gcgtcaccca ataagcatgg tggccttatc 1140
accactgggt gctttggtag cctgttatga cggtatgagt tgttccattg gaagcaacaa 1200
ggttggaata atcagacctt tggggaaagg gtgttcatac atcagcaatc aagatgctga 1260
cactgttaca attgacaaca cagtgtacca attgagcaaa gttgaaggag aacaacacac 1320
aattaaaggg aagccagtat ctagcaattt tgaccctata gagttccctg aagatcagtt 1380
caacgtagcc ctggatcagg tgtttgaaag tgttgagaag agtcagaatc tgatagacca 1440
gtcaaacaag atattggata gcattgaaaa ggggaatgca ggatttgtca tagtgatagt 1500
cctcattgtc ctgctcatgc tggcagcagt tggtgtgggt gtcttctttg tggttaagaa 1560
gagaaaagct gctcccaaat tcccaatgga aatgaatggt gtgaacaaca aaggatttat 1620
cccttaattt tagttattaa aaaaaaaaaa aaaaaaaaaa aaaaaa 1666

<210> 4
<211> 1636
<212> DNA
<213> rhinotracheitis virus

<220>
<221> CDS
<222> (13)...(1629)
<223> Turkey rhinotracheitis virus gene for fusion
      protein (F1 and F2 subunits), complete cds

<400> 4
gggacaagta ggatggatgt aagaatctgt ctcctattgt tccttatatc taatcctagt 60
agctgcatac aagaaacata caatgaagaa tcctgcagta ctgtaactag aggttataag 120
agtgtgttaa ggacagggtg gtatacgaat gtatttaacc tcgaaatagg gaatgttgag 180
aacatcactt gcaatgatgg acccagccta attgacactg agttagtact cacaaagaat 240
gctttgaggg agctcaaaac agtgtcagct gatcaagtgg ctaaggaaag cagactatcc 300
tcacccagga gacgtagatt tgtactgggt gcaatagcac ttggtgttgc gacagctgct 360
gccgtaacag ctggtgtagc acttgcaaag acaattagat tagagggaga ggtgaaggca 420
attaagaatg ccctccggaa cacaaatgag gcagtatcca cattagggaa tggtgtgagg 480
gtactagcaa ctgcagtcaa tgacctcaaa gaatttataa gtaaaaaatt gactcctgct 540
attaaccaga acaaatgcaa tatagcagat ataaagatgg caattagttt tggccaaaat 600
aacagaaggt tcctgaatgt ggtgaggcaa ttctctgata gtgcaggtat cacatcagct 660
gtgtctcttg atttaatgac agatgatgaa cttgttagag caattaacag aatgccaact 720
tcatcaggac agattagttt gatgttgaac aatcgtgcca tggttagaag gaaggggttt 780
ggtatattga ttggtgttta tgatggaacg gtcgtttata tggtacaact gcccatattc 840
ggcgtgattg agacaccttg ttggagggtg gtggcagcac cactctgtag gaaagagaaa 900
ggcaattatg cttgtatact gagagaagat caagggtggt actgtacaaa tgctggctct 960

acagcttatt atcctaataa agatgattgt gaggtaaggg atgattatgt attttgtgac 1020
acagcagctg gcattaatgt ggccctagaa gttgaacagt gcaactataa catatcgact 1080
tctaaatacc catgcaaagt cagcacaggt agacaccctg tcagtatggt agccttaacc 1140
cccctagggg gtctagtgtc ttgttatgag agtgtaagtt gctccatagg tagcaataaa 1200
gtagggataa taaaacagct aggcaaaggg tgcacccaca ttcccaacaa cgaagctgac 1260
acgataacca ttgataacac tgtgtaccaa ttgagcaagg ttgtaggcga acagaggacc 1320
ataaaaggag ctccagttgt gaacaatttt aacccaatat tattccctga ggatcagttc 1380
aatgttgcac ttgaccaagt atttgagagt atagatagat ctcaggactt aatagataag 1440
tctaacgact tgctaggtgc agatgccaag agcaaggctg gaattgctat agcaatagta 1500
gtgctagtca ttctaggaat cttcttttta cttgcagtga tatattactg ttccagagtc 1560
cggaagacca aaccaaagca tgattacccg gccacgacag gtcatagcag catggcttat 1620
gtcagttaag ttattt 1636

<210> 5
<211> 1860
<212> DNA
<213> pneumovirus

<220>
<221> CDS
<222> (1)...(110)
<223> Avian pneumovirus matrix protein (M) gene, partial
      cds

<220>
<221> CDS
<222> (216)...(1829)
<223> Avian pneumovirus fusion glycoprotein (F) gene,
      complete cds

<400> 5
gagttcaggt aatagtggag ttaggggcat acgttcaagc agaaagcata agcagaatct 60
gcaggaactg gagccaccag ggtacgagat atgtcctgaa gtcaagataa acacagagag 120
tacacttacc aaatcacagt aacaatttcg tttttaaccc tctcatagtt attacctagc 180
ttgatattat ttagaaaaaa ttgggacaag tgaaaatgtc ttggaaagtg gtactgctat 240
tggtattgct agctacccca acgggggggc tagaagaaag ttatctagag gagtcatgca 300
gtactgttac tagaggatac ctgagtgttt tgaggacagg atggtataca aatgtgttca 360
cacttgaggt tggagatgtg gaaaatctca catgtaccga cgggcccagc ttaataagaa 420
cagaacttga actgacaaaa aatgcacttg aggaactcaa gacagtatca gcagatcaat 480
tggcaaagga agctaggata atgtcaccaa gaaaagcccg gtttgttctg ggtgccatag 540
cattaggtgt ggcaactgct gctgctgtga cggctggtgt agcgatagcc aagacaatta 600
ggctagaagg agaagtggct gcaatcaagg gtgcgctcag gaaaacaaat gaggctgtat 660
ctacattagg aaatggcgtg agggtacttg caacagctgt gaatgatctc aaggacttta 720
taagtaaaaa attgacacct gcaataaaca ggaacaagtg tgacatctca gaccttaaga 780
tggcagtgag ctttggacaa tacaatcgga ggttcctcaa tgtggtaaga cagttttctg 840
acaatgcagg tattacgcct gcaatatctc tagatttaat gactgacgct gagcttgtaa 900
gagctgtaag caacatgccc acatcttcag gacagatcaa tctgatgctt gagaatcggg 960
caatggtcag aaggaaagga tttgggattt tgattggagt ttatggtagc tctgtggtct 1020
atatagtgca gcttcctatt ttcggtgtga tagatacacc gtgttggaag gtgaaggctg 1080
ctccattatg ttcagggaaa gacgggaatt atgcatgtct cttgcgagag gaccaaggtt 1140
ggtattgtca aaatgctgga tccacagttt attatccaaa tgaggaggac tgtgaagtaa 1200
gaagtgatca tgtgttttgt gacacagcag ctgggataaa tgtagcaaag gagtcagaag 1260
agtgcaacag gaatatctca acaacaaagt acccttgcaa ggtaagtaca gggcgtcacc 1320
caataagcat ggtggcctta tcaccactgg gtgctttggt agcctgttat gacggtatga 1380
gttgttccat tggaagcaac aaggttggaa taatcagacc tttggggaaa gggtgttcat 1440
acatcagcaa tcaagatgct gacactgtta caattgacaa cacagtgtac caattgagca 1500
aagttgaagg agaacaacac acaattaaag ggaagccagt atctagcaat tttgacccta 1560
tagagttccc tgaagatcag ttcaacatag ccctggatca ggtgtttgaa agtgttgaga 1620
agagtcagaa tctgatagac cagtcaaaca agatattgga tagcattgaa aaggggaatg 1680
caggatttgt catagtgata gtcctcattg tcctgctcat gctggcagca gttggtgtgg 1740
gtgtcttctt tgtggttaag aagagaaaag ctgctcccaa attcccaatg gaaatgaatg 1800
gtgtgaacaa caaaggattt atcccttaat tttagttact aaaaaattgg gacaagtgaa 1860

<210> 6
<211> 574
<212> PRT
<213> paramyxovirus

<400> 6
Met Glu Leu Leu Ile His Arg Leu Ser Ala Ile Phe Leu Thr Leu Ala
1               5                   10                  15
Ile Asn Ala Leu Tyr Leu Thr Ser Ser Gln Asn Ile Thr Glu Glu Phe
            20                  25                  30
Tyr Gln Ser Thr Cys Ser Ala Val Ser Arg Gly Tyr Phe Ser Ala Leu
        35                  40                  45
Arg Thr Gly Trp Tyr Thr Ser Val Ile Thr Ile Glu Leu Ser Asn Ile
    50                  55                  60
Lys Glu Thr Lys Cys Asn Gly Thr Asp Thr Lys Val Lys Leu Ile Lys
65                  70                  75                  80
Gln Glu Leu Asp Lys Tyr Lys Asn Ala Val Thr Glu Leu Gln Leu Leu
                85                  90                  95
Met Gln Asn Thr Pro Ala Ala Asn Asn Arg Ala Arg Arg Glu Ala Pro
            100                 105                 110
Gln Tyr Met Asn Tyr Thr Ile Asn Thr Thr Lys Asn Leu Asn Val Ser
        115                 120                 125
Ile Ser Lys Lys Arg Lys Arg Arg Phe Leu Gly Phe Leu Leu Gly Val
    130                 135                 140
Gly Ser Ala Ile Ala Ser Gly Ile Ala Val Ser Lys Val Leu His Leu
145                 150                 155                 160
Glu Gly Glu Val Asn Lys Ile Lys Asn Ala Leu Leu Ser Thr Asn Lys
                165                 170                 175
Ala Val Val Ser Leu Ser Asn Gly Val Ser Val Leu Thr Ser Lys Val
            180                 185                 190
Leu Asp Leu Lys Asn Tyr Ile Asn Asn Gln Leu Leu Pro Ile Val Asn
        195                 200                 205
Gln Gln Ser Cys Arg Ile Ser Asn Ile Glu Thr Val Ile Glu Phe Gln
    210                 215                 220
Gln Lys Asn Ser Arg Leu Leu Glu Ile Asn Arg Glu Phe Ser Val Asn
225                 230                 235                 240
Ala Gly Val Thr Thr Pro Leu Ser Thr Tyr Met Leu Thr Asn Ser Glu
                245                 250                 255
Leu Leu Ser Leu Ile Asn Asp Met Pro Ile Thr Asn Asp Gln Lys Lys
            260                 265                 270
Leu Met Ser Ser Asn Val Gln Ile Val Arg Gln Gln Ser Tyr Ser Ile
        275                 280                 285
Met Ser Ile Ile Lys Glu Glu Val Leu Ala Tyr Val Val Gln Leu Pro
    290                 295                 300
Ile Tyr Gly Val Ile Asp Thr Pro Cys Trp Lys Leu His Thr Ser Pro
305                 310                 315                 320
Leu Cys Thr Thr Asn Ile Lys Glu Gly Ser Asn Ile Cys Leu Thr Arg
                325                 330                 335
Thr Asp Arg Gly Trp Tyr Cys Asp Asn Ala Gly Ser Val Ser Phe Phe
            340                 345                 350
Pro Gln Ala Asp Thr Cys Lys Val Gln Ser Asn Arg Val Phe Cys Asp
        355                 360                 365
Thr Met Asn Ser Leu Thr Leu Pro Ser Glu Val Ser Leu Cys Asn Thr
    370                 375                 380
Asp Ile Phe Asn Ser Lys Tyr Asp Cys Lys Ile Met Thr Ser Lys Thr
385                 390                 395                 400
Asp Ile Ser Ser Ser Val Ile Thr Ser Leu Gly Ala Ile Val Ser Cys
                405                 410                 415
Tyr Gly Lys Thr Lys Cys Thr Ala Ser Asn Lys Asn Arg Gly Ile Ile
            420                 425                 430
Lys Thr Phe Ser Asn Gly Cys Asp Tyr Val Ser Asn Lys Gly Val Asp

435                 440                 445

Thr Val Ser Val Gly Asn Thr Leu Tyr Tyr Val Asn Lys Leu Glu Gly
    450                 455                 460
Lys Asn Leu Tyr Val Lys Gly Glu Pro Ile Ile Asn Tyr Tyr Asp Pro
465                 470                 475                 480
Leu Val Phe Pro Ser Asp Glu Phe Asp Ala Ser Ile Ser Gln Val Asn
                485                 490                 495
Glu Lys Ile Asn Gln Ser Leu Ala Phe Ile Arg Arg Ser Asp Glu Leu
            500                 505                 510
Leu His Asn Val Asn Thr Gly Lys Ser Thr Thr Asn Ile Met Ile Thr
        515                 520                 525
Thr Ile Ile Ile Val Ile Ile Val Val Leu Leu Ser Leu Ile Ala Ile
    530                 535                 540
Gly Leu Leu Leu Tyr Cys Lys Ala Lys Asn Thr Pro Val Thr Leu Ser
545                 550                 555                 560
Lys Asp Gln Leu Ser Gly Ile Asn Asn Ile Ala Phe Ser Lys
                565                 570


<210> 7
<211> 574
<212> PRT
<213> paramyxovirus

<400> 7
Met Glu Leu Leu Ile Leu Lys Ala Asn Ala Ile Thr Thr Ile Leu Thr
1               5                   10                  15
Ala Val Thr Phe Cys Phe Ala Ser Gly Gln Asn Ile Thr Glu Glu Phe
            20                  25                  30
Tyr Gln Ser Thr Cys Ser Ala Val Ser Lys Gly Tyr Leu Ser Ala Leu
        35                  40                  45
Arg Thr Gly Trp Tyr Thr Ser Val Ile Thr Ile Glu Leu Ser Asn Ile
    50                  55                  60
Lys Glu Asn Lys Cys Asn Gly Thr Asp Ala Lys Val Lys Leu Ile Lys
65                  70                  75                  80
Gln Glu Leu Asp Lys Tyr Lys Asn Ala Val Thr Glu Leu Gln Leu Leu
                85                  90                  95
Met Gln Ser Thr Pro Pro Thr Asn Asn Arg Ala Arg Arg Glu Leu Pro
            100                 105                 110
Arg Phe Met Asn Tyr Thr Leu Asn Asn Ala Lys Lys Thr Asn Val Thr
        115                 120                 125
Leu Ser Lys Lys Arg Lys Arg Arg Phe Leu Gly Phe Leu Leu Gly Val
    130                 135                 140
Gly Ser Ala Ile Ala Ser Gly Val Ala Val Ser Lys Val Leu His Leu
145                 150                 155                 160
Glu Gly Glu Val Asn Lys Ile Lys Ser Ala Leu Leu Ser Thr Asn Lys
                165                 170                 175
Ala Val Val Ser Leu Ser Asn Gly Val Ser Val Leu Thr Ser Lys Val
            180                 185                 190
Leu Asp Leu Lys Asn Tyr Ile Asp Lys Gln Leu Leu Pro Ile Val Asn
        195                 200                 205
Lys Gln Ser Cys Ser Ile Ser Asn Ile Glu Thr Val Ile Glu Phe Gln
    210                 215                 220
Gln Lys Asn Asn Arg Leu Leu Glu Ile Thr Arg Glu Phe Ser Val Asn
225                 230                 235                 240
Ala Gly Val Thr Thr Pro Val Ser Thr Tyr Met Leu Thr Asn Ser Glu
                245                 250                 255
Leu Leu Ser Leu Ile Asn Asp Met Pro Ile Thr Asn Asp Gln Lys Lys
            260                 265                 270
Leu Met Ser Asn Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300
Leu Tyr Gly Val Ile Asp Thr Pro Cys Trp Lys Leu His Thr Ser Pro
305                 310                 315                 320
Leu Cys Thr Thr Asn Thr Lys Glu Gly Ser Asn Ile Cys Leu Thr Arg
                325                 330                 335
Thr Asp Arg Gly Trp Tyr Cys Asp Asn Ala Gly Ser Val Ser Phe Phe
            340                 345                 350
Pro Gln Ala Glu Thr Cys Lys Val Gln Ser Asn Arg Val Phe Cys Asp
        355                 360                 365
Thr Met Asn Ser Leu Thr Leu Pro Ser Glu Ile Asn Leu Cys Asn Val
    370                 375                 380
Asp Ile Phe Asn Pro Lys Tyr Asp Cys Lys Ile Met Thr Ser Lys Thr
385                 390                 395                 400
Asp Val Ser Ser Ser Val Ile Thr Ser Leu Gly Ala Ile Val Ser Cys
                405                 410                 415
Tyr Gly Lys Thr Lys Cys Thr Ala Ser Asn Lys Asn Arg Gly Ile Ile
            420                 425                 430
Lys Thr Phe Ser Asn Gly Cys Asp Tyr Val Ser Asn Lys Gly Met Asp
        435                 440                 445
Thr Val Ser Val Gly Asn Thr Leu Tyr Tyr Val Asn Lys Gln Glu Gly
    450                 455                 460
Lys Ser Leu Tyr Val Lys Gly Glu Pro Ile Ile Asn Phe Tyr Asp Pro
465                 470                 475                 480
Leu Val Phe Pro Ser Asp Glu Phe Asp Ala Ser Ile Ser Gln Val Asn
                485                 490                 495
Glu Lys Ile Asn Gln Ser Leu Ala Phe Ile Arg Lys Ser Asp Glu Leu
            500                 505                 510
Leu His Asn Val Asn Ala Gly Lys Ser Thr Thr Asn Ile Met Ile Thr
        515                 520                 525
Thr Ile Ile Ile Val Ile Ile Val Ile Leu Leu Ser Leu Ile Ala Val
    530                 535                 540
Gly Leu Leu Leu Tyr Cys Lys Ala Arg Ser Thr Pro Val Thr Leu Ser
545                 550                 555                 560
Lys Asp Gln Leu Ser Gly Ile Asn Asn Ile Ala Phe Ser Asn
                565                 570

<210> 8
<211> 121
<212> PRT
<213> metapneumovirus

<400> 8
Leu Leu Ile Thr Pro Gln His Gly Leu Lys Glu Ser Tyr Leu Glu Glu
 1               5                  10                  15
Ser Cys Ser Thr Ile Thr Glu Gly Tyr Leu Ser Val Leu Arg Thr Gly
            20                  25                  30
Trp Tyr Thr Asn Val Phe Thr Leu Glu Val Gly Asp Val Glu Asn Leu
        35                  40                  45
Thr Cys Ala Asp Gly Pro Ser Leu Ile Lys Thr Glu Leu Asp Leu Thr
    50                  55                  60
Lys Ser Ala Leu Arg Glu Leu Arg Thr Val Ser Ala Asp Gln Leu Ala
65                  70                  75                  80
Arg Glu Glu Gln Ile Glu Asn Pro Arg Gln Ser Arg Phe Val Leu Gly
                85                  90                  95
Ala Ile Ala Leu Gly Val Ala Thr Ala Ala Ala Val Thr Ala Gly Val
            100                 105                 110
Ala Ile Ala Lys Thr Ile Arg Leu Glu
        115                 120

<210> 9
<211> 539
<212> PRT
<213> metapneumovirus

<400> 9
Met Ser Trp Lys Val Val Ile Ile Phe Ser Leu Leu Ile Thr Pro Gln
1               5                   10                  15
His Gly Leu Lys Glu Ser Tyr Leu Glu Glu Ser Cys Ser Thr Ile Thr
            20                  25                  30
Glu Gly Tyr Leu Ser Val Leu Arg Thr Gly Trp Tyr Thr Asn Val Phe
        35                  40                  45
Thr Leu Glu Val Gly Asp Val Glu Asn Leu Thr Cys Ala Asp Gly Pro
    50                  55                  60
Ser Leu Ile Lys Thr Glu Leu Asp Leu Thr Lys Ser Ala Leu Arg Glu
65                  70                  75                  80
Leu Arg Thr Val Ser Ala Asp Gln Leu Ala Arg Glu Glu Gln Ile Glu
                85                  90                  95
Asn Pro Arg Gln Ser Arg Phe Val Leu Gly Ala Ile Ala Leu Gly Val
            100                 105                 110
Ala Thr Ala Ala Ala Val Thr Ala Gly Val Ala Ile Ala Lys Thr Ile
        115                 120                 125
Arg Leu Glu Ser Glu Val Thr Ala Ile Lys Asn Ala Leu Lys Lys Thr
    130                 135                 140
Asn Glu Ala Val Ser Thr Leu Gly Asn Gly Val Arg Val Leu Ala Thr
145                 150                 155                 160
Ala Val Arg Glu Leu Lys Asp Phe Val Ser Lys Asn Leu Thr Arg Ala
                165                 170                 175
Ile Asn Lys Asn Lys Cys Asp Ile Ala Asp Leu Lys Met Ala Val Ser
            180                 185                 190
Phe Ser Gln Phe Asn Arg Arg Phe Leu Asn Val Val Arg Gln Phe Ser
        195                 200                 205
Asp Asn Ala Gly Ile Thr Pro Ala Ile Ser Leu Asp Leu Met Thr Asp
    210                 215                 220
Ala Glu Leu Ala Arg Ala Val Ser Asn Met Pro Thr Ser Ala Gly Gln
225                 230                 235                 240
Ile Lys Leu Met Leu Glu Asn Arg Ala Met Val Arg Arg Lys Gly Phe
                245                 250                 255
Gly Phe Leu Ile Gly Val Tyr Gly Ser Ser Val Ile Tyr Met Val Gln
            260                 265                 270
Leu Pro Ile Phe Gly Val Ile Asp Thr Pro Cys Trp Ile Val Lys Ala
        275                 280                 285
Ala Pro Ser Cys Ser Gly Lys Lys Gly Asn Tyr Ala Cys Leu Leu Arg
    290                 295                 300
Glu Asp Gln Gly Trp Tyr Cys Gln Asn Ala Gly Ser Thr Val Tyr Tyr
305                 310                 315                 320
Pro Asn Glu Lys Asp Cys Glu Thr Arg Gly Asp His Val Phe Cys Asp
                325                 330                 335
Thr Ala Ala Gly Ile Asn Val Ala Glu Gln Ser Lys Glu Cys Asn Ile
            340                 345                 350
Asn Ile Ser Thr Thr Asn Tyr Pro Cys Lys Val Ser Thr Gly Arg His
        355                 360                 365
Pro Ile Ser Met Val Ala Leu Ser Pro Leu Gly Ala Leu Val Ala Cys
    370                 375                 380
Tyr Lys Gly Val Ser Cys Ser Ile Gly Ser Asn Arg Val Gly Ile Ile
385                 390                 395                 400
Lys Gln Leu Asn Lys Gly Cys Ser Tyr Ile Thr Asn Gln Asp Ala Asp
                405                 410                 415
Thr Val Thr Ile Asp Asn Thr Val Tyr Gln Leu Ser Lys Val Glu Gly
            420                 425                 430
Glu Gln His Val Ile Lys Gly Arg Pro Val Ser Ser Ser Phe Asp Pro
        435                 440                 445

Val Lys Phe Pro Glu Asp Gln Phe Asn Val Ala Leu Asp Gln Val Phe
    450                 455                 460
Glu Ser Ile Glu Asn Ser Gln Ala Leu Val Asp Gln Ser Asn Arg Ile
465                 470                 475                 480
Leu Ser Ser Ala Glu Lys Gly Asn Thr Gly Phe Ile Ile Val Ile Ile
                485                 490                 495
Leu Ile Ala Val Leu Gly Ser Thr Met Ile Leu Val Ser Val Phe Ile
            500                 505                 510
Ile Ile Lys Lys Thr Lys Arg Pro Thr Gly Ala Pro Pro Glu Leu Ser
        515                 520                 525
Gly Val Thr Asn Asn Gly Phe Ile Pro His Asn
    530                 535

<210> 10
<211> 532
<212> PRT
<213> Avian pneumovirus

<400> 10
Met Ser Trp Lys Val Val Leu Leu Leu Val Leu Leu Ala Thr Pro Thr
 1               5                  10                  15
Gly Gly Leu Glu Glu Ser Tyr Leu Glu Glu Ser Cys Ser Thr Val Thr
            20                  25                  30
Arg Gly Tyr Leu Ser Val Leu Arg Thr Gly Trp Tyr Thr Asn Val Phe
        35                  40                  45
Thr Leu Gly Val Gly Asp Val Lys Asn Leu Thr Cys Thr Asp Gly Pro
    50                  55                  60
Ser Leu Ile Arg Thr Glu Leu Glu Leu Thr Lys Asn Ala Leu Glu Glu
65                  70                  75                  80
Leu Lys Thr Val Ser Ala Asp Gln Leu Ala Lys Glu Ala Arg Ile Met
                85                  90                  95
Ser Pro Arg Lys Ala Arg Phe Val Leu Gly Ala Ile Ala Leu Gly Val
            100                 105                 110
Ala Thr Ala Ala Ala Val Thr Ala Gly Val Ala Ile Ala Lys Thr Ile
        115                 120                 125
Arg Leu Glu Gly Glu Val Ala Ala Ile Lys Gly Ala Leu Arg Lys Thr
    130                 135                 140
Asn Glu Ala Val Ser Thr Leu Gly Asn Gly Val Arg Val Leu Ala Thr
145                 150                 155                 160
Ala Val Asn Asp Leu Lys Asp Phe Ile Ser Lys Lys Leu Thr Pro Ala
                165                 170                 175
Ile Asn Arg Asn Lys Cys Asp Ile Ser Asp Leu Lys Met Ala Val Ser
            180                 185                 190
Phe Gly Gln Tyr Asn Arg Arg Phe Leu Asn Val Val Arg Gln Phe Ser
        195                 200                 205
Asp Asn Ala Gly Ile Thr Pro Ala Ile Ser Leu Asp Leu Met Thr Asp
    210                 215                 220
Ala Glu Leu Val Arg Ala Val Ser Asn Met Pro Thr Ser Ser Gly Gln
225                 230                 235                 240
Ile Asn Leu Met Leu Glu Asn Arg Ala Met Val Arg Arg Lys Gly Phe
                245                 250                 255
Gly Ile Leu Ile Gly Val Tyr Gly Ser Ser Val Val Tyr Ile Val Gln
            260                 265                 270
Leu Pro Ile Phe Gly Val Ile Asp Thr Pro Cys Trp Arg Val Lys Ala
        275                 280                 285
Ala Pro Leu Cys Ser Gly Lys Asp Gly Asn Tyr Ala Cys Leu Leu Arg
    290                 295                 300
Glu Asp Gln Gly Trp Tyr Cys Gln Asn Ala Gly Ser Thr Val Tyr Tyr
305                 310                 315                 320
Pro Asn Glu Glu Asp Cys Glu Val Arg Ser Asp His Val Phe Cys Asp
                325                 330                 335

Thr Ala Ala Gly Ile Asn Val Ala Lys Glu Ser Glu Glu Cys Asn Arg
                340                 345                 350
Asn Ile Ser Thr Thr Lys Tyr Pro Cys Lys Val Ser Thr Gly Arg His
        355                 360                 365
Pro Ile Ser Met Val Ala Leu Ser Pro Leu Gly Ala Leu Val Ala Cys
    370                 375                 380
Tyr Asp Gly Met Ser Cys Ser Ile Gly Ser Asn Lys Val Gly Ile Ile
385                 390                 395                 400
Arg Pro Leu Gly Lys Gly Cys Ser Tyr Ile Ser Asn Gln Asp Ala Asp
                405                 410                 415
Thr Val Thr Ile Asp Asn Thr Val Tyr Gln Leu Ser Lys Val Glu Gly
            420                 425                 430
Glu Gln His Thr Ile Lys Gly Lys Pro Val Ser Ser Asn Phe Asp Pro
        435                 440                 445
Ile Glu Phe Pro Glu Asp Gln Phe Asn Val Ala Leu Asp Gln Val Phe
    450                 455                 460
Glu Ser Val Glu Lys Ser Gln Asn Leu Ile Asp Gln Ser Asn Lys Ile
465                 470                 475                 480
Leu Asp Ser Ile Glu Lys Gly Asn Ala Gly Phe Val Ile Val Ile Val
                485                 490                 495
Leu Ile Val Leu Leu Met Leu Ala Ala Val Gly Val Gly Val Phe Phe
            500                 505                 510
Val Val Lys Lys Arg Lys Ala Ala Pro Lys Phe Pro Met Glu Met Asn
        515                 520                 525
Gly Val Asn Asn
    530

<210> 11
<211> 537
<212> PRT
<213> Avian pneumovirus

<400> 11
Met Ser Trp Lys Val Val Leu Leu Leu Val Leu Leu Ala Thr Pro Thr
 1               5                  10                  15
Gly Gly Leu Glu Glu Ser Tyr Leu Glu Glu Ser Cys Ser Thr Val Thr
            20                  25                  30
Arg Gly Tyr Leu Ser Val Leu Arg Thr Gly Trp Tyr Thr Asn Val Phe
        35                  40                  45
Thr Leu Glu Val Gly Asp Val Glu Asn Leu Thr Cys Thr Asp Gly Pro
    50                  55                  60
Ser Leu Ile Arg Thr Glu Leu Glu Leu Thr Lys Asn Ala Leu Glu Glu
65                  70                  75                  80
Leu Lys Thr Val Ser Ala Asp Gln Leu Ala Lys Glu Ala Arg Ile Met
                85                  90                  95
Ser Pro Arg Lys Ala Arg Phe Val Leu Gly Ala Ile Ala Leu Gly Val
            100                 105                 110
Ala Thr Ala Ala Ala Val Thr Ala Gly Val Ala Ile Ala Lys Thr Ile
        115                 120                 125
Arg Leu Glu Gly Glu Val Ala Ala Ile Lys Gly Ala Leu Arg Lys Thr
    130                 135                 140
Asn Glu Ala Val Ser Thr Leu Gly Asn Gly Val Arg Val Leu Ala Thr
145                 150                 155                 160
Ala Val Asn Asp Leu Lys Asp Phe Ile Ser Lys Lys Leu Thr Pro Ala
                165                 170                 175
Ile Asn Arg Asn Lys Cys Asp Ile Ser Asp Leu Lys Met Ala Val Ser
            180                 185                 190
Phe Gly Gln Tyr Asn Arg Arg Phe Leu Asn Val Val Arg Gln Phe Ser
        195                 200                 205
Asp Asn Ala Gly Ile Thr Pro Ala Ile Ser Leu Asp Leu Met Thr Asp
    210                 215                 220

Ala Glu Leu Val Arg Ala Val Ser Asn Met Pro Thr Ser Ser Gly Gln
225                 230                 235                 240
Ile Asn Leu Met Leu Glu Asn Arg Ala Met Val Arg Arg Lys Gly Phe
                245                 250                 255
Gly Ile Leu Ile Gly Val Tyr Gly Ser Ser Val Val Tyr Ile Val Gln
            260                 265                 270
Leu Pro Ile Phe Gly Val Ile Asp Thr Pro Cys Trp Lys Val Lys Ala
        275                 280                 285
Ala Pro Leu Cys Ser Gly Lys Asp Gly Asn Tyr Ala Cys Leu Leu Arg
    290                 295                 300
Glu Asp Gln Gly Trp Tyr Cys Gln Asn Ala Gly Ser Thr Val Tyr Tyr
305                 310                 315                 320
Pro Asn Glu Glu Asp Cys Glu Val Arg Ser Asp His Val Phe Cys Asp
                325                 330                 335
Thr Ala Ala Gly Ile Asn Val Ala Lys Glu Ser Glu Glu Cys Asn Arg
            340                 345                 350
Asn Ile Ser Thr Thr Lys Tyr Pro Cys Lys Val Ser Thr Gly Arg His
        355                 360                 365
Pro Ile Ser Met Val Ala Leu Ser Pro Leu Gly Ala Leu Val Ala Cys
    370                 375                 380
Tyr Asp Gly Met Ser Cys Ser Ile Gly Ser Asn Lys Val Gly Ile Ile
385                 390                 395                 400
Arg Pro Leu Gly Lys Gly Cys Ser Tyr Ile Ser Asn Gln Asp Ala Asp
                405                 410                 415
Thr Val Thr Ile Asp Asn Thr Val Tyr Gln Leu Ser Lys Val Glu Gly
            420                 425                 430
Glu Gln His Thr Ile Lys Gly Lys Pro Val Ser Ser Asn Phe Asp Pro
        435                 440                 445
Ile Glu Phe Pro Glu Asp Gln Phe Asn Val Ala Leu Asp Gln Val Phe
    450                 455                 460
Glu Ser Val Glu Lys Ser Gln Asn Leu Ile Asp Gln Ser Asn Lys Ile
465                 470                 475                 480
Leu Asp Ser Ile Glu Lys Gly Asn Ala Gly Phe Val Ile Val Ile Val
                485                 490                 495
Leu Ile Val Leu Leu Met Leu Ala Ala Val Gly Val Gly Val Phe Phe
            500                 505                 510
Val Val Lys Lys Arg Lys Ala Ala Pro Lys Phe Pro Met Glu Met Asn
        515                 520                 525
Gly Val Asn Asn Lys Gly Phe Ile Pro
    530                 535

<210> 12
<211> 538
<212> PRT
<213> Turkey rhinotracheitis virus

<400> 12
Met Asp Val Arg Ile Cys Leu Leu Leu Phe Leu Ile Ser Asn Pro Ser
1               5                   10                  15
Ser Cys Ile Gln Glu Thr Tyr Asn Glu Glu Ser Cys Ser Thr Val Thr
            20                  25                  30
Arg Gly Tyr Lys Ser Val Leu Arg Thr Gly Trp Tyr Thr Asn Val Phe
        35                  40                  45
Asn Leu Glu Ile Gly Asn Val Glu Asn Ile Thr Cys Asn Asp Gly Pro
    50                  55                  60
Ser Leu Ile Asp Thr Glu Leu Val Leu Thr Lys Asn Ala Leu Arg Glu
65                  70                  75                  80
Leu Lys Thr Val Ser Ala Asp Gln Val Ala Lys Glu Ser Arg Leu Ser
                85                  90                  95
Ser Pro Arg Arg Arg Arg Phe Val Leu Gly Ala Ile Ala Leu Gly Val
            100                 105                 110

Ala Thr Ala Ala Ala Val Thr Ala Gly Val Ala Leu Ala Lys Thr Ile
        115                 120                 125
Arg Leu Glu Gly Glu Val Lys Ala Ile Lys Asn Ala Leu Arg Asn Thr
    130                 135                 140
Asn Glu Ala Val Ser Thr Leu Gly Asn Gly Val Arg Val Leu Ala Thr
145                 150                 155                 160
Ala Val Asn Asp Leu Lys Glu Phe Ile Ser Lys Lys Leu Thr Pro Ala
                165                 170                 175
Ile Asn Gln Asn Lys Cys Asn Ile Ala Asp Ile Lys Met Ala Ile Ser
            180                 185                 190
Phe Gly Gln Asn Asn Arg Arg Phe Leu Asn Val Val Arg Gln Phe Ser
        195                 200                 205
Asp Ser Ala Gly Ile Thr Ser Ala Val Ser Leu Asp Leu Met Thr Asp
    210                 215                 220
Asp Glu Leu Val Arg Ala Ile Asn Arg Met Pro Thr Ser Ser Gly Gln
225                 230                 235                 240
Ile Ser Leu Met Leu Asn Asn Arg Ala Met Val Arg Arg Lys Gly Phe
                245                 250                 255
Gly Ile Leu Ile Gly Val Tyr Asp Gly Thr Val Val Tyr Met Val Gln
            260                 265                 270
Leu Pro Ile Phe Gly Val Ile Glu Thr Pro Cys Trp Arg Val Val Ala
        275                 280                 285
Ala Pro Leu Cys Arg Lys Glu Lys Gly Asn Tyr Ala Cys Ile Leu Arg
    290                 295                 300
Glu Asp Gln Gly Trp Tyr Cys Thr Asn Ala Gly Ser Thr Ala Tyr Tyr
305                 310                 315                 320
Pro Asn Lys Asp Asp Cys Glu Val Arg Asp Asp Tyr Val Phe Cys Asp
                325                 330                 335
Thr Ala Ala Gly Ile Asn Val Ala Leu Glu Val Glu Gln Cys Asn Tyr
            340                 345                 350
Asn Ile Ser Thr Ser Lys Tyr Pro Cys Lys Val Ser Thr Gly Arg His
        355                 360                 365
Pro Val Ser Met Val Ala Leu Thr Pro Leu Gly Gly Leu Val Ser Cys
    370                 375                 380
Tyr Glu Ser Val Ser Cys Ser Ile Gly Ser Asn Lys Val Gly Ile Ile
385                 390                 395                 400
Lys Gln Leu Gly Lys Gly Cys Thr His Ile Pro Asn Asn Glu Ala Asp
                405                 410                 415
Thr Ile Thr Ile Asp Asn Thr Val Tyr Gln Leu Ser Lys Val Val Gly
            420                 425                 430
Glu Gln Arg Thr Ile Lys Gly Ala Pro Val Val Asn Asn Phe Asn Pro
        435                 440                 445
Ile Leu Phe Pro Glu Asp Gln Phe Asn Val Ala Leu Asp Gln Val Phe
    450                 455                 460
Glu Ser Ile Asp Arg Ser Gln Asp Leu Ile Asp Lys Ser Asn Asp Leu
465                 470                 475                 480
Leu Gly Ala Asp Ala Lys Ser Lys Ala Gly Ile Ala Ile Ala Ile Val
                485                 490                 495
Val Leu Val Ile Leu Gly Ile Phe Phe Leu Leu Ala Val Ile Tyr Tyr
            500                 505                 510
Cys Ser Arg Val Arg Lys Thr Lys Pro Lys His Asp Tyr Pro Ala Thr
        515                 520                 525
Thr Gly His Ser Ser Met Ala Tyr Val Ser
    530                 535

<210> 13
<211> 537
<212> PRT
<213> Avian penumovirus

<400> 13

Met Ser Trp Lys Val Val Leu Leu Leu Val Leu Leu Ala Thr Pro Thr
1               5                   10                  15
Gly Gly Leu Glu Glu Ser Tyr Leu Glu Glu Ser Cys Ser Thr Val Thr
            20                  25                  30
Arg Gly Tyr Leu Ser Val Leu Arg Thr Gly Trp Tyr Thr Asn Val Phe
        35                  40                  45
Thr Leu Glu Val Gly Asp Val Glu Asn Leu Thr Cys Thr Asp Gly Pro
    50                  55                  60
Ser Leu Ile Arg Thr Glu Leu Glu Leu Thr Lys Asn Ala Leu Glu Glu
65                  70                  75                  80
Leu Lys Thr Val Ser Ala Asp Gln Leu Ala Lys Glu Ala Arg Ile Met
                85                  90                  95
Ser Pro Arg Lys Ala Arg Phe Val Leu Gly Ala Ile Ala Leu Gly Val
            100                 105                 110
Ala Thr Ala Ala Ala Val Thr Ala Gly Val Ala Ile Ala Lys Thr Ile
        115                 120                 125
Arg Leu Glu Gly Glu Val Ala Ala Ile Lys Gly Ala Leu Arg Lys Thr
    130                 135                 140
Asn Glu Ala Val Ser Thr Leu Gly Asn Gly Val Arg Val Leu Ala Thr
145                 150                 155                 160
Ala Val Asn Asp Leu Lys Asp Phe Ile Ser Lys Lys Leu Thr Pro Ala
                165                 170                 175
Ile Asn Arg Asn Lys Cys Asp Ile Ser Asp Leu Lys Met Ala Val Ser
            180                 185                 190
Phe Gly Gln Tyr Asn Arg Arg Phe Leu Asn Val Val Arg Gln Phe Ser
        195                 200                 205
Asp Asn Ala Gly Ile Thr Pro Ala Ile Ser Leu Asp Leu Met Thr Asp
    210                 215                 220
Ala Glu Leu Val Arg Ala Val Ser Asn Met Pro Thr Ser Ser Gly Gln
225                 230                 235                 240
Ile Asn Leu Met Leu Glu Asn Arg Ala Met Val Arg Arg Lys Gly Phe
                245                 250                 255
Gly Ile Leu Ile Gly Val Tyr Gly Ser Ser Val Val Tyr Ile Val Gln
            260                 265                 270
Leu Pro Ile Phe Gly Val Ile Asp Thr Pro Cys Trp Lys Val Lys Ala
        275                 280                 285
Ala Pro Leu Cys Ser Gly Lys Asp Gly Asn Tyr Ala Cys Leu Leu Arg
    290                 295                 300
Glu Asp Gln Gly Trp Tyr Cys Gln Asn Ala Gly Ser Thr Val Tyr Tyr
305                 310                 315                 320
Pro Asn Glu Glu Asp Cys Glu Val Arg Ser Asp His Val Phe Cys Asp
                325                 330                 335
Thr Ala Ala Gly Ile Asn Val Ala Lys Glu Ser Glu Glu Cys Asn Arg
            340                 345                 350
Asn Ile Ser Thr Thr Lys Tyr Pro Cys Lys Val Ser Thr Gly Arg His
        355                 360                 365
Pro Ile Ser Met Val Ala Leu Ser Pro Leu Gly Ala Leu Val Ala Cys
    370                 375                 380
Tyr Asp Gly Met Ser Cys Ser Ile Gly Ser Asn Lys Val Gly Ile Ile
385                 390                 395                 400
Arg Pro Leu Gly Lys Gly Cys Ser Tyr Ile Ser Asn Gln Asp Ala Asp
                405                 410                 415
Thr Val Thr Ile Asp Asn Thr Val Tyr Gln Leu Ser Lys Val Glu Gly
            420                 425                 430
Glu Gln His Thr Ile Lys Gly Lys Pro Val Ser Ser Asn Phe Asp Pro
        435                 440                 445
Ile Glu Phe Pro Glu Asp Gln Phe Asn Ile Ala Leu Asp Gln Val Phe
    450                 455                 460
Glu Ser Val Glu Lys Ser Gln Asn Leu Ile Asp Gln Ser Asn Lys Ile
465                 470                 475                 480
Leu Asp Ser Ile Glu Lys Gly Asn Ala Gly Phe Val Ile Val Ile Val
                485                 490                 495

Leu Ile Val Leu Leu Met Leu Ala Ala Val Gly Val Gly Val Phe Phe
            500                 505                 510
Val Val Lys Lys Arg Lys Ala Ala Pro Lys Phe Pro Met Glu Met Asn
        515                 520                 525
Gly Val Asn Asn Lys Gly Phe Ile Pro
    530                 535

<210> 14
<211> 1193
<212> DNA
<213> rhinotracheitis virus

<220>
<221> CDS
<222> (16)...(1191)
<223> Turkey rhinotracheitis virus (strain CVL14/1)
      attachment protien (G) mRNA, complete cds

<400> 14
gggacaagta tctctatggg gtccaaacta tatatggctc agggcaccag tgcatatcaa 60
actgcagtgg ggttctggct ggacatcggg aggaggtaca tattggctat agtcctatca 120
gctttcgggc tgacctgcac agtcactatt gcactcactg ttagcgtcat agttgaacag 180
tcagtgttag aggagtgcag aaactacaat ggaggagata gagattggtg gtcaaccacc 240
caggagcagc caactactgc accaagtgcg actccagcag gaaattatgg aggattacaa 300
acggctcgaa caagaaagtc tgaaagctgt ttgcatgtgc aaatttctta tggtgatatg 360
tatagccgca gtgatactgt actgggtggt tttgattgta tgggcttatt ggttctttgc 420
aaatcaggac caatttgtca gcgagataat caagttgacc caacagccct ctgccattgc 480
agggtagatc tttcaagtgt ggactgctgc aaggtgaaca agattagcac taacagcagc 540
accacctctg agccccagaa gaccaacccg gcatggccta gccaagacaa cacagactcc 600
gatccaaatc cccaaggcat aaccaccagc acagccactc tgctctcaac aagtctgggc 660
ctcatgctca catcgaagac tgggacacac aaatcagggc ccccccaagc cttgccgggg 720
agcaacacca acggaaaaac aaccacagac cgagaaccag ggcccacaaa ccaaccaaat 780
tcaaccacca atgggcaaca caataaacac acccaacgaa tgacaccccc gccaagtcac 840
gacaacacaa gaaccatcct ccagcacaca acaccctggg aaaagacatt cagtacatac 900
aagcccacac actctccgac caacgaatca gatcaatccc tccccacaac tcaaaacagc 960
atcaactgtg aacattttga cccccaaggc aaggaaaaaa tctgctacag agtaggttct 1020
tacaactcca atattacaaa gcaatgcaga attgatgtgc ctttgtgttc cacttatagc 1080
acagtgtgca tgaaaacata ctataccgaa ccattcaact gttggaggcg tatctggcgt 1140
tgcttgtgtg atgacggagt tggtctggtt gagtggtgtt gcactagtta act       1193

<210> 15
<211> 1260
<212> DNA
<213> rhinotracheitis virus

<220>
<221> CDS
<222> (16)...(1260)
<223> Turkey rhinotracheitis virus (strain 6574)
      attachment protein (G), complete cds

<400> 15
gggacaagta tccagatggg gtcagagctc tacatcatag agggggtgag ctcatctgaa 60
atagtcctca agcaagtcct cagaaggagc caaaaaatac tgttaggact ggtgttatca 120
gccttaggct tgacgctcac tagcactatt gttatatcta tttgtattag tgtagaacag 180
gtcaaattac gacagtgtgt ggacacttat tgggcggaaa atggatcctt acatccagga 240
cagtcaacag aaaatacttc aacaagaggt aagactacaa caaaagaccc tagaagatta 300
caggcgactg gagcaggaaa gtttgagagc tgtgggtatg tgcaagttgt tgatggtgat 360
atgcatgatc gcagttatgc tgtactgggt ggtgttgatt gtttgggctt attggctctt 420
tgtgaatcag gaccaatttg tcagggagat acttggtctg aagacggaaa cttctgccga 480
tgcacttttt cttcccatgg ggtgagttgc tgcaaaaaac ccaaaagcaa ggcaaccact 540

gcccagagga actccaaacc agctaacagc aaatcaactc ctccggtaca ttcagacagg 600
gccagcaaag aacataatcc ctcccaaggg gagcaacccc gcagggggcc aaccagcagc 660
aagacaacta ttgctagcac cccttcaaca gaggacactg ctaaaccaac gattagcaaa 720
cctaaactca ccatcaggcc ctcgcaaaga ggtccatccg gcagcacaaa agcagcctcc 780
agcaccccca gccacaagac caacaccaga ggcaccagca agacgaccga ccagagaccc 840
cgcaccggac ccactcccga aaggcccaga caaacccaca gcacagcaac tccgcccccc 900
acaaccccaa tccacaaggg ccgggcccca acccccaaac caacaacaga cctcaaggtc 960
aacccaaggg aaggcagcac aagcccaact gcaatacaga aaaacccaac cacacaaagt 1020
aatcttgttg actgcacact gtctgatcca gatgagccac aaaggatttg ttaccaggta 1080
ggaacttaca atcctagtca atcgggaacc tgcaacatag aggttccaaa atgttccact 1140
tatgggcatg cttgtatggc tacattatat gacaccccat tcaactgctg gcgcaggacc 1200
aggagatgca tctgtgattc cggaggggag ctgattgagt ggtgctgtac tagtcaataa 1260

<210> 16
<211> 391
<212> PRT
<213> Turkey rhinotracheitis virus

<400> 16
Met Gly Ser Lys Leu Tyr Met Ala Gln Gly Thr Ser Ala Tyr Gln Thr
1               5                   10                  15
Ala Val Gly Phe Trp Leu Asp Ile Gly Arg Arg Tyr Ile Leu Ala Ile
            20                  25                  30
Val Leu Ser Ala Phe Gly Leu Thr Cys Thr Val Thr Ile Ala Leu Thr
        35                  40                  45
Val Ser Val Ile Val Glu Gln Ser Val Leu Glu Glu Cys Arg Asn Tyr
    50                  55                  60
Asn Gly Gly Asp Arg Asp Trp Trp Ser Thr Thr Gln Glu Gln Pro Thr
65                  70                  75                  80
Thr Ala Pro Ser Ala Thr Pro Ala Gly Asn Tyr Gly Gly Leu Gln Thr
                85                  90                  95
Ala Arg Thr Arg Lys Ser Glu Ser Cys Leu His Val Gln Ile Ser Tyr
            100                 105                 110
Gly Asp Met Tyr Ser Arg Ser Asp Thr Val Leu Gly Gly Phe Asp Cys
        115                 120                 125
Met Gly Leu Leu Val Leu Cys Lys Ser Gly Pro Ile Cys Gln Arg Asp
    130                 135                 140
Asn Gln Val Asp Pro Thr Ala Leu Cys His Cys Arg Val Asp Leu Ser
145                 150                 155                 160
Ser Val Asp Cys Cys Lys Val Asn Lys Ile Ser Thr Asn Ser Ser Thr
                165                 170                 175
Thr Ser Glu Pro Gln Lys Thr Asn Pro Ala Trp Pro Ser Gln Asp Asn
            180                 185                 190
Thr Asp Ser Asp Pro Asn Pro Gln Gly Ile Thr Thr Ser Thr Ala Thr
        195                 200                 205
Leu Leu Ser Thr Ser Leu Gly Leu Met Leu Thr Ser Lys Thr Gly Thr
    210                 215                 220
His Lys Ser Gly Pro Pro Gln Ala Leu Pro Gly Ser Asn Thr Asn Gly
225                 230                 235                 240
Lys Thr Thr Thr Asp Arg Glu Pro Gly Pro Thr Asn Gln Pro Asn Ser
                245                 250                 255
Thr Thr Asn Gly Gln His Asn Lys His Thr Gln Arg Met Thr Pro Pro
            260                 265                 270
Pro Ser His Asp Asn Thr Arg Thr Ile Leu Gln His Thr Thr Pro Trp
        275                 280                 285
Glu Lys Thr Phe Ser Thr Tyr Lys Pro Thr His Ser Pro Thr Asn Glu
    290                 295                 300
Ser Asp Gln Ser Leu Pro Thr Thr Gln Asn Ser Ile Asn Cys Glu His
305                 310                 315                 320
Phe Asp Pro Gln Gly Lys Glu Lys Ile Cys Tyr Arg Val Gly Ser Tyr
                325                 330                 335

Asn Ser Asn Ile Thr Lys Gln Cys Arg Ile Asp Val Pro Leu Cys Ser
                340                 345                 350
Thr Tyr Ser Thr Val Cys Met Lys Thr Tyr Tyr Thr Glu Pro Phe Asn
        355                 360                 365
Cys Trp Arg Arg Ile Trp Arg Cys Leu Cys Asp Asp Gly Val Gly Leu
    370                 375                 380
Val Glu Trp Cys Cys Thr Ser
385                 390

<210> 17
<211> 414
<212> PRT
<213> rhinotracheitis virus

<400> 17
Met Gly Ser Glu Leu Tyr Ile Ile Glu Gly Val Ser Ser Ser Glu Ile
 1               5                  10                  15
Val Leu Lys Gln Val Leu Arg Arg Ser Gln Lys Ile Leu Leu Gly Leu
                20                  25                  30
Val Leu Ser Ala Leu Gly Leu Thr Leu Thr Ser Thr Ile Val Ile Ser
        35                  40                  45
Ile Cys Ile Ser Val Glu Gln Val Lys Leu Arg Gln Cys Val Asp Thr
    50                  55                  60
Tyr Trp Ala Glu Asn Gly Ser Leu His Pro Gly Gln Ser Thr Glu Asn
65                  70                  75                  80
Thr Ser Thr Arg Gly Lys Thr Thr Thr Lys Asp Pro Arg Arg Leu Gln
                85                  90                  95
Ala Thr Gly Ala Gly Lys Phe Glu Ser Cys Gly Tyr Val Gln Val Val
                100                 105                 110
Asp Gly Asp Met His Asp Arg Ser Tyr Ala Val Leu Gly Gly Val Asp
        115                 120                 125
Cys Leu Gly Leu Leu Ala Leu Cys Glu Ser Gly Pro Ile Cys Gln Gly
    130                 135                 140
Asp Thr Trp Ser Glu Asp Gly Asn Phe Cys Arg Cys Thr Phe Ser Ser
145                 150                 155                 160
His Gly Val Ser Cys Cys Lys Lys Pro Lys Ser Lys Ala Thr Thr Ala
                165                 170                 175
Gln Arg Asn Ser Lys Pro Ala Asn Ser Lys Ser Thr Pro Pro Val His
                180                 185                 190
Ser Asp Arg Ala Ser Lys Glu His Asn Pro Ser Gln Gly Glu Gln Pro
        195                 200                 205
Arg Arg Gly Pro Thr Ser Ser Lys Thr Thr Ile Ala Ser Thr Pro Ser
    210                 215                 220
Thr Glu Asp Thr Ala Lys Pro Thr Ile Ser Lys Pro Lys Leu Thr Ile
225                 230                 235                 240
Arg Pro Ser Gln Arg Gly Pro Ser Gly Ser Thr Lys Ala Ala Ser Ser
                245                 250                 255
Thr Pro Ser His Lys Thr Asn Thr Arg Gly Thr Ser Lys Thr Thr Asp
                260                 265                 270
Gln Arg Pro Arg Thr Gly Pro Thr Pro Glu Arg Pro Arg Gln Thr His
        275                 280                 285
Ser Thr Ala Thr Pro Pro Pro Thr Thr Pro Ile His Lys Gly Arg Ala
    290                 295                 300
Pro Thr Pro Lys Pro Thr Thr Asp Leu Lys Val Asn Pro Arg Glu Gly
305                 310                 315                 320
Ser Thr Ser Pro Thr Ala Ile Gln Lys Asn Pro Thr Thr Gln Ser Asn
                325                 330                 335
Leu Val Asp Cys Thr Leu Ser Asp Pro Asp Glu Pro Gln Arg Ile Cys
                340                 345                 350
Tyr Gln Val Gly Thr Tyr Asn Pro Ser Gln Ser Gly Thr Cys Asn Ile
        355                 360                 365

Glu Val Pro Lys Cys Ser Thr Tyr Gly His Ala Cys Met Ala Thr Leu
    370                 375                 380
Tyr Asp Thr Pro Phe Asn Cys Trp Arg Arg Thr Arg Arg Cys Ile Cys
385                 390                 395                 400
Asp Ser Gly Gly Glu Leu Ile Glu Trp Cys Cys Thr Ser Gln
                405                 410

<210> 18
<211> 539
<212> PRT
<213> human Metapneumo virus

<400> 18
Met Ser Trp Lys Val Val Ile Ile Phe Ser Leu Leu Ile Thr Pro Gln
 1               5                  10                  15
His Gly Leu Lys Glu Ser Tyr Leu Glu Glu Ser Cys Ser Thr Ile Thr
            20                  25                  30
Glu Gly Tyr Leu Ser Val Leu Arg Thr Gly Trp Tyr Thr Asn Val Phe
        35                  40                  45
Thr Leu Glu Val Gly Asp Val Glu Asn Leu Thr Cys Ala Asp Gly Pro
    50                  55                  60
Ser Leu Ile Lys Thr Glu Leu Asp Leu Thr Lys Ser Ala Leu Arg Glu
65                  70                  75                  80
Leu Arg Thr Val Ser Ala Asp Gln Leu Ala Arg Glu Glu Gln Ile Glu
                85                  90                  95
Asn Pro Arg Gln Ser Arg Phe Val Leu Gly Ala Ile Ala Leu Gly Val
            100                 105                 110
Ala Thr Ala Ala Ala Val Thr Ala Gly Val Ala Ile Ala Lys Thr Ile
        115                 120                 125
Arg Leu Glu Ser Glu Val Thr Ala Ile Lys Asn Ala Leu Lys Lys Thr
    130                 135                 140
Asn Glu Ala Val Ser Thr Leu Gly Asn Gly Val Arg Val Leu Ala Thr
145                 150                 155                 160
Ala Val Arg Glu Leu Lys Asp Phe Val Ser Lys Asn Leu Thr Arg Ala
                165                 170                 175
Ile Asn Lys Asn Lys Cys Asp Ile Ala Asp Leu Lys Met Ala Val Ser
            180                 185                 190
Phe Ser Gln Phe Asn Arg Arg Phe Leu Asn Val Val Arg Gln Phe Ser
        195                 200                 205
Asp Asn Ala Gly Ile Thr Pro Ala Ile Ser Leu Asp Leu Met Thr Asp
    210                 215                 220
Ala Glu Leu Ala Arg Ala Val Ser Asn Met Pro Thr Ser Ala Gly Gln
225                 230                 235                 240
Ile Lys Leu Met Leu Glu Asn Arg Ala Met Val Arg Arg Lys Gly Phe
                245                 250                 255
Gly Ile Leu Ile Gly Val Tyr Gly Ser Ser Val Ile Tyr Met Val Gln
            260                 265                 270
Leu Pro Ile Phe Gly Val Ile Asp Thr Pro Cys Trp Ile Val Lys Ala
        275                 280                 285
Ala Pro Ser Cys Ser Gly Lys Lys Gly Asn Tyr Ala Cys Leu Leu Arg
    290                 295                 300
Glu Asp Gln Gly Trp Tyr Cys Gln Asn Ala Gly Ser Thr Val Tyr Tyr
305                 310                 315                 320
Pro Asn Glu Lys Asp Cys Glu Thr Arg Gly Asp His Val Phe Cys Asp
                325                 330                 335
Thr Ala Ala Gly Ile Asn Val Ala Glu Gln Ser Lys Glu Cys Asn Ile
            340                 345                 350
Asn Ile Ser Thr Thr Asn Tyr Pro Cys Lys Val Ser Thr Gly Arg His
        355                 360                 365
Pro Ile Ser Met Val Ala Leu Ser Pro Leu Gly Ala Leu Val Ala Cys
    370                 375                 380

Tyr Lys Gly Val Ser Cys Ser Ile Gly Ser Asn Arg Val Gly Ile Ile
385                 390                 395                 400
Lys Gln Leu Asn Lys Gly Cys Ser Tyr Ile Thr Asn Gln Asp Ala Asp
                405                 410                 415
Thr Val Thr Ile Asp Asn Thr Val Tyr Gln Leu Ser Lys Val Glu Gly
            420                 425                 430
Glu Gln His Val Ile Lys Gly Arg Pro Val Ser Ser Ser Phe Asp Pro
        435                 440                 445
Val Lys Phe Pro Glu Asp Gln Phe Asn Val Ala Leu Asp Gln Val Phe
    450                 455                 460
Glu Ser Ile Glu Asn Ser Gln Ala Leu Val Asp Gln Ser Asn Arg Ile
465                 470                 475                 480
Leu Ser Ser Ala Glu Lys Gly Asn Thr Gly Phe Ile Ile Val Ile Ile
                485                 490                 495
Leu Ile Ala Val Leu Gly Ser Thr Met Ile Leu Val Ser Val Phe Ile
            500                 505                 510
Ile Ile Lys Lys Thr Lys Lys Pro Thr Gly Ala Pro Pro Glu Leu Ser
        515                 520                 525
Gly Val Thr Asn Asn Gly Phe Ile Pro His Asn
    530                 535

<210> 19
<211> 539
<212> PRT
<213> human Metapneumo virus

<400> 19
Met Ser Trp Lys Val Val Ile Ile Phe Ser Leu Leu Ile Thr Pro Gln
1               5                   10                  15
His Gly Leu Lys Glu Ser Tyr Leu Glu Glu Ser Cys Ser Thr Ile Thr
            20                  25                  30
Glu Gly Tyr Leu Ser Val Leu Arg Thr Gly Trp Tyr Thr Asn Val Phe
        35                  40                  45
Thr Leu Glu Val Gly Asp Val Glu Asn Leu Thr Cys Ser Asp Gly Pro
    50                  55                  60
Ser Leu Ile Lys Thr Glu Leu Asp Leu Thr Lys Ser Ala Leu Arg Glu
65                  70                  75                  80
Leu Lys Thr Val Ser Ala Asp Gln Leu Ala Arg Glu Glu Gln Ile Glu
                85                  90                  95
Asn Pro Arg Gln Ser Arg Phe Val Leu Gly Ala Ile Ala Leu Gly Val
            100                 105                 110
Ala Thr Ala Ala Ala Val Thr Ala Gly Val Ala Ile Ala Lys Thr Ile
        115                 120                 125
Arg Leu Glu Ser Glu Val Thr Ala Ile Lys Asn Ala Leu Lys Thr Thr
    130                 135                 140
Asn Glu Ala Val Ser Thr Leu Gly Asn Gly Val Arg Val Leu Ala Thr
145                 150                 155                 160
Ala Val Arg Glu Leu Lys Asp Phe Val Ser Lys Asn Leu Thr Arg Ala
                165                 170                 175
Ile Asn Lys Asn Lys Cys Asp Ile Asp Asp Leu Lys Met Ala Val Ser
            180                 185                 190
Phe Ser Gln Phe Asn Arg Arg Phe Leu Asn Val Val Arg Gln Phe Ser
        195                 200                 205
Asp Asn Ala Gly Ile Thr Pro Ala Ile Ser Leu Asp Leu Met Thr Asp
    210                 215                 220
Ala Glu Leu Ala Arg Ala Val Ser Asn Met Pro Thr Ser Ala Gly Gln
225                 230                 235                 240
Ile Lys Leu Met Leu Glu Asn Arg Ala Met Val Arg Arg Lys Gly Phe
                245                 250                 255
Gly Ile Leu Ile Gly Val Tyr Gly Ser Ser Val Ile Tyr Thr Val Gln
            260                 265                 270

Leu Pro Ile Phe Gly Val Ile Asp Thr Pro Cys Trp Ile Val Lys Ala
        275                 280                 285
Ala Pro Ser Cys Ser Glu Lys Lys Gly Asn Tyr Ala Cys Leu Leu Arg
    290                 295                 300
Glu Asp Gln Gly Trp Tyr Cys Gln Asn Ala Gly Ser Thr Val Tyr Tyr
305                 310                 315                 320
Pro Asn Glu Lys Asp Cys Glu Thr Arg Gly Asp His Val Phe Cys Asp
                325                 330                 335
Thr Ala Ala Gly Ile Asn Val Ala Glu Gln Ser Lys Glu Cys Asn Ile
            340                 345                 350
Asn Ile Ser Thr Thr Asn Tyr Pro Cys Lys Val Ser Thr Gly Arg His
        355                 360                 365
Pro Ile Ser Met Val Ala Leu Ser Pro Leu Gly Ala Leu Val Ala Cys
    370                 375                 380
Tyr Lys Gly Val Ser Cys Ser Ile Gly Ser Asn Arg Val Gly Ile Ile
385                 390                 395                 400
Lys Gln Leu Asn Lys Gly Cys Ser Tyr Ile Thr Asn Gln Asp Ala Asp
                405                 410                 415
Thr Val Thr Ile Asp Asn Thr Val Tyr Gln Leu Ser Lys Val Glu Gly
            420                 425                 430
Glu Gln His Val Ile Lys Gly Arg Pro Val Ser Ser Ser Phe Asp Pro
        435                 440                 445
Ile Lys Phe Pro Glu Asp Gln Phe Asn Val Ala Leu Asp Gln Val Phe
    450                 455                 460
Glu Asn Ile Glu Asn Ser Gln Ala Leu Val Asp Gln Ser Asn Arg Ile
465                 470                 475                 480
Leu Ser Ser Ala Glu Lys Gly Asn Thr Gly Phe Ile Ile Val Ile Ile
                485                 490                 495
Leu Ile Ala Val Leu Gly Ser Ser Met Ile Leu Val Ser Ile Phe Ile
            500                 505                 510
Ile Ile Lys Lys Thr Lys Lys Pro Thr Gly Ala Pro Pro Glu Leu Ser
        515                 520                 525
Gly Val Thr Asn Asn Gly Phe Ile Pro His Ser
    530                 535

<210> 20
<211> 539
<212> PRT
<213> human Metapneumo virus

<400> 20
Met Ser Trp Lys Val Met Ile Ile Ile Ser Leu Leu Ile Thr Pro Gln
 1               5                  10                  15
His Gly Leu Lys Glu Ser Tyr Leu Glu Glu Ser Cys Ser Thr Ile Thr
            20                  25                  30
Glu Gly Tyr Leu Ser Val Leu Arg Thr Gly Trp Tyr Thr Asn Val Phe
        35                  40                  45
Thr Leu Glu Val Gly Asp Val Glu Asn Leu Thr Cys Thr Asp Gly Pro
    50                  55                  60
Ser Leu Ile Lys Thr Glu Leu Asp Leu Thr Lys Ser Ala Leu Arg Glu
65                  70                  75                  80
Leu Lys Thr Val Ser Ala Asp Gln Leu Ala Arg Glu Glu Gln Ile Glu
                85                  90                  95
Asn Pro Arg Gln Ser Arg Phe Val Leu Gly Ala Ile Ala Leu Gly Val
            100                 105                 110
Ala Thr Ala Ala Ala Val Thr Ala Gly Ile Ala Ile Ala Lys Thr Ile
        115                 120                 125
Arg Leu Glu Ser Glu Val Asn Ala Ile Lys Gly Ala Leu Lys Gln Thr
    130                 135                 140
Asn Glu Ala Val Ser Thr Leu Gly Asn Gly Val Arg Val Leu Ala Thr
145                 150                 155                 160

Ala Val Arg Glu Leu Lys Glu Phe Val Ser Lys Asn Leu Thr Ser Ala
                165                 170                 175
Ile Asn Arg Asn Lys Cys Asp Ile Ala Asp Leu Lys Met Ala Val Ser
            180                 185                 190
Phe Ser Gln Phe Asn Arg Arg Phe Leu Asn Val Val Arg Gln Phe Ser
        195                 200                 205
Asp Asn Ala Gly Ile Thr Pro Ala Ile Ser Leu Asp Leu Met Thr Asp
    210                 215                 220
Ala Glu Leu Ala Arg Ala Val Ser Tyr Met Pro Thr Ser Ala Gly Gln
225                 230                 235                 240
Ile Lys Leu Met Leu Glu Asn Arg Ala Met Val Arg Arg Lys Gly Phe
                245                 250                 255
Gly Ile Leu Ile Gly Val Tyr Gly Ser Ser Val Ile Tyr Met Val Gln
            260                 265                 270
Leu Pro Ile Phe Gly Val Ile Asp Thr Pro Cys Trp Ile Ile Lys Ala
        275                 280                 285
Ala Pro Ser Cys Ser Glu Lys Asn Gly Asn Tyr Ala Cys Leu Leu Arg
    290                 295                 300
Glu Asp Gln Gly Trp Tyr Cys Lys Asn Ala Gly Ser Thr Val Tyr Tyr
305                 310                 315                 320
Pro Asn Glu Lys Asp Cys Glu Thr Arg Gly Asp His Val Phe Cys Asp
                325                 330                 335
Thr Ala Ala Gly Ile Asn Val Ala Glu Gln Ser Arg Glu Cys Asn Ile
            340                 345                 350
Asn Ile Ser Thr Thr Asn Tyr Pro Cys Lys Val Ser Thr Gly Arg His
        355                 360                 365
Pro Ile Ser Met Val Ala Leu Ser Pro Leu Gly Ala Leu Val Ala Cys
    370                 375                 380
Tyr Lys Gly Val Ser Cys Ser Ile Gly Ser Asn Trp Val Gly Ile Ile
385                 390                 395                 400
Lys Gln Leu Pro Lys Gly Cys Ser Tyr Ile Thr Asn Gln Asp Ala Asp
                405                 410                 415
Thr Val Thr Ile Asp Asn Thr Val Tyr Gln Leu Ser Lys Val Glu Gly
            420                 425                 430
Glu Gln His Val Ile Lys Gly Arg Pro Val Ser Ser Ser Phe Asp Pro
        435                 440                 445
Ile Lys Phe Pro Glu Asp Gln Phe Asn Val Ala Leu Asp Gln Val Phe
    450                 455                 460
Glu Ser Ile Glu Asn Ser Gln Ala Leu Val Asp Gln Ser Asn Lys Ile
465                 470                 475                 480
Leu Asn Ser Ala Glu Lys Gly Asn Thr Gly Phe Ile Ile Val Val Ile
                485                 490                 495
Leu Val Ala Val Leu Gly Leu Thr Met Ile Ser Val Ser Ile Ile Ile
            500                 505                 510
Ile Ile Lys Lys Thr Arg Lys Pro Thr Gly Ala Pro Pro Glu Leu Asn
        515                 520                 525
Gly Val Thr Asn Gly Gly Phe Ile Pro His Ser
    530                 535

<210> 21
<211> 539
<212> PRT
<213> human Metapneumo virus

<400> 21
Met Ser Trp Lys Val Met Ile Ile Ile Ser Leu Leu Ile Thr Pro Gln
1               5                   10                  15
His Gly Leu Lys Glu Ser Tyr Leu Glu Glu Ser Cys Ser Thr Ile Thr
            20                  25                  30
Glu Gly Tyr Leu Ser Val Leu Arg Thr Gly Trp Tyr Thr Asn Val Phe
        35                  40                  45

Thr Leu Glu Val Gly Asp Val Glu Asn Leu Thr Cys Thr Asp Gly Pro
    50                  55                  60
Ser Leu Ile Lys Thr Glu Leu Asp Leu Thr Lys Ser Ala Leu Arg Glu
65                  70                  75                  80
Leu Lys Thr Val Ser Ala Asp Gln Leu Ala Arg Glu Glu Gln Ile Glu
                85                  90                  95
Asn Pro Arg Gln Ser Arg Phe Val Leu Gly Ala Ile Ala Leu Gly Val
            100                 105                 110
Ala Thr Ala Ala Ala Val Thr Ala Gly Ile Ala Ile Ala Lys Thr Ile
        115                 120                 125
Arg Leu Glu Ser Glu Val Asn Ala Ile Lys Gly Ala Leu Lys Thr Thr
    130                 135                 140
Asn Glu Ala Val Ser Thr Leu Gly Asn Gly Val Arg Val Leu Ala Thr
145                 150                 155                 160
Ala Val Arg Glu Leu Lys Glu Phe Val Ser Lys Asn Leu Thr Ser Ala
                165                 170                 175
Ile Asn Lys Asn Lys Cys Asp Ile Ala Asp Leu Lys Met Ala Val Ser
            180                 185                 190
Phe Ser Gln Phe Asn Arg Arg Phe Leu Asn Val Val Arg Gln Phe Ser
        195                 200                 205
Asp Asn Ala Gly Ile Thr Pro Ala Ile Ser Leu Asp Leu Met Thr Asp
    210                 215                 220
Ala Glu Leu Ala Arg Ala Val Ser Tyr Met Pro Thr Ser Ala Gly Gln
225                 230                 235                 240
Ile Lys Leu Met Leu Glu Asn Arg Ala Met Val Arg Arg Lys Gly Phe
                245                 250                 255
Gly Ile Leu Ile Gly Val Tyr Gly Ser Ser Val Ile Tyr Met Val Gln
            260                 265                 270
Leu Pro Ile Phe Gly Val Ile Asp Thr Pro Cys Trp Ile Ile Lys Ala
        275                 280                 285
Ala Pro Ser Cys Ser Glu Lys Asp Gly Asn Tyr Ala Cys Leu Leu Arg
    290                 295                 300
Glu Asp Gln Gly Trp Tyr Cys Lys Asn Ala Gly Ser Thr Val Tyr Tyr
305                 310                 315                 320
Pro Asn Glu Lys Asp Cys Glu Thr Arg Gly Asp His Val Phe Cys Asp
                325                 330                 335
Thr Ala Ala Gly Ile Asn Val Ala Glu Gln Ser Arg Glu Cys Asn Ile
            340                 345                 350
Asn Ile Ser Thr Thr Asn Tyr Pro Cys Lys Val Ser Thr Gly Arg His
        355                 360                 365
Pro Ile Ser Met Val Ala Leu Ser Pro Leu Gly Ala Leu Val Ala Cys
    370                 375                 380
Tyr Lys Gly Val Ser Cys Ser Ile Gly Ser Asn Arg Val Gly Ile Ile
385                 390                 395                 400
Lys Gln Leu Pro Lys Gly Cys Ser Tyr Ile Thr Asn Gln Asp Ala Asp
                405                 410                 415
Thr Val Thr Ile Asp Asn Thr Val Tyr Gln Leu Ser Lys Val Glu Gly
            420                 425                 430
Glu Gln His Val Ile Lys Gly Arg Pro Val Ser Ser Ser Phe Asp Pro
        435                 440                 445
Ile Arg Phe Pro Glu Asp Gln Phe Asn Val Ala Leu Asp Gln Val Phe
    450                 455                 460
Glu Ser Ile Glu Asn Ser Gln Ala Leu Val Asp Gln Ser Asn Lys Ile
465                 470                 475                 480
Leu Asn Ser Ala Glu Lys Gly Asn Thr Gly Phe Ile Ile Val Ile Ile
                485                 490                 495
Leu Ile Ala Val Leu Gly Leu Thr Met Ile Ser Val Ser Ile Ile Ile
            500                 505                 510
Ile Ile Lys Lys Thr Arg Lys Pro Thr Gly Ala Pro Pro Glu Leu Asn
        515                 520                 525
Gly Val Thr Asn Gly Gly Phe Ile Pro His Ser
    530                 535

<210> 22
<211> 1620
<212> DNA
<213> human Metapneumo virus

<400> 22
atgtcttgga aagtggtgat cattttttca ttgttaataa cacctcaaca cggtcttaaa 60
gagagctact tagaagagtc atgtagcact ataactgaag gatatctcag tgttctgagg 120
acaggttggt acaccaatgt ttttacactg gaggtaggcg atgtagagaa ccttacatgt 180
gccgatggac ccagcttaat aaaaacagaa ttagacctga ccaaaagtgc actaagagag 240
ctcagaacag tttctgctga tcaactggca agagaggagc aaattgaaaa tcccagacaa 300
tctagattcg ttctaggagc aatagcactc ggtgttgcaa ctgcagctgc agttacagca 360
ggtgttgcaa ttgccaaaac catccggctt gaaagtgaag taacagcaat taagaatgcc 420
ctcaaaaaga ccaatgaagc agtatctaca ttggggaatg gagttcgtgt gttggcaact 480
gcagtgagag agctgaaaga ttttgtgagc aagaatctaa cacgtgcaat caacaaaaac 540
aagtgcgaca ttgctgacct gaaaatggcc gttagcttca gtcaattcaa cagaaggttc 600
ctaaatgttg tgcggcaatt ttcagacaac gctggaataa caccagcaat atctttggac 660
ttaatgacag atgctgaact agccagagct gtttccaaca tgccaacatc tgcaggacaa 720
ataaaactga tgttggagaa ccgtgcaatg gtaagaagaa aagggttcgg aatcctgata 780
ggagtttacg gaagctccgt aatttacatg gtgcaactgc caatctttgg ggttatagac 840
acgccttgct ggatagtaaa agcagcccct tcttgttcag gaaaaaaggg aaactatgct 900
tgcctcttaa gagaagacca aggatggtat tgtcaaaatg cagggtcaac tgtttactac 960
ccaaatgaaa aagactgtga aacaagagga gaccatgtct tttgcgacac agcagcagga 1020
atcaatgttg ctgagcagtc aaaggagtgc aacataaaca tatctactac taattaccca 1080
tgcaaagtta gcacaggaag acatcctatc agtatggttg cactatctcc tcttggggct 1140
ttggttgctt gctacaaggg agtgagctgt tccattggca gcaacagagt agggatcatc 1200
aagcaactga acaaaggctg ctcttatata accaaccaag acgcagacac agtgacaata 1260
gacaacactg tataccagct aagcaaagtt gaaggcgaac agcatgttat aaaaggaagg 1320
ccagtgtcaa gcagctttga cccagtcaag tttcctgaag atcaattcaa tgttgcactt 1380
gaccaagttt tcgagagcat tgagaacagt caggccttgg tggatcaatc aaacagaatc 1440
ctaagcagtg cagagaaagg aaacactggc ttcatcattg taataattct aattgctgtc 1500
cttggctcta ccatgatcct agtgagtgtt tttatcataa taaagaaaac aaagaaaccc 1560
acaggagcac ctccagagct gagtggtgtc acaaacaatg gcttcatacc acataattag 1620

<210> 23
<211> 1620
<212> DNA
<213> human Metapneumo virus

<400> 23
atgtcttgga aagtggtgat cattttttca ttgctaataa cacctcaaca cggtcttaaa 60
gagagctacc tagaagaatc atgtagcact ataactgagg gatatcttag tgttctgagg 120
acaggttggt ataccaacgt ttttacatta gaggtgggtg atgtagaaaa ccttacatgt 180
tctgatggac ctagcctaat aaaaacagaa ttagatctga ccaaaagtgc actaagagag 240
ctcaaaacag tctctgctga ccaattggca agagaggaac aaattgagaa tcccagacaa 300
tctaggtttg ttctaggagc aatagcactc ggtgttgcaa cagcagctgc agtcacagca 360
ggtgttgcaa ttgccaaaac catccggctt gagagtgaag tcacagcaat taagaatgcc 420
ctcaaaacga ccaatgaagc agtatctaca ttggggaatg gagttcgagt gttggcaact 480
gcagtgagag agctaaaaga ctttgtgagc aagaatttaa ctcgtgcaat caacaaaaac 540
aagtgcgaca ttgatgacct aaaaatggct gttagcttca gtcaattcaa cagaaggttt 600
ctaaatgttg tgcggcaatt ttcagacaat gctggaataa caccagcaat atctttggac 660
ttaatgacag atgctgaact agccagggcc gtttctaaca tgccgacatc tgcaggacaa 720
ataaaattga tgttggagaa ccgtgcgatg gtgcgaagaa aggggttcgg aatcctgata 780
ggggtctacg ggagctccgt aatttacacg gtgcagctgc caatctttgg cgttatagac 840
acgccttgct ggatagtaaa agcagcccct tcttgttccg aaaaaaaggg aaactatgct 900
tgcctcttaa gagaagacca agggtggtat tgtcagaatg cagggtcaac tgtttactac 960
ccaaatgaga aagactgtga aacaagagga gaccatgtct tttgcgacac agcagcagga 1020
attaatgttg ctgagcaatc aaaggagtgc aacatcaaca tatccactac aaattaccca 1080
tgcaaagtca gcacaggaag acatcctatc agtatggttg cactgtctcc tcttggggct 1140
ctggttgctt gctacaaagg agtaagctgt tccattggca gcaacagagt agggatcatc 1200

aagcagctga acaaaggttg ctcctatata accaaccaag atgcagacac agtgacaata 1260
gacaacactg tatatcagct aagcaaagtt gagggtgaac agcatgttat aaaaggcaga 1320
ccagtgtcaa gcagctttga tccaatcaag tttcctgaag atcaattcaa tgttgcactt 1380
gaccaagttt ttgagaacat tgaaaacagc caggccttag tagatcaatc aaacagaatc 1440
ctaagcagtg cagagaaagg gaatactggc tttatcattg taataattct aattgctgtc 1500
cttggctcta gcatgatcct agtgagcatc ttcattataa tcaagaaaac aaagaaacca 1560
acgggagcac ctccagagct gagtggtgtc acaaacaatg gcttcatacc acacagttag 1620

<210> 24
<211> 1620
<212> DNA
<213> human Metapneumo virus

<400> 24
atgtcttgga aagtgatgat catcatttcg ttactcataa caccccagca cgggctaaag 60
gagagttatt tggaagaatc atgtagtact ataactgagg gatacctcag tgttttaaga 120
acaggctggt acactaatgt cttcacatta gaagttggtg atgttgaaaa tcttacatgt 180
actgatggac ctagcttaat caaaacagaa cttgatctaa caaaaagtgc tttaagggaa 240
ctcaaaacag tctctgctga tcagttggcg agagaggagc aaattgaaaa tcccagacaa 300
tcaagatttg tcttaggtgc gatagctctc ggagttgcta cagcagcagc agtcacagca 360
ggcattgcaa tagccaaaac cataaggctt gagagtgagg tgaatgcaat taaaggtgct 420
ctcaaacaaa ctaatgaagc agtatccaca ttagggaatg gtgtgcgggt cctagccact 480
gcagtgagag agctaaaaga atttgtgagc aaaaacctga ctagtgcaat caacaggaac 540
aaatgtgaca ttgctgatct gaagatggct gtcagcttca gtcaattcaa cagaagattt 600
ctaaatgttg tgcggcagtt ttcagacaat gcagggataa caccagcaat atcattggac 660
ctgatgactg atgctgagtt ggccagagct gtatcataca tgccaacatc tgcagggcag 720
ataaaactga tgttggagaa ccgcgcaatg gtaaggagaa aaggatttgg aatcctgata 780
ggggtctacg gaagctctgt gatttacatg gttcaattgc cgatctttgg tgtcatagat 840
acaccttgtt ggatcatcaa ggcagctccc tcttgctcag aaaaaaacgg gaattatgct 900
tgcctcctaa gagaggatca agggtggtat tgtaaaaatg caggatctac tgtttactac 960
ccaaatgaaa aagactgcga aacaagaggt gatcatgttt tttgtgacac agcagcaggg 1020
atcaatgttg ctgagcaatc aagagaatgc aacatcaaca tatctactac caactaccca 1080
tgcaaagtca gcacaggaag acaccctata agcatggttg cactatcacc tctcggtgct 1140
ttggtggctt gctataaagg ggtaagctgc tcgattggca gcaattgggt tggaatcatc 1200
aaacaattac ccaaaggctg ctcatacata accaaccagg atgcagacac tgtaacaatt 1260
gacaataccg tgtatcaact aagcaaagtt gaaggtgaac agcatgtaat aaaaggagga 1320
ccagtttcaa gcagttttga tccaatcaag tttcctgagg atcagttcaa tgttgcgctt 1380
gatcaagtct tcgaaagcat tgagaacagt caggcactag tggaccagtc aaacaaaatt 1440
ctaaacagtg cagaaaaagg aaacactggt ttcattatcg tagtaatttt ggttgctgtt 1500
cttggtctaa ccatgatttc agtgagcatc atcatcataa tcaagaaaac aaggaagccc 1560
acaggagcac ctccagagct gaatggtgtc accaacggcg gtttcatacc acatagttag 1620

<210> 25
<211> 1620
<212> DNA
<213> human Metapneumo virus

<400> 25
atgtcttgga aagtgatgat tatcatttcg ttactcataa cacctcagca cggactaaaa 60
gaaagttatt tagaagaatc atgtagtact ataactgaag gatatctcag tgttttaaga 120
acaggttggt acaccaatgt ctttacatta gaagttggtg atgttgaaaa tcttacatgt 180
actgatggac ctagcttaat caaaacagaa cttgacctaa ccaaaagtgc tctgagagaa 240
ctcaaaacag tttctgctga tcagttagcg agagaagaac aaattgaaaa tcccagacaa 300
tcaaggtttg tcctaggtgc aatagctctt ggagttgcca cagcagcagc agtcacagca 360
ggcattgcaa tagccaaaac cataagactt gagagtgaag tgaatgcaat caaaggtgct 420
ctcaaaacaa ccaacgaggc agtatccaca ctaggaaatg gagtgcgagt cctagccact 480
gcagtaagag agctgaaaga atttgtgagc aaaaacctga ctagtgcgat caacaagaac 540
aaatgtgaca ttgctgatct gaagatggct gtcagcttca gtcaattcaa cagaagattc 600
ctaaatgttg tgcggcagtt ttcagacaat gcagggataa caccagcaat atcattggac 660
ctaatgactg atgctgagct ggccagagct gtatcataca tgccaacatc tgcaggacag 720

ataaaactaa tgttagagaa ccgtgcaatg gtgaggagaa aaggatttgg aatcttgata 780
ggggtctacg gaagctctgt gatttacatg gtccagctgc cgatctttgg tgtcatagat 840
acaccttgtt ggataatcaa ggcagctccc tcttgttcag aaaaagatgg aaattatgct 900
tgcctcctaa gagaggatca agggtggtat tgcaaaaatg caggatccac tgtttactac 960
ccaaatgaaa aagactgcga aacaagaggt gatcatgttt tttgtgacac agcagcaggg 1020
atcaatgttg ctgagcaatc aagagaatgc aacatcaaca tatctaccac caactaccca 1080
tgcaaagtca gcacaggaag acaccctatc agcatggttg cactatcacc tctcggtgct 1140
ttggtagctt gctacaaggg ggttagctgc tcgattggca gtaatcgggt tggaataatc 1200
aaacaactac ctaaaggctg ctcatacata actaaccagg acgcagacac tgtaacaatt 1260
gacaacactg tgtatcaact aagcaaagtt gagggtgaac agcatgtaat aaaagggaga 1320
ccagtttcaa gcagttttga tccaatcagg tttcctgagg atcagttcaa tgttgcgctt 1380
gatcaagtct ttgaaagcat tgaaaacagt caagcactag tggaccagtc aaacaaaatt 1440
ctgaacagtg cagaaaaagg aaacactggt ttcattattg taataatttt gattgctgtt 1500
cttgggttaa ccatgatttc agtgagcatc atcatcataa tcaaaaaaac aaggaagccc 1560
acaggggcac ctccagagct gaatggtgtt accaacggcg gttttatacc gcatagttag 1620

<210> 26
<211> 236
<212> PRT
<213> human Metapneumo virus

<400> 26
Met Glu Val Lys Val Glu Asn Ile Arg Thr Ile Asp Met Leu Lys Ala
1               5                   10                  15
Arg Val Lys Asn Arg Val Ala Arg Ser Lys Cys Phe Lys Asn Ala Ser
            20                  25                  30
Leu Val Leu Ile Gly Ile Thr Thr Leu Ser Ile Ala Leu Asn Ile Tyr
        35                  40                  45
Leu Ile Ile Asn Tyr Lys Met Gln Lys Asn Thr Ser Glu Ser Glu His
    50                  55                  60
His Thr Ser Ser Ser Pro Met Glu Ser Ser Arg Glu Thr Pro Thr Val
65                  70                  75                  80
Pro Thr Asp Asn Ser Asp Thr Asn Ser Ser Pro Gln His Pro Thr Gln
                85                  90                  95
Gln Ser Thr Glu Gly Ser Thr Leu Tyr Phe Ala Ala Ser Ala Ser Ser
            100                 105                 110
Pro Glu Thr Glu Pro Thr Ser Thr Pro Asp Thr Thr Asn Arg Pro Pro
        115                 120                 125
Phe Val Asp Thr His Thr Thr Pro Pro Ser Ala Ser Arg Thr Lys Thr
    130                 135                 140
Ser Pro Ala Val His Thr Lys Asn Asn Pro Arg Thr Ser Ser Arg Thr
145                 150                 155                 160
His Ser Pro Pro Arg Ala Thr Thr Arg Thr Ala Arg Arg Thr Thr Thr
                165                 170                 175
Leu Arg Thr Ser Ser Thr Arg Lys Arg Pro Ser Thr Ala Ser Val Gln
            180                 185                 190
Pro Asp Ile Ser Ala Thr Thr His Lys Asn Glu Glu Ala Ser Pro Ala
        195                 200                 205
Ser Pro Gln Thr Ser Ala Ser Thr Thr Arg Ile Gln Arg Lys Ser Val
    210                 215                 220
Glu Ala Asn Thr Ser Thr Thr Tyr Asn Gln Thr Ser
225                 230                 235

<210> 27
<211> 219
<212> PRT
<213> human Metapneumo virus

<400> 27
Met Glu Val Lys Val Glu Asn Ile Arg Ala Ile Asp Met Leu Lys Ala

1               5                   10                  15
Arg Val Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Lys Thr Thr Ser Glu Ser Glu His
    50                  55                  60
His Thr Ser Ser Pro Pro Thr Glu Pro Asn Lys Glu Ala Ser Thr Ile
65                  70                  75                  80
Ser Thr Asp Asn Pro Asp Ile Asn Pro Ser Ser Gln His Pro Thr Gln
                85                  90                  95
Gln Ser Thr Glu Asn Pro Thr Leu Asn Pro Ala Ala Ser Ala Ser Pro
            100                 105                 110
Ser Glu Thr Glu Pro Ala Ser Thr Pro Asp Thr Thr Asn Arg Leu Ser
        115                 120                 125
Ser Val Asp Arg Ser Thr Ala Gln Pro Ser Glu Ser Arg Thr Lys Thr
    130                 135                 140
Lys Pro Thr Val His Thr Ile Asn Asn Pro Asn Thr Ala Ser Ser Thr
145                 150                 155                 160
Gln Ser Pro Pro Arg Thr Thr Thr Lys Ala Ile Arg Arg Ala Thr Thr
                165                 170                 175
Phe Arg Met Ser Ser Thr Gly Lys Arg Pro Thr Thr Thr Leu Val Gln
            180                 185                 190
Ser Asp Ser Ser Thr Thr Thr Gln Asn His Glu Glu Thr Gly Ser Ala
        195                 200                 205
Asn Pro Gln Ala Ser Ala Ser Thr Met Gln Asn
    210                 215


<210> 28
<211> 224
<212> PRT
<213> human Metapneumo virus

<400> 28
Met Glu Val Arg Val Glu Asn Ile Arg Ala Ile Asp Met Phe Lys Ala
1               5                   10                  15
Lys Ile Lys Asn Arg Ile Arg Ser Ser Arg Cys Tyr Arg Asn Ala Thr
            20                  25                  30
Leu Ile Leu Ile Gly Leu Thr Ala Leu Ser Met Ala Leu Asn Ile Phe
        35                  40                  45
Leu Ile Ile Asp His Ala Thr Leu Arg Asn Met Ile Lys Thr Glu Asn
    50                  55                  60
Cys Ala Asn Met Pro Ser Ala Glu Pro Ser Lys Lys Thr Pro Met Thr
65                  70                  75                  80
Ser Thr Ala Gly Pro Asn Thr Lys Pro Asn Pro Gln Gln Ala Thr Gln
                85                  90                  95
Trp Thr Thr Glu Asn Ser Thr Ser Pro Val Ala Thr Pro Glu Gly His
            100                 105                 110
Pro Tyr Thr Gly Thr Thr Gln Thr Ser Asp Thr Thr Ala Pro Gln Gln
        115                 120                 125
Thr Thr Asp Lys His Thr Ala Pro Leu Lys Ser Thr Asn Glu Gln Ile
    130                 135                 140
Thr Gln Thr Thr Thr Glu Lys Lys Thr Ile Arg Ala Thr Thr Gln Lys
145                 150                 155                 160
Arg Glu Lys Gly Lys Glu Asn Thr Asn Gln Thr Thr Ser Thr Ala Ala
                165                 170                 175
Thr Gln Thr Thr Asn Thr Thr Asn Gln Ile Arg Asn Ala Ser Glu Thr
            180                 185                 190
Ile Thr Thr Ser Asp Arg Pro Arg Thr Asp Thr Thr Thr Gln Ser Ser
        195                 200                 205
Glu Gln Thr Thr Arg Ala Thr Asp Pro Ser Ser Pro Pro His His Ala

210                 215                 220

<210> 29
<211> 236
<212> PRT
<213> human Metapneumo virus

<400> 29
Met Glu Val Arg Val Glu Asn Ile Arg Ala Ile Asp Met Phe Lys Ala
1               5                   10                  15
Lys Met Lys Asn Arg Ile Arg Ser Ser Lys Cys Tyr Arg Asn Ala Thr
            20                  25                  30
Leu Ile Leu Ile Gly Leu Thr Ala Leu Ser Met Ala Leu Asn Ile Phe
        35                  40                  45
Leu Ile Ile Asp Tyr Ala Met Leu Lys Asn Met Thr Lys Val Glu His
    50                  55                  60
Cys Val Asn Met Pro Pro Val Glu Pro Ser Lys Lys Thr Pro Met Thr
65                  70                  75                  80
Ser Ala Val Asp Leu Asn Thr Lys Pro Asn Pro Gln Gln Ala Thr Gln
                85                  90                  95
Leu Ala Ala Glu Asp Ser Thr Ser Leu Ala Ala Thr Ser Glu Asp His
            100                 105                 110
Leu His Thr Gly Thr Thr Pro Thr Pro Asp Ala Thr Val Ser Gln Gln
        115                 120                 125
Thr Thr Asp Glu Tyr Thr Thr Leu Leu Arg Ser Thr Asn Arg Gln Thr
    130                 135                 140
Thr Gln Thr Thr Thr Glu Lys Lys Pro Thr Gly Ala Thr Thr Lys Lys
145                 150                 155                 160
Glu Thr Thr Thr Arg Thr Thr Ser Thr Ala Ala Thr Gln Thr Leu Asn
                165                 170                 175
Thr Thr Asn Gln Thr Ser Tyr Val Arg Glu Ala Thr Thr Thr Ser Ala
            180                 185                 190
Arg Ser Arg Asn Ser Ala Thr Thr Gln Ser Ser Asp Gln Thr Thr Gln
        195                 200                 205
Ala Ala Asp Pro Ser Ser Gln Pro His His Thr Gln Lys Ser Thr Thr
    210                 215                 220
Thr Thr Tyr Asn Thr Asp Thr Ser Ser Pro Ser Ser
225                 230                 235

<210> 30
<211> 708
<212> DNA
<213> human Metapneumo virus

<400> 30
gaggtgaaag tggagaacat tcgaacaata gatatgctca aagcaagagt aaaaaatcgt 60
gtggcacgca gcaaatgctt taaaaatgcc tctttggtcc tcataggaat aactacattg 120
agtattgccc tcaatatcta tctgatcata aactataaaa tgcaaaaaaa cacatctgaa 180
tcagaacatc acaccagctc atcacccatg gaatccagca gagaaactcc aacggtcccc 240
acagacaact cagacaccaa ctcaagccca cagcatccaa ctcaacagtc cacagaaggc 300
tccacactct actttgcagc ctcagcaagc tcaccagaga cagaaccaac atcaacacca 360
gatacaacaa accgcccgcc cttcgtcgac acacacacaa caccaccaag cgcaagcaga 420
acaaagacaa gtccggcagt ccacacaaaa aacaacccaa ggacaagctc tagaacacat 480
tctccaccac gggcaacgac aaggacggca cgcagaacca ccactctccg cacaagcagc 540
acaagaaaga gaccgtccac agcatcagtc caacctgaca tcagcgcaac aacccacaaa 600
aacgaagaag caagtccagc gagcccacaa acatctgcaa gcacaacaag aatacaaagg 660
aaaagcgtgg aggccaacac atcaacaaca tacaaccaaa ctagttaa             708

<210> 31
<211> 660

<212> DNA
<213> human Metapneumo virus

<400> 31
atggaggtga aagtagagaa cattcgagca atagacatgc tcaaagcaag agtgaaaaat 60
cgtgtggcac gtagcaaatg ctttaaaaat gcttctttaa tcctcatagg aataactaca 120
ctgagtatag ctctcaatat ctatctgatc ataaactaca caatacaaaa aaccacatcc 180
gaatcagaac accacaccag ctcaccaccc acagaaccca acaaggaagc ttcaacaatc 240
tccacagaca acccagacat caatccaagc tcacagcatc caactcaaca gtccacagaa 300
aaccccacac tcaaccccgc agcatcagcg agcccatcag aaacagaacc agcatcaaca 360
ccagacacaa caaaccgcct gtcctccgta gacaggtcca cagcacaacc aagtgaaagc 420
agaacaaaga caaaaccgac agtccacaca atcaacaacc caaacacagc ttccagtaca 480
caatccccac cacggacaac aacgaaggca atccgcagag ccaccacttt ccgcatgagc 540
agcacaggaa aaagaccaac cacaacatta gtccagtccg acagcagcac cacaacccaa 600
aatcatgaag aaacaggttc agcgaaccca caggcgtctg caagcacaat gcaaaactag 660

<210> 32
<211> 675
<212> DNA
<213> human Metapneumo virus

<400> 32
atggaagtaa gagtggagaa cattcgagcg atagacatgt tcaaagcaaa gataaaaaac 60
cgtataagaa gcagcaggtg ctatagaaat gctacactga tccttattgg actaacagcg 120
ttaagcatgg cacttaatat tttcctgatc atcgatcatg caacattaag aaacatgatc 180
aaaacagaaa actgtgctaa catgccgtcg gcagaaccaa gcaaaaagac cccaatgacc 240
tccacagcag gcccaaacac caaacccaat ccacagcaag caacacagtg gaccacagag 300
aactcaacat ccccagtagc aaccccagag ggccatccat acacagggac aactcaaaca 360
tcagacacaa cagctcccca gcaaaccaca gacaaacaca cagcaccgct aaaatcaacc 420
aatgaacaga tcacccagac aaccacagag aaaaagacaa tcagagcaac aacccaaaaa 480
agggaaaaag gaaaagaaaa cacaaaccaa accacaagca cagctgcaac ccaaacaacc 540
aacaccacca accaaatcag aaatgcaagt gagacaatca caacatccga cagacccaga 600
actgacacca caacccaaag cagcgaacag acaacccggg caacagaccc aagctcccca 660
ccacaccatg catag 675

<210> 33
<211> 711
<212> DNA
<213> human Metapneumo virus

<400> 33
atggaagtaa gagtggagaa cattcgggca atagacatgt tcaaagcaaa aatgaaaaac 60
cgtataagaa gtagcaagtg ctatagaaat gctacactga tccttattgg attaacagca 120
ttaagtatgg cacttaatat ttttttaatc attgattatg caatgttaaa aaacatgacc 180
aaagtggaac actgtgttaa tatgccgccg gtagaaccaa gcaagaagac cccaatgacc 240
tctgcagtag acttaaacac caaacccaat ccacagcagg caacacagtt ggccgcagag 300
gattcaacat ctctagcagc aacctcagag gaccatctac acacagggac aactccaaca 360
ccagatgcaa cagtctctca gcaaaccaca gacgagtaca caacattgct gagatcaacc 420
aacagacaga ccacccaaac aaccacagag aaaaagccaa ccggagcaac aaccaaaaaa 480
gaaaccacaa ctcgaactac aagcacagct gcaacccaaa cactcaacac taccaaccaa 540
actagctatg tgagagaggc aaccacaaca tccgccagat ccagaaacag tgccacaact 600
caaagcagcg accaaacaac ccaggcagca gacccaagct cccaaccaca ccatacacag 660
aaaagcacaa caacaacata caacacagac acatcctctc caagtagtta a 711

<210> 34
<211> 2005
<212> PRT
<213> human Metapneumo virus

<400> 34
Met Asp Pro Leu Asn Glu Ser Thr Val Asn Val Tyr Leu Pro Asp Ser

1               5                   10                  15
Tyr Leu Lys Gly Val Ile Ser Phe Ser Glu Thr Asn Ala Ile Gly Ser
                20                  25                  30
Cys Leu Leu Lys Arg Pro Tyr Leu Lys Asn Asp Asn Thr Ala Lys Val
            35                  40                  45
Ala Ile Glu Asn Pro Val Ile Glu His Val Arg Leu Lys Asn Ala Val
        50                  55                  60
Asn Ser Lys Met Lys Ile Ser Asp Tyr Lys Ile Val Glu Pro Val Asn
65                  70                  75                  80
Met Gln His Glu Ile Met Lys Asn Val His Ser Cys Glu Leu Thr Leu
                85                  90                  95
Leu Lys Gln Phe Leu Thr Arg Ser Lys Asn Ile Ser Thr Leu Lys Leu
            100                 105                 110
Asn Met Ile Cys Asp Trp Leu Gln Leu Lys Ser Thr Ser Asp Asp Thr
        115                 120                 125
Ser Ile Leu Ser Phe Ile Asp Val Glu Phe Ile Pro Ser Trp Val Ser
    130                 135                 140
Asn Trp Phe Ser Asn Trp Tyr Asn Leu Asn Lys Leu Ile Leu Glu Phe
145                 150                 155                 160
Arg Lys Glu Glu Val Ile Arg Thr Gly Ser Ile Leu Cys Arg Ser Leu
                165                 170                 175
Gly Lys Leu Val Phe Val Val Ser Ser Tyr Gly Cys Ile Val Lys Ser
            180                 185                 190
Asn Lys Ser Lys Arg Val Ser Phe Phe Thr Tyr Asn Gln Leu Leu Thr
        195                 200                 205
Trp Lys Asp Val Met Leu Ser Arg Phe Asn Ala Asn Phe Cys Ile Trp
    210                 215                 220
Val Ser Asn Ser Leu Asn Glu Asn Gln Glu Gly Leu Gly Leu Arg Ser
225                 230                 235                 240
Asn Leu Gln Gly Ile Leu Thr Asn Lys Leu Tyr Glu Thr Val Asp Tyr
                245                 250                 255
Met Leu Ser Leu Cys Cys Asn Glu Gly Phe Ser Leu Val Lys Glu Phe
            260                 265                 270
Glu Gly Phe Ile Met Ser Glu Ile Leu Arg Ile Thr Glu His Ala Gln
        275                 280                 285
Phe Ser Thr Arg Phe Arg Asn Thr Leu Leu Asn Gly Leu Thr Asp Gln
    290                 295                 300
Leu Thr Lys Leu Lys Asn Lys Asn Arg Leu Arg Val His Gly Thr Val
305                 310                 315                 320
Leu Glu Asn Asn Asp Tyr Pro Met Tyr Glu Val Val Leu Lys Leu Leu
                325                 330                 335
Gly Asp Thr Leu Arg Cys Ile Lys Leu Leu Ile Asn Lys Asn Leu Glu
            340                 345                 350
Asn Ala Ala Glu Leu Tyr Tyr Ile Phe Arg Ile Phe Gly His Pro Met
        355                 360                 365
Val Asp Glu Arg Asp Ala Met Asp Ala Val Lys Leu Asn Asn Glu Ile
    370                 375                 380
Thr Lys Ile Leu Arg Trp Glu Ser Leu Thr Glu Leu Arg Gly Ala Phe
385                 390                 395                 400
Ile Leu Arg Ile Ile Lys Gly Phe Val Asp Asn Asn Lys Arg Trp Pro
                405                 410                 415
Lys Ile Lys Asn Leu Lys Val Leu Ser Lys Arg Trp Thr Met Tyr Phe
            420                 425                 430
Lys Ala Lys Ser Tyr Pro Ser Gln Leu Glu Leu Ser Glu Gln Asp Phe
        435                 440                 445
Leu Glu Leu Ala Ala Ile Gln Phe Glu Gln Glu Phe Ser Val Pro Glu
    450                 455                 460
Lys Thr Asn Leu Glu Met Val Leu Asn Asp Lys Ala Ile Ser Pro Pro
465                 470                 475                 480
Lys Arg Leu Ile Trp Ser Val Tyr Pro Lys Asn Tyr Leu Pro Glu Lys
                485                 490                 495
Ile Lys Asn Arg Tyr Leu Glu Glu Thr Phe Asn Ala Ser Asp Ser Leu

            500                 505                 510
Lys Thr Arg Arg Val Leu Glu Tyr Tyr Leu Lys Asp Asn Lys Phe Asp
        515                 520                 525
Gln Lys Glu Leu Lys Ser Tyr Val Val Lys Gln Glu Tyr Leu Asn Asp
    530                 535                 540
Lys Asp His Ile Val Ser Leu Thr Gly Lys Glu Arg Glu Leu Ser Val
545                 550                 555                 560
Gly Arg Met Phe Ala Met Gln Pro Gly Lys Gln Arg Gln Ile Gln Ile
                565                 570                 575
Leu Ala Glu Lys Leu Leu Ala Asp Asn Ile Val Pro Phe Phe Pro Glu
            580                 585                 590
Thr Leu Thr Lys Tyr Gly Asp Leu Asp Leu Gln Arg Ile Met Glu Ile
        595                 600                 605
Lys Ser Glu Leu Ser Ser Ile Lys Thr Arg Arg Asn Asp Ser Tyr Asn
    610                 615                 620
Asn Tyr Ile Ala Arg Ala Ser Ile Val Thr Asp Leu Ser Lys Phe Asn
625                 630                 635                 640
Gln Ala Phe Arg Tyr Glu Thr Thr Ala Ile Cys Ala Asp Val Ala Asp
                645                 650                 655
Glu Leu His Gly Thr Gln Ser Leu Phe Cys Trp Leu His Leu Ile Val
            660                 665                 670
Pro Met Thr Thr Met Ile Cys Ala Tyr Arg His Ala Pro Pro Glu Thr
        675                 680                 685
Lys Gly Glu Tyr Asp Ile Asp Lys Ile Glu Glu Gln Ser Gly Leu Tyr
    690                 695                 700
Arg Tyr His Met Gly Gly Ile Glu Gly Trp Cys Gln Lys Leu Trp Thr
705                 710                 715                 720
Met Glu Ala Ile Ser Leu Leu Asp Val Val Ser Val Lys Thr Arg Cys
                725                 730                 735
Gln Met Thr Ser Leu Leu Asn Gly Asp Asn Gln Ser Ile Asp Val Ser
            740                 745                 750
Lys Pro Val Lys Leu Ser Glu Gly Leu Asp Glu Val Lys Ala Asp Tyr
        755                 760                 765
Ser Leu Ala Val Lys Met Leu Lys Glu Ile Arg Asp Ala Tyr Arg Asn
    770                 775                 780
Ile Gly His Lys Leu Lys Glu Gly Glu Thr Tyr Ile Ser Arg Asp Leu
785                 790                 795                 800
Gln Phe Ile Ser Lys Val Ile Gln Ser Glu Gly Val Met His Pro Thr
                805                 810                 815
Pro Ile Lys Lys Ile Leu Arg Val Gly Pro Trp Ile Asn Thr Ile Leu
            820                 825                 830
Asp Asp Ile Lys Thr Ser Ala Glu Ser Ile Gly Ser Leu Cys Gln Glu
        835                 840                 845
Leu Glu Phe Arg Gly Glu Ser Ile Ile Val Ser Leu Ile Leu Arg Asn
    850                 855                 860
Phe Trp Leu Tyr Asn Leu Tyr Met His Glu Ser Lys Gln His Pro Leu
865                 870                 875                 880
Ala Gly Lys Gln Leu Phe Lys Gln Leu Asn Lys Thr Leu Thr Ser Val
                885                 890                 895
Gln Arg Phe Phe Glu Ile Lys Lys Glu Asn Glu Val Val Asp Leu Trp
            900                 905                 910
Met Asn Ile Pro Met Gln Phe Gly Gly Gly Asp Pro Val Val Phe Tyr
        915                 920                 925
Arg Ser Phe Tyr Arg Arg Thr Pro Asp Phe Leu Thr Glu Ala Ile Ser
    930                 935                 940
His Val Asp Ile Leu Leu Arg Ile Ser Ala Asn Ile Arg Asn Glu Ala
945                 950                 955                 960
Lys Ile Ser Phe Phe Lys Ala Leu Leu Ser Ile Glu Lys Asn Glu Arg
                965                 970                 975
Ala Thr Leu Thr Thr Leu Met Arg Asp Pro Gln Ala Val Gly Ser Glu
            980                 985                 990
Arg Gln Ala Lys Val Thr Ser Asp Ile Asn Arg Thr Ala Val Thr Ser

995                 1000                1005
Ile Leu Ser Leu Ser Pro Asn Gln Leu Phe Ser Asp Ser Ala Ile His
    1010                1015                1020
Tyr Ser Arg Asn Glu Glu Glu Val Gly Ile Ile Ala Asp Asn Ile Thr
1025                1030                1035                1040
Pro Val Tyr Pro His Gly Leu Arg Val Leu Tyr Glu Ser Leu Pro Phe
                1045                1050                1055
His Lys Ala Glu Lys Val Val Asn Met Ile Ser Gly Thr Lys Ser Ile
            1060                1065                1070
Thr Asn Leu Leu Gln Arg Thr Ser Ala Ile Asn Gly Glu Asp Ile Asp
        1075                1080                1085
Arg Ala Val Ser Met Met Leu Glu Asn Leu Gly Leu Leu Ser Arg Ile
    1090                1095                1100
Leu Ser Val Val Val Asp Ser Ile Glu Ile Pro Thr Lys Ser Asn Gly
1105                1110                1115                1120
Arg Leu Ile Cys Cys Gln Ile Ser Arg Thr Leu Arg Glu Thr Ser Trp
                1125                1130                1135
Asn Asn Met Glu Ile Val Gly Val Thr Ser Pro Ser Ile Thr Thr Cys
            1140                1145                1150
Met Asp Val Ile Tyr Ala Thr Ser Ser His Leu Lys Gly Ile Ile Ile
        1155                1160                1165
Glu Lys Phe Ser Thr Asp Arg Thr Thr Arg Gly Gln Arg Gly Pro Lys
    1170                1175                1180
Ser Pro Trp Val Gly Ser Ser Thr Gln Glu Lys Lys Leu Val Pro Val
1185                1190                1195                1200
Tyr Asn Arg Gln Ile Leu Ser Lys Gln Gln Arg Glu Gln Leu Glu Ala
                1205                1210                1215
Ile Gly Lys Met Arg Trp Val Tyr Lys Gly Thr Pro Gly Leu Arg Arg
            1220                1225                1230
Leu Leu Asn Lys Ile Cys Leu Gly Ser Leu Gly Ile Ser Tyr Lys Cys
        1235                1240                1245
Val Lys Pro Leu Leu Pro Arg Phe Met Ser Val Asn Phe Leu His Arg
    1250                1255                1260
Leu Ser Val Ser Ser Arg Pro Met Glu Phe Pro Ala Ser Val Pro Ala
1265                1270                1275                1280
Tyr Arg Thr Thr Asn Tyr His Phe Asp Thr Ser Pro Ile Asn Gln Ala
                1285                1290                1295
Leu Ser Glu Arg Phe Gly Asn Glu Asp Ile Asn Leu Val Phe Gln Asn
            1300                1305                1310
Ala Ile Ser Cys Gly Ile Ser Ile Met Ser Val Val Glu Gln Leu Thr
        1315                1320                1325
Gly Arg Ser Pro Lys Gln Leu Val Leu Ile Pro Gln Leu Glu Glu Ile
    1330                1335                1340
Asp Ile Met Pro Pro Pro Val Phe Gln Gly Lys Phe Asn Tyr Lys Leu
1345                1350                1355                1360
Val Asp Lys Ile Thr Ser Asp Gln His Ile Phe Ser Pro Asp Lys Ile
                1365                1370                1375
Asp Met Leu Thr Leu Gly Lys Met Leu Met Pro Thr Ile Lys Gly Gln
            1380                1385                1390
Lys Thr Asp Gln Phe Leu Asn Lys Arg Glu Asn Tyr Phe His Gly Asn
        1395                1400                1405
Asn Leu Ile Glu Ser Leu Ser Ala Ala Leu Ala Cys His Trp Cys Gly
    1410                1415                1420
Ile Leu Thr Glu Gln Cys Ile Glu Asn Asn Ile Phe Lys Lys Asp Trp
1425                1430                1435                1440
Gly Asp Gly Phe Ile Ser Asp His Ala Phe Met Asp Phe Lys Ile Phe
                1445                1450                1455
Leu Cys Val Phe Lys Thr Lys Leu Leu Cys Ser Trp Gly Ser Gln Gly
            1460                1465                1470
Lys Asn Ile Lys Asp Glu Asp Ile Val Asp Glu Ser Ile Asp Lys Leu
        1475                1480                1485
Leu Arg Ile Asp Asn Thr Phe Trp Arg Met Phe Ser Lys Val Met Phe

1490                1495                1500
Glu Ser Lys Val Lys Lys Arg Ile Met Leu Tyr Asp Val Lys Phe Leu
1505                1510                1515                1520
Ser Leu Val Gly Tyr Ile Gly Phe Lys Asn Trp Phe Ile Glu Gln Leu
                1525                1530                1535
Arg Ser Ala Glu Leu His Glu Val Pro Trp Ile Val Asn Ala Glu Gly
            1540                1545                1550
Asp Leu Val Glu Ile Lys Ser Ile Lys Ile Tyr Leu Gln Leu Ile Glu
        1555                1560                1565
Gln Ser Leu Phe Leu Arg Ile Thr Val Leu Asn Tyr Thr Asp Met Ala
    1570                1575                1580
His Ala Leu Thr Arg Leu Ile Arg Lys Lys Leu Met Cys Asp Asn Ala
1585                1590                1595                1600
Leu Leu Thr Pro Ile Pro Ser Pro Met Val Asn Leu Thr Gln Val Ile
                1605                1610                1615
Asp Pro Thr Glu Gln Leu Ala Tyr Phe Pro Lys Ile Thr Phe Glu Arg
            1620                1625                1630
Leu Lys Asn Tyr Asp Thr Ser Ser Asn Tyr Ala Lys Gly Lys Leu Thr
        1635                1640                1645
Arg Asn Tyr Met Ile Leu Leu Pro Trp Gln His Val Asn Arg Tyr Asn
    1650                1655                1660
Phe Val Phe Ser Ser Thr Gly Cys Lys Val Ser Leu Lys Thr Cys Ile
1665                1670                1675                1680
Gly Lys Leu Met Lys Asp Leu Asn Pro Lys Val Leu Tyr Phe Ile Gly
                1685                1690                1695
Glu Gly Ala Gly Asn Trp Met Ala Arg Thr Ala Cys Glu Tyr Pro Asp
            1700                1705                1710
Ile Lys Phe Val Tyr Arg Ser Leu Lys Asp Asp Leu Asp His His Tyr
        1715                1720                1725
Pro Leu Glu Tyr Gln Arg Val Ile Gly Glu Leu Ser Arg Ile Ile Asp
    1730                1735                1740
Ser Gly Glu Gly Leu Ser Met Glu Thr Thr Asp Ala Thr Gln Lys Thr
1745                1750                1755                1760
His Trp Asp Leu Ile His Arg Val Ser Lys Asp Ala Leu Leu Ile Thr
                1765                1770                1775
Leu Cys Asp Ala Glu Phe Lys Asp Arg Asp Asp Phe Phe Lys Met Val
            1780                1785                1790
Ile Leu Trp Arg Lys His Val Leu Ser Cys Arg Ile Cys Thr Thr Tyr
        1795                1800                1805
Gly Thr Asp Leu Tyr Leu Phe Ala Lys Tyr His Ala Lys Asp Cys Asn
    1810                1815                1820
Val Lys Leu Pro Phe Phe Val Arg Ser Val Ala Thr Phe Ile Met Gln
1825                1830                1835                1840
Gly Ser Lys Leu Ser Gly Ser Glu Cys Tyr Ile Leu Leu Thr Leu Gly
                1845                1850                1855
His His Asn Asn Leu Pro Cys His Gly Glu Ile Gln Asn Ser Lys Met
            1860                1865                1870
Lys Ile Ala Val Cys Asn Asp Phe Tyr Ala Ala Lys Lys Leu Asp Asn
        1875                1880                1885
Lys Ser Ile Glu Ala Asn Cys Lys Ser Leu Leu Ser Gly Leu Arg Ile
    1890                1895                1900
Pro Ile Asn Lys Lys Glu Leu Asn Arg Gln Arg Arg Leu Leu Thr Leu
1905                1910                1915                1920
Gln Ser Asn His Ser Ser Val Ala Thr Val Gly Gly Ser Lys Val Ile
                1925                1930                1935
Glu Ser Lys Trp Leu Thr Asn Lys Ala Asn Thr Ile Ile Asp Trp Leu
            1940                1945                1950
Glu His Ile Leu Asn Ser Pro Lys Gly Glu Leu Asn Tyr Asp Phe Phe
        1955                1960                1965
Glu Ala Leu Glu Asn Thr Tyr Pro Asn Met Ile Lys Leu Ile Asp Asn
    1970                1975                1980
Leu Gly Asn Ala Glu Ile Lys Lys Leu Ile Lys Val Thr Gly Tyr Met

1985                1990                1995                2000
Leu Val Ser Lys Lys
                2005

<210> 35
<211> 2005
<212> PRT
<213> human Metapneumo virus

<400> 35
Met Asp Pro Leu Asn Glu Ser Thr Val Asn Val Tyr Leu Pro Asp Ser
1               5                   10                  15
Tyr Leu Lys Gly Val Ile Ser Phe Ser Glu Thr Asn Ala Ile Gly Ser
            20                  25                  30
Cys Leu Leu Lys Arg Pro Tyr Leu Lys Asn Asp Asn Thr Ala Lys Val
        35                  40                  45
Ala Ile Glu Asn Pro Val Ile Glu His Val Arg Leu Lys Asn Ala Val
    50                  55                  60
Asn Ser Lys Met Lys Ile Ser Asp Tyr Lys Val Val Glu Pro Val Asn
65                  70                  75                  80
Met Gln His Glu Ile Met Lys Asn Val His Ser Cys Glu Leu Thr Leu
                85                  90                  95
Leu Lys Gln Phe Leu Thr Arg Ser Lys Asn Ile Ser Thr Leu Lys Leu
            100                 105                 110
Asn Met Ile Cys Asp Trp Leu Gln Leu Lys Ser Thr Ser Asp Asp Thr
        115                 120                 125
Ser Ile Leu Ser Phe Ile Asp Val Glu Phe Ile Pro Ser Trp Val Ser
    130                 135                 140
Asn Trp Phe Ser Asn Trp Tyr Asn Leu Asn Lys Leu Ile Leu Glu Phe
145                 150                 155                 160
Arg Arg Glu Glu Val Ile Arg Thr Gly Ser Ile Leu Cys Arg Ser Leu
                165                 170                 175
Gly Lys Leu Val Phe Ile Val Ser Ser Tyr Gly Cys Ile Val Lys Ser
            180                 185                 190
Asn Lys Ser Lys Arg Val Ser Phe Phe Thr Tyr Asn Gln Leu Leu Thr
        195                 200                 205
Trp Lys Asp Val Met Leu Ser Arg Phe Asn Ala Asn Phe Cys Ile Trp
    210                 215                 220
Val Ser Asn Ser Leu Asn Glu Asn Gln Glu Gly Leu Gly Leu Arg Ser
225                 230                 235                 240
Asn Leu Gln Gly Met Leu Thr Asn Lys Leu Tyr Glu Thr Val Asp Tyr
                245                 250                 255
Met Leu Ser Leu Cys Cys Asn Glu Gly Phe Ser Leu Val Lys Glu Phe
            260                 265                 270
Glu Gly Phe Ile Met Ser Glu Ile Leu Arg Ile Thr Glu His Ala Gln
        275                 280                 285
Phe Ser Thr Arg Phe Arg Asn Thr Leu Leu Asn Gly Leu Thr Asp Gln
    290                 295                 300
Leu Thr Lys Leu Lys Asn Lys Asn Arg Leu Arg Val His Gly Thr Val
305                 310                 315                 320
Leu Glu Asn Asn Asp Tyr Pro Met Tyr Glu Val Val Leu Lys Leu Leu
                325                 330                 335
Gly Asp Thr Leu Arg Cys Ile Lys Leu Leu Ile Asn Lys Asn Leu Glu
            340                 345                 350
Asn Ala Ala Glu Leu Tyr Tyr Ile Phe Arg Ile Phe Gly His Pro Met
        355                 360                 365
Val Asp Glu Arg Asp Ala Met Asp Ala Val Lys Leu Asn Asn Glu Ile
    370                 375                 380
Thr Lys Ile Leu Arg Leu Glu Ser Leu Thr Glu Leu Arg Gly Ala Phe
385                 390                 395                 400
Ile Leu Arg Ile Ile Lys Gly Phe Val Asp Asn Asn Lys Arg Trp Pro

                405                 410                 415
Lys Ile Lys Asn Leu Ile Val Leu Ser Lys Arg Trp Thr Met Tyr Phe
            420                 425                 430

Lys Ala Lys Asn Tyr Pro Ser Gln Leu Glu Leu Ser Glu Gln Asp Phe
        435                 440                 445
Leu Glu Leu Ala Ala Ile Gln Phe Glu Gln Glu Phe Ser Val Pro Glu
    450                 455                 460
Lys Thr Asn Leu Glu Met Val Leu Asn Asp Lys Ala Ile Ser Pro Pro
465                 470                 475                 480
Lys Arg Leu Ile Trp Ser Val Tyr Pro Lys Asn Tyr Leu Pro Glu Thr
                485                 490                 495
Ile Lys Asn Arg Tyr Leu Glu Glu Thr Phe Asn Ala Ser Asp Ser Leu
            500                 505                 510
Lys Thr Arg Arg Val Leu Glu Tyr Tyr Leu Lys Asp Asn Lys Phe Asp
        515                 520                 525
Gln Lys Glu Leu Lys Ser Tyr Val Val Arg Gln Glu Tyr Leu Asn Asp
    530                 535                 540
Lys Glu His Ile Val Ser Leu Thr Gly Lys Glu Arg Glu Leu Ser Val
545                 550                 555                 560
Gly Arg Met Phe Ala Met Gln Pro Gly Lys Gln Arg Gln Ile Gln Ile
                565                 570                 575
Leu Ala Glu Lys Leu Leu Ala Asp Asn Ile Val Pro Phe Phe Pro Glu
            580                 585                 590
Thr Leu Thr Lys Tyr Gly Asp Leu Asp Leu Gln Arg Ile Met Glu Ile
        595                 600                 605
Lys Ser Glu Leu Ser Ser Ile Lys Thr Arg Arg Asn Asp Ser Tyr Asn
    610                 615                 620
Asn Tyr Ile Ala Arg Ala Ser Ile Val Thr Asp Leu Ser Lys Phe Asn
625                 630                 635                 640
Gln Ala Phe Arg Tyr Glu Thr Thr Ala Ile Cys Ala Asp Val Ala Asp
                645                 650                 655
Glu Leu His Gly Thr Gln Ser Leu Phe Cys Trp Leu His Leu Ile Val
            660                 665                 670
Pro Met Thr Thr Met Ile Cys Ala Tyr Arg His Ala Pro Pro Glu Thr
        675                 680                 685
Lys Gly Glu Tyr Asp Ile Asp Lys Ile Glu Glu Gln Ser Gly Leu Tyr
    690                 695                 700
Arg Tyr His Met Gly Gly Ile Glu Gly Trp Cys Gln Lys Leu Trp Thr
705                 710                 715                 720
Met Glu Ala Ile Ser Leu Leu Asp Val Val Ser Val Lys Thr Arg Cys
                725                 730                 735
Gln Met Thr Ser Leu Leu Asn Gly Asp Asn Gln Ser Ile Asp Val Ser
            740                 745                 750
Lys Pro Val Lys Leu Ser Glu Gly Leu Asp Glu Val Lys Ala Asp Tyr
        755                 760                 765
Arg Leu Ala Ile Lys Met Leu Lys Glu Ile Arg Asp Ala Tyr Arg Asn
    770                 775                 780
Ile Gly His Lys Leu Lys Glu Gly Glu Thr Tyr Ile Ser Arg Asp Leu
785                 790                 795                 800
Gln Phe Ile Ser Lys Val Ile Gln Ser Glu Gly Val Met His Pro Thr
                805                 810                 815
Pro Ile Lys Lys Val Leu Arg Val Gly Pro Trp Ile Asn Thr Ile Leu
            820                 825                 830
Asp Asp Ile Lys Thr Ser Ala Glu Ser Ile Gly Ser Leu Cys Gln Glu
        835                 840                 845
Leu Glu Phe Arg Gly Glu Ser Ile Ile Val Ser Leu Ile Leu Arg Asn
    850                 855                 860
Phe Trp Leu Tyr Asn Leu Tyr Met His Glu Ser Lys Gln His Pro Leu
865                 870                 875                 880
Ala Gly Lys Gln Leu Phe Lys Gln Leu Asn Lys Thr Leu Thr Ser Val
                885                 890                 895

Gln Arg Phe Phe Glu Ile Lys Lys Glu Asn Glu Val Val Asp Leu Trp
            900                 905                 910
Met Asn Ile Pro Met Gln Phe Gly Gly Gly Asp Pro Val Val Phe Tyr
        915                 920                 925
Arg Ser Phe Tyr Arg Arg Thr Pro Asp Phe Leu Thr Glu Ala Ile Ser
    930                 935                 940
His Val Asp Ile Leu Leu Lys Ile Ser Ala Asn Ile Lys Asn Glu Thr
945                 950                 955                 960
Lys Val Ser Phe Phe Lys Ala Leu Leu Ser Ile Glu Lys Asn Glu Arg
                965                 970                 975
Ala Thr Leu Thr Thr Leu Met Arg Asp Pro Gln Ala Val Gly Ser Glu
            980                 985                 990
Arg Gln Ala Lys Val Thr Ser Asp Ile Asn Arg Thr Ala Val Thr Ser
        995                 1000                1005
Ile Leu Ser Leu Ser Pro Asn Gln Leu Phe Ser Asp Ser Ala Ile His
    1010                1015                1020
Tyr Ser Arg Asn Glu Glu Glu Val Gly Ile Ile Ala Glu Asn Ile Thr
1025                1030                1035                1040
Pro Val Tyr Pro His Gly Leu Arg Val Leu Tyr Glu Ser Leu Pro Phe
                1045                1050                1055
His Lys Ala Glu Lys Val Val Asn Met Ile Ser Gly Thr Lys Ser Ile
            1060                1065                1070
Thr Asn Leu Leu Gln Arg Thr Ser Ala Ile Asn Gly Glu Asp Ile Asp
        1075                1080                1085
Arg Ala Val Ser Met Met Leu Glu Asn Leu Gly Leu Leu Ser Arg Ile
    1090                1095                1100
Leu Ser Val Val Val Asp Ser Ile Glu Ile Pro Ile Lys Ser Asn Gly
1105                1110                1115                1120
Arg Leu Ile Cys Cys Gln Ile Ser Arg Thr Leu Arg Glu Thr Ser Trp
                1125                1130                1135
Asn Asn Met Glu Ile Val Gly Val Thr Ser Pro Ser Ile Thr Thr Cys
            1140                1145                1150
Met Asp Val Ile Tyr Ala Thr Ser Ser His Leu Lys Gly Ile Ile Ile
        1155                1160                1165
Glu Lys Phe Ser Thr Asp Arg Thr Thr Arg Gly Gln Arg Gly Pro Lys
    1170                1175                1180
Ser Pro Trp Val Gly Ser Ser Thr Gln Glu Lys Lys Leu Val Pro Val
1185                1190                1195                1200
Tyr Asn Arg Gln Ile Leu Ser Lys Gln Gln Arg Glu Gln Leu Glu Ala
                1205                1210                1215
Ile Gly Lys Met Arg Trp Val Tyr Lys Gly Thr Pro Gly Leu Arg Arg
            1220                1225                1230
Leu Leu Asn Lys Ile Cys Leu Gly Ser Leu Gly Ile Ser Tyr Lys Cys
        1235                1240                1245
Val Lys Pro Leu Leu Pro Arg Phe Met Ser Val Asn Phe Leu His Arg
    1250                1255                1260
Leu Ser Val Ser Ser Arg Pro Met Glu Phe Pro Ala Ser Val Pro Ala
1265                1270                1275                1280
Tyr Arg Thr Thr Asn Tyr His Phe Asp Thr Ser Pro Ile Asn Gln Ala
                1285                1290                1295
Leu Ser Glu Arg Phe Gly Asn Glu Asp Ile Asn Leu Val Phe Gln Asn
            1300                1305                1310
Ala Ile Ser Cys Gly Ile Ser Ile Met Ser Val Val Glu Gln Leu Thr
        1315                1320                1325
Gly Arg Ser Pro Lys Gln Leu Val Leu Ile Pro Gln Leu Glu Glu Ile
    1330                1335                1340
Asp Ile Met Pro Pro Pro Val Phe Gln Gly Lys Phe Asn Tyr Lys Leu
1345                1350                1355                1360
Val Asp Lys Ile Thr Ser Asp Gln His Ile Phe Ser Pro Asp Lys Ile
                1365                1370                1375
Asp Met Leu Thr Leu Gly Lys Met Leu Met Pro Thr Ile Lys Gly Gln
            1380                1385                1390

Lys Thr Asp Gln Phe Leu Asn Lys Arg Glu Asn Tyr Phe His Gly Asn
        1395                1400                1405
Asn Leu Ile Glu Ser Leu Ser Ala Ala Leu Ala Cys His Trp Cys Gly
    1410                1415                1420
Ile Leu Thr Glu Gln Cys Ile Glu Asn Asn Ile Phe Lys Lys Asp Trp
1425                1430                1435                1440
Gly Asp Gly Phe Ile Ser Asp His Ala Phe Met Asp Phe Lys Ile Phe
                1445                1450                1455
Leu Cys Val Phe Lys Thr Lys Leu Leu Cys Ser Trp Gly Ser Gln Gly
            1460                1465                1470
Lys Asn Ile Lys Asp Glu Asp Ile Val Asp Glu Ser Ile Asp Lys Leu
        1475                1480                1485
Leu Arg Ile Asp Asn Thr Phe Trp Arg Met Phe Ser Lys Val Met Phe
    1490                1495                1500
Glu Pro Lys Val Lys Lys Arg Ile Met Leu Tyr Asp Val Lys Phe Leu
1505                1510                1515                1520
Ser Leu Val Gly Tyr Ile Gly Phe Lys Asn Trp Phe Ile Glu Gln Leu
                1525                1530                1535
Arg Ser Ala Glu Leu His Glu Ile Pro Trp Ile Val Asn Ala Glu Gly
            1540                1545                1550
Asp Leu Val Glu Ile Lys Ser Ile Lys Ile Tyr Leu Gln Leu Ile Glu
        1555                1560                1565
Gln Ser Leu Phe Leu Arg Ile Thr Val Leu Asn Tyr Thr Asp Met Ala
    1570                1575                1580
His Ala Leu Thr Arg Leu Ile Arg Lys Lys Leu Met Cys Asp Asn Ala
1585                1590                1595                1600
Leu Leu Thr Pro Ile Ser Ser Pro Met Val Asn Leu Thr Gln Val Ile
                1605                1610                1615
Asp Pro Thr Thr Gln Leu Asp Tyr Phe Pro Lys Ile Thr Phe Glu Arg
            1620                1625                1630
Leu Lys Asn Tyr Asp Thr Ser Ser Asn Tyr Ala Lys Gly Lys Leu Thr
        1635                1640                1645
Arg Asn Tyr Met Ile Leu Leu Pro Trp Gln His Val Asn Arg Tyr Asn
    1650                1655                1660
Phe Val Phe Ser Ser Thr Gly Cys Lys Val Ser Leu Lys Thr Cys Ile
1665                1670                1675                1680
Gly Lys Leu Met Lys Asp Leu Asn Pro Lys Val Leu Tyr Phe Ile Gly
                1685                1690                1695
Glu Gly Ala Gly Asn Trp Met Ala Arg Thr Ala Cys Glu Tyr Pro Asp
            1700                1705                1710
Ile Lys Phe Val Tyr Arg Ser Leu Lys Asp Asp Leu Asp His His Tyr
        1715                1720                1725
Pro Leu Glu Tyr Gln Arg Val Ile Gly Glu Leu Ser Arg Ile Ile Asp
    1730                1735                1740
Ser Gly Glu Gly Leu Ser Met Glu Thr Thr Asp Ala Thr Gln Lys Thr
1745                1750                1755                1760
His Trp Asp Leu Ile His Arg Val Ser Lys Asp Ala Leu Leu Ile Thr
                1765                1770                1775
Leu Cys Asp Ala Glu Phe Lys Asp Arg Asp Asp Phe Phe Lys Met Val
            1780                1785                1790
Ile Leu Trp Arg Lys His Val Leu Ser Cys Arg Ile Cys Thr Thr Tyr
        1795                1800                1805
Gly Thr Asp Leu Tyr Leu Phe Ala Lys Tyr His Ala Lys Asp Cys Asn
    1810                1815                1820
Val Lys Leu Pro Phe Phe Val Arg Ser Val Ala Thr Phe Ile Met Gln
1825                1830                1835                1840
Gly Ser Lys Leu Ser Gly Ser Glu Cys Tyr Ile Leu Leu Thr Leu Gly
                1845                1850                1855
His His Asn Ser Leu Pro Cys His Gly Glu Ile Gln Asn Ser Lys Met
            1860                1865                1870
Lys Ile Ala Val Cys Asn Asp Phe Tyr Ala Ala Lys Lys Leu Asp Asn
        1875                1880                1885

Lys Ser Ile Glu Ala Asn Cys Lys Ser Leu Leu Ser Gly Leu Arg Ile
    1890                1895                1900
Pro Ile Asn Lys Lys Glu Leu Asp Arg Gln Arg Arg Leu Leu Thr Leu
1905                1910                1915                1920
Gln Ser Asn His Ser Ser Val Ala Thr Val Gly Gly Ser Lys Ile Ile
                1925                1930                1935
Glu Ser Lys Trp Leu Thr Asn Lys Ala Ser Thr Ile Ile Asp Trp Leu
            1940                1945                1950
Glu His Ile Leu Asn Ser Pro Lys Gly Glu Leu Asn Tyr Asp Phe Phe
        1955                1960                1965
Glu Ala Leu Glu Asn Thr Tyr Pro Asn Met Ile Lys Leu Ile Asp Asn
    1970                1975                1980
Leu Gly Asn Ala Glu Ile Lys Lys Leu Ile Lys Val Thr Gly Tyr Met
1985                1990                1995                2000
Leu Val Ser Lys Lys
                2005

<210> 36
<211> 2005
<212> PRT
<213> human Metapneumo virus

<400> 36
Met Asp Pro Phe Cys Glu Ser Thr Val Asn Val Tyr Leu Pro Asp Ser
 1               5                  10                  15
Tyr Leu Lys Gly Val Ile Ser Phe Ser Glu Thr Asn Ala Ile Gly Ser
            20                  25                  30
Cys Leu Leu Lys Arg Pro Tyr Leu Lys Asn Asp Asn Thr Ala Lys Val
        35                  40                  45
Ala Val Glu Asn Pro Val Val Glu His Val Arg Leu Arg Asn Ala Val
    50                  55                  60
Met Thr Lys Met Lys Ile Ser Asp Tyr Lys Val Val Glu Pro Val Asn
65                  70                  75                  80
Met Gln His Glu Ile Met Lys Asn Ile His Ser Cys Glu Leu Thr Leu
                85                  90                  95
Leu Lys Gln Phe Leu Thr Arg Ser Lys Asn Ile Ser Ser Leu Lys Leu
            100                 105                 110
Asn Met Ile Cys Asp Trp Leu Gln Leu Lys Ser Thr Ser Asp Asn Thr
        115                 120                 125
Ser Ile Leu Asn Phe Ile Asp Val Glu Phe Ile Pro Val Trp Val Ser
    130                 135                 140
Asn Trp Phe Ser Asn Trp Tyr Asn Leu Asn Lys Leu Ile Leu Glu Phe
145                 150                 155                 160
Arg Arg Glu Glu Val Ile Arg Thr Gly Ser Ile Leu Cys Arg Ser Leu
                165                 170                 175
Gly Lys Leu Val Phe Ile Val Ser Ser Tyr Gly Cys Val Val Lys Ser
            180                 185                 190
Asn Lys Ser Lys Arg Val Ser Phe Phe Thr Tyr Asn Gln Leu Leu Thr
        195                 200                 205
Trp Lys Asp Val Met Leu Ser Arg Phe Asn Ala Asn Phe Cys Ile Trp
    210                 215                 220
Val Ser Asn Asn Leu Asn Lys Asn Gln Glu Gly Leu Gly Leu Arg Ser
225                 230                 235                 240
Asn Leu Gln Gly Met Leu Thr Asn Lys Leu Tyr Glu Thr Val Asp Tyr
                245                 250                 255
Met Leu Ser Leu Cys Cys Asn Glu Gly Phe Ser Leu Val Lys Glu Phe
            260                 265                 270
Glu Gly Phe Ile Met Ser Glu Ile Leu Lys Ile Thr Glu His Ala Gln
        275                 280                 285
Phe Ser Thr Arg Phe Arg Asn Thr Leu Leu Asn Gly Leu Thr Glu Gln
    290                 295                 300

Leu Ser Val Leu Lys Ala Lys Asn Arg Ser Arg Val Leu Gly Thr Ile
305                 310                 315                 320
Leu Glu Asn Asn Asn Tyr Pro Met Tyr Glu Val Val Leu Lys Leu Leu
                325                 330                 335
Gly Asp Thr Leu Lys Ser Ile Lys Leu Leu Ile Asn Lys Asn Leu Glu
            340                 345                 350
Asn Ala Ala Glu Leu Tyr Tyr Ile Phe Arg Ile Phe Gly His Pro Met
        355                 360                 365
Val Asp Glu Arg Glu Ala Met Asp Ala Val Lys Leu Asn Asn Glu Ile
    370                 375                 380
Thr Lys Ile Leu Lys Leu Glu Ser Leu Thr Glu Leu Arg Gly Ala Phe
385                 390                 395                 400
Ile Leu Arg Ile Ile Lys Gly Phe Val Asp Asn Asn Lys Arg Trp Pro
                405                 410                 415
Lys Ile Lys Asn Leu Lys Val Leu Ser Lys Arg Trp Ala Met Tyr Phe
            420                 425                 430
Lys Ala Lys Ser Tyr Pro Ser Gln Leu Glu Leu Ser Val Gln Asp Phe
        435                 440                 445
Leu Glu Leu Ala Ala Val Gln Phe Glu Gln Glu Phe Ser Val Pro Glu
    450                 455                 460
Lys Thr Asn Leu Glu Met Val Leu Asn Asp Lys Ala Ile Ser Pro Pro
465                 470                 475                 480
Lys Lys Leu Ile Trp Ser Val Tyr Pro Lys Asn Tyr Leu Pro Glu Thr
                485                 490                 495
Ile Lys Asn Gln Tyr Leu Glu Glu Ala Phe Asn Ala Ser Asp Ser Gln
            500                 505                 510
Arg Thr Arg Arg Val Leu Glu Phe Tyr Leu Lys Asp Cys Lys Phe Asp
        515                 520                 525
Gln Lys Glu Leu Lys Arg Tyr Val Ile Lys Gln Glu Tyr Leu Asn Asp
    530                 535                 540
Lys Asp His Ile Val Ser Leu Thr Gly Lys Glu Arg Glu Leu Ser Val
545                 550                 555                 560
Gly Arg Met Phe Ala Met Gln Pro Gly Lys Gln Arg Gln Ile Gln Ile
                565                 570                 575
Leu Ala Glu Lys Leu Leu Ala Asp Asn Ile Val Pro Phe Phe Pro Glu
            580                 585                 590
Thr Leu Thr Lys Tyr Gly Asp Leu Asp Leu Gln Arg Ile Met Glu Ile
        595                 600                 605
Lys Ser Glu Leu Ser Ser Ile Lys Thr Arg Lys Asn Asp Ser Tyr Asn
    610                 615                 620
Asn Tyr Ile Ala Arg Ala Ser Ile Val Thr Asp Leu Ser Lys Phe Asn
625                 630                 635                 640
Gln Ala Phe Arg Tyr Glu Thr Thr Ala Ile Cys Ala Asp Val Ala Asp
                645                 650                 655
Glu Leu His Gly Thr Gln Ser Leu Phe Cys Trp Leu His Leu Ile Val
            660                 665                 670
Pro Met Thr Thr Met Ile Cys Ala Tyr Arg His Ala Pro Pro Glu Thr
        675                 680                 685
Lys Gly Glu Tyr Asp Ile Asp Lys Ile Gln Glu Gln Ser Gly Leu Tyr
    690                 695                 700
Arg Tyr His Met Gly Gly Ile Glu Gly Trp Cys Gln Lys Leu Trp Thr
705                 710                 715                 720
Met Glu Ala Ile Ser Leu Leu Asp Val Val Ser Val Lys Thr Arg Cys
                725                 730                 735
Gln Met Thr Ser Leu Leu Asn Gly Asp Asn Gln Ser Ile Asp Val Ser
            740                 745                 750
Lys Pro Val Lys Leu Ser Glu Gly Ile Asp Glu Val Lys Ala Asp Tyr
        755                 760                 765
Ser Leu Ala Ile Arg Met Leu Lys Glu Ile Arg Asp Ala Tyr Lys Asn
    770                 775                 780
Ile Gly His Lys Leu Lys Glu Gly Glu Thr Tyr Ile Ser Arg Asp Leu
785                 790                 795                 800

Gln Phe Ile Ser Lys Val Ile Gln Ser Glu Gly Val Met His Pro Thr
                805                 810                 815
Pro Ile Lys Lys Ile Leu Arg Val Gly Pro Trp Ile Asn Thr Ile Leu
            820                 825                 830
Asp Asp Ile Lys Thr Ser Ala Glu Ser Ile Gly Ser Leu Cys Gln Glu
        835                 840                 845
Leu Glu Phe Arg Gly Glu Ser Ile Leu Val Ser Leu Ile Leu Arg Asn
    850                 855                 860
Phe Trp Leu Tyr Asn Leu Tyr Met Tyr Glu Ser Lys Gln His Pro Leu
865                 870                 875                 880
Ala Gly Lys Gln Leu Phe Lys Gln Leu Asn Lys Thr Leu Thr Ser Val
                885                 890                 895
Gln Arg Phe Phe Glu Leu Lys Lys Glu Asn Asp Val Val Asp Leu Trp
            900                 905                 910
Met Asn Ile Pro Met Gln Phe Gly Gly Gly Asp Pro Val Val Phe Tyr
        915                 920                 925
Arg Ser Phe Tyr Arg Arg Thr Pro Asp Phe Leu Thr Glu Ala Ile Ser
    930                 935                 940
His Val Asp Leu Leu Leu Lys Val Ser Asn Asn Ile Lys Asp Glu Thr
945                 950                 955                 960
Lys Ile Arg Phe Phe Lys Ala Leu Leu Ser Ile Glu Lys Asn Glu Arg
                965                 970                 975
Ala Thr Leu Thr Thr Leu Met Arg Asp Pro Gln Ala Val Gly Ser Glu
            980                 985                 990
Arg Gln Ala Lys Val Thr Ser Asp Ile Asn Arg Thr Ala Val Thr Ser
        995                 1000                1005
Ile Leu Ser Leu Ser Pro Asn Gln Leu Phe Cys Asp Ser Ala Ile His
    1010                1015                1020
Tyr Ser Arg Asn Glu Glu Glu Val Gly Ile Ile Ala Asp Asn Ile Thr
1025                1030                1035                1040
Pro Val Tyr Pro His Gly Leu Arg Val Leu Tyr Glu Ser Leu Pro Phe
                1045                1050                1055
His Lys Ala Glu Lys Val Val Asn Met Ile Ser Gly Thr Lys Ser Ile
            1060                1065                1070
Thr Asn Leu Leu Gln Arg Thr Ser Ala Ile Asn Gly Glu Asp Ile Asp
        1075                1080                1085
Arg Ala Val Ser Met Met Leu Glu Asn Leu Gly Leu Leu Ser Arg Ile
    1090                1095                1100
Leu Ser Val Ile Ile Asn Ser Ile Glu Ile Pro Ile Lys Ser Asn Gly
1105                1110                1115                1120
Arg Leu Ile Cys Cys Gln Ile Ser Lys Thr Leu Arg Glu Lys Ser Trp
                1125                1130                1135
Asn Asn Met Glu Ile Val Gly Val Thr Ser Pro Ser Ile Val Thr Cys
            1140                1145                1150
Met Asp Val Val Tyr Ala Thr Ser Ser His Leu Lys Gly Ile Ile Ile
        1155                1160                1165
Glu Lys Phe Ser Thr Asp Lys Thr Thr Arg Gly Gln Arg Gly Pro Lys
    1170                1175                1180
Ser Pro Trp Val Gly Ser Ser Thr Gln Glu Lys Lys Leu Val Pro Val
1185                1190                1195                1200
Tyr Asn Arg Gln Ile Leu Ser Lys Gln Gln Lys Glu Gln Leu Glu Ala
                1205                1210                1215
Ile Gly Lys Met Arg Trp Val Tyr Lys Gly Thr Pro Gly Leu Arg Arg
            1220                1225                1230
Leu Leu Asn Lys Ile Cys Ile Gly Ser Leu Gly Ile Ser Tyr Lys Cys
        1235                1240                1245
Val Lys Pro Leu Leu Pro Arg Phe Met Ser Val Asn Phe Leu His Arg
    1250                1255                1260
Leu Ser Val Ser Ser Arg Pro Met Glu Phe Pro Ala Ser Val Pro Ala
1265                1270                1275                1280
Tyr Arg Thr Thr Asn Tyr His Phe Asp Thr Ser Pro Ile Asn Gln Ala
                1285                1290                1295

Leu Ser Glu Arg Phe Gly Asn Glu Asp Ile Asn Leu Val Phe Gln Asn
            1300                1305                1310
Ala Ile Ser Cys Gly Ile Ser Ile Met Ser Val Val Glu Gln Leu Thr
        1315                1320                1325
Gly Arg Ser Pro Lys Gln Leu Val Leu Ile Pro Gln Leu Glu Glu Ile
    1330                1335                1340
Asp Ile Met Pro Pro Pro Val Phe Gln Gly Lys Phe Asn Tyr Lys Leu
1345                1350                1355                1360
Val Asp Lys Ile Thr Ser Asp Gln His Ile Phe Ser Pro Asp Lys Ile
            1365                1370                1375
Asp Ile Leu Thr Leu Gly Lys Met Leu Met Pro Thr Ile Lys Gly Gln
        1380                1385                1390
Lys Thr Asp Gln Phe Leu Asn Lys Arg Glu Asn Tyr Phe His Gly Asn
        1395                1400                1405
Asn Leu Ile Glu Ser Leu Ser Ala Ala Leu Ala Cys His Trp Cys Gly
    1410                1415                1420
Ile Leu Thr Glu Gln Cys Ile Glu Asn Asn Ile Phe Arg Lys Asp Trp
1425                1430                1435                1440
Gly Asp Gly Phe Ile Ser Asp His Ala Phe Met Asp Phe Lys Val Phe
            1445                1450                1455
Leu Cys Val Phe Lys Thr Lys Leu Leu Cys Ser Trp Gly Ser Gln Gly
        1460                1465                1470
Lys Asn Val Lys Asp Glu Asp Ile Ile Asp Glu Ser Ile Asp Lys Leu
        1475                1480                1485
Leu Arg Ile Asp Asn Thr Phe Trp Arg Met Phe Ser Lys Val Met Phe
    1490                1495                1500
Glu Ser Lys Val Lys Lys Arg Ile Met Leu Tyr Asp Val Lys Phe Leu
1505                1510                1515                1520
Ser Leu Val Gly Tyr Ile Gly Phe Lys Asn Trp Phe Ile Glu Gln Leu
            1525                1530                1535
Arg Val Val Glu Leu His Glu Val Pro Trp Ile Val Asn Ala Glu Gly
        1540                1545                1550
Glu Leu Val Glu Ile Lys Ser Ile Lys Ile Tyr Leu Gln Leu Ile Glu
        1555                1560                1565
Gln Ser Leu Ser Leu Arg Ile Thr Val Leu Asn Tyr Thr Asp Met Ala
    1570                1575                1580
His Ala Leu Thr Arg Leu Ile Arg Lys Lys Leu Met Cys Asp Asn Ala
1585                1590                1595                1600
Leu Phe Asn Pro Ser Ser Ser Pro Met Phe Asn Leu Thr Gln Val Ile
            1605                1610                1615
Asp Pro Thr Thr Gln Leu Asp Tyr Phe Pro Arg Ile Ile Phe Glu Arg
        1620                1625                1630
Leu Lys Ser Tyr Asp Thr Ser Ser Asp Tyr Asn Lys Gly Lys Leu Thr
        1635                1640                1645
Arg Asn Tyr Met Thr Leu Leu Pro Trp Gln His Val Asn Arg Tyr Asn
    1650                1655                1660
Phe Val Phe Ser Ser Thr Gly Cys Lys Val Ser Leu Lys Thr Cys Ile
1665                1670                1675                1680
Gly Lys Leu Ile Lys Asp Leu Asn Pro Lys Val Leu Tyr Phe Ile Gly
            1685                1690                1695
Glu Gly Ala Gly Asn Trp Met Ala Arg Thr Ala Cys Glu Tyr Pro Asp
        1700                1705                1710
Ile Lys Phe Val Tyr Arg Ser Leu Lys Asp Asp Leu Asp His His Tyr
        1715                1720                1725
Pro Leu Glu Tyr Gln Arg Val Ile Gly Asp Leu Asn Arg Val Ile Asp
    1730                1735                1740
Ser Gly Glu Gly Leu Ser Met Glu Thr Thr Asp Ala Thr Gln Lys Thr
1745                1750                1755                1760
His Trp Asp Leu Ile His Arg Ile Ser Lys Asp Ala Leu Leu Ile Thr
            1765                1770                1775
Leu Cys Asp Ala Glu Phe Lys Asn Arg Asp Asp Phe Phe Lys Met Val
        1780                1785                1790

Ile Leu Trp Arg Lys His Val Leu Ser Cys Arg Ile Cys Thr Ala Tyr
        1795                1800                1805
Gly Thr Asp Leu Tyr Leu Phe Ala Lys Tyr His Ala Val Asp Cys Asn
    1810                1815                1820
Ile Lys Leu Pro Phe Phe Val Arg Ser Val Ala Thr Phe Ile Met Gln
1825                1830                1835                1840
Gly Ser Lys Leu Ser Gly Ser Glu Cys Tyr Ile Leu Leu Thr Leu Gly
                1845                1850                1855
His His Asn Asn Leu Pro Cys His Gly Glu Ile Gln Asn Ser Lys Met
            1860                1865                1870
Arg Ile Ala Val Cys Asn Asp Phe Tyr Ala Ser Lys Lys Leu Asp Asn
        1875                1880                1885
Lys Ser Ile Glu Ala Asn Cys Lys Ser Leu Leu Ser Gly Leu Arg Ile
    1890                1895                1900
Pro Ile Asn Lys Lys Glu Leu Asn Arg Gln Lys Lys Leu Leu Thr Leu
1905                1910                1915                1920
Gln Ser Asn His Ser Ser Ile Ala Thr Val Gly Gly Ser Lys Ile Ile
                1925                1930                1935
Glu Ser Lys Trp Leu Lys Asn Lys Ala Ser Thr Ile Ile Asp Trp Leu
            1940                1945                1950
Glu His Ile Leu Asn Ser Pro Lys Gly Glu Leu Asn Tyr Asp Phe Phe
        1955                1960                1965
Glu Ala Leu Glu Asn Thr Tyr Pro Asn Met Ile Lys Leu Ile Asp Asn
    1970                1975                1980
Leu Gly Asn Ala Glu Ile Lys Lys Leu Ile Lys Val Thr Gly Tyr Met
1985                1990                1995                2000
Leu Val Ser Lys Lys
                2005

<210> 37
<211> 2005
<212> PRT
<213> human Metapneumo virus

<400> 37
Met Asp Pro Phe Cys Glu Ser Thr Val Asn Val Tyr Leu Pro Asp Ser
1               5                   10                  15
Tyr Leu Lys Gly Val Ile Ser Phe Ser Glu Thr Asn Ala Ile Gly Ser
            20                  25                  30
Cys Leu Leu Lys Arg Pro Tyr Leu Lys Lys Asp Asn Thr Ala Lys Val
        35                  40                  45
Ala Val Glu Asn Pro Val Val Glu His Val Arg Leu Arg Asn Ala Val
    50                  55                  60
Met Thr Lys Met Lys Ile Ser Asp Tyr Lys Val Val Glu Pro Ile Asn
65                  70                  75                  80
Met Gln His Glu Ile Met Lys Asn Ile His Ser Cys Glu Leu Thr Leu
                85                  90                  95
Leu Lys Gln Phe Leu Thr Arg Ser Lys Asn Ile Ser Ser Leu Lys Leu
            100                 105                 110
Ser Met Ile Cys Asp Trp Leu Gln Leu Lys Ser Thr Ser Asp Asn Thr
        115                 120                 125
Ser Ile Leu Asn Phe Ile Asp Val Glu Phe Ile Pro Val Trp Val Ser
    130                 135                 140
Asn Trp Phe Ser Asn Trp Tyr Asn Leu Asn Lys Leu Ile Leu Glu Phe
145                 150                 155                 160
Arg Arg Glu Glu Val Ile Arg Thr Gly Ser Ile Leu Cys Arg Ser Leu
                165                 170                 175
Gly Lys Leu Val Phe Ile Val Ser Ser Tyr Gly Cys Val Val Lys Ser
            180                 185                 190
Asn Lys Ser Lys Arg Val Ser Phe Phe Thr Tyr Asn Gln Leu Leu Thr
        195                 200                 205

Trp Lys Asp Val Met Leu Ser Arg Phe Asn Ala Asn Phe Cys Ile Trp
    210                 215                 220
Val Ser Asn Asn Leu Asn Lys Asn Gln Glu Gly Leu Gly Phe Arg Ser
225                 230                 235                 240
Asn Leu Gln Gly Met Leu Thr Asn Lys Leu Tyr Glu Thr Val Asp Tyr
                245                 250                 255
Met Leu Ser Leu Cys Ser Asn Glu Gly Phe Ser Leu Val Lys Glu Phe
            260                 265                 270
Glu Gly Phe Ile Met Ser Glu Ile Leu Lys Ile Thr Glu His Ala Gln
        275                 280                 285
Phe Ser Thr Arg Phe Arg Asn Thr Leu Leu Asn Gly Leu Thr Glu Gln
    290                 295                 300
Leu Ser Met Leu Lys Ala Lys Asn Arg Ser Arg Val Leu Gly Thr Ile
305                 310                 315                 320
Leu Glu Asn Asn Asp Tyr Pro Met Tyr Glu Val Val Leu Lys Leu Leu
                325                 330                 335
Gly Asp Thr Leu Lys Ser Ile Lys Leu Leu Ile Asn Lys Asn Leu Glu
            340                 345                 350
Asn Ala Ala Glu Leu Tyr Tyr Ile Phe Arg Ile Phe Gly His Pro Met
        355                 360                 365
Val Asp Glu Arg Glu Ala Met Asp Ala Val Lys Leu Asn Asn Glu Ile
    370                 375                 380
Thr Lys Ile Leu Lys Leu Glu Ser Leu Thr Glu Leu Arg Gly Ala Phe
385                 390                 395                 400
Ile Leu Arg Ile Ile Lys Gly Phe Val Asp Asn Asn Lys Arg Trp Pro
                405                 410                 415
Lys Ile Lys Asn Leu Lys Val Leu Ser Lys Arg Trp Val Met Tyr Phe
            420                 425                 430
Lys Ala Lys Ser Tyr Pro Ser Gln Leu Glu Leu Ser Val Gln Asp Phe
        435                 440                 445
Leu Glu Leu Ala Ala Val Gln Phe Glu Gln Glu Phe Ser Val Pro Glu
    450                 455                 460
Lys Thr Asn Leu Glu Met Val Leu Asn Asp Lys Ala Ile Ser Pro Pro
465                 470                 475                 480
Lys Lys Leu Ile Trp Ser Val Tyr Pro Lys Asn Tyr Leu Pro Glu Ile
                485                 490                 495
Ile Lys Asn Gln Tyr Leu Glu Glu Val Phe Asn Ala Ser Asp Ser Gln
            500                 505                 510
Arg Thr Arg Arg Val Leu Glu Phe Tyr Leu Lys Asp Cys Lys Phe Asp
        515                 520                 525
Gln Lys Asp Leu Lys Arg Tyr Val Leu Lys Gln Glu Tyr Leu Asn Asp
    530                 535                 540
Lys Asp His Ile Val Ser Leu Thr Gly Lys Glu Arg Glu Leu Ser Val
545                 550                 555                 560
Gly Arg Met Phe Ala Met Gln Pro Gly Lys Gln Arg Gln Ile Gln Ile
                565                 570                 575
Leu Ala Glu Lys Leu Leu Ala Asp Asn Ile Val Pro Phe Phe Pro Glu
            580                 585                 590
Thr Leu Thr Lys Tyr Gly Asp Leu Asp Leu Gln Arg Ile Met Glu Met
        595                 600                 605
Lys Ser Glu Leu Ser Ser Ile Lys Thr Arg Lys Asn Asp Ser Tyr Asn
    610                 615                 620
Asn Tyr Ile Ala Arg Ala Ser Ile Val Thr Asp Leu Ser Lys Phe Asn
625                 630                 635                 640
Gln Ala Phe Arg Tyr Glu Thr Thr Ala Ile Cys Ala Asp Val Ala Asp
                645                 650                 655
Glu Leu His Gly Thr Gln Ser Leu Phe Cys Trp Leu His Leu Ile Val
            660                 665                 670
Pro Met Thr Thr Met Ile Cys Ala Tyr Arg His Ala Pro Pro Glu Thr
        675                 680                 685
Lys Gly Glu Tyr Asp Ile Asp Lys Ile Glu Glu Gln Ser Gly Leu Tyr
    690                 695                 700

Arg Tyr His Met Gly Gly Ile Glu Gly Trp Cys Gln Lys Leu Trp Thr
705                 710                 715                 720
Met Glu Ala Ile Ser Leu Leu Asp Val Val Ser Val Lys Thr Arg Cys
                725                 730                 735
Gln Met Thr Ser Leu Leu Asn Gly Asp Asn Gln Ser Ile Asp Val Ser
            740                 745                 750
Lys Pro Val Lys Leu Ser Glu Gly Ile Asp Glu Val Lys Ala Asp Tyr
        755                 760                 765
Ser Leu Ala Ile Lys Met Leu Lys Glu Ile Arg Asp Ala Tyr Lys Asn
    770                 775                 780
Ile Gly His Lys Leu Lys Glu Gly Glu Thr Tyr Ile Ser Arg Asp Leu
785                 790                 795                 800
Gln Phe Ile Ser Lys Val Ile Gln Ser Glu Gly Val Met His Pro Thr
                805                 810                 815
Pro Ile Lys Lys Ile Leu Arg Val Gly Pro Trp Ile Asn Thr Ile Leu
            820                 825                 830
Asp Asp Ile Lys Thr Ser Ala Glu Ser Ile Gly Ser Leu Cys Gln Glu
        835                 840                 845
Leu Glu Phe Arg Gly Glu Ser Met Leu Val Ser Leu Ile Leu Arg Asn
    850                 855                 860
Phe Trp Leu Tyr Asn Leu Tyr Met His Glu Ser Lys Gln His Pro Leu
865                 870                 875                 880
Ala Gly Lys Gln Leu Phe Lys Gln Leu Asn Lys Thr Leu Thr Ser Val
                885                 890                 895
Gln Arg Phe Phe Glu Leu Lys Lys Glu Asn Asp Val Val Asp Leu Trp
            900                 905                 910
Met Asn Ile Pro Met Gln Phe Gly Gly Gly Asp Pro Val Val Phe Tyr
        915                 920                 925
Arg Ser Phe Tyr Arg Arg Thr Pro Asp Phe Leu Thr Glu Ala Ile Ser
    930                 935                 940
His Val Asp Leu Leu Leu Lys Val Ser Asn Asn Ile Lys Asn Glu Thr
945                 950                 955                 960
Lys Ile Arg Phe Phe Lys Ala Leu Leu Ser Ile Glu Lys Asn Glu Arg
                965                 970                 975
Ala Thr Leu Thr Thr Leu Met Arg Asp Pro Gln Ala Val Gly Ser Glu
            980                 985                 990
Arg Gln Ala Lys Val Thr Ser Asp Ile Asn Arg Thr Ala Val Thr Ser
        995                 1000                1005
Ile Leu Ser Leu Ser Pro Asn Gln Leu Phe Cys Asp Ser Ala Ile His
    1010                1015                1020
Tyr Ser Arg Asn Glu Glu Glu Val Gly Ile Ile Ala Asp Asn Ile Thr
1025                1030                1035                1040
Pro Val Tyr Pro His Gly Leu Arg Val Leu Tyr Glu Ser Leu Pro Phe
                1045                1050                1055
His Lys Ala Glu Lys Val Val Asn Met Ile Ser Gly Thr Lys Ser Ile
            1060                1065                1070
Thr Asn Leu Leu Gln Arg Thr Ser Ala Ile Asn Gly Glu Asp Ile Asp
        1075                1080                1085
Arg Ala Val Ser Met Met Leu Glu Asn Leu Gly Leu Leu Ser Arg Ile
    1090                1095                1100
Leu Ser Val Ile Ile Asn Ser Ile Glu Ile Pro Ile Lys Ser Asn Gly
1105                1110                1115                1120
Arg Leu Ile Cys Cys Gln Ile Ser Lys Thr Leu Arg Glu Lys Ser Trp
                1125                1130                1135
Asn Asn Met Glu Ile Val Gly Val Thr Ser Pro Ser Ile Val Thr Cys
            1140                1145                1150
Met Asp Val Val Tyr Ala Thr Ser Ser His Leu Lys Gly Ile Ile Ile
        1155                1160                1165
Glu Lys Phe Ser Thr Asp Lys Thr Thr Arg Gly Gln Arg Gly Pro Lys
    1170                1175                1180
Ser Pro Trp Val Gly Ser Ser Thr Gln Glu Lys Lys Leu Val Pro Val
1185                1190                1195                1200

Tyr Asn Arg Gln Ile Leu Ser Lys Gln Gln Lys Glu Gln Leu Glu Ala
                1205                1210                1215
Ile Gly Lys Met Arg Trp Val Tyr Lys Gly Thr Pro Gly Leu Arg Arg
            1220                1225                1230
Leu Leu Asn Lys Ile Cys Ile Gly Ser Leu Gly Ile Ser Tyr Lys Cys
        1235                1240                1245
Val Lys Pro Leu Leu Pro Arg Phe Met Ser Val Asn Phe Leu His Arg
    1250                1255                1260
Leu Ser Val Ser Ser Arg Pro Met Glu Phe Pro Ala Ser Val Pro Ala
1265                1270                1275                1280
Tyr Arg Thr Thr Asn Tyr His Phe Asp Thr Ser Pro Ile Asn Gln Ala
                1285                1290                1295
Leu Ser Glu Arg Phe Gly Asn Glu Asp Ile Asn Leu Val Phe Gln Asn
            1300                1305                1310
Ala Ile Ser Cys Gly Ile Ser Ile Met Ser Val Val Glu Gln Leu Thr
        1315                1320                1325
Gly Arg Ser Pro Lys Gln Leu Val Leu Ile Pro Gln Leu Glu Glu Ile
    1330                1335                1340
Asp Ile Met Pro Pro Pro Val Phe Gln Gly Lys Phe Asn Tyr Lys Leu
1345                1350                1355                1360
Val Asp Lys Ile Thr Ser Asp Gln His Ile Phe Ser Pro Asp Lys Ile
                1365                1370                1375
Asp Ile Leu Thr Leu Gly Lys Met Leu Met Pro Thr Ile Lys Gly Gln
            1380                1385                1390
Lys Thr Asp Gln Phe Leu Asn Lys Arg Glu Asn Tyr Phe His Gly Asn
        1395                1400                1405
Asn Leu Ile Glu Ser Leu Ser Ala Ala Leu Ala Cys His Trp Cys Gly
    1410                1415                1420
Ile Leu Thr Glu Gln Cys Val Glu Asn Asn Ile Phe Arg Lys Asp Trp
1425                1430                1435                1440
Gly Asp Gly Phe Ile Ser Asp His Ala Phe Met Asp Phe Lys Ile Phe
                1445                1450                1455
Leu Cys Val Phe Lys Thr Lys Leu Leu Cys Ser Trp Gly Ser Gln Gly
            1460                1465                1470
Lys Asn Val Lys Asp Glu Asp Ile Ile Asp Glu Ser Ile Asp Lys Leu
        1475                1480                1485
Leu Arg Ile Asp Asn Thr Phe Trp Arg Met Phe Ser Lys Val Met Phe
    1490                1495                1500
Glu Ser Lys Val Lys Lys Arg Ile Met Leu Tyr Asp Val Lys Phe Leu
1505                1510                1515                1520
Ser Leu Val Gly Tyr Ile Gly Phe Lys Asn Trp Phe Ile Glu Gln Leu
                1525                1530                1535
Arg Val Val Glu Leu His Glu Val Pro Trp Ile Val Asn Ala Glu Gly
            1540                1545                1550
Glu Leu Val Glu Ile Lys Pro Ile Lys Ile Tyr Leu Gln Leu Ile Glu
        1555                1560                1565
Gln Ser Leu Ser Leu Arg Ile Thr Val Leu Asn Tyr Thr Asp Met Ala
    1570                1575                1580
His Ala Leu Thr Arg Leu Ile Arg Lys Lys Leu Met Cys Asp Asn Ala
1585                1590                1595                1600
Leu Phe Asn Pro Ser Ser Ser Pro Met Phe Ser Leu Thr Gln Val Ile
                1605                1610                1615
Asp Pro Thr Thr Gln Leu Asp Tyr Phe Pro Lys Val Ile Phe Glu Arg
            1620                1625                1630
Leu Lys Ser Tyr Asp Thr Ser Ser Asp Tyr Asn Lys Gly Lys Leu Thr
        1635                1640                1645
Arg Asn Tyr Met Thr Leu Leu Pro Trp Gln His Val Asn Arg Tyr Asn
    1650                1655                1660
Phe Val Phe Ser Ser Thr Gly Cys Lys Ile Ser Leu Lys Thr Cys Ile
1665                1670                1675                1680
Gly Lys Leu Ile Lys Asp Leu Asn Pro Lys Val Leu Tyr Phe Ile Gly
                1685                1690                1695

Glu Gly Ala Gly Asn Trp Met Ala Arg Thr Ala Cys Glu Tyr Pro Asp
            1700                1705                1710
Ile Lys Phe Val Tyr Arg Ser Leu Lys Asp Asp Leu Asp His His Tyr
        1715                1720                1725
Pro Leu Glu Tyr Gln Arg Val Ile Gly Asp Leu Asn Arg Val Ile Asp
    1730                1735                1740
Gly Gly Glu Gly Leu Ser Met Glu Thr Thr Asp Ala Thr Gln Lys Thr
1745                1750                1755                1760
His Trp Asp Leu Ile His Arg Ile Ser Lys Asp Ala Leu Leu Ile Thr
                1765                1770                1775
Leu Cys Asp Ala Glu Phe Lys Asn Arg Asp Asp Phe Phe Lys Met Val
            1780                1785                1790
Ile Leu Trp Arg Lys His Val Leu Ser Cys Arg Ile Cys Thr Ala Tyr
        1795                1800                1805
Gly Thr Asp Leu Tyr Leu Phe Ala Lys Tyr His Ala Thr Asp Cys Asn
    1810                1815                1820
Ile Lys Leu Pro Phe Phe Val Arg Ser Val Ala Thr Phe Ile Met Gln
1825                1830                1835                1840
Gly Ser Lys Leu Ser Gly Ser Glu Cys Tyr Ile Leu Leu Thr Leu Gly
                1845                1850                1855
His His Asn Asn Leu Pro Cys His Gly Glu Ile Gln Asn Ser Lys Met
            1860                1865                1870
Arg Ile Ala Val Cys Asn Asp Phe His Ala Ser Lys Lys Leu Asp Asn
        1875                1880                1885
Lys Ser Ile Glu Ala Asn Cys Lys Ser Leu Leu Ser Gly Leu Arg Ile
    1890                1895                1900
Pro Ile Asn Lys Lys Glu Leu Asn Arg Gln Lys Lys Leu Leu Thr Leu
1905                1910                1915                1920
Gln Ser Asn His Ser Ser Ile Ala Thr Val Gly Gly Ser Lys Ile Ile
                1925                1930                1935
Glu Ser Lys Trp Leu Lys Asn Lys Ala Ser Thr Ile Ile Asp Trp Leu
            1940                1945                1950
Glu His Ile Leu Asn Ser Pro Lys Gly Glu Leu Asn Tyr Asp Phe Phe
        1955                1960                1965
Glu Ala Leu Glu Asn Thr Tyr Pro Asn Met Ile Lys Leu Ile Asp Asn
    1970                1975                1980
Leu Gly Asn Ala Glu Ile Lys Lys Leu Ile Lys Val Pro Gly Tyr Met
1985                1990                1995                2000
Leu Val Ser Lys Lys
                2005

<210> 38
<211> 6018
<212> DNA
<213> human Metapneumo virus

<400> 38
atggatcctc tcaatgaatc cactgttaat gtctatcttc ctgactcata tcttaaagga 60
gtgatttcct ttagtgagac taatgcaatt ggttcatgtc tcttaaaaag accttaccta 120
aaaaatgaca acactgcaaa agttgccata gagaatcctg ttatcgagca tgttagactc 180
aaaaatgcag tcaattctaa gatgaaaata tcagattaca agatagtaga gccagtaaac 240
atgcaacatg aaattatgaa gaatgtacac agttgtgagc tcacattatt aaaacagttt 300
ttaacaagga gtaaaaatat tagcactctc aaattaaata tgatatgtga ttggctgcag 360
ttaaagtcta catcagatga tacctcaatc ctaagtttta tagatgtaga atttatacct 420
agctgggtaa gcaattggtt tagtaattgg tacaatctca acaagttgat tctggaattc 480
aggaaagaag aagtaataag aactggttca atcttgtgta ggtcattggg taaattagtt 540
tttgttgtat catcatatgg atgtatagtc aagagcaaca aaagcaaaag agtgagcttc 600
ttcacataca atcaactgtt aacatggaaa gatgtgatgt taagtagatt caatgcaaat 660
ttttgtatat gggtaagcaa cagtctgaat gaaaatcaag aagggctagg gttgagaagt 720
aatctgcaag gcatattaac taataagcta tatgaaactg tagattatat gcttagttta 780
tgttgcaatg aaggtttctc acttgtgaaa gagttcgaag gctttattat gagtgaaatt 840

cttaggatta ctgaacatgc tcaattcagt actagattta gaaatacttt attaaatgga 900
ttaactgatc aattaacaaa attaaaaaat aaaaacagac tcagagttca tggtaccgtg 960
ttagaaaata atgattatcc aatgtacgaa gttgtactta agttattagg agatactttg 1020
agatgtatta aattattaat caataaaaac ttagagaatg ctgctgaatt atactatata 1080
tttagaatat tcggtcaccc aatggtagat gaaagagatg caatggatgc tgtcaaatta 1140
aacaatgaaa tcacaaaaat ccttaggtgg gagagcttga cagaactaag aggggcattc 1200
atattaagga ttatcaaagg atttgtagac aacaacaaaa gatggcccaa aattaaaaac 1260
ttaaaagtgc ttagtaagag atggactatg tacttcaaag caaaaagtta ccccagtcaa 1320
cttgaattaa gcgaacaaga ttttttagag cttgctgcaa tacagtttga acaagagttt 1380
tctgtccctg aaaaaaccaa ccttgagatg gtattaaatg ataaagctat atcacctcct 1440
aaaagattaa tatggtctgt gtatccaaaa aattacttac ctgagaaaat aaaaaatcga 1500
tatctagaag agactttcaa tgcaagtgat agtctcaaaa caagaagagt actagagtac 1560
tatttgaaag ataataaatt cgaccaaaaa gaacttaaaa gttatgttgt taaacaagaa 1620
tatttaaatg ataaggatca tattgtctcg ctaactggaa aagaaagaga attaagtgta 1680
ggtagaatgt ttgctatgca accaggaaaa cagcgacaaa tacaaatatt ggctgaaaaa 1740
ttgttagctg ataatattgt accttttttc ccagaaacct taacaaagta tggtgatcta 1800
gatcttcaga gaataatgga aatcaaatcg gaactttctt ctattaaaac tagaagaaat 1860
gatagttata ataattacat tgcaagagca tccatagtaa cagatttaag taagttcaac 1920
caagccttta ggtatgaaac tacagcgatc tgtgcggatg tagcagatga actacatgga 1980
acacaaagcc tattctgttg gttacatctt atcgtcccta tgacaacaat gatatgtgcc 2040
tatagacatg caccaccaga aacaaaaggt gaatatgata tagataagat agaagagcaa 2100
agtggtttat atagatatca tatgggtggt attgaaggat ggtgtcaaaa actctggaca 2160
atggaagcta tatctctatt agatgttgta tctgtaaaaa cacgatgtca aatgacatct 2220
ttattaaacg gtgacaacca atcaatagat gtaagtaaac cagttaagtt atctgagggt 2280
ttagatgaag tgaaagcaga ttatagcttg gctgtaaaaa tgttaaaaga aataagagat 2340
gcatacagaa atataggcca taaacttaaa gaaggggaaa catatatatc aagagatctt 2400
cagtttataa gtaaggtgat tcaatctgaa ggagtaatgc atcctacccc tataaaaaag 2460
atcttaagag tgggaccatg gataaacaca atattagatg acattaaaac cagtgcagag 2520
tcaataggga gtctatgtca ggaattagaa tttagggggg aaagcataat agttagtctg 2580
atattaagga atttttggct gtataattta tacatgcatg aatcaaagca acacccccta 2640
gcagggaagc agttattcaa acaactaaat aaaacattaa catcagtgca gagatttttt 2700
gaaataaaaa aggaaaatga agtagtagat ctatggatga acataccaat gcagtttgga 2760
ggaggagatc cagtagtctt ctatagatct ttctatagaa ggacccctga ttttttaact 2820
gaagcaatca gtcatgtgga tattctgtta agaatatcag ccaacataag aaatgaagcg 2880
aaaataagtt tcttcaaagc cttactgtca atagaaaaaa atgaacgtgc tacactgaca 2940
acactaatga gagatcctca agctgttggc tcagagcgac aagcaaaagt aacaagtgat 3000
atcaatagaa cagcagttac cagcatctta agtctttctc caaatcaact tttcagcgat 3060
agtgctatac actacagtag aaatgaagaa gaggtcggaa tcattgctga caacataaca 3120
cctgtttatc ctcatggact gagagttttg tatgaatcat taccttttca taaagctgaa 3180
aaagttgtga atatgatatc aggaacgaaa tccataacca acttattaca gagaacatct 3240
gctattaatg gtgaagatat tgacagagct gtatccatga tgctggagaa cctaggatta 3300
ttatctagaa tattgtcagt agttgttgat agtatagaaa ttccaaccaa atctaatggt 3360
aggctgatat gttgtcagat atctagaacc ctaagggaga catcatggaa taatatggaa 3420
atagttggag taacatcccc tagcatcact acatgcatgg atgtcatata tgcaactagc 3480
tctcatttga aagggataat cattgaaaag ttcagcactg acagaactac aagaggtcaa 3540
agaggtccaa agagcccttg ggtagggtcg agcactcaag agaaaaaatt agttcctgtt 3600
tataacagac aaattctttc aaaacaacaa agagaacagc tagaagcaat tggaaaaatg 3660
agatgggtat ataaagggac accaggttta agacgattac tcaataagat ttgtcttgga 3720
agtttaggca ttagttacaa atgtgtaaaa cctttattac ctaggtttat gagtgtaaat 3780
ttcctacaca ggttatctgt cagtagtaga cctatggaat tcccagcatc agttccagct 3840
tatagaacaa caaattacca ttttgacact agtcctatta atcaagcact aagtgagaga 3900
tttgggaatg aagatattaa tttggtcttc caaaatgcaa tcagctgtgg aattagcata 3960
atgagtgtag tagaacaatt aactggtagg agtccaaaac agttagtttt aatacctcaa 4020
ttagaagaaa tagacattat gccaccacca gtgtttcaag ggaaattcaa ttataagcta 4080
gtagataaga taacttctga tcaacatatc ttcagtccag acaaaataga tatgttaaca 4140
ctggggaaaa tgctcatgcc cactataaaa ggtcagaaaa cagatcagtt cctgaacaag 4200
agagagaatt atttccatgg gaataatctt attgagtctt tgtcagcagc gttagcatgt 4260
cattggtgtg ggatattaac agagcaatgt atagaaaata atattttcaa gaaagactgg 4320
ggtgacgggt tcatatcgga tcatgctttt atggacttca aaatattcct atgtgtcttt 4380
aaaactaaac ttttatgtag ttgggggtcc caagggaaaa acattaaaga tgaagatata 4440
gtagatgaat caatagataa actgttaagg attgataata ctttttggag aatgttcagc 4500
aaggttatgt ttgaatcaaa ggttaagaaa aggataatgt tatatgatgt aaaatttcta 4560

tcattagtag gttatatagg gtttaagaat tggtttatag aacagttgag atcagctgag 4620
ttgcatgagg taccttggat tgtcaatgcc gaaggtgatc tggttgagat caagtcaatt 4680
aaaatctatt tgcaactgat agagcaaagt ttatttttaa gaataactgt tttgaactat 4740
acagatatgg cacatgctct cacaagatta atcagaaaga agttgatgtg tgataatgca 4800
ctattaactc cgattccatc cccaatggtt aatttaactc aagttattga tcctacagaa 4860
caattagctt atttccctaa gataacattt gaaaggctaa aaaattatga cactagttca 4920
aattatgcta aaggaaagct aacaaggaat tacatgatac tgttgccatg gcaacatgtt 4980
aatagatata actttgtctt tagttctact ggatgtaaag ttagtctaaa aacatgcatt 5040
ggaaaactta tgaaagatct aaaccctaaa gttctgtact ttattggaga aggggcagga 5100
aattggatgg ccagaacagc atgtgaatat cctgacatca aatttgtata cagaagttta 5160
aaagatgacc ttgatcatca ttatcctttg gaataccaga gagttatagg agaattaagc 5220
aggataatag atagcggtga agggctttca atggaaacaa cagatgcaac tcaaaaaact 5280
cattgggatt tgatacacag agtaagcaaa gatgctttat taataacttt atgtgatgca 5340
gaatttaagg acagagatga tttttttaag atggtaattc tatggaggaa acatgtatta 5400
tcatgcagaa tttgcactac ttatgggaca gacctctatt tattcgcaaa gtatcatgct 5460
aaagactgca atgtaaaatt accttttttt gtgagatcag tagccacctt tattatgcaa 5520
ggtagtaaac tgtcaggctc agaatgctac atactcttaa cactaggcca ccacaacaat 5580
ttaccctgcc atggagaaat acaaaattct aagatgaaaa tagcagtgtg taatgatttt 5640
tatgctgcaa aaaaacttga caataaatct attgaagcca actgtaaatc acttttatca 5700
gggctaagaa taccgataaa taagaaagaa ttaaatagac agagaaggtt attaacacta 5760
caaagcaacc attcttctgt agcaacagtt ggaggtagca aggtcataga gtctaaatgg 5820
ttaacaaaca aggcaaacac aataattgat tggttagaac atattttaaa ttctccaaaa 5880
ggtgaattaa attatgattt ttttgaagca ttagaaaata cttaccctaa tatgattaaa 5940
ctaatagata atctagggaa tgcagagata aaaaaactga tcaaagtaac tggatatatg 6000
cttgtaagta aaaaatga 6018

<210> 39
<211> 6018
<212> DNA
<213> human Metapneumo virus

<400> 39
atggatcctc ttaatgaatc cactgttaat gtctatctcc ctgattcgta ccttaaagga 60
gtaatttctt ttagtgaaac taatgcaatt ggttcatgtc tcttaaaaag accttactta 120
aaaaatgaca acactgcaaa agttgccata gagaatcctg ttattgagca tgtgagactc 180
aaaaatgcag tcaattctaa aatgaaaata tcagattaca aggtagtaga gccagtaaac 240
atgcaacatg aaataatgaa gaatgtacac agttgtgagc tcacactatt gaaacagttt 300
ttaacaagga gtaaaaacat tagcactctc aaattaaata tgatatgtga ttggctgcaa 360
ttaaagtcta catcagatga tacctcaatc ctaagtttca tagatgtaga atttatacct 420
agttgggtaa gcaactggtt tagtaattgg tacaatctca ataagttaat tttggaattc 480
agaagagagg aagtaataag aaccggttca atcttatgca ggtcattggg taaattagtt 540
tttattgtat catcatacgg atgtatcgtc aagagcaaca aaagcaaaag agtgagcttc 600
ttcacataca atcaactgtt aacatggaaa gatgtgatgt taagtagatt taatgcgaat 660
ttttgtatat gggtaagcaa tagtctgaat gaaaatcagg aagggctagg gttaagaagt 720
aatctacaag gtatgttaac taataaacta tatgaaactg tagattatat gctaagttta 780
tgttgcaatg aaggtttctc acttgtaaaa gagttcgaag gttttattat gagtgaaatc 840
cttaggatta ctgaacatgc tcaattcagt actagattta gaaatacttt attaaatgga 900
ttaacagatc aattaacaaa attaaaaaat aaaaacagac tcagagttca tggtaccgta 960
ttagaaaata atgattatcc aatgtatgaa gttgtactta aattattagg agatactttg 1020
agatgtatca aattattaat caataaaaac ttagagaatg ctgcagaatt atactatata 1080
ttcagaattt ttggtcatcc aatggtagat gaaagagatg caatggatgc tgtcaaatta 1140
aacaatgaaa tcacaaaaat cctaaggttg gagagcttga cagaactaag aggagcattc 1200
atattaagga ttatcaaagg atttgtggac aacaacaaaa ggtggcccaa aattaaaaat 1260
ttaatagtgc ttagcaaaag atggactatg tacttcaaag ctaaaaatta tcccagtcaa 1320
ctcgaattaa gtgaacaaga ctttctagag cttgctgcaa tacaatttga acaagagttt 1380
tctgttcctg aaaaaaccaa tcttgagatg gtattaaatg acaaagccat atcacctcct 1440
aaaagattaa tatggtctgt gtatccaaag aattacttac ctgagacgat aaaaaatcga 1500
tatttagaag aaactttcaa tgcgagtgat agtctcaaaa caagaagagt actagagtac 1560
tatttaaaag acaataaatt tgatcaaaag gaacttaaaa gttatgtagt tagacaagaa 1620
tatttaaatg ataaggagca cattgtctca ttaactggaa aagaaagaga attaagtgta 1680

ggtagaatgt ttgctatgca accaggaaaa cagcgacaaa tacaaatatt ggcagaaaaa 1740
ttgttagctg ataacattgt acctttcttc ccggaaacct taacaaagta tggtgatcta 1800
gatcttcaga gaataatgga aatcaaatca gaactttctt ctatcaaaac cagaagaaat 1860
gacagttata ataattacat tgcaagagca tccatagtaa cagatttgag caagttcaac 1920
caagccttta gatatgaaac tacagcgatc tgtgcggatg tagcagacga attacatgga 1980
acacaaagct tattctgttg gttacatctt atcgttccta tgactacaat gatatgtgcc 2040
tatagacatg caccaccaga aacaaaaggt gaatatgata tagataagat agaagagcaa 2100
agtggtctat atagatatca catgggcggt attgaaggat ggtgtcaaaa actctggaca 2160
atggaagcta tatctttatt ggatgttgta tctgtaaaga cacggtgtca aatgacatct 2220
ttattaaacg gtgataacca atcaatagat gtaagtaaac cagtcaagtt atctgaaggt 2280
ttagatgaag tgaaggcaga ttatcgctta gcaataaaaa tgctaaaaga aataagagat 2340
gcatacagaa atataggcca taaacttaaa gaaggggaaa catatatatc aagggatctt 2400
caatttataa gcaaggtgat tcaatctgaa ggagtgatgc atcctacccc tataaaaaag 2460
gtcttgagag taggaccatg gataaacaca atattagatg acattaaaac tagtgctgag 2520
tcaataggga gtctatgtca agaattagaa tttaggggag aaagcataat agttagtctg 2580
atattaagaa acttctggct gtataactta tacatgcatg aatcaaagca acatcctttg 2640
gcagggaaac agttattcaa acaactaaat aaaacattaa catcagtgca gagatttttt 2700
gaaattaaaa aggaaaatga ggtagtagat ctatggatga acataccaat gcaatttgga 2760
ggaggagatc cagtagtctt ctatagatct ttctatagaa ggacccctga ttttttaact 2820
gaggcaatca gccatgtaga tattctgtta aaaatatcag ctaacataaa aaatgaaacg 2880
aaagtaagtt tcttcaaagc cttactatca atagaaaaaa atgaacgtgc tacactgaca 2940
acgctaatga gagatcctca agctgttgga tcagaacgac aagcaaaagt aacaagtgac 3000
atcaatagaa cagcagttac cagtatctta agtctttccc caaatcaact tttcagtgat 3060
agtgctatac actatagcag gaatgaagaa gaagtgggaa tcattgcaga aaacataaca 3120
cctgtttatc ctcatgggct gagagtatta tatgaatcat tgccctttca caaagctgaa 3180
aaagttgtaa acatgatatc agggacaaaa tctataacca acttattaca gagaacatcc 3240
gctattaatg gtgaagatat tgacagggct gtatctatga tgttggagaa tctaggatta 3300
ttatctagaa tattgtcagt agttgttgat agtatagaaa ttccaatcaa atctaatggt 3360
aggctgatat gttgtcaaat ctctaggact ttaagagaga catcatggaa taatatggaa 3420
atagttggag taacatctcc tagcatcact acatgtatgg atgtcatata tgcaactagt 3480
tctcatttga aggggataat tatagaaaag ttcagcactg acagaactac aaggggtcaa 3540
agaggtccaa aaagcccttg ggtagggtcg agtactcaag agaaaaaatt agtacctgtt 3600
tataacagac aaattctttc aaaacaacaa agagaacagc tagaagcaat tggaaaaatg 3660
agatgggtgt ataaagggac accaggcttg cgacgattac tcaacaagat ctgtcttggg 3720
agtttaggca ttagttacaa atgtgtaaaa cctttattac ctaggtttat gagtgtaaat 3780
ttcttacata ggttatctgt cagtagtaga cctatggaat tcccagcatc agttccagct 3840
tatagaacaa caaattacca tttcgacact agtcctatta atcaagcact aagtgagaga 3900
tttgggaatg aagatattaa cttggtcttc caaaatgcga tcagctgtgg aattagcata 3960
atgagtgtag tagaacaatt aacaggtaga agcccaaaac agttagtttt aataccccaa 4020
ttagaagaaa tagacattat gccaccacca gtgtttcaag ggaaattcaa ttataaatta 4080
gtagataaga taacttctga tcaacatatc ttcagtccgg acaaaataga tatgttaaca 4140
ctagggaaaa tgctcatgcc tactataaaa ggtcagaaaa cagatcagtt cttaaataag 4200
agagagaatt atttccatgg gaacaatctt attgagtctt tatcagcagc attagcatgt 4260
cattggtgtg ggatattaac agaacaatgc atagaaaata atattttcaa gaaggactgg 4320
ggtgacgggt ttatatcaga tcatgctttt atggacttca aaatattcct atgtgtcttt 4380
aaaactaaac ttttatgtag ttggggatcc caagggaaaa acattaaaga tgaagatata 4440
gtagatgaat caatagataa attgttaagg attgacaata ctttttggag aatgttcagc 4500
aaagttatgt ttgaaccaaa agttaagaaa aggataatgt tatatgatgt aaaattccta 4560
tcactagtag gctacatagg gtttaagaac tggtttatag agcagttgag atcagctgaa 4620
ttgcatgaaa taccttggat tgtcaatgcc gaaggtgatt tggttgagat caagtcaatt 4680
aaaatctatt tgcaactgat agaacaaagc ttatttttaa gaataactgt tttgaactat 4740
acagatatgg cacatgctct cacacgatta atcagaaaga agttaatgtg tgataatgca 4800
ctgttaaccc caatttcatc cccaatggtt aacttaactc aagttattga tcccacaaca 4860
caattagatt acttccccaa gataacattc gaaaggctaa aaaattatga cacaagttca 4920
aattatgcta aaggaaagct aacaagaaat tacatgatac tattgccatg gcagcatgtt 4980
aatagatata actttgtctt tagttctact ggatgtaaag ttagtctgaa aacatgtatt 5040
ggaaaactta tgaaagactt aaatcctaaa gttttgtact ttattggaga aggagcagga 5100
aattggatgg ccagaacagc atgtgaatat cctgatatta aatttgtata tagaagtctg 5160
aaagatgacc ttgatcatca ttatcctctg gaataccaga gagtgatagg tgaattaagc 5220
agaatcatag atagtggtga aggactttca atggaaacaa cagacgcaac tcaaaaaact 5280
cattgggatt tgatacacag ggtaagcaaa gatgctttat taataacttt atgtgatgca 5340
gaatttaagg acagagatga tttttttaag atggtaattc tatggagaaa acatgtatta 5400

tcatgcagaa tttgcactac ttatgggacg gacctctatt tattcgcaaa gtatcatgct 5460
aaagactgca atgtaaaatt accttttttt gtgagatcag ttgctacttt cattatgcag 5520
ggtagtaagc tgtcaggttc agaatgctac atactcttaa cactaggcca ccacaacagt 5580
ttaccttgcc atggagaaat acaaaattct aagatgaaaa tagcagtgtg taatgatttt 5640
tatgctgcaa aaaaactcga caataaatca attgaagcta attgtaaatc acttttgtca 5700
gggctaagaa tacctataaa taagaaggaa ctagatagac agagaagatt attaacacta 5760
caaagcaatc attcttctgt ggcaacagtt ggcggtagca agatcataga gtctaaatgg 5820
ttaacaaaca aagcaagtac aataattgat tggttagaac atattttaaa ttctccaaag 5880
ggcgaattaa attatgattt ttttgaagca ttggagaaca cttaccctaa tatgattaaa 5940
ctaatagata acttagggaa tgcagagatt aaaaaactta tcaaagtaac aggatacatg 6000
cttgtaagta aaaaatga 6018

<210> 40
<211> 6018
<212> DNA
<213> human Metapneumo virus

<400> 40
atggatccct tttgtgaatc tactgttaat gtttatctcc ctgattcata tctcaaagga 60
gtaatatctt ttagtgaaac caatgcaatt ggatcatgtc ttttgaaaag accctatcta 120
aaaaatgaca acactgccaa agttgctgta gaaaaccctg ttgttgaaca tgtgaggctt 180
agaaatgcag tcatgaccaa aatgaagata tcagattata aagtggttga accagttaat 240
atgcagcatg aaataatgaa aaatatacat agttgtgagc ttacattatt aaaacaattc 300
ttaacgagaa gcaaaaacat tagctctcta aaattaaata tgatatgtga ttggttacag 360
ttaaaatcca cttcagataa cacatcaatt ctcaatttta tagatgtgga gttcataccc 420
gtttgggtaa gcaattggtt cagtaactgg tataatctca ataaattaat cttagagttt 480
agaagagaag aagtaataag aactggttca attttatgta gatcactagg caagttagtt 540
tttattgtat catcttatgg atgtgtagta aaaagcaaca aaagtaaaag agtgagcttt 600
ttcacctata accaactgtt aacatggaaa gatgtgatgt taagtagatt caatgcaaac 660
ttttgtatat gggtaagtaa caacctgaac aaaaatcaag aaggactagg acttagaagc 720
aatctgcaag gtatgttaac caataaatta tatgaaactg ttgattacat gctaagccta 780
tgctgcaatg aaggattctc tctggtgaaa gagtttgaag gatttattat gagtgaaatt 840
ctaaaaatta ctgagcatgc tcagttcagt actaggttta ggaatacttt attgaatggg 900
ttaactgaac aattatcagt gttgaaagct aagaacagat ctagagttct tggaactata 960
ttagaaaaca acaattaccc tatgtacgaa gtagtactta aattattagg ggacaccttg 1020
aaaagcataa agttattaat taacaagaat ttagaaaatg ctgcagaatt atattatata 1080
ttcagaattt ttggacaccc tatggtagat gagagggaag caatggatgc tgttaaatta 1140
aacaatgaga ttacaaaaat tcttaaatta gagagtttaa cagaactaag aggagcattt 1200
atactaagaa ttataaaagg gtttgtagac aataataaaa gatggcctaa aattaagaat 1260
ttaaaagtgc tcagcaaaag atgggctatg tatttcaaag ctaaaagtta ccctagccaa 1320
cttgagctaa gtgtacaaga ttttttagaa cttgctgcag tacaatttga gcaggaattc 1380
tctgtacctg aaaaaaccaa ccttgagatg gtattaaatg ataaagcaat atcacctcca 1440
aaaaagctaa tatggtctgt atatccaaaa aactacctgc ctgaaactat aaaaaatcaa 1500
tatttagaag aggctttcaa tgcaagtgac agccaaagaa caaggagagt cttagaattt 1560
tacttaaaag attgtaaatt tgatcaaaaa gaacttaaac gttatgtaat taaacaagag 1620
tatctgaatg acaaagacca cattgtctcg ttaactggga aggaaagaga attaagtgta 1680
ggtaggatgt ttgcaatgca accaggaaaa caaagacaga tacagatatt agctgagaaa 1740
cttctagctg ataatattgt accttttttc ccagaaactt taacaaagta tggtgactta 1800
gatctccaaa gaattatgga aataaaatca gaactttctt ccattaaaac tagaaagaat 1860
gatagctaca acaattatat tgcaaggccc tctatagtaa cagacttaag taagttcaat 1920
caggccttta gatatgaaac cacagctata tgtgcagatg tagctgatga gttacatggg 1980
acacaaagct tattctgttg gttacatctt attgttccca tgactacaat gatatgtgca 2040
tacagacatg caccaccaga aacaaaaggg gaatatgata tagacaaaat acaagagcaa 2100
agcggattat acagatatca tatgggaggg attgaagggt ggtgccagaa gttatggaca 2160
atggaagcaa tatccttgtt agatgtagta tctgtgaaga ctcgctgtca gatgacctct 2220
ctattaaacg gagacaatca gtcaatagat gttagtaaac cagtaaaatt gtctgaaggt 2280
atagatgaag taaaagcaga ctatagctta gcaattagaa tgcttaaaga aataagagat 2340
gcttataaaa acattggtca taaactcaaa gaaggtgaaa catatatatc aagggatctc 2400
caatttataa gtaaggtgat tcaatctgaa ggagtcatgc atcctacccc tataaaaaag 2460
atattaagag taggtccttg gataaataca atactagatg atattaaaac cagtgcagaa 2520

tcaataggaa gtctatgtca agaactagaa ttcagagggg agagtatact agttagcttg 2580
atattaagga atttctggct gtataacttg tacatgtatg agtcaaaaca gcacccatta 2640
gctgggaagc aactgttcaa gcaattgaac aaaacattaa catctgtgca gagatttttt 2700
gaactgaaga aagaaaatga tgtggttgac ctatggatga atataccaat gcagtttgga 2760
gggggagatc cagtagtttt ttacagatct ttttacagaa ggactcccga tttcctaact 2820
gaagcaatca gccatgtgga tttactgtta aaagtgtcaa acaatatcaa agatgagact 2880
aagatacgat ttttcaaagc cttattatct atagaaaaga atgaacgtgc tacattaaca 2940
acactaatga gagaccctca ggcagtagga tcagaacgac aagctaaggt aacaagtgat 3000
ataaatagaa cagcagttac cagcatactg agtctatctc cgaatcagct cttctgtgat 3060
agtgctatac attatagtag aaatgaggaa gaagttggga tcattgcaga caacataaca 3120
cctgtctatc ctcatgggct gagagtgctc tatgaatcac taccttttca taaggctgaa 3180
aaggttgtca atatgatatc aggcacaaag tctataacta atctattaca gagaacatct 3240
gctatcaatg gtgaagatat tgatagagca gtgtctatga tgttagagaa cttagggttg 3300
ttatctagaa tattgtcagt aataattaat agtatagaaa taccaatcaa gtccaatggc 3360
agattgatat gctgtcaaat ttccaagacc ttgagagaaa aatcatggaa caatatggaa 3420
atagtaggag tgacatctcc tagtattgtg acatgtatgg atgttgtgta tgcaactagt 3480
tctcatttaa aaggaataat tattgaaaaa ttcagtactg acaagaccac aagaggtcag 3540
aggggaccaa aaagcccctg ggtaggatca agcactcaag agaaaaaatt ggttcctgtt 3600
tataatagac aaattctttc aaaacaacaa aaagagcaac tggaagcaat agggaaaatg 3660
aggtgggtgt acaaaggaac tccagggcta agaagattgc tcaacaagat ttgcatagga 3720
agcttaggta ttagctataa atgtgtgaaa cctttattac caagattcat gagtgtaaac 3780
ttcttacata ggttatctgt tagtagtaga cccatggaat tcccagcttc tgttccagct 3840
tacaggacaa caaattacca ttttgacact agtccaatca accaagcatt aagtgagagg 3900
ttcgggaacg aagacattaa tttagtgttc caaaatgcaa tcagctgcgg aattagtata 3960
atgagtgttg tagaacagtt aactggtaga agcccaaaac aattagtcct aatccctcaa 4020
ttagaagaga tagatattat gcctcctcct gtatttcaag gaaaattcaa ttataaacta 4080
gttgataaga taacctccga tcaacacatc ttcagtcctg acaaaataga catattaaca 4140
ctagggaaga tgcttatgcc taccataaaa ggtcaaaaaa ctgatcagtt cttaaataag 4200
agagaaaact attttcatgg aaataattta attgaatctt tatctgcagc acttgcatgc 4260
cactggtgtg ggatattaac agaacagtgc atagaaaaca atatctttag gaaagattgg 4320
ggtgatgggt tcatctcaga tcatgccttc atggatttca aggtatttct atgtgtattt 4380
aaaaccaaac ttttatgtag ttggggatct caaggaaaga atgtaaaaga tgaagatata 4440
atagatgaat ccattgacaa attattaaga attgacaaca ccttttggag aatgttcagc 4500
aaagtcatgt ttgaatcaaa agtcaaaaaa agaataatgt tatatgatgt gaaattccta 4560
tcattagtag gttatatagg atttaaaaac tggtttatag aacagttaag agtggtagaa 4620
ttgcatgagg taccttggat tgtcaatgct gaaggagagt tagttgaaat taaatcaatc 4680
aaaatttatc tgcagttaat agaacaaagt ctatctttga gaataactgt attgaattat 4740
acagacatgg cacatgctct tacacgatta attaggaaaa aattgatgtg tgataatgca 4800
ctctttaatc caagttcatc accaatgttt aatctaactc aggttattga tcccacaaca 4860
caactagact attttcctag gataatattt gagaggttaa aaagttatga taccagttca 4920
gactacaaca aagggaagtt aacaaggaat tacatgacat tattaccatg gcaacacgta 4980
aacaggtaca attttgtctt tagttctaca ggttgtaaag tcagtttgaa gacatgcatc 5040
gggaaattga taaaggattt aaatcctaaa gttctttact ttattggaga aggagcaggt 5100
aactggatgg caagaacagc atgtgaatat cctgatataa aatttgtata taggagttta 5160
aaggatgacc ttgatcacca ttacccatta gaatatcaaa gggtaatagg tgatctaaat 5220
agggtgatag atagtggtga aggattatca atggaaacca cagatgcaac tcaaaaaact 5280
cattgggact tgatacacag aataagtaaa gatgctttat tgataacatt gtgtgatgca 5340
gaattcaaaa acagagatga tttctttaag atggtaatcc tttggagaaa acatgtatta 5400
tcttgtagaa tctgtacagc ttatggaaca gatctttact tatttgcaaa gtatcatgcg 5460
gtggactgca atataaaatt accatttttt gtaagatctg tagctacttt tattatgcaa 5520
ggaagcaaat tatcagggtc agaatgttac atacttttaa cattaggtca tcacaataat 5580
ctaccctgtc atggagaaat acaaaattcc aaaatgagaa tagcagtgtg taatgatttc 5640
tatgcctcaa agaaactgga caacaaatca attgaagcaa actgcaaatc tcttctatca 5700
ggattgagaa tacctataaa caaaaaggag ttaaatagac aaaagaaatt gttaacacta 5760
caaagtaacc attcttctat agcaacagtt ggcggcagta agattataga atccaaatgg 5820
ttaaagaata aagcaagtac aataattgat tggttagagc atattttgaa ttctccaaaa 5880
ggtgaattaa actatgattt ctttgaagca ttagagaaca cataccccaa tatgatcaag 5940
cttatagata atttgggaaa tgcagaaata aagaaactaa tcaaggtcac tgggtatatg 6000
cttgtgagta agaagtaa 6018

<210> 41
<211> 6018
<212> DNA
<213> human Metapneumo virus

<400> 41
atggatccat tttgtgaatc cactgtcaat gtttatcttc ctgactcata tctcaaagga 60
gtaatatctt tcagtgaaac caatgcaatt ggctcatgcc ttttgaaaag accctatcta 120
aaaaaagata acactgctaa agttgctgta gaaaaccctg ttgttgaaca tgtcaggctt 180
agaaatgcag tcatgaccaa aatgaagata tcagattata aagtggttga accaattaat 240
atgcagcatg aaataatgaa aaatatacac agttgtgagc tcacattatt aaaacaattc 300
ttaacaagaa gtaaaaacat tagctctcta aaattaagta tgatatgtga ttggttacag 360
ttaaaatcca cctcagataa cacatcaatt cttaatttta tagatgtgga gtttatacct 420
gtttgggtga gcaattggtt tagtaactgg tataatctca ataaattaat cttagagttt 480
agaagagagg aagtaataag aactggttca attttatgta gatcactagg caagttagtt 540
ttcattgtat catcttatgg gtgtgtagta aaaagcaaca aaagtaaaag agtaagtttt 600
ttcacatata accaactgtt aacatggaaa gatgtgatgt taagtaggtt caatgcaaac 660
ttttgtatat gggtaagtaa caacctgaac aaaaatcaag aaggactagg atttagaagt 720
aatctgcaag gtatgttaac caataaatta tatgaaactg ttgattatat gttaagtcta 780
tgtagtaatg aagggttctc actagtgaaa gagttcgaag gctttattat gagtgaaatt 840
cttaaaatta ctgagcatgc tcaattcagt actaggttta ggaatacttt attaaatggg 900
ttgactgaac aattatcaat gttgaaagct aaaaacagat ctagagttct tggcactata 960
ttagaaaaca atgattaccc catgtatgaa gtagtactta aattattagg ggacactttg 1020
aaaagtataa aattattaat taacaagaat ttagaaaatg ctgcagaatt atattatata 1080
ttcagaattt ttggacaccc tatggtagat gagagggaag caatggatgc tgttaaatta 1140
aataatgaga ttacaaaaat tcttaaactg gagagcttaa cagaactaag aggagcattt 1200
atactaagaa ttataaaagg gtttgtagat aataataaaa gatggcctaa aattaagaat 1260
ttaaaagtgc tcagtaaaag atgggttatg tatttcaaag ccaaaagtta ccctagccaa 1320
cttgagctaa gtgtacaaga ttttttagaa cttgctgcag tacaattcga acaggaattt 1380
tctgtccctg aaaaaaccaa ccttgagatg gtattaaatg ataaagcaat atctcctcca 1440
aaaaagttaa tatggtcggt atatccaaaa aattatctac ctgaaattat aaaaaatcaa 1500
tatttagaag aggtcttcaa tgcaagtgac agtcaaagaa cgaggagagt cttagaattt 1560
tacttaaaag attgcaaatt tgatcaaaaa gaccttaaac gttatgtact taaacaagag 1620
tatctaaatg acaaagacca cattgtctca ttaactggga aggaaagaga attaagtgta 1680
ggcaggatgt ttgcaatgca accaggcaaa caaagacaaa tacagatact agctgagaaa 1740
cttctagctg ataatattgt accctttttc ccagaaactt taacaaagta tggtgacttg 1800
gatctccaaa gaattatgga aatgaaatca gaactttctt ccattaaaac taggaagaat 1860
gatagttaca acaattatat tgcaagagcc tccatagtaa cagacctaag taaattcaat 1920
caagccttta gatatgaaac cacagctatc tgtgcagatg tagcagatga gttacatggt 1980
acgcaaagct tattttgttg gttacatctt attgttccca tgaccacaat gatatgtgca 2040
tacagacatg caccaccaga aacaaagggg gagtatgaca tagacaaaat agaagagcaa 2100
agtgggctat acagatatca tatgggaggg attgaagggt ggtgtcagaa gttatggaca 2160
atggaagcga tatccttgtt agatgtagta tctgttaaga ctcgttgtca gatgacctct 2220
ctattaaacg gagacaatca atcaatagat gtcagtaaac cagtaaaatt gtctgaaggt 2280
atagatgaag taaaagcaga ttatagctta gcaattaaaa tgcttaaaga gataagagat 2340
gcctataaaa acattggcca taaactcaaa gaaggtgaaa catatatatc aagagatctc 2400
caatttataa gtaaggtgat tcaatctgag ggggtcatgc atcctacccc cataaaaaag 2460
atattaaggg taggtccctg gataaataca atactagatg acattaaaac cagtgcagaa 2520
tcaataggga gtctgtgtca agaactagag ttcagaggag aaagtatgct agttagcttg 2580
atattaagga atttctggct gtataactta tacatgcatg agtcaaaaca gcatccgtta 2640
gctggaaaac aactgtttaa gcaattgaac aaaacactaa catctgtgca aagatttttt 2700
gagctgaaga aagaaaatga tgtggttgac ctatggatga atataccaat gcagtttgga 2760
gggggagacc cagtagtttt ttacagatct ttttacagaa ggactcctga tttcctgact 2820
gaagcaatca gccatgtgga tttactgtta aaagtttcga acaatattaa aaatgagact 2880
aagatacgat tctttaaagc cttattatct atagaaaaga atgaacgtgc aacattaaca 2940
acactaatga gagaccccca ggcggtagga tcggaaagac aagctaaggt aacaagtgat 3000
ataaatagaa cagcagttac tagcatactg agtctatctc cgaatcagct cttttgtgat 3060
agtgctatac actatagcag aaatgaagaa gaagtcggga tcattgcaga caacataaca 3120
cctgtttatc ctcacggatt gagagtgctc tatgaatcac taccttttca taaggctgaa 3180
aaggttgtca atatgatatc aggtacaaag tctataacta acctattgca gagaacatct 3240
gctatcaatg gtgaagatat tgatagagca gtgtctatga tgttagagaa cttagggttg 3300
ttatctagga tattgtcagt aataattaat agtatagaaa taccaattaa gtccaatggc 3360

agattgatat gctgtcaaat ttctaagact ttgagagaaa aatcatggaa caatatggaa 3420
atagtaggag tgacatctcc aagtattgta acatgtatgg atgttgtgta tgcaactagt 3480
tctcatttaa aaggaataat tattgaaaaa ttcagtactg acaagaccac aagaggtcag 3540
aggggaccaa aaagcccctg ggtaggatca agcactcaag agaaaaaatt agttcctgtt 3600
tataatagac aaattctttc aaaacaacaa aaggagcaac tggaagcaat aggaaaaatg 3660
aggtgggtgt ataaaggaac tccagggcta agaagattgc tcaataagat ttgcatagga 3720
agtttaggta ttagctataa atgtgtaaaa cctctattac caagattcat gagtgtaaac 3780
ttcttacata ggttatctgt tagtagcaga cccatggaat tcccagcttc tgttccagct 3840
tataggacaa caaattacca ctttgacact agtccaatca accaagcatt aagtgagagg 3900
ttcgggaacg aagacattaa tctagtgttc caaaatgcaa tcagctgcgg aattagtata 3960
atgagtgttg tagaacagtt aactggtaga agcccaaaac aattagtctt aatcccccaa 4020
ttagaagaga tagatattat gcctcctcct gtatttcaag gaaaattcaa ttataaacta 4080
gttgataaaa taacctctga tcaacacatc ttcagtcctg acaaaataga catattaaca 4140
ctagggaaga tgcttatgcc tactataaaa ggtcaaaaaa ctgatcagtt cttaaataag 4200
agagaaaact atttccatgg aaataattta attgaatctt tatctgcagc acttgcatgc 4260
cactggtgtg gaatattaac agaacagtgt gtagaaaaca atatctttag gaaagactgg 4320
ggtgatgggt tcatatcaga tcatgccttc atggatttca agatatttct atgtgtattt 4380
aaaaccaaac ttttatgtag ttggggatcc caagggaaaa atgtaaaaga tgaagatata 4440
atagatgaat ccattgacaa attattaaga attgacaaca ctttttggag aatgttcagc 4500
aaagtcatgt ttgaatcaaa ggtcaaaaaa agaataatgt tatatgatgt aaaattccta 4560
tcattagtag gttatatagg atttaaaaac tggtttatag agcagttaag agtagtagaa 4620
ttgcatgaag tgccctggat tgtcaatgct gaaggggagc tagttgaaat taaaccaatc 4680
aaaatttatt tgcagttaat agaacaaagt ctatctttaa gaataactgt tttgaattat 4740
acagacatgg cacatgctct tacacgatta attaggaaga aattgatgtg tgataatgca 4800
ctcttcaatc caagttcatc accaatgttt agtctaactc aagttatcga tcctacaaca 4860
cagctagact attttcctaa ggtgatattt gaaaggttaa aaagttatga taccagttca 4920
gactacaaca aagggaagtt aacaagaaat tacatgacat tattaccatg gcagcacgta 4980
aacaggtata attttgtctt tagttcaaca ggatgtaaaa tcagcttgaa gacatgcatc 5040
gggaaattga taaaggactt aaaccctaag gttctttact ttattggaga aggagcaggt 5100
aactggatgg caagaacagc atgtgagtat cctgacataa aatttgtata taggagttta 5160
aaggatgatc ttgatcatca ttacccatta gaatatcaaa gggtaatagg tgatttaaat 5220
agggtaatag atggtggtga aggactatca atggagacca cagatgcaac tcaaaagact 5280
cattgggact taatacacag aataagtaaa gatgctttat tgataacatt gtgtgatgca 5340
gaattcaaaa acagagatga tttctttaaa atggtaattc tttggagaaa acatgtatta 5400
tcatgtagaa tctgtacagc ttatggaaca gatctttact tatttgcaaa gtatcatgcg 5460
acggactgca atataaaatt accatttttt gtaaggtctg tagctacttt tattatgcaa 5520
ggaagcaaat tgtcaggatc agaatgttac atacttttaa cattaggtca tcacaataat 5580
ctgccatgcc atggagaaat acaaaattcc aaaatgagaa tagcagtgtg taatgatttc 5640
catgcctcaa aaaaactaga caacaaatca attgaagcta actgtaaatc tcttctatca 5700
ggattaagaa taccaataaa caaaaaagag ttaaatagac aaaagaaact gttaacacta 5760
caaagcaatc attcttccat agcaacagtt ggcggcagta agattataga atccaaatgg 5820
ttaaagaata aagcaagtac aataattgat tggttagagc atatcttgaa ttctccaaaa 5880
ggtgaattaa actatgattt ctttgaagca ttagagaaca cataccccaa tatgatcaag 5940
cttatagata acctgggaaa tgcagagata aaaaaactaa tcaaagttcc tgggtatatg 6000
cttgtgagta agaagtaa 6018

<210> 42
<211> 187
<212> PRT
<213> human Metapneumo virus

<400> 42
Met Ser Arg Lys Ala Pro Cys Lys Tyr Glu Val Arg Gly Lys Cys Asn
1               5                   10                  15
Arg Gly Ser Glu Cys Lys Phe Asn His Asn Tyr Trp Ser Trp Pro Asp
            20                  25                  30
Arg Tyr Leu Leu Ile Arg Ser Asn Tyr Leu Leu Asn Gln Leu Leu Arg
        35                  40                  45
Asn Thr Asp Arg Ala Asp Gly Leu Ser Ile Ile Ser Gly Ala Gly Arg
    50                  55                  60

Glu Asp Arg Thr Gln Asp Phe Val Leu Gly Ser Thr Asn Val Val Gln
65                  70                  75                  80
Gly Tyr Ile Asp Asp Asn Gln Ser Ile Thr Lys Ala Ala Ala Cys Tyr
                85                  90                  95
Ser Leu His Asn Ile Ile Lys Gln Leu Gln Glu Val Glu Val Arg Gln
            100                 105                 110
Ala Arg Asp Asn Lys Leu Ser Asp Ser Lys His Val Ala Leu His Asn
        115                 120                 125
Leu Val Leu Ser Tyr Met Glu Met Ser Lys Thr Pro Ala Ser Leu Ile
    130                 135                 140
Asn Asn Leu Lys Arg Leu Pro Arg Glu Lys Leu Lys Lys Leu Ala Lys
145                 150                 155                 160
Leu Ile Ile Asp Leu Ser Ala Gly Ala Glu Asn Asp Ser Ser Tyr Ala
                165                 170                 175
Leu Gln Asp Ser Glu Ser Thr Asn Gln Val Gln
            180                 185

<210> 43
<211> 187
<212> PRT
<213> human Metapneumo virus

<400> 43
Met Ser Arg Lys Ala Pro Cys Lys Tyr Glu Val Arg Gly Lys Cys Asn
 1               5                  10                  15
Arg Gly Ser Glu Cys Lys Phe Asn His Asn Tyr Trp Ser Trp Pro Asp
            20                  25                  30
Arg Tyr Leu Leu Ile Arg Ser Asn Tyr Leu Leu Asn Gln Leu Leu Arg
        35                  40                  45
Asn Thr Asp Arg Ala Asp Gly Leu Ser Ile Ile Ser Gly Ala Gly Arg
    50                  55                  60
Glu Asp Arg Thr Gln Asp Phe Val Leu Gly Ser Thr Asn Val Val Gln
65                  70                  75                  80
Gly Tyr Ile Asp Asp Asn Gln Ser Ile Thr Lys Ala Ala Ala Cys Tyr
                85                  90                  95
Ser Leu His Asn Ile Ile Lys Gln Leu Gln Glu Val Glu Val Arg Gln
            100                 105                 110
Ala Arg Asp Ser Lys Leu Ser Asp Ser Lys His Val Ala Leu His Asn
        115                 120                 125
Leu Ile Leu Ser Tyr Met Glu Met Ser Lys Thr Pro Ala Ser Leu Ile
    130                 135                 140
Asn Asn Leu Lys Arg Leu Pro Arg Glu Lys Leu Lys Lys Leu Ala Lys
145                 150                 155                 160
Leu Ile Ile Asp Leu Ser Ala Gly Ala Asp Asn Asp Ser Ser Tyr Ala
                165                 170                 175
Leu Gln Asp Ser Glu Ser Thr Asn Gln Val Gln
            180                 185

<210> 44
<211> 187
<212> PRT
<213> human Metapneumo virus

<400> 44
Met Ser Arg Lys Ala Pro Cys Lys Tyr Glu Val Arg Gly Lys Cys Asn
 1               5                  10                  15
Arg Gly Ser Asp Cys Lys Phe Asn His Asn Tyr Trp Ser Trp Pro Asp
            20                  25                  30
Arg Tyr Leu Leu Leu Arg Ser Asn Tyr Leu Leu Asn Gln Leu Leu Arg
        35                  40                  45

Asn Thr Asp Lys Ala Asp Gly Leu Ser Ile Ile Ser Gly Ala Gly Arg
    50                  55                  60
Glu Asp Arg Thr Gln Asp Phe Val Leu Gly Ser Thr Asn Val Val Gln
65                  70                  75                  80

Gly Tyr Ile Asp Asp Asn Gln Gly Ile Thr Lys Ala Ala Ala Cys Tyr
                85                  90                  95
Ser Leu His Asn Ile Ile Lys Gln Leu Gln Glu Thr Glu Val Arg Gln
            100                 105                 110
Ala Arg Asp Asn Lys Leu Ser Asp Ser Lys His Val Ala Leu His Asn
        115                 120                 125
Leu Ile Leu Ser Tyr Met Glu Met Ser Lys Thr Pro Ala Ser Leu Ile
    130                 135                 140
Asn Asn Leu Lys Lys Leu Pro Arg Glu Lys Leu Lys Lys Leu Ala Arg
145                 150                 155                 160
Leu Ile Ile Asp Leu Ser Ala Gly Thr Asp Asn Asp Ser Ser Tyr Ala
                165                 170                 175
Leu Gln Asp Ser Glu Ser Thr Asn Gln Val Gln
            180                 185

<210> 45
<211> 187
<212> PRT
<213> human Metapneumo virus

<400> 45
Met Ser Arg Lys Ala Pro Cys Lys Tyr Glu Val Arg Gly Lys Cys Asn
 1               5                  10                  15
Arg Gly Ser Glu Cys Lys Phe Asn His Asn Tyr Trp Ser Trp Pro Asp
            20                  25                  30
Arg Tyr Leu Leu Leu Arg Ser Asn Tyr Leu Leu Asn Gln Leu Leu Arg
        35                  40                  45
Asn Thr Asp Lys Ala Asp Gly Leu Ser Ile Ile Ser Gly Ala Gly Arg
    50                  55                  60
Glu Asp Arg Thr Gln Asp Phe Val Leu Gly Ser Thr Asn Val Val Gln
65                  70                  75                  80
Gly Tyr Ile Asp Asn Asn Gln Gly Ile Thr Lys Ala Ala Ala Cys Tyr
                85                  90                  95
Ser Leu His Asn Ile Ile Lys Gln Leu Gln Glu Ile Glu Val Arg Gln
            100                 105                 110
Ala Arg Asp Asn Lys Leu Ser Asp Ser Lys His Val Ala Leu His Asn
        115                 120                 125
Leu Ile Leu Ser Tyr Met Glu Met Ser Lys Thr Pro Ala Ser Leu Ile
    130                 135                 140
Asn Asn Leu Lys Lys Leu Pro Arg Glu Lys Leu Lys Lys Leu Ala Lys
145                 150                 155                 160
Leu Ile Ile Asp Leu Ser Ala Gly Thr Asp Asn Asp Ser Ser Tyr Ala
                165                 170                 175
Leu Gln Asp Ser Glu Ser Thr Asn Gln Val Gln
            180                 185

<210> 46
<211> 564
<212> DNA
<213> human Metapneumo virus

<400> 46
atgtctcgca aggctccgtg caaatatgaa gtgcggggca aatgcaatag aggaagtgag 60
tgcaagttta accacaatta ctggagttgg ccagatagat acttattaat aagatcaaat 120
tatttattaa atcaactttt aaggaacact gatagagctg atggcttatc aataatatca 180

ggagcaggca gagaagatag gacacaagat tttgtcctag gttccaccaa tgtggttcaa 240
ggttatattg atgataacca aagcataaca aaagctgcag cctgttacag tctacataat 300
ataatcaaac aactacaaga agttgaagtt aggcaggcta gagataacaa actatctgac 360
agcaaacatg tagcacttca caacttagtc ctatcttata tggagatgag caaaactcct 420
gcatctttaa tcaacaatct caagagactg ccgagagaga aactgaaaaa attagcaaag 480
ctcataattg acttatcagc aggtgctgaa aatgactctt catatgcctt gcaagacagt 540
gaaagcacta atcaagtgca gtga 564

<210> 47
<211> 564
<212> DNA
<213> human Metapneumo virus

<400> 47
atgtctcgca aggctccatg caaatatgaa gtgcggggca aatgcaacag aggaagtgag 60
tgtaagttta accacaatta ctggagttgg ccagatagat acttattaat aagatcaaac 120
tatctattaa atcagctttt aaggaacact gatagagctg atggcctatc aataatatca 180
ggcgcaggca gagaagacag aacgcaagat tttgttctag gttccaccaa tgtggttcaa 240
ggttatattg atgataacca aagcataaca aaagctgcag cctgctacag tctacacaac 300
ataatcaagc aactacaaga agttgaagtt aggcaggcta gagatagcaa actatctgac 360
agcaagcatg tggcactcca taacttaatc ttatcttaca tggagatgag caaaactccc 420
gcatctttaa tcaacaatct taaaagactg ccgagagaaa aactgaaaaa attagcaaag 480
ctgataattg acttatcagc aggcgctgac aatgactctt catatgccct gcaagacagt 540
gaaagcacta atcaagtgca gtga 564

<210> 48
<211> 564
<212> DNA
<213> human Metapneumo virus

<400> 48
atgtctcgta aggctccatg caaatatgaa gtgcggggca aatgcaacag agggagtgat 60
tgcaaattca atcacaatta ctggagttgg cctgatagat atttattgtt aagatcaaat 120
tatctcttaa atcagctttt aagaaacaca gataaggctg atggtttgtc aataatatca 180
ggagcaggta gagaagatag aactcaagac tttgttcttg gttctactaa tgtggttcaa 240
gggtacattg atgacaacca aggaataacc aaggctgcag cttgctatag tctacacaac 300
ataatcaagc aactacaaga aacagaagta agacaggcta gagacaacaa gctttctgat 360
agcaaacatg tggcgctcca caacttgata ttatcctata tggagatgag caaaactcct 420
gcatctctaa tcaacaacct aaagaaacta ccaaggaaaa aactgaagaa attagcaaga 480
ttaataattg atttatcagc aggaactgac aatgactctt catatgcctt gcaagacagt 540
gaaagcacta atcaagtgca gtaa 564

<210> 49
<211> 564
<212> DNA
<213> human Metapneumo virus

<400> 49
atgtctcgca aagctccatg caaatatgaa gtacggggca agtgcaacag gggaagtgag 60
tgcaaattca accacaatta ctggagctgg cctgataggt atttattgtt aagatcaaat 120
tatctcttga atcagctttt aagaaacact gataaggctg atggtttgtc aataatatca 180
ggagcaggta gagaagatag gactcaagac tttgttcttg gttctactaa tgtggttcaa 240
gggtacattg ataacaatca aggaataaca aaggctgcag cttgctatag tctacataac 300
ataataaaac agctacaaga aatagaagta agacaggcta gagataataa gctttctgac 360
agcaaacatg tggcacttca caacttgata ttatcctata tggagatgag caaaactcct 420
gcatccctga ttaataacct aaagaaacta ccaagagaaa aactgaagaa attagcgaaa 480
ttaataattg atttatcagc aggaactgat aatgactctt catatgcctt gcaagacagt 540
gaaagcacta atcaagtgca gtaa 564

<210> 50
<211> 71
<212> PRT

<213> human Metapneumo virus

<400> 50
Met Thr Leu His Met Pro Cys Lys Thr Val Lys Ala Leu Ile Lys Cys
1               5                   10                  15
Ser Glu His Gly Pro Val Phe Ile Thr Ile Glu Val Asp Asp Met Ile
            20                  25                  30
Trp Thr His Lys Asp Leu Lys Glu Ala Leu Ser Asp Gly Ile Val Lys
        35                  40                  45
Ser His Thr Asn Ile Tyr Asn Cys Tyr Leu Glu Asn Ile Glu Ile Ile
    50                  55                  60
Tyr Val Lys Ala Tyr Leu Ser
65                  70

<210> 51
<211> 71
<212> PRT
<213> human Metapneumo virus

<400> 51
Met Thr Leu His Met Pro Cys Lys Thr Val Lys Ala Leu Ile Lys Cys
1               5                   10                  15
Ser Glu His Gly Pro Val Phe Ile Thr Ile Glu Val Asp Glu Met Ile
            20                  25                  30
Trp Thr Gln Lys Glu Leu Lys Glu Ala Leu Ser Asp Gly Ile Val Lys
        35                  40                  45
Ser His Thr Asn Ile Tyr Asn Cys Tyr Leu Glu Asn Ile Glu Ile Ile
    50                  55                  60
Tyr Val Lys Ala Tyr Leu Ser
65                  70

<210> 52
<211> 71
<212> PRT
<213> human Metapneumo virus

<400> 52
Met Thr Leu His Met Pro Cys Lys Thr Val Lys Ala Leu Ile Lys Cys
1               5                   10                  15
Ser Lys His Gly Pro Lys Phe Ile Thr Ile Glu Ala Asp Asp Met Ile
            20                  25                  30
Trp Thr His Lys Glu Leu Lys Glu Thr Leu Ser Asp Gly Ile Val Lys
        35                  40                  45
Ser His Thr Asn Ile Tyr Ser Cys Tyr Leu Glu Asn Ile Glu Ile Ile
    50                  55                  60
Tyr Val Lys Thr Tyr Leu Ser
65                  70

<210> 53
<211> 71
<212> PRT
<213> human Metapneumo virus

<400> 53
Met Thr Leu His Met Pro Cys Lys Thr Val Lys Ala Leu Ile Lys Cys
1               5                   10                  15
Ser Lys His Gly Pro Lys Phe Ile Thr Ile Glu Ala Asp Asp Met Ile
            20                  25                  30
Trp Thr His Lys Glu Leu Lys Glu Thr Leu Ser Asp Gly Ile Val Lys

        35                  40                  45
Ser His Thr Asn Ile Tyr Ser Cys Tyr Leu Glu Asn Ile Glu Ile Ile
    50                  55                  60
Tyr Val Lys Ala Tyr Leu Ser
65                  70

<210> 54
<211> 216
<212> DNA
<213> human Metapneumo virus

<400> 54
atgactcttc atatgccttg caagacagtg aaagcactaa tcaagtgcag tgagcatggt 60
ccagttttca ttactataga ggttgatgac atgatatgga ctcacaagga cttaaaagaa 120
gctttatctg atgggatagt gaagtctcat actaacattt acaattgtta tttagaaaac 180
atagaaatta tatatgtcaa ggcttactta agttag 216

<210> 55
<211> 216
<212> DNA
<213> human Metapneumo virus

<400> 55
atgactcttc atatgccctg caagacagtg aaagcactaa tcaagtgcag tgagcatggt 60
cctgttttca ttactataga ggttgatgaa atgatatgga ctcaaaaaga attaaaagaa 120
gctttgtccg atgggatagt gaagtctcac accaacattt acaattgtta tttagaaaac 180
atagaaatta tatatgtcaa ggcttactta agttag 216

<210> 56
<211> 216
<212> DNA
<213> human Metapneumo virus

<400> 56
atgactcttc atatgccttg caagacagtg aaagcactaa tcaagtgcag taaacatggt 60
cccaaattca ttaccataga ggcagatgat atgatatgga ctcacaaaga attaaaagaa 120
acactgtctg atgggatagt aaaatcacac accaatattt atagttgtta cttagaaaat 180
atagaaataa tatatgttaa aacttactta agttag 216

<210> 57
<211> 216
<212> DNA
<213> human Metapneumo virus

<400> 57
atgactcttc atatgccttg caagacagtg aaagcactaa tcaagtgcag taagcatggt 60
cccaaattca ttaccataga ggcagatgat atgatatgga cacacaaaga attaaaggag 120
acactgtctg atgggatagt aaaatcacac accaatattt acagttgtta tttagaaaat 180
atagaaataa tatatgttaa agcttactta agttag 216

<210> 58
<211> 727
<212> DNA
<213> human Metapneumo virus

<400> 58
atgtctcgca aggctccgtg caaatatgaa gtgcggggca aatgcaatag aggaagtgag 60
tgcaagttta accacaatta ctggagttgg ccagatagat acttattaat aagatcaaat 120
tatttattaa atcaactttt aaggaacact gatagagctg atggcttatc aataatatca 180
ggagcaggca gagaagatag gacacaagat tttgtcctag gttccaccaa tgtggttcaa 240
ggttatattg atgataacca aagcataaca aaagctgcag cctgttacag tctacataat 300

ataatcaaac aactacaaga agttgaagtt aggcaggcta gagataacaa actatctgac 360
agcaaacatg tagcacttca caacttagtc ctatcttata tggagatgag caaaactcct 420
gcatctttaa tcaacaatct caagagactg ccgagagaga aactgaaaaa attagcaaag 480
ctcataattg acttatcagc aggtgctgaa aatgactctt catatgcctt gcaagacagt 540
gaaagcacta atcaagtgca gtgagcatgg tccagttttc attactatag aggttgatga 600
catgatatgg actcacaagg acttaaaaga agctttatct gatgggatag tgaagtctca 660
tactaacatt tacaattgtt atttagaaaa catagaaatt atatatgtca aggcttactt 720
aagttag 727

<210> 59
<211> 727
<212> DNA
<213> human Metapneumo virus

<400> 59
atgtctcgca aggctccatg caaatatgaa gtgcggggca aatgcaacag aggaagtgag 60
tgtaagttta accacaatta ctggagttgg ccagatagat acttattaat aagatcaaac 120
tatctattaa atcagctttt aaggaacact gatagagctg atggcctatc aataatatca 180
ggcgcaggca gagaagacag aacgcaagat tttgttctag gttccaccaa tgtggttcaa 240
ggttatattg atgataacca aagcataaca aaagctgcag cctgctacag tctacacaac 300
ataatcaagc aactacaaga agttgaagtt aggcaggcta gagatagcaa actatctgac 360
agcaagcatg tggcactcca taacttaatc ttatcttaca tggagatgag caaaactccc 420
gcatctttaa tcaacaatct taaaagactg ccgagagaaa aactgaaaaa attagcaaag 480
ctgataattg acttatcagc aggcgctgac aatgactctt catatgccct gcaagacagt 540
gaaagcacta atcaagtgca gtgagcatgg tcctgttttc attactatag aggttgatga 600
aatgatatgg actcaaaaag aattaaaaga agctttgtcc gatgggatag tgaagtctca 660
caccaacatt tacaattgtt atttagaaaa catagaaatt atatatgtca aggcttactt 720
aagttag 727

<210> 60
<211> 727
<212> DNA
<213> human Metapneumo virus

<400> 60
atgtctcgta aggctccatg caaatatgaa gtgcggggca aatgcaacag agggagtgat 60
tgcaaattca atcacaatta ctggagttgg cctgatagat atttattgtt aagatcaaat 120
tatctcttaa atcagctttt aagaaacaca gataaggctg atggtttgtc aataatatca 180
ggagcaggta gagaagatag aactcaagac tttgttcttg gttctactaa tgtggttcaa 240
gggtacattg atgacaacca aggaataacc aaggctgcag cttgctatag tctacacaac 300
ataatcaagc aactacaaga aacagaagta agacaggcta gagacaacaa gctttctgat 360
agcaaacatg tggcgctcca caacttgata ttatcctata tggagatgag caaaactcct 420
gcatctctaa tcaacaacct aaagaaacta ccaagggaaa aactgaagaa attagcaaga 480
ttaataattg atttatcagc aggaactgac aatgactctt catatgcctt gcaagacagt 540
gaaagcacta atcaagtgca gtaaacatgg tcccaaattc attaccatag aggcagatga 600
tatgatatgg actcacaaag aattaaaaga aacactgtct gatgggatag taaaatcaca 660
caccaatatt tatagttgtt acttagaaaa tatagaaata atatatgtta aaacttactt 720
aagttag 727

<210> 61
<211> 727
<212> DNA
<213> human Metapneumo virus

<400> 61
atgtctcgca aagctccatg caaatatgaa gtacggggca agtgcaacag gggaagtgag 60
tgcaaattca accacaatta ctggagctgg cctgataggt atttattgtt aagatcaaat 120
tatctcttga atcagctttt aagaaacact gataaggctg atggtttgtc aataatatca 180
ggagcaggta gagaagatag gactcaagac tttgttcttg gttctactaa tgtggttcaa 240
gggtacattg ataacaatca aggaataaca aaggctgcag cttgctatag tctacataac 300
ataataaaac agctacaaga aatagaagta agacaggcta gagataataa gctttctgac 360
agcaaacatg tggcacttca caacttgata ttatcctata tggagatgag caaaactcct 420

gcatccctga ttaataacct aaagaaacta ccaagagaaa aactgaagaa attagcgaaa 480
ttaataattg atttatcagc aggaactgat aatgactctt catatgcctt gcaagacagt 540
gaaagcacta atcaagtgca gtaagcatgg tcccaaattc attaccatag aggcagatga 600
tatgatatgg acacacaaag aattaaagga gacactgtct gatgggatag taaaatcaca 660
caccaatatt tacagttgtt atttagaaaa tatagaaata atatatgtta aagcttactt 720
aagttag 727

<210> 62
<211> 254
<212> PRT
<213> human Metapneumo virus

<400> 62
Met Glu Ser Tyr Leu Val Asp Thr Tyr Gln Gly Ile Pro Tyr Thr Ala
1               5                   10                  15
Ala Val Gln Val Asp Leu Ile Glu Lys Asp Leu Leu Pro Ala Ser Leu
            20                  25                  30
Thr Ile Trp Phe Pro Leu Phe Gln Ala Asn Thr Pro Pro Ala Val Leu
        35                  40                  45
Leu Asp Gln Leu Lys Thr Leu Thr Ile Thr Thr Leu Tyr Ala Ala Ser
    50                  55                  60
Gln Asn Gly Pro Ile Leu Lys Val Asn Ala Ser Ala Gln Gly Ala Ala
65                  70                  75                  80
Met Ser Val Leu Pro Lys Lys Phe Glu Val Asn Ala Thr Val Ala Leu
                85                  90                  95
Asp Glu Tyr Ser Lys Leu Glu Phe Asp Lys Leu Thr Val Cys Glu Val
            100                 105                 110
Lys Thr Val Tyr Leu Thr Thr Met Lys Pro Tyr Gly Met Val Ser Lys
        115                 120                 125
Phe Val Ser Ser Ala Lys Ser Val Gly Lys Lys Thr His Asp Leu Ile
    130                 135                 140
Ala Leu Cys Asp Phe Met Asp Leu Glu Lys Asn Thr Pro Val Thr Ile
145                 150                 155                 160
Pro Ala Phe Ile Lys Ser Val Ser Ile Lys Glu Ser Glu Ser Ala Thr
                165                 170                 175
Val Glu Ala Ala Ile Ser Ser Glu Ala Asp Gln Ala Leu Thr Gln Ala
            180                 185                 190
Lys Ile Ala Pro Tyr Ala Gly Leu Ile Met Ile Met Thr Met Asn Asn
        195                 200                 205
Pro Lys Gly Ile Phe Lys Lys Leu Gly Ala Gly Thr Gln Val Ile Val
    210                 215                 220
Glu Leu Gly Ala Tyr Val Gln Ala Glu Ser Ile Ser Lys Ile Cys Lys
225                 230                 235                 240
Thr Trp Ser His Gln Gly Thr Arg Tyr Val Leu Lys Ser Arg
                245                 250

<210> 63
<211> 254
<212> PRT
<213> human Metapneumo virus

<400> 63
Met Glu Ser Tyr Leu Val Asp Thr Tyr Gln Gly Ile Pro Tyr Thr Ala
1               5                   10                  15
Ala Val Gln Val Asp Leu Val Glu Lys Asp Leu Leu Pro Ala Ser Leu
            20                  25                  30
Thr Ile Trp Phe Pro Leu Phe Gln Ala Asn Thr Pro Pro Ala Val Leu
        35                  40                  45
Leu Asp Gln Leu Lys Thr Leu Thr Ile Thr Thr Leu Tyr Ala Ala Ser
    50                  55                  60
Gln Ser Gly Pro Ile Leu Lys Val Asn Ala Ser Ala Gln Gly Ala Ala

65                  70                  75                  80
Met Ser Val Leu Pro Lys Lys Phe Glu Val Asn Ala Thr Val Ala Leu
                85                  90                  95
Asp Glu Tyr Ser Lys Leu Glu Phe Asp Lys Leu Thr Val Cys Glu Val
            100                 105                 110
Lys Thr Val Tyr Leu Thr Thr Met Lys Pro Tyr Gly Met Val Ser Lys
        115                 120                 125
Phe Val Ser Ser Ala Lys Ser Val Gly Lys Lys Thr His Asp Leu Ile
    130                 135                 140
Ala Leu Cys Asp Phe Met Asp Leu Glu Lys Asn Thr Pro Val Thr Ile
145                 150                 155                 160
Pro Ala Phe Ile Lys Ser Val Ser Ile Lys Glu Ser Glu Ser Ala Thr
                165                 170                 175
Val Glu Ala Ala Ile Ser Ser Glu Ala Asp Gln Ala Leu Thr Gln Ala
            180                 185                 190
Lys Ile Ala Pro Tyr Ala Gly Leu Ile Met Ile Met Thr Met Asn Asn
        195                 200                 205
Pro Lys Gly Ile Phe Lys Lys Leu Gly Ala Gly Thr Gln Val Ile Val
    210                 215                 220
Glu Leu Gly Ala Tyr Val Gln Ala Glu Ser Ile Ser Lys Ile Cys Lys
225                 230                 235                 240
Thr Trp Ser His Gln Gly Thr Arg Tyr Val Leu Lys Ser Ser
                245                 250

<210> 64
<211> 254
<212> PRT
<213> human Metapneumo virus

<400> 64
Met Glu Ser Tyr Leu Val Asp Thr Tyr Gln Gly Ile Pro Tyr Thr Ala
 1               5                  10                  15
Ala Val Gln Val Asp Leu Val Glu Lys Asp Leu Leu Pro Ala Ser Leu
            20                  25                  30
Thr Ile Trp Phe Pro Leu Phe Gln Ala Asn Thr Pro Pro Ala Val Leu
        35                  40                  45
Leu Asp Gln Leu Lys Thr Leu Thr Ile Thr Thr Leu Tyr Ala Ala Ser
    50                  55                  60
Gln Asn Gly Pro Ile Leu Lys Val Asn Ala Ser Ala Gln Gly Ala Ala
65                  70                  75                  80
Met Ser Val Leu Pro Lys Lys Phe Glu Val Asn Ala Thr Val Ala Leu
                85                  90                  95
Asp Glu Tyr Ser Lys Leu Asp Phe Asp Lys Leu Thr Val Cys Asp Val
            100                 105                 110
Lys Thr Val Tyr Leu Thr Thr Met Lys Pro Tyr Gly Met Val Ser Lys
        115                 120                 125
Phe Val Ser Ser Ala Lys Ser Val Gly Lys Lys Thr His Asp Leu Ile
    130                 135                 140
Ala Leu Cys Asp Phe Met Asp Leu Glu Lys Asn Ile Pro Val Thr Ile
145                 150                 155                 160
Pro Ala Phe Ile Lys Ser Val Ser Ile Lys Glu Ser Glu Ser Ala Thr
                165                 170                 175
Val Glu Ala Ala Ile Ser Ser Glu Ala Asp Gln Ala Leu Thr Gln Ala
            180                 185                 190
Lys Ile Ala Pro Tyr Ala Gly Leu Ile Met Ile Met Thr Met Asn Asn
        195                 200                 205
Pro Lys Gly Ile Phe Lys Lys Leu Gly Ala Gly Thr Gln Val Ile Val
    210                 215                 220
Glu Leu Gly Ala Tyr Val Gln Ala Glu Ser Ile Ser Arg Ile Cys Lys
225                 230                 235                 240
Ser Trp Ser His Gln Gly Thr Arg Tyr Val Leu Lys Ser Arg

245                 250

<210> 65
<211> 254
<212> PRT
<213> human Metapneumo virus

<400> 65
Met Glu Ser Tyr Leu Val Asp Thr Tyr Gln Gly Ile Pro Tyr Thr Ala
1               5                   10                  15
Ala Val Gln Val Asp Leu Val Glu Lys Asp Leu Leu Pro Ala Ser Leu
            20                  25                  30
Thr Ile Trp Phe Pro Leu Phe Gln Ala Asn Thr Pro Pro Ala Val Leu
        35                  40                  45
Leu Asp Gln Leu Lys Thr Leu Thr Ile Thr Thr Leu Tyr Ala Ala Ser
    50                  55                  60
Gln Asn Gly Pro Ile Leu Lys Val Asn Ala Ser Ala Gln Gly Ala Ala
65                  70                  75                  80
Met Ser Val Leu Pro Lys Lys Phe Glu Val Asn Ala Thr Val Ala Leu
                85                  90                  95
Asp Glu Tyr Ser Lys Leu Asp Phe Asp Lys Leu Thr Val Cys Asp Val
            100                 105                 110
Lys Thr Val Tyr Leu Thr Thr Met Lys Pro Tyr Gly Met Val Ser Lys
        115                 120                 125
Phe Val Ser Ser Ala Lys Ser Val Gly Lys Lys Thr His Asp Leu Ile
    130                 135                 140
Ala Leu Cys Asp Phe Met Asp Leu Glu Lys Asn Ile Pro Val Thr Ile
145                 150                 155                 160
Pro Ala Phe Ile Lys Ser Val Ser Ile Lys Glu Ser Glu Ser Ala Thr
                165                 170                 175
Val Glu Ala Ala Ile Ser Ser Glu Ala Asp Gln Ala Leu Thr Gln Ala
            180                 185                 190
Lys Ile Ala Pro Tyr Ala Gly Leu Ile Met Ile Met Thr Met Asn Asn
        195                 200                 205
Pro Lys Gly Ile Phe Lys Lys Leu Gly Ala Gly Thr Gln Val Ile Val
    210                 215                 220
Glu Leu Gly Ala Tyr Val Gln Ala Glu Ser Ile Ser Arg Ile Cys Lys
225                 230                 235                 240
Ser Trp Ser His Gln Gly Thr Arg Tyr Val Leu Lys Ser Arg
                245                 250

<210> 66
<211> 765
<212> DNA
<213> human Metapneumo virus

<400> 66
atggagtcct acctagtaga cacctatcaa ggcattcctt acacagcagc tgttcaagtt 60
gatctaatag aaaaggacct gttacctgca agcctaacaa tatggttccc tttgtttcag 120
gccaacacac caccagcagt gctgctcgat cagctaaaaa ccctgacaat aaccactctg 180
tatgctgcat cacaaaatgg tccaatactc aaagtgaatg catcagccca aggtgcagca 240
atgtctgtac ttcccaaaaa atttgaagtc aatgcgactg tagcactcga tgaatatagc 300
aaactggaat ttgacaaact cacagtctgt gaagtaaaaa cagtttactt aacaaccatg 360
aaaccatacg ggatggtatc aaaatttgtg agctcagcca aatcagttgg caaaaaaaca 420
catgatctaa tcgcactatg tgattttatg gatctagaaa agaacacacc tgttacaata 480
ccagcattca tcaaatcagt ttcaatcaaa gagagtgagt cagctactgt tgaagctgct 540
ataagcagtg aagcagacca agctctaaca caggccaaaa ttgcacctta tgcgggatta 600
attatgatca tgactatgaa caatcccaaa ggcatattca aaaagcttgg agctgggact 660
caagtcatag tagaactagg agcatatgtc caggctgaaa gcataagcaa aatatgcaag 720
acttggagcc atcaagggac aagatatgtc ttgaagtcca gataa 765

<210> 67
<211> 765
<212> DNA
<213> human Metapneumo virus

<400> 67
atggagtcct atctggtaga cacttatcaa ggcatccctt acacagcagc tgttcaagtt 60
gatctagtag aaaaggacct gttacctgca agcctaacaa tatggttccc cttgtttcag 120
gccaatacac caccagcagt tctgcttgat cagctaaaga ctctgactat aactactctg 180
tatgctgcat cacaaagtgg tccaatacta aaagtgaatg catcagccca gggtgcagca 240
atgtctgtac ttcccaaaaa gtttgaagtc aatgcgactg tagcacttga cgaatatagc 300
aaattagaat ttgacaaact tacagtctgt gaagtaaaaa cagtttactt aacaaccatg 360
aaaccatatg ggatggtatc aaagtttgtg agctcggcca aatcagttgg caaaaaaaca 420
catgatctaa tcgcattatg tgattttatg gatctagaaa agaacacacc agttacaata 480
ccagcattta tcaaatcagt ttctatcaag gagagtgaat cagccactgt tgaagctgca 540
ataagcagtg aagcagacca agctctaaca caagccaaaa ttgcacctta tgcgggactg 600
atcatgatta tgaccatgaa caatcccaaa ggcatattca agaagcttgg agctgggacc 660
caagttatag tagaactagg agcatatgtc caggctgaaa gcataagtaa aatatgcaag 720
acttggagcc atcaaggaac aagatatgtg ctgaagtcca gttaa 765

<210> 68
<211> 765
<212> DNA
<213> human Metapneumo virus

<400> 68
atggagtcct atctagtaga cacttatcaa ggcattccat atacagctgc tgttcaagtt 60
gacctggtag aaaaagattt actgccagca agtttgacaa tatggtttcc tttatttcag 120
gccaacacac caccagcagt tctgcttgat cagctaaaaa ccttgacaat aacaactctg 180
tatgctgcat cacagaatgg tccaatactc aaggtaaatg catctgccca aggtgctgcc 240
atgtctgtac ttcccaaaaa attcgaggta aatgcaactg tagcacttga tgaatacagt 300
aaacttgatt ttgacaagct gacggtctgc gatgttaaaa cagtttattt gacaactatg 360
aaaccgtacg ggatggtgtc aaaatttgtg agttcagcca aatcagttgg caaaaagaca 420
catgatctaa ttgcactatg tgacttcatg gacctagaga aaaatatacc tgtgacaata 480
ccagcattca taaagtcagt ttcaatcaaa gagagtgaat cagccactgt tgaagctgca 540
ataagcagcg aagccgacca agccttgaca caagccaaga ttgcgcccta tgcaggacta 600
attatgatca tgaccatgaa caatccaaaa ggtatattca agaaactagg ggctggaaca 660
caagtgatag tagagctggg ggcatatgtt caggctgaga gcatcagtag gatctgcaag 720
agctggagtc accaaggaac aagatacgta ctaaaatcca gataa 765

<210> 69
<211> 765
<212> DNA
<213> human Metapneumo virus

<400> 69
atggagtcct atctagtgga cacttatcaa ggcattccct acacagctgc tgttcaagtt 60
gatctggtag aaaaagactt actaccagca agtttgacaa tatggtttcc tctattccaa 120
gccaacacac caccagcggt tttgctcgat cagctaaaaa ccttgactat aacaactctg 180
tatgctgcat cacagaatgg tccaatactc aaagtaaatg catcagctca gggtgctgct 240
atgtctgtac ttcccaaaaa attcgaagta aatgcaactg tggcacttga tgaatacagc 300
aaacttgact ttgacaagtt aacggtttgc gatgttaaaa cagtttattt gacaaccatg 360
aagccatatg ggatggtgtc aaaatttgtg agttcagcca aatcagttgg caaaaagaca 420
catgatctaa ttgcactgtg tgacttcatg gacctagaga aaaatatacc tgtgacaata 480
ccagcattca taaagtcagt ttcaatcaaa gagagtgagt cagccactgt tgaagctgca 540
ataagcagtg aggccgacca agcattaaca caagccaaaa ttgcacccta tgcaggacta 600
atcatgatca tgaccatgaa caatccaaaa ggtatattca agaaactagg agctggaaca 660
caagtgatag tagagctagg ggcatatgtt caagccgaga gcatcagcag gatctgcaag 720
agctggagtc accaaggaac aagatatgta ctaaaatcca gataa 765

<210> 70
<211> 394

<212> PRT
<213> human Metapneumo virus

<400> 70
Met Ser Leu Gln Gly Ile His Leu Ser Asp Leu Ser Tyr Lys His Ala
1               5                   10                  15
Ile Leu Lys Glu Ser Gln Tyr Thr Ile Lys Arg Asp Val Gly Thr Thr
            20                  25                  30
Thr Ala Val Thr Pro Ser Ser Leu Gln Gln Glu Ile Thr Leu Leu Cys
        35                  40                  45
Gly Glu Ile Leu Tyr Ala Lys His Ala Asp Tyr Lys Tyr Ala Ala Glu
    50                  55                  60
Ile Gly Ile Gln Tyr Ile Ser Thr Ala Leu Gly Ser Glu Arg Val Gln
65                  70                  75                  80
Gln Ile Leu Arg Asn Ser Gly Ser Glu Val Gln Val Val Leu Thr Arg
                85                  90                  95
Thr Tyr Ser Leu Gly Lys Ile Lys Asn Asn Lys Gly Glu Asp Leu Gln
            100                 105                 110
Met Leu Asp Ile His Gly Val Glu Lys Ser Trp Val Glu Glu Ile Asp
        115                 120                 125
Lys Glu Ala Arg Lys Thr Met Ala Thr Leu Leu Lys Glu Ser Ser Gly
    130                 135                 140
Asn Ile Pro Gln Asn Gln Arg Pro Ser Ala Pro Asp Thr Pro Ile Ile
145                 150                 155                 160
Leu Leu Cys Val Gly Ala Leu Ile Phe Thr Lys Leu Ala Ser Thr Ile
                165                 170                 175
Glu Val Gly Leu Glu Thr Thr Val Arg Arg Ala Asn Arg Val Leu Ser
            180                 185                 190
Asp Ala Leu Lys Arg Tyr Pro Arg Met Asp Ile Pro Lys Ile Ala Arg
        195                 200                 205
Ser Phe Tyr Asp Leu Phe Glu Gln Lys Val Tyr His Arg Ser Leu Phe
    210                 215                 220
Ile Glu Tyr Gly Lys Ala Leu Gly Ser Ser Ser Thr Gly Ser Lys Ala
225                 230                 235                 240
Glu Ser Leu Phe Val Asn Ile Phe Met Gln Ala Tyr Gly Ala Gly Gln
                245                 250                 255
Thr Met Leu Arg Trp Gly Val Ile Ala Arg Ser Ser Asn Asn Ile Met
            260                 265                 270
Leu Gly His Val Ser Val Gln Ala Glu Leu Lys Gln Val Thr Glu Val
        275                 280                 285
Tyr Asp Leu Val Arg Glu Met Gly Pro Glu Ser Gly Leu Leu His Leu
    290                 295                 300
Arg Gln Ser Pro Lys Ala Gly Leu Leu Ser Leu Ala Asn Cys Pro Asn
305                 310                 315                 320
Phe Ala Ser Val Val Leu Gly Asn Ala Ser Gly Leu Gly Ile Ile Gly
                325                 330                 335
Met Tyr Arg Gly Arg Val Pro Asn Thr Glu Leu Phe Ser Ala Ala Glu
            340                 345                 350
Ser Tyr Ala Lys Ser Leu Lys Glu Ser Asn Lys Ile Asn Phe Ser Ser
        355                 360                 365
Leu Gly Leu Thr Asp Glu Glu Lys Glu Ala Ala Glu His Phe Leu Asn
    370                 375                 380
Val Ser Asp Asp Ser Gln Asn Asp Tyr Glu
385                 390

<210> 71
<211> 394
<212> PRT
<213> human Metapneumo virus

<400> 71

Met Ser Leu Gln Gly Ile His Leu Ser Asp Leu Ser Tyr Lys His Ala
1               5                   10                  15
Ile Leu Lys Glu Ser Gln Tyr Thr Ile Lys Arg Asp Val Gly Thr Thr
            20                  25                  30
Thr Ala Val Thr Pro Ser Ser Leu Gln Gln Glu Ile Thr Leu Leu Cys
        35                  40                  45
Gly Glu Ile Leu Tyr Ala Lys His Ala Asp Tyr Lys Tyr Ala Ala Glu
    50                  55                  60
Ile Gly Ile Gln Tyr Ile Ser Thr Ala Leu Gly Ser Glu Arg Val Gln
65                  70                  75                  80
Gln Ile Leu Arg Asn Ser Gly Ser Glu Val Gln Val Val Leu Thr Arg
                85                  90                  95
Thr Tyr Ser Leu Gly Lys Val Lys Asn Asn Lys Gly Glu Asp Leu Gln
            100                 105                 110
Met Leu Asp Ile His Gly Val Glu Lys Ser Trp Val Glu Glu Ile Asp
        115                 120                 125
Lys Glu Ala Arg Lys Thr Met Ala Thr Leu Leu Lys Glu Ser Ser Gly
    130                 135                 140
Asn Ile Pro Gln Asn Gln Arg Pro Ser Ala Pro Asp Thr Pro Ile Ile
145                 150                 155                 160
Leu Leu Cys Val Gly Ala Leu Ile Phe Thr Lys Leu Ala Ser Thr Ile
                165                 170                 175
Glu Val Gly Leu Glu Thr Thr Val Arg Arg Ala Asn Arg Val Leu Ser
            180                 185                 190
Asp Ala Leu Lys Arg Tyr Pro Arg Met Asp Ile Pro Lys Ile Ala Arg
        195                 200                 205
Ser Phe Tyr Asp Leu Phe Glu Gln Lys Val Tyr Tyr Arg Ser Leu Phe
    210                 215                 220
Ile Glu Tyr Gly Lys Ala Leu Gly Ser Ser Ser Thr Gly Ser Lys Ala
225                 230                 235                 240
Glu Ser Leu Phe Val Asn Ile Phe Met Gln Ala Tyr Gly Ala Gly Gln
                245                 250                 255
Thr Met Leu Arg Trp Gly Val Ile Ala Arg Ser Ser Asn Asn Ile Met
            260                 265                 270
Leu Gly His Val Ser Val Gln Ala Glu Leu Lys Gln Val Thr Glu Val
        275                 280                 285
Tyr Asp Leu Val Arg Glu Met Gly Pro Glu Ser Gly Leu Leu His Leu
    290                 295                 300
Arg Gln Ser Pro Lys Ala Gly Leu Leu Ser Leu Ala Asn Cys Pro Asn
305                 310                 315                 320
Phe Ala Ser Val Val Leu Gly Asn Ala Ser Gly Leu Gly Ile Ile Gly
                325                 330                 335
Met Tyr Arg Gly Arg Val Pro Asn Thr Glu Leu Phe Ser Ala Ala Glu
            340                 345                 350
Ser Tyr Ala Lys Ser Leu Lys Glu Ser Asn Lys Ile Asn Phe Ser Ser
        355                 360                 365
Leu Gly Leu Thr Asp Glu Glu Lys Glu Ala Ala Glu His Phe Leu Asn
    370                 375                 380
Val Ser Asp Asp Ser Gln Asn Asp Tyr Glu
385                 390

<210> 72
<211> 394
<212> PRT
<213> human Metapneumo virus

<400> 72
Met Ser Leu Gln Gly Ile His Leu Ser Asp Leu Ser Tyr Lys His Ala
1               5                   10                  15
Ile Leu Lys Glu Ser Gln Tyr Thr Ile Lys Arg Asp Val Gly Thr Thr
            20                  25                  30

Thr Ala Val Thr Pro Ser Ser Leu Gln Gln Glu Ile Thr Leu Leu Cys
        35                  40                  45
Gly Glu Ile Leu Tyr Thr Lys His Thr Asp Tyr Lys Tyr Ala Ala Glu
    50                  55                  60
Ile Gly Ile Gln Tyr Ile Cys Thr Ala Leu Gly Ser Glu Arg Val Gln
65                  70                  75                  80

Gln Ile Leu Arg Asn Ser Gly Ser Glu Val Gln Val Val Leu Thr Lys
                85                  90                  95
Thr Tyr Ser Leu Gly Lys Gly Lys Asn Ser Lys Gly Glu Glu Leu Gln
            100                 105                 110
Met Leu Asp Ile His Gly Val Glu Lys Ser Trp Ile Glu Glu Ile Asp
        115                 120                 125
Lys Glu Ala Arg Lys Thr Met Val Thr Leu Leu Lys Glu Ser Ser Gly
    130                 135                 140
Asn Ile Pro Gln Asn Gln Arg Pro Ser Ala Pro Asp Thr Pro Ile Ile
145                 150                 155                 160
Leu Leu Cys Val Gly Ala Leu Ile Phe Thr Lys Leu Ala Ser Thr Ile
                165                 170                 175
Glu Val Gly Leu Glu Thr Thr Val Arg Arg Ala Asn Arg Val Leu Ser
            180                 185                 190
Asp Ala Leu Lys Arg Tyr Pro Arg Ile Asp Ile Pro Lys Ile Ala Arg
        195                 200                 205
Ser Phe Tyr Glu Leu Phe Glu Gln Lys Val Tyr Tyr Arg Ser Leu Phe
    210                 215                 220
Ile Glu Tyr Gly Lys Ala Leu Gly Ser Ser Ser Thr Gly Ser Lys Ala
225                 230                 235                 240
Glu Ser Leu Phe Val Asn Ile Phe Met Gln Ala Tyr Gly Ala Gly Gln
                245                 250                 255
Thr Leu Leu Arg Trp Gly Val Ile Ala Arg Ser Ser Asn Asn Ile Met
            260                 265                 270
Leu Gly His Val Ser Val Gln Ser Glu Leu Lys Gln Val Thr Glu Val
        275                 280                 285
Tyr Asp Leu Val Arg Glu Met Gly Pro Glu Ser Gly Leu Leu His Leu
    290                 295                 300
Arg Gln Ser Pro Lys Ala Gly Leu Leu Ser Leu Ala Asn Cys Pro Asn
305                 310                 315                 320
Phe Ala Ser Val Val Leu Gly Asn Ala Ser Gly Leu Gly Ile Ile Gly
                325                 330                 335
Met Tyr Arg Gly Arg Val Pro Asn Thr Glu Leu Phe Ser Ala Ala Glu
            340                 345                 350
Ser Tyr Ala Arg Ser Leu Lys Glu Ser Asn Lys Ile Asn Phe Ser Ser
        355                 360                 365
Leu Gly Leu Thr Asp Glu Glu Lys Glu Ala Ala Glu His Phe Leu Asn
    370                 375                 380
Met Ser Gly Asp Asn Gln Asn Asp Tyr Glu
385                 390


<210> 73
<211> 394
<212> PRT
<213> human Metapneumo virus

<400> 73
Met Ser Leu Gln Gly Ile His Leu Ser Asp Leu Ser Tyr Lys His Ala
1               5                   10                  15
Ile Leu Lys Glu Ser Gln Tyr Thr Ile Lys Arg Asp Val Gly Thr Thr
            20                  25                  30
Thr Ala Val Thr Pro Ser Ser Leu Gln Gln Glu Ile Thr Leu Leu Cys
        35                  40                  45
Gly Glu Ile Leu Tyr Thr Lys His Thr Asp Tyr Lys Tyr Ala Ala Glu

    50                  55                  60
Ile Gly Ile Gln Tyr Ile Cys Thr Ala Leu Gly Ser Glu Arg Val Gln
65                  70                  75                  80
Gln Ile Leu Arg Asn Ser Gly Ser Glu Val Gln Val Val Leu Thr Lys
                85                  90                  95
Thr Tyr Ser Leu Gly Lys Gly Lys Asn Ser Lys Gly Glu Glu Leu Gln
            100                 105                 110
Met Leu Asp Ile His Gly Val Glu Lys Ser Trp Val Glu Glu Ile Asp
        115                 120                 125
Lys Glu Ala Arg Lys Thr Met Val Thr Leu Leu Lys Glu Ser Ser Gly
    130                 135                 140
Asn Ile Pro Gln Asn Gln Arg Pro Ser Ala Pro Asp Thr Pro Ile Ile
145                 150                 155                 160
Leu Leu Cys Val Gly Ala Leu Ile Phe Thr Lys Leu Ala Ser Thr Ile
                165                 170                 175
Glu Val Gly Leu Glu Thr Thr Val Arg Arg Ala Asn Arg Val Leu Ser
            180                 185                 190
Asp Ala Leu Lys Arg Tyr Pro Arg Val Asp Ile Pro Lys Ile Ala Arg
        195                 200                 205
Ser Phe Tyr Glu Leu Phe Glu Gln Lys Val Tyr Tyr Arg Ser Leu Phe
    210                 215                 220
Ile Glu Tyr Gly Lys Ala Leu Gly Ser Ser Ser Thr Gly Ser Lys Ala
225                 230                 235                 240
Glu Ser Leu Phe Val Asn Ile Phe Met Gln Ala Tyr Gly Ala Gly Gln
                245                 250                 255
Thr Met Leu Arg Trp Gly Val Ile Ala Arg Ser Ser Asn Asn Ile Met
            260                 265                 270
Leu Gly His Val Ser Val Gln Ala Glu Leu Lys Gln Val Thr Glu Val
        275                 280                 285
Tyr Asp Leu Val Arg Glu Met Gly Pro Glu Ser Gly Leu Leu His Leu
    290                 295                 300
Arg Gln Ser Pro Lys Ala Gly Leu Leu Ser Leu Ala Asn Cys Pro Asn
305                 310                 315                 320
Phe Ala Ser Val Val Leu Gly Asn Ala Ser Gly Leu Gly Ile Ile Gly
                325                 330                 335
Met Tyr Arg Gly Arg Val Pro Asn Thr Glu Leu Phe Ser Ala Ala Glu
            340                 345                 350
Ser Tyr Ala Arg Ser Leu Lys Glu Ser Asn Lys Ile Asn Phe Ser Ser
        355                 360                 365
Leu Gly Leu Thr Asp Glu Glu Lys Glu Ala Ala Glu His Phe Leu Asn
    370                 375                 380
Met Ser Asp Asp Asn Gln Asp Asp Tyr Glu
385                 390

<210> 74
<211> 1185
<212> DNA
<213> human Metapneumo virus

<400> 74
atgtctcttc aagggattca cctgagtgat ttatcataca agcatgctat attaaaagag 60
tctcagtaca caataaaaag agatgtgggt acaacaactg cagtgacacc ctcatcattg 120
caacaagaaa taacactgtt gtgtggagaa attctgtatg ctaaacatgc tgactacaaa 180
tatgctgcag aaataggaat acaatatatt agcacagctt taggatcaga gagagtgcag 240
cagattctga ggaactcagg cagtgaagtc caagtggtct taaccagaac gtactctctg 300
gggaaaatta aaaacaataa aggagaagat ttacagatgt tagacataca cggggtagag 360
aagagctggg tagaagagat agacaaagaa gcaaggaaaa caatggcaac cttgcttaag 420
gaatcatcag gtaatatccc acaaaatcag aggccctcag caccagacac acccataatc 480
ttattatgtg taggtgcctt aatattcact aaactagcat caaccataga agtgggacta 540
gagaccacag tcagaagggc taaccgtgta ctaagtgatg cactcaagag ataccctaga 600
atggacatac caaagattgc cagatccttc tatgacttat ttgaacaaaa agtgtatcac 660

agaagtttgt tcattgagta tggcaaagca ttaggctcat catctacagg cagcaaagca 720
gaaagtctat ttgttaatat attcatgcaa gcttatgggg ccggtcaaac aatgctaagg 780
tggggggtca ttgccaggtc atccaacaat ataatgttag gacatgtatc cgtccaagct 840
gagttaaaac aggtcacaga agtctatgac ttggtgcgag aaatgggccc tgaatctgga 900
cttctacatt taaggcaaag cccaaaagct ggactgttat cactagccaa ctgtcccaac 960
tttgcaagtg ttgttctcgg aaatgcctca ggcttaggca taatcggtat gtatcgaggg 1020
agagtaccaa acacagaatt attttcagca gctgaaagtt atgccaaaag tttgaaagaa 1080
agcaataaaa taaatttctc ttcattagga cttacagatg aagagaaaga ggctgcagaa 1140
catttcttaa atgtgagtga cgacagtcaa aatgattatg agtaa 1185

<210> 75
<211> 1185
<212> DNA
<213> human Metapneumo virus

<400> 75
atgtctcttc aagggattca cctgagtgat ctatcataca agcatgctat attaaaagag 60
tctcagtata caataaagag agatgtaggc acaacaaccg cagtgacacc ctcatcattg 120
caacaagaaa taacactatt gtgtggagaa attctatatg ctaagcatgc tgattacaaa 180
tatgctgcag aaataggaat acaatatatt agcacagctc taggatcaga gagagtacag 240
cagattctaa gaaactcagg tagtgaagtc caagtggttt taaccagaac gtactccttg 300
gggaaagtta aaaacaacaa aggagaagat ttacagatgt tagacataca cggagtagag 360
aaaagctggg tggaagagat agacaaagaa gcaagaaaaa caatggcaac tttgcttaaa 420
gaatcatcag gcaatattcc acaaaatcag aggccttcag caccagacac acccataatc 480
ttattatgtg taggtgcctt aatatttacc aaactagcat caactataga agtgggatta 540
gagaccacag tcagaagagc taaccgtgta ctaagtgatg cactcaaaag ataccctagg 600
atggacatac caaaaatcgc tagatctttc tatgacttat ttgaacaaaa agtgtattac 660
agaagtttgt tcattgagta tggcaaagca ttaggctcat cctctacagg cagcaaagca 720
gaaagtttat tcgttaatat attcatgcaa gcttacggtg ctggtcaaac aatgctgagg 780
tggggagtca ttgccaggtc atctaacaat ataatgttag gacatgtatc tgttcaagct 840
gagttaaaac aagtcacaga agtctatgac ctggtgcgag aaatgggccc tgaatctggg 900
ctcctacatt taaggcaaag cccaaaagct ggactgttat cactagccaa ttgtcccaac 960
tttgctagtg ttgttctcgg caatgcctca ggcttaggca taataggtat gtatcgcggg 1020
agagtgccaa acacagaact attttcagca gcagaaagct atgccaagag tttgaaagaa 1080
agcaataaaa ttaacttttc ttcattagga ctcacagatg aagaaaaaga ggctgcagaa 1140
cacttcctaa atgtgagtga cgacagtcaa aatgattatg agtaa 1185

<210> 76
<211> 1185
<212> DNA
<213> human Metapneumo virus

<400> 76
atgtctcttc aagggattca cctaagtgat ctatcatata aacatgctat attaaaagag 60
tctcaataca caataaaaag agatgtaggc accacaactg cagtgacacc ttcatcatta 120
caacaagaaa taacactttt gtgtggggaa atactttaca ctaaacacac tgattacaaa 180
tatgctgctg agataggaat acaatatatt tgcacagctc taggatcaga aagagtacaa 240
cagattttga gaaactcagg tagtgaagtt caggtggttc taaccaaaac atactcctta 300
gggaaaggca aaaacagtaa aggggaagag ctgcagatgt tagatataca tggagtggaa 360
aagagttgga tagaagaaat agacaaagag gcaagaaaga caatggtaac tttgcttaag 420
gaatcatcag gtaacatccc acaaaaccag agaccttcag caccagacac accaataatt 480
ttattatgtg taggtgccct aatattcact aaactagcat caacaataga agttggatta 540
gagactacag ttagaagagc taatagagtg ctaagtgatg cactcaaaag atacccaagg 600
atagatatac caaagattgc tagatctttt tatgaactat ttgaacaaaa agtgtactac 660
agaagtttat tcattgagta cggaaaagct ttaggctcat cttcaacagg aagcaaagca 720
gaaagtttgt ttgtaaatat atttatgcaa gcttatggag ctggccaaac actgctaagg 780
tggggtgtca ttgccagatc atccaacaac ataatgctag ggcatgtatc tgtgcaatct 840
gaattgaagc aagttacaga ggtttatgac ttggtgagag aaatgggtcc tgaatctggg 900
cttttacatc taagacaaag tccaaaggca gggctgttat cattggccaa ttgccccaat 960
tttgctagtg ttgttcttgg caatgcttca ggtctaggca taatcggaat gtacagaggg 1020
agagtaccaa acacagagct attttctgca gcagaaagtt atgccagaag cttaaaagaa 1080
agcaataaaa tcaacttctc ttcgttaggg cttacagatg aagaaaaaga agctgcagaa 1140

cacttcttaa acatgagtgg tgacaatcaa aatgattatg agtaa 1185

<210> 77
<211> 1185
<212> DNA
<213> human Metapneumo virus

<400> 77
atgtctcttc aagggattca cctaagtgat ctgtcatata aacatgctat attaaaagag 60
tctcaataca caataaaaag agatgtaggc accacaactg cagtgacacc ttcatcattg 120
cagcaagaga taacactttt gtgtggagag attctttaca ctaaacatac tgattacaaa 180
tatgctgcag agatagggat acaatatatt tgcacagctc taggatcaga aagagtacaa 240
cagattttaa gaaattcagg tagtgaggtt caggtggttc taaccaagac atactcttta 300
gggaaaggta aaaatagtaa aggggaagag ttgcaaatgt tagatataca tggagtggaa 360
aagagttggg tagaagaaat agacaaagag gcaagaaaaa caatggtgac tttgctaaag 420
gaatcatcag gcaacatccc acaaaaccag aggccttcag caccagacac accaataatt 480
ttattgtgtg taggtgcttt aatattcact aaactagcat caacaataga agttggacta 540
gagactacag ttagaagggc taacagagtg ttaagtgatg cgctcaaaag ataccctagg 600
gtagatatac caaagattgc tagatctttt tatgaactat ttgagcagaa agtgtattac 660
aggagtctat tcattgagta tgggaaagct ttaggctcat cttcaacagg aagcaaagca 720
gaaagtttgt ttgtaaatat atttatgcaa gcttatggag ccggtcagac aatgctaagg 780
tggggtgtca ttgccagatc atctaacaac ataatgctag ggcatgtatc tgtgcaagct 840
gaattgaaac aagttacaga ggtttatgat ttggtaagag aaatgggtcc tgaatctggg 900
cttttacatc taagacaaag tccaaaggca ggactgttat cgttggctaa ttgccccaat 960
tttgctagtg ttgttcttgg taatgcttca ggtctaggta taatcggaat gtacagggga 1020
agagtgccaa acacagagct attttctgca gcagaaagtt atgccagaag cttaaaagaa 1080
agcaacaaaa tcaacttctc ctcattaggg ctcacagacg aagaaaaaga agctgcagaa 1140
cacttcttaa acatgagtga tgacaatcaa gatgattatg agtaa 1185

<210> 78
<211> 294
<212> PRT
<213> human Metapneumo virus

<400> 78
Met Ser Phe Pro Glu Gly Lys Asp Ile Leu Phe Met Gly Asn Glu Ala
1               5                   10                  15
Ala Lys Leu Ala Glu Ala Phe Gln Lys Ser Leu Arg Lys Pro Gly His
            20                  25                  30
Lys Arg Ser Gln Ser Ile Ile Gly Glu Lys Val Asn Thr Val Ser Glu
        35                  40                  45
Thr Leu Glu Leu Pro Thr Ile Ser Arg Pro Ala Lys Pro Thr Ile Pro
    50                  55                  60
Ser Glu Pro Lys Leu Ala Trp Thr Asp Lys Gly Gly Ala Thr Lys Thr
65                  70                  75                  80
Glu Ile Lys Gln Ala Ile Lys Val Met Asp Pro Ile Glu Glu Glu Glu
                85                  90                  95
Ser Thr Glu Lys Lys Val Leu Pro Ser Ser Asp Gly Lys Thr Pro Ala
            100                 105                 110
Glu Lys Lys Leu Lys Pro Ser Thr Asn Thr Lys Lys Lys Val Ser Phe
        115                 120                 125
Thr Pro Asn Glu Pro Gly Lys Tyr Thr Lys Leu Glu Lys Asp Ala Leu
    130                 135                 140
Asp Leu Leu Ser Asp Asn Glu Glu Glu Asp Ala Glu Ser Ser Ile Leu
145                 150                 155                 160
Thr Phe Glu Glu Arg Asp Thr Ser Ser Leu Ser Ile Glu Ala Arg Leu
                165                 170                 175
Glu Ser Ile Glu Glu Lys Leu Ser Met Ile Leu Gly Leu Leu Arg Thr
            180                 185                 190
Leu Asn Ile Ala Thr Ala Gly Pro Thr Ala Ala Arg Asp Gly Ile Arg
        195                 200                 205
Asp Ala Met Ile Gly Val Arg Glu Glu Leu Ile Ala Asp Ile Ile Lys

210                 215                 220
Glu Ala Lys Gly Lys Ala Ala Glu Met Met Glu Glu Glu Met Ser Gln
225                 230                 235                 240
Arg Ser Lys Ile Gly Asn Gly Ser Val Lys Leu Thr Glu Lys Ala Lys
                245                 250                 255
Glu Leu Asn Lys Ile Val Glu Asp Glu Ser Thr Ser Gly Glu Ser Glu
            260                 265                 270
Glu Glu Glu Glu Pro Lys Asp Thr Gln Asp Asn Ser Gln Glu Asp Asp
        275                 280                 285
Ile Tyr Gln Leu Ile Met
    290

<210> 79
<211> 294
<212> PRT
<213> human Metapneumo virus

<400> 79
Met Ser Phe Pro Glu Gly Lys Asp Ile Leu Phe Met Gly Asn Glu Ala
1               5                   10                  15
Ala Lys Leu Ala Glu Ala Phe Gln Lys Ser Leu Arg Lys Pro Asn His
            20                  25                  30
Lys Arg Ser Gln Ser Ile Ile Gly Glu Lys Val Asn Thr Val Ser Glu
        35                  40                  45
Thr Leu Glu Leu Pro Thr Ile Ser Arg Pro Thr Lys Pro Thr Ile Leu
    50                  55                  60
Ser Glu Pro Lys Leu Ala Trp Thr Asp Lys Gly Gly Ala Ile Lys Thr
65                  70                  75                  80
Glu Ala Lys Gln Thr Ile Lys Val Met Asp Pro Ile Glu Glu Glu Glu
                85                  90                  95
Phe Thr Glu Lys Arg Val Leu Pro Ser Ser Asp Gly Lys Thr Pro Ala
            100                 105                 110
Glu Lys Lys Leu Lys Pro Ser Thr Asn Thr Lys Lys Lys Val Ser Phe
        115                 120                 125
Thr Pro Asn Glu Pro Gly Lys Tyr Thr Lys Leu Glu Lys Asp Ala Leu
    130                 135                 140
Asp Leu Leu Ser Asp Asn Glu Glu Glu Asp Ala Glu Ser Ser Ile Leu
145                 150                 155                 160
Thr Phe Glu Glu Arg Asp Thr Ser Ser Leu Ser Ile Glu Ala Arg Leu
                165                 170                 175
Glu Ser Ile Glu Glu Lys Leu Ser Met Ile Leu Gly Leu Leu Arg Thr
            180                 185                 190
Leu Asn Ile Ala Thr Ala Gly Pro Thr Ala Ala Arg Asp Gly Ile Arg
        195                 200                 205
Asp Ala Met Ile Gly Ile Arg Glu Glu Leu Ile Ala Asp Ile Ile Lys
    210                 215                 220
Glu Ala Lys Gly Lys Ala Ala Glu Met Met Glu Glu Glu Met Asn Gln
225                 230                 235                 240
Arg Thr Lys Ile Gly Asn Gly Ser Val Lys Leu Thr Glu Lys Ala Lys
                245                 250                 255
Glu Leu Asn Lys Ile Val Glu Asp Glu Ser Thr Ser Gly Glu Ser Glu
            260                 265                 270
Glu Glu Glu Glu Pro Lys Asp Thr Gln Glu Asn Asn Gln Glu Asp Asp
        275                 280                 285
Ile Tyr Gln Leu Ile Met
    290

<210> 80
<211> 294
<212> PRT

<213> human Metapneumo virus

<400> 80
Met Ser Phe Pro Glu Gly Lys Asp Ile Leu Phe Met Gly Asn Glu Ala
1               5                   10                  15
Ala Lys Ile Ala Glu Ala Phe Gln Lys Ser Leu Lys Lys Ser Gly His
            20                  25                  30
Lys Arg Thr Gln Ser Ile Val Gly Glu Lys Val Asn Thr Ile Ser Glu
        35                  40                  45
Thr Leu Glu Leu Pro Thr Ile Ser Lys Pro Ala Arg Ser Ser Thr Leu
    50                  55                  60
Leu Glu Pro Lys Leu Ala Trp Ala Asp Asn Ser Gly Ile Thr Lys Ile
65                  70                  75                  80
Thr Glu Lys Pro Ala Thr Lys Thr Thr Asp Pro Val Glu Glu Glu Glu
                85                  90                  95
Phe Asn Glu Lys Lys Val Leu Pro Ser Ser Asp Gly Lys Thr Pro Ala
            100                 105                 110
Glu Lys Lys Ser Lys Phe Ser Thr Ser Val Lys Lys Lys Val Ser Phe
        115                 120                 125
Thr Ser Asn Glu Pro Gly Lys Tyr Thr Lys Leu Glu Lys Asp Ala Leu
    130                 135                 140
Asp Leu Leu Ser Asp Asn Glu Glu Glu Asp Ala Glu Ser Ser Ile Leu
145                 150                 155                 160
Thr Phe Glu Glu Lys Asp Thr Ser Ser Leu Ser Ile Glu Ala Arg Leu
                165                 170                 175
Glu Ser Ile Glu Glu Lys Leu Ser Met Ile Leu Gly Leu Leu Arg Thr
            180                 185                 190
Leu Asn Ile Ala Thr Ala Gly Pro Thr Ala Ala Arg Asp Gly Ile Arg
        195                 200                 205
Asp Ala Met Ile Gly Ile Arg Glu Glu Leu Ile Ala Glu Ile Ile Lys
    210                 215                 220
Glu Ala Lys Gly Lys Ala Ala Glu Met Met Glu Glu Glu Met Asn Gln
225                 230                 235                 240
Arg Ser Lys Ile Gly Asn Gly Ser Val Lys Leu Thr Glu Lys Ala Lys
                245                 250                 255
Glu Leu Asn Lys Ile Val Glu Asp Glu Ser Thr Ser Gly Glu Ser Glu
            260                 265                 270
Glu Glu Glu Glu Pro Lys Glu Thr Gln Asp Asn Asn Gln Gly Glu Asp
        275                 280                 285
Ile Tyr Gln Leu Ile Met
    290


<210> 81
<211> 294
<212> PRT
<213> human Metapneumo virus

<400> 81
Met Ser Phe Pro Glu Gly Lys Asp Ile Leu Phe Met Gly Asn Glu Ala
1               5                   10                  15
Ala Lys Ile Ala Glu Ala Phe Gln Lys Ser Leu Lys Arg Ser Gly His
            20                  25                  30
Lys Arg Thr Gln Ser Ile Val Gly Glu Lys Val Asn Thr Ile Ser Glu
        35                  40                  45
Thr Leu Glu Leu Pro Thr Ile Ser Lys Pro Ala Arg Ser Ser Thr Leu
    50                  55                  60
Leu Glu Pro Lys Leu Ala Trp Ala Asp Ser Ser Gly Ala Thr Lys Thr
65                  70                  75                  80
Thr Glu Lys Gln Thr Thr Lys Thr Thr Asp Pro Val Glu Glu Glu Glu
                85                  90                  95
Leu Asn Glu Lys Lys Val Ser Pro Ser Ser Asp Gly Lys Thr Pro Ala

                100                 105                 110
Glu Lys Lys Ser Lys Ser Pro Thr Asn Val Lys Lys Lys Val Ser Phe
        115                 120                 125
Thr Ser Asn Glu Pro Gly Lys Tyr Thr Lys Leu Glu Lys Asp Ala Leu
    130                 135                 140
Asp Leu Leu Ser Asp Asn Glu Glu Glu Asp Ala Glu Ser Ser Ile Leu
145                 150                 155                 160
Thr Phe Glu Glu Arg Asp Thr Ser Ser Leu Ser Ile Glu Ala Arg Leu
                165                 170                 175
Glu Ser Ile Glu Glu Lys Leu Ser Met Ile Leu Gly Leu Leu Arg Thr
            180                 185                 190
Leu Asn Ile Ala Thr Ala Gly Pro Thr Ala Ala Arg Asp Gly Ile Arg
        195                 200                 205
Asp Ala Met Ile Gly Ile Arg Glu Glu Leu Ile Ala Glu Ile Ile Lys
    210                 215                 220
Glu Ala Lys Gly Lys Ala Ala Glu Met Met Glu Glu Glu Met Asn Gln
225                 230                 235                 240
Arg Ser Lys Ile Gly Asn Gly Ser Val Lys Leu Thr Glu Lys Ala Lys
                245                 250                 255
Glu Leu Asn Lys Ile Val Glu Asp Glu Ser Thr Ser Gly Glu Ser Glu
            260                 265                 270
Glu Glu Glu Glu Pro Lys Glu Thr Gln Asp Asn Asn Gln Gly Glu Asp
        275                 280                 285
Ile Tyr Gln Leu Ile Met
    290

<210> 82
<211> 885
<212> DNA
<213> human Metapneumo virus

<400> 82
atgtcattcc ctgaaggaaa agatattctt ttcatgggta atgaagcagc aaaattagca 60
gaagctttcc agaaatcatt aagaaaacca ggtcataaaa gatctcaatc tattatagga 120
gaaaaagtga atactgtatc agaaacattg gaattaccta ctatcagtag acctgcaaaa 180
ccaaccatac cgtcagaacc aaagttagca tggacagata aaggtggggc aaccaaaact 240
gaaataaagc aagcaatcaa agtcatggat cccattgaag aagaagagtc taccgagaag 300
aaggtgctac cctccagtga tgggaaaacc cctgcagaaa agaaactgaa accatcaact 360
aacaccaaaa agaaggtttc atttacacca aatgaaccag ggaaatatac aaagttggaa 420
aaagatgctc tagatttgct ctcagataat gaagaagaag atgcagaatc ttcaatctta 480
acctttgaag aaagagatac ttcatcatta agcattgagg ccagattgga atcaatagag 540
gagaaattaa gcatgatatt agggctatta agaacactca acattgctac agcaggaccc 600
acagcagcaa gagatgggat cagagatgca atgattggcg taagagagga attaatagca 660
gacataataa aggaagctaa agggaaagca gcagaaatga tggaagagga aatgagtcaa 720
cgatcaaaaa taggaaatgg tagtgtaaaa ttaacagaaa aagcaaaaga gctcaacaaa 780
attgttgaag atgaaagcac aagtggagaa tccgaagaag aagaagaacc aaaagacaca 840
caagacaata gtcaagaaga tgacatttac cagttaatta tgtag                 885

<210> 83
<211> 885
<212> DNA
<213> human Metapneumo virus

<400> 83
atgtcattcc ctgaaggaaa agatattctt ttcatgggta atgaagcagc aaaattggca 60
gaagcttttc aaaaatcatt aagaaaacct aatcataaaa gatctcaatc tattatagga 120
gaaaaagtga acactgtatc tgaaacattg gaattaccta ctatcagtag acctaccaaa 180
ccgaccatat tgtcagagcc gaagttagca tggacagaca aaggtggggc aatcaaaact 240
gaagcaaagc aaacaatcaa agttatggat cctattgaag aagaagagtt tactgagaaa 300
agggtgctgc cctccagtga tgggaaaact cctgcagaaa agaagttgaa accatcaacc 360
aacactaaaa agaaggtctc atttacacca aatgaaccag gaaaatacac aaagttggag 420

aaagatgctc tagacttgct ttcagacaat gaagaagaag atgcagaatc ctcaatctta 480
accttcgaag aaagagatac ttcatcatta agcattgaag ccagactaga atcgattgag 540
gagaaattaa gcatgatatt agggctatta agaacactca acattgctac agcaggaccc 600
acagcagcaa gagatgggat cagagatgca atgattggca taagggagga actaatagca 660
gacataataa aagaagccaa gggaaaagca gcagaaatga tggaagaaga aatgaaccag 720
cggacaaaaa taggaaacgg tagtgtaaaa ttaactgaaa aggcaaagga gctcaacaaa 780
attgttgaag acgaaagcac aagtggtgaa tccgaagaag aagaagaacc aaaagacaca 840
caggaaaata atcaagaaga tgacatttac cagttaatta tgtag 885

<210> 84
<211> 885
<212> DNA
<213> human Metapneumo virus

<400> 84
atgtcattcc ctgaaggaaa ggatattctg ttcatgggta atgaagcagc aaaaatagcc 60
gaagctttcc agaaatcact gaaaaaatca ggtcacaaga gaactcaatc tattgtaggg 120
gaaaaagtta acactatatc agaaactcta gaactaccta ccatcagcaa acctgcacga 180
tcatctacac tgctggaacc aaaattggca tgggcagaca acagcggaat caccaaaatc 240
acagaaaaac cagcaaccaa aacaacagat cctgttgaag aagaggaatt caatgaaaag 300
aaagtgttac cttccagtga tgggaagact cctgcagaga aaaaatcaaa gttttcaacc 360
agtgtaaaaa agaaagtttc ctttacatca aatgaaccag ggaaatacac caaactagag 420
aaagatgccc tagatttgct ctcagacaat gaggaagaag acgcagaatc ctcaatccta 480
acttttgagg agaaagatac atcatcacta agcattgaag ctagactaga atctatagaa 540
gagaagttga gcatgatatt aggactgctt cgtacactta acattgcaac agcaggacca 600
acagctgcac gagatggaat tagggatgca atgattggta taagagaaga gctaatagca 660
gagataatta aggaagccaa gggaaaagca gctgaaatga tggaagaaga gatgaatcaa 720
agatcaaaaa taggaaatgg cagtgtaaaa ctaaccgaga aggcaaaaga gctcaacaaa 780
attgttgaag acgagagcac aagcggtgaa tcagaagaag aagaagaacc aaaagaaact 840
caggataaca atcaaggaga agatatttat cagttaatca tgtag 885

<210> 85
<211> 885
<212> DNA
<213> human Metapneumo virus

<400> 85
atgtcattcc ctgaaggaaa agatatcctg ttcatgggta atgaagcagc aaaaatagca 60
gaagctttcc agaaatcact aaaaagatca ggtcacaaaa gaacccagtc tattgtaggg 120
gaaaaagtaa acactatatc agaaactcta gagctaccta ccatcagcaa acctgcacga 180
tcatctacac tgctagagcc aaaattggca tgggcagaca gcagcggagc caccaaaacc 240
acagaaaaac aaacaaccaa aacaacagat cctgttgaag aagaggaact caatgaaaag 300
aaggtatcac cttccagtga tgggaagact cctgcagaga aaaaatcaaa atctccaacc 360
aatgtaaaaa agaaagtttc cttcacatca aatgaaccag ggaaatatac taaactagaa 420
aaagatgccc tagatttgct ctcagacaat gaggaagaag acgcagagtc ctcaatccta 480
acctttgaag agagagacac atcatcacta agcattgagg ctagactaga atcaatagaa 540
gagaagctaa gcatgatatt aggactgctt cgtacactta acattgcaac agcaggacca 600
acggctgcaa gggatggaat cagagatgca atgattggta taagagaaga actaatagca 660
gaaataataa aagaagcaaa gggaaaagca gccgaaatga tggaagagga aatgaatcaa 720
aggtcaaaaa taggtaatgg cagtgtaaaa ctaaccgaga aggcaaaaga acttaataaa 780
attgttgaag acgagagcac aagtggtgaa tcagaagaag aagaagaacc aaaagaaact 840
caggataaca atcaaggaga agatatctac cagttaatca tgtag 885

<210> 86
<211> 183
<212> PRT
<213> human Metapneumo virus

<400> 86
Met Ile Thr Leu Asp Val Ile Lys Ser Asp Gly Ser Ser Lys Thr Cys
1               5                   10                  15
Thr His Leu Lys Lys Ile Ile Lys Asp His Ser Gly Lys Val Leu Ile

                20                  25                  30
Val Leu Lys Leu Ile Leu Ala Leu Leu Thr Phe Leu Thr Val Thr Ile
        35                  40                  45
Thr Ile Asn Tyr Ile Lys Val Glu Asn Asn Leu Gln Ile Cys Gln Ser
    50                  55                  60
Lys Thr Glu Ser Asp Lys Lys Asp Ser Ser Ser Asn Thr Thr Ser Val
65                  70                  75                  80
Thr Thr Lys Thr Thr Leu Asn His Asp Ile Thr Gln Tyr Phe Lys Ser
                85                  90                  95
Leu Ile Gln Arg Tyr Thr Asn Ser Ala Ile Asn Ser Asp Thr Cys Trp
                100                 105                 110
Lys Ile Asn Arg Asn Gln Cys Thr Asn Ile Thr Thr Tyr Lys Phe Leu
        115                 120                 125
Cys Phe Lys Ser Glu Asp Thr Lys Thr Asn Asn Cys Asp Lys Leu Thr
    130                 135                 140
Asp Leu Cys Arg Asn Lys Pro Lys Pro Ala Val Gly Val Tyr His Ile
145                 150                 155                 160
Val Glu Cys His Cys Ile Tyr Thr Val Lys Trp Lys Cys Tyr His Tyr
                165                 170                 175
Pro Thr Asp Glu Thr Gln Ser
            180

<210> 87
<211> 179
<212> PRT
<213> human Metapneumo virus

<400> 87
Met Ile Thr Leu Asp Val Ile Lys Ser Asp Gly Ser Ser Lys Thr Cys
 1               5                  10                  15
Thr His Leu Lys Lys Ile Ile Lys Asp His Ser Gly Lys Val Leu Ile
                20                  25                  30
Ala Leu Lys Leu Ile Leu Ala Leu Leu Thr Phe Phe Thr Ile Thr Ile
        35                  40                  45
Thr Ile Asn Tyr Ile Lys Val Glu Asn Asn Leu Gln Ile Cys Gln Ser
    50                  55                  60
Lys Thr Glu Ser Asp Lys Glu Asp Ser Pro Ser Asn Thr Thr Ser Val
65                  70                  75                  80
Thr Thr Lys Thr Thr Leu Asp His Asp Ile Thr Gln Tyr Phe Lys Arg
                85                  90                  95
Leu Ile Gln Arg Tyr Thr Asp Ser Val Ile Asn Lys Asp Thr Cys Trp
                100                 105                 110
Lys Ile Ser Arg Asn Gln Cys Thr Asn Ile Thr Thr Tyr Lys Phe Leu
        115                 120                 125
Cys Phe Lys Pro Glu Asp Ser Lys Ile Asn Ser Cys Asp Arg Leu Thr
    130                 135                 140
Asp Leu Cys Arg Asn Lys Ser Lys Ser Ala Ala Glu Ala Tyr His Thr
145                 150                 155                 160
Val Glu Cys His Cys Ile Tyr Thr Ile Glu Trp Lys Cys Tyr His His
                165                 170                 175
Pro Ile Asp

<210> 88
<211> 177
<212> PRT
<213> human Metapneumo virus

<400> 88
Met Lys Thr Leu Asp Val Ile Lys Ser Asp Gly Ser Ser Glu Thr Cys

1                   5                   10                  15
Asn Gln Leu Lys Lys Ile Ile Lys Lys His Ser Gly Lys Val Leu Ile
                20                  25                  30
Ala Leu Lys Leu Ile Leu Ala Leu Leu Thr Phe Phe Thr Ala Thr Ile
        35                  40                  45
Thr Val Asn Tyr Ile Lys Val Glu Asn Asn Leu Gln Ala Cys Gln Pro
    50                  55                  60
Lys Asn Glu Ser Asp Lys Lys Val Thr Lys Pro Asn Thr Thr Ser Thr
65                  70                  75                  80
Thr Ile Arg Pro Thr Pro Asp Pro Thr Val Val His His Leu Lys Arg
                85                  90                  95
Leu Ile Gln Arg His Thr Asn Ser Val Thr Lys Asp Ser Asp Thr Cys
            100                 105                 110
Trp Arg Ile His Lys Asn Gln Arg Thr Asn Ile Lys Ile Tyr Lys Phe
        115                 120                 125
Leu Cys Ser Gly Phe Thr Asn Ser Lys Gly Thr Asp Cys Glu Glu Pro
    130                 135                 140
Thr Ala Leu Cys Asp Lys Lys Leu Lys Thr Ile Val Glu Lys His Arg
145                 150                 155                 160
Lys Ala Glu Cys His Cys Leu His Thr Thr Glu Trp Gly Cys Leu His
                165                 170                 175
Pro

<210> 89
<211> 177
<212> PRT
<213> human Metapneumo virus

<400> 89
Met Lys Thr Leu Asp Val Ile Lys Ser Asp Gly Ser Ser Glu Thr Cys
1                   5                   10                  15
Asn Gln Leu Lys Lys Ile Ile Lys Lys His Ser Gly Lys Leu Leu Ile
                20                  25                  30
Ala Leu Lys Leu Ile Leu Ala Leu Leu Thr Phe Phe Thr Val Thr Ile
        35                  40                  45
Thr Val Asn Tyr Ile Lys Val Glu Asn Asn Leu Gln Ala Cys Gln Leu
    50                  55                  60
Lys Asn Glu Ser Asp Lys Lys Asp Thr Lys Leu Asn Thr Thr Ser Thr
65                  70                  75                  80
Thr Ile Arg Pro Ile Pro Asp Leu Asn Ala Val Gln Tyr Leu Lys Arg
                85                  90                  95
Leu Ile Gln Lys His Thr Asn Phe Val Ile Lys Asp Arg Asp Thr Cys
            100                 105                 110
Trp Arg Ile His Thr Asn Gln Cys Thr Asn Ile Lys Ile Tyr Lys Phe
        115                 120                 125
Leu Cys Phe Gly Phe Met Asn Ser Thr Asn Thr Asp Cys Glu Glu Leu
    130                 135                 140
Thr Val Leu Cys Asp Lys Lys Ser Lys Thr Met Thr Glu Lys His Arg
145                 150                 155                 160
Lys Ala Glu Cys His Cys Leu His Thr Thr Glu Trp Trp Cys Tyr Tyr
                165                 170                 175
Leu

<210> 90
<211> 552
<212> DNA
<213> human Metapneumo virus

<400> 90
atgataacat tagatgtcat taaaagtgat gggtcttcaa aaacatgtac tcacctcaaa 60
aaaataatta aagaccactc tggtaaagtg cttattgtac ttaagttaat attagcttta 120
ctaacatttc tcacagtaac aatcaccatc aattatataa aagtggaaaa caatctgcaa 180
atatgccagt caaaaactga atcagacaaa aaggactcat catcaaatac cacatcagtc 240
acaaccaaga ctactctaaa tcatgatatc acacagtatt ttaaaagttt gattcaaagg 300
tatacaaact ctgcaataaa cagtgacaca tgctggaaaa taaacagaaa tcaatgcaca 360
aatataacaa catacaaatt tttatgtttt aaatctgaag acacaaaaac caacaattgt 420
gataaactga cagatttatg cagaaacaaa ccaaaaccag cagttggagt gtatcacata 480
gtagaatgcc attgtatata cacagttaaa tggaagtgct atcattaccc aaccgatgaa 540
acccaatcct aa 552

<210> 91
<211> 540
<212> DNA
<213> human Metapneumo virus

<400> 91
atgataacat tagatgtcat taaaagtgat gggtcttcaa aaacatgtac tcacctcaaa 60
aaaataatca aagaccattc tggtaaagtg cttattgcac ttaagttaat attagcttta 120
ctaacatttt tcacaataac aatcactata aattacataa aagtagaaaa caatctacaa 180
atatgccagt caaaaactga atcagacaaa gaagactcac catcaaatac cacatccgtc 240
acaaccaaga ctactctaga ccatgatata acacagtatt ttaaaagatt aattcaaagg 300
tatacagatt ctgtgataaa caaggacaca tgctggaaaa taagcagaaa tcaatgcaca 360
aatataacaa catataaatt tttatgcttt aaacctgagg actcaaaaat caacagttgt 420
gatagactga cagatctatg cagaaacaaa tcaaaatcag cagctgaagc atatcataca 480
gtagaatgcc attgcatata cacaattgag tggaagtgct atcaccaccc aatagattaa 540

<210> 92
<211> 534
<212> DNA
<213> human Metapneumo virus

<400> 92
atgaaaacat tagatgtcat aaaaagtgat ggatcctcag aaacgtgtaa tcaactcaaa 60
aaaataataa aaaaacactc aggtaaagtg cttattgcac taaaactgat attggcctta 120
ctgacatttt tcacagcaac aatcactgtc aactatataa aagtagaaaa caatttgcag 180
gcatgtcaac caaaaaatga atcagacaaa aaggtcacaa agccaaatac cacatcaaca 240
acaatcagac ccacacccga tccaactgta gtacatcatt tgaaaaggct gattcagaga 300
cacaccaact ctgtcacaaa agacagcgat acttgttgga gaatacacaa gaatcaacgt 360
acaaatataa aaatatacaa gttcttatgc tctgggttca caaattcaaa aggtacagat 420
tgtgaggaac caacagccct atgcgacaaa aagttaaaaa ccatagtaga aaaacataga 480
aaagcagaat gtcactgtct acatacaacc gagtgggggt gccttcatcc ctaa 534

<210> 93
<211> 534
<212> DNA
<213> human Metapneumo virus

<400> 93
atgaaaacat tagatgtcat aaaaagtgat ggatcctcag aaacatgtaa tcaactcaaa 60
aaaataataa aaaaacactc aggtaaattg cttattgcat taaaactgat attggcctta 120
ttgacgtttt tcacagtaac aattactgtt aactatataa aagtagaaaa caatttgcag 180
gcatgtcaat taaaaaatga atcagacaaa aaggacacaa agctaaatac cacatcaaca 240
acaatcagac ccattcctga tctaaatgca gtacagtact tgaaaaggct gattcagaaa 300
cacaccaact ttgtcataaa agacagagat acctgttgga gaatacacac gaatcaatgc 360
acaaatataa aaatatataa gttcttatgt ttcgggttta tgaattcaac aaatacagac 420
tgtgaagaac taacagtttt atgtgataaa aagtcaaaaa ccatgacaga aaaacatagg 480
aaagcagagt gtcactgtct acatacaacc gagtggtggt gttattatct ttaa 534

<210> 94
<211> 13294
<212> DNA
<213> human metapneumo virus

<220>
<221> misc_feature
<222> (0)...(0)
<223> human MPV protein

<400> 94
acgcgaaaaa aacgcgtata aattaaattc caaacaaaac gggacaaata aaaatgtctc 60
ttcaagggat tcacctaagt gatctatcat ataaacatgc tatattaaaa gagtctcaat 120
acacaataaa aagagatgta ggcaccacaa ctgcagtgac accttcatca ttacaacaag 180
aaataacact tttgtgtggg gaaatacttt acactaaaca cactgattac aaatatgctg 240
ctgagatagg aatacaatat atttgcacag ctctaggatc agaaagagta caacagattt 300
tgagaaactc aggtagtgaa gttcaggtgg ttctaaccaa aacatactcc ttagggaaag 360
gcaaaaacag taaaggggaa gagctgcaga tgttagatat acatggagtg gaaaagagtt 420
ggatagaaga aatagacaaa gaggcaagaa agacaatggt aactttgctt aaggaatcat 480
caggtaacat cccacaaaac cagagacctt cagcaccaga cacaccaata attttattat 540
gtgtaggtgc cctaatattc actaaactag catcaacaat agaagttgga ttagagacta 600
cagttagaag agctaataga gtgctaagtg atgcactcaa aagataccca aggatagata 660
taccaaagat tgctagatct ttttatgaac tatttgaaca aaaagtgtac tacagaagtt 720
tattcattga gtacggaaaa gctttaggct catcttcaac aggaagcaaa gcagaaagtt 780
tgtttgtaaa tatatttatg caagcttatg gagctggcca aacactgcta aggtggggtg 840
tcattgccag atcatccaac aacataatgc tagggcatgt atctgtgcaa tctgaattga 900
agcaagttac agaggtttat gacttggtga gagaaatggg tcctgaatct gggcttttac 960
atctaagaca aagtccaaag gcagggctgt tatcattggc caattgcccc aattttgcta 1020
gtgttgttct tggcaatgct tcaggtctag gcataatcgg aatgtacaga gggagagtac 1080
caaacacaga gctattttct gcagcagaaa gttatgccag aagcttaaaa gaaagcaata 1140
aaatcaactt ctcttcgtta gggcttacag atgaagaaaa agaagctgca gaacacttct 1200
taaacatgag tggtgacaat caagatgatt atgagtaatt aaaaaactgg gacaagtcaa 1260
aatgtcattc cctgaaggaa aggatattct gttcatgggt aatgaagcag caaaaatagc 1320
cgaagctttc cagaaatcac tgaaaaaatc aggtcacaag agaactcaat ctattgtagg 1380
ggaaaaagtt aacactatat cagaaactct agaactacct accatcagca aacctgcacg 1440
atcatctaca ctgctggaac caaaattggc atgggcagac aacagcggaa tcaccaaaat 1500
cacagaaaaa ccagcaacca aaacaacaga tcctgttgaa gaagaggaat tcaatgaaaa 1560
gaaagtgtta ccttccagtg atgggaagac tcctgcagag aaaaaatcaa agttttcaac 1620
cagtgtaaaa aagaaagttt cctttacatc aaatgaacca gggaaataca ccaaactaga 1680
gaaagatgcc ctagatttgc tctcagacaa tgaggaagaa gacgcagaat cctcaatcct 1740
aacttttgag gagaaagata catcatcact aagcattgaa gctagactag aatctataga 1800
agagaagttg agcatgatat taggactgct tcgtacactt aacattgcaa cagcaggacc 1860
aacagctgca cgagatggaa ttagggatgc aatgattggt ataagagaag agctaatagc 1920
agagataatt aaggaagcca agggaaaagc agctgaaatg atggaagaag agatgaatca 1980
aagatcaaaa ataggaaatg gcagtgtaaa actaaccgag aaggcaaaag agctcaacaa 2040
aattgttgaa gacgagagca caagcggtga atcagaagaa gaagaagaac caaaagaaac 2100
tcaggataac aatcaaggag aagatattta tcagttaatc atgtagttta ataaaaataa 2160
acaatgggac aagtcaagat ggagtcctat ctagtagaca cttatcaagg cattccatat 2220
acagctgctg ttcaagttga cctggtagaa aaagatttac tgccagcaag tttgacaata 2280
tggtttcctt tatttcaggc caacacacca ccagcagttc tgcttgatca gctaaaaacc 2340
ctgacaataa ccactctgta tgctgcatca cagaatggtc caatactcaa ggtaaatgca 2400
tctgcccaag gtgctgccat gtctgtactt cccaaaaaat tcgaggtaaa tgcaactgta 2460
gcacttgatg aatacagtaa acttgatttt gacaagctga cggtctgcga tgttaaaaca 2520
gtttatttga caactatgaa accgtacggg atggtgtcaa aatttgtgag ttcagccaaa 2580
tcagttggca aaaagacaca tgatctaatt gcactatgtg acttcatgga cctagagaaa 2640
aatatacctg tgacaatacc agcattcata aagtcagttt caatcaaaga gagtgaatca 2700
gccactgttg aagctgcaat aagcagcgaa gccgaccaag ccttgacaca agccaagatt 2760
gcgccctatg caggactaat tatgatcatg accatgaaca atccaaaagg tatattcaag 2820
aaactagggg ctggaacaca agtgatagta gagctggggg catatgttca ggctgagagc 2880
atcagtagga tctgcaagag ctggagtcac caaggaacaa gatacgtact aaaatccaga 2940
taaaaataac tgtcttaatc aataattgct tatataactc tagagattaa taagcttatt 3000
attatagtta tataaaaata aattagaatt agaagggcat caatagaaag cgggacaaat 3060

aaaaatgtct tggaaagtga tgatcatcat ttcgttactc ataacacccc agcacgggct 3120
aaaggagagt tatttggaag aatcatgtag tactataact gagggatacc tcagtgtttt 3180
aagaacaggc tggtacacta atgtcttcac attagaagtt ggtgatgttg aaaatcttac 3240
atgtactgat ggacctagct taatcaaaac agaacttgat ctaacaaaaa gtgctttaag 3300
ggaactcaaa acagtctctg ctgatcagtt ggcgagagag gagcaaattg aaaatcccag 3360
acaatcaaga tttgtcttag gtgcgatagc tctcggagtt gctacagcag cagcagtcac 3420
agcaggcatt gcaatagcca aaaccataag gcttgagagt gaggtgaatg caattaaagg 3480
tgctctcaaa caaactaatg aagcagtatc cacattaggg aatggtgtgc gggtcctagc 3540
cactgcagtg agagagctaa aagaatttgt gagcaaaaac ctgactagtg caatcaacag 3600
gaacaaatgt gacattgctg atctgaagat ggctgtcagc ttcagtcaat tcaacagaag 3660
atttctaaat gttgtgcggc agttttcaga caatgcaggg ataacaccag caatatcatt 3720
ggacctgatg actgatgctg agttggccag agctgtatca tacatgccaa catctgcagg 3780
gcagataaaa ctgatgttgg agaaccgcgc aatggtaagg agaaaaggat ttggaatcct 3840
gataggggtc tacggaagct ctgtgattta catggttcaa ttgccgatct ttggtgtcat 3900
agatacacct tgttggatca tcaaggcagc tccctcttgc tcagaaaaaa acgggaatta 3960
tgcttgcctc ctaagagagg atcaagggtg gtattgtaaa aatgcaggat ctactgttta 4020
ctacccaaat gaaaaagact gcgaaacaag aggtgatcat gtttttgtgg acacagcagc 4080
agggatcaat gttgctgagc aatcaagaga atgcaacatc aacatatcta ctaccaacta 4140
cccatgcaaa gtcagcacag gaagacaccc tataagcatg gttgcactat cacctctcgg 4200
tgctttggtg gcttgctata aaggggtaag ctgctcgatt ggcagcaatt gggttggaat 4260
catcaaacaa ttacccaaag gctgctcata cataaccaac caggatgcag acactgtaac 4320
aattgacaat accgtgtatc aactaagcaa agttgaaggt gaacagcatg taataaaagg 4380
gagaccagtt tcaagcagtt ttgatccaat caagtttcct gaggatcagt tcaatgttgc 4440
gcttgatcaa gtcttcgaaa gcattgagaa cagtcaggca ctagtggacc agtcaaacaa 4500
aattctaaac agtgcagaaa aaggaaacac tggtttcatt atcgtagtaa ttttggttgc 4560
tgttcttggt ctaaccatga tttcagtgag catcatcatc ataatcaaga aaacaaggaa 4620
gcccacagga gcacctccag agctgaatgg tgtcaccaac ggcggtttca taccacatag 4680
ttagttaatt aaaaaatggg acaaatcatc atgtctcgta aggctccatg caaatatgaa 4740
gtgcggggca aatgcaacag agggagtgat tgcaaattca atcacaatta ctggagttgg 4800
cctgatagat atttattgtt aagatcaaat tatctcttaa atcagctttt aagaaacaca 4860
gataaggctg atggtttgtc aataatatca ggagcaggta gagaagatag aactcaagac 4920
tttgttcttg gttctactaa tgtggttcaa gggtacattg atgacaacca aggaataacc 4980
aaggctgcag cttgctatag tctacacaac ataatcaagc aactacaaga aacagaagta 5040
agacaggcta gagacaacaa gctttctgat agcaaacatg tggcgctcca caacttgata 5100
ttatcctata tggagatgag caaaactcct gcatctctaa tcaacaacct aaagaaacta 5160
ccaagggaaa aactgaagaa attagcaaga ttaataattg atttatcagc aggaactgac 5220
aatgactctt catatgcctt gcaagacagt gaaagcacta atcaagtgca gtaaacatgg 5280
tcccaaattc attaccatag aggcagatga tatgatatgg actcacaaag aattaaaaga 5340
aacactgtct gatgggatag taaaatcaca caccaatatt tatagttgtt acttagaaaa 5400
tatagaaata atatatgtta aaacttactt aagttagtaa aaaataaaaa tagaatggga 5460
taaatgacaa tgaaaacatt agatgtcata aaaagtgatg gatcctcaga aacgtgtaat 5520
caactcaaaa aaataataaa aaaacactca ggtaaagtgc ttattgcact aaaactgata 5580
ttggccttac tgacattttt cacagcaaca atcactgtca actatataaa agtagaaaac 5640
aatttgcagg catgtcaacc aaaaaatgaa tcagacaaaa aggtcacaaa gccaaatacc 5700
acatcaacaa caatcagacc cacacccgat ccaactgtag tacatcattt gaaaaggctg 5760
attcagagac acaccaactc tgtcacaaaa gacagcgata cttgttggag aatacacaag 5820
aatcaacgta caaatataaa aatatacaag ttcttatgct ctgggttcac aaattcaaaa 5880
ggtacagatt gtgaggaacc aacagcccta tgcgacaaaa agttaaaaac catagtagaa 5940
aaacatagaa aagcagaatg tcactgtcta catacaaccg agtgggggtg ccttcatccc 6000
taaaataaca cggctttcaa cattaaaatc agaacaacct ccacccaggt ctatcaatac 6060
agtggtttag ccatttaaaa accgaatatt atctaggctg cacgacactt tgcaataata 6120
tgcaatagtc aatagttaaa ccactgctgc aaactcatcc ataatataat cactgagtaa 6180
tacaaaatca agaaaatggg acaagtggct atggaagtaa gagtggagaa cattcgagcg 6240
atagacatgt tcaaagcaaa gataaaaaac cgtataagaa gcagcaggtg ctatagaaat 6300
gctacactga tccttattgg actaacagcg ttaagcatgg cacttaatat tttcctgatc 6360
atcgatcatg caacattaag aaacatgatc aaaacagaaa actgtgctaa catgccgtcg 6420
gcagaaccaa gcaaaaagac cccaatgacc tccacagcag gcccaaacac caaacccaat 6480
ccacagcaag caacacagtg gaccacagag aactcaacat ccccagtagc aaccccagag 6540
ggccatccat acacagggac aactcaaaca tcagacacaa cagctcccca gcaaaccaca 6600
gacaaacaca cagcaccgct aaaatcaacc aatgaacaga tcacccagac aaccacagag 6660
aaaaagacaa tcagagcaac aacccaaaaa agggaaaaag gaaaagaaaa cacaaaccaa 6720
accacaagca cagctgcaac ccaaacaacc aacaccacca accaaatcag aaatgcaagt 6780

gagacaatca caacatccga cagacccaga actgacacca caacccaaag cagcgaacag 6840
acaacccggg caacagaccc aagctcccca ccacaccatg catagagagg tgcaaaactc 6900
aaatgagcac aacacacaaa catcccatcc aagtagttaa caaaaaacca caaaataacc 6960
ttgaaaacca aaaaaccaaa acataaaccc agacccagaa aaacatagac accatatgga 7020
aggttctagc atatgcacca atgagatggc atctgttcat gtatcaatag caccaccatc 7080
attcaaggaa taagaagagg cgaaaattta agggataaat gacaatggat cccttttgtg 7140
aatctactgt taatgtttat ctccctgatt catatctcaa aggagtaata tcttttagtg 7200
aaaccaatgc aattggatca tgtcttttga aaagacccta tctaaaaaat gacaacactg 7260
ccaaagttgc tgtagaaaac cctgttgttg aacatgtgag gcttagaaat gcagtcatga 7320
ccaaaatgaa gatatcagat tataaagtgg ttgaaccagt taatatgcag catgaaataa 7380
tgaaaaatat acatagttgt gagcttacat tattaaaaca attcttaacg agaagcaaaa 7440
acattagctc tctaaaatta aatatgatat gtgattggtt acagttaaaa tccacttcag 7500
ataacacatc aattctcaat tttatagatg tggagttcat acccgtttgg gtaagcaatt 7560
ggttcagtaa ctggtataat ctcaataaat taatcttaga gtttagaaga gaagaagtaa 7620
taagaactgg ttcaatttta tgtagatcac taggcaagtt agtttttatt gtatcatctt 7680
atggatgtgt agtaaaaagc aacaaaagta aaagagtgag ctttttcacc tataaccaac 7740
tgttaacatg gaaagatgtg atgttaagta gattcaatgc aaacttttgt atatgggtaa 7800
gtaacaacct gaacaaaaat caagaaggac taggacttag aagcaatctg caaggtatgt 7860
taaccaataa attatatgaa actgttgatt acatgctaag cctatgctgc aatgaaggat 7920
tctctctggt gaaagagttt gaaggattta ttatgagtga aattctaaaa attactgagc 7980
atgctcagtt cagtactagg tttaggaata ctttattgaa tgggttaact gaacaattat 8040
cagtgttgaa agctaagaac agatctagag ttcttggaac tatattagaa aacaacaatt 8100
accctatgta cgaagtagta cttaaattat taggggacac cttgaaaagc ataaagttat 8160
taattaacaa gaatttagaa aatgctgcag aattatatta tatattcaga atttttggac 8220
accctatggt agatgagagg gaagcaatgg atgctgttaa attaaacaat gagattacaa 8280
aaattcttaa attagagagt ttaacagaac taagaggagc atttatacta agaattataa 8340
aagggtttgt agacaataat aaaagatggc ctaaaattaa gaatttaaaa gtgctcagca 8400
aaagatgggc tatgtatttc aaagctaaaa gttaccctag ccaacttgag ctaagtgtac 8460
aagatttttt agaacttgct gcagtacaat ttgagcagga attctctgta cctgaaaaaa 8520
ccaaccttga gatggtatta aatgataaag caatatcacc tccaaaaaag ctaatatggt 8580
ctgtatatcc aaaaaactac ctgcctgaaa ctataaaaaa tcaatattta gaagaggctt 8640
tcaatgcaag tgacagccaa agaacaagga gagtcttaga attttactta aaagattgta 8700
aatttgatca aaaagaactt aaacgttatg taattaaaca agagtatctg aatgacaaag 8760
accacattgt ctcgttaact gggaaggaaa gagaattaag tgtaggtagg atgtttgcaa 8820
tgcaaccagg aaaacaaaga cagatacaga tattagctga gaaacttcta gctgataata 8880
ttgtaccttt tttcccagaa actttaacaa agtatggtga cttagatctc caaagaatta 8940
tggaaataaa atcagaactt tcttccatta aaactagaaa gaatgatagc tacaacaatt 9000
atattgcaag ggcctctata gtaacagact taagtaagtt caatcaggcc tttagatatg 9060
aaaccacagc tatatgtgca gatgtagctg atgagttaca tgggacacaa agcttattct 9120
gttggttaca tcttattgtt cccatgacta caatgatatg tgcatacaga catgcaccac 9180
cagaaacaaa aggggaatat gatatagaca aaatacaaga gcaaagcgga ttatacagat 9240
atcatatggg agggattgaa gggtggtgcc agaagttatg gacaatggaa gcaatatcct 9300
tgttagatgt agtatctgtg aagactcgct gtcagatgac ctctctatta aacggagaca 9360
atcagtcaat agatgttagt aaaccagtaa aattgtctga aggtatagat gaagtaaaag 9420
cagactatag cttagcaatt agaatgctta aagaaataag agatgcttat aaaaacattg 9480
gtcataaact caaagaaggt gaaacatata tatcaaggga tctccaattt ataagtaagg 9540
tgattcaatc tgaaggagtc atgcatccta cccctataaa aaagatatta agagtaggtc 9600
cttggataaa tacaatacta gatgatatta aaaccagtgc agaatcaata ggaagtctat 9660
gtcaagaact agaattcaga ggggagagta tactagttag cttgatatta aggaatttct 9720
ggctgtataa cttgtacatg tatgagtcaa aacagcaccc attagctggg aagcaactgt 9780
tcaagcaatt gaacaaaaca ttaacatctg tgcagagatt ttttgaactg aagaaagaaa 9840
atgatgtggt tgacctatgg atgaatatac caatgcagtt tggaggggga gatccagtag 9900
ttttttacag atctttttac agaaggactc ccgatttcct aactgaagca atcagccatg 9960
tggatttact gttaaaagtg tcaaacaata tcaaagatga gactaagata cgatttttca 10020
aagccttatt atctatagaa aagaatgaac gtgctacatt aacaacacta atgagagacc 10080
ctcaggcagt aggatcagaa cgacaagcta aggtaacaag tgatataaat agaacagcag 10140
ttaccagcat actgagtcta tctccgaatc agctcttctg tgatagtgct atacattata 10200
gtagaaatga ggaagaagtt gggatcattg cagacaacat aacacctgtc tatcctcatg 10260
ggctgagagt gctctatgaa tcactacctt ttcataaggc tgaaaaggtt gtcaatatga 10320
tatcaggcac aaagtctata actaatctat tacagagaac atctgctatc aatggtgaag 10380
atattgatag agcagtgtct atgatgttag agaacttagg gttgttatct agaatattgt 10440
cagtaataat taatagtata gaaataccaa tcaagtccaa tggcagattg atatgctgtc 10500

aaatttccaa gaccttgaga gaaaaatcat ggaacaatat ggaaatagta ggagtgacat 10560
ctcctagtat tgtgacatgt atggatgttg tgtatgcaac tagttctcat ttaaaaggaa 10620
taattattga aaaattcagt actgacaaga ccacaagagg tcagagggga ccaaaaagcc 10680
cctgggtagg atcaagcact caagagaaaa aattggttcc tgtttataat agacaaattc 10740
tttcaaaaca acaaaaagag caactggaag caatagggaa aatgaggtgg gtgtacaaag 10800
gaactccagg gctaagaaga ttgctcaaca agatttgcat aggaagctta ggtattagct 10860
ataaatgtgt gaaaccttta ttaccaagat tcatgagtgt aaacttctta cataggttat 10920
ctgttagtag tagacccatg gaattcccag cttctgttcc agcttacagg acaacaaatt 10980
accattttga cactagtcca atcaaccaag cattaagtga gaggttcggg aacgaagaca 11040
ttaatttagt gttccaaaat gcaatcagct gcggaattag tataatgagt gttgtagaac 11100
agttaactgg tagaagccca aaacaattag tcctaatccc tcaattagaa gagatagata 11160
ttatgcctcc tcctgtattt caaggaaaat tcaattataa actagttgat aagataacct 11220
ccgatcaaca catcttcagt cctgacaaaa tagacatatt aacactaggg aagatgctta 11280
tgcctaccat aaaaggtcaa aaaactgatc agttcttaaa taagagagaa aactattttc 11340
atggaaataa tttaattgaa tctttatctg cagcacttgc atgccactgg tgtgggatat 11400
taacagaaca gtgcatagaa aacaatatct ttaggaaaga ttggggtgat gggttcatct 11460
cagatcatgc cttcatggat ttcaaggtat ttctatgtgt atttaaaacc aaacttttat 11520
gtagttgggg atctcaagga aagaatgtaa aagatgaaga tataatagat gaatccattg 11580
acaaattatt aagaattgac aacacctttt ggagaatgtt cagcaaagtc atgtttgaat 11640
caaaagtcaa aaaaagaata atgttatatg atgtgaaatt cctatcatta gtaggttata 11700
taggatttaa aaactggttt atagaacagt taagagtggt agaattgcat gaggtacctt 11760
ggattgtcaa tgctgaagga gagttagttg aaattaaatc aatcaaaatt tatctgcagt 11820
taatagaaca aagtctatct ttgagaataa ctgtattgaa ttatacagac atggcacatg 11880
ctcttacacg attaattagg aaaaaattga tgtgtgataa tgcactcttt aatccaagtt 11940
catcaccaat gtttaatcta actcaggtta ttgatcccac aacacaacta gactattttc 12000
ctaggataat atttgagagg ttaaaaagtt atgataccag ttcagactac aacaaaggga 12060
agttaacaag gaattacatg acattattac catggcaaca cgtaaacagg tacaattttg 12120
tctttagttc tacaggttgt aaagtcagtt tgaagacatg catcgggaaa ttgataaagg 12180
atttaaatcc taaagttctt tactttattg gagaaggagc aggtaactgg atggcaagaa 12240
cagcatgtga atatcctgat ataaaatttg tatataggag tttaaaggat gaccttgatc 12300
accattaccc attagaatat caaagggtaa taggtgatct aaatagggtg atagatagtg 12360
gtgaaggatt atcaatggaa accacagatg caactcaaaa aactcattgg gacttgatac 12420
acagaataag taaagatgct ttattgataa cattgtgtga tgcagaattc aaaaacagag 12480
atgatttctt taagatggta atcctttgga gaaaacatgt attatcttgt agaatctgta 12540
cagcttatgg aacagatctt tacttatttg caaagtatca tgcggtggac tgcaatataa 12600
aattaccatt ttttgtaaga tctgtagcta cttttattat gcaaggaagc aaattatcag 12660
ggtcagaatg ttacatactt ttaacattag gtcatcacaa taatctaccc tgtcatggag 12720
aaatacaaaa ttccaaaatg agaatagcag tgtgtaatga tttctatgcc tcaaagaaac 12780
tggacaacaa atcaattgaa gcaaactgca aatctcttct atcaggattg agaataccta 12840
taaacaaaaa ggagttaaat agacaaaaga aattgttaac actacaaagt aaccattctt 12900
ctatagcaac agttggcggc agtaagatta tagaatccaa atggttaaag aataaagcaa 12960
gtacaataat tgattggtta gagcatattt tgaattctcc aaaaggtgaa ttaaactatg 13020
atttctttga agcattagag aacacatacc ccaatatgat caagcttata gataatttgg 13080
gaaatgcaga aataaagaaa ctaatcaagg tcactgggta tatgcttgtg agtaagaagt 13140
aataataatg ataatgatta accataatct cacacaactg agaaaataat cgtctaacag 13200
tttagttgat cattagttat ttaaaattat aaaatagtaa ctaactgata aaaaatcaga 13260
aattgaaatt gaatgtatac ggtttttttg ccgt 13294

<210> 95
<211> 13350
<212> DNA
<213> human metapneumo virus

<400> 95
gtataaatta gattccaaaa aaatatggga caagtgaaaa tgtctcttca agggattcac 60
ctgagtgatt tatcatacaa gcatgctata ttaaaagagt ctcagtacac aataaaaaga 120
gatgtgggta caacaactgc agtgacaccc tcatcattgc aacaagaaat aacactgttg 180
tgtggagaaa ttctgtatgc taaacatgct gactacaaat atgctgcaga aataggaata 240
caatatatta gcacagcttt aggatcagag agagtgcagc agattctgag gaactcaggc 300
agtgaagtcc aagtggtctt aaccagaacg tactctctgg ggaaaattaa aaacaataaa 360

ggagaagatt tacagatgtt agacatacac ggggtagaga agagctgggt agaagagata 420
gacaaagaag caaggaaaac aatggcaacc ttgcttaagg aatcatcagg taatatccca 480
caaaatcaga ggccctcagc accagacaca cccataatct tattatgtgt aggtgcctta 540
atattcacta aactagcatc aaccatagaa gtgggactag agaccacagt cagaagggct 600
aaccgtgtac taagtgatgc actcaagaga taccctagaa tggacatacc aaagattgcc 660
agatccttct atgacttatt tgaacaaaaa gtgtatcaca gaagtttgtt cattgagtat 720
ggcaaagcat taggctcatc atctacaggc agcaaagcag aaagtctatt tgttaatata 780
ttcatgcaag cttatggggc cggtcaaaca atgctaaggt ggggggtcat tgccaggtca 840
tccaacaata taatgttagg acatgtatcc gtccaagctg agttaaaaca ggtcacagaa 900
gtctatgact tggtgcgaga aatgggccct gaatctggac ttctacattt aaggcaaagc 960
ccaaaagctg gactgttatc actagccaac tgtcccaact ttgcaagtgt tgttctcgga 1020
aatgcctcag gcttaggcat aatcggtatg tatcgaggga gagtaccaaa cacagaatta 1080
ttttcagcag ctgaaagtta tgccaaaagt ttgaaagaaa gcaataaaat aaatttctct 1140
tcattaggac ttacagatga agagaaagag gctgcagaac atttcttaaa tgtgagtgac 1200
gacagtcaaa atgattatga gtaattaaaa aagtgggaca agtcaaaatg tcattccctg 1260
aaggaaaaga tattcttttc atgggtaatg aagcagcaaa attagcagaa gctttccaga 1320
aatcattaag aaaaccaggt cataaaagat ctcaatctat tataggagaa aaagtgaata 1380
ctgtatcaga aacattggaa ttacctacta tcagtagacc tgcaaaacca accataccgt 1440
cagaaccaaa gttagcatgg acagataaag gtggggcaac caaaactgaa ataaagcaag 1500
caatcaaagt catggatccc attgaagaag aagagtctac cgagaagaag gtgctaccct 1560
ccagtgatgg gaaaacccct gcagaaaaga aactgaaacc atcaactaac accaaaaaga 1620
aggtttcatt tacaccaaat gaaccaggga aatatacaaa gttggaaaaa gatgctctag 1680
atttgctctc agataatgaa gaagaagatg cagaatcttc aatcttaacc tttgaagaaa 1740
gagatacttc atcattaagc attgaggcca gattggaatc aatagaggag aaattaagca 1800
tgatattagg gctattaaga acactcaaca ttgctacagc aggacccaca gcagcaagag 1860
atgggatcag agatgcaatg attggcgtaa gagaggaatt aatagcagac ataataaagg 1920
aagctaaagg gaaagcagca gaaatgatgg aagaggaaat gagtcaacga tcaaaaatag 1980
gaaatggtag tgtaaaatta acagaaaaag caaaagagct caacaaaatt gttgaagatg 2040
aaagcacaag tggagaatcc gaagaagaag aagaaccaaa agacacacaa gacaatagtc 2100
aagaagatga catttaccag ttaattatgt agtttaataa aaataaacaa tgggacaagt 2160
aaaaatggag tcctacctag tagacaccta tcaaggcatt ccttacacag cagctgttca 2220
agttgatcta atagaaaagg acctgttacc tgcaagccta acaatatggt tccctttgtt 2280
tcaggccaac acaccaccag cagtgctgct cgatcagcta aaaaccctga caataaccac 2340
tctgtatgct gcatcacaaa atggtccaat actcaaagtg aatgcatcag cccaaggtgc 2400
agcaatgtct gtacttccca aaaaatttga agtcaatgcg actgtagcac tcgatgaata 2460
tagcaaactg gaatttgaca aactcacagt ctgtgaagta aaaacagttt acttaacaac 2520
catgaaacca tacgggatgg tatcaaaatt tgtgagctca gccaaatcag ttggcaaaaa 2580
aacacatgat ctaatcgcac tatgtgattt tatggatcta gaaaagaaca cacctgttac 2640
aataccagca ttcatcaaat cagtttcaat caaagagagt gagtcagcta ctgttgaagc 2700
tgctataagc agtgaagcag accaagctct aacacaggcc aaaattgcac cttatgcggg 2760
attaattatg atcatgacta tgaacaatcc caaaggcata ttcaaaaagc ttggagctgg 2820
gactcaagtc atagtagaac taggagcata tgtccaggct gaaagcataa gcaaaatatg 2880
caagacttgg agccatcaag ggacaagata tgtcttgaag tccagataac aaccaagcac 2940
cttggccaag agctactaac cctatctcat agatcataaa gtcaccattc tagttatata 3000
aaaatcaagt tagaacaaga attaaatcaa tcaagaacgg gacaaataaa aatgtcttgg 3060
aaagtggtga tcattttttc attgttaata acacctcaac acggtcttaa agagagctac 3120
ttagaagagt catgtagcac tataactgaa ggatatctca gtgttctgag gacaggttgg 3180
tacaccaatg tttttacact ggaggtaggc gatgtagaga accttacatg tgccgatgga 3240
cccagcttaa taaaaacaga attagacctg accaaaagtg cactaagaga gctcagaaca 3300
gtttctgctg atcaactggc aagagaggag caaattgaaa atcccagaca atctagattc 3360
gttctaggag caatagcact cggtgttgca actgcagctg cagttacagc aggtgttgca 3420
attgccaaaa ccatccggct tgaaagtgaa gtaacagcaa ttaagaatgc cctcaaaaag 3480
accaatgaag cagtatctac attggggaat ggagttcgtg tgttggcaac tgcagtgaga 3540
gagctgaaag attttgtgag caagaatcta acacgtgcaa tcaacaaaaa caagtgcgac 3600
attgctgacc tgaaaatggc cgttagcttc agtcaattca acagaaggtt cctaaatgtt 3660
gtgcggcaat tttcagacaa cgctggaata acaccagcaa tatctttgga cttaatgaca 3720
gatgctgaac tagccagagc tgtttccaac atgccaacat ctgcaggaca aataaaactg 3780
atgttggaga accgtgcaat ggtaagaaga aaagggttcg gattcctgat aggagtttac 3840
ggaagctccg taatttacat ggtgcaactg ccaatctttg gggttataga cacgccttgc 3900
tggatagtaa aagcagcccc ttcttgttca ggaaaaaagg gaaactatgc ttgcctctta 3960
agagaagacc aaggatggta ttgtcaaaat gcagggtcaa ctgtttacta cccaaatgaa 4020
aaagactgtg aaacaagagg agaccatgtc ttttgcgaca cagcagcagg aatcaatgtt 4080

gctgagcagt caaaggagtg caacataaac atatctacta ctaattaccc atgcaaagtt 4140
agcacaggaa gacatcctat cagtatggtt gcactatctc ctcttggggc tttggttgct 4200
tgctacaagg gagtgagctg ttccattggc agcaacagag tagggatcat caagcaactg 4260
aacaaaggct gctcttatat aaccaaccaa gacgcagaca cagtgacaat agacaacact 4320
gtataccagc taagcaaagt tgaaggcgaa cagcatgtta taaaaggaag gccagtgtca 4380
agcagctttg acccagtcaa gtttcctgaa gatcaattca atgttgcact tgaccaagtt 4440
ttcgagagca ttgagaacag tcaggccttg gtggatcaat caaacagaat cctaagcagt 4500
gcagagaaag gaaacactgg cttcatcatt gtaataattc taattgctgt ccttggctct 4560
accatgatcc tagtgagtgt ttttatcata ataaagaaaa caaagaaacc cacaggagca 4620
cctccagagc tgagtggtgt cacaaacaat ggcttcatac cacataatta gttaattaaa 4680
aataaagtaa attaaaataa attaaaatta aaaataaaaa tttgggacaa atcataatgt 4740
ctcgcaaggc tccgtgcaaa tatgaagtgc ggggcaaatg caatagagga agtgagtgca 4800
agtttaacca caattactgg agttggccag atagatactt attaataaga tcaaattatt 4860
tattaaatca acttttaagg aacactgata gagctgatgg cttatcaata atatcaggag 4920
caggcagaga agataggaca caagattttg tcctaggttc caccaatgtg gttcaaggtt 4980
atattgatga taaccaaagc ataacaaaag ctgcagcctg ttacagtcta cataatataa 5040
tcaaacaact acaagaagtt gaagttaggc aggctagaga taacaaacta tctgacagca 5100
aacatgtagc acttcacaac ttagtcctat cttatatgga gatgagcaaa actcctgcat 5160
ctttaatcaa caatctcaag agactgccga gagagaaact gaaaaaatta gcaaagctca 5220
taattgactt atcagcaggt gctgaaaatg actcttcata tgccttgcaa gacagtgaaa 5280
gcactaatca agtgcagtga gcatggtcca gttttcatta ctatagaggt tgatgacatg 5340
atatggactc acaaggactt aaaagaagct ttatctgatg ggatagtgaa gtctcatact 5400
aacatttaca attgttattt agaaaacata gaaattatat atgtcaaggc ttacttaagt 5460
tagtaaaaac acatcagagt gggataaatg acaatgataa cattagatgt cattaaaagt 5520
gatgggtctt caaaaacatg tactcacctc aaaaaaataa ttaaagacca ctctggtaaa 5580
gtgcttattg tacttaagtt aatattagct ttactaacat ttctcacagt aacaatcacc 5640
atcaattata taaaagtgga aaacaatctg caaatatgcc agtcaaaaac tgaatcagac 5700
aaaaaggact catcatcaaa taccacatca gtcacaacca agactactct aaatcatgat 5760
atcacacagt attttaaaag tttgattcaa aggtatacaa actctgcaat aaacagtgac 5820
acatgctgga aaataaacag aaatcaatgc acaaatataa caacatacaa atttttatgt 5880
tttaaatctg aagacacaaa aaccaacaat tgtgataaac tgacagattt atgcagaaac 5940
aaaccaaaac cagcagttgg agtgtatcac atagtagaat gccattgtat atacacagtt 6000
aaatggaagt gctatcatta cccaaccgat gaaacccaat cctaaatgtt aacaccagat 6060
taggatccat ccaagtctgt tagttcaaca atttagttat ttaaaaatat tttgaaaaca 6120
agtaagtttc tatgatactt cataataata agtaataatt aattgcttaa tcatcatcac 6180
aacattattc gaaaccataa ctattcaatt taaaaagtaa aaaacaataa catgggacaa 6240
gtagttatgg aggtgaaagt ggagaacatt cgaacaatag atatgctcaa agcaagagta 6300
aaaaatcgtg tggcacgcag caaatgcttt aaaaatgcct ctttggtcct cataggaata 6360
actacattga gtattgccct caatatctat ctgatcataa actataaaat gcaaaaaaac 6420
acatctgaat cagaacatca caccagctca tcacccatgg aatccagcag agaaactcca 6480
acggtcccca cagacaactc agacaccaac tcaagcccac agcatccaac tcaacagtcc 6540
acagaaggct ccacactcta ctttgcagcc tcagcaagct caccagagac agaaccaaca 6600
tcaacaccag atacaacaaa ccgcccgccc ttcgtcgaca cacacacaac accaccaagc 6660
gcaagcagaa caaagacaag tccggcagtc cacacaaaaa acaacccaag gacaagctct 6720
agaacacatt ctccaccacg ggcaacgaca aggacggcac gcagaaccac cactctccgc 6780
acaagcagca caagaaagag accgtccaca gcatcagtcc aacctgacat cagcgcaaca 6840
acccacaaaa acgaagaagc aagtccagcg agcccacaaa catctgcaag cacaacaaga 6900
atacaaagga aaagcgtgga ggccaacaca tcaacaacat acaaccaaac tagttaacaa 6960
aaaatacaaa ataactctaa gataaaccat gcagacacca acaatggaga agccaaaaga 7020
caattcacaa tctccccaaa aaggcaacaa caccatatta gctctgccca aatctccctg 7080
gaaaaaacac tcgcccatat accaaaaata ccacaaccac cccaagaaaa aaactgggca 7140
aaacaacacc caagagacaa ataacaatgg atcctctcaa tgaatccact gttaatgtct 7200
atcttcctga ctcatatctt aaaggagtga tttcctttag tgagactaat gcaattggtt 7260
catgtctctt aaaaagacct tacctaaaaa atgacaacac tgcaaaagtt gccatagaga 7320
atcctgttat cgagcatgtt agactcaaaa atgcagtcaa ttctaagatg aaaatatcag 7380
attacaagat agtagagcca gtaaacatgc aacatgaaat tatgaagaat gtacacagtt 7440
gtgagctcac attattaaaa cagtttttaa caaggagtaa aaatattagc actctcaaat 7500
taaatatgat atgtgattgg ctgcagttaa agtctacatc agatgatacc tcaatcctaa 7560
gttttataga tgtagaattt atacctagct gggtaagcaa ttggtttagt aattggtaca 7620
atctcaacaa gttgattctg gaattcagga aagaagaagt aataagaact ggttcaatct 7680
tgtgtaggtc attgggtaaa ttagtttttg ttgtatcatc atatggatgt atagtcaaga 7740
gcaacaaaag caaaagagtg agcttcttca catacaatca actgttaaca tggaaagatg 7800

tgatgttaag tagattcaat gcaaattttt gtatatgggt aagcaacagt ctgaatgaaa 7860
atcaagaagg gctagggttg agaagtaatc tgcaaggcat attaactaat aagctatatg 7920
aaactgtaga ttatatgctt agtttatgtt gcaatgaagg tttctcactt gtgaaagagt 7980
tcgaaggctt tattatgagt gaaattctta ggattactga acatgctcaa ttcagtacta 8040
gatttagaaa tactttatta aatggattaa ctgatcaatt aacaaaatta aaaaataaaa 8100
acagactcag agttcatggt accgtgttag aaaataatga ttatccaatg tacgaagttg 8160
tacttaagtt attaggagat actttgagat gtattaaatt attaatcaat aaaaacttag 8220
agaatgctgc tgaattatac tatatattta gaatattcgg tcacccaatg gtagatgaaa 8280
gagatgcaat ggatgctgtc aaattaaaca atgaaatcac aaaaatcctt aggtgggaga 8340
gcttgacaga actaagaggg gcattcatat taaggattat caaaggattt gtagacaaca 8400
acaaaagatg gcccaaaatt aaaaacttaa aagtgcttag taagagatgg actatgtact 8460
tcaaagcaaa aagttacccc agtcaacttg aattaagcga acaagatttt ttagagcttg 8520
ctgcaataca gtttgaacaa gagttttctg tccctgaaaa aaccaacctt gagatggtat 8580
taaatgataa agctatatca cctcctaaaa gattaatatg gtctgtgtat ccaaaaaatt 8640
acttacctga gaaaataaaa aatcgatatc tagaagagac tttcaatgca agtgatagtc 8700
tcaaaacaag aagagtacta gagtactatt tgaaagataa taaattcgac caaaaagaac 8760
ttaaaagtta tgttgttaaa caagaatatt taaatgataa ggatcatatt gtctcgctaa 8820
ctggaaaaga aagagaatta agtgtaggta gaatgtttgc tatgcaacca ggaaaacagc 8880
gacaaataca aatattggct gaaaaattgt tagctgataa tattgtacct tttttcccag 8940
aaaccttaac aaagtatggt gatctagatc ttcagagaat aatggaaatc aaatcggaac 9000
tttcttctat taaaactaga agaaatgata gttataataa ttacattgca agagcatcca 9060
tagtaacaga tttaagtaag ttcaaccaag cctttaggta tgaaactaca gcgatctgtg 9120
cggatgtagc agatgaacta catggaacac aaagcctatt ctgttggtta catcttatcg 9180
tccctatgac aacaatgata tgtgcctata gacatgcacc accagaaaca aaaggtgaat 9240
atgatataga taagatagaa gagcaaagtg gtttatatag atatcatatg ggtggtattg 9300
aaggatggtg tcaaaaactc tggacaatgg aagctatatc tctattagat gttgtatctg 9360
taaaaacacg atgtcaaatg acatctttat taaacggtga caaccaatca atagatgtaa 9420
gtaaaccagt taagttatct gagggtttag atgaagtgaa agcagattat agcttggctg 9480
taaaaatgtt aaaagaaata agagatgcat acagaaatat aggccataaa cttaaagaag 9540
gggaaacata tatatcaaga gatcttcagt ttataagtaa ggtgattcaa tctgaaggag 9600
taatgcatcc tacccctata aaaaagatct taagagtggg accatggata aacacaatat 9660
tagatgacat taaaaccagt gcagagtcaa tagggagtct atgtcaggaa ttagaattta 9720
ggggggaaag cataatagtt agtctgatat taaggaattt ttggctgtat aatttataca 9780
tgcatgaatc aaagcaacac cccctagcag ggaagcagtt attcaaacaa ctaaataaaa 9840
cattaacatc agtgcagaga ttttttgaaa taaaaaagga aaatgaagta gtagatctat 9900
ggatgaacat accaatgcag tttggaggag gagatccagt agtcttctat agatctttct 9960
atagaaggac ccctgatttt ttaactgaag caatcagtca tgtggatatt ctgttaagaa 10020
tatcagccaa cataagaaat gaagcgaaaa taagtttctt caaagcctta ctgtcaatag 10080
aaaaaaatga acgtgctaca ctgacaacac taatgagaga tcctcaagct gttggctcag 10140
agcgacaagc aaaagtaaca agtgatatca atagaacagc agttaccagc atcttaagtc 10200
tttctccaaa tcaacttttc agcgatagtg ctatacacta cagtagaaat gaagaagagg 10260
tcggaatcat tgctgacaac ataacacctg tttatcctca tggactgaga gttttgtatg 10320
aatcattacc ttttcataaa gctgaaaaag ttgtgaatat gatatcagga acgaaatcca 10380
taaccaactt attacagaga acatctgcta ttaatggtga agatattgac agagctgtat 10440
ccatgatgct ggagaaccta ggattattat ctagaatatt gtcagtagtt gttgatagta 10500
tagaaattcc aaccaaatct aatggtaggc tgatatgttg tcagatatct agaaccctaa 10560
gggagacatc atggaataat atggaaatag ttggagtaac atcccctagc atcactacat 10620
gcatggatgt catatatgca actagctctc atttgaaagg gataatcatt gaaaagttca 10680
gcactgacag aactacaaga ggtcaaagag gtccaaagag cccttgggta gggtcgagca 10740
ctcaagagaa aaaattagtt cctgtttata acagacaaat tctttcaaaa caacaaagag 10800
aacagctaga agcaattgga aaaatgagat gggtatataa agggacacca ggtttaagac 10860
gattactcaa taagatttgt cttggaagtt taggcattag ttacaaatgt gtaaaacctt 10920
tattacctag gtttatgagt gtaaatttcc tacacaggtt atctgtcagt agtagaccta 10980
tggaattccc agcatcagtt ccagcttata gaacaacaaa ttaccatttt gacactagtc 11040
ctattaatca agcactaagt gagagatttg ggaatgaaga tattaatttg gtcttccaaa 11100
atgcaatcag ctgtggaatt agcataatga gtgtagtaga acaattaact ggtaggagtc 11160
caaaacagtt agttttaata cctcaattag aagaaataga cattatgcca ccaccagtgt 11220
ttcaagggaa attcaattat aagctagtag ataagataac ttctgatcaa catatcttca 11280
gtccagacaa aatagatatg ttaacactgg ggaaaatgct catgcccact ataaaaggtc 11340
agaaaacaga tcagttcctg aacaagagag agaattattt ccatgggaat aatcttattg 11400
agtctttgtc agcagcgtta gcatgtcatt ggtgtgggat attaacagag caatgtatag 11460
aaaataatat tttcaagaaa gactggggtg acgggttcat atcggatcat gcttttatgg 11520

acttcaaaat attcctatgt gtctttaaaa ctaaactttt atgtagttgg gggtcccaag 11580
ggaaaaacat taaagatgaa gatatagtag atgaatcaat agataaactg ttaaggattg 11640
ataatacttt ttggagaatg ttcagcaagg ttatgtttga atcaaaggtt aagaaaagga 11700
taatgttata tgatgtaaaa tttctatcat tagtaggtta tatagggttt aagaattggt 11760
ttatagaaca gttgagatca gctgagttgc atgaggtacc ttggattgtc aatgccgaag 11820
gtgatctggt tgagatcaag tcaattaaaa tctatttgca actgatagag caaagtttat 11880
ttttaagaat aactgttttg aactatacag atatggcaca tgctctcaca agattaatca 11940
gaaagaagtt gatgtgtgat aatgcactat taactccgat tccatcccca atggttaatt 12000
taactcaagt tattgatcct acagaacaat tagcttattt ccctaagata acatttgaaa 12060
ggctaaaaaa ttatgacact agttcaaatt atgctaaagg aaagctaaca aggaattaca 12120
tgatactgtt gccatggcaa catgttaata gatataactt tgtctttagt tctactggat 12180
gtaaagttag tctaaaaaca tgcattggaa aacttatgaa agatctaaac cctaaagttc 12240
tgtactttat tggagaaggg gcaggaaatt ggatggccag aacagcatgt gaatatcctg 12300
acatcaaatt tgtatacaga agtttaaaag atgaccttga tcatcattat cctttggaat 12360
accagagagt tataggagaa ttaagcagga taatagatag cggtgaaggg ctttcaatgg 12420
aaacaacaga tgcaactcaa aaaactcatt gggatttgat acacagagta agcaaagatg 12480
ctttattaat aactttatgt gatgcagaat ttaaggacag agatgatttt tttaagatgg 12540
taattctatg gaggaaacat gtattatcat gcagaatttg cactacttat gggacagacc 12600
tctatttatt cgcaaagtat catgctaaag actgcaatgt aaaattacct ttttttgtga 12660
gatcagtagc cacctttatt atgcaaggta gtaaactgtc aggctcagaa tgctacatac 12720
tcttaacact aggccaccac aacaatttac cctgccatgg agaaatacaa aattctaaga 12780
tgaaaatagc agtgtgtaat gatttttatg ctgcaaaaaa acttgacaat aaatctattg 12840
aagccaactg taaatcactt ttatcagggc taagaatacc gataaataag aaagaattaa 12900
atagacagag aaggttatta acactacaaa gcaaccattc ttctgtagca acagttggag 12960
gtagcaaggt catagagtct aaatggttaa caaacaaggc aaacacaata attgattggt 13020
tagaacatat tttaaattct ccaaaaggtg aattaaatta tgattttttt gaagcattag 13080
aaaatactta ccctaatatg attaaactaa tagataatct agggaatgca gagataaaaa 13140
aactgatcaa agtaactgga tatatgcttg taagtaaaaa atgaaaaatg ataaaaatga 13200
taaaataggt gacaacttca tactattcca aagtaatcat ttgattatgc aattatgtaa 13260
tagttaatta aaaactaaaa atcaaaagtt agaaactaac aactgtcatt aagtttatta 13320
aaaataagaa attataattg gatgtatacg 13350

<210> 96
<211> 13215
<212> DNA
<213> human metapneumo virus

<400> 96
acgcgaaaaa aacgcgtata aattaagtta caaaaaacca tgggacaagt gaaaatgtct 60
cttcaaggga ttcacctgag tgatctatca tacaagcatg ctatattaaa agagtctcag 120
tatacaataa agagagatgt aggcacaaca accgcagtga caccctcatc attgcaacaa 180
gaaataacac tattgtgtgg agaaattcta tatgctaagc atgctgatta caaatatgct 240
gcagaaatag gaatacaata tattagcaca gctctaggat cagagagagt acagcagatt 300
ctaagaaact caggtagtga agtccaagtg gttttaacca gaacgtactc cttggggaaa 360
gttaaaaaca acaaaggaga agatttacag atgttagaca tacacggagt agagaaaagc 420
tgggtggaag agatagacaa agaagcaaga aaaacaatgg caactttgct taaagaatca 480
tcaggcaata ttccacaaaa tcagaggcct tcagcaccag acacacccat aatcttatta 540
tgtgtaggtg ccttaatatt taccaaacta gcatcaacta tagaagtggg attagagacc 600
acagtcagaa gagctaaccg tgtactaagt gatgcactca aaagataccc taggatggac 660
ataccaaaaa tcgctagatc tttctatgac ttatttgaac aaaaagtgta ttacagaagt 720
ttgttcattg agtatggcaa agcattaggc tcatcctcta caggcagcaa agcagaaagt 780
ttattcgtta atatattcat gcaagcttac ggtgctggtc aaacaatgct gaggtgggga 840
gtcattgcca ggtcatctaa caatataatg ttaggacatg tatctgttca agctgagtta 900
aaacaagtca cagaagtcta tgacctggtg cgagaaatgg gccctgaatc tgggctccta 960
catttaaggc aaagcccaaa agctggactg ttatcactag ccaattgtcc caactttgct 1020
agtgttgttc tcggcaatgc ctcaggctta ggcataatag gtatgtatcg cgggagagtg 1080
ccaaacacag aactattttc agcagcagaa agctatgcca agagtttgaa agaaagcaat 1140
aaaattaact tttcttcatt aggactcaca gatgaagaaa aagaggctgc agaacacttc 1200
ctaaatgtga gtgacgacag tcaaaatgat tatgagtaat taaaaaaatg ggacaagtca 1260
aaatgtcatt ccctgaagga aaagatattc ttttcatggg taatgaagca gcaaaattgg 1320

cagaagcttt tcaaaaatca ttaagaaaac ctaatcataa aagatctcaa tctattatag 1380
gagaaaaagt gaacactgta tctgaaacat tggaattacc tactatcagt agacctacca 1440
aaccgaccat attgtcagag ccgaagttag catggacaga caaaggtggg gcaatcaaaa 1500
ctgaagcaaa gcaaacaatc aaagttatgg atcctattga agaagaagag tttactgaga 1560
aaagggtgct gccctccagt gatgggaaaa ctcctgcaga aaagaagttg aaaccatcaa 1620
ccaacactaa aaagaaggtc tcatttacac caaatgaacc aggaaaatac acaaagttgg 1680
agaaagatgc tctagacttg ctttcagaca atgaagaaga agatgcagaa tcctcaatct 1740
taaccttcga agaaagagat acttcatcat taagcattga agccagacta gaatcgattg 1800
aggagaaatt aagcatgata ttagggctat taagaacact caacattgct acagcaggac 1860
ccacagcagc aagagatggg atcagagatg caatgattgg cataagggag gaactaatag 1920
cagacataat aaaagaagcc aagggaaaag cagcagaaat gatggaagaa gaaatgaacc 1980
agcggacaaa aataggaaac ggtagtgtaa aattaactga aaaggcaaag gagctcaaca 2040
aaattgttga agacgaaagc acaagtggtg aatccgaaga agaagaagaa ccaaaagaca 2100
cacaggaaaa taatcaagaa gatgacattt accagttaat tatgtagttt aataaaaata 2160
aaaatgggac aagtgaaaat ggagtcctat ctggtagaca cttatcaagg catcccttac 2220
acagcagctg ttcaagttga tctagtagaa aaggacctgt tacctgcaag cctaacaata 2280
tggttcccct tgtttcaggc caatacacca ccagcagttc tgcttgatca gctaaagact 2340
ctgactataa ctactctgta tgctgcatca caaagtggtc caatactaaa agtgaatgca 2400
tcagcccagg gtgcagcaat gtctgtactt cccaaaaagt ttgaagtcaa tgcgactgta 2460
gcacttgacg aatatagcaa attagaattt gacaaactta cagtctgtga agtaaaaaca 2520
gtttacttaa caaccatgaa accatatggg atggtatcaa agtttgtgag ctcggccaaa 2580
tcagttggca aaaaaacaca tgatctaatc gcattatgtg attttatgga tctagaaaag 2640
aacacaccag ttacaatacc agcatttatc aaatcagttt ctatcaagga gagtgaatca 2700
gccactgttg aagctgcaat aagcagtgaa gcagaccaag ctctaacaca agccaaaatt 2760
gcaccttatg cgggactgat catgattatg accatgaaca atcccaaagg catattcaag 2820
aagcttggag ctgggaccca agttatagta gaactaggag catatgtcca ggctgaaagc 2880
ataagtaaaa tatgcaagac ttggagccat caaggaacaa gatatgtgct gaagtccagt 2940
taacagccaa gcaacctggc caagaactac caactctatt ctatagacta aaaagtcgcc 3000
attttagtta tataaaaatc aagttagaat aagaattaaa tcaatcaaga acgggacaaa 3060
taaaaatgtc ttggaaagtg gtgatcattt tttcattgct aataacacct caacacggtc 3120
ttaaagagag ctacctagaa gaatcatgta gcactataac tgagggatat cttagtgttc 3180
tgaggacagg ttggtatacc aacgttttta cattagaggt gggtgatgta gaaaacctta 3240
catgttctga tggacctagc ctaataaaaa cagaattaga tctgaccaaa agtgcactaa 3300
gagagctcaa aacagtctct gctgaccaat tggcaagaga ggaacaaatt gagaatccca 3360
gacaatctag gtttgttcta ggagcaatag cactcggtgt tgcaacagca gctgcagtca 3420
cagcaggtgt tgcaattgcc aaaaccatcc ggcttgagag tgaagtcaca gcaattaaga 3480
atgccctcaa aacgaccaat gaagcagtat ctacattggg gaatggagtt cgagtgttgg 3540
caactgcagt gagagagcta aaagactttg tgagcaagaa tttaactcgt gcaatcaaca 3600
aaaacaagtg cgacattgat gacctaaaaa tggctgttag cttcagtcaa ttcaacagaa 3660
ggtttctaaa tgttgtgcgg caattttcag acaatgctgg aataacacca gcaatatctt 3720
tggacttaat gacagatgct gaactagcca gggccgtttc taacatgccg acatctgcag 3780
gacaaataaa attgatgttg gagaaccgtg cgatggtgcg aagaaagggg ttcggaatcc 3840
tgataggggt ctacgggagc tccgtaattt acacggtgca gctgccaatc tttggcgtta 3900
tagacacgcc ttgctggata gtaaaagcag ccccttcttg ttccgaaaaa aagggaaact 3960
atgcttgcct cttaagagaa gaccaagggt ggtattgtca gaatgcaggg tcaactgttt 4020
actacccaaa tgagaaagac tgtgaaacaa gaggagacca tgtcttttgc gacacagcag 4080
caggaattaa tgttgctgag caatcaaagg agtgcaacat caacatatcc actacaaatt 4140
acccatgcaa agtcagcaca ggaagacatc ctatcagtat ggttgcactg tctcctcttg 4200
gggctctggt tgcttgctac aaaggagtaa gctgttccat tggcagcaac agagtaggga 4260
tcatcaagca gctgaacaaa ggttgctcct atataaccaa ccaagatgca gacacagtga 4320
caatagacaa cactgtatat cagctaagca aagttgaggg tgaacagcat gttataaaag 4380
gcagaccagt gtcaagcagc tttgatccaa tcaagtttcc tgaagatcaa ttcaatgttg 4440
cacttgacca agtttttgag aacattgaaa acagccaggc cttagtagat caatcaaaca 4500
gaatcctaag cagtgcagag aaagggaata ctggctttat cattgtaata attctaattg 4560
ctgtccttgg ctctagcatg atcctagtga gcatcttcat tataatcaag aaaacaaaga 4620
aaccaacggg agcacctcca gagctgagtg gtgtcacaaa caatggcttc ataccacaca 4680
gttagttaat taaaaataaa ataaaatttg ggacaaatca taatgtctcg caaggctcca 4740
tgcaaatatg aagtgcgggg caaatgcaac agaggaagtg agtgtaagtt taaccacaat 4800
tactggagtt ggccagatag atacttatta ataagatcaa actatctatt aaatcagctt 4860
ttaaggaaca ctgatagagc tgatggccta tcaataatat caggcgcagg cagagaagac 4920
agaacgcaag attttgttct aggttccacc aatgtggttc aaggttatat tgatgataac 4980
caaagcataa caaaagctgc agcctgctac agtctacaca acataatcaa gcaactacaa 5040

gaagttgaag ttaggcaggc tagagatagc aaactatctg acagcaagca tgtggcactc 5100
cataacttaa tcttatctta catggagatg agcaaaactc ccgcatcttt aatcaacaat 5160
cttaaaagac tgccgagaga aaaactgaaa aaattagcaa agctgataat tgacttatca 5220
gcaggcgctg acaatgactc ttcatatgcc ctgcaagaca gtgaaagcac taatcaagtg 5280
cagtgagcat ggtcctgttt tcattactat agaggttgat gaaatgatat ggactcaaaa 5340
agaattaaaa gaagctttgt ccgatgggat agtgaagtct cacaccaaca tttacaattg 5400
ttatttagaa aacatagaaa ttatatatgt caaggcttac ttaagttagt aaaaacacac 5460
atcagagtgg gataagtgac aatgataaca ttagatgtca ttaaaagtga tgggtcttca 5520
aaaacatgta ctcacctcaa aaaaataatc aaagaccatt ctggtaaagt gcttattgca 5580
cttaagttaa tattagcttt actaacattt ttcacaataa caatcactat aaattacata 5640
aaagtagaaa acaatctaca aatatgccag tcaaaaactg aatcagacaa agaagactca 5700
ccatcaaata ccacatccgt cacaaccaag actactctag accatgatat aacacagtat 5760
tttaaaagat taattcaaag gtatacagat tctgtgataa acaaggacac atgctggaaa 5820
ataagcagaa atcaatgcac aaatataaca acatataaat ttttatgctt taaacctgag 5880
gactcaaaaa tcaacagttg tgatagactg acagatctat gcagaaacaa atcaaaatca 5940
gcagctgaag catatcatac agtagaatgc cattgcatat acacaattga gtggaagtgc 6000
tatcaccacc caatagatta aacccaattt tgaatgttaa aactagacta ggatccgtct 6060
aagactatca gttcaatagt ttagttattt aaaaatattt gagaacaggt aagtttctat 6120
ggcacttcat agcaataggt aataattaac agcttaatta taattaaaac attatttaaa 6180
accgtaacta tttaatttac aaagtaaaaa caaaaatatg ggacaagtag ttatggaggt 6240
gaaagtagag aacattcgag caatagacat gctcaaagca agagtgaaaa atcgtgtggc 6300
acgtagcaaa tgctttaaaa atgcttcttt aatcctcata ggaataacta cactgagtat 6360
agctctcaat atctatctga tcataaacta cacaatacaa aaaaccacat ccgaatcaga 6420
acaccacacc agctcaccac ccacagaacc caacaaggaa gcttcaacaa tctccacaga 6480
caacccagac atcaatccaa gctcacagca tccaactcaa cagtccacag aaaaccccac 6540
actcaacccc gcagcatcag cgagcccatc agaaacagaa ccagcatcaa caccagacac 6600
aacaaaccgc ctgtcctccg tagacaggtc cacagcacaa ccaagtgaaa gcagaacaaa 6660
gacaaaaccg acagtccaca caatcaacaa cccaaacaca gcttccagta cacaatcccc 6720
accacggaca acaacgaagg caatccgcag agccaccact ttccgcatga gcagcacagg 6780
aaaaagacca accacaacat tagtccagtc cgacagcagc accacaaccc aaaatcatga 6840
agaaacaggt tcagcgaacc cacaggcgtc tgcaagcaca atgcaaaact agcacaccaa 6900
taatataaaa ccaaattagt taacaaaaaa tgcgagatag ctctaaagca aaacatgtag 6960
gtaccaacaa tcaagaaacc aaaagacaac tcacaatctc cctaaaacag caacgacacc 7020
atgtcagctt tgctcaaatc tctctgggag aaacttctac ccacatacta acaacatcac 7080
aaccatctca agaaaagaaa ctgggcaaaa cagcatccaa gagacaaata gcaatggatc 7140
ctcttaatga atccactgtt aatgtctatc tccctgattc gtaccttaaa ggagtaattt 7200
cttttagtga aactaatgca attggttcat gtctcttaaa aagaccttac ttaaaaaatg 7260
acaacactgc aaaagttgcc atagagaatc ctgttattga gcatgtgaga ctcaaaaatg 7320
cagtcaattc taaaatgaaa atatcagatt acaaggtagt agagccagta aacatgcaac 7380
atgaaataat gaagaatgta cacagttgtg agctcacact attgaaacag tttttaacaa 7440
ggagtaaaaa cattagcact ctcaaattaa atatgatatg tgattggctg caattaaagt 7500
ctacatcaga tgatacctca atcctaagtt tcatagatgt agaatttata cctagttggg 7560
taagcaactg gtttagtaat tggtacaatc tcaataagtt aattttggaa ttcagaagag 7620
aggaagtaat aagaaccggt tcaatcttat gcaggtcatt gggtaaatta gtttttattg 7680
tatcatcata cggatgtatc gtcaagagca acaaaagcaa aagagtgagc ttcttcacat 7740
acaatcaact gttaacatgg aaagatgtga tgttaagtag atttaatgcg aatttttgta 7800
tatgggtaag caatagtctg aatgaaaatc aggaagggct agggttaaga agtaatctac 7860
aaggtatgtt aactaataaa ctatatgaaa ctgtagatta tatgctaagt ttatgttgca 7920
atgaaggttt ctcacttgta aaagagttcg aaggttttat tatgagtgaa atccttagga 7980
ttactgaaca tgctcaattc agtactagat ttagaaatac tttattaaat ggattaacag 8040
atcaattaac aaaattaaaa aataaaaaca gactcagagt tcatggtacc gtattagaaa 8100
ataatgatta tccaatgtat gaagttgtac ttaaattatt aggagatact ttgagatgta 8160
tcaaattatt aatcaataaa aacttagaga atgctgcaga attatactat atattcagaa 8220
tttttggtca tccaatggta gatgaaagag atgcaatgga tgctgtcaaa ttaaacaatg 8280
aaatcacaaa aatcctaagg ttggagagct tgacagaact aagaggagca ttcatattaa 8340
ggattatcaa aggatttgtg gacaacaaca aaaggtggcc caaaattaaa aatttaatag 8400
tgcttagcaa aagatggact atgtacttca aagctaaaaa ttatcccagt caactcgaat 8460
taagtgaaca agactttcta gagcttgctg caatacaatt tgaacaagag ttttctgttc 8520
ctgaaaaaac caatcttgag atggtattaa atgacaaagc catatcacct cctaaaagat 8580
taatatggtc tgtgtatcca aagaattact tacctgagac gataaaaaat cgatatttag 8640
aagaaacttt caatgcgagt gatagtctca aaacaagaag agtactagag tactatttaa 8700
aagacaataa atttgatcaa aaggaactta aaagttatgt agttagacaa gaatatttaa 8760

atgataagga gcacattgtc tcattaactg gaaaagaaag agaattaagt gtaggtagaa 8820
tgtttgctat gcaaccagga aaacagcgac aaatacaaat attggcagaa aaattgttag 8880
ctgataacat tgtacctttc ttcccggaaa ccttaacaaa gtatggtgat ctagatcttc 8940
agagaataat ggaaatcaaa tcagaacttt cttctatcaa aaccagaaga aatgacagtt 9000
ataataatta cattgcaaga gcatccatag taacagattt gagcaagttc aaccaagcct 9060
ttagatatga aactacagcg atctgtgcgg atgtagcaga cgaattacat ggaacacaaa 9120
gcttattctg ttggttacat cttatcgttc ctatgactac aatgatatgt gcctatagac 9180
atgcaccacc agaaacaaaa ggtgaatatg atatagataa gatagaagag caaagtggtc 9240
tatatagata tcacatgggc ggtattgaag gatggtgtca aaaactctgg acaatggaag 9300
ctatatcttt attggatgtt gtatctgtaa agacacggtg tcaaatgaca tctttattaa 9360
acggtgataa ccaatcaata gatgtaagta aaccagtcaa gttatctgaa ggtttagatg 9420
aagtgaaggc agattatcgc ttagcaataa aaatgctaaa agaaataaga gatgcataca 9480
gaaatatagg ccataaactt aaagaagggg aaacatatat atcaagggat cttcaattta 9540
taagcaaggt gattcaatct gaaggagtga tgcatcctac ccctataaaa aaggtcttga 9600
gagtaggacc atggataaac acaatattag atgacattaa aactagtgct gagtcaatag 9660
ggagtctatg tcaagaatta gaatttaggg gagaaagcat aatagttagt ctgatattaa 9720
gaaacttctg gctgtataac ttatacatgc atgaatcaaa gcaacatcct ttggcaggga 9780
aacagttatt caaacaacta aataaaacat taacatcagt gcagagattt tttgaaatta 9840
aaaaggaaaa tgaggtagta gatctatgga tgaacatacc aatgcaattt ggaggaggag 9900
atccagtagt cttctataga tctttctata gaaggacccc tgatttttta actgaggcaa 9960
tcagccatgt agatattctg ttaaaaatat cagctaacat aaaaaatgaa acgaaagtaa 10020
gtttcttcaa agccttacta tcaatagaaa aaaatgaacg tgctacactg acaacgctaa 10080
tgagagatcc tcaagctgtt ggatcagaac gacaagcaaa agtaacaagt gacatcaata 10140
gaacagcagt taccagtatc ttaagtcttt ccccaaatca acttttcagt gatagtgcta 10200
tacactatag caggaatgaa gaagaagtgg gaatcattgc agaaaacata acacctgttt 10260
atcctcatgg gctgagagta ttatatgaat cattgccctt tcacaaagct gaaaaagttg 10320
taaacatgat atcagggaca aaatctataa ccaacttatt acagagaaca tccgctatta 10380
atggtgaaga tattgacagg gctgtatcta tgatgttgga gaatctagga ttattatcta 10440
gaatattgtc agtagttgtt gatagtatag aaattccaat caaatctaat ggtaggctga 10500
tatgttgtca aatctctagg actttaagag agacatcatg gaataatatg gaaatagttg 10560
gagtaacatc tcctagcatc actacatgta tggatgtcat atatgcaact agttctcatt 10620
tgaaggggat aattatagaa aagttcagca ctgacagaac tacaaggggt caaagaggtc 10680
caaaaagccc ttgggtaggg tcgagtactc aagagaaaaa attagtacct gtttataaca 10740
gacaaattct ttcaaaacaa caaagagaac agctagaagc aattggaaaa atgagatggg 10800
tgtataaagg gacaccaggc ttgcgacgat tactcaacaa gatctgtctt gggagtttag 10860
gcattagtta caaatgtgta aaacctttat tacctaggtt tatgagtgta aatttcttac 10920
ataggttatc tgtcagtagt agacctatgg aattcccagc atcagttcca gcttatagaa 10980
caacaaatta ccatttcgac actagtccta ttaatcaagc actaagtgag agatttggga 11040
atgaagatat taacttggtc ttccaaaatg cgatcagctg tggaattagc ataatgagtg 11100
tagtagaaca attaacaggt agaagcccaa aacagttagt tttaataccc caattagaag 11160
aaatagacat tatgccacca ccagtgtttc aagggaaatt caattataaa ttagtagata 11220
agataacttc tgatcaacat atcttcagtc cggacaaaat agatatgtta acactaggga 11280
aaatgctcat gcctactata aaaggtcaga aaacagatca gttcttaaat aagagagaga 11340
attatttcca tgggaacaat cttattgagt ctttatcagc agcattagca tgtcattggt 11400
gtgggatatt aacagaacaa tgcatagaaa ataatatttt caagaaggac tggggtgacg 11460
ggtttatatc agatcatgct tttatggact tcaaaatatt cctatgtgtc tttaaaacta 11520
aacttttatg tagttgggga tcccaaggga aaaacattaa agatgaagat atagtagatg 11580
aatcaataga taaattgtta aggattgaca atactttttg gagaatgttc agcaaagtta 11640
tgtttgaacc aaaagttaag aaaaggataa tgttatatga tgtaaaattc ctatcactag 11700
taggctacat agggtttaag aactggttta tagagcagtt gagatcagct gaattgcatg 11760
aaataccttg gattgtcaat gccgaaggtg atttggttga gatcaagtca attaaaatct 11820
atttgcaact gatagaacaa agcttatttt taagaataac tgttttgaac tatacagata 11880
tggcacatgc tctcacacga ttaatcagaa agaagttaat gtgtgataat gcactgttaa 11940
ccccaatttc atccccaatg gttaacttaa ctcaagttat tgatcccaca acacaattag 12000
attacttccc caagataaca ttcgaaaggc taaaaaatta tgacacaagt tcaaattatg 12060
ctaaaggaaa gctaacaaga aattacatga tactattgcc atggcagcat gttaatagat 12120
ataactttgt ctttagttct actggatgta aagttagtct gaaaacatgt attggaaaac 12180
ttatgaaaga cttaaatcct aaagttttgt actttattgg agaaggagca ggaaattgga 12240
tggccagaac agcatgtgaa tatcctgata ttaaatttgt atatagaagt ctgaaagatg 12300
accttgatca tcattatcct ctggaatacc agagagtgat aggtgaatta agcagaatca 12360
tagatagtgg tgaaggactt tcaatggaaa caacagacgc aactcaaaaa actcattggg 12420
atttgataca cagggtaagc aaagatgctt tattaataac tttatgtgat gcagaattta 12480

aggacagaga tgattttttt aagatggtaa ttctatggag aaaacatgta ttatcatgca 12540
gaatttgcac tacttatggg acggacctct atttattcgc aaagtatcat gctaaagact 12600
gcaatgtaaa attacctttt tttgtgagat cagttgctac tttcattatg cagggtagta 12660
agctgtcagg ttcagaatgc tacatactct taacactagg ccaccacaac agtttacctt 12720
gccatggaga aatacaaaat tctaagatga aaatagcagt gtgtaatgat ttttatgctg 12780
caaaaaaact cgacaataaa tcaattgaag ctaattgtaa atcacttttg tcagggctaa 12840
gaatacctat aaataagaag gaactagata gacagagaag attattaaca ctacaaagca 12900
atcattcttc tgtggcaaca gttggcggta gcaagatcat agagtctaaa tggttaacaa 12960
acaaagcaag tacaataatt gattggttag aacatatttt aaattctcca aagggcgaat 13020
taaattatga tttttttgaa gcattggaga acacttaccc taatatgatt aaactaatag 13080
ataacttagg gaatgcagag attaaaaaac ttatcaaagt aacaggatac atgcttgtaa 13140
gtaaaaaatg aaaaatgatg aagatgacaa aatagatgac aacttcatac tattctaaat 13200
taattatttg attat 13215

<210> 97
<211> 13135
<212> DNA
<213> human metapneumo virus

<400> 97
acgcgaaaaa aacgcgtata aattaaattc caaacaaaac gggacaaata aaaatgtctc 60
ttcaagggat tcacctaagt gatctgtcat ataaacatgc tatattaaaa gagtctcaat 120
acacaataaa aagagatgta ggcaccacaa ctgcagtgac accttcatca ttgcagcaag 180
agataacact tttgtgtgga gagattcttt acactaaaca tactgattac aaatatgctg 240
cagagatagg gatacaatat atttgcacag ctctaggatc agaaagagta caacagattt 300
taagaaattc aggtagtgag gttcaggtgg ttctaaccaa gacatactct ttagggaaag 360
gtaaaaatag taaaggggaa gagttgcaaa tgttagatat acatggagtg gaaaagagtt 420
gggtagaaga aatagacaaa gaggcaagaa aaacaatggt gactttgcta aaggaatcat 480
caggcaacat cccacaaaac cagaggcctt cagcaccaga cacaccaata attttattgt 540
gtgtaggtgc tttaatattc actaaactag catcaacaat agaagttgga ctagagacta 600
cagttagaag ggctaacaga gtgttaagtg atgcgctcaa aagataccct agggtagata 660
taccaaagat tgctagatct ttttatgaac tatttgagca gaaagtgtat tacaggagtc 720
tattcattga gtatgggaaa gctttaggct catcttcaac aggaagcaaa gcagaaagtt 780
tgtttgtaaa tatatttatg caagcttatg gagccggtca gacaatgcta aggtggggtg 840
tcattgccag atcatctaac aacataatgc tagggcatgt atctgtgcaa gctgaattga 900
aacaagttac agaggtttat gatttggtaa gagaaatggg tcctgaatct gggcttttac 960
atctaagaca aagtccaaag gcaggactgt tatcgttggc taattgcccc aattttgcta 1020
gtgttgttct tggtaatgct tcaggtctag gtataatcgg aatgtacagg ggaagagtgc 1080
caaacacaga gctattttct gcagcagaaa gttatgccag aagcttaaaa gaaagcaaca 1140
aaatcaactt ctcctcatta gggctcacag acgaagaaaa agaagctgca gaacacttct 1200
taaacatgag tgatgacaat caagatgatt atgagtaatt aaaaaactgg gacaagtcaa 1260
aatgtcattc cctgaaggaa aagatatcct gttcatgggt aatgaagcag caaaaatagc 1320
agaagctttc cagaaatcac taaaaagatc aggtcacaaa agaacccagt ctattgtagg 1380
ggaaaaagta aacactatat cagaaactct agagctacct accatcagca aacctgcacg 1440
atcatctaca ctgctagagc caaaattggc atgggcagac agcagcggag ccaccaaaac 1500
cacagaaaaa caaacaacca aaacaacaga tcctgttgaa gaagaggaac tcaatgaaaa 1560
gaaggtatca ccttccagtg atgggaagac tcctgcagag aaaaaatcaa aatctccaac 1620
caatgtaaaa aagaaagttt ccttcacatc aaatgaacca gggaaatata ctaaactaga 1680
aaaagatgcc ctagatttgc tctcagacaa tgaggaagaa gacgcagagt cctcaatcct 1740
aacctttgaa gagagagaca catcatcact aagcattgag gctagactag aatcaataga 1800
agagaagcta agcatgatat taggactgct tcgtacactt aacattgcaa cagcaggacc 1860
aacggctgca agggatggaa tcagagatgc aatgattggt ataagagaag aactaatagc 1920
agaaataata aaagaagcaa agggaaaagc agccgaaatg atggaagagg aaatgaatca 1980
aaggtcaaaa ataggtaatg gcagtgtaaa actaaccgag aaggcaaaag aacttaataa 2040
aattgttgaa gacgagagca caagtggtga atcagaagaa gaagaagaac caaaagaaac 2100
tcaggataac aatcaaggag aagatatcta ccagttaatc atgtagttta ataaaaataa 2160
acaatgggac aagtcaagat ggagtcctat ctagtggaca cttatcaagg cattccctac 2220
acagctgctg ttcaagttga tctggtagaa aaagacttac taccagcaag tttgacaata 2280
tggtttcctc tattccaagc caacacacca ccagcggttt tgctcgatca gctaaaaacc 2340
ttgactataa caactctgta tgctgcatca cagaatggtc caatactcaa agtaaatgca 2400

tcagctcagg gtgctgctat gtctgtactt cccaaaaaat tcgaagtaaa tgcaactgtg 2460
gcacttgatg aatacagcaa acttgacttt gacaagttaa cggtttgcga tgttaaaaca 2520
gtttatttga caaccatgaa gccatatggg atggtgtcaa aatttgtgag ttcagccaaa 2580
tcagttggca aaaagacaca tgatctaatt gcactgtgtg acttcatgga cctagagaaa 2640
aatatacctg tgacaatacc agcattcata aagtcagttt caatcaaaga gagtgagtca 2700
gccactgttg aagctgcaat aagcagtgag gccgaccaag cattaacaca agccaaaatt 2760
gcaccctatg caggactaat catgatcatg accatgaaca atccaaaagg tatattcaag 2820
aaactaggag ctggaacaca agtgatagta gagctagggg catatgttca agccgagagc 2880
atcagcagga tctgcaagag ctggagtcac caaggaacaa gatatgtact aaaatccaga 2940
taaaaataac tgtcctaatc aataattgct tatataatcc taaagatcaa tgagcttatt 3000
attatagtta tataaaaata atttagaact agaaaggtat taatagaaag cgggacaagt 3060
aaaaatgtct tggaaagtga tgattatcat ttcgttactc ataacacctc agcacggact 3120
aaaagaaagt tatttagaag aatcatgtag tactataact gaaggatatc tcagtgtttt 3180
aagaacaggt tggtacacca atgtctttac attagaagtt ggtgatgttg aaaatcttac 3240
atgtactgat ggacctagct taatcaaaac agaacttgac ctaaccaaaa gtgctctgag 3300
agaactcaaa acagtttctg ctgatcagtt agcgagagaa gaacaaattg aaaatcccag 3360
acaatcaagg tttgtcctag gtgcaatagc tcttggagtt gccacagcag cagcagtcac 3420
agcaggcatt gcaatagcca aaaccataag acttgagagt gaagtgaatg caatcaaagg 3480
tgctctcaaa acaaccaacg aggcagtatc cacactagga aatggagtgc gagtcctagc 3540
cactgcagta agagagctga aagaatttgt gagcaaaaac ctgactagtg cgatcaacaa 3600
gaacaaatgt gacattgctg atctgaagat ggctgtcagc ttcagtcaat tcaacagaag 3660
attcctaaat gttgtgcggc agttttcaga caatgcaggg ataacaccag caatatcatt 3720
ggacctaatg actgatgctg agctggccag agctgtatca tacatgccaa catctgcagg 3780
acagataaaa ctaatgttag agaaccgtgc aatggtgagg agaaaaggat ttggaatctt 3840
gataggggtc tacggaagct ctgtgattta catggtccag ctgccgatct ttggtgtcat 3900
agatacacct tgttggataa tcaaggcagc tccctcttgt tcagaaaaag atggaaatta 3960
tgcttgcctc ctaagagagg atcaagggtg gtattgcaaa aatgcaggat ccactgttta 4020
ctacccaaat gaaaaagact gcgaaacaag aggtgatcat gttttttgtg acacagcagc 4080
agggatcaat gttgctgagc aatcaagaga atgcaacatc aacatatcta ccaccaacta 4140
cccatgcaaa gtcagcacag gaagacaccc tatcagcatg gttgcactat cacctctcgg 4200
tgctttggta gcttgctaca agggggttag ctgctcgatt ggcagtaatc gggttggaat 4260
aatcaaacaa ctacctaaag gctgctcata cataactaac caggacgcag acactgtaac 4320
aattgacaac actgtgtatc aactaagcaa agttgagggt gaacagcatg taataaaagg 4380
gagaccagtt tcaagcagtt ttgatccaat caggtttcct gaggatcagt tcaatgttgc 4440
gcttgatcaa gtctttgaaa gcattgaaaa cagtcaagca ctagtggacc agtcaaacaa 4500
aattctgaac agtgcagaaa aaggaaacac tggtttcatt attgtaataa ttttgattgc 4560
tgttcttggg ttaaccatga tttcagtgag catcatcatc ataatcaaaa aaacaaggaa 4620
gcccacaggg gcacctccag agctgaatgg tgttaccaac ggcggtttta taccgcatag 4680
ttagttaatt aaaaaatggg acaaatcatc atgtctcgca aagctccatg caaatatgaa 4740
gtacggggca agtgcaacag gggaagtgag tgcaaattca accacaatta ctggagctgg 4800
cctgataggt atttattgtt aagatcaaat tatctcttga atcagctttt aagaaacact 4860
gataaggctg atggtttgtc aataatatca ggagcaggta gagaagatag gactcaagac 4920
tttgttcttg gttctactaa tgtggttcaa gggtacattg ataacaatca aggaataaca 4980
aaggctgcag cttgctatag tctacataac ataataaaac agctacaaga aatagaagta 5040
agacaggcta gagataataa gctttctgac agcaaacatg tggcacttca caacttgata 5100
ttatcctata tggagatgag caaaactcct gcatccctga ttaataacct aaagaaacta 5160
ccaagagaaa aactgaagaa attagcgaaa ttaataattg atttatcagc aggaactgat 5220
aatgactctt catatgcctt gcaagacagt gaaagcacta atcaagtgca gtaagcatgg 5280
tcccaaattc attaccatag aggcagatga tatgatatgg acacacaaag aattaaagga 5340
gacactgtct gatgggatag taaaatcaca caccaatatt tacagttgtt atttagaaaa 5400
tatagaaata atatatgtta aagcttactt aagttagtaa aaaataaata gaatgggata 5460
aatgacaatg aaaacattag atgtcataaa aagtgatgga tcctcagaaa catgtaatca 5520
actcaaaaaa ataataaaaa aacactcagg taaattgctt attgcattaa aactgatatt 5580
ggccttattg acgtttttca cagtaacaat tactgttaac tatataaaag tagaaaacaa 5640
tttgcaggca tgtcaattaa aaaatgaatc agacaaaaag gacacaaagc taaataccac 5700
atcaacaaca atcagaccca ttcctgatct aaatgcagta cagtacttga aaaggctgat 5760
tcagaaacac accaactttg tcataaaaga cagagatacc tgttggagaa tacacacgaa 5820
tcaatgcaca aatataaaaa tatataagtt cttatgtttc gggtttatga attcaacaaa 5880
tacagactgt gaagaactaa cagttttatg tgataaaaag tcaaaaacca tgacagaaaa 5940
acataggaaa gcagagtgtc actgtctaca tacaaccgag tggtggtgtt attatcttta 6000
agagaaaact cggctttcaa cattaaaatc agaacaaatc ctatccagat ctattaatat 6060
aatagtttag tcattcaaaa actctaaata ttgtctagac ttcacaacac tttgcggtca 6120

tatgcaataa tcaatggtca aaccactgtt gcaaactcac ccataatata atcactgagt 6180
aatacaaaac aagaaaatgg gacaagtggc catggaagta agagtggaga acattcgggc 6240
aatagacatg ttcaaagcaa aaatgaaaaa ccgtataaga agtagcaagt gctatagaaa 6300
tgctacactg atccttattg gattaacagc attaagtatg gcacttaata tttttttaat 6360
cattgattat gcaatgttaa aaaacatgac caaagtggaa cactgtgtta atatgccgcc 6420
ggtagaacca agcaagaaga ccccaatgac ctctgcagta gacttaaaca ccaaacccaa 6480
tccacagcag gcaacacagt tggccgcaga ggattcaaca tctctagcag caacctcaga 6540
ggaccatcta cacacaggga caactccaac accagatgca acagtctctc agcaaaccac 6600
agacgagtac acaacattgc tgagatcaac caacagacag accacccaaa caaccacaga 6660
gaaaaagcca accggagcaa caaccaaaaa agaaaccaca actcgaacta caagcacagc 6720
tgcaacccaa acactcaaca ctaccaacca aactagctat gtgagagagg caaccacaac 6780
atccgccaga tccagaaaca gtgccacaac tcaaagcagc gaccaaacaa cccaggcagc 6840
agacccaagc tcccaaccac accatacaca gaaaagcaca acaacaacat acaacacaga 6900
cacatcctct ccaagtagtt aacaaaaaaa ctataaaata atcatgaaaa ccgaaaaact 6960
agaaaagtta atttgaactc agaaaagaac acaaacacta tatgaattgt ttgagcgtat 7020
atactaatga aatagcatct gtttgtgcat caataatacc atcattattt aagaaataag 7080
aagaagctaa aattcaaggg acaaataaca atggatccat tttgtgaatc cactgtcaat 7140
gtttatcttc ctgactcata tctcaaagga gtaatatctt tcagtgaaac caatgcaatt 7200
ggctcatgcc ttttgaaaag accctatcta aaaaaagata acactgctaa agttgctgta 7260
gaaaaccctg ttgttgaaca tgtcaggctt agaaatgcag tcatgaccaa aatgaagata 7320
tcagattata aagtggttga accaattaat atgcagcatg aaataatgaa aaatatacac 7380
agttgtgagc tcacattatt aaaacaattc ttaacaagaa gtaaaaacat tagctctcta 7440
aaattaagta tgatatgtga ttggttacag ttaaaatcca cctcagataa cacatcaatt 7500
cttaatttta tagatgtgga gtttatacct gtttgggtga gcaattggtt tagtaactgg 7560
tataatctca ataaattaat cttagagttt agaagagagg aagtaataag aactggttca 7620
attttatgta gatcactagg caagttagtt ttcattgtat catcttatgg gtgtgtagta 7680
aaaagcaaca aaagtaaaag agtaagtttt ttcacatata accaactgtt aacatggaaa 7740
gatgtgatgt taagtaggtt caatgcaaac ttttgtatat gggtaagtaa caacctgaac 7800
aaaaatcaag aaggactagg atttagaagt aatctgcaag gtatgttaac caataaatta 7860
tatgaaactg ttgattatat gttaagtcta tgtagtaatg aagggttctc actagtgaaa 7920
gagttcgaag gctttattat gagtgaaatt cttaaaatta ctgagcatgc tcaattcagt 7980
actaggttta ggaatacttt attaaatggg ttgactgaac aattatcaat gttgaaagct 8040
aaaaacagat ctagagttct tggcactata ttagaaaaca atgattaccc catgtatgaa 8100
gtagtactta aattattagg ggacactttg aaaagtataa aattattaat taacaagaat 8160
ttagaaaatg ctgcagaatt atattatata ttcagaattt ttggacaccc tatggtagat 8220
gagagggaag caatggatgc tgttaaatta aataatgaga ttacaaaaat tcttaaactg 8280
gagagcttaa cagaactaag aggagcattt atactaagaa ttataaaagg gtttgtagat 8340
aataataaaa gatggcctaa aattaagaat ttaaaagtgc tcagtaaaag atgggttatg 8400
tatttcaaag ccaaaagtta ccctagccaa cttgagctaa gtgtacaaga ttttttagaa 8460
cttgctgcag tacaattcga acaggaattt tctgtccctg aaaaaaccaa ccttgagatg 8520
gtattaaatg ataaagcaat atctcctcca aaaaagttaa tatggtcggt atatccaaaa 8580
aattatctac ctgaaattat aaaaaatcaa tatttagaag aggtcttcaa tgcaagtgac 8640
agtcaaagaa cgaggagagt cttagaattt tacttaaaag attgcaaatt tgatcaaaaa 8700
gaccttaaac gttatgtact taaacaagag tatctaaatg acaaagacca cattgtctca 8760
ttaactggga aggaaagaga attaagtgta ggcaggatgt ttgcaatgca accaggcaaa 8820
caaagacaaa tacagatact agctgagaaa cttctagctg ataatattgt accctttttc 8880
ccagaaactt taacaaagta tggtgacttg gatctccaaa gaattatgga aatgaaatca 8940
gaactttctt ccattaaaac taggaagaat gatagttaca acaattatat tgcaagagcc 9000
tccatagtaa cagacctaag taaattcaat caagccttta gatatgaaac cacagctatc 9060
tgtgcagatg tagcagatga gttacatggt acgcaaagct tattttgttg gttacatctt 9120
attgttccca tgaccacaat gatatgtgca tacagacatg caccaccaga aacaaagggg 9180
gagtatgaca tagacaaaat agaagagcaa agtgggctat acagatatca tatgggaggg 9240
attgaagggt ggtgtcagaa gttatggaca atggaagcga tatccttgtt agatgtagta 9300
tctgttaaga ctcgttgtca gatgacctct ctattaaacg gagacaatca atcaatagat 9360
gtcagtaaac cagtaaaatt gtctgaaggt atagatgaag taaaagcaga ttatagctta 9420
gcaattaaaa tgcttaaaga gataagagat gcctataaaa acattggcca taaactcaaa 9480
gaaggtgaaa catatatatc aagagatctc caatttataa gtaaggtgat tcaatctgag 9540
ggggtcatgc atcctacccc cataaaaaag atattaaggg taggtccctg gataaataca 9600
atactagatg acattaaaac cagtgcagaa tcaataggga gtctgtgtca agaactagag 9660
ttcagaggag aaagtatgct agttagcttg atattaagga atttctggct gtataactta 9720
tacatgcatg agtcaaaaca gcatccgtta gctggaaaac aactgtttaa gcaattgaac 9780
aaaacactaa catctgtgca aagatttttt gagctgaaga aagaaaatga tgtggttgac 9840

ctatggatga atataccaat gcagtttgga gggggagacc cagtagtttt ttacagatct 9900
ttttacagaa ggactcctga tttcctgact gaagcaatca gccatgtgga tttactgtta 9960
aaagtttcga acaatattaa aaatgagact aagatacgat tctttaaagc cttattatct 10020
atagaaaaga atgaacgtgc aacattaaca acactaatga gagaccccca ggcggtagga 10080
tcggaaagac aagctaaggt aacaagtgat ataaatagaa cagcagttac tagcatactg 10140
agtctatctc cgaatcagct cttttgtgat agtgctatac actatagcag aaatgaagaa 10200
gaagtcggga tcattgcaga caacataaca cctgtttatc ctcacggatt gagagtgctc 10260
tatgaatcac taccttttca taaggctgaa aaggttgtca atatgatatc aggtacaaag 10320
tctataacta acctattgca gagaacatct gctatcaatg gtgaagatat tgatagagca 10380
gtgtctatga tgttagagaa cttagggttg ttatctagga tattgtcagt aataattaat 10440
agtatagaaa taccaattaa gtccaatggc agattgatat gctgtcaaat ttctaagact 10500
ttgagagaaa aatcatggaa caatatggaa atagtaggag tgacatctcc aagtattgta 10560
acatgtatgg atgttgtgta tgcaactagt tctcatttaa aaggaataat tattgaaaaa 10620
ttcagtactg acaagaccac aagaggtcag aggggaccaa aaagcccctg ggtaggatca 10680
agcactcaag agaaaaaatt agttcctgtt tataatagac aaattctttc aaaacaacaa 10740
aaggagcaac tggaagcaat aggaaaaatg aggtgggtgt ataaaggaac tccagggcta 10800
agaagattgc tcaataagat ttgcatagga agtttaggta ttagctataa atgtgtaaaa 10860
cctctattac caagattcat gagtgtaaac ttcttacata ggttatctgt tagtagcaga 10920
cccatggaat tcccagcttc tgttccagct tataggacaa caaattacca ctttgacact 10980
agtccaatca accaagcatt aagtgagagg ttcgggaacg aagacattaa tctagtgttc 11040
caaaatgcaa tcagctgcgg aattagtata atgagtgttg tagaacagtt aactggtaga 11100
agcccaaaac aattagtctt aatcccccaa ttagaagaga tagatattat gcctcctcct 11160
gtatttcaag gaaaattcaa ttataaacta gttgataaaa taacctctga tcaacacatc 11220
ttcagtcctg acaaaataga catattaaca ctagggaaga tgcttatgcc tactataaaa 11280
ggtcaaaaaa ctgatcagtt cttaaataag agagaaaact atttccatgg aaataattta 11340
attgaatctt tatctgcagc acttgcatgc cactggtgtg gaatattaac agaacagtgt 11400
gtagaaaaca atatctttag gaaagactgg ggtgatgggt tcatatcaga tcatgccttc 11460
atggatttca agatatttct atgtgtattt aaaaccaaac ttttatgtag ttggggatcc 11520
caagggaaaa atgtaaaaga tgaagatata atagatgaat ccattgacaa attattaaga 11580
attgacaaca ctttttggag aatgttcagc aaagtcatgt ttgaatcaaa ggtcaaaaaa 11640
agaataatgt tatatgatgt aaaattccta tcattagtag gttatatagg atttaaaaac 11700
tggtttatag agcagttaag agtagtagaa ttgcatgaag tgccctggat tgtcaatgct 11760
gaaggggagc tagttgaaat taaaccaatc aaaatttatt tgcagttaat agaacaaagt 11820
ctatctttaa gaataactgt tttgaattat acagacatgg cacatgctct tacacgatta 11880
attaggaaga aattgatgtg tgataatgca ctcttcaatc caagttcatc accaatgttt 11940
agtctaactc aagttatcga tcctacaaca cagctagact attttcctaa ggtgatattt 12000
gaaaggttaa aaagttatga taccagttca gactacaaca aagggaagtt aacaagaaat 12060
tacatgacat tattaccatg gcagcacgta aacaggtata atttgtcttt tagttcaaca 12120
ggatgtaaaa tcagcttgaa gacatgcatc gggaaattga taaaggactt aaaccctaag 12180
gttctttact ttattggaga aggagcaggt aactggatgg caagaacagc atgtgagtat 12240
cctgacataa aatttgtata taggagttta aaggatgatc ttgatcatca ttacccatta 12300
gaatatcaaa gggtaatagg tgatttaaat agggtaatag atggtggtga aggactatca 12360
atggagacca cagatgcaac tcaaaagact cattgggact taatacacag aataagtaaa 12420
gatgctttat tgataacatt gtgtgatgca gaattcaaaa acagagatga tttctttaaa 12480
atggtaattc tttggagaaa acatgtatta tcatgtagaa tctgtacagc ttatggaaca 12540
gatctttact tatttgcaaa gtatcatgcg acggactgca atataaaatt accatttttt 12600
gtaaggtctg tagctacttt tattatgcaa ggaagcaaat tgtcaggatc agaatgttac 12660
atacttttaa cattaggtca tcacaataat ctgccatgcc atggagaaat acaaaattcc 12720
aaaatgagaa tagcagtgtg taatgatttc catgcctcaa aaaaactaga caacaaatca 12780
attgaagcta actgtaaatc tcttctatca ggattaagaa taccaataaa caaaaaagag 12840
ttaaatagac aaaagaaact gttaacacta caaagcaatc attcttccat agcaacagtt 12900
ggcggcagta agattataga atccaaatgg ttaaagaata aagcaagtac aataattgat 12960
tggttagagc atatcttgaa ttctccaaaa ggtgaattaa actatgattt ctttgaagca 13020
ttagagaaca cataccccaa tatgatcaag cttatagata acctgggaaa tgcagagata 13080
aaaaaactaa tcaaagttcc tgggtatatg cttgtgagta agaagtaata ataat 13135

<210> 98
<211> 907
<212> DNA
<213> Human metapneumo virus

<400> 98

atggaggtga aagtggagaa cattcgaaca atagatatgc tcaaagcaag agtaaaaaat 60
cgtgtggcac gcagcaaatg ctttaaaaat gcctctttgg tcctcatagg aataactaca 120
ttgagtattg ccctcaatat ctatctgatc ataaactata aaatgcaaaa aaacacatct 180
gaatcagaac atcacaccag ctcatcaccc atggaatcca gcagagaaac tccaacggtc 240
cccacagaca actcagacac caactcaagc ccacagcatc caactcaaca gtccacagaa 300
ggctccacac tctactttgc agcctcagca agctcaccag agacagaacc aacatcaaca 360
ccagatacaa caaaccgccc gcccttcgtc gacacacaca caacaccacc aagcgcaagc 420
agaacaaaga caagtccggc agtccacaca aaaaacaacc caaggacaag ctctagaaca 480
cattctccac cacgggcaac gacaaggacg gcacgcagaa ccaccactct ccgcacaagc 540
agcacaagaa agagaccgtc cacagcatca gtccaacctg acatcagcgc aacaacccac 600
aaaaacgaag aagcaagtcc agcgagccca caaacatctg caagcacaac aagaatacaa 660
aggaaaagcg tggaggccaa cacatcaaca acatacaacc aaactagtta acaaaaaata 720
caaaataact ctaagataaa ccatgcagac accaacaatg gagaagccaa aagacaattc 780
acaatctccc caaaaaggca acaacaccat attagctctg cccaaatctc cctggaaaaa 840
acactcgccc atataccaaa aataccacaa ccaccccaag aaaaaaactg ggcaaaacaa 900
cacccaa 907

<210> 99
<211> 908
<212> DNA
<213> Human metapneumo virus

<400> 99
atggaggtga aagtggagaa cattcgaaca atagatatgc tcaaagcaag tgtaaaaaat 60
cgtgtggcac gcagcaaatg ctttaaaaat gcctctttgg tcctcatagg aataactaca 120
ttgagtattg ccctcaatat ctatctgatc ataaactata aaatgcaaaa aaacacatct 180
gaatcagaac atcacaccag ctcatcaccc atggaatcca gcagagaaac tccaacggtc 240
cccacagaca actcagacac caactcaagc ccacagcatc caactcaaca gtccacagaa 300
ggctccacac tctactttgc agcctcagca agctcaccag agacagaacc aacatcaaca 360
ccagatacaa caaaccgccc gcccttcgtc gacacacaca caacaccacc aagcgcaagc 420
agaacaaaga caagtccggc agtccacaca aaaaacaacc caaggacaag ctctagaaca 480
cattctccac cacgggcaac gacaaggacg gcacgcagga accaccactc tccgcacaag 540
cagcacaaga aagagaccgt ccacagcatc agtccaacct gacatcagcg caacaaccca 600
caaaaacgaa gaagcaagtc cagcgagccc acaaacatct gcaagcacaa caagaataca 660
aaggaaaagc gtggaggcca acacatcaac aacatacaac caaactagtt aacaaaaaat 720
acaaaataac tctaagataa accatgcaga caccaacaat ggagaagcca aaagacaatt 780
cacaatctcc ccaaaaaggc aacaacacca tattagctct gcccaaatct ccctggaaaa 840
aacactcgcc catataccaa aaataccaca accaccccaa gaaaaaaact gggcaaaaca 900
acacccaa 908

<210> 100
<211> 907
<212> DNA
<213> Human metapneumo virus

<400> 100
atggaggtga aagtggagaa cattcgaaca atagatatgc tcaaagcaag agtaaaaaat 60
cgtgtggcac gcagcaaatg ctttaaaaat gcctctttgg tcctcatagg aataactaca 120
ctgagtattg ccctcaatat ctatctgatc ataaactata aaatgcaaaa aaacacatct 180
gaatcagaac atcacaccag ctcatcaccc atggaatcca gcagagaaac tccaacggtc 240
cccacagata attcagacac caactcaagc ccacaacatc caactcaaca gtccacagaa 300
ggctccacac tctactttgc agcctcagca aactcaccag agacagaacc aacatcaaca 360
ccagacacaa caaaccgccc gcccttcgtc gacacacaca caacaccacc aagcgcaagc 420
agaacaaaga caagtccggc agtccacaca aaaaacaacc caaggataag ctccagaaca 480
cactctccac catgggcaac gacaaggacg gcacgcagaa ccaccactct ccgcacaagc 540
agcacaagaa agagaccgtc cacagcatca gcccaacccg acatcagcgc aacaacccac 600
aaaaacgaag aagcaagtcc agcgagccca caaacatctg caagcacaac aagaacacaa 660
aggaaaagcg tggaggccaa cacatcaaca acatacaacc aaactagtta acaaaaaata 720
caaaataact ctaagataaa ccatgcagac accaacaatg gagaagtcaa aagacaattc 780
acaatctccc caaaaaggca acaacaccat attagctctg cccaaatctc cctggaaaaa 840
acactcgccc atataccaaa aataccacaa ccaccccaag aaaaaaactg ggcaaaacaa 900
cacccaa 907

<210> 101
<211> 907
<212> DNA
<213> Human metapneumo virus

<400> 101
atggaggtga aagtggagaa cattcgaaca atagatatgc tcaaagcaag agtaaaaaat 60
cgtgtggcac gcagcaaatg ctttaaaaat gcctctttgg tcctcatagg aataactaca 120
ttgagtattg ccctcaatat ctatctgatc ataaactata aaatgcaaaa aaacacatct 180
gaatcagaac atcacaccag ctcatcaccc atggaatcca gcagagaaac tccaacggtc 240
cccacagata attcagacac caactcaagc ccacaacatc caactcaaca gtccacagaa 300
ggctccacac tctactttgc agcctcagca aactcaccag agacagaacc aacatcaaca 360
ccagacacaa cagaccgccc gcccttcgtc gacacacaca caacaccacc aagcgcaagc 420
agaacaaaga caagtccggc agtccacaca aaaaacaacc caaggataag ctccagaaca 480
cattctccac catgggcaac gacaaggacg gcacgcagaa ccaccactct ccgcacaagc 540
agcacaagaa agagaccgtc cacagcatca gtccaacccg acatcagcgc aacaacccac 600
aaaaacgaag aagcaagtcc agcgagccca caaacatctg caagcacaac aagaacacaa 660
aggaaaagcg tggaggccaa cacatcaaca acatacaacc aaactagtta acaaaaaata 720
caaaataact ctaagataaa ccatgcagac accaacaatg gagaagtcaa aagacaattc 780
acaatctccc caaaaaggca acaacaccat attagctctg cccaaatctc cctggaaaaa 840
acactcgccc atataccaaa aataccacaa ccaccccaag aaaaaaactg ggcaaaacaa 900
cacccaa 907

<210> 102
<211> 907
<212> DNA
<213> Human metapneumo virus

<400> 102
atggaggtga aagtggagaa cattcgaaca atagatatgc tcaaagcaag agtaaaaaat 60
cgtgtggcac gcagcaaatg ctttaaaaat gcctctttgg tcctcatagg aataactaca 120
ttgagtattg ccctcaatat ctatctgatc ataaactata aaatgcaaaa aaacacatct 180
gaatcagaac atcacaccag ctcatcaccc atggaatcca gcagagaaac tccaacggtc 240
cccacagata attcagacac caactcaagc ccacaacatc caactcaaca gtccacagaa 300
ggctccacac tctactttgc agcctcagca agctcaccag agacagaacc aacatcaaca 360
ccagacacaa cagaccgccc gcccttcgtc gacacacaca caacaccacc aagcgcaagc 420
agaacaaaga caagtccggc agtccacaca aaaaacaacc caaggataag ctccagaaca 480
cattctccac catgggcaac gacaaggacg gcacgcagaa ccaccactct ccgcacaagc 540
agcacaagaa agagaccgtc cacagcatca gtccaacccg acatcagcgc aacaacccac 600
aaaaacgaag aagcaagtcc agcgagccca caaacatctg caagcacaac aagaacacaa 660
aggaaaagcg tggaggccaa cacatcaaca acatacaacc aaactagtta acaaaaaata 720
caaaataact ctaagataaa ccatgcagac accaacaatg gagaagtcaa aagacaattc 780
acaatctccc caaaaaggca acaacaccat attagctctg cccaaatctc cctggaaaaa 840
acactcgccc atataccaaa aataccacaa ccaccccaag aaaaaaactg ggcaaaacaa 900
cacccaa 907

<210> 103
<211> 907
<212> DNA
<213> Human metapneumo virus

<400> 103
atggaggtga aagtggagaa cattcgaaca atagatatgc tcaaagcaag agtgaaaaat 60
cgtgtggcac gcagcaaatg ctttaaaaat gcctctttga tcctaatagg aataactaca 120
ttgagtatag ccctcaatat ctatctgatc ataaactata caatgcaaga aaacacatcc 180
gaatcagaac atcacaccag ctcatcaccc atggaatcca gcagggaaac tccaacggtc 240
cccatagaca actcagacac caatccaggc tcacagtatc caactcaaca gtccacagaa 300
gactccacac tccactctgc agcttcagca agctcaccag agacagaacc aacatcaaca 360
ccagacacaa caagccgccc gcccttcgtc gacacacaca caacaccacc aagtgcaagc 420
aggacaagga caagtccggc agtccacaca aaaaacaatc caagggtaag ccccagaaca 480
cattccccac catgggcaat gacaaggacg gtccgcggaa ccaccactct ccgcacaagc 540
agcacaagaa aaagactgtc tacagcatca gtccaacccg acagcagcgc aacaacccac 600

aaacacgaag aaacaagccc agtgagccca caaacatctg caagcacagc aagaccacaa 660
aggaagggca tggaggccag cacatcaaca acatacaacc aaactagtta acaaaaaata 720
caaaataact ctaagataaa ccatgtagac accaacaatt gagaagccaa aaggcaattc 780
acaatctccc aaaaaagcaa caacaccata ttagctccgc ttaaatctcc ctgaaaaaaa 840
cactcaccca tataccaact ataccacaac catcccaaga aaaaaggctg ggcaaaacaa 900
cacccaa 907

<210> 104
<211> 908
<212> DNA
<213> Human metapneumo virus

<400> 104
atggaggtga aagtggagaa cattcgaaca atagatatgc tcaaagcaag agtgaaaaat 60
cgtgtggcac gcagcaaatg ctttaaaaat gcctctttga tcctaatagg aataactaca 120
ttgagtatag ccctcaatat ctatctgatc ataaactata caatgcaaga aaacacatcc 180
gaatcagaac atcacaccag ttcatcaccc atggaatcca gcagggaaac tccaacggtc 240
cctatggaca actcagacac caatccaggc tcacagtatc caactcaaca gtccacagaa 300
ggctccacac tccactttgc agcctcagca agctcaccag agacagaacc aacatcaaca 360
ccagacacaa caagccgccc gcccttcgtc gacacacaca caacaccatc aagtgcaagc 420
agaacaaaga caagtccggc agtccacaca aaaaacaatc taaggataag ccccagaaca 480
cattccccac catgggcaat gacaaggacg gtccgtggaa ccaccactct ccgcacaagc 540
agcataagaa aaagaccgtc cacagcatca gtccaacctg acagcagcgc aacaacccac 600
aaacacgaag aagcaagccc agtgagcccg caagcatctg caagcacagc aagaccacaa 660
aggaagggca tggaggccag cacatcaaca acatacaacc aaactagtta acaaaaaata 720
taaaataact ctaagataaa ccatgtagac accaacaatt gagaagccaa aaggcaattc 780
acaatctccc caaaaaggca acaacaccat attagctccg cttaaatctc cctggaaaaa 840
acactcgccc atataccaac tataccacaa ccatcccaag gaaaaaagct gggtaaaaca 900
acacccaa 908

<210> 105
<211> 908
<212> DNA
<213> Human metapneumo virus

<400> 105
atggaggtga aagtggagaa cattcgaaca atagatatgc tcaaagcaag agtgaaaaat 60
cgtgtggcac gcagcaaatg ctttaaaaat gcctctttga tcctaatagg aataactaca 120
ttgagtatag ccctcaatat ctatctgatc ataaactata caatgcaaga aaacacatcc 180
gaatcagaac atcacaccag ctcatcaccc atggaatcca gcagagaaac tccaacggtc 240
cctatggaca actcagacac caatccaggc tcacagtatc caactcaaca gtccacagaa 300
ggctccacac tccactttgc agcctcagca agctcaccag agacagaacc aacatcaaca 360
ccagacacaa caagccgccc gcccttcgtc gacacacaca caacaccatc aagtgcaagc 420
agaataagga caagtccggc agtccacaca aaaaacaatc taaggataag ccccagaaca 480
cattccccac catgggcaat gacaaggacg gtccgtggaa ccaccactct ccgcacaagc 540
agcataagaa aaagaccgtc cacagcatca gtccaacctg acagcagcgc aacaacccac 600
aaacacgaag aagcaagccc agtgagcccg caagcatctg caagcacagc aagaccacaa 660
aggaagggca tggaggccag cacatcaaca acatacaacc aaactagtta acaaaaaata 720
tacaataact ctaagataaa ccatgtagac accaacaatt gagaagccaa aaggcaattc 780
acaatctccc caaaaaggca acaacaccat attagctccg cttaagtctc cctggaaaaa 840
acactcgccc atataccaac tataccacaa ccatccaaag aaaaaaagct gggcaaaaca 900
acacccaa 908

<210> 106
<211> 888
<212> DNA
<213> Human metapneumo virus

<400> 106
atggaggtga aagtagagaa cattcgagca atagacatgc tcaaagcaag agtgaaaaat 60
cgtgtggcac gtagcaaatg ctttaaaaat gcttctttaa tcctcatagg aataactaca 120
ctgagtatag ctctcaatat ctatctgatc ataaactaca caatacaaaa aaccacatcc 180

gaatcagaac accacaccag ctcaccaccc acagaaccca acaaggaagc ttcaacaatc 240
tccacagaca acccagacat caatccaagc tcacagcatc caactcaaca gtccacagaa 300
aaccccacac tcaaccccgc agcatcagcg agcccatcag aaacagaacc agcatcaaca 360
ccagacacaa caaaccgcct gtcctccgta gacaggtcca cagcacaacc aagtgaaagc 420
agaacaaaga caaaaccgac agtccacaca atcaacaacc caaacacagc ttccagtaca 480
caatccccac cacggacaac aacgaaggca atccgcagag ccaccacttt ccgcatgagc 540
agcacaggaa aaagaccaac cacaacatta gtccagtccg acagcagcac cacaacccaa 600
aatcatgaag aaacaggttc agcgaaccca caggcgtctg caagcacaat gcaaaactag 660
cacaccaata atataaaacc aaattagtta acaaaaaatg cgagatagct ctaaagcaaa 720
acatgtaggt accaacaatc aagaaaccaa aagacaactc acaatctccc taaaacagca 780
acgacaccat gtcagctttg ctcaaatctc tctgggagaa acttctaccc acatactaac 840
aacatcacaa ccatctcaag aaaagaaact gggcaaaaca gcatccaa 888

<210> 107
<211> 888
<212> DNA
<213> Human metapneumo virus

<400> 107
atggaggtga aagtagagaa cattcgagca atagacatgc tcaaagcaag agtgaaaaat 60
cgtgtggcac gcagcaaatg ctttaaaaat gcttctttaa tcctcatagg aataactaca 120
ctgagtatag ccctcaatat ctatctgatc ataaactaca caatacaaaa aaccacatct 180
gaatcagaac accacactag ctcaccaccc acagaatcca acaaagaaac ttcaacaatc 240
cccatagaca acccagacat caatccaaac tcacagcatc caacccaaca gtccacagaa 300
agccccacac tcaaccccgc agcctcggtg agcccatcag aaacagaacc agcatcaaca 360
ccagacacaa caaaccgcct gtcctccgta gacagatcca caacacaacc aagtgaaagc 420
agaacaaaga caaaaccaac agtccacaca aaaaacaatc caagtacagt ttccagaaca 480
caatccccac tacgggcaac aacgaaggcg gtcctcagag ccaccgcttt ccgcacgagc 540
agcacaagaa aaagaccaac cacaacatca gtccagtctg acagcagcac cacaacccaa 600
aatcatgaag aaacaagttc agcgaaccca caggcatctg caagcacaat gcaaagccag 660
cacaccaaca acataaaacc aaattagtta acaaaaaata cgagatagct ctaaagtaaa 720
acatgtaggt accaacaatc aaggaatcaa aagacaactc acaatctccc taaaacagca 780
acaacatcat gtcagttttg ctcaaatctc cctgggagaa actttcgccc acatactaac 840
aacatcacaa ccatctcaag aaaagaaact gggcaaaaca gcacccaa 888

<210> 108
<211> 888
<212> DNA
<213> Human metapneumo virus

<400> 108
atggaggtga aagtagagaa catccgagca gtagacatgc tcaaagcaag agtcaaaaat 60
cgtgtggcac gcagcaaatg ctttaaaaat gcctccttaa tcctcgtagg aataactaca 120
ctgagcatag ccctcaatat ctatctgatc gtaaactaca caatacaaaa aaccacatcc 180
gaatcagaac accacaccag ctcatcaccc acagaatcca acaaaggaac ttcaacaatc 240
cccacagaca acccagacat caatccaaat tcacaacatc caactcaaca gtccacagaa 300
agccccacac tcaacaccgc agcctcggtg agcccatcag aaacagaacc agcatcaaca 360
ccagacacaa caaaccgcct gtcctccgca gacagatcca caacacaacc aagtgaaagc 420
agaacaaaga caaagctgac agtccacaca aaaaacaacc taagtacagc ctccagaaca 480
caatcaccac cacgggcaac aacgaaggcg gtcctcagag acaccgcctt ccacacgagc 540
agcacaggaa aaagaccaac cacaacatca gtccagtctg gcagcagcac cacaactcaa 600
aatcatgaag aaacaagttc atcgaaccca caggcatctg caagcacaat gcaagaccag 660
gacaccaaca atacaaaaca aaattagtta acaaaaaata caagatagct ctaaagtaaa 720
acatgtaggt accaacagta aagaaatcaa aagacaactc acaatctccc caaaacagca 780
acaacatcat gtcagcttcg ctcaaatctc cctgggagaa actctcgccc acatactaac 840
aacatcacaa ctatctcaag aaaagaaact gggcaaaaaa acactcaa 888

<210> 109
<211> 887
<212> DNA
<213> Human metapneumo virus

<400> 109
atggaggtga aagtagagaa catccgagca gtagacatgc tcaaagcaag agttaaaaat 60
cgtgtggcac gcagcaaatg ctttaaaaat gcctctttaa tcctcgtagg aataactaca 120
ctgagtatag ccctcaatat ctatctgatc gtaaactaca caatacaaaa aaccacatcc 180
gaatcagaac accacactag ctcatcaccc acagaatcca acaaaggaac ttcaacaatc 240
ccacagacaa cccagacatc aatccaaatt cacaacatcc aactcaacag tccacagaaa 300
gccccacact caacaccgca gcctcggtga gcccatcaga aacagaacca gcatcaacac 360
cagacacaac aaaccgcctg tcctccgcag acagatccac aacacaacca agtgaaagca 420
gaacaaagac aaagctgaca gtccacacaa aaaacaacct aagtacagcc tccagaacac 480
aatcaccacc acgggcaaca acgaaggcgg tcctcagaga caccgccttc cacacgagca 540
gcacaggaaa aagaccaacc acaacatcag tccagtctgg cagcagcacc acaactcaaa 600
atcatgaaga aacaagttca tcgaacccac aggcatctgc aagcacaatg caagaccagg 660
acaccaacaa tacaaaacaa aattagttaa caaaaaatac aagatagctc taaagtaaaa 720
catgtaggta ccaacagtaa agaaatcaaa agacaactca taatctcccc aaaacagcaa 780
caacatcatg tcagcttcgc tcaaatctcc ctgggagaaa ctctcgccca catactaaca 840
acatcacaac tatctcaaga aaagaaactg ggcaaaaaaa cactcaa 887

<210> 110
<211> 888
<212> DNA
<213> Human metapneumo virus

<400> 110
atggaggtga aagtagagaa cattcgagca atagacatgc tcaaagcaag aatgaaaaat 60
cgtgtggcac gcagcaaatg ctttaaaaat gcttctttaa tcctcatagg aataactact 120
ctgagtatag ccctcaatat ctatctgatc ataaactaca caatacaaaa aaccacatct 180
gaatcagaac accacactag ctcaccaccc acagaatcca acaaagaaac ttcaacaatc 240
cctatagaca acccagacat caatccaaac tcacagcatc caactcaaca gtccacagaa 300
agcctcacac tcaaccccgc agcctcggtg agcccatcag aaacagaacc agcatcaaca 360
ccagacacaa caaaccgcct gtcctccgta gacagatcca caacacaacc aagtgaaagc 420
agaacaaaga caaaactgac agtccacaaa aaaaacatcc caagtacagt ctctagaaca 480
caatcctcaa tacgggcaac aacgaaggcg gtcctcagag ccaccgcctt tcgcacgagc 540
agcacaggag aaagaccaac tacaacatca gtccagtctg acagcagcac cacaacccaa 600
aatcatgaag aaacaggttc agcgaaccca caggcatctg caagcacaat gcaaaactag 660
cacaccaaca ttgtaaaacc aaattagtta acaaaaaata tgaaatagct ctaaagtaaa 720
acatgtaggt gctaacaatc aagaaatcaa aagacatctc ataatctctc caaaacagca 780
acaacatcat gtcaactttg ctcaaatctc cctgggagaa actttcgccc ccatactgac 840
aacatcacaa tcatctcaag aaaagaaact gggcaaaaca gcaccaaa 888

<210> 111
<211> 888
<212> DNA
<213> Human metapneumo virus

<400> 111
atggaggtga aagtagagaa cattcgagca atagacatgc tcaaagcaag agtgaaaaat 60
cgtgtggcac gcagcaaatg ctttaaaaat gcttctttaa tcctcatagg aataactact 120
ctgagtatag ccctcaacat ctatctgatc ataaactaca caatacaaaa aaccacatct 180
gaatcagaac accacactag ctcaccaccc acagaatcta acaaagaaac ttcaacaatc 240
tctatagaca acccagacat caatccaaac tcacagcatc caactcaaca gtccacagaa 300
agcctcacac tcagccccac agcctcggtg agcccatcag aaacagaacc agcatcaaca 360
tcagacacaa caagccgcct gtcttccgta gacagatcca caacacaacc aagtgaaagc 420
agagcaagga caaaaccgac agtccacaag aaaaacatcc caagtacagt ttctagaaca 480
caatccccac tacgggcaac aacgaaggcg gtcctcagag ccaccgcctt tcgcacgagc 540
agcacaggag agggaccaac cacaacatcg gtccagtctg acagcagcac cacaacccaa 600
aatcatgaag aaacaggttc agcgaaccca caggcatctg caagcacaat gcaaaactag 660
cacaccaaca ttgtaaaacc aaattagtta acaaaaaata tgaaatagtt ctaaagtaaa 720
acatgtaggt gctaacaatc aagaaatcaa aagacaactc ataatctccc taaaacagca 780
acaacatcat gtcaactttg ctcaaatctc cctgggagaa actttcgccc ccatactgac 840
aacatcacaa tcatctcaag aaaagaaact gggcaaaaca gcaccaaa 888

<210> 112

<211> 888
<212> DNA
<213> Human metapneumo virus

<400> 112
atggaggtga aagtagagaa cattcgagca atagacatgc tcaaagcaag agtgaaaaat 60
cgtgtggcac gtagcaaatg ctttaaaaat gcttctttaa tcctcatagg aataactaca 120
ctgagtatag ctctcaatat ctatctgatc ataaactaca caatacaaaa aaccacatct 180
gaatcagaac accacaccag ctcaccaccc acagaatcca acaaggaagc ttcaacaatc 240
tccacagaca atccagacat caatccaaac tcacagcatc caactcaaca gtccacagaa 300
aaccccacac taaaccccgc agcatcggtg agctcatcag aaacagaacc agcatcaaca 360
ccagacacaa caaaccgcct gtcctccgta gacaggtcca cagcacaacc aagtgaaagc 420
agaacaaaga caaaaccgac agtccacaca agaaacaacc caagcacagc ttccagcaca 480
caatccccac cacgggtaac aacgaaggca atcctcagag ccaccgtctt ccgcatgagc 540
agcacaggaa aaagaccagc cacaacatta gtccagtccg acagcagcac cacaacccaa 600
aatcatgaag aaacaggttc agcaaactca caggcatctg caagcacaat gcaaaactag 660
cactccaaca atataaaacc aaattagtta acaaaaaata cgagatagct ctaaagtaaa 720
acatgtaggc accaacaatc aggaaattaa aagacaactc acaacctccc taaaacagca 780
acgacaccat gtcaactttg ctcaaatctc tctgggagaa acttttgccc acatactaac 840
aacatcacaa tcatctcaag aaaagaaact gggcaaaaca gcatccaa 888

<210> 113
<211> 888
<212> DNA
<213> Human metapneumo virus

<400> 113
atggaggtga aagtagagaa cattcgagca atagacatgc tcaaagcaag agtgaaaaat 60
cgtgtggcac gcagcaaatg ctttaaaaat gcttctttaa tcctcatagg aataactact 120
ctgagtatag ccctcaacat ctatctgatc ataaactaca caatacaaaa aaccacatct 180
gaatcagaac accacactag ctcaccaccc acagaatcta acaaagaaac ttcaacaatc 240
tctatagaca actcagacat caatccaaac tcacagcatc caactcaaca gtccacagaa 300
agcctcacac tcagccccac agcctcggtg agcccatcag aaacagaacc agcatcaaca 360
tcagacacaa caaaccgcct gtcttccgta gacagatcca caacacaacc aagtgaaagc 420
agagcaagaa caaaaccgac agtccacaag aaaaacatcc caagtacagt ttctagaaca 480
caatccccac tacgggcaac aacgaaggcg gtcctcagag ccaccgcctt tcgcatgagc 540
agcacaggag agggaccaac cacaacatcg gtccagtctg acagcagcac cacaacccaa 600
aatcatgaag aaacaggctc agcgaaccca caggcatctg caagcacaat gcaaaaccag 660
cacaccaaca ttgcaaaacc aaattagtta acaaaaaata tgaaatagtt ctaaagtaaa 720
acatgtaggt gccaacaatc aagaaatcaa aagacaactc acaatctccc taaaacagca 780
acaacatcat gccaactttg ctcaaatctc cctgggagaa accctcgccc ccatactgac 840
aacatcacaa tcatctcaag aaaagaaact gggcaaaaca gcaccaaa 888

<210> 114
<211> 888
<212> DNA
<213> Human metapneumo virus

<400> 114
atggaggtga aagtagagaa cattcgagca atagacatgc tcaaagcaag agtgaaaaat 60
cgtgtggcac gcagcaaatg ctttaaaaat gcttctttaa tcctcatagg aataactact 120
ctgagtatag ccctcaatat ctatctgatc ataaactaca caatacaaaa aaccacatct 180
gaatcagaac accacactag ctcaccaccc acagaatcta acaaggaaac ttcaacaatc 240
cctatagaca acccagacat caatccaaac tcacagcatc caactcaaca gtccacagaa 300
agcctcacac tctaccccac atcctcggtg agctcatcag aaacagaacc agcatcaaca 360
ccaggcataa caaaccacct gtcctttgta gacagatcca caacacaacc aagtgaaagc 420
agaacaaaga caaaccggac agtccacaaa aaaaacatct caagtacagt ttctagaaca 480
cagtccccac cacggacaac agcgaaggcg gtccccagag ccaccgccct tcgcacgagc 540
agcacaggag aaagaccaac cacaacacca gtccagcccg atagcagcac cacaacacaa 600
aatcatgaag aaacaggctc agcgaaccca caggcatccg caagcacaat gcaaaaccag 660
cacaccaaca ttgcaagacc aaattagtta acaaaaaata tgaaatagct ctaaagtaaa 720
acatgtaggt gccaacaatc aagaaatcaa aagataactc ataatctctc taaaacatca 780

acaacatcat gttaactttg ctcaaatctc tctgggagaa accttcgccc ccatactggc 840
aacatcacaa tcatctcaag aaaagaaact gggcaaaaca acaccaaa 888

<210> 115
<211> 888
<212> DNA
<213> Human metapneumo virus

<400> 115
atggaggtga aagtagagaa cattcgagca atagacatgc tcaaagcaag agtgaaaaat 60
cgtgtggcac gcagcaaatg ctttaaaaat gcttctttaa tcctcatagg aataactact 120
ctgagtatag ccctcaatat ctatctgatc ataaactaca caatacaaaa aaccacatct 180
gaatcagaac accacactag ctcaccaccc acagaatcta acaaggaaac ttcaacaatc 240
cctatagaca acccagacat caatccaaac tcacagcatc caactcaaca gtccgcagaa 300
agcctcacac tctaccccac atcctcggtg agctcatcag aaacagaacc agcatcaaca 360
ccaggcataa caaaccacct gtcctttgta gacagatcca caacacaacc aagtgaaagc 420
agaacaaaga caaaccggac agtccacaaa aaaaacatct caagtacagt ttctagaaca 480
cagtccccac cacggacaac agcgaaggcg gtccccagag ccaccgccct tcgcacgagc 540
agcacaggag aaagaccaac cacaacacca gtccagcccg atagcagcac cacaacacaa 600
aatcatgaag aaacaggctc agcgaaccca caggcatccg caagcacaat gcaaaaccag 660
cacaccaaca ttgcaagacc aaattagtta acaaaaaata tgaaatagct ctaaagtaaa 720
acatgtaggt gccaacaatc aagaaatcaa aagataactc ataatctctc taaaacatca 780
acaacatcat gttaactttg ctcaaatctc tctgggagaa accttcgccc ccatactggc 840
aacatcacaa tcatctcaag aaaagaaact gggcaaaaca acaccaaa 888

<210> 116
<211> 888
<212> DNA
<213> Human metapneumo virus

<400> 116
atggaggtga aagtagagaa tattcgagca atagacatgc tcaaagcaag agtgaaaaat 60
cgtgtggcac gcagcaaatg ctttaaaaat gcttctttaa tcctcatagg aataactact 120
ctgagtatag ccctcaatat ctatctgatc ataaactaca caatacaaaa aaccacatct 180
gaatcagaac accacactag ctcaccaccc acagaatcta acaaggaaac ttcaacaatc 240
cctatagaca acccagacat caatccaaac tcacagcatc caactcaaca gtccacagaa 300
agcctcacac tctaccccac atcctcggtg agctcatcag aaacagaacc agcatcaaca 360
ccaggcataa caaaccacct gtcctttgta gacagatcca caacacaacc aagtgaaagc 420
agaacaaaga caaaccggac agtccacaaa aaaaacatct caagtacagt ttctagaaca 480
cagtccccac cacggacaac agcgaaggcg gtccccagag ccaccgccct tcgcacgagc 540
agcacaggag aaagaccaac cacaacacca gtccagcccg atagcagcac cacaacacaa 600
aatcatgaag aaacaggctc agcgaaccca caggcatccg caagcacaat gcaaaaccag 660
cacaccaaca ttgcaagacc aaattagtta acaaaaaata tgaaatagct ctaaagtaaa 720
acatgtaggt gccaacaatc aagaaatcaa aagataactc ataatctctc taaaacatca 780
acaacatcat gttaactttg ctcaaatctc tctgggagaa accttcgccc ccatactggc 840
aacatcacaa tcatctcaag aaaagaaact gggcaaaaca acacccaa 888

<210> 117
<211> 888
<212> DNA
<213> Human metapneumo virus

<400> 117
atggaggtga aagtagagaa cattcgagca atagacatgc tcaaagcaag agtgaaaaat 60
cgtgtggcac gtagcaaatg ctttaaaaat gcttctttaa tcctcatagg aataactaca 120
ctgagcatag ccctcaatat ctatctgatc ataaactaca caatacaaca aaccacatct 180
gaatcagaac accacaccag ctcaccaccc acagaatcca acaaggaagc ttcaacaatc 240
tccacagaca acccagacat caatccaaac tcacagcatc caactcaaca gtccacagaa 300
aaccccacac tcaacccagc agcatcagcg agcccatcag aaacagaatc agcatcaaca 360
ccagatacaa caaaccgcct gtcctccgta gacaggtcca cggtacaacc aagtgaaaac 420
agaacaaaga caaaactgac agtccacaca agaaacaacc taagcacagc ctccagtaca 480
caatccccac cacgggcaac aacgaaggca atccgcagag ccaccaccct ccgcatgagc 540

agcacaggaa gaagaccaac cacaacacta gtccagtccg acagcagcac cacaacccaa 600
aatcatgaag aaacaggctc agcgaaccca caggcatctg caagcacaat gcaaaaccag 660
cacaccaaca atataaaacc aaattagtta acaaaaaata cgagatagct ctaaagtaaa 720
acatgtaggc accaacaatc aagaaaccaa aagataactc acaatccccc caaaacagca 780
acgacaccat gtcagctttg ctcaaatctc tctgggagaa acttttgccc acatactaac 840
aacatcacaa ccatctcaag aaaagaaact gggcaaaaca gcatccaa 888

<210> 118
<211> 888
<212> DNA
<213> Human metapneumo virus

<400> 118
atggaggtga aagtagagaa cattcgagca atagacatgc tcaaagcaag agtgaaaaat 60
cgtgtggcac gtagcaaatg ctttaaaaat gcttctttaa tcctcatagg aataactaca 120
ctgagcatag ccctcaatat ctatctgatc ataaactaca caatacaaaa aaccacatct 180
gaatcagaac accacaccag ctcaccaccc acagaatcca acaaggaagc ttcaacaatc 240
tccacagaca acccagacat caatccaaac tcacagcatc caactcaaca gtccacagaa 300
aaccccacac tcaacccagc agcatcagcg agcccatcag aaacagaatc agcatcaaca 360
ccagatacaa caaaccgcct gtcctccgta gacaggtcca cggtacaacc aagtgaaaac 420
agaacaaaga caaaactgac agtccacaca agaaacaacc taagcacagc ctccagtaca 480
caatccccac cacgggcaac aacgaaggca atccgcagag ccaccaccct ccgcatgagc 540
agcacaggaa gaagaccaac cacaacacta gtccagtccg acagcagcac cacaacccaa 600
aatcatgaag aaacaggctc agcgaaccca caggcatctg caagcacaat gcaaaaccag 660
cacaccaaca atataaaacc aaattagtta acaaaaaata cgagatagct ctaaagtaaa 720
acatgtaggc accaacaatc aagaaaccaa aagataactc acaatccccc caaaacagca 780
acgacaccat gtcagctttg ctcaaatctc tctgggagaa acttttgccc acatactaac 840
aacatcacaa ccatctcaag aaaagaaact gggcaaaaca gcatccaa 888

<210> 119
<211> 901
<212> DNA
<213> Human metapneumo virus

<400> 119
atggaagtaa gagtggagaa cattcgagcg atagacatgt tcaaagcaaa gataaaaaac 60
cgtataagaa gcagcaggtg ctatagaaat gctacactga tccttattgg actaacagcg 120
ttaagcatgg cacttaatat tttcctgatc atcgatcatg caacattaag aaacatgatc 180
aaaacagaaa actgtgctaa catgccgtcg gcagaaccaa gcaaaaagac cccaatgacc 240
tccacagcag gcccaaacac caaacccaat ccacagcaag caacacagtg gaccacagag 300
aactcaacat ccccagtagc aaccccagag ggccatccat acacagggac aactcaaaca 360
tcagacacaa cagctcccca gcaaaccaca gacaaacaca cagcaccgct aaaatcaacc 420
aatgaacaga tcacccagac aaccacagag aaaaagacaa tcagagcaac aacccaaaaa 480
agggaaaaag gaaaagaaaa cacaaaccaa accacaagca cagctgcaac ccaaacaacc 540
aacaccacca accaaatcag aaatgcaagt gagacaatca caacatccga cagacccaga 600
actgacacca caacccaaag cagcgaacag acaacccggg caacagaccc aagctcccca 660
ccacaccatg catagagagg tgcaaaactc aaatgagcac aacacacaaa catcccatcc 720
aagtagttaa caaaaaacca caaaataacc ttgaaaacca aaaaaccaaa acataaaccc 780
agacccagaa aaacatagac accatatgga aggttctagc atatgcacca atgagatggc 840
atctgttcat gtatcaatag caccaccatc attcaaggaa taagaagagg cgaaaattta 900
a 901

<210> 120
<211> 901
<212> DNA
<213> Human metapneumo virus

<400> 120
atggaagtaa gagtggagaa cattcgagcg atagacatgt tcaaagcaaa gataaagaac 60
cgtataagaa gcagcaggtg ctatagaaat gctacactga tccttattgg actaacagcg 120
ttaagcatgg cacttaatat tttcctgatc attgatcatg caacattaag aaacatgatc 180
aaaacagaaa actgtgctaa catgccatcg gcagaaccaa gcaaaaagac cccaatgacc 240

tccacagcag gcccaagcac cgaacccaat ccacagcaag caacacaatg gaccacagag 300
aactcaacat ccccagcagc aaccctagag agccatccat acacagggac aacccaaaca 360
ccagacataa cagctcccca acaaaccaca gacaaacaca cagcactgcc aaaatcaacc 420
aatgaacaga tcacccagac aaccacagag aaaaagacaa ccagagcaac aacccaaaaa 480
agggaaaaag aaaaagaaaa cacaaaccaa accacaagca cagctgcaac ccaaacaacc 540
aacaccacca accaaaccag aaatgcaagt gagacaatca caacatccga cagacccaga 600
attgacacca caacccaaag cagcgatcag acaacccggg caacagaccc aagctcccca 660
ccacaccatg cacagagtgg tgcaaaaccc aaatgaacac aacacacaaa catctcatcc 720
aagtagttaa caaaaaacca caaaataacc ttgaaaacca aaaaaccaaa ccacaaactt 780
agacccagaa aaacatagac actatatgga aggtttgagc atatgcacca atgaaatggt 840
atctgttcat gtatcaatag cgccaccatt atttaaggaa taagaagagg caaaaattca 900
a 901

<210> 121
<211> 860
<212> DNA
<213> Human metapneumo virus

<400> 121
atggaagtaa gagtggagaa cattcgagcg atagacatgt tcaaagcaaa gataaaaaac 60
cgtataagaa gcagcaggtg ctatagaaat gctacactga tccttattgg actaacagcg 120
ttaagcatgg cacttaatat tttcctgatc atcgatcatg caacattaag aaacatgatc 180
aaaacagaaa attgtgctaa catgccgccg gcagaaccaa gcaaaaagac cccaatgacc 240
tctacagcag gcccaaacac caaacccaat ccacagcaag caacacagtg gaccacggag 300
aactcaacat tcccagcagc aacctcagag ggccatctac acacagggac aactcaaaca 360
ccagacacaa cagctcctca gcaaaccaca gacaaacaca cagcactgcc aaaatcaacc 420
aatgaacaaa tcacccagac aaccacagag aaaaagacaa ccagagcaac aacccaaaga 480
agggaaaaag ggaaagaaaa cacaaaccaa accacaagca cagctgctac ccaaacaacc 540
aacaccacca accaaatcag aaatgcaagc gagacaatca caacatccga cagacccaga 600
actgactcca caacccaaag cagcgaacag acaacccggg caacagaccc aagctcccca 660
ccacatcatg cacagggaag tgcaaaaccc aaatgaacac aacacacaaa catcccatcc 720
aagtagttaa caaaaaatca gacccagaaa aacatagaca ctatatggaa ggtccgagca 780
tatgcaccga tgaaatggca tttgttcatg tatcaatagc gccaccatta tttaaggaat 840
aagaagaggc aaaaattcaa 860

<210> 122
<211> 861
<212> DNA
<213> Human metapneumo virus

<400> 122
atggaagtaa gagtggagaa cattcgagcg atagacatgt tcaaagcaaa gataaaaaac 60
cgtataagaa gcagcaggtg ctatagaaat gctacactga tccttattgg actaacagcg 120
ttaagcatgg cacttaatat tttcctgatc atcgatcatg caacattaag aaacatgatc 180
aaaacagaaa attgtgctaa catgccgccg gcagaaccaa gcagaaagac cccaatgacc 240
tccacagcag gcccaaacac caaacccaat ccacagcaag caacacagtg gaccacggag 300
aactcaacat ccccagcagc aaccccagag ggccatctac acacagggac aactcaaaca 360
ccagacacaa cagctcctca gcaaaccaca gacaaacaca cagcactgcc aaaatcaacc 420
aatgaacaga tcacccaggc aaccacagag aaaaagacaa ccagagaaac aacccaaaga 480
agggaaaaag gaaaagaaaa cacaaaccaa accacaagca cagctgcaac ccaaacaacc 540
aacaccacca accaaatcag aaatgcaagc gagacaatca caacatccga cagacccaga 600
actgactcca caacccaaag cagcgaacag acaacccagg caacagaccc aagctcccca 660
gcacaccatg cacagggaag tgcaaaaccc aaatgaacac aacacacaaa catcccatcc 720
aagtagttaa caaaaaaatc agacccagaa aaacacagac actatatgga aggtccgagc 780
atatgcaccg atgaaatggc atctgttcat gtatcaatag caccaccatt atttaaggaa 840
taagaagagg caaaaattca a 861

<210> 123
<211> 860
<212> DNA
<213> Human metapneumo virus

<400> 123
atggaagtaa gagtggagaa cattcgagcg atagacatgt tcaaagcaaa gataaaaaac 60
cgtataagaa gcagcaggtg ctatagaaat gctacattga tccttattgg actaacagcg 120
ttaagcatgg cacttaatat tttcctgatc atcgatcatg caacattaag aaacatgatc 180
aaaacagaaa attgtgctaa catgccaccg gcagaaccaa gcaaaaagac cccaatgacc 240
tccacagcag gcctaaacac taaacccaat ccacagcaag caacacagtg gaccacggag 300
aactcaacat ccccagcagc aaccccagag ggccatctac acacagggac aactcaaaca 360
ccagacacaa cagctcctca gcaaaccaca gacaagcaca cagcactgcc aaaatcaacc 420
aatgaacaga tcacccagac aaccacagag aaaaagacaa ccagagcaac aacccaaaga 480
agggaaaaag gaaaagaaaa cacaaaccaa accacaagca cagctgcaac ccaaacaacc 540
aacaccacca accaaatcag aaatgcaagc gagacaatca caacatccga cagacccaga 600
actgactcca caacccaaag cagcgaacag acaacccggg caacagaccc aagctcccca 660
ccacaccatg cacagggaag tgcaaaaccc aaatgaacac aacacacaaa catcccatcc 720
aagtagttaa caaaaaatca gacccagaaa aacatagaca ctatatggaa ggtccgagca 780
tatgcaccga tgaaatggca tctgttcatg tatcaatagc gccaccatta tttaaggaat 840
aagaagaggc aaaaattcaa 860

<210> 124
<211> 860
<212> DNA
<213> Human metapneumo virus

<400> 124
atggaagtaa gagtggagaa cattcgagcg atagacatgt tcaaagcaaa gataaaaaac 60
cgtataagaa gcagcaggtg ctatagaaat gctacactga tccttattgg actaacagcg 120
ttaagcatgg cacttaatat tttcctgatc atcgatcatg caacattaag aaacatgatc 180
aaaacagaaa attgtgctaa catgccgccg gcagaaccaa gcaaaaagac cccaatgacc 240
tccacagcag gcccaaacac caaacccaat ccacagcaag caacacagtg gaccacggag 300
aactcaacat ccccagcagc aaccccagag ggccatctac acacagggac aactcaaaca 360
ccagacacaa cagctcctca gcaaaccaca gacaaacaca cagcactgcc aaaatcaacc 420
aatgaacaga tcacccagac aaccacagag aaaaagacaa ccagagcaac aacccaaaga 480
agggaaaaag gaaaagaaaa cacaaaccaa accacaagca cagctgcaac ccaaacaacc 540
aacaccacca accaaatcag aaatgcaatt gagacaatca caacatccga cagacccaga 600
actgactcca caacccaaag cagcgaacag acaacccggg caacagaccc aagctcccac 660
ccacaccatg cacagggaag tgcaaaaccc aaatgaacac aacacacaaa catcccatcc 720
aagtagttaa caaaaaatca gacccagaaa aacatagaca ctatatggaa ggtccgagca 780
tatgcaccga tgaaatggca tctgttcatg tatcaatagc gccaccatta tttaaggaat 840
aagaagaggc aagaattcaa 860

<210> 125
<211> 886
<212> DNA
<213> Human metapneumo virus

<400> 125
atggaagtaa gagtggagaa cattcgggca atagacatgt tcaaagcaaa aatgaaaaac 60
cgtataagaa gtagcaagtg ctatagaaat gctacactga tccttattgg attaacagca 120
ttaagtatgg cacttaatat ttttttaatc attgattatg caatgttaaa aaacatgacc 180
aaagtggaac actgtgttaa tatgccgccg gtagaaccaa gcaagaagac cccaatgacc 240
tctgcagtag acttaaacac caaacccaat ccacagcagg caacacagtt ggccgcagag 300
gattcaacat ctctagcagc aacctcagag gaccatctac acacagggac aactccaaca 360
ccagatgcaa cagtctctca gcaaaccaca gacgagtaca caacattgct gagatcaacc 420
aacagacaga ccacccaaac aaccacagag aaaaagccaa ccggagcaac aaccaaaaaa 480
gaaaccacaa ctcgaactac aagcacagct gcaacccaaa cactcaacac taccaaccaa 540
actagctatg tgagagaggc aaccacaaca tccgccagat ccagaaacag tgccacaact 600
caaagcagcg accaaacaac ccaggcagca gacccaagct cccaaccaca ccatacacag 660
aaaagcacaa caacaacata caacacagac acatcctctc caagtagtta acaaaaaaac 720
tataaaataa tcatgaaaac cgaaaaacta gaaaagttaa tttgaactca gaaaagaaca 780
caaacactat atgaattgtt tgagcgtata tactaatgaa atagcatctg tttgtgcatc 840
aataatacca tcattattta agaaataaga agaagctaaa attcaa 886

<210> 126

<211> 889
<212> DNA
<213> Human metapneumo virus

<400> 126
atggaagtaa gagtggagaa cattcggaca atagacatgt tcaaagcaaa gatgaaaaac 60
cgtataagaa gcagcaagtg ctatagaaat gctacactga tccttattgg actgacagca 120
ttaagtatgg cacttaatat tttcttgatc atcgattatg caacatttaa aaacatgacc 180
aaagtggaac actgtgctaa tatgccgccg gtagaaccga gtaagaagac cccaatgacc 240
tctacagtag actcaagcac cggacccaat ccacagcaga caacacagtg gaccacagag 300
gattcaacat ctctagcagc aacctcagag gaccatctac acacagggac aactccaaca 360
ctagatgcaa cagtttctca gcaaacccca gacaagcaca caacaccgct gagatcaacc 420
aatggacaga ccacccagac aaccacagag aaaaagccaa ccagagcaat agccaaaaaa 480
gaaaccacaa accaaaccac aagcacagct gcaacccaaa cattcaacac caccaatcaa 540
accagaaatg gaagagagac aaccataaca tctgccagat ccagaaacga cgccacaact 600
caaagcagcg aacaaacaaa ccagacaaca gacccaagct cccaaccaca tcatgcatag 660
ataagcacaa taacaatatg aacacaacac agacacatct tctccaagta gttaacaaaa 720
aactataaaa taaccatgaa aaccaaaaaa ctagaaaagt aaatttgaac tcagaaaaga 780
acacaaacac taaatgaatt gtttgagcat atatactaat gaaatagcat ctgttcatgc 840
atcaataata ccatcattac ttaagaaata agaagaagca aaaattcaa 889

<210> 127
<211> 885
<212> DNA
<213> Human metapneumo virus

<400> 127
atggaagtaa gagtggagaa cattcgggca atagacatgt tcaaagcaaa gatgaaaaac 60
cgtataagaa gtagcaagtg ctatagaaat gctacactga tccttattgg attaacagca 120
ttaagtatgg cacttaatat ttttttaatc attgattatg caatgttaaa aaacatgacc 180
aaagtggaac actgtgttaa tatgccgccg gtagaaccaa gcaagaagac cccaatgacc 240
tctgcagtag acttaaacac caaactcaat ccacagcagg caacacagtt gaccacagag 300
gattcaacat ctctagcagc aacctcggag gatcatttac tcacagggac aactccaaca 360
ccagatgcaa cagtctctca gcaaaccaca gacgagcaca caacactgct gagatcaacc 420
aacagacaga ccacccaaac aaccacagag aaaaagccaa ccggagcaac aaccaaaaaa 480
gaaaccacaa ctcgaaccac aagcacagct gcaacccaaa cactcaacac caccaaccaa 540
actagcaatg gaagagaggc aaccacaaca tccaccagat ccagaaacgg tgccacaact 600
caaaacagcg atcaaacaac ctagacagca gacccaagct cccaaccaca ccatacacag 660
aaaagcacaa caacaacata caacacagac acatcttctc caagtagtta acaaaaaact 720
ataaaataac catgaaaact aaaaaactag aaaagttaat ttgaactcag aaaagaacac 780
aaacactata tgaattgttt gagcgtatat actaatgaaa tagcatctgt ttgtgcatca 840
ataataccat cattatttaa gaaataagaa gaagctaaaa ttcaa 885

<210> 128
<211> 885
<212> DNA
<213> Human metapneumo virus

<400> 128
atggaagtaa gagtggagaa cattcgggca atagacatgt tcaaagcaaa gatgaaaaac 60
cgcataagaa gtagcaagtg ctatagaaat gctacactga tccttattgg attaacagca 120
ttaagtatgg cacttaatat ttttttaatc attgattatg caacattaaa aaacatgacc 180
aaagtggaac actgtgttaa tatgccgccg gtagaaccaa gcaagaagac cccaatgacc 240
tctgcagtag acttaaacac caaactcaat ccacagcagg caacacagtt gaccacagag 300
gattcaacat ctctagcagc aacctcagag ggccatccac acacaggaac aactccaaca 360
ccagacgcaa cagtctctca gcaaaccaca gacgagcaca caacactgct gagatcaacc 420
aacagacaga ccacccaaac agccacagag aaaaagccaa ctggagcaac aaccaaaaaa 480
gaaaccacaa cccgaactac aagtacagct gcaacccaaa cacccaacac caccaaccaa 540
accagcaatg gaagagaggc aaccacaaca tccgccaggt ccagaaacgg tgccacaact 600
caaaacagcg atcaaataac ccaggcagca gactcaagct cccaaccaca ccatacacag 660
aaaagcacaa caacagcata caacacagac acatcttttc caagtagtta acaaaaaact 720
ataaaataac catgaaaacc aaaaaactag aaaagttaat ttgaactcag aaaagaacac 780

aaacactata tgaattgttt gagcgtatat actaatgaaa tagcatctgt ttgtgcatca 840
ataataccat cattatttaa gaaataagaa gaagctaaaa ttcaa 885

<210> 129
<211> 886
<212> DNA
<213> Human metapneumo virus

<400> 129
atggaagtaa gagtggagaa cattcgggca atagacatgt tcaaagcaaa gatgaaaaac 60
cgtataagaa gtagcaagtg ctatagaaat gctacactga tccttattgg attaacagca 120
ctaagtatgg cacttaatat ttttttaatc attgattatg caacattaaa aaacatgacc 180
aaagtggaac actgtgttaa tatgccgccg gtagaaccaa gcaagaagac cccaatgacc 240
tctgcagtag actcaaacac caaacccaat ccacagcagg caacacagtt gaccacagag 300
gattctacat ctttagcagc aaccctagag gaccatccac acacagggac aactccaaca 360
ccagatgcaa cagtctctca gcaaaccaca gacgagcaca caacactgct gagatcaacc 420
aacagacaga ccacccaaac aactgcagag aaaaagccaa ccagggcaac aaccaaaaaa 480
gaaaccacaa ctcgaaccac aagcacagct gcaacccaaa cactcaacac caccaaccaa 540
actagcaatg gaagagaggc aaccacaaca tctgccagat ccagaaacaa tgccacaact 600
caaagcagcg atcaaacaac ccaggcagca gaaccaagct cccaatcaca acatacacag 660
aaaagcacaa caacaacata caacacagac acatcttctc taagtagtta acaaaaaaac 720
tataaaataa ccatgaaaac caaaaaacta gaaaagttaa tttgaactca gaaaagaaca 780
caaacactat atgaattatt tgagcgtata tactaatgaa atagcatctg tttgtgcatc 840
aataatacca tcattattta agaaataaga agaagctaaa attcaa 886

<210> 130
<211> 887
<212> DNA
<213> Human metapneumo virus

<400> 130
atggaagtaa gagtggagaa cattcgggca atagacatgt tcaaagcaaa gatgaaaaac 60
cgtataagaa gtagcaagtg ctatagaaat gctacactga tccttattgg attatcagca 120
ctaagtatgg cacttaatat ttttttaatc attgattatg caaaatcaaa aaacatgacc 180
agagtggaac actgtgtcaa tatgccgccg gtagaaccaa gcaagaagac cccaatgacc 240
tctgcagtag acttaaacac caaacccaat ccacagcggg caacacagtt gaccacagag 300
gattcaacat ctctagcagc aaccctagag ggccatctac acacagggac aactccaaca 360
ccagatgtaa cagtctctca gcaaaccaca gacgagcaca caacactgct gagatcaacc 420
aacagacaga ccacccaaac agccgcagag aaaaagccaa ccagagtaac aactaacaaa 480
gaaaccataa ctcgaaccac aagcacagcc gcaacccaaa cactcaacac caccaaccaa 540
accaacaatg gaagagaggc aaccacaaca tctgccagat ccagaaacaa tgccacaact 600
caaagcagcg accaaacaac ccaggcagca gacccaagct cccaatcaca acatacacag 660
aaaagcataa caacaacata caacacagac acatcttctc caagtagtta acaaaaaaac 720
tataaaataa ccatgaaaac caaaaaaact agaaaagtta atttgaactc agaaaagaac 780
acaaacacta tatgaattgt ttgagcgtat atactaatga aatagcatct gtttgtgcat 840
caataatacc atcattattt aagaattaag aagaagctaa aattcaa 887

<210> 131
<211> 887
<212> DNA
<213> Human metapneumo virus

<400> 131
atggaagtaa gagtggagaa cattcgggca atagacatgt tcaaagcaaa gatgaaaaac 60
cgtataagaa gtagcaagtg ctatagaaat gctacactga tccttattgg attatcagca 120
ctaagtatgg cacttaatat ttttttaatc attgattatg caaaatcaaa aaccatgacc 180
agagtggaac actgtgttaa tatgccgccg gtagaaccaa gcaagaagac cccaatgacc 240
tctgcagtag acttaaacac caaacccaat ccacagcagg caacacagtt gaccacagag 300
gattcaacat ctccagcagc aaccctagag ggccatctac acacagggac aactccaaca 360
ccagatgcaa cagtctctca gcaaaccaca gacgagcaca caacactgct gagatcaacc 420
aacagacaga ccacccaaac aaccgcagag aaaaagccaa ccagagcaac aaccaaaaaa 480
gaaaccataa ctcgaaccac aagcacagct gcaacccaaa cactcaacac caccaaccaa 540

accagcaatg gaagagaggc aaccacaaca tctgccagat ccagaaacaa tgccacaact 600
caaagcagcg accaaacaac ccaggcagca gacccaagct cccaatcaca acatacaaag 660
aaaagcacaa caacaacata caacacagac acatcttctc caagtagtta acaaaaaaac 720
tataaaataa ccatgaaaac caaaaaaact agaaaagtta atttgaactc agaaaagaac 780
acaaacacta tatgaattgt ttgagcgtat atactaatga aatagcatct gtttgtgcat 840
caataatacc atcattattt aagaattaag aagaagctaa aattcaa 887

<210> 132
<211> 886
<212> DNA
<213> Human metapneumo virus

<400> 132
atggaagtaa gagtggagaa cattcgggca atagacatgt tcaaagcaaa gatgaaaaac 60
cgtataagaa gtagcaagtg ctatagaaat gctacactga tccttattgg attaacagca 120
ctaagtatgg cacttaatat ttttttaatc attgattatg caacattaaa aaacatgacc 180
aaagtggaac actgtgttaa tatgccgccg gtagaaccaa gcaagaagac cccaatgacc 240
tctgcagtag acttaaacac caaacccaat ccacagcagg caacacagtt gaccacagag 300
gactctacat ctttagcagc aaccctagag gaccatccac acacagggac aactccaaca 360
ccagatgcaa cagtctctca gcaaaccaca gacgagcaca caacactgct gagatcaacc 420
aacagacaga ccacccaaac aactgcagag aaaaagccaa ccagagcaac aaccaaaaaa 480
gaaaccacaa ctcgaaccac aagcacagct gcaacccaaa cactcaacac caccaaccaa 540
actagcaatg gaagagaggc aaccacaaca tctgccagat ccagaaacaa tgccacaact 600
caaagcagcg atcaaacaac ccaagcagca gaaccaaact cccaatcaca acatacacag 660
aaaagcacaa caacaacata caacacagac acatcttctc taagtagtta acaaaaaaac 720
tataaaataa ccatgaaaac caaaaaacta gaaaagttaa tttgaactca gaaaggaaca 780
caaacactat atgaattatt tgagcgtata tactaatgaa atagcatctg tttgtgcatc 840
aataatacca tcattattta agaaataaga agaagctaaa attcaa 886

<210> 133
<211> 236
<212> PRT
<213> Human metapneumo virus

<400> 133
Met Glu Val Lys Val Glu Asn Ile Arg Thr Ile Asp Met Leu Lys Ala
1               5                   10                  15
Arg Val Lys Asn Arg Val Ala Arg Ser Lys Cys Phe Lys Asn Ala Ser
            20                  25                  30
Leu Val Leu Ile Gly Ile Thr Thr Leu Ser Ile Ala Leu Asn Ile Tyr
        35                  40                  45
Leu Ile Ile Asn Tyr Lys Met Gln Lys Asn Thr Ser Glu Ser Glu His
    50                  55                  60
His Thr Ser Ser Ser Pro Met Glu Ser Ser Arg Glu Thr Pro Thr Val
65                  70                  75                  80
Pro Thr Asp Asn Ser Asp Thr Asn Ser Ser Pro Gln His Pro Thr Gln
                85                  90                  95
Gln Ser Thr Glu Gly Ser Thr Leu Tyr Phe Ala Ala Ser Ala Ser Ser
            100                 105                 110
Pro Glu Thr Glu Pro Thr Ser Thr Pro Asp Thr Thr Asn Arg Pro Pro
        115                 120                 125
Phe Val Asp Thr His Thr Thr Pro Pro Ser Ala Ser Arg Thr Lys Thr
    130                 135                 140
Ser Pro Ala Val His Thr Lys Asn Asn Pro Arg Thr Ser Ser Arg Thr
145                 150                 155                 160
His Ser Pro Pro Arg Ala Thr Thr Arg Thr Ala Arg Arg Thr Thr Thr
                165                 170                 175
Leu Arg Thr Ser Ser Thr Arg Lys Arg Pro Ser Thr Ala Ser Val Gln
            180                 185                 190
Pro Asp Ile Ser Ala Thr Thr His Lys Asn Glu Glu Ala Ser Pro Ala
        195                 200                 205
Ser Pro Gln Thr Ser Ala Ser Thr Thr Arg Ile Gln Arg Lys Ser Val

210                 215                 220
Glu Ala Asn Thr Ser Thr Thr Tyr Asn Gln Thr Ser
225                 230                 235

<210> 134
<211> 236
<212> PRT
<213> Human metapneumo virus

<400> 134
Met Glu Val Lys Val Glu Asn Ile Arg Thr Ile Asp Met Leu Lys Ala
1               5                   10                  15
Ser Val Lys Asn Arg Val Ala Arg Ser Lys Cys Phe Lys Asn Ala Ser
            20                  25                  30
Leu Val Leu Ile Gly Ile Thr Thr Leu Ser Ile Ala Leu Asn Ile Tyr
        35                  40                  45
Leu Ile Ile Asn Tyr Lys Met Gln Lys Asn Thr Ser Glu Ser Glu His
    50                  55                  60
His Thr Ser Ser Ser Pro Met Glu Ser Ser Arg Glu Thr Pro Thr Val
65                  70                  75                  80
Pro Thr Asp Asn Ser Asp Thr Asn Ser Ser Pro Gln His Pro Thr Gln
                85                  90                  95
Gln Ser Thr Glu Gly Ser Thr Leu Tyr Phe Ala Ala Ser Ala Ser Ser
            100                 105                 110
Pro Glu Thr Glu Pro Thr Ser Thr Pro Asp Thr Thr Asn Arg Pro Pro
        115                 120                 125
Phe Val Asp Thr His Thr Thr Pro Pro Ser Ala Ser Arg Thr Lys Thr
    130                 135                 140
Ser Pro Ala Val His Thr Lys Asn Asn Pro Arg Thr Ser Ser Arg Thr
145                 150                 155                 160
His Ser Pro Pro Arg Ala Thr Thr Arg Thr Ala Arg Arg Thr Thr Thr
                165                 170                 175
Leu Arg Thr Ser Ser Thr Arg Lys Arg Pro Ser Thr Ala Ser Val Gln
            180                 185                 190
Pro Asp Ile Ser Ala Thr Thr His Lys Asn Glu Glu Ala Ser Pro Ala
        195                 200                 205
Ser Pro Gln Thr Ser Ala Ser Thr Thr Arg Ile Gln Arg Lys Ser Val
    210                 215                 220
Glu Ala Asn Thr Ser Thr Thr Tyr Asn Gln Thr Ser
225                 230                 235

<210> 135
<211> 236
<212> PRT
<213> Human metapneumo virus

<400> 135
Met Glu Val Lys Val Glu Asn Ile Arg Thr Ile Asp Met Leu Lys Ala
1               5                   10                  15
Arg Val Lys Asn Arg Val Ala Arg Ser Lys Cys Phe Lys Asn Ala Ser
            20                  25                  30
Leu Val Leu Ile Gly Ile Thr Thr Leu Ser Ile Ala Leu Asn Ile Tyr
        35                  40                  45
Leu Ile Ile Asn Tyr Lys Met Gln Lys Asn Thr Ser Glu Ser Glu His
    50                  55                  60
His Thr Ser Ser Ser Pro Met Glu Ser Ser Arg Glu Thr Pro Thr Val
65                  70                  75                  80
Pro Thr Asp Asn Ser Asp Thr Asn Ser Ser Pro Gln His Pro Thr Gln
                85                  90                  95
Gln Ser Thr Glu Gly Ser Thr Leu Tyr Phe Ala Ala Ser Ala Asn Ser

                100                 105                 110
Pro Glu Thr Glu Pro Thr Ser Thr Pro Asp Thr Thr Asn Arg Pro Pro
            115                 120                 125
Phe Val Asp Thr His Thr Thr Pro Pro Ser Ala Ser Arg Thr Lys Thr
    130                 135                 140
Ser Pro Ala Val His Thr Lys Asn Asn Pro Arg Ile Ser Ser Arg Thr
145                 150                 155                 160
His Ser Pro Pro Trp Ala Thr Thr Arg Thr Ala Arg Arg Thr Thr Thr
                165                 170                 175
Leu Arg Thr Ser Ser Thr Arg Lys Arg Pro Ser Thr Ala Ser Ala Gln
            180                 185                 190
Pro Asp Ile Ser Ala Thr Thr His Lys Asn Glu Glu Ala Ser Pro Ala
        195                 200                 205
Ser Pro Gln Thr Ser Ala Ser Thr Thr Arg Thr Gln Arg Lys Ser Val
    210                 215                 220
Glu Ala Asn Thr Ser Thr Thr Tyr Asn Gln Thr Ser
225                 230                 235

<210> 136
<211> 236
<212> PRT
<213> Human metapneumo virus

<400> 136
Met Glu Val Lys Val Glu Asn Ile Arg Thr Ile Asp Met Leu Lys Ala
 1               5                  10                  15
Arg Val Lys Asn Arg Val Ala Arg Ser Lys Cys Phe Lys Asn Ala Ser
            20                  25                  30
Leu Val Leu Ile Gly Ile Thr Thr Leu Ser Ile Ala Leu Asn Ile Tyr
        35                  40                  45
Leu Ile Ile Asn Tyr Lys Met Gln Lys Asn Thr Ser Glu Ser Glu His
    50                  55                  60
His Thr Ser Ser Ser Pro Met Glu Ser Ser Arg Glu Thr Pro Thr Val
65                  70                  75                  80
Pro Thr Asp Asn Ser Asp Thr Asn Ser Ser Pro Gln His Pro Thr Gln
                85                  90                  95
Gln Ser Thr Glu Gly Ser Thr Leu Tyr Phe Ala Ala Ser Ala Asn Ser
            100                 105                 110
Pro Glu Thr Glu Pro Thr Ser Thr Pro Asp Thr Thr Asp Arg Pro Pro
        115                 120                 125
Phe Val Asp Thr His Thr Thr Pro Pro Ser Ala Ser Arg Thr Lys Thr
    130                 135                 140
Ser Pro Ala Val His Thr Lys Asn Asn Pro Arg Ile Ser Ser Arg Thr
145                 150                 155                 160
His Ser Pro Pro Trp Ala Thr Thr Arg Thr Ala Arg Arg Thr Thr Thr
                165                 170                 175
Leu Arg Thr Ser Ser Thr Arg Lys Arg Pro Ser Thr Ala Ser Val Gln
            180                 185                 190
Pro Asp Ile Ser Ala Thr Thr His Lys Asn Glu Glu Ala Ser Pro Ala
        195                 200                 205
Ser Pro Gln Thr Ser Ala Ser Thr Thr Arg Thr Gln Arg Lys Ser Val
    210                 215                 220
Glu Ala Asn Thr Ser Thr Thr Tyr Asn Gln Thr Ser
225                 230                 235

<210> 137
<211> 236
<212> PRT
<213> Human metapneumo virus

<400> 137
Met Glu Val Lys Val Glu Asn Ile Arg Thr Ile Asp Met Leu Lys Ala
1               5                   10                  15
Arg Val Lys Asn Arg Val Ala Arg Ser Lys Cys Phe Lys Asn Ala Ser
            20                  25                  30
Leu Val Leu Ile Gly Ile Thr Thr Leu Ser Ile Ala Leu Asn Ile Tyr
        35                  40                  45
Leu Ile Ile Asn Tyr Lys Met Gln Lys Asn Thr Ser Glu Ser Glu His
    50                  55                  60
His Thr Ser Ser Ser Pro Met Glu Ser Ser Arg Glu Thr Pro Thr Val
65                  70                  75                  80
Pro Thr Asp Asn Ser Asp Thr Asn Ser Ser Pro Gln His Pro Thr Gln
                85                  90                  95
Gln Ser Thr Glu Gly Ser Thr Leu Tyr Phe Ala Ala Ser Ala Ser Ser
            100                 105                 110
Pro Glu Thr Glu Pro Thr Ser Thr Pro Asp Thr Thr Asp Arg Pro Pro
        115                 120                 125
Phe Val Asp Thr His Thr Thr Pro Pro Ser Ala Ser Arg Thr Lys Thr
    130                 135                 140
Ser Pro Ala Val His Thr Lys Asn Asn Pro Arg Ile Ser Ser Arg Thr
145                 150                 155                 160
His Ser Pro Pro Trp Ala Thr Thr Arg Thr Ala Arg Arg Thr Thr Thr
                165                 170                 175
Leu Arg Thr Ser Ser Thr Arg Lys Arg Pro Ser Thr Ala Ser Val Gln
            180                 185                 190
Pro Asp Ile Ser Ala Thr Thr His Lys Asn Glu Glu Ala Ser Pro Ala
        195                 200                 205
Ser Pro Gln Thr Ser Ala Ser Thr Thr Arg Thr Gln Arg Lys Ser Val
    210                 215                 220
Glu Ala Asn Thr Ser Thr Thr Tyr Asn Gln Thr Ser
225                 230                 235

<210> 138
<211> 236
<212> PRT
<213> Human metapneumo virus

<400> 138
Met Glu Val Lys Val Glu Asn Ile Arg Thr Ile Asp Met Leu Lys Ala
1               5                   10                  15
Arg Val Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Met Gln Glu Asn Thr Ser Glu Ser Glu His
    50                  55                  60
His Thr Ser Ser Ser Pro Met Glu Ser Ser Arg Glu Thr Pro Thr Val
65                  70                  75                  80
Pro Ile Asp Asn Ser Asp Thr Asn Pro Gly Ser Gln Tyr Pro Thr Gln
                85                  90                  95
Gln Ser Thr Glu Asp Ser Thr Leu His Ser Ala Ala Ser Ala Ser Ser
            100                 105                 110
Pro Glu Thr Glu Pro Thr Ser Thr Pro Asp Thr Thr Ser Arg Pro Pro
        115                 120                 125
Phe Val Asp Thr His Thr Thr Pro Pro Ser Ala Ser Arg Thr Arg Thr
    130                 135                 140
Ser Pro Ala Val His Thr Lys Asn Asn Pro Arg Val Ser Pro Arg Thr
145                 150                 155                 160
His Ser Pro Pro Trp Ala Met Thr Arg Thr Val Arg Gly Thr Thr Thr
                165                 170                 175
Leu Arg Thr Ser Ser Thr Arg Lys Arg Leu Ser Thr Ala Ser Val Gln

180                 185                 190
Pro Asp Ser Ser Ala Thr Thr His Lys His Glu Glu Thr Ser Pro Val
        195                 200                 205
Ser Pro Gln Thr Ser Ala Ser Thr Ala Arg Pro Gln Arg Lys Gly Met
    210                 215                 220
Glu Ala Ser Thr Ser Thr Thr Tyr Asn Gln Thr Ser
225                 230                 235

<210> 139
<211> 236
<212> PRT
<213> Human metapneumo virus

<400> 139
Met Glu Val Lys Val Glu Asn Ile Arg Thr Ile Asp Met Leu Lys Ala
 1               5                  10                  15
Arg Val Lys Asn Arg Val Ala Arg Ser Lys Cys Phe Lys Asn Ala Ser
                20                  25                  30
Leu Ile Leu Ile Gly Ile Thr Thr Leu Ser Ile Ala Leu Asn Ile Tyr
        35                  40                  45
Leu Ile Ile Asn Tyr Thr Met Gln Glu Asn Thr Ser Glu Ser Glu His
    50                  55                  60
His Thr Ser Ser Ser Pro Met Glu Ser Ser Arg Glu Thr Pro Thr Val
65                  70                  75                  80
Pro Met Asp Asn Ser Asp Thr Asn Pro Gly Ser Gln Tyr Pro Thr Gln
                85                  90                  95
Gln Ser Thr Glu Gly Ser Thr Leu His Phe Ala Ala Ser Ala Ser Ser
                100                 105                 110
Pro Glu Thr Glu Pro Thr Ser Thr Pro Asp Thr Thr Ser Arg Pro Pro
        115                 120                 125
Phe Val Asp Thr His Thr Thr Pro Ser Ser Ala Ser Arg Thr Lys Thr
    130                 135                 140
Ser Pro Ala Val His Thr Lys Asn Asn Leu Arg Ile Ser Pro Arg Thr
145                 150                 155                 160
His Ser Pro Pro Trp Ala Met Thr Arg Thr Val Arg Gly Thr Thr Thr
                165                 170                 175
Leu Arg Thr Ser Ser Ile Arg Lys Arg Pro Ser Thr Ala Ser Val Gln
                180                 185                 190
Pro Asp Ser Ser Ala Thr Thr His Lys His Glu Glu Ala Ser Pro Val
        195                 200                 205
Ser Pro Gln Ala Ser Ala Ser Thr Ala Arg Pro Gln Arg Lys Gly Met
    210                 215                 220
Glu Ala Ser Thr Ser Thr Thr Tyr Asn Gln Thr Ser
225                 230                 235

<210> 140
<211> 236
<212> PRT
<213> Human metapneumo virus

<400> 140
Met Glu Val Lys Val Glu Asn Ile Arg Thr Ile Asp Met Leu Lys Ala
 1               5                  10                  15
Arg Val Lys Asn Arg Val Ala Arg Ser Lys Cys Phe Lys Asn Ala Ser
                20                  25                  30
Leu Ile Leu Ile Gly Ile Thr Thr Leu Ser Ile Ala Leu Asn Ile Tyr
        35                  40                  45
Leu Ile Ile Asn Tyr Thr Met Gln Glu Asn Thr Ser Glu Ser Glu His
    50                  55                  60
His Thr Ser Ser Ser Pro Met Glu Ser Ser Arg Glu Thr Pro Thr Val

65                  70                  75                  80
Pro Met Asp Asn Ser Asp Thr Asn Pro Gly Ser Gln Tyr Pro Thr Gln
                85                  90                  95
Gln Ser Thr Glu Gly Ser Thr Leu His Phe Ala Ala Ser Ala Ser Ser
            100                 105                 110
Pro Glu Thr Glu Pro Thr Ser Thr Pro Asp Thr Thr Ser Arg Pro Pro
        115                 120                 125
Phe Val Asp Thr His Thr Thr Pro Ser Ser Ala Ser Arg Ile Arg Thr
    130                 135                 140
Ser Pro Ala Val His Thr Lys Asn Asn Leu Arg Ile Ser Pro Arg Thr
145                 150                 155                 160
His Ser Pro Pro Trp Ala Met Thr Arg Thr Val Arg Gly Thr Thr Thr
                165                 170                 175
Leu Arg Thr Ser Ser Ile Arg Lys Arg Pro Ser Thr Ala Ser Val Gln
            180                 185                 190
Pro Asp Ser Ser Ala Thr Thr His Lys His Glu Glu Ala Ser Pro Val
        195                 200                 205
Ser Pro Gln Ala Ser Ala Ser Thr Ala Arg Pro Gln Arg Lys Gly Met
    210                 215                 220
Glu Ala Ser Thr Ser Thr Thr Tyr Asn Gln Thr Ser
225                 230                 235


<210> 141
<211> 228
<212> PRT
<213> Human metapneumovirus

<220>
<221> VARIANT
<222> 220
<223> Xaa = unknown amino acid or other

<400> 141
Met Glu Val Lys Val Glu Asn Ile Arg Ala Ile Asp Met Leu Lys Ala
 1               5                  10                  15
Arg Val Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Lys Thr Thr Ser Glu Ser Glu His
    50                  55                  60
His Thr Ser Ser Pro Pro Thr Glu Pro Asn Lys Glu Ala Ser Thr Ile
65                  70                  75                  80
Ser Thr Asp Asn Pro Asp Ile Asn Pro Ser Ser Gln His Pro Thr Gln
                85                  90                  95
Gln Ser Thr Glu Asn Pro Thr Leu Asn Pro Ala Ala Ser Ala Ser Pro
            100                 105                 110
Ser Glu Thr Glu Pro Ala Ser Thr Pro Asp Thr Thr Asn Arg Leu Ser
        115                 120                 125
Ser Val Asp Arg Ser Thr Ala Gln Pro Ser Glu Ser Arg Thr Lys Thr
    130                 135                 140
Lys Pro Thr Val His Thr Ile Asn Asn Pro Asn Thr Ala Ser Ser Thr
145                 150                 155                 160
Gln Ser Pro Pro Arg Thr Thr Thr Lys Ala Ile Arg Arg Ala Thr Thr
                165                 170                 175
Phe Arg Met Ser Ser Thr Gly Lys Arg Pro Thr Thr Thr Leu Val Gln
            180                 185                 190
Ser Asp Ser Ser Thr Thr Thr Gln Asn His Glu Glu Thr Gly Ser Ala
        195                 200                 205
Asn Pro Gln Ala Ser Ala Ser Thr Met Gln Asn Xaa His Thr Asn Asn
    210                 215                 220

Ile Lys Pro Asn
225

<210> 142
<211> 228
<212> PRT
<213> Human metapneumo virus

<400> 142
Met Glu Val Lys Val Glu Asn Ile Arg Ala Ile Asp Met Leu Lys Ala
1               5                   10                  15
Arg Val Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Lys Thr Thr Ser Glu Ser Glu His
    50                  55                  60
His Thr Ser Ser Pro Pro Thr Glu Ser Asn Lys Glu Thr Ser Thr Ile
65                  70                  75                  80
Pro Ile Asp Asn Pro Asp Ile Asn Pro Asn Ser Gln His Pro Thr Gln
                85                  90                  95
Gln Ser Thr Glu Ser Pro Thr Leu Asn Pro Ala Ala Ser Val Ser Pro
            100                 105                 110
Ser Glu Thr Glu Pro Ala Ser Thr Pro Asp Thr Thr Asn Arg Leu Ser
        115                 120                 125
Ser Val Asp Arg Ser Thr Thr Gln Pro Ser Glu Ser Arg Thr Lys Thr
    130                 135                 140
Lys Pro Thr Val His Thr Lys Asn Asn Pro Ser Thr Val Ser Arg Thr
145                 150                 155                 160
Gln Ser Pro Leu Arg Ala Thr Thr Lys Ala Val Leu Arg Ala Thr Ala
                165                 170                 175
Phe Arg Thr Ser Ser Thr Arg Lys Arg Pro Thr Thr Thr Ser Val Gln
            180                 185                 190
Ser Asp Ser Ser Thr Thr Thr Gln Asn His Glu Glu Thr Ser Ser Ala
        195                 200                 205
Asn Pro Gln Ala Ser Ala Ser Thr Met Gln Ser Gln His Thr Asn Asn
    210                 215                 220
Ile Lys Pro Asn
225

<210> 143
<211> 228
<212> PRT
<213> Human metapneumo virus

<400> 143
Met Glu Val Lys Val Glu Asn Ile Arg Ala Val Asp Met Leu Lys Ala
1               5                   10                  15
Arg Val Lys Asn Arg Val Ala Arg Ser Lys Cys Phe Lys Asn Ala Ser
            20                  25                  30
Leu Ile Leu Val Gly Ile Thr Thr Leu Ser Ile Ala Leu Asn Ile Tyr
        35                  40                  45
Leu Ile Val Asn Tyr Thr Ile Gln Lys Thr Thr Ser Glu Ser Glu His
    50                  55                  60
His Thr Ser Ser Ser Pro Thr Glu Ser Asn Lys Gly Thr Ser Thr Ile
65                  70                  75                  80
Pro Thr Asp Asn Pro Asp Ile Asn Pro Asn Ser Gln His Pro Thr Gln
                85                  90                  95
Gln Ser Thr Glu Ser Pro Thr Leu Asn Thr Ala Ala Ser Val Ser Pro
            100                 105                 110

Ser Glu Thr Glu Pro Ala Ser Thr Pro Asp Thr Thr Asn Arg Leu Ser
        115                 120                 125
Ser Ala Asp Arg Ser Thr Thr Gln Pro Ser Glu Ser Arg Thr Lys Thr
    130                 135                 140
Lys Leu Thr Val His Thr Lys Asn Asn Leu Ser Thr Ala Ser Arg Thr
145                 150                 155                 160
Gln Ser Pro Pro Arg Ala Thr Thr Lys Ala Val Leu Arg Asp Thr Ala
                165                 170                 175
Phe His Thr Ser Ser Thr Gly Lys Arg Pro Thr Thr Thr Ser Val Gln
            180                 185                 190
Ser Gly Ser Ser Thr Thr Thr Gln Asn His Glu Glu Thr Ser Ser Ser
        195                 200                 205
Asn Pro Gln Ala Ser Ala Ser Thr Met Gln Asp Gln Asp Thr Asn Asn
    210                 215                 220
Thr Lys Gln Asn
225


<210> 144
<211> 228
<212> PRT
<213> Human metapneumo virus

<220>
<221> VARIANT
<222> 81
<223> Xaa = Any Amino Acid

<400> 144
Met Glu Val Lys Val Glu Asn Ile Arg Ala Val Asp Met Leu Lys Ala
 1               5                  10                  15
Arg Val Lys Asn Arg Val Ala Arg Ser Lys Cys Phe Lys Asn Ala Ser
            20                  25                  30
Leu Ile Leu Val Gly Ile Thr Thr Leu Ser Ile Ala Leu Asn Ile Tyr
        35                  40                  45
Leu Ile Val Asn Tyr Thr Ile Gln Lys Thr Thr Ser Glu Ser Glu His
    50                  55                  60
His Thr Ser Ser Ser Pro Thr Glu Ser Asn Lys Gly Thr Ser Thr Ile
65                  70                  75                  80
Xaa Thr Asp Asn Pro Asp Ile Asn Pro Asn Ser Gln His Pro Thr Gln
                85                  90                  95
Gln Ser Thr Glu Ser Pro Thr Leu Asn Thr Ala Ala Ser Val Ser Pro
            100                 105                 110
Ser Glu Thr Glu Pro Ala Ser Thr Pro Asp Thr Thr Asn Arg Leu Ser
        115                 120                 125
Ser Ala Asp Arg Ser Thr Thr Gln Pro Ser Glu Ser Arg Thr Lys Thr
    130                 135                 140
Lys Leu Thr Val His Thr Lys Asn Asn Leu Ser Thr Ala Ser Arg Thr
145                 150                 155                 160
Gln Ser Pro Pro Arg Ala Thr Thr Lys Ala Val Leu Arg Asp Thr Ala
                165                 170                 175
Phe His Thr Ser Ser Thr Gly Lys Arg Pro Thr Thr Thr Ser Val Gln
            180                 185                 190
Ser Gly Ser Ser Thr Thr Thr Gln Asn His Glu Glu Thr Ser Ser Ser
        195                 200                 205
Asn Pro Gln Ala Ser Ala Ser Thr Met Gln Asp Gln Asp Thr Asn Asn
    210                 215                 220
Thr Lys Gln Asn
225


<210> 145

<211> 228
<212> PRT
<213> Human metapneumovirus

<220>
<221> VARIANT
<222> 220
<223> Xaa = unknown amino acid or other

<400> 145
Met Glu Val Lys Val Glu Asn Ile Arg Ala Ile Asp Met Leu Lys Ala
1               5                   10                  15
Arg Met Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Lys Thr Thr Ser Glu Ser Glu His
    50                  55                  60
His Thr Ser Ser Pro Pro Thr Glu Ser Asn Lys Glu Thr Ser Thr Ile
65                  70                  75                  80
Pro Ile Asp Asn Pro Asp Ile Asn Pro Asn Ser Gln His Pro Thr Gln
                85                  90                  95
Gln Ser Thr Glu Ser Leu Thr Leu Asn Pro Ala Ala Ser Val Ser Pro
            100                 105                 110
Ser Glu Thr Glu Pro Ala Ser Thr Pro Asp Thr Thr Asn Arg Leu Ser
        115                 120                 125
Ser Val Asp Arg Ser Thr Thr Gln Pro Ser Glu Ser Arg Thr Lys Thr
    130                 135                 140
Lys Leu Thr Val His Lys Lys Asn Ile Pro Ser Thr Val Ser Arg Thr
145                 150                 155                 160
Gln Ser Ser Ile Arg Ala Thr Thr Lys Ala Val Leu Arg Ala Thr Ala
                165                 170                 175
Phe Arg Thr Ser Ser Thr Gly Glu Arg Pro Thr Thr Thr Ser Val Gln
            180                 185                 190
Ser Asp Ser Ser Thr Thr Thr Gln Asn His Glu Glu Thr Gly Ser Ala
        195                 200                 205
Asn Pro Gln Ala Ser Ala Ser Thr Met Gln Asn Xaa His Thr Asn Ile
    210                 215                 220
Val Lys Pro Asn
225


<210> 146
<211> 228
<212> PRT
<213> Human metapneumovirus

<220>
<221> VARIANT
<222> 220
<223> Xaa = unknown amino acid or other

<400> 146
Met Glu Val Lys Val Glu Asn Ile Arg Ala Ile Asp Met Leu Lys Ala
1               5                   10                  15
Arg Val Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Lys Thr Thr Ser Glu Ser Glu His
    50                  55                  60
His Thr Ser Ser Pro Pro Thr Glu Ser Asn Lys Glu Thr Ser Thr Ile

65                  70                  75                  80
Ser Ile Asp Asn Pro Asp Ile Asn Pro Asn Ser Gln His Pro Thr Gln
                85                  90                  95
Gln Ser Thr Glu Ser Leu Thr Leu Ser Pro Thr Ala Ser Val Ser Pro
            100                 105                 110
Ser Glu Thr Glu Pro Ala Ser Thr Ser Asp Thr Thr Ser Arg Leu Ser
        115                 120                 125
Ser Val Asp Arg Ser Thr Thr Gln Pro Ser Glu Ser Arg Ala Arg Thr
    130                 135                 140
Lys Pro Thr Val His Lys Lys Asn Ile Pro Ser Thr Val Ser Arg Thr
145                 150                 155                 160
Gln Ser Pro Leu Arg Ala Thr Thr Lys Ala Val Leu Arg Ala Thr Ala
                165                 170                 175
Phe Arg Thr Ser Ser Thr Gly Glu Gly Pro Thr Thr Thr Ser Val Gln
            180                 185                 190
Ser Asp Ser Ser Thr Thr Thr Gln Asn His Glu Glu Thr Gly Ser Ala
        195                 200                 205
Asn Pro Gln Ala Ser Ala Ser Thr Met Gln Asn Xaa His Thr Asn Ile
    210                 215                 220
Val Lys Pro Asn
225

<210> 147
<211> 228
<212> PRT
<213> Human metapneumovirus

<220>
<221> VARIANT
<222> 220
<223> Xaa = unknown amino acid or other

<400> 147
Met Glu Val Lys Val Glu Asn Ile Arg Ala Ile Asp Met Leu Lys Ala
 1               5                  10                  15
Arg Val Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Lys Thr Thr Ser Glu Ser Glu His
    50                  55                  60
His Thr Ser Ser Pro Pro Thr Glu Ser Asn Lys Glu Ala Ser Thr Ile
65                  70                  75                  80
Ser Thr Asp Asn Pro Asp Ile Asn Pro Asn Ser Gln His Pro Thr Gln
                85                  90                  95
Gln Ser Thr Glu Asn Pro Thr Leu Asn Pro Ala Ala Ser Val Ser Ser
            100                 105                 110
Ser Glu Thr Glu Pro Ala Ser Thr Pro Asp Thr Thr Asn Arg Leu Ser
        115                 120                 125
Ser Val Asp Arg Ser Thr Ala Gln Pro Ser Glu Ser Arg Thr Lys Thr
    130                 135                 140
Lys Pro Thr Val His Thr Arg Asn Asn Pro Ser Thr Ala Ser Ser Thr
145                 150                 155                 160
Gln Ser Pro Pro Arg Val Thr Thr Lys Ala Ile Leu Arg Ala Thr Val
                165                 170                 175
Phe Arg Met Ser Ser Thr Gly Lys Arg Pro Ala Thr Thr Leu Val Gln
            180                 185                 190
Ser Asp Ser Ser Thr Thr Thr Gln Asn His Glu Glu Thr Gly Ser Ala
        195                 200                 205
Asn Ser Gln Ala Ser Ala Ser Thr Met Gln Asn Xaa His Ser Asn Asn
    210                 215                 220

Ile Lys Pro Asn
225

<210> 148
<211> 228
<212> PRT
<213> Human metapneumo virus

<400> 148
Met Glu Val Lys Val Glu Asn Ile Arg Ala Ile Asp Met Leu Lys Ala
1               5                   10                  15
Arg Val Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Lys Thr Thr Ser Glu Ser Glu His
    50                  55                  60
His Thr Ser Ser Pro Pro Thr Glu Ser Asn Lys Glu Thr Ser Thr Ile
65                  70                  75                  80
Ser Ile Asp Asn Ser Asp Ile Asn Pro Asn Ser Gln His Pro Thr Gln
                85                  90                  95
Gln Ser Thr Glu Ser Leu Thr Leu Ser Pro Thr Ala Ser Val Ser Pro
            100                 105                 110
Ser Glu Thr Glu Pro Ala Ser Thr Ser Asp Thr Thr Asn Arg Leu Ser
        115                 120                 125
Ser Val Asp Arg Ser Thr Thr Gln Pro Ser Glu Ser Arg Ala Arg Thr
    130                 135                 140
Lys Pro Thr Val His Lys Lys Asn Ile Pro Ser Thr Val Ser Arg Thr
145                 150                 155                 160
Gln Ser Pro Leu Arg Ala Thr Thr Lys Ala Val Leu Arg Ala Thr Ala
                165                 170                 175
Phe Arg Met Ser Ser Thr Gly Glu Gly Pro Thr Thr Thr Ser Val Gln
            180                 185                 190
Ser Asp Ser Ser Thr Thr Thr Gln Asn His Glu Glu Thr Gly Ser Ala
        195                 200                 205
Asn Pro Gln Ala Ser Ala Ser Thr Met Gln Asn Gln His Thr Asn Ile
    210                 215                 220
Ala Lys Pro Asn
225

<210> 149
<211> 228
<212> PRT
<213> Human metapneumo virus

<400> 149
Met Glu Val Lys Val Glu Asn Ile Arg Ala Ile Asp Met Leu Lys Ala
1               5                   10                  15
Arg Val Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Lys Thr Thr Ser Glu Ser Glu His
    50                  55                  60
His Thr Ser Ser Pro Pro Thr Glu Ser Asn Lys Glu Thr Ser Thr Ile
65                  70                  75                  80
Pro Ile Asp Asn Pro Asp Ile Asn Pro Asn Ser Gln His Pro Thr Gln
                85                  90                  95
Gln Ser Thr Glu Ser Leu Thr Leu Tyr Pro Thr Ser Ser Val Ser Ser
            100                 105                 110

Ser Glu Thr Glu Pro Ala Ser Thr Pro Gly Ile Thr Asn His Leu Ser
        115                 120                 125
Phe Val Asp Arg Ser Thr Thr Gln Pro Ser Glu Ser Arg Thr Lys Thr
    130                 135                 140
Asn Arg Thr Val His Lys Lys Asn Ile Ser Ser Thr Val Ser Arg Thr
145                 150                 155                 160
Gln Ser Pro Pro Arg Thr Thr Ala Lys Ala Val Pro Arg Ala Thr Ala
                165                 170                 175
Leu Arg Thr Ser Ser Thr Gly Glu Arg Pro Thr Thr Thr Pro Val Gln
            180                 185                 190
Pro Asp Ser Ser Thr Thr Thr Gln Asn His Glu Glu Thr Gly Ser Ala
        195                 200                 205
Asn Pro Gln Ala Ser Ala Ser Thr Met Gln Asn Gln His Thr Asn Ile
    210                 215                 220
Ala Arg Pro Asn
225

<210> 150
<211> 228
<212> PRT
<213> Human metapneumo virus

<400> 150
Met Glu Val Lys Val Glu Asn Ile Arg Ala Ile Asp Met Leu Lys Ala
 1               5                  10                  15
Arg Val Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Lys Thr Thr Ser Glu Ser Glu His
    50                  55                  60
His Thr Ser Ser Pro Pro Thr Glu Ser Asn Lys Glu Thr Ser Thr Ile
65                  70                  75                  80
Pro Ile Asp Asn Pro Asp Ile Asn Pro Asn Ser Gln His Pro Thr Gln
                85                  90                  95
Gln Ser Ala Glu Ser Leu Thr Leu Tyr Pro Thr Ser Ser Val Ser Ser
            100                 105                 110
Ser Glu Thr Glu Pro Ala Ser Thr Pro Gly Ile Thr Asn His Leu Ser
        115                 120                 125
Phe Val Asp Arg Ser Thr Thr Gln Pro Ser Glu Ser Arg Thr Lys Thr
    130                 135                 140
Asn Arg Thr Val His Lys Lys Asn Ile Ser Ser Thr Val Ser Arg Thr
145                 150                 155                 160
Gln Ser Pro Pro Arg Thr Thr Ala Lys Ala Val Pro Arg Ala Thr Ala
                165                 170                 175
Leu Arg Thr Ser Ser Thr Gly Glu Arg Pro Thr Thr Thr Pro Val Gln
            180                 185                 190
Pro Asp Ser Ser Thr Thr Thr Gln Asn His Glu Glu Thr Gly Ser Ala
        195                 200                 205
Asn Pro Gln Ala Ser Ala Ser Thr Met Gln Asn Gln His Thr Asn Ile
    210                 215                 220
Ala Arg Pro Asn
225

<210> 151
<211> 228
<212> PRT
<213> Human metapneumo virus

<400> 151

Met Glu Val Lys Val Glu Asn Ile Arg Ala Ile Asp Met Leu Lys Ala
1               5                   10                  15
Arg Val Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Lys Thr Thr Ser Glu Ser Glu His
    50                  55                  60
His Thr Ser Ser Pro Pro Thr Glu Ser Asn Lys Glu Thr Ser Thr Ile
65                  70                  75                  80
Pro Ile Asp Asn Pro Asp Ile Asn Pro Asn Ser Gln His Pro Thr Gln
                85                  90                  95
Gln Ser Thr Glu Ser Leu Thr Leu Tyr Pro Thr Ser Ser Val Ser Ser
            100                 105                 110
Ser Glu Thr Glu Pro Ala Ser Thr Pro Gly Ile Thr Asn His Leu Ser
        115                 120                 125
Phe Val Asp Arg Ser Thr Thr Gln Pro Ser Glu Ser Arg Thr Lys Thr
    130                 135                 140
Asn Arg Thr Val His Lys Lys Asn Ile Ser Ser Thr Val Ser Arg Thr
145                 150                 155                 160
Gln Ser Pro Pro Arg Thr Thr Ala Lys Ala Val Pro Arg Ala Thr Ala
                165                 170                 175
Leu Arg Thr Ser Ser Thr Gly Glu Arg Pro Thr Thr Thr Pro Val Gln
            180                 185                 190
Pro Asp Ser Ser Thr Thr Thr Gln Asn His Glu Glu Thr Gly Ser Ala
        195                 200                 205
Asn Pro Gln Ala Ser Ala Ser Thr Met Gln Asn Gln His Thr Asn Ile
    210                 215                 220
Ala Arg Pro Asn
225

<210> 152
<211> 228
<212> PRT
<213> Human metapneumo virus

<400> 152
Met Glu Val Lys Val Glu Asn Ile Arg Ala Ile Asp Met Leu Lys Ala
1               5                   10                  15
Arg Val Lys Asn Arg Val Ala Arg Ser Lys Cys Phe Lys Asn Ala Ser
            20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Gln Thr Thr Ser Glu Ser Glu His
    50                  55                  60
His Thr Ser Ser Pro Pro Thr Glu Ser Asn Lys Glu Ala Ser Thr Ile
65                  70                  75                  80
Ser Thr Asp Asn Pro Asp Ile Asn Pro Asn Ser Gln His Pro Thr Gln
                85                  90                  95
Gln Ser Thr Glu Asn Pro Thr Leu Asn Pro Ala Ala Ser Ala Ser Pro
            100                 105                 110
Ser Glu Thr Glu Ser Ala Ser Thr Pro Asp Thr Thr Asn Arg Leu Ser
        115                 120                 125
Ser Val Asp Arg Ser Thr Val Gln Pro Ser Glu Asn Arg Thr Lys Thr
    130                 135                 140
Lys Leu Thr Val His Thr Arg Asn Asn Leu Ser Thr Ala Ser Ser Thr
145                 150                 155                 160
Gln Ser Pro Pro Arg Ala Thr Thr Lys Ala Ile Arg Arg Ala Thr Thr
                165                 170                 175
Leu Arg Met Ser Ser Thr Gly Arg Arg Pro Thr Thr Thr Leu Val Gln
            180                 185                 190

Ser Asp Ser Ser Thr Thr Thr Gln Asn His Glu Glu Thr Gly Ser Ala
        195                 200                 205
Asn Pro Gln Ala Ser Ala Ser Thr Met Gln Asn Gln His Thr Asn Asn
    210                 215                 220
Ile Lys Pro Asn
225

<210> 153
<211> 228
<212> PRT
<213> Human metapneumo virus

<400> 153
Met Glu Val Lys Val Glu Asn Ile Arg Ala Ile Asp Met Leu Lys Ala
 1               5                  10                  15
Arg Val Lys Asn Arg Val Ala Arg Ser Lys Cys Phe Lys Asn Ala Ser
                20                  25                  30
Leu Ile Leu Ile Gly Ile Thr Thr Leu Ser Ile Ala Leu Asn Ile Tyr
        35                  40                  45
Leu Ile Ile Asn Tyr Thr Ile Gln Lys Thr Thr Ser Glu Ser Glu His
    50                  55                  60
His Thr Ser Ser Pro Pro Thr Glu Ser Asn Lys Glu Ala Ser Thr Ile
65                  70                  75                  80
Ser Thr Asp Asn Pro Asp Ile Asn Pro Asn Ser Gln His Pro Thr Gln
                85                  90                  95
Gln Ser Thr Glu Asn Pro Thr Leu Asn Pro Ala Ala Ser Ala Ser Pro
            100                 105                 110
Ser Glu Thr Glu Ser Ala Ser Thr Pro Asp Thr Thr Asn Arg Leu Ser
        115                 120                 125
Ser Val Asp Arg Ser Thr Val Gln Pro Ser Glu Asn Arg Thr Lys Thr
    130                 135                 140
Lys Leu Thr Val His Thr Arg Asn Asn Leu Ser Thr Ala Ser Ser Thr
145                 150                 155                 160
Gln Ser Pro Pro Arg Ala Thr Thr Lys Ala Ile Arg Arg Ala Thr Thr
                165                 170                 175
Leu Arg Met Ser Ser Thr Gly Arg Arg Pro Thr Thr Thr Leu Val Gln
            180                 185                 190
Ser Asp Ser Ser Thr Thr Thr Gln Asn His Glu Glu Thr Gly Ser Ala
        195                 200                 205
Asn Pro Gln Ala Ser Ala Ser Thr Met Gln Asn Gln His Thr Asn Asn
    210                 215                 220
Ile Lys Pro Asn
225

<210> 154
<211> 231
<212> PRT
<213> Human metapneumovirus

<220>
<221> VARIANT
<222> 225
<223> Xaa = unknown amino acid or other

<400> 154
Met Glu Val Arg Val Glu Asn Ile Arg Ala Ile Asp Met Phe Lys Ala
 1               5                  10                  15
Lys Ile Lys Asn Arg Ile Arg Ser Ser Arg Cys Tyr Arg Asn Ala Thr
                20                  25                  30
Leu Ile Leu Ile Gly Leu Thr Ala Leu Ser Met Ala Leu Asn Ile Phe

35                  40                  45
Leu Ile Ile Asp His Ala Thr Leu Arg Asn Met Ile Lys Thr Glu Asn
    50                  55                  60
Cys Ala Asn Met Pro Ser Ala Glu Pro Ser Lys Lys Thr Pro Met Thr
65                  70                  75                  80
Ser Thr Ala Gly Pro Asn Thr Lys Pro Asn Pro Gln Gln Ala Thr Gln
                85                  90                  95
Trp Thr Thr Glu Asn Ser Thr Ser Pro Val Ala Thr Pro Glu Gly His
            100                 105                 110
Pro Tyr Thr Gly Thr Thr Gln Thr Ser Asp Thr Thr Ala Pro Gln Gln
        115                 120                 125
Thr Thr Asp Lys His Thr Ala Pro Leu Lys Ser Thr Asn Glu Gln Ile
    130                 135                 140
Thr Gln Thr Thr Thr Glu Lys Lys Thr Ile Arg Ala Thr Thr Gln Lys
145                 150                 155                 160
Arg Glu Lys Gly Lys Glu Asn Thr Asn Gln Thr Thr Ser Thr Ala Ala
                165                 170                 175
Thr Gln Thr Thr Asn Thr Thr Asn Gln Ile Arg Asn Ala Ser Glu Thr
            180                 185                 190
Ile Thr Thr Ser Asp Arg Pro Arg Thr Asp Thr Thr Thr Gln Ser Ser
        195                 200                 205
Glu Gln Thr Thr Arg Ala Thr Asp Pro Ser Ser Pro Pro His His Ala
    210                 215                 220
Xaa Arg Gly Ala Lys Leu Lys
225                 230

<210> 155
<211> 231
<212> PRT
<213> Human metapneumo virus

<400> 155
Met Glu Val Arg Val Glu Asn Ile Arg Ala Ile Asp Met Phe Lys Ala
1               5                   10                  15
Lys Ile Lys Asn Arg Ile Arg Ser Ser Arg Cys Tyr Arg Asn Ala Thr
            20                  25                  30
Leu Ile Leu Ile Gly Leu Thr Ala Leu Ser Met Ala Leu Asn Ile Phe
        35                  40                  45
Leu Ile Ile Asp His Ala Thr Leu Arg Asn Met Ile Lys Thr Glu Asn
    50                  55                  60
Cys Ala Asn Met Pro Ser Ala Glu Pro Ser Lys Lys Thr Pro Met Thr
65                  70                  75                  80
Ser Thr Ala Gly Pro Ser Thr Glu Pro Asn Pro Gln Gln Ala Thr Gln
                85                  90                  95
Trp Thr Thr Glu Asn Ser Thr Ser Pro Ala Ala Thr Leu Glu Ser His
            100                 105                 110
Pro Tyr Thr Gly Thr Thr Gln Thr Pro Asp Ile Thr Ala Pro Gln Gln
        115                 120                 125
Thr Thr Asp Lys His Thr Ala Leu Pro Lys Ser Thr Asn Glu Gln Ile
    130                 135                 140
Thr Gln Thr Thr Thr Glu Lys Lys Thr Thr Arg Ala Thr Thr Gln Lys
145                 150                 155                 160
Arg Glu Lys Glu Lys Glu Asn Thr Asn Gln Thr Thr Ser Thr Ala Ala
                165                 170                 175
Thr Gln Thr Thr Asn Thr Thr Asn Gln Thr Arg Asn Ala Ser Glu Thr
            180                 185                 190
Ile Thr Thr Ser Asp Arg Pro Arg Ile Asp Thr Thr Thr Gln Ser Ser
        195                 200                 205
Asp Gln Thr Thr Arg Ala Thr Asp Pro Ser Ser Pro Pro His His Ala
    210                 215                 220
Gln Ser Gly Ala Lys Pro Lys

225                 230

<210> 156
<211> 231
<212> PRT
<213> Human metapneumo virus

<400> 156
Met Glu Val Arg Val Glu Asn Ile Arg Ala Ile Asp Met Phe Lys Ala
1               5                   10                  15
Lys Ile Lys Asn Arg Ile Arg Ser Ser Arg Cys Tyr Arg Asn Ala Thr
            20                  25                  30
Leu Ile Leu Ile Gly Leu Thr Ala Leu Ser Met Ala Leu Asn Ile Phe
        35                  40                  45
Leu Ile Ile Asp His Ala Thr Leu Arg Asn Met Ile Lys Thr Glu Asn
    50                  55                  60
Cys Ala Asn Met Pro Pro Ala Glu Pro Ser Lys Lys Thr Pro Met Thr
65                  70                  75                  80
Ser Thr Ala Gly Pro Asn Thr Lys Pro Asn Pro Gln Gln Ala Thr Gln
                85                  90                  95
Trp Thr Thr Glu Asn Ser Thr Phe Pro Ala Ala Thr Ser Glu Gly His
            100                 105                 110
Leu His Thr Gly Thr Thr Gln Thr Pro Asp Thr Thr Ala Pro Gln Gln
        115                 120                 125
Thr Thr Asp Lys His Thr Ala Leu Pro Lys Ser Thr Asn Glu Gln Ile
    130                 135                 140
Thr Gln Thr Thr Thr Glu Lys Lys Thr Thr Arg Ala Thr Thr Gln Arg
145                 150                 155                 160
Arg Glu Lys Gly Lys Glu Asn Thr Asn Gln Thr Thr Ser Thr Ala Ala
                165                 170                 175
Thr Gln Thr Thr Asn Thr Thr Asn Gln Ile Arg Asn Ala Ser Glu Thr
            180                 185                 190
Ile Thr Thr Ser Asp Arg Pro Arg Thr Asp Ser Thr Thr Gln Ser Ser
        195                 200                 205
Glu Gln Thr Thr Arg Ala Thr Asp Pro Ser Ser Pro Pro His His Ala
    210                 215                 220
Gln Gly Ser Ala Lys Pro Lys
225                 230

<210> 157
<211> 231
<212> PRT
<213> Human metapneumo virus

<400> 157
Met Glu Val Arg Val Glu Asn Ile Arg Ala Ile Asp Met Phe Lys Ala
1               5                   10                  15
Lys Ile Lys Asn Arg Ile Arg Ser Ser Arg Cys Tyr Arg Asn Ala Thr
            20                  25                  30
Leu Ile Leu Ile Gly Leu Thr Ala Leu Ser Met Ala Leu Asn Ile Phe
        35                  40                  45
Leu Ile Ile Asp His Ala Thr Leu Arg Asn Met Ile Lys Thr Glu Asn
    50                  55                  60
Cys Ala Asn Met Pro Pro Ala Glu Pro Ser Arg Lys Thr Pro Met Thr
65                  70                  75                  80
Ser Thr Ala Gly Pro Asn Thr Lys Pro Asn Pro Gln Gln Ala Thr Gln
                85                  90                  95
Trp Thr Thr Glu Asn Ser Thr Ser Pro Ala Ala Thr Pro Glu Gly His
            100                 105                 110
Leu His Thr Gly Thr Thr Gln Thr Pro Asp Thr Thr Ala Pro Gln Gln

        115                 120                 125
Thr Thr Asp Lys His Thr Ala Leu Pro Lys Ser Thr Asn Glu Gln Ile
    130                 135                 140
Thr Gln Ala Thr Thr Glu Lys Lys Thr Thr Arg Glu Thr Thr Gln Arg
145                 150                 155                 160
Arg Glu Lys Gly Lys Glu Asn Thr Asn Gln Thr Thr Ser Thr Ala Ala
                165                 170                 175
Thr Gln Thr Thr Asn Thr Thr Asn Gln Ile Arg Asn Ala Ser Glu Thr
            180                 185                 190
Ile Thr Thr Ser Asp Arg Pro Arg Thr Asp Ser Thr Thr Gln Ser Ser
        195                 200                 205
Glu Gln Thr Thr Gln Ala Thr Asp Pro Ser Ser Pro Ala His His Ala
    210                 215                 220
Gln Gly Ser Ala Lys Pro Lys
225                 230


<210> 158
<211> 231
<212> PRT
<213> Human metapneumo virus

<400> 158
Met Glu Val Arg Val Glu Asn Ile Arg Ala Ile Asp Met Phe Lys Ala
 1               5                  10                  15
Lys Ile Lys Asn Arg Ile Arg Ser Ser Arg Cys Tyr Arg Asn Ala Thr
            20                  25                  30
Leu Ile Leu Ile Gly Leu Thr Ala Leu Ser Met Ala Leu Asn Ile Phe
        35                  40                  45
Leu Ile Ile Asp His Ala Thr Leu Arg Asn Met Ile Lys Thr Glu Asn
    50                  55                  60
Cys Ala Asn Met Pro Pro Ala Glu Pro Ser Lys Lys Thr Pro Met Thr
65                  70                  75                  80
Ser Thr Ala Gly Leu Asn Thr Lys Pro Asn Pro Gln Gln Ala Thr Gln
                85                  90                  95
Trp Thr Thr Glu Asn Ser Thr Ser Pro Ala Ala Thr Pro Glu Gly His
            100                 105                 110
Leu His Thr Gly Thr Thr Gln Thr Pro Asp Thr Thr Ala Pro Gln Gln
        115                 120                 125
Thr Thr Asp Lys His Thr Ala Leu Pro Lys Ser Thr Asn Glu Gln Ile
    130                 135                 140
Thr Gln Thr Thr Thr Glu Lys Lys Thr Thr Arg Ala Thr Thr Gln Arg
145                 150                 155                 160
Arg Glu Lys Gly Lys Glu Asn Thr Asn Gln Thr Thr Ser Thr Ala Ala
                165                 170                 175
Thr Gln Thr Thr Asn Thr Thr Asn Gln Ile Arg Asn Ala Ser Glu Thr
            180                 185                 190
Ile Thr Thr Ser Asp Arg Pro Arg Thr Asp Ser Thr Thr Gln Ser Ser
        195                 200                 205
Glu Gln Thr Thr Arg Ala Thr Asp Pro Ser Ser Pro Pro His His Ala
    210                 215                 220
Gln Gly Ser Ala Lys Pro Lys
225                 230


<210> 159
<211> 231
<212> PRT
<213> Human metapneumo virus

<400> 159
Met Glu Val Arg Val Glu Asn Ile Arg Ala Ile Asp Met Phe Lys Ala

1               5                   10                  15
Lys Ile Lys Asn Arg Ile Arg Ser Ser Arg Cys Tyr Arg Asn Ala Thr
                20                  25                  30
Leu Ile Leu Ile Gly Leu Thr Ala Leu Ser Met Ala Leu Asn Ile Phe
            35                  40                  45
Leu Ile Ile Asp His Ala Thr Leu Arg Asn Met Ile Lys Thr Glu Asn
    50                  55                  60
Cys Ala Asn Met Pro Pro Ala Glu Pro Ser Lys Lys Thr Pro Met Thr
65                  70                  75                  80
Ser Thr Ala Gly Pro Asn Thr Lys Pro Asn Pro Gln Gln Ala Thr Gln
                85                  90                  95
Trp Thr Thr Glu Asn Ser Thr Ser Pro Ala Ala Thr Pro Glu Gly His
                100                 105                 110
Leu His Thr Gly Thr Thr Gln Thr Pro Asp Thr Thr Ala Pro Gln Gln
            115                 120                 125
Thr Thr Asp Lys His Thr Ala Leu Pro Lys Ser Thr Asn Glu Gln Ile
    130                 135                 140
Thr Gln Thr Thr Thr Glu Lys Lys Thr Thr Arg Ala Thr Thr Gln Arg
145                 150                 155                 160
Arg Glu Lys Gly Lys Glu Asn Thr Asn Gln Thr Thr Ser Thr Ala Ala
                165                 170                 175
Thr Gln Thr Thr Asn Thr Thr Asn Gln Ile Arg Asn Ala Ile Glu Thr
            180                 185                 190
Ile Thr Thr Ser Asp Arg Pro Arg Thr Asp Ser Thr Thr Gln Ser Ser
        195                 200                 205
Glu Gln Thr Thr Arg Ala Thr Asp Pro Ser Ser His Pro His His Ala
    210                 215                 220
Gln Gly Ser Ala Lys Pro Lys
225                 230

<210> 160
<211> 236
<212> PRT
<213> Human metapneumo virus

<400> 160
Met Glu Val Arg Val Glu Asn Ile Arg Ala Ile Asp Met Phe Lys Ala
1               5                   10                  15
Lys Met Lys Asn Arg Ile Arg Ser Ser Lys Cys Tyr Arg Asn Ala Thr
                20                  25                  30
Leu Ile Leu Ile Gly Leu Thr Ala Leu Ser Met Ala Leu Asn Ile Phe
            35                  40                  45
Leu Ile Ile Asp Tyr Ala Met Leu Lys Asn Met Thr Lys Val Glu His
    50                  55                  60
Cys Val Asn Met Pro Pro Val Glu Pro Ser Lys Lys Thr Pro Met Thr
65                  70                  75                  80
Ser Ala Val Asp Leu Asn Thr Lys Pro Asn Pro Gln Gln Ala Thr Gln
                85                  90                  95
Leu Ala Ala Glu Asp Ser Thr Ser Leu Ala Ala Thr Ser Glu Asp His
                100                 105                 110
Leu His Thr Gly Thr Thr Pro Thr Pro Asp Ala Thr Val Ser Gln Gln
            115                 120                 125
Thr Thr Asp Glu Tyr Thr Thr Leu Leu Arg Ser Thr Asn Arg Gln Thr
    130                 135                 140
Thr Gln Thr Thr Thr Glu Lys Lys Pro Thr Gly Ala Thr Thr Lys Lys
145                 150                 155                 160
Glu Thr Thr Thr Arg Thr Thr Ser Thr Ala Ala Thr Gln Thr Leu Asn
                165                 170                 175
Thr Thr Asn Gln Thr Ser Tyr Val Arg Glu Ala Thr Thr Thr Ser Ala
            180                 185                 190
Arg Ser Arg Asn Ser Ala Thr Thr Gln Ser Ser Asp Gln Thr Thr Gln

195                 200                 205
Ala Ala Asp Pro Ser Ser Gln Pro His His Thr Gln Lys Ser Thr Thr
    210                 215                 220
Thr Thr Tyr Asn Thr Asp Thr Ser Ser Pro Ser Ser
225                 230                 235

<210> 161
<211> 236
<212> PRT
<213> Human metapneumovirus

<220>
<221> VARIANT
<222> 220, 227
<223> Xaa = unknown amino acid or other

<400> 161
Met Glu Val Arg Val Glu Asn Ile Arg Thr Ile Asp Met Phe Lys Ala
 1               5                  10                  15
Lys Met Lys Asn Arg Ile Arg Ser Ser Lys Cys Tyr Arg Asn Ala Thr
            20                  25                  30
Leu Ile Leu Ile Gly Leu Thr Ala Leu Ser Met Ala Leu Asn Ile Phe
        35                  40                  45
Leu Ile Ile Asp Tyr Ala Thr Phe Lys Asn Met Thr Lys Val Glu His
    50                  55                  60
Cys Ala Asn Met Pro Pro Val Glu Pro Ser Lys Lys Thr Pro Met Thr
65                  70                  75                  80
Ser Thr Val Asp Ser Ser Thr Gly Pro Asn Pro Gln Gln Thr Thr Gln
                85                  90                  95
Trp Thr Thr Glu Asp Ser Thr Ser Leu Ala Ala Thr Ser Glu Asp His
            100                 105                 110
Leu His Thr Gly Thr Thr Pro Thr Leu Asp Ala Thr Val Ser Gln Gln
        115                 120                 125
Thr Pro Asp Lys His Thr Thr Pro Leu Arg Ser Thr Asn Gly Gln Thr
    130                 135                 140
Thr Gln Thr Thr Thr Glu Lys Lys Pro Thr Arg Ala Ile Ala Lys Lys
145                 150                 155                 160
Glu Thr Thr Asn Gln Thr Thr Ser Thr Ala Ala Thr Gln Thr Phe Asn
                165                 170                 175
Thr Thr Asn Gln Thr Arg Asn Gly Arg Glu Thr Thr Ile Thr Ser Ala
            180                 185                 190
Arg Ser Arg Asn Asp Ala Thr Thr Gln Ser Ser Glu Gln Thr Asn Gln
        195                 200                 205
Thr Thr Asp Pro Ser Ser Gln Pro His His Ala Xaa Ile Ser Thr Ile
    210                 215                 220
Thr Ile Xaa Thr Gln His Arg His Ile Phe Ser Lys
225                 230                 235

<210> 162
<211> 236
<212> PRT
<213> Human metapneumovirus

<220>
<221> VARIANT
<222> 208
<223> Xaa = unknown amino acid or other

<400> 162
Met Glu Val Arg Val Glu Asn Ile Arg Ala Ile Asp Met Phe Lys Ala

 1               5                   10                  15
Lys Met Lys Asn Arg Ile Arg Ser Ser Lys Cys Tyr Arg Asn Ala Thr
            20                  25                  30
Leu Ile Leu Ile Gly Leu Thr Ala Leu Ser Met Ala Leu Asn Ile Phe
        35                  40                  45
Leu Ile Ile Asp Tyr Ala Met Leu Lys Asn Met Thr Lys Val Glu His
    50                  55                  60
Cys Val Asn Met Pro Pro Val Glu Pro Ser Lys Lys Thr Pro Met Thr
65                  70                  75                  80
Ser Ala Val Asp Leu Asn Thr Lys Leu Asn Pro Gln Gln Ala Thr Gln
                85                  90                  95
Leu Thr Thr Glu Asp Ser Thr Ser Leu Ala Ala Thr Ser Glu Asp His
            100                 105                 110
Leu Leu Thr Gly Thr Thr Pro Thr Pro Asp Ala Thr Val Ser Gln Gln
        115                 120                 125
Thr Thr Asp Glu His Thr Thr Leu Leu Arg Ser Thr Asn Arg Gln Thr
    130                 135                 140
Thr Gln Thr Thr Thr Glu Lys Lys Pro Thr Gly Ala Thr Thr Lys Lys
145                 150                 155                 160
Glu Thr Thr Thr Arg Thr Thr Ser Thr Ala Ala Thr Gln Thr Leu Asn
                165                 170                 175
Thr Thr Asn Gln Thr Ser Asn Gly Arg Glu Ala Thr Thr Thr Ser Thr
            180                 185                 190
Arg Ser Arg Asn Gly Ala Thr Thr Gln Asn Ser Asp Gln Thr Thr Xaa
        195                 200                 205
Thr Ala Asp Pro Ser Ser Gln Pro His His Thr Gln Lys Ser Thr Thr
    210                 215                 220
Thr Thr Tyr Asn Thr Asp Thr Ser Ser Pro Ser Ser
225                 230                 235

<210> 163
<211> 236
<212> PRT
<213> Human metapneumo virus

<400> 163
Met Glu Val Arg Val Glu Asn Ile Arg Ala Ile Asp Met Phe Lys Ala
 1               5                   10                  15
Lys Met Lys Asn Arg Ile Arg Ser Ser Lys Cys Tyr Arg Asn Ala Thr
            20                  25                  30
Leu Ile Leu Ile Gly Leu Thr Ala Leu Ser Met Ala Leu Asn Ile Phe
        35                  40                  45
Leu Ile Ile Asp Tyr Ala Thr Leu Lys Asn Met Thr Lys Val Glu His
    50                  55                  60
Cys Val Asn Met Pro Pro Val Glu Pro Ser Lys Lys Thr Pro Met Thr
65                  70                  75                  80
Ser Ala Val Asp Leu Asn Thr Lys Leu Asn Pro Gln Gln Ala Thr Gln
                85                  90                  95
Leu Thr Thr Glu Asp Ser Thr Ser Leu Ala Ala Thr Ser Glu Gly His
            100                 105                 110
Pro His Thr Gly Thr Thr Pro Thr Pro Asp Ala Thr Val Ser Gln Gln
        115                 120                 125
Thr Thr Asp Glu His Thr Thr Leu Leu Arg Ser Thr Asn Arg Gln Thr
    130                 135                 140
Thr Gln Thr Ala Thr Glu Lys Lys Pro Thr Gly Ala Thr Thr Lys Lys
145                 150                 155                 160
Glu Thr Thr Thr Arg Thr Thr Ser Thr Ala Ala Thr Gln Thr Pro Asn
                165                 170                 175
Thr Thr Asn Gln Thr Ser Asn Gly Arg Glu Ala Thr Thr Thr Ser Ala
            180                 185                 190
Arg Ser Arg Asn Gly Ala Thr Thr Gln Asn Ser Asp Gln Ile Thr Gln

195                 200                 205
Ala Ala Asp Ser Ser Ser Gln Pro His His Thr Gln Lys Ser Thr Thr
    210                 215                 220
Thr Ala Tyr Asn Thr Asp Thr Ser Phe Pro Ser Ser
225                 230                 235

<210> 164
<211> 236
<212> PRT
<213> Human metapneumo virus

<400> 164
Met Glu Val Arg Val Glu Asn Ile Arg Ala Ile Asp Met Phe Lys Ala
 1               5                  10                  15
Lys Met Lys Asn Arg Ile Arg Ser Ser Lys Cys Tyr Arg Asn Ala Thr
                20                  25                  30
Leu Ile Leu Ile Gly Leu Thr Ala Leu Ser Met Ala Leu Asn Ile Phe
        35                  40                  45
Leu Ile Ile Asp Tyr Ala Thr Leu Lys Asn Met Thr Lys Val Glu His
    50                  55                  60
Cys Val Asn Met Pro Pro Val Glu Pro Ser Lys Lys Thr Pro Met Thr
65                  70                  75                  80
Ser Ala Val Asp Ser Asn Thr Lys Pro Asn Pro Gln Gln Ala Thr Gln
                85                  90                  95
Leu Thr Thr Glu Asp Ser Thr Ser Leu Ala Ala Thr Leu Glu Asp His
            100                 105                 110
Pro His Thr Gly Thr Thr Pro Thr Pro Asp Ala Thr Val Ser Gln Gln
        115                 120                 125
Thr Thr Asp Glu His Thr Thr Leu Leu Arg Ser Thr Asn Arg Gln Thr
    130                 135                 140
Thr Gln Thr Thr Ala Glu Lys Lys Pro Thr Arg Ala Thr Thr Lys Lys
145                 150                 155                 160
Glu Thr Thr Thr Arg Thr Thr Ser Thr Ala Ala Thr Gln Thr Leu Asn
                165                 170                 175
Thr Thr Asn Gln Thr Ser Asn Gly Arg Glu Ala Thr Thr Thr Ser Ala
            180                 185                 190
Arg Ser Arg Asn Asn Ala Thr Thr Gln Ser Ser Asp Gln Thr Thr Gln
        195                 200                 205
Ala Ala Glu Pro Ser Ser Gln Ser Gln His Thr Gln Lys Ser Thr Thr
    210                 215                 220
Thr Thr Tyr Asn Thr Asp Thr Ser Ser Leu Ser Ser
225                 230                 235

<210> 165
<211> 236
<212> PRT
<213> Human metapneumo virus

<400> 165
Met Glu Val Arg Val Glu Asn Ile Arg Ala Ile Asp Met Phe Lys Ala
 1               5                  10                  15
Lys Met Lys Asn Arg Ile Arg Ser Ser Lys Cys Tyr Arg Asn Ala Thr
                20                  25                  30
Leu Ile Leu Ile Gly Leu Ser Ala Leu Ser Met Ala Leu Asn Ile Phe
        35                  40                  45
Leu Ile Ile Asp Tyr Ala Lys Ser Lys Asn Met Thr Arg Val Glu His
    50                  55                  60
Cys Val Asn Met Pro Pro Val Glu Pro Ser Lys Lys Thr Pro Met Thr
65                  70                  75                  80
Ser Ala Val Asp Leu Asn Thr Lys Pro Asn Pro Gln Arg Ala Thr Gln

                    85                      90                      95
Leu Thr Thr Glu Asp Ser Thr Ser Leu Ala Ala Thr Leu Glu Gly His
            100                     105                     110
Leu His Thr Gly Thr Thr Pro Thr Pro Asp Val Thr Val Ser Gln Gln
        115                     120                     125
Thr Thr Asp Glu His Thr Thr Leu Leu Arg Ser Thr Asn Arg Gln Thr
    130                     135                     140
Thr Gln Thr Ala Ala Glu Lys Lys Pro Thr Arg Val Thr Thr Asn Lys
145                     150                     155                     160
Glu Thr Ile Thr Arg Thr Thr Ser Thr Ala Ala Thr Gln Thr Leu Asn
                165                     170                     175
Thr Thr Asn Gln Thr Asn Asn Gly Arg Glu Ala Thr Thr Thr Ser Ala
            180                     185                     190
Arg Ser Arg Asn Asn Ala Thr Thr Gln Ser Ser Asp Gln Thr Thr Gln
        195                     200                     205
Ala Ala Asp Pro Ser Ser Gln Ser Gln His Thr Gln Lys Ser Ile Thr
    210                     215                     220
Thr Thr Tyr Asn Thr Asp Thr Ser Ser Pro Ser Ser
225                     230                     235


<210> 166
<211> 236
<212> PRT
<213> Human metapneumo virus

<400> 166
Met Glu Val Arg Val Glu Asn Ile Arg Ala Ile Asp Met Phe Lys Ala
 1               5                  10                  15
Lys Met Lys Asn Arg Ile Arg Ser Ser Lys Cys Tyr Arg Asn Ala Thr
            20                  25                  30
Leu Ile Leu Ile Gly Leu Ser Ala Leu Ser Met Ala Leu Asn Ile Phe
        35                  40                  45
Leu Ile Ile Asp Tyr Ala Lys Ser Lys Thr Met Thr Arg Val Glu His
    50                  55                  60
Cys Val Asn Met Pro Pro Val Glu Pro Ser Lys Lys Thr Pro Met Thr
65                  70                  75                  80
Ser Ala Val Asp Leu Asn Thr Lys Pro Asn Pro Gln Gln Ala Thr Gln
                85                  90                  95
Leu Thr Thr Glu Asp Ser Thr Ser Pro Ala Ala Thr Leu Glu Gly His
            100                 105                 110
Leu His Thr Gly Thr Thr Pro Thr Pro Asp Ala Thr Val Ser Gln Gln
        115                 120                 125
Thr Thr Asp Glu His Thr Thr Leu Leu Arg Ser Thr Asn Arg Gln Thr
    130                 135                 140
Thr Gln Thr Thr Ala Glu Lys Lys Pro Thr Arg Ala Thr Thr Lys Lys
145                 150                 155                 160
Glu Thr Ile Thr Arg Thr Thr Ser Thr Ala Ala Thr Gln Thr Leu Asn
                165                 170                 175
Thr Thr Asn Gln Thr Ser Asn Gly Arg Glu Ala Thr Thr Thr Ser Ala
            180                 185                 190
Arg Ser Arg Asn Asn Ala Thr Thr Gln Ser Ser Asp Gln Thr Thr Gln
        195                 200                 205
Ala Ala Asp Pro Ser Ser Gln Ser Gln His Thr Lys Lys Ser Thr Thr
    210                 215                 220
Thr Thr Tyr Asn Thr Asp Thr Ser Ser Pro Ser Ser
225                 230                 235


<210> 167
<211> 236
<212> PRT

<213> Human metapneumo virus

<400> 167
Met Glu Val Arg Val Glu Asn Ile Arg Ala Ile Asp Met Phe Lys Ala
1               5                   10                  15
Lys Met Lys Asn Arg Ile Arg Ser Ser Lys Cys Tyr Arg Asn Ala Thr
            20                  25                  30
Leu Ile Leu Ile Gly Leu Thr Ala Leu Ser Met Ala Leu Asn Ile Phe
        35                  40                  45
Leu Ile Ile Asp Tyr Ala Thr Leu Lys Asn Met Thr Lys Val Glu His
    50                  55                  60
Cys Val Asn Met Pro Pro Val Glu Pro Ser Lys Lys Thr Pro Met Thr
65                  70                  75                  80
Ser Ala Val Asp Leu Asn Thr Lys Pro Asn Pro Gln Gln Ala Thr Gln
                85                  90                  95
Leu Thr Thr Glu Asp Ser Thr Ser Leu Ala Ala Thr Leu Glu Asp His
            100                 105                 110
Pro His Thr Gly Thr Thr Pro Thr Pro Asp Ala Thr Val Ser Gln Gln
        115                 120                 125
Thr Thr Asp Glu His Thr Thr Leu Leu Arg Ser Thr Asn Arg Gln Thr
    130                 135                 140
Thr Gln Thr Thr Ala Glu Lys Lys Pro Thr Arg Ala Thr Thr Lys Lys
145                 150                 155                 160
Glu Thr Thr Thr Arg Thr Thr Ser Thr Ala Ala Thr Gln Thr Leu Asn
                165                 170                 175
Thr Thr Asn Gln Thr Ser Asn Gly Arg Glu Ala Thr Thr Thr Ser Ala
            180                 185                 190
Arg Ser Arg Asn Asn Ala Thr Thr Gln Ser Ser Asp Gln Thr Thr Gln
        195                 200                 205
Ala Ala Glu Pro Asn Ser Gln Ser Gln His Thr Gln Lys Ser Thr Thr
    210                 215                 220
Thr Thr Tyr Asn Thr Asp Thr Ser Ser Leu Ser Ser
225                 230                 235


<210> 168
<211> 449
<212> DNA
<213> Human metapneumo virus

<400> 168
ataggagttt acggaagctc cgtaatttac atggtgcaac tgccaatctt tggggttata 60
gacacgcctt gctggatagt aaaagcagcc ccttcttgtt caggaaaaaa gggaaactat 120
gcttgcctct taagagaaga ccaaggatgg tattgtcaaa atgcagggtc aactgtttac 180
tacccaaatg aaaaagactg tgaaacaaga ggagaccatg tcttttgcga cacagcagca 240
ggaatcaatg ttgctgagca gtcaaaggag tgcaacataa acatatctac tactaattac 300
ccatgcaaag ttagcacagg aagacatcct atcagtatgg ttgcactatc tcctcttggg 360
gctttggttg cttgctacaa gggagtgagc tgttccattg gcagcaacag agtagggatc 420
atcaagcaac tgaacaaagg ctgctctta                                   449

<210> 169
<211> 449
<212> DNA
<213> Human metapneumo virus

<400> 169
ataggagttt acggaagctc cgtaatttac atggtgcaac tgccaatctt tggggttata 60
gacacgcctt gctggatagt aaaagcagcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360

gctttggttg cttgctacaa gggagtgagc tgttccattg gcagcaacag agtagggatc 420
atcaagcaac tgaacaaagg ctgctctta 449

<210> 170
<211> 449
<212> DNA
<213> Human metapneumo virus

<400> 170
ataggagttt acggaagctc cgtaatttac atggtgcaac tgccaatctt tggggttata 60
gacacgcctt gctggatagt aaaagcagcc ccttcttgct cagaaaaaaa gggaaactat 120
gcttgcctct taagagaaga tcaaggatgg tattgtcaga atgcagggtc aactgtttac 180
tacccaaatg aaaaagattg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 171
<211> 449
<212> DNA
<213> Human metapneumo virus

<400> 171
ataggagttt acggaagctc cgtaatttac atggtgcaac tgccaatctt tggggttata 60
gacacgcctt gctggatagt aaaagcagcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 172
<211> 449
<212> DNA
<213> Human metapneumo virus

<400> 172
ataggagttt acggaagctc cgtaatttac atggtgcaac tgccaatctt tggggttata 60
gacacgcctt gctggatagt aaaagcagcc ccttcttgct cagaaaaaaa gggaaactat 120
gcttgcctct taagagaaga tcaaggatgg tattgtcaga atgcagggtc aactgtttac 180
tacccaaatg aaaaagattg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gtagcaacag agtagggatc 420
atcaagcaac tgaacaaagg ctgctctta 449

<210> 173
<211> 449
<212> DNA
<213> Human metapneumo virus

<400> 173
ataggagttt acggaagctc cgtaatttac atggtgcaac tgccaatctt tggggttata 60
gacacgcctt gctggatagt aaaagcagcc ccttcttgtt cagaaaaaaa gggaaactat 120
gcttgcctct taagagaaga ccaaggatgg tattgtcaga atgcagggtc aactgtttac 180
tacccaaatg aaaaagactg tgaaacaaga ggagaccatg tcttttgcga cacagcagca 240
ggaatcaatg ttgctgagca gtcaaaggag tgcaacataa acatatctac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 174
<211> 449
<212> DNA
<213> Human metapneumo virus

<400> 174
ataggagttt acggaagctc cgtaatttac atggtgcaac tgccaatctt tggggttata 60
gacacgcctt gctggatagt aaaagcagcc ccttcttgtt cagaaaaaaa gggaaactat 120
gcttgcctct taagagaaga ccaaggatgg tattgtcaga atgcagggtc aactgtttac 180
tacccaaatg aaaaagactg tgaaacaaga ggagaccatg tcttttgcga cacagcagca 240
ggaatcaatg ttgctgagca gtcaaaggag tgcaacataa acatatctac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 175
<211> 449
<212> DNA
<213> Human metapneumo virus

<400> 175
ataggagttt acggaagctc cgtaatttac atggtgcaac tgccaatctt tggggttata 60
gacacgcctt gctggatagt aaaagcagcc ccttcttgtt cagaaaaaaa gggaaactat 120
gcttgcctct taagagaaga ccaaggatgg tattgtcaga atgcagggtc aactgtttac 180
tacccaaatg aaaaagactg tgaaacaaga ggagaccatg tcttttgcga cacagcagca 240
ggaatcaatg ttgctgagca gtcaaaggag tgcaacataa acatatctac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 176
<211> 449
<212> DNA
<213> Human metapneumo virus

<400> 176
ataggagttt acggaagctc cgtaatttac atggtgcaac tgccaatctt tggggttata 60
gacacgcctt gctggatagt aaaagcagcc ccttcttgct cagaaaaaaa gggaaactat 120
gcttgcctct taagagaaga tcagggatgg tattgtcaga atgcagggtc aactgtttac 180
tacccaaatg aaaaagactg tgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtaggaatc 420
atcaagcaac tgaacaaagg ctgctctta 449

<210> 177
<211> 449
<212> DNA
<213> Human metapneumo virus

<400> 177
ataggagttt acggaagctc cgtaatttac atggtgcaac tgccaatctt tggagttata 60
gacacgcctt gctggatagt aaaagcagcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 178
<211> 449

<212> DNA
<213> Human metapneumo virus

<400> 178
ataggagttt acggaagctc cgtaatttac atggtgcaac tgccaatctt tggggttata 60
gacacgcctt gttggatagt aaaagcagcc ccttcttgct cagaaaaaaa ggggaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 179
<211> 449
<212> DNA
<213> Human metapneumo virus

<400> 179
ataggagttt acggaagctc cgtaatttac atggtgcaac tgccaatctt tggagttata 60
gacacgcctt gctggatagt aaaagcggcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 180
<211> 449
<212> DNA
<213> Human metapneumo virus

<400> 180
ataggagttt acggaagctc cgtaatttac atggtgcaac tgccaatctt tggggttata 60
gacacgcctt gctggatagt aaaagcagcc ccttcttgct cagaaaaaaa gggaaactat 120
gcttgccttt taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca atatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 181
<211> 449
<212> DNA
<213> Human metapneumo virus

<400> 181
ataggagttt acggaagctc cgtaatttac atggtgcaac tgccaatctt tggagttata 60
gacacgcctt gctggatagt aaaagcggcc ccttcttgct cggaaaaaaa gggaaactat 120
gcttgcctct taagagaaga tcaaagatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 182
<211> 449
<212> DNA
<213> Human metapneumo virus

<400> 182
ataggagttt atggaagctc cgtaatttac atggtgcaac tgccaatctt tggagttata 60
gacacgcctt gctggatagt aaaagcggcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttctattg gcagcaacag agtagggatc 420
atcaagcaac tgaacaaagg ctgctctta 449

<210> 183
<211> 449
<212> DNA
<213> Human metapneumo virus

<400> 183
ataggagttt atggaagctc cgtaatttac atggtgcaac tgccaatctt tggagttata 60
gacacgcctt gctggatagt aaaagcggcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 184
<211> 449
<212> DNA
<213> Human metapneumo virus

<400> 184
ataggagttt acggaagctc cgtaatttac atggtgcaac tgccaatctt tggagttata 60
gacacgcctt gctggatagt aaaagcggcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac cactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 185
<211> 449
<212> DNA
<213> Human metapneumo virus

<400> 185
ataggagttt atggaagctc cgtaatttac atggtgcaac tgccaatctt tggagttata 60
gacacgcctt gctggatagt aaaagcggcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 186
<211> 449
<212> DNA
<213> Human metapneumo virus

<400> 186
ataggagttt atggaagctc cgtaatttac atggtgcaac tgccaatctt tggagttata 60

gacacgcctt gctggatagt aaaagcggcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 187
<211> 449
<212> DNA
<213> Human metapneumo virus

<400> 187
ataggagttt atggaagctc cgtaatttac atggtgcaac tgccaatctt tggagttata 60
gacacgcctt gctggatagt aaaagcggcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 188
<211> 449
<212> DNA
<213> Human metapneumo virus

<400> 188
ataggagttt acggaagctc cgtaatttac atggtgcaac tgccaatctt tggggttata 60
gacacgcctt gctggatagt aaaagcagcc ccttcttgtt caggaaaaaa gggaaactat 120
gcttgcctct taagagaaga ccaaggatgg tattgtcaaa atgcagggtc aactgtttac 180
tacccaaatg aaaaagactg tgaaacaaga ggagaccatg tcttttgcga cacagcagca 240
ggaatcaatg ttgctgagca gtcaaaggag tgcaacataa acatatctac tactaattac 300
ccatgcaaag ttagcacagg aagacatcct atcagtatgg ttgcactatc tcctcttggg 360
gctttggttg cttgctacaa gggagtgagc tgttccattg gcagcaacag agtagggatc 420
atcaagcaac tgaacaaagg ctgctctta 449

<210> 189
<211> 449
<212> DNA
<213> Human metapneumo virus

<400> 189
ataggagttt acggaagctc cgtaatttac atggtgcaac tgccaatctt tggggttata 60
gacacgcctt gctggatagt aaaagcagcc ccttcttgtt caggaaaaaa gggaaactat 120
gcttgcctct taagagaaga ccaaggatgg tattgtcaaa atgcagggtc aactgtttac 180
tacccaaatg aaaaagactg tgaaacaaga ggagaccatg tcttttgcga cacagcagca 240
ggaatcaatg ttgctgagca gtcaaaggag tgcaacataa acatatctac tactaattac 300
ccatgcaaag ttagcacagg aagacatcct atcagtatgg ttgcactatc tcctcttggg 360
gctttggttg cttgctacaa gggagtgagc tgttccattg gcagcaacag agtagggatc 420
atcaagcaac tgaacaaagg ctgctctta 449

<210> 190
<211> 449
<212> DNA
<213> Human metapneumo virus

<400> 190
ataggagttt acggaagctc cgtaatttac atggtgcaac tgccaatctt tggagttata 60
gacacgcctt gctggatagt aaaagcggcc ccttcttgct cagaaaaaaa gggaaactat 120
gcttgcctct taagagaaga tcaaggatgg tattgtcaga atgcagggtc aactgtttac 180

tacccaaatg aaaaagactg cgaaacaaga ggagaccatg tcttttgcga cacagcagca 240
ggaatcaatg ttgctgagca gtcaaaggag tgcaacatca acatatccac cactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 191
<211> 449
<212> DNA
<213> Human metapneumo virus

<400> 191
ataggagttt acggaagctc cgtaatttac atggtgcaac tgccaatctt tggggttata 60
gacacgcctt gctggatagt aaaagcagcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctctggttg cttgctacaa gggagtgagc tgctccattg gcagcaacag agtagggatc 420
atcaagcaac tgaacaaagg ctgctctta 449

<210> 192
<211> 449
<212> DNA
<213> Human metapneumo virus

<400> 192
ataggagttt acggaagctc cgtaatttac atggtgcaac tgccaatctt tggagttata 60
gacacgcctt gctggatagt aaaagcggcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac cactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 193
<211> 449
<212> DNA
<213> Human metapneumo virus

<400> 193
ataggagttt acggaagctc cgtaatttac atggtgcaac tgccaatctt tggagttata 60
gacacgcctt gctggatagt aaaagcggcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac cactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 194
<211> 449
<212> DNA
<213> Human metapneumo virus

<400> 194
ataggagttt acggaagctc cgtaatttac atggtgcaac tgccaatctt tggggttata 60
gacacgcctt gctggatagt aaaagcagcc ccttcttgtt caggaaaaaa gggaaactat 120
gcttgcctct taagagaaga ccaaggatgg tattgtcaaa atgcagggtc aactgtttac 180
tacccaaatg aaaaagactg tgaaacaaga ggagaccatg tcttttgcga cacagcagca 240
ggaatcaatg ttgctgagca gtcaaaggag tgcaacataa acatatctac tactaattac 300

ccatgcaaag ttagcacagg aagacatcct atcagtatgg ttgcactatc tcctcttggg 360
gctttggttg cttgctacaa gggagtgagc tgttccattg gcagcaacag agtagggatc 420
atcaagcaac tgaacaaagg ctgctctta 449

<210> 195
<211> 449
<212> DNA
<213> Human metapneumo virus

<400> 195
ataggagttt atggaagctc cgtaatttac atggtgcaac tgccaatctt tggagttata 60
gacacgcctt gctggatagt aaaagcggcc ccttcttgct cagaaaaaaa gggaaactat 120
gcttgcctct taagagaaga tcaaggatgg tattgtcaga atgcagggtc aactgtttac 180
tacccaaatg aaaaagactg cgaaacaaga ggagaccatg tcttttgcga cacagcagca 240
ggaatcaatg ttgctgagca gtcaaaggag tgcaacatca acatatccac tactaattac 300
ccatgcaaag ttagcacagg aagacatcct atcagtatgg ttgcactgtc tcctcttggg 360
gctttggttg cttgctacaa gggagtgagc tgttccattg gcagcaacag agtagggatc 420
atcaagcaac tgaacaaagg ctgctctta 449

<210> 196
<211> 449
<212> DNA
<213> Human metapneumo virus

<400> 196
ataggagttt acggaagctc cgtaatttac atggtgcaac tgccaatctt tggggttata 60
gacacgcctt gctggatagt aaaagcagcc ccttcttgtt caggaaaaaa gggaaactat 120
gcttgcctct taagagaaga ccaaggatgg tattgtcaaa atgcagggtc aactgtttac 180
tacccaaatg aaaaagactg tgaaacaaga ggagaccatg tcttttgcga cacagcagca 240
ggaatcaatg ttgctgagca gtcaaaggag tgcaacataa acatatctac tactaattac 300
ccatgcaaag ttagcacagg aagacatcct atcagtatgg ttgcactatc tcctcttggg 360
gctttggttg cttgctacaa gggagtgagc tgttccattg gcagcaacag agtagggatc 420
atcaagcaac tgaacaaagg ctgctctta 449

<210> 197
<211> 449
<212> DNA
<213> Human metapneumo virus

<400> 197
ataggggtct acgggagctc tgtaatttac atggtgcagc tgccaatctt tggcgttata 60
gacacgcctt gctggatagt aaaagcagcc ccctcttgtt ccgaaaaaaa gggaaactat 120
gcttgcctct taagagaaga ccaagggtgg tattgtcaga atgcagggtc aactgtttac 180
tacccaaatg agaaagactg tgaaacaaga ggagaccatg tcttttgcga cacagcagca 240
ggaattaatg ttgctgagca atcaaaggag tgcaacatca acatatccac tacaaattac 300
ccatgcaaag tcagcacagg aagacatcct atcagtatgg ttgcactgtc ccctcttggg 360
gctctggttg cttgctacaa aggagtaagc tgttccattg gcagcaatag agtagggatt 420
atcaagcagc tgaacaaagg ttgctctta 449

<210> 198
<211> 449
<212> DNA
<213> Human metapneumo virus

<400> 198
ataggggtct acgggagctc cgtaatttac atggtgcagc tgccaatctt tggcgtcata 60
gacacgcctt gctggatagt aaaagcagcc ccctcttgtt ccgaaaaaaa gggaaactat 120
gcttgccttt taagagaaga tcaagggtgg tattgtcaga atgcagggtc aactgtttac 180
tacccaaatg agaaagactg tgaaacaaga ggagaccatg tcttttgcga cacagcagca 240
ggaattaatg ttgctgagca atcaaaagag tgcaacatca acatatccac tacaaattac 300
ccatgcaaag tcagcacagg aagacatcct atcagtatgg ttgcactgtc ccctcttggg 360
gctctagttg cttgctacaa aggagtaagc tgttccattg gcagcaatag agtagggatc 420

atcaagcagc tgaacaaagg ttgctccta 449

<210> 199
<211> 449
<212> DNA
<213> Human metapneumo virus

<400> 199
ataggggtct acgggagctc cgtaatttac atggtgcagc tgccaatctt tggcgtcata 60
gacacgcctt gctggatagt aaaagcagcc ccctcttgtt ccgaaaaaaa gggaaactat 120
gcttgccttt taagagaaga tcaagggtgg tattgtcaga atgcagggtc aactgtttac 180
tacccaaatg agaaagactg tgaaacaaga ggagaccatg tcttttgcga cacagtagca 240
ggaattaatg ttgctgagca atcaaaagag tgcaacatca acatatccac tacaaattac 300
ccatgcaaag tcagcacagg aagacatcct atcagtatgg ttgcactgtc ccctcttggg 360
gctctagttg cttgctacaa aggagtaagc tgttccattg gcagcaatag agtagggatc 420
atcaagcagc tgaacaaagg ttgctccta 449

<210> 200
<211> 449
<212> DNA
<213> Human metapneumo virus

<400> 200
ataggggtct acgggagctc cgtaatttac atggtgcagc tgccaatctt tggcgtcata 60
gacacgcctt gctggatagt aaaagcagcc ccctcttgtt ccgaaaaaaa gggaaactat 120
gcttgccttt taagagaaga tcaagggtgg tattgtcaga atgcagggtc aactgtttac 180
tacccaaatg agaaagactg tgaaacaaga ggagaccatg tcttttgcga cacagcagca 240
ggaattaatg ttgctgagca atcaaaagag tgcaacatca acatatccac tacaaattac 300
ccatgcaaag tcagcacagg aagacatcct atcagtatgg ttgcactgtc ccctcttggg 360
gctctagttg cttgctacaa aggagtaagc tgttccattg gcagcaatag agtagggatc 420
atcaagcagc tgaacaaagg ttgctccta 449

<210> 201
<211> 449
<212> DNA
<213> Human metapneumo virus

<400> 201
ataggggtct acgggagctc cgtaatttac atggtgcagc tgccaatctt tggcgttata 60
gacacgccct gctggatagt aaaagcagcc ccctcttgtt ccgaaaaaaa gggaaactat 120
gcttgccttc taagagaaga ccaagggtgg tattgtcaga atgcagggtc aactgtttac 180
tacccaaatg agaaggactg tgaaacaaga ggagaccatg tcttttgcga cacagcagca 240
ggaattaatg ttgctgagca atcaaaggag tgcaacatca acatatccac cacaaattac 300
ccatgcaaag tcagcacagg aaggcatcct atcagtatgg ttgcactgtc ccctcttggg 360
gctctggttg cttgttacaa aggagtaagc tgttctattg gcagcaatag agtagggatc 420
atcaagcagc tgaacaaagg ttgctctta 449

<210> 202
<211> 449
<212> DNA
<213> Human metapneumo virus

<400> 202
ataggggtct acgggagctc cgtaatttac atggtgcagc tgccaatctt tggcgttata 60
gacacgcctt gctggatagt aaaagcagcc ccctcttgtt ccgaaaaaaa gggaaactat 120
gcttgcctct taagagaaga ccaagggtgg tattgtcaga atgcagggtc aactgtttac 180
tacccaaatg agaaagactg tgaaacaaga ggagaccatg tcttttgcga cacagcagca 240
ggaattaatg ttgctgagca atcaaaggag tgcaacatca acatatccac tacaaattac 300
ccatgcaaag tcagcacagg aagacatcct atcagtatgg ttgcactgtc tcctcttggg 360
gctctagttg cttgctacaa aggagtaagc tgttccattg gcagcaacag agtagggatc 420
atcaagcagc tgaacaaagg ttgctccta 449

<210> 203
<211> 449
<212> DNA
<213> Human metapneumo virus

<400> 203
ataggggtct acgggagctc cgtaatttac atggtgcagc tgccaatctt tggcgttata 60
gacacgccct gctggatagt aaaagcagcc ccctcttgtt ccgaaaaaaa gggaaactat 120
gcttgccttc taagagaaga ccaagggtgg tattgtcaga atgcagggtc aactgtttac 180
tacccaaatg agaaggactg tgaaacaaga ggagaccatg tcttttgcga cacagcagca 240
ggaattaatg ttgctgagca atcaaaggag tgcaacatca acatatccac cacaaattac 300
ccatgcaaag tcagcacagg aaggcatcct atcagtatgg ttgcactgtc ccctcttggg 360
gctctggttg cttgttacaa aggagtaagc tgttctattg gcagcaatag agtagggatc 420
atcaagcagc tgaacaaagg ttgctctta 449

<210> 204
<211> 449
<212> DNA
<213> Human metapneumo virus

<400> 204
ataggggtct acgggagctc cgtaatttac atggtgcagc tgccaatctt tggcgttata 60
gacacgcctt gctggatagt aaaagcagcc ccttcttgtt ccgaaaaaaa gggaaactat 120
gcttgcctct taagagaaga ccaagggtgg tattgtcaga atgcagggtc aactgtttac 180
tacccaaatg agaaagactg tgaaacaaga ggagaccatg tcttttgcga cacagcagca 240
ggaattaatg ttgctgagca atcaaaggag tgcaacatca acatatccac tacaaattac 300
ccatgcaaag tcagcacagg aagacatcct atcagtatgg ttgcactgtc tcctcttggg 360
gctctggttg cttgctacaa aggagtaagc tgttccattg gcagcaacag agtagggatc 420
atcaagcagc tgaacaaagg ttgctccta 449

<210> 205
<211> 449
<212> DNA
<213> Human metapneumo virus

<400> 205
ataggggtct acgggagctc cgtaatttac atggtgcagc tgccaatctt tggcgttata 60
gacacgcctt gctggatagt aaaagcagcc ccttcttgtt ccgaaaaaaa gggaaactat 120
gcttgcctct taagagaaga ccaagggtgg tattgtcaga atgcagggtc aactgtttac 180
tacccaaatg agaaagactg tgaaacaaga ggagaccatg tcttttgcga cacagcagca 240
ggaattaatg ttgctgagca atcaaaggag tgcaacatca acatatccac tacaaattac 300
ccatgcaaag tcagcacagg aagacatcct atcagtatgg ttgcactgtc tcctcttggg 360
gctctggttg cttgctacaa aggagtaagc tgttccattg gcagcaacag agtagggatc 420
atcaagcagc tgaacaaagg ttgctccta 449

<210> 206
<211> 449
<212> DNA
<213> Human metapneumo virus

<400> 206
ataggggtct acgggagctc cgtaatttac atggtgcagc tgccaatctt tggcgttata 60
gacacgcctt gctggatagt aaaagcagcc ccttcttgtt ccgaaaaaaa gggaaactat 120
gcttgcctct taagagaaga ccaaggatgg tattgtcaga atgcagggtc aactgtttac 180
tacccaaatg agaaagactg tgaaacaaga ggagaccatg tcttttgcga cacagcagca 240
ggaattaatg ttgctgagca atcaaaggag tgcaacatca acatatccac cacaaattac 300
ccatgcaaag tcagcacagg aaggcatcct atcagtatgg ttgcactgtc ccctctcggg 360
gctctggttg cctgttacaa aggagtaagt tgttccattg gcagcaatag agtagggatc 420
atcaagcagc tgaacaaagg ttgctctta 449

<210> 207
<211> 449

<212> DNA
<213> Human metapneumo virus

<400> 207
ataggggtct acgggagctc cgtaatttac atggtgcagc tgccaatctt tggcgttata 60
gacacgcctt gctggatagt aaaagcagcc ccttcttgtt ccgaaaaaaa gggaaactat 120
gcttgcctct taagagaaga ccaagggtgg tattgtcaga atgcagggtc aactgtttac 180
tacccaaatg agaaagactg tgaaacaaga ggagaccatg tcttttgcga cacagcagca 240
ggaattaatg ttgctgagca atcaaaggag tgcaacatca acatatccac tacaaattac 300
ccatgcaaag tcagcacagg aagacatcct atcagtatgg ttgcactgtc tcctcttggg 360
gctctggttg cttgctacaa aggagtaagc tgttccattg gcagcaacag agtagggatc 420
ataaagcagc tgaacaaagg ttgctccta 449

<210> 208
<211> 449
<212> DNA
<213> Human metapneumo virus

<400> 208
ataggggtct acgggagctc cgtaatttac atggtgcagc tgccaatctt tggcgttata 60
gacacgcctt gctggatagt aaaagcagcc ccttcttgtt ccgaaaaaaa gggaaactat 120
gcttgcctct taagagaaga ccaaggatgg tattgtcaga atgcagggtc aactgtttac 180
tacccaaatg agaaagactg tgaaacaaga ggagaccatg tcttttgcga cacagcagca 240
ggaattaatg ttgctgagca atcaaaggag tgcaacatca acatatccac cacaaattac 300
ccatgcaaag tcagcacagg aaggcatcct atcagtatgg ttgcactgtc ccctctcggg 360
gctctggttg cctgttacaa aggagtaagt tgttccattg gcagcaatag agtagggatc 420
atcaagcagc tgaacaaagg ttgctctta 449

<210> 209
<211> 449
<212> DNA
<213> Human metapneumo virus

<400> 209
ataggggtct acgggagctc cgtaatttac atggtgcagc tgccaatctt tggcgttata 60
gacacacctt gctggatagt aaaagcagcc ccttcttgtt ccgaaaaaaa gggaaattat 120
gcttgcctct taagagaaga ccaagggtgg tattgtcaga atgcagggtc aactgtttac 180
tacccaaatg agaaagactg tgaaacaaga ggagaccatg tcttttgcga cacagcagca 240
ggaattaatg ttgctgagca atcaaaggaa tgcaacatca acatatccac tacaaattac 300
ccatgcaaag tcagcacagg aagacatcct atcagtatgg ttgcactgtc tcctcttggg 360
gctctggttg cttgctacaa aggagtaagc tgttccattg gcagcaacag agtagggatc 420
atcaagcagc tgaacaaagg ttgctccta 449

<210> 210
<211> 449
<212> DNA
<213> Human metapneumo virus

<400> 210
ataggggtct acgggagctc cgtaatttac atggtgcagc tgccaatctt tggcgttata 60
gacacgcctt gctggatagt aaaagcagcc ccttcttgtt ccgaaaaaaa gggaaactat 120
gcttgcctct taagagaaga ccaaggatgg tattgtcaga atgcagggtc aactgtttac 180
tacccaaatg agaaagactg tgaaacaaga ggagaccatg tcttttgcga cacagcagca 240
ggaattaatg ttgctgagca atcaaaggag tgcaacatca acatatccac cacaaattac 300
ccatgcaaag tcagcacagg aaggcatcct atcagtatgg ttgcactgtc ccctctcggg 360
gctctggttg cctgttacaa aggagtaagt tgttccattg gcagcaatag agtagggatc 420
atcaagcagc tgaacaaagg ttgctctta 449

<210> 211
<211> 449
<212> DNA
<213> Human metapneumo virus

<400> 211
ataggggtct acgggagctc cgtaatttac atggtgcagc tgccaatctt tggcgttata 60
gacacgcctt gctggatagt aaaagcagcc ccttcttgtt ccgaaaaaaa gggaaactat 120
gcttgcctct taagagaaga ccaagggtgg tattgtcaga atgcagggtc aactgtttac 180
tacccaaatg agaaagactg tgaaacaaga ggagaccatg tcttttgcga cacagcagca 240
ggaattaatg ttgctgagca atcaaaggag tgcaacatca acatatccac tacaaattac 300
ccatgcaaag tcagcacagg aagacatcct atcagtatgg ttgcactgtc tcctcttggg 360
gctctggttg cttgctacaa aggagtaagc tgttccattg gcagcaacag agtagggatc 420
ataaagcagc tgaacaaagg ttgctccta 449

<210> 212
<211> 449
<212> DNA
<213> Human metapneumo virus

<400> 212
ataggggtct acggaagctc cgtgatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tattgtaaaa atgcaggatc cactgtttac 180
tacccaaatg aaaaagactg cgaaacaaga ggtgatcatg tttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacctaaagg ctgctcata 449

<210> 213
<211> 449
<212> DNA
<213> Human metapneumo virus

<400> 213
ataggggtct acggaagctc tgtgatttac atggttcaat tgccgatctt tggtgtcata 60
gatacacctt gttggatcat caaggcagct ccctcttgct cagaaaaaaa cgggaattat 120
gcttgcctcc taagagagga tcaagggtgg tattgtaaaa atgcaggatc tactgtttac 180
tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaattg ggttggaatc 420
atcaaacaat tacccaaagg ctgctcata 449

<210> 214
<211> 449
<212> DNA
<213> Human metapneumo virus

<400> 214
ataggggtct acggaagctc tgtgatttac atggttcaat tgccgatctt tggtgtcata 60
gatacacctt gttggatcat caaggcagct ccctcttgct cagaaaaaaa cgggaattat 120
gcttgcctcc taagagagga tcaagggtgg tattgtaaaa atgcaggatc tactgtttac 180
tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacccaaagg ctgctcata 449

<210> 215
<211> 449
<212> DNA
<213> Human metapneumo virus

<400> 215
ataggggtct acggaagctc tgtgatttac atggttcaat tgccgatctt tggtgtcata 60

gatacacctt gttggatcat caaggcagct ccctcttgct cagaaaaaaa cgggaattat 120
gcttgcctcc taagagagga tcaagggtgg tattgtaaaa atgcaggatc tactgtttac 180
tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacccaaagg ctgctcata 449

<210> 216
<211> 449
<212> DNA
<213> Human metapneumo virus

<400> 216
ataggggtct acggaagctc tgtgatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tattgcaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacctaaagg ctgctcata 449

<210> 217
<211> 449
<212> DNA
<213> Human metapneumo virus

<400> 217
ataggggtct acggaagctc cgtgatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacctaaagg ctgctcata 449

<210> 218
<211> 449
<212> DNA
<213> Human metapneumo virus

<400> 218
ataggggtct acggaagctc tgtaatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacctaaagg ctgctcata 449

<210> 219
<211> 449
<212> DNA
<213> Human metapneumo virus

<400> 219
ataggggtct acggaagctc cgtgatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tattgtaaaa atgcaggatc cactgtttac 180

tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacactct ataagcatgg ttgcactatc acctctcggt 360
gctttggtgg cttgctataa aggggtaagc tgctcgattg gcagcaatcg ggttggaatc 420
atcaaacaat tacctaaagg ctgctcata 449

<210> 220
<211> 449
<212> DNA
<213> Human metapneumo virus

<400> 220
ataggggtct acggaagctc cgtgatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacctaaagg ctgctcata 449

<210> 221
<211> 449
<212> DNA
<213> Human metapneumo virus

<400> 221
ataggggtct acggaagctc tgtgatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tattgtaaaa atgcaggatc cactgtttac 180
tacccaaatg aaaaagactg tgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacctaaagg ctgctcata 449

<210> 222
<211> 449
<212> DNA
<213> Human metapneumo virus

<400> 222
ataggggtct acggaagctc cgtgatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tactgtaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacctaaagg ctgctcata 449

<210> 223
<211> 449
<212> DNA
<213> Human metapneumo virus

<400> 223
ataggggtct acggaagctc tgtgatttac atggttcaat tgccgatctt tggggtcata 60
gatacacctt gttggataat caaggcagct ccctcttgct cagaaaaaaa cgggaattat 120
gcttgcctcc taagagagga tcaagggtgg tattgtaaaa atgcaggatc cactgtttac 180
tacccaaatg aaaaagactg cgaaacaaga ggtgatcatg ttttttgtga tacagcagca 240
gggatcaatg ttgctgagca atcaagagaa tgcaacatca acatatctac taccaactac 300

ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacccaaagg ctgctcata 449

<210> 224
<211> 449
<212> DNA
<213> Human metapneumo virus

<400> 224
ataggggtct acggaagctc tgtgatttac atggttcaat tgccgatctt tggtgtcata 60
gatacacctt gttggataat caaggcagcc ccctcttgct cagagaaaaa cgggaattat 120
gcttgcctcc taagagagga tcaagggtgg tattgtaaaa atgcaggatc cactgtttac 180
tacccaaatg aaaaagactg cgaaacaaga ggtgatcatg tgttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatt 420
atcaaacaat tacctaaagg ctgctcata 449

<210> 225
<211> 449
<212> DNA
<213> Human metapneumo virus

<400> 225
ataggggtct acggaagctc tgtgatttac atggttcaat tgccgatctt tggtgtcata 60
gatacacctt gttggataat caaggcagct ccctcttgct cagagaaaaa cgggaattat 120
gcttgcctcc taagagagga tcaagggtgg tattgtaaaa atgcaggatc cactgtttac 180
tacccaaatg aaaaagactg cgaaacaaga ggtgatcatg tgttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacctaaagg ctgctcata 449

<210> 226
<211> 449
<212> DNA
<213> Human metapneumo virus

<400> 226
ataggggtct acggaagctc tgtgatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tattgtaaaa atgcaggatc cactgtttac 180
tacccaaatg aaaaagactg tgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacctaaagg ctgctcata 449

<210> 227
<211> 449
<212> DNA
<213> Human metapneumo virus

<400> 227
ataggggtct acggaagctc cgtgatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420

atcaaacaat tacctaaagg ctgctcata                                    449

<210> 228
<211> 449
<212> DNA
<213> Human metapneumo virus

<400> 228
ataggggtct acggaagctc tgtgatttac atggttcaat tgccgatctt tggtgtcata 60
gatacacctt gttggataat caaggcagct ccctcttgct cagagaaaaa cgggaattat 120
gcttgcctcc taagagagga tcaagggtgg tattgtaaaa atgcaggatc cactgtttac 180
tacccaaatg aaaaagactg cgaaacaaga ggtgatcatg tgttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacctaaagg ctgctcata                                    449

<210> 229
<211> 449
<212> DNA
<213> Human metapneumo virus

<400> 229
ataggggtct acggaagctc tgtgatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tattgtaaaa atgcaggatc cactgtttac 180
tacccaaatg aaaaagactg cgaaacaaga ggtgatcatg ttttttgtga tacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacccaaagg ctgctcata                                    449

<210> 230
<211> 449
<212> DNA
<213> Human metapneumo virus

<400> 230
ataggggtct acggaagctc cgtgatttac atggttcaat tgccgatctt tggtgtcata 60
gatacacctt gttggataat caaggcagct ccctcttgct cagaaaaaaa cgggaattat 120
gcttgcctcc taagagagga tcaagggtgg tactgtaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct ataagcatgg ttgcactatc acctctcggt 360
gctttggtgg cttgctataa aggggtaagc tgctcgattg gcagcaatcg ggttggaatc 420
atcaaacaat tacctaaagg ctgctcata                                    449

<210> 231
<211> 449
<212> DNA
<213> Human metapneumo virus

<400> 231
ataggggtct acggaagctc tgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgcaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac caccaactac 300
ccatgcaaag tcagcacagg aagacaccct atcagcatgg ttgcactatc acctctcggt 360
gctttggtag cttgctacaa gggggttagc tgctcgattg gcagtaatcg ggttggaata 420
atcaaacaac tacctaaagg ctgctcata                                    449

<210> 232
<211> 449
<212> DNA
<213> Human metapneumo virus

<400> 232
ataggggtct acggaagctc tgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac caccaactac 300
ccatgcaaag tcagcacagg aagacacccc atcagcatgg ttgcactatc acctctcggt 360
gctttggtag cttgctacaa aggggttagc tgctcgattg gcagtaatcg ggttggaata 420
atcaaacaac tacctaaagg ctgctcata 449

<210> 233
<211> 449
<212> DNA
<213> Human metapneumo virus

<400> 233
ataggggtct acggaagctc tgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac caccaactac 300
ccatgcaaag tcagcacagg aagacaccct atcagcatgg ttgcactatc acctctcggt 360
gctttggtag cttgctacaa aggggttagc tgctcgattg gcagtaatcg ggttggaata 420
atcaaacaac tacctaaagg ctgctcata 449

<210> 234
<211> 449
<212> DNA
<213> Human metapneumo virus

<400> 234
ataggggtct acggaagctc cgtgatttac atggtccagc tgccgatctt tggtgtcata 60
gatacacctt gttggataat caaggcagct ccctcttgtt cagaaaaaga tggaaattat 120
gcttgcctcc taagagagga ccaagggtgg tattgtaaaa atgcgggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac aaccaactac 300
ccatgcaaag tcagcacagg aagacaccct atcagcatgg ttgcactatc acctctcggt 360
gctttggtag cttgctacaa aggggttagc tgttcgattg gcagtaatcg ggttggaata 420
atcaaacaac tacctaaagg ctgctcata 449

<210> 235
<211> 449
<212> DNA
<213> Human metapneumo virus

<400> 235
ataggggtct acggaagctc cgtgatttac atggtccagc taccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagctgca 240
gggatcaatg ttgctgagca atcaagagaa tgcaacatca acatatccac aaccaactac 300
ccatgcaaag tcagcacagg aagacaccct atcagcatgg ttgcactgtc acctctcggc 360
gctttggtag cttgctacaa aggggttagc tgttcgattg gcagtaatcg ggttggaata 420
atcaaacaac tacctaaagg ctgctcata 449

<210> 236
<211> 449

<212> DNA
<213> Human metapneumo virus

<400> 236
ataggggtct acggaagctc cgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac taccaactac 300
ccatgcaaag tcagcacagg aagacaccct atcagcatgg ttgcactatc acctctcggt 360
gctttggtag cttgctacaa aggggttagc tgttcgattg gcagtaatcg ggttggaata 420
atcaaacaac tacctaaagg ctgctcata 449

<210> 237
<211> 449
<212> DNA
<213> Human metapneumo virus

<400> 237
ataggggtct acggaagctc cgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg tgaaacaaga ggtgatcatg ttttttgtga cacagcagca 240
gggatcaatg ttgctgagca atcaagagaa tgcaacatca acatatctac aaccaactac 300
ccatgcaaag tcagcacagg aagacaccct atcagcatgg ttgcactatc acctctcggt 360
gctttggtag cttgctacaa aggggttagc tgttcgattg gcagtaatcg ggttggaata 420
atcaaacaac tacctaaagg ctgctcata 449

<210> 238
<211> 449
<212> DNA
<213> Human metapneumo virus

<400> 238
ataggggtct acggaagctc cgtgatttac atggtccagc tgccgatctt tggtgtcata 60
gatacacctt gttggataat caaggcagct ccctcttgtt cagaaaaaga tggaaattat 120
gcttgcctcc taagagagga ccaagggtgg tattgtaaaa atgcgggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac aaccaactac 300
ccatgcaaag tcagcacagg aagacaccct atcagcatgg ttgcactatc acctctcggt 360
gctttggtag cttgctacaa aggggttagc tgttcgattg gcagtaatcg ggttggaata 420
atcaaacaac tacctaaagg ctgctcata 449

<210> 239
<211> 449
<212> DNA
<213> Human metapneumo virus

<400> 239
ataggggtct acggaagctc cgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac aaccaactac 300
ccatgcaaag tcagcacagg aagacaccct atcagcatgg ttgcactatc acctctcggt 360
gctttggtag cttgctacaa aggggttagc tgttcgattg gcagtaatcg ggttggaata 420
atcaaacaac tacctaaagg ctgctcata 449

<210> 240
<211> 449
<212> DNA
<213> Human metapneumo virus

<400> 240
ataggggtct acggaagctc cgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg tgaaacaaga ggtgatcatg ttttttgtga cacagcagca 240
gggatcaatg ttgctgagca atcaagagaa tgcaacatca acatatctac aaccaactac 300
ccatgcaaag tcagcacagg aagacaccct atcagcatgg ttgcactatc acctctcggt 360
gctttggtag cttgctacaa aggggttagc tgttcgattg gcagtaatcg ggttggaata 420
atcaaacaac tacctaaagg ctgctcata 449

<210> 241
<211> 449
<212> DNA
<213> Human metapneumo virus

<400> 241
ataggggtct acggaagctc cgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagctgca 240
gggatcaatg ttgctgagca atcaagagaa tgcaacatca acatatctac aaccaactac 300
ccatgcaaag tcagcacagg aagacaccct atcagcatgg ttgcactatc acctctcggt 360
gctttggtag cttgctacaa aggggttagc tgttcaattg gcagtaatcg ggttggaata 420
atcaaacaac tacctaaagg ctgctcata 449

<210> 242
<211> 449
<212> DNA
<213> Human metapneumo virus

<400> 242
ataggggtct acggaagctc cgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagctgca 240
gggatcaatg ttgctgagca atcaagagaa tgcaacatca acatatctac aaccaactac 300
ccatgcaaag tcagcacagg aagacaccct atcagcatgg ttgcactatc acctctcggt 360
gctttggtag cttgctacaa aggggttagc tgttcaattg gcagtaatcg ggttggaata 420
atcaaacaac tacctaaagg ctgctcata 449

<210> 243
<211> 449
<212> DNA
<213> Human metapneumo virus

<400> 243
ataggggtct acggaagctc cgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagctgca 240
gggatcaatg ttgctgagca atcaagagaa tgcaacatca acatatctac aaccaactac 300
ccatgcaaag tcagcacagg aagacaccct atcagcatgg ttgcactatc acctctcggt 360
gctttggtag cttgctacaa gggggttagc tgttcgattg gcagtaatcg ggttggaata 420
atcaaacaac tacctaaagg ctgctcata 449

<210> 244
<211> 449
<212> DNA
<213> Human metapneumo virus

<400> 244
ataggggtct acggaagctc tgtgatttac atggtccagc tgccgatctt tggtgtcata 60

gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagctgca 240
gggatcaatg ttgctgagca atcaagagaa tgcaacatca acatatccac aaccaactac 300
ccatgcaaag tcagcacagg aagacaccct atcagcatgg ttgcactgtc acctctcggc 360
gctttggtag cttgctacaa aggggttagc tgttcgattg gcagtaatcg ggttggaata 420
atcaaacaac tacctaaagg ctgctcata 449

<210> 245
<211> 449
<212> DNA
<213> Human metapneumo virus

<400> 245
ataggggtct acggaagctc tgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagcagca 240
gggatcaacg ttgctgagca atcaagagaa tgcaacatca acatatctac caccaactat 300
ccgtgcaaag tcagcacagg aagacaccct atcagcatgg ttgcactatc acctctcggt 360
gctttggtag cttgctacaa aggggttagc tgctcgattg gcagtaatcg ggttggaata 420
atcaaacaac tacctaaagg ctgctcata 449

<210> 246
<211> 449
<212> DNA
<213> Human metapneumo virus

<400> 246
ataggggtct acggaagctc cgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagctgca 240
gggatcaatg ttgctgagca atcaagagaa tgcaacatca acatatctac aaccaactac 300
ccatgcaaag tcagcacagg aagacaccct atcagcatgg ttgcactatc acctctcggt 360
gctttggtag cttgctacaa aggggttagc tgttcaattg gcagtaatcg ggttggaata 420
atcaaacaac tacctaaagg ctgctcata 449

<210> 247
<211> 449
<212> DNA
<213> Human metapneumo virus

<400> 247
ataggggtct acggaagctc cgtgatttac atggtccagc tgccgatctt tggtgtcata 60
gatacacctt gttggataat caaggcagct ccctcttgtt cagaaaaaga tggaaattat 120
gcttgcctcc taagagagga tcaagggtgg tattgtaaaa atgcaggatc cactgtttac 180
tacccaaatg aaaaagactg cgaaacaaga ggtgatcatg ttttttgtga cacagcagca 240
gggatcaatg ttgctgagca atcaagagaa tgcaacatca acatatctac aaccaactac 300
ccatgcaaag tcagcacagg aagacaccct atcagcatgg ttgcactatc acctctcggt 360
gctttggtag cttgctacaa aggggttagc tgttcgattg gcagtaatcg ggttggaata 420
atcaaacaac tacctaaagg ctgctcata 449

<210> 248
<211> 149
<212> PRT
<213> Human metapneumo virus

<400> 248
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser

20                  25                  30
Cys Ser Gly Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 249
<211> 149
<212> PRT
<213> Human metapneumo virus

<400> 249
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 250
<211> 149
<212> PRT
<213> Human metapneumo virus

<400> 250
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala

65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145


<210> 251
<211> 149
<212> PRT
<213> Human metapneumo virus

<400> 251
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145


<210> 252
<211> 149
<212> PRT
<213> Human metapneumo virus

<400> 252
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly

115                 120                 125
Val Ser Cys Ser Ile Gly Ser Asn Arg Val Gly Ile Ile Lys Gln Leu
    130                 135                 140
Asn Lys Gly Cys Ser
145

<210> 253
<211> 149
<212> PRT
<213> Human metapneumo virus

<400> 253
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 254
<211> 149
<212> PRT
<213> Human metapneumo virus

<400> 254
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 255
<211> 149
<212> PRT
<213> Human metapneumo virus

<400> 255
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 256
<211> 149
<212> PRT
<213> Human metapneumo virus

<400> 256
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 257
<211> 149
<212> PRT
<213> Human metapneumo virus

<400> 257

Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 258
<211> 149
<212> PRT
<213> Human metapneumo virus

<400> 258
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 259
<211> 149
<212> PRT
<213> Human metapneumo virus

<400> 259
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45

Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 260
<211> 149
<212> PRT
<213> Human metapneumo virus

<400> 260
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 261
<211> 149
<212> PRT
<213> Human metapneumo virus

<400> 261
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Arg Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95

Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 262
<211> 149
<212> PRT
<213> Human metapneumo virus

<400> 262
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 263
<211> 149
<212> PRT
<213> Human metapneumo virus

<400> 263
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140

Asn Lys Gly Cys Ser
145

<210> 264
<211> 149
<212> PRT
<213> Human metapneumo virus

<400> 264
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 265
<211> 149
<212> PRT
<213> Human metapneumo virus

<400> 265
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 266
<211> 149

<212> PRT
<213> Human metapneumo virus

<400> 266
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 267
<211> 149
<212> PRT
<213> Human metapneumo virus

<400> 267
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 268
<211> 149
<212> PRT
<213> Human metapneumo virus

<400> 268
Ile Gly Val Tyr Gly Ser Ser Val Ile Tyr Met Val Gln Leu Pro Ile
1               5                   10                  15

Phe Gly Val Ile Asp Thr Pro Cys Trp Ile Val Lys Ala Ala Pro Ser
            20                  25                  30
Cys Ser Gly Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 269
<211> 149
<212> PRT
<213> Human metapneumo virus

<400> 269
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Gly Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 270
<211> 149
<212> PRT
<213> Human metapneumo virus

<400> 270
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60

Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 271
<211> 149
<212> PRT
<213> Human metapneumo virus

<400> 271
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 272
<211> 149
<212> PRT
<213> Human metapneumo virus

<400> 272
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110

Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 273
<211> 149
<212> PRT
<213> Human metapneumo virus

<400> 273
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 274
<211> 149
<212> PRT
<213> Human metapneumo virus

<400> 274
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Gly Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 275
<211> 149
<212> PRT
<213> Human metapneumo virus

<400> 275
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 276
<211> 149
<212> PRT
<213> Human metapneumo virus

<400> 276
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Gly Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 277
<211> 149
<212> PRT
<213> Human metapneumo virus

<400> 277
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 278
<211> 149
<212> PRT
<213> Human metapneumo virus

<400> 278
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 279
<211> 149
<212> PRT
<213> Human metapneumo virus

<400> 279
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln

35                  40                  45
Gly Trp Tyr Cys Gln Asn Ala Gly Ser Thr Val Tyr Tyr Pro Asn Glu
    50                  ,55                 60
Lys Asp Cys Glu Thr Arg Gly Asp His Val Phe Cys Asp Thr Val Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 280
<211> 149
<212> PRT
<213> Human metapneumo virus

<400> 280
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 281
<211> 149
<212> PRT
<213> Human metapneumo virus

<400> 281
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser

                    85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 282
<211> 149
<212> PRT
<213> Human metapneumo virus

<400> 282
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 283
<211> 149
<212> PRT
<213> Human metapneumo virus

<400> 283
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu

130                 135                 140
Asn Lys Gly Cys Ser
145


<210> 284
<211> 149
<212> PRT
<213> Human metapneumo virus

<400> 284
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145


<210> 285
<211> 149
<212> PRT
<213> Human metapneumo virus

<400> 285
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145


<210> 286

<211> 149
<212> PRT
<213> Human metapneumo virus

<400> 286
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145


<210> 287
<211> 149
<212> PRT
<213> Human metapneumo virus

<400> 287
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
            20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145


<210> 288
<211> 149
<212> PRT
<213> Human metapneumo virus

<400> 288
Ile Gly Val Tyr Gly Ser Ser Val Ile Tyr Met Val Gln Leu Pro Ile

1                   5                   10                  15
Phe Gly Val Ile Asp Thr Pro Cys Trp Ile Val Lys Ala Ala Pro Ser
                20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 289
<211> 149
<212> PRT
<213> Human metapneumo virus

<400> 289
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
                20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 290
<211> 149
<212> PRT
<213> Human metapneumo virus

<400> 290
Ile Gly Val Tyr Gly Ser Ser Val Ile Tyr Met Val Gln Leu Pro Ile
1               5                   10                  15
Phe Gly Val Ile Asp Thr Pro Cys Trp Ile Val Lys Ala Ala Pro Ser
                20                  25                  30
Cys Ser Glu Lys Lys Gly Asn Tyr Ala Cys Leu Leu Arg Glu Asp Gln
        35                  40                  45
Gly Trp Tyr Cys Gln Asn Ala Gly Ser Thr Val Tyr Tyr Pro Asn Glu

50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 291
<211> 149
<212> PRT
<213> Human metapneumo virus

<400> 291
Ile Gly Val Tyr Gly Ser Ser Val Ile Tyr Met Val Gln Leu Pro Ile
 1               5                  10                  15
Phe Gly Val Ile Asp Thr Pro Cys Trp Ile Val Lys Ala Ala Pro Ser
                20                  25                  30
Cys Ser Glu Lys Lys Gly Asn Tyr Ala Cys Leu Leu Arg Glu Asp Gln
            35                  40                  45
Gly Trp Tyr Cys Gln Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Lys Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Asn Lys Gly Cys Ser
145

<210> 292
<211> 149
<212> PRT
<213> Human metapneumo virus

<400> 292
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
                20                  25                  30
Cys Ser Glu Lys Asn Gly Asn Tyr Ala Cys Leu Leu Arg Glu Asp Gln
            35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser

                100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 293
<211> 149
<212> PRT
<213> Human metapneumo virus

<400> 293
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Trp Val Gly Ile Ile Lys Gln Leu
    130                 135                 140
Pro Lys Gly Cys Ser
145

<210> 294
<211> 149
<212> PRT
<213> Human metapneumo virus

<400> 294
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser

145

<210> 295
<211> 149
<212> PRT
<213> Human metapneumo virus

<400> 295
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 296
<211> 149
<212> PRT
<213> Human metapneumo virus

<400> 296
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 297
<211> 149
<212> PRT

<213> Human metapneumo virus

<400> 297
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145


<210> 298
<211> 149
<212> PRT
<213> Human metapneumo virus

<400> 298
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145


<210> 299
<211> 149
<212> PRT
<213> Human metapneumo virus

<400> 299
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser

20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Ser Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 300
<211> 149
<212> PRT
<213> Human metapneumo virus

<400> 300
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 301
<211> 149
<212> PRT
<213> Human metapneumo virus

<400> 301
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala

65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145


<210> 302
<211> 149
<212> PRT
<213> Human metapneumo virus

<400> 302
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145


<210> 303
<211> 149
<212> PRT
<213> Human metapneumo virus

<400> 303
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly

115                 120                 125
Val Ser Cys Ser Ile Gly Ser Asn Arg Val Gly Ile Ile Lys Gln Leu
    130                 135                 140
Pro Lys Gly Cys Ser
145



<210> 304
<211> 149
<212> PRT
<213> Human metapneumo virus

<400> 304
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145



<210> 305
<211> 149
<212> PRT
<213> Human metapneumo virus

<400> 305
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 306
<211> 149
<212> PRT
<213> Human metapneumo virus

<400> 306
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 307
<211> 149
<212> PRT
<213> Human metapneumo virus

<400> 307
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 308
<211> 149
<212> PRT
<213> Human metapneumo virus

<400> 308

Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 309
<211> 149
<212> PRT
<213> Human metapneumo virus

<400> 309
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 310
<211> 149
<212> PRT
<213> Human metapneumo virus

<400> 310
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asn Gly Asn Tyr Ala Cys Leu Leu Arg Glu Asp Gln
        35                  40                  45

Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145


<210> 311
<211> 149
<212> PRT
<213> Human metapneumo virus

<400> 311
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145


<210> 312
<211> 149
<212> PRT
<213> Human metapneumo virus

<400> 312
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95

Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 313
<211> 149
<212> PRT
<213> Human metapneumo virus

<400> 313
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 314
<211> 149
<212> PRT
<213> Human metapneumo virus

<400> 314
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140

Pro Lys Gly Cys Ser
145

<210> 315
<211> 149
<212> PRT
<213> Human metapneumo virus

<400> 315
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 316
<211> 149
<212> PRT
<213> Human metapneumo virus

<400> 316
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 317
<211> 149

<212> PRT
<213> Human metapneumo virus

<400> 317
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 318
<211> 149
<212> PRT
<213> Human metapneumo virus

<400> 318
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 319
<211> 149
<212> PRT
<213> Human metapneumo virus

<400> 319
Ile Gly Val Tyr Gly Ser Ser Val Ile Tyr Met Val Gln Leu Pro Ile
1               5                   10                  15

Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 320
<211> 149
<212> PRT
<213> Human metapneumo virus

<400> 320
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 321
<211> 149
<212> PRT
<213> Human metapneumo virus

<400> 321
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60

Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 322
<211> 149
<212> PRT
<213> Human metapneumo virus

<400> 322
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 323
<211> 149
<212> PRT
<213> Human metapneumo virus

<400> 323
Ile Gly Val Tyr Gly Ser Ser Val Ile Tyr Met Val Gln Leu Pro Ile
 1               5                  10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110

Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 324
<211> 149
<212> PRT
<213> Human metapneumo virus

<400> 324
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 325
<211> 149
<212> PRT
<213> Human metapneumo virus

<400> 325
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 326
<211> 149
<212> PRT
<213> Human metapneumo virus

<400> 326
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

<210> 327
<211> 149
<212> PRT
<213> Human metapneumo virus

<400> 327
Ile Gly Val Tyr Gly Ser Ser Val Ile Tyr Met Val Gln Leu Pro Ile
1               5                   10                  15
Phe Gly Val Ile Asp Thr Pro Cys Trp Ile Ile Lys Ala Ala Pro Ser
            20                  25                  30
Cys Ser Glu Lys Asp Gly Asn Tyr Ala Cys Leu Leu Arg Glu Asp Gln
        35                  40                  45
Gly Trp Tyr Cys Lys Asn Ala Gly Ser Thr Val Tyr Tyr Pro Asn Glu
    50                  55                  60
Lys Asp Cys Glu Thr Arg Gly Asp His Val Phe Cys Asp Thr Ala Ala
65                  70                  75                  80
Gly Ile Asn Val Ala Glu Gln Ser Arg Glu Cys Asn Ile Asn Ile Ser
                85                  90                  95
Thr Thr Asn Tyr Pro Cys Lys Val Ser Thr Gly Arg His Pro Ile Ser
            100                 105                 110
Met Val Ala Leu Ser Pro Leu Gly Ala Leu Val Ala Cys Tyr Lys Gly
        115                 120                 125
Val Ser Cys Ser Ile Gly Ser Asn Arg Val Gly Ile Ile Lys Gln Leu
    130                 135                 140
Pro Lys Gly Cys Ser
145

## Description

This application claims priority to U.S. Provisional Patent Application No. 60/358,934, filed February 21, 2002, which is incorporated by reference herein in its entirety.

Copending and co-assigned U.S. Patent Application _, filed on even date herewith, listing Ronaldus Fouchier, Bernadetta van den Hoogen, Albertus Osterhaus, Aurelia Haller, and Roderick Tang as Inventors, entitled "METAPNEUMOVIRUS STRAINS AND THEIR USE IN VACCINE FORMULATIONS AND AS VECTORS FOR EXPRESSION OF ANTIGENIC SEQUENCES", is incorporated herein by reference in its entirety.

### 1. INTRODUCTION

The present invention relates to recombinant parainfluenza virus (PIV) cDNA or RNA that may be used to express heterologous gene products in appropriate host cell systems and/or to rescue negative strand RNA recombinant viruses that express, package, and/or present the heterologous gene product. In particular, the present invention encompasses vaccine preparations comprising chimeric PIV expressing a heterologous gene product, wherein the heterologous gene product is preferably an antigenic peptide or polypeptide. In one embodiment, the PIV vector of the invention expresses one, two, or three heterologous gene products that may be encoded by the same or different viruses. In a preferred embodiment, the heterologous sequence encodes a heterologous gene product that is an antigenic polypeptide from another species of PIV or from another negative strand RNA virus, including but not limited to, influenza virus, respiratory syncytial virus (RSV), mammalian metapneumovirus, and avian pneumovirus. The vaccine preparations of the invention encompass multivalent vaccines, including bivalent and trivalent vaccine preparations. The multivalent vaccines of the invention may be administered in the form of one PIV vector expressing each heterologous antigenic sequence or two or more PIV vectors each encoding different heterologous antigenic sequences. The vaccine preparations of the invention can be administered alone or in combination with other vaccines, prophylactic agents, or therapeutic agents.

### 2. BACKGROUND OF THE INVENTION

Parainfluenza viral infection results in serious respiratory tract disease in infants and children. (Tao et al., 1999, Vaccine 17: 1100-08). Infectious parainfluenza viral infections account for approximately 20% of all hospitalizations of pediatric patients that suffer from respiratory tract infections worldwide. *Id.* An effective antiviral therapy is not available to treat PIV related diseases, and a vaccine to prevent PIV infection has not yet been approved.

PIV is a member of the genus respirovirus (PIV1, PIV3) or rubulavirus (PIV2, PIV4) of the paramyxoviridae family. PIV is made up of two structural modules: (1) an internal ribonucleoprotein core, or nucleocapsid, containing the viral genome, and (2) an outer, roughly spherical lipoprotein envelope. Its genome consists of a single strand of negative sense RNA, that is approximately 15,456 nucleotides in length and encodes at least eight polypeptides. These proteins include the nucleocapsid structural protein (NP, NC, or N depending on the genera), the phosphoprotein (P), the matrix protein (M), the fusion glycoprotein (F), the hemagglutinin-neuraminidase glycoprotein (HN), the large polymerase protein (L), and the C and D proteins of unknown function. *Id.*

The parainfluenza nucleocapsid protein (NP, NC, or N) contains two domains within each protein unit. These domains include: an amino-terminal domain, that comprises nearly two-thirds of the molecule and interacts directly with the RNA, and a carboxyl-terminal domain, that lies on the surface of the assembled nucleocapsid. A hinge is thought to exist at the junction of these two domains, thereby imparting some flexibility on this protein (see Fields et al. (ed.), 1991, FUNDAMENTAL VIROLOGY, 2nd ed, Raven Press, New York, incorporated by reference herein in its entirety). The matrix protein (M) is apparently involved in viral assembly, and it interacts with both the viral membrane and the nucleocapsid proteins. The phosphoprotein (P) is subject to phosphorylation and has been implicated in transcription regulation, methylation, phosphorylation and polyadenylation. Produced initially as an inactive precursor, the fusion glycoprotein (F) is cleaved upon translation to produce two disulfide linked polypeptides. The active F protein interacts with the viral membrane where it facilitates penetration of the parainfluenza virion into host cells by promoting the fusion of the viral envelope with the host cell plasma membrane. *Id.* The glycoprotein, hemagglutinin-neuraminidase (HN) protrudes from the envelope and imparts hemagglutinin and neuraminidase activities on the virus. HN has a strongly hydrophobic amino terminus that functions to anchor the HN protein into the lipid bilayer. *Id.* Finally, the large polymerase protein (L) plays an important role in both transcription and replication. *Id.*

Bovine parainfluenza virus was first isolated in 1959 from calves showing signs of shipping fever. It has since been isolated from normal cattle, aborted fetuses, and cattle exhibiting signs of respiratory disease (Breker-Klassen et al., 1996, Can. J. Vet. Res. 60: 228-236. *See also* Shibuta, 1977, Microbiol. Immunol. 23 (7), 617-628). Human and bovine PIV3 share neutralizing epitopes but show distinct antigenic properties. Significant differences exist between the human and bovine viral strains in the HN protein. In fact, a bovine strain induces some neutralizing antibodies to hPIV infection while a human strain seems to induce a wider spectrum of neutralizing antibodies against human PIV3 (Van Wyke Coelingh et al., 1990, J. Virol. 64:3833-3843).

The replication of all negative-strand RNA viruses, including PIV, is complicated by the absence of the cellular machinery that is required to replicate RNA. Additionally, the negative-strand genome must be transcribed into a positive-strand (mRNA) copy before translation can occur. Consequently, the genomic RNA alone cannot synthesize the required RNA-dependent RNA polymerase upon entry into the cell. The L, P and N proteins must enter the host cell along with the genomic RNA.

It is hypothesized that most or all of the viral proteins that transcribe PIV mRNA also carry out the replication of the genome. The mechanism that regulates the alternative uses (*i.e.*, transcription or replication) of the same complement of proteins has not been clearly identified, but the process appears to involve the abundance of free forms of one or more of the nucleocapsid proteins. Directly following penetration of the virus, transcription is initiated by the L protein using the negative-sense RNA in the nucleocapsid as a template. Viral RNA synthesis is regulated such that it produces monocistronic mRNAs during transcription.

Following transcription, virus genome replication is the second essential event in infection by negative-strand RNA viruses. As with other negative-strand RNA viruses, virus genome replication in PIV is mediated by virus-specified proteins. The first products of replicative RNA synthesis are complementary copies (*i.e.*, plus-polarity) of the PIV genomic RNA (cRNA). These plus-stranded copies (anti-genomes) differ from the plus-stranded mRNA transcripts in the structure of their termini. Unlike the mRNA transcripts, the anti-genomic cRNAs are not capped or methylated at the 5' termini, and they are not truncated nor polyadenylated at the 3' termini. The cRNAs are coterminal with their negative strand templates and contain all the genetic information in the complementary form. The cRNAs serve as templates for the synthesis of PIV negative-strand viral genomes (vRNAs).

The bPIV negative strand genomes (vRNAs) and antigenomes (cRNAs) are encapsidated by nucleocapsid proteins; the only unencapsidated RNA species are viral mRNAs. Replication and transcription of bPIV RNA occurs in the cytoplasm of the host cell. Assembly of the viral components appears to take place at the host cell plasma membrane where the mature virus is released by buddin.

### 2.1. PARAMYXOVIRUS

Classically, as devastating agents of disease, paramyxoviruses account for many animal and human deaths worldwide each year. The Paramyxoviridae form a family within the order of Mononegavirales (negative-sense single stranded RNA viruses), consisting of the sub-families Paramyxovirinae and Pneumovirinae. The latter sub-family is at present taxonomically divided in the genera Pneumovirus and Metapneumovirus (Pringle, 1999, Arch. Virol. 144/2, 2065-2070). Human respiratory syncytial virus (hRSV), a species of the Pneumovirus genus, is the single most important cause of lower respiratory tract infections during infancy and early childhood worldwide (Domachowske, & Rosenberg, 1999, Clin. Microbio. Rev. 12(2): 298-309). Other members of the Pneumovirus genus include the bovine and ovine respiratory syncytial viruses and pneumonia virus of mice (PVM).

In the past decades several etiological agents of mammalian disease, in particular of respiratory tract illnesses (RTI), in particular of humans, have been identified (Evans, In: Viral Infections of Humans, Epidemiology and Control. 3th ed. (ed. Evans, A.S) 22-28 (Plenum Publishing Corporation, New York, 1989)). Classical etiological agents of RTI with mammals are respiratory syncytial viruses belonging to the genus Pneumovirus found with humans (hRSV) and ruminants such as cattle or sheep (bRSV and/or oRSV). In human RSV differences in reciprocal cross neutralization assays, reactivity of the G proteins in immunological assays and nucleotide sequences of the G gene are used to define two hRSV antigenic subgroups. Within the subgroups the amino acid sequences show 94 % (subgroup A) or 98% (subgroup B) identity, while only 53% amino acid sequence identity is found between the subgroups. Additional variability is observed within subgroups based on monoclonal antibodies, RT-PCR assays and RNAse protection assays. Viruses from both subgroups have a worldwide distribution and may occur during a single season. Infection may occur in the presence of pre-existing immunity and the antigenic variation is not strictly required to allow re-infection. See, for example Sullender, 2000, Clinical Microbiology Reviews 13(1): 1-15; Collins et al. Fields Virology, ed. B.N. Knipe, Howley, P.M. 1996, Philadelphia: Lippencott-Raven. 1313-1351; Johnson et al., 1987, (Proc Natl Acad Sci USA, 84(16): 5625-9; Collins, in The Paramyxoviruses, D.W. Kingsbury, Editor. 1991, Plenum Press: New York. p. 103-153.

Another classical Pneumovirus is the pneumonia virus of mice (PVM), in general only found with laboratory mice. However, a proportion of the illnesses observed among mammals can still not be attributed to known pathogens.

### 2.2. RSV INFECTIONS

Respiratory syncytial virus (RSV) is the leading cause of serious lower respiratory tract disease in infants and children (Feigen et al., eds., 1987, In: Textbook of Pediatric Infectious Diseases, WB Saunders, Philadelphia at pages 1653-1675; New Vaccine Development, Establishing Priorities, Vol. 1, 1985, National Academy Press, Washington DC at pages 397-409; and Ruuskanen et al., 1993, Curr. Probl. Pediatr. 23:50-79). The yearly epidemic nature of RSV infection is evident worldwide, but the incidence and severity of RSV disease in a given season vary by region (Hall, 1993, Contemp. Pediatr. 10:92-110). In temperate regions of the northern hemisphere, it usually begins in late fall and ends in late spring. Primary RSV infection occurs most often in children from 6 weeks to 2 years of age and uncommonly in the first 4 weeks of life during nosocomial epidemics (Hall et al., 1979, New Engl. J. Med. 300:393-396). Children at increased risk for RSV infection include, but are not limited to, preterm infants (Hall et al., 1979, New Engl. J. Med. 300:393-396) and children with bronchopulmonary dysplasia (Groothuis et al., 1988, Pediatrics 82:199-203), congenital heart disease (MacDonald et al., New Engl. J. Med. 307:397-400), congenital or acquired immunodeficiency (Ogra et al., 1988, Pediatr. Infect. Dis. J. 7:246-249; and Pohl et al., 1992, J. Infect. Dis. 165:166-169), and cystic fibrosis (Abman et al., 1988, J. Pediatr. 113:826-830). The fatality rate in infants with heart or lung disease who are hospitalized with RSV infection is 3%-4% (Navas et al., 1992, J. Pediatr. 121:348-354).

RSV infects adults as well as infants and children. In healthy adults, RSV causes predominantly upper respiratory tract disease. It has recently become evident that some adults, especially the elderly, have symptomatic RSV infections more frequently than had been previously reported (Evans, A.S., eds., 1989, Viral Infections of Humans. Epidemiology and Control, 3rd ed., Plenum Medical Book, New York at pages 525-544). Several epidemics also have been reported among nursing home patients and institutionalized young adults (Falsey, A.R., 1991, Infect. Control Hosp. Epidemiol. 12:602-608; and Garvie et al., 1980, Br. Med. J. 281:1253-1254). Finally, RSV may cause serious disease in immunosuppressed persons, particularly bone marrow transplant patients (Hertz et al., 1989, Medicine 68:269-281).

Treatment options for established RSV disease are limited. Severe RSV disease of the lower respiratory tract often requires considerable supportive care, including administration of humidified oxygen and respiratory assistance (Fields et al., eds, 1990, Fields Virology, 2nd ed., Vol. 1, Raven Press, New York at pages 1045-1072).

While a vaccine might prevent RSV infection, and/or RSV-related disease, no vaccine is yet licensed for this indication. A major obstacle to vaccine development is safety. A formalin-inactivated vaccine, though immunogenic, unexpectedly caused a higher and more severe incidence of lower respiratory tract disease due to RSV in immunized infants than in infants immunized with a similarly prepared trivalent parainfluenza vaccine (Kim et al., 1969, Am. J. Epidemiol. 89:422-434; and Kapikian et al., 1969, Am. J. Epidemiol. 89:405-421). Several candidate RSV vaccines have been abandoned and others are under development (Murphy et al., 1994, Virus Res. 32:13-36), but even if safety issues are resolved, vaccine efficacy must also be improved. A number of problems remain to be solved. Immunization would be required in the immediate neonatal period since the peak incidence of lower respiratory tract disease occurs at 2-5 months of age. The immaturity of the neonatal immune response together with high titers of maternally acquired RSV antibody may be expected to reduce vaccine immunogenicity in the neonatal period (Murphy et al., 1988, J. Virol. 62:3907-3910; and Murphy et al., 1991, Vaccine 9:185-189). Finally, primary RSV infection and disease do not protect well against subsequent RSV disease (Henderson et al., 1979, New Engl. J. Med. 300:530-534).

Currently, the only approved approach to prophylaxis of RSV disease is passive immunization. Initial evidence suggesting a protective role for IgG was obtained from observations involving maternal antibody in ferrets (Prince, G.A., Ph.D. diss., University of California, Los Angeles, 1975) and humans (Lambrecht et al, 1976, J. Infect. Dis. 134:211-217; and Glezen et al., 1981, J. Pediatr. 98:708-715). Hemming et al. (Morell et al., eds., 1986, Clinical Use of Intravenous Immunoglobulins, Academic Press, London at pages 285-294) recognized the possible utility of RSV antibody in treatment or prevention of RSV infection during studies involving the pharmacokinetics of an intravenous immune globulin (IVIG) in newborns suspected of having neonatal sepsis. In this study, it was noted that one infant, whose respiratory secretions yielded RSV, recovered rapidly after IVIG infusion. Subsequent analysis of the IVIG lot revealed an unusually high titer of RSV neutralizing antibody. This same group of investigators then examined the ability of hyperimmune serum or immune globulin, enriched for RSV neutralizing antibody, to protect cotton rats and primates against RSV infection (Prince et al., 1985, Virus Res. 3:193-206; Prince et al., 1990, J. Virol. 64:3091-3092; Hemming et al., 1985, J. Infect. Dis. 152:1083-1087; Prince et al., 1983, Infect. Immun. 42:81-87; and Prince et al., 1985, J. Virol. 55:517-520). Results of these studies indicate that IVIG may be used to prevent RSV infection, in addition to treating or preventing RSV-related disorders.

Recent clinical studies have demonstrated the ability of this passively administered RSV hyperimmune globulin (RSV IVIG) to protect at-risk children from severe lower respiratory infection by RSV (Groothius et al., 1993, New Engl. J. Med. 329:1524-1530; and The PREVENT Study Group, 1997, Pediatrics 99:93-99). While this is a major advance in preventing RSV infection, this treatment poses certain limitations in its widespread use. First, RSV IVIG must be infused intravenously over several hours to achieve an effective dose. Second, the concentrations of active material in hyperimmune globulins are insufficient to treat adults at risk or most children with comprised cardiopulmonary function. Third, intravenous infusion necessitates monthly hospital visits during the RSV season. Finally, it may prove difficult to select sufficient donors to produce a hyperimmune globulin for RSV to meet the demand for this product. Currently, only approximately 8% of normal donors have RSV neutralizing antibody titers high enough to qualify for the production of hyperimmune globulin.

One way to improve the specific activity of the immunoglobulin would be to develop one or more highly potent RSV neutralizing monoclonal antibodies (MAbs). Such MAbs should be human or humanized in order to retain favorable pharmacokinetics and to avoid generating a human anti-mouse antibody response, as repeat dosing would be required throughout the RSV season. Two glycoproteins, F and G, on the surface of RSV have been shown to be targets of neutralizing antibodies (Fields et al., 1990, supra; and Murphy et al., 1994 , supra).

A humanized antibody directed to an epitope in the A antigenic site of the F protein of RSV, SYNAGIS®, is approved for intramuscular administration to pediatric patients for prevention of serious lower respiratory tract disease caused by RSV at recommended monthly doses of 15 mg/kg of body weight throughout the RSV season (November through April in the northern hemisphere). SYNAGIS® is a composite of human (95%) and murine (5%) antibody sequences. See, Johnson et al., 1997, J. Infect. Diseases 176:1215-1224 and U.S. Patent No. 5,824,307, the entire contents of which are incorporated herein by reference. The human heavy chain sequence was derived from the constant domains of human IgG1 and the variable framework regions of the VH genes of Cor (Press et al., 1970, Biochem. J. 117:641-660) and Cess (Takashi et al., 1984, Proc. Natl. Acad. Sci. USA 81:194-198). The human light chain sequence was derived from the constant domain of C and the variable framework regions of the VL gene K104 with J -4 (Bentley et al., 1980, Nature 288:5194-5198). The murine sequences derived from a murine monoclonal antibody, Mab 1129 (Beeler et al., 1989, J. Virology 63:2941-2950), in a process which involved the grafting of the murine complementarity determining regions into the human antibody frameworks.

### 2.3. AVIAN PNEUMOVIRUSES

Respiratory disease caused by an avian pneumovirus (APV) was first described in South Africa in the late 1970s (Buys et al., 1980, Turkey 28:36-46) where it had a devastating effect on the turkey industry. The disease in turkeys was characterized by sinusitis and rhinitis and was called turkey rhinotracheitis (TRT). The European isolates of APV have also been strongly implicated as factors in swollen head syndrome (SHS) in chickens (O'Brien, 1985, Vet. Rec. 117:619-620). Originally, the disease appeared in broiler chicken flocks infected with Newcastle disease virus (NDV) and was assumed to be a secondary problem associated with Newcastle disease (ND). Antibody against European APV was detected in affected chickens after the onset of SHS (Cook et al., 1988, Avian Pathol. 17:403-410), thus implicating APV as the cause.

Avian pneumovirus (APV) also known as turkey rhinotracheitis virus (TRTV), the aetiological agent of avian rhinotracheitis, an upper respiratory tract infection of turkeys (Giraud et al., 1986, Vet. Res. 119:606-607), is the sole member of the recently assigned Metapneumovirus genus, which, as said was until now not associated with infections, or what is more, with disease of mammals. Serological subgroups of APV can be differentiated on the basis of nucleotide or amino acid sequences of the G glycoprotein and neutralization tests using monoclonal antibodies that also recognize the G glycoprotein. However, other differences in the nucleotide and amino acid sequences can be used to distinguish serological subgroups of APV. Within subgroups A, B and D, the G protein shows 98.5 to 99.7% aa sequence identity within subgroups while between the subgroups only 31.2- 38% aa identity is observed. See for example Collins et al., 1993, Avian Pathology, 22: p. 469-479; Cook et al., 1993, Avian Pathology, 22: 257-273; Bayon-Auboyer et al., J Gen Virol, 81(Pt 11): 2723-33; Seal, 1998, Virus Res, 58(1-2): 45-52; Bayon-Auboyer et al., 1999, Arch Virol, 144(6): 91-109; Juhasz, et al., 1994, J Gen Virol, 75(Pt 11): 2873-80.

A further serotype of APV is provided in WO00/20600, incorporated by reference herein, which describes the Colorado isolate of APV and compared it to known APV or TRT strains with in vitro serum neutralization tests. First, the Colorado isolate was tested against monospecific polyclonal antisera to recognized TRT isolates. The Colorado isolate was not neutralized by monospecific antisera to any of the TRT strains. It was, however, neutralized by a hyperimmune antiserum raised against a subgroup A strain. This antiserum neutralized the homologous virus to a titre of 1:400 and the Colorado isolate to a titer of 1: 80. Using the above method, the Colorado isolate was then tested against TRT monoclonal antibodies. In each case, the reciprocal neutralization titer was <10. Monospecific antiserum raised to the Colorado isolate was also tested against TRT strains of both subgroups. None of the TRT strains tested were neutralized by the antiserum to the Colorado isolate.

The Colorado strain of APV does not protect SPF chicks against challenge with either a subgroup A or a subgroup B strain of TRT virus. These results suggest that the Colorado isolate may be the first example of a further serotype of avian pneumovirus (See, Bayon-Auboyer et al., 2000, J. Gen. Vir. 81:2723-2733).

The avian pneumovirus is a single stranded, non-segmented RNA virus that belongs to the sub-family Pneumovirinae of the family Paramyxoviridae, genus metapneumovirus (Cavanagh and Barrett, 1988, Virus Res. 11:241-256; Ling et al., 1992, J. Gen. Virol. 73:1709-1715; Yu et al., 1992, J. Gen. Virol. 73:1355-1363). The Paramyxoviridae family is divided into two sub-families: the Paramyxovirinae and Pneumovirinae. The subfamily Paramyxovirinae includes, but is not limited to, the genera: Paramyxovirus, Rubulavirus, and Morbillivirus. Recently, the sub-family Pneumovirinae was divided into two genera based on gene order, and sequence homology, i.e. pneumovirus and metapneumovirus (Naylor et al., 1998, J. Gen. Virol., 79:1393-1398; Pringle, 1998, Arch. Virol. 143:1449-1159). The pneumovirus genus includes, but is not limited to, human respiratory syncytial virus (hRSV), bovine respiratory syncytial virus (bRSV), ovine respiratory syncytial virus, and mouse pneumovirus. The metapneumovirus genus includes, but is not limited to, European avian pneumovirus (subgroups A and B), which is distinguished from hRSV, the type species for the genus pneumovirus (Naylor et al., 1998, J. Gen. Virol., 79:1393-1398; Pringle, 1998, Arch. Virol. 143:1449-1159). The US isolate of APV represents a third subgroup (subgroup C) within metapneumovirus genus because it has been found to be antigenically and genetically different from European isolates (Seal, 1998, Virus Res. 58:45-52; Senne et al., 1998, In: Proc. 47th WPDC, California, pp. 67-68).

Electron microscopic examination of negatively stained APV reveals pleomorphic, sometimes spherical, virions ranging from 80 to 200 nm in diameter with long filaments ranging from 1000 to 2000 nm in length (Collins and Gough, 1988, J. Gen. Virol. 69:909-916). The envelope is made of a membrane studded with spikes 13 to 15 nm in length. The nucleocapsid is helical, 14 nm in diameter and has 7 nm pitch. The nucleocapsid diameter is smaller than that of the genera Paramyxovirus and Morbillivirus, which usually have diameters of about 18 nm.

Avian pneumovirus infection is an emerging disease in the USA despite its presence elsewhere in the world in poultry for many years. In May 1996, a highly contagious respiratory disease of turkeys appeared in Colorado, and an APV was subsequently isolated at the National Veterinary Services Laboratory (NVSL) in Ames, Iowa (Senne et al., 1997, Proc. 134th Ann. Mtg., AVMA, pp. 190). Prior to this time, the United States and Canada were considered free of avian pneumovirus (Pearson et al., 1993, In: Newly Emerging and Re-emerging Avian Diseases: Applied Research and Practical Applications for Diagnosis and Control, pp. 78-83; Hecker and Myers, 1993, Vet. Rec. 132:172). Early in 1997, the presence of APV was detected serologically in turkeys in Minnesota. By the time the first confirmed diagnosis was made, APV infections had already spread to many farms. The disease is associated with clinical signs in the upper respiratory tract: foamy eyes, nasal discharge and swelling of the sinuses. It is exacerbated by secondary infections. Morbidity in infected birds can be as high as 100%. The mortality can range from 1 to 90% and is highest in six to twelve week old poults.

Avian pneumovirus is transmitted by contact. Nasal discharge, movement of affected birds, contaminated water, contaminated equipment; contaminated feed trucks and load-out activities can contribute to the transmission of the virus. Recovered turkeys are thought to be carriers. Because the virus is shown to infect the epithelium of the oviduct of laying turkeys and because APV has been detected in young poults, egg transmission is considered a possibility.

A significant portion of human respiratory disease is caused by members of the viral sub-families Paramyxovirinae and Pneumovirinae, there still remains a need for an effective vaccine to confer protection against a variety of viruses that result in respiratory tract infection.

Citation or discussion of a reference herein shall not be construed as an admission that such is prior art to the present invention.

### 3. SUMMARY OF THE INVENTION

The present invention relates to recombinant parainfluenza virus cDNA and RNA that may be engineered to express heterologous or non-native gene products, in particular, to express antigenic polypeptides and peptides. In one embodiment, the present invention relates to recombinant bovine or human parainfluenza viruses which are engineered to express heterologous antigens or immunogenic and/or antigenic fragments of heterologous antigens. In another embodiment of the invention, the recombinant bovine or human parainfluenza viruses are engineered to express sequences that are non-native to the PIV genome, including mutated PIV nucleotide sequences. In particular, the invention relates to recombinant Kansas-strain bovine parainfluenza type 3 virus as well as cDNA and RNA molecules coding for the same. The present invention also relates to recombinant PIV that contain modifications that result in chimeric viruses with phenotypes more suitable for use in vaccine formulations.

The present invention provides for the first time a chimeric PIV formulated as a vaccine that is able to confer protection against various viral infections, in particular, viruses that result in respiratory tract infections. In a specific embodiment, the present invention provides a vaccine that is able to confer protection against parainfluenza, influenza, or respiratory syncytial viral infection. The present invention provides for the first time a vaccine that is able to confer protection against metapneumovirus infection in a mammalian host.

In accordance with the present invention, a recombinant virus is one derived from a bovine parainfluenza virus or a human parainfluenza virus that is encoded by endogenous or native genomic sequences or non-native genomic sequences. In accordance with the invention, a non-native sequence is one that is different from the native or endogenous genomic sequence due to one or more mutations, including, but not limited to, point mutations, rearrangements, insertions, deletions, etc. to the genomic sequence that may or may not result in a phenotypic change.

In accordance with the present invention, a chimeric virus of the invention is a recombinant bPIV or hPIV which further comprises one or more heterologous nucleotide sequences. In accordance with the invention, a chimeric virus may be encoded by a nucleotide sequence in which heterologous nucleotide sequences have been added to the genome or in which nucleotide sequences have been replaced with heterologous nucleotide sequences.

The present invention also relates to engineered recombinant parainfluenza viruses and viral vectors that encode combinations of heterologous sequences which encode gene products, including but not limited to, genes from different strains of PIV, influenza virus, respiratory syncytial virus, mammalian metapneumovirus (*e.g.*, human metapneumovirus), avian pneumovirus, measles, mumps, other viruses, pathogens, cellular genes, tumor antigens, or combinations thereof. Furthermore, the invention relates to engineered recombinant parainfluenza viruses that contain a nucleotide sequence derived from a metapneumovirus in combination with a nucleotide sequence derived from a respiratory syncytial virus, and further in combination with a nucleotide sequence derived from a human parainfluenza virus. The invention also encompasses recombinant parainfluenza vectors and viruses that are engineered to encode genes from different species and strains of the parainfluenza virus, including the F and HN genes of human PIV3.

In one embodiment, the PIV vector of the invention is engineered to express one or more heterologous sequences, wherein the heterologous sequences encode gene products that are preferably antigenic gene products. In a preferred embodiment, the PIV vector of the invention expresses one, two or three heterologous sequences that encode antigenic polypeptides and peptides. In some embodiments, the heterologous sequences are derived from the same virus or from different viruses. In a preferred embodiment, the heterologous sequences encode heterologous gene products that are antigenic polypeptides from another species of PIV, such as a human PIV, a mutant strain of PIV, or from another negative strand RNA virus, including but not limited to, influenza virus, respiratory syncytial virus (RSV), mammalian metapneumovirus (*e.g.*, human metapneumovirus (hMPV)), and avian pneumovirus. In one embodiment, the heterologous sequence encodes an immunogenic and/or antigenic fragment of a heterologous gene product.

In a preferred embodiment, the recombinant PIV is a bovine PIV type 3, or an attenuated human PIV type 3. In one embodiment, the sequences encoding fusion (F) protein, hemagglutinin (HN) glycoprotein, or other non-essential genes of the PIV genome are deleted and are substituted by heterologous antigenic sequences. In yet another embodiment, the PIV genome contains mutations or modifications, in addition to the heterologous nucleotide sequences, that result in a chimeric virus having a phenotype that is more suitable for use in vaccine formulations, *e.g.*, an attenuated phenotype or a phenotype with enhanced antigenicity.

In a specific embodiment, the heterologous nucleotide sequence to be inserted into the PIV genome is derived from the nucleotide sequences encoding a F protein, a G protein or an HN protein. In certain embodiments, the nucleotide sequence to be inserted encodes a chimeric F protein, a chimeric G protein or a chimeric HN protein. In a specific embodiment, the F protein comprises an ectodomain of a F protein of a metapneumovirus, a transmembrane domain of a F protein of a parainfluenza virus, and a luminal domain of a F protein of a parainfluenza virus. In certain embodiments, the nucleotide sequence to be inserted encodes a F protein, wherein the transmembrane domain of the F protein is deleted so that a soluble F protein is expressed.

In another specific embodiment, the invention provides a chimeric virus comprising a PIV genome comprising a heterologous nucleotide sequence derived from a metapneumovirus. In a specific embodiment, the PIV virus is a Kansas-strain bovine parainfluenza type 3 virus. In other embodiments, the PIV virus is a human parainfluenza virus with an attenuated phenotype. In yet other embodiments, the invention provides a chimeric bovine parainfluenza virus type 3/human parainfluenza virus engineered to contain human parainfluenza F and HN genes in a bovine parainfluenza backbone. The chimeric virus may further comprise a heterologous nucleotide sequence derived from a metapneumovirus, and/or further comprise a heterologous nucleotide sequence derived from a respiratory syncytial virus.

In certain embodiments, the virus of the invention comprises heterologous nucleotide sequences derived from at least two different genes of a metapneumovirus. In a specific embodiment, the heterologous sequence is derived from a metapneumovirus, *e.g.*, avian pneumovirus and human metapneumovirus. More specifically, the heterologous sequence is derived from an avian pneumovirus, including avian pneumovirus type A, B, C or D, preferably C.

The present invention also provides vaccine preparations and immunogenic compositions comprising chimeric PIV expressing one or more heterologous antigenic sequences. In a specific embodiment, the present invention provides multivalent vaccines, including bivalent and trivalent vaccines. The multivalent vaccines of the invention may be administered in the form of one PIV vector expressing each heterologous antigenic sequence or two or more PIV vectors each encoding different heterologous antigenic sequences. In one embodiment, the vaccine preparation of the invention comprises chimeric PIV expressing one, two or three heterologous polypeptides, wherein the heterologous polypeptides can be encoded by sequences derived from one strain of the same virus, different strains of the same virus, or different viruses. Preferably, the heterologous antigenic sequences are derived from a negative strand RNA virus, including but not limited to, influenza virus, parainfluenza virus, respiratory syncytial virus (RSV), mammalian metapneumovirus (*e.g.*, human metapneumovirus (hMPV)), and avian pneumovirus (APV). The heterologous antigenic sequences include, but are not limited to, sequences that encode human parainfluenza virus F or HN protein, F protein of RSV, HA protein of influenza virus type A, B, and C, and F protein of human MPV and avian pneumovirus. More preferably, the vaccine preparation of the invention comprises attenuated chimeric viruses that are viable and infectious. In a preferred embodiment, the recombinant PIV is a bovine PIV type 3, or an attenuated strain of human PIV.

In one embodiment, the vaccine preparation comprises the chimeric virus of the present invention, wherein the F, HN, or some other nonessential genes of the PIV genome have been substituted or deleted. In a preferred embodiment, the vaccine preparation of the present invention is prepared by engineering a strain of PIV with an attenuated phenotype in an intended host. In another preferred embodiment, the vaccine preparation of the present invention is prepared by engineering an attenuated strain of PIV.

In another embodiment, the heterologous nucleotide sequence is added to the complete PIV genome. In certain embodiments, the PIV genome is engineered so that the heterologous sequences are inserted at position one, two, three, four, five or six, so that the heterologous sequences are expressed as the first, second, third, fourth, fifth, or sixth gene of the viral genome. In specific embodiments, the heterologous sequence is inserted at position one, two, or three of the viral genome. In certain embodiments, the intergenic region between the end of the coding sequence of an inserted heterologous gene and the start of the coding sequence of the downstream gene is altered to a desirable length, resulting in enhanced expression of the heterologous sequence or enhanced growth of the chimeric virus. Alternatively, the intergenic region is altered to a desirable length, with a potential to alter the expression of the heterologous sequence or growth of the recombinant or chimeric virus, *e.g.*, attenuated phenotype. In some embodiments, both the position of the insertion and the length of the intergenic region flanking a heterologous nucleotide sequence are engineered to select a recombinant or chimeric virus with desirable levels of expression of the heterologous sequence and desirable viral growth characteristics.

In certain embodiments, the invention provides a vaccine formulation comprising the recombinant or chimeric virus of the invention and a pharmaceutically acceptable excipient. In specific embodiments, the vaccine formulation of the invention is used to modulate the immune response of a subject, such as a human, a primate, a horse, a cow, a sheep, a pig, a goat, a dog, a cat, a rodent or a subject of avian species. In a more specific embodiment, the vaccine is used to modulate the immune response of a human infant or a child. In another embodiment, the present invention relates to vaccine formulations for veterinary uses. The vaccine preparation of the invention can be administered alone or in combination with other vaccines or other prophylactic or therapeutic agents.

### 3.1. CONVENTIONS AND ABBREVIATIONS

- cDNA: complementary DNA
- CPE: cytopathic effects
- L: large protein
- M: matrix protein (lines inside of envelope)
- F: fusion glycoprotein
- HN: hemagglutinin-neuraminidase glycoprotein
- N, NP or NC: nucleoprotein (associated with RNA and required for polymerase activity)
- P: phosphoprotein
- MOI: multiplicity of infection
- NA: neuraminidase (envelope glycoprotein)
- PIV: parainfluenza virus
- bPIV: bovine parainfluenza virus
- bPIV3: bovine parainfluenza virus type 3
- hPIV: human parainfluenza virus
- hPIV3: human parainfluenza virus type 3
- bPIV/hPIV or b/h PIV: recombinant bPIV with hPIV sequences
- b/h PIV3 or bPIV3/hPIV3: recombinant bPIV type 3 with hPIV type 3 sequences
- nt: nucleotide
- RNP: ribonucleoprotein
- rRNP: recombinant RNP
- vRNA: genomic virus RNA
- cRNA: antigenomic virus RNA
- hMPV: human metapneumovirus
- APV: avian pneumovirus
- position: when position is used regarding engineering any virus, it refers to the position of the gene of the viral genome to be transcribed. For example, if a gene is located at position one, it is the first gene of the viral genome to be transcribe; if a gene is located at position two, it is the second gene of the viral genome to be transcribed.
- position 1 of bPIV3,:
- b/h PIV3 and derivatives thereof: nucleotide position 104 of the genome, or alternatively, the position of the first gene of the viral genome to be transcribed
- position 2 of bPIV3,:
- b/h PIV3 and derivatives thereof: nucleotide position 1774 of the genome, or alternatively the position between the first and the second open reading frame of the native parainfluenza virus, or alternatively, the position of the second gene of the viral genome to be transcribed
- position 3 of bPIV3,:
- b/h PIV3 and derivatives thereof: nucleotide position 3724 of the genome, or alternatively the position between the second and the third open reading frame of the native parainfluenza virus, or alternatively, the position of the third gene of the viral genome to be transcribed.
- position 4 of bPIV3,:
- b/h PIV3 and derivatives thereof: nucleotide position 5042 of the genome, or alternatively the position between the third and the fourth open reading frame of the native parainfluenza virus, or alternatively, the position of the fourth gene of the viral genome to be transcribed.
- position 5 of bPIV3,:
- b/h PIV3 and derivatives thereof: nucleotide position 6790 of the genome, or alternatively the position between the fourth and the fifth open reading frame of the native parainfluenza virus, or alternatively, the position of the fifth gene of the viral genome to be transcribed.
- position 6 of PIV3,:
- b/h PIV3 and derivatives thereof: nucleotide position 8631 of the genome, or alternatively the position between the fifth and the sixth open reading frame of the native parainfluenza virus, or alternatively, the position of the sixth gene of the viral genome to be transcribed.

### 4. DESCRIPTION OF FIGURES

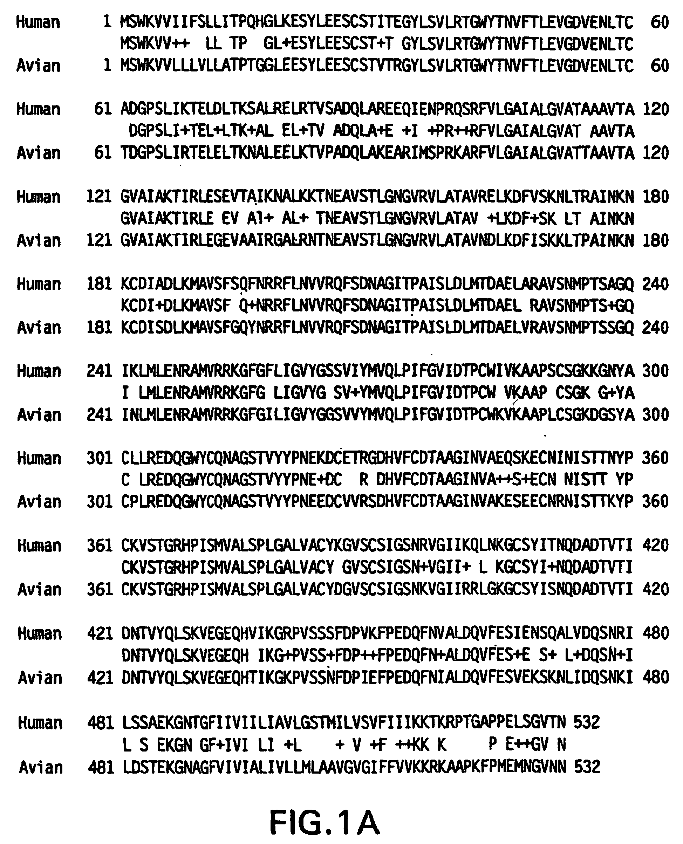

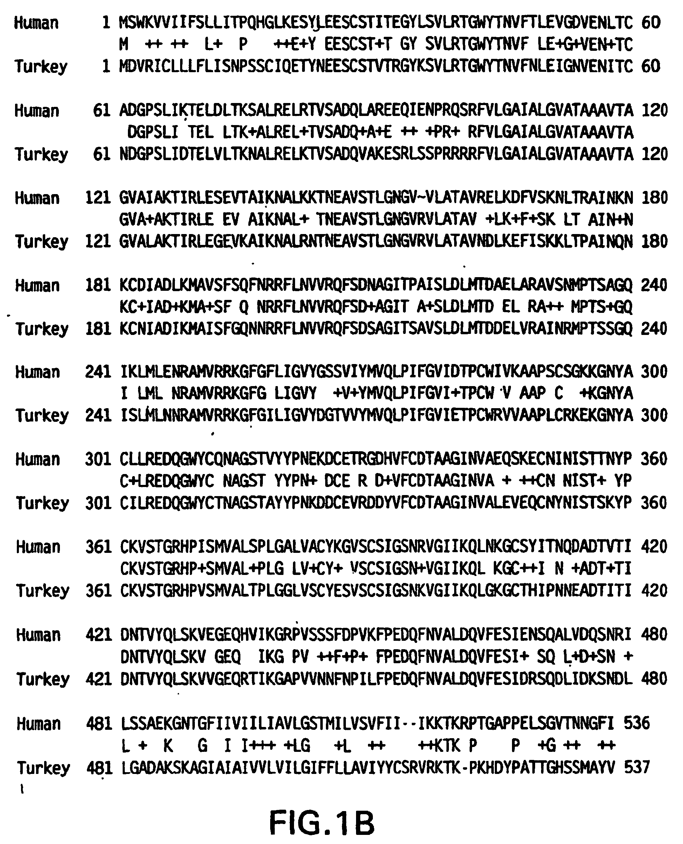

Figure 1. Pairwise alignments of the amino acid sequence of the F protein of the human metapneumovirus with different F proteins from different avian pneumoviruses. Identical amino acids between the two sequences are indicated by the one-letter-symbol for the amino acid. Conserved amino acid exchanges between the two amino acid sequences are indicated by a "+" sign, and a space indicates a non-conserved amino acid exchange. A) Alignment of the human metapneumoviral F protein with the F protein of an avian pneumovirus isolated from Mallard Duck (85.6% identity in the ectodomain). B) Alignment of the human metapneumoviral F protein with the F protein of an avian pneumovirus isolated from Turkey (subgroup B; 75% identity in the ectodomain).

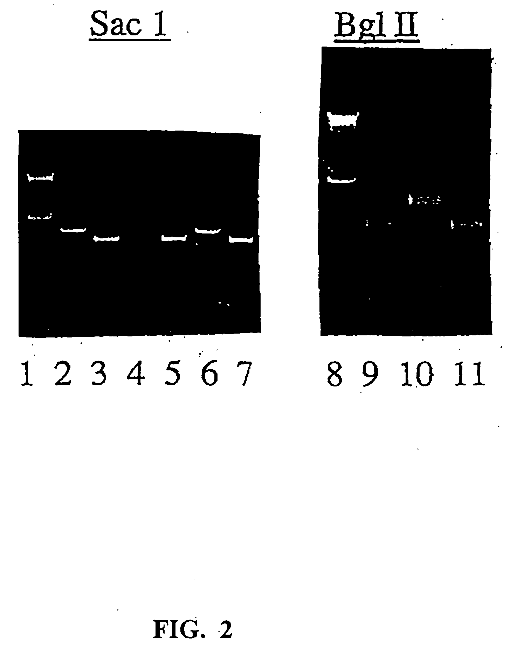

Figure 2. PCR fragments from nt 5255 to nt 6255 derived from three different isolates of the b/h PIV3 chimeric virus were amplified. The resulting 1 kb DNA fragments were digested with enzymes specific for the F gene of human PIV3. These enzymes do not cut in the corresponding fragment of bovine PIV3. The 1% agarose gel shows the undigested fragment (lanes 2,5, and 6) and the Sacl or BgIII digested fragments (lanes 4, 6 and lanes 9, 10, and 11, respectively). The sample in lane 10 is undigested, however, upon a repeat of digestion with BgIII, this sample was cut (data not shown). Lanes 1 and 8 show a DNA size marker.

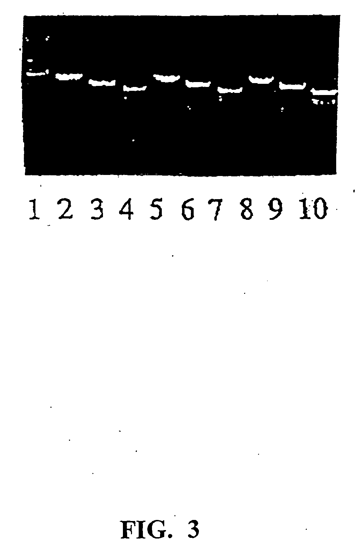

Figure 3. PCR fragments from nt 9075 to nt 10469 derived from three different isolates of the b/h PIV3 chimeric virus were amplified. The resulting 1.4kb DNA fragments were digested with enzymes specific for the L gene of bovine PIV3. These enzymes do not cut in the corresponding fragment of human PIV3. The 1% agarose gel shows the undigested 1.4 kb fragment (lanes 2, 5, and 8). The smaller DNA fragments produced by digestion with BamHl and PvuII are shown in lanes 3, 4, 6, 7, 9, and 10). Lane 1 shows a DNA size marker.

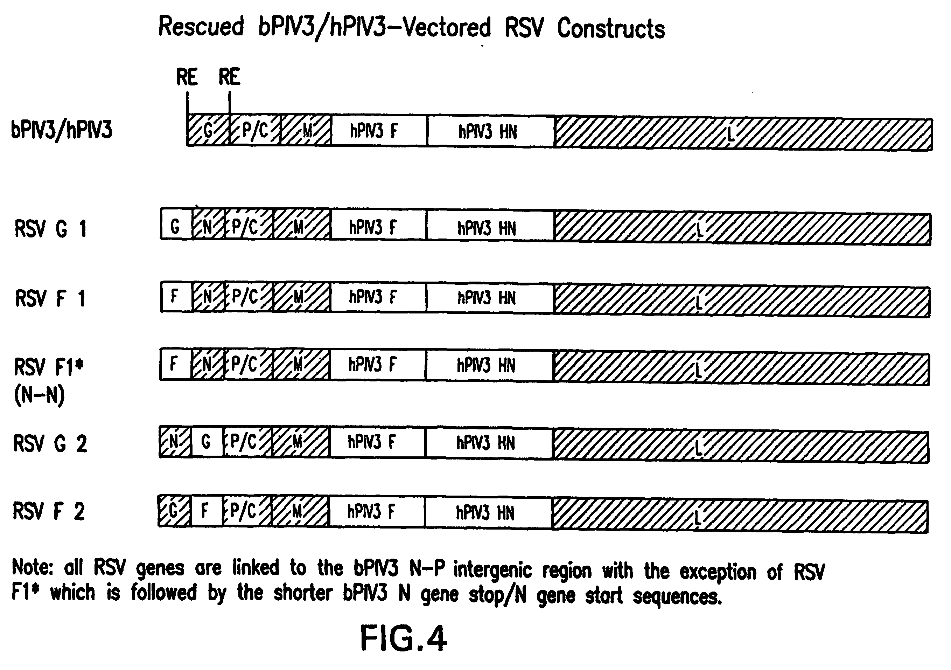

Figure 4. Six constructs, including the bPIV3/hPIV3 vector and b/h PIV3 vectored RSV F or G cDNA, are demonstrated. The bovine PIV3 F gene and HN gene are deleted and replaced with human PIV3 F and HN gene respectively. The RSV F or G genes are cloned into either position 1 or position 2. All RSV genes are linked to the bPIV3 N-P intergenic region with the exception of RSV F1* (N-N), which is followed by the shorter bPIV3 N gene stop/N gene start sequences.

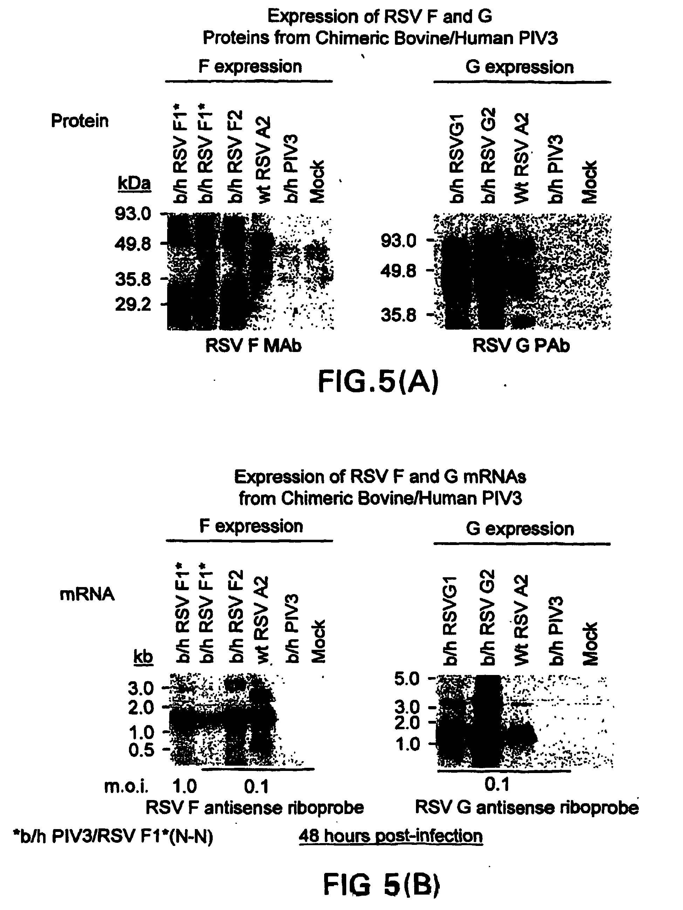

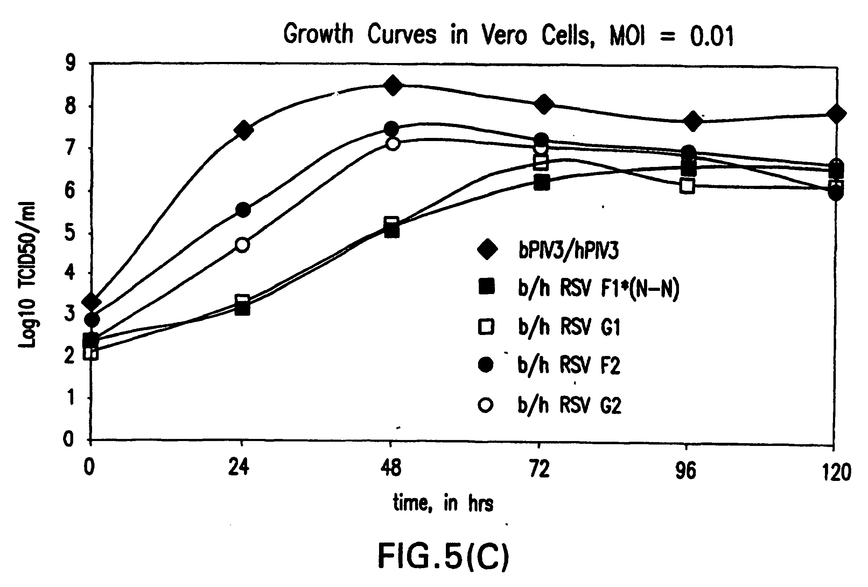

Figure 5. b/h PIV3 vectored RSV F or G gene displayed a positional effect. (A) is a Western blot analysis of chimeric virus-infected cell lysates. F protein was detected using monoclonal antibodies (MAbs) against the RSV F protein, and G protein was detected using polyclonal antibodies (PAbs) against the RSV G protein. A 50 kDa band representing the F₁ fragment was detected in cells infected with all chimeric viruses as well as wild-type RSV. There was a greater accumulation of a 20 kDa F fragment in infected cell lysates of chimeric viruses compared to wild-type RSV. The experiment was done at MOI of 0.1, except that in lane 1, b/h PIV3 vectored RSV F1* N-N infections were repeated at a higher MOI of 1.0. Both the immature and glycosylated forms of RSV G protein that migrated at approximately 50 kDa and 90 kDa were detected. (B) is a Northern blot analysis, which showed that the mRNA transcription correlated with the result of the protein expression demonstrated in Figure 5A. Equal amounts of total RNA were separated on 1% agarose gels containing 1% formaldehyde and transferred to nylon membranes. The blots were hybridized with digoxigenin (DIG)-UTP-labeled riboprobes synthesized by in vitro transcription using a DIG RNA labeling kit. (C) is growth curves of chimeric viruses comprising b/h PIV3 vectored RSV F or G protein in Vero cells. Vero cells were grown to 90% confluence and infected at an MOI of 0.01. The infected monolayers were incubated at 37°C. Virus titers for each time point harvest were determined by TCID₅₀ assays, which were performed by inspecting visually for CPE following incubation at 37°C for 6 days.

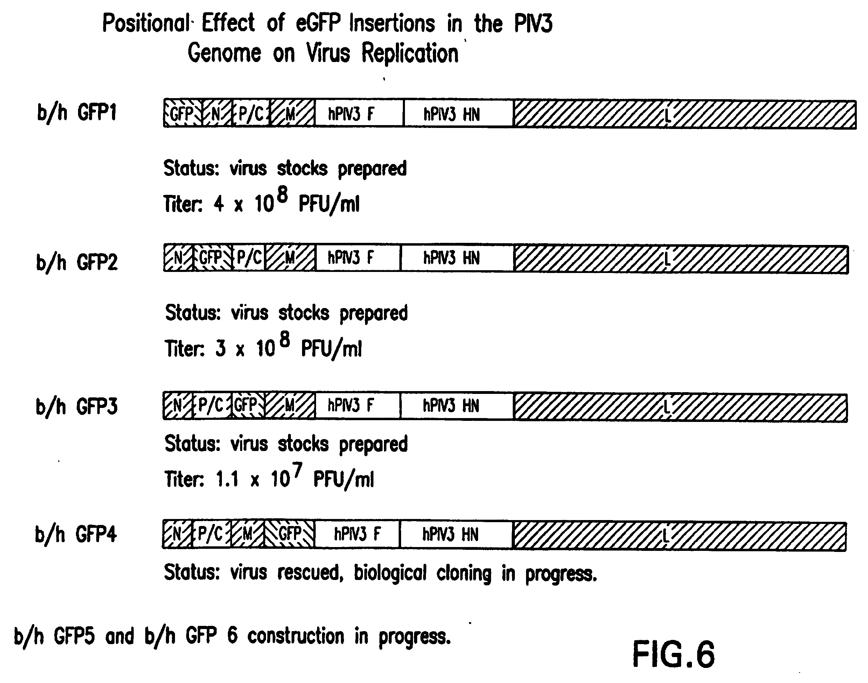

Figure 6. The b/h PIV3 vectored enhanced green fluorescence protein (eGFP) constructs. The eGFP gene is introduced into the b/h PIV3 vector sequentially between all genes of PIV3 (only position 1, 2, 3, and 4 are shown here). The eGFP gene was linked to the bPIV3 N-P intergenic region. The b/h GFP 1 construct harbors the eGFP gene cassette in the 3' most proximal position of the b/h PIV3 genome. The b/h GFP 2 construct contains the eGFP gene cassette between the N and P genes. The b/h GFP 3 construct contains the eGFP gene cassette between the P and M gene, and the b/h GFP4 construct contains the eGFP gene between M and F of b/h PIV3.

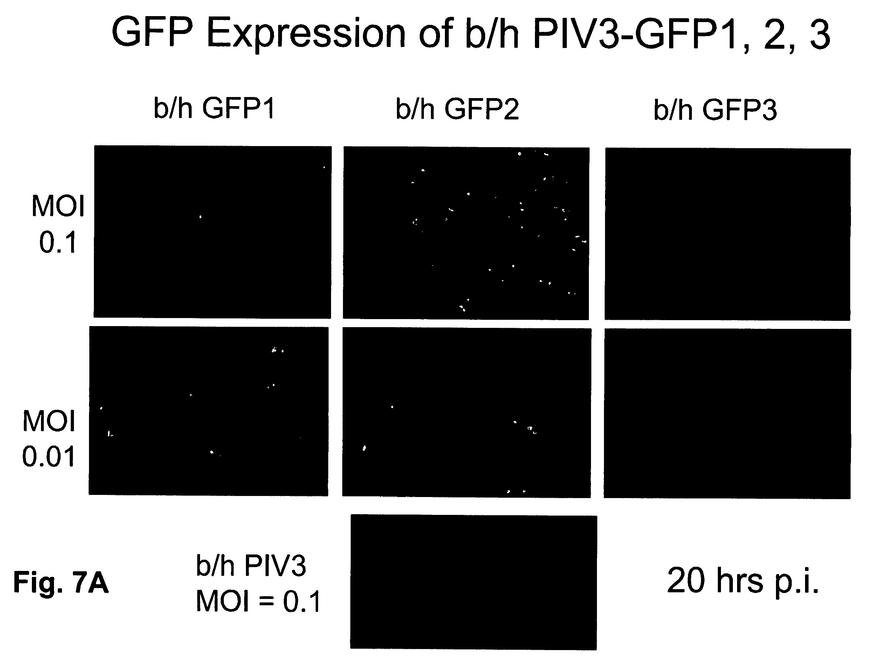

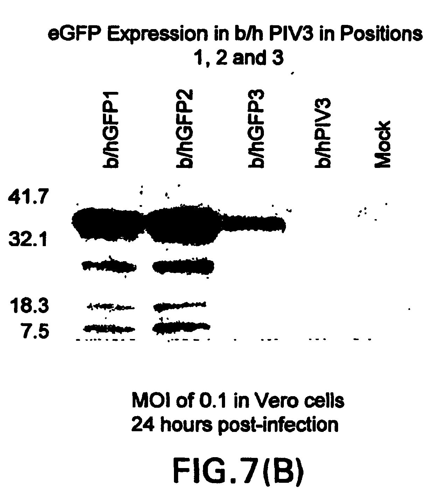

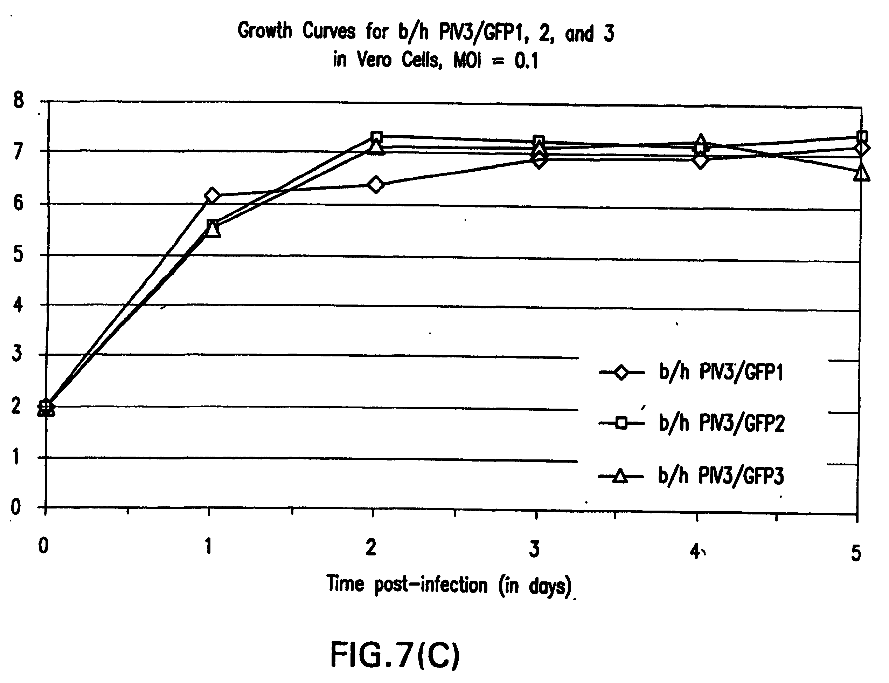

Figure 7. Positional effect of enhanced green fluorescence protein (eGFP) insertions in the b/h PIV3 genome. (A) shows the amount of green cells produced upon infecting Vero cells with b/h PIV3 vectored eGFP 1,2, and 3 at MOI 0.1 and MOI 0.01 for 20 hours. The green cells were visualized by using a fluorescent microscope. (B) is a Western blot analysis of infected cell lysates. The blots were probed with a GFP MAb as well as a PIV3 PAb. PIV3 antibody was also used to show that the blots had same volume loading. (C) is growth curves of b/h PIV3 vectored GFP constructs (at position 1, 2, and 3) in Vero cells.

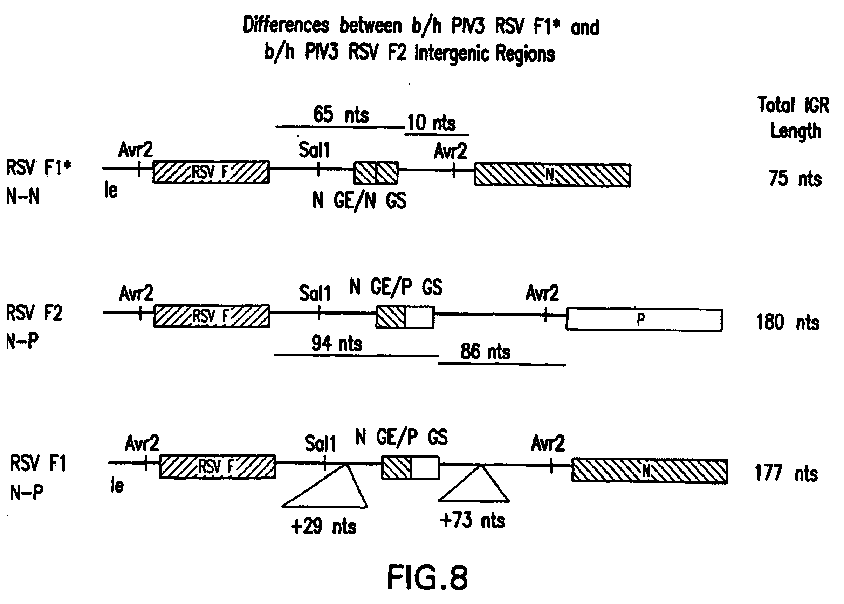

Figure 8. Constructs of b/h PIV3 vectored RSV F gene with different intergenic regions. The three constructs, RSV F1* N-N, RSV F2 N-P, and RSV F1 N-P are the same as the RSV F* (N-N), RSV F2, and RSV F1 in Figure 4 respectively. The distance between the N gene start sequence and the N gene translation start codon in RSV F1* N-N is only 10 nucleotides (nts) long. In contrast, this distance is 86 nts long in RSV F2 construct. RSV F1* N-N also uses the N gene start sequence rather than the P gene start sequence as is done in RSV F2 construct.

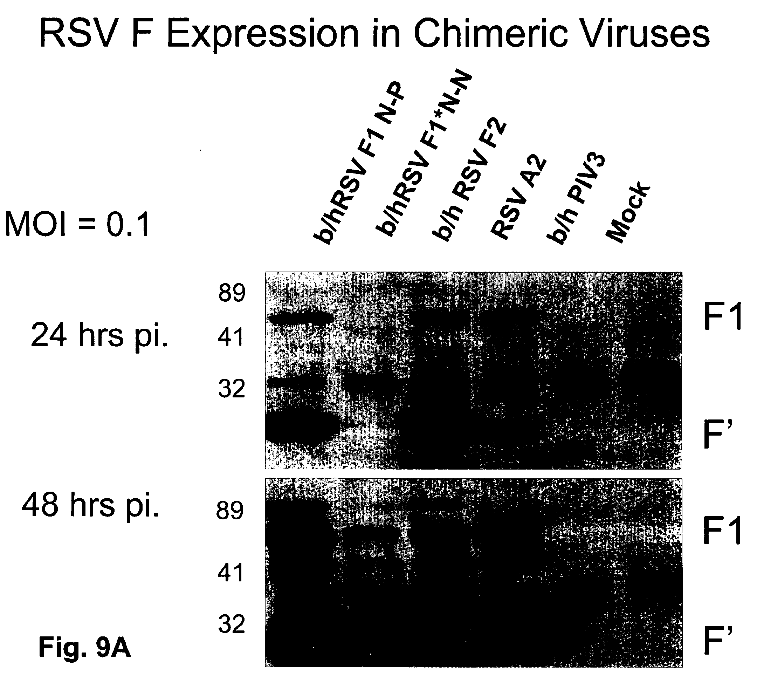

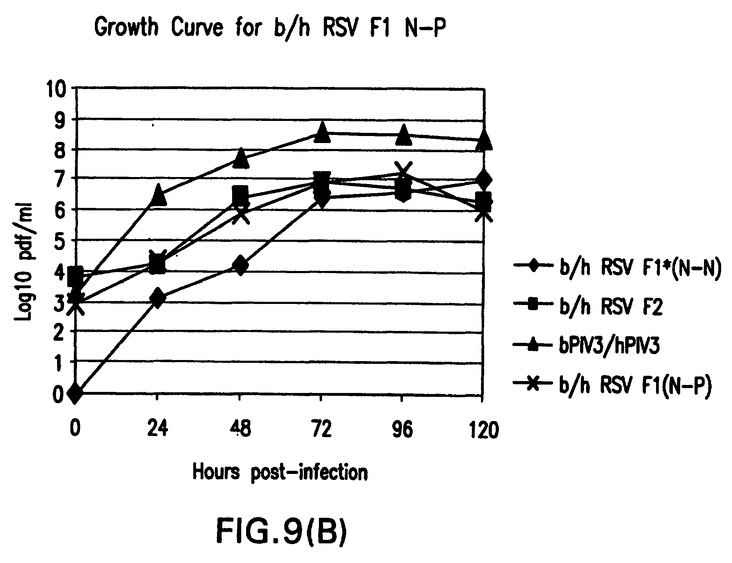

Figure 9. The length and/or nature of the intergenic region downstream of the inserted RSV gene has an effect on virus replication. (A) Western blot analysis of RSV F protein expression in chimeric viruses. Blots were probed with monoclonal antibodies against the RSV F protein. F1 protein levels expressed by RSV F1 construct and measured at 24 and 48 hours post-infection were close to the levels observed for RSV F2 construct, but much higher than those of RSV F1* N-N construct. (B) is multicycle growth curves comparing the kinetics of virus replication of RSV F1, RSV F1*N-N and RSV F2 constructs in Vero cells at an MOI of 0.1. Virus titers for each time point harvest were determined by plaque assays, which were performed by immunostaining with RSV polyclonal antisera for quantification after 5 days of incubation.

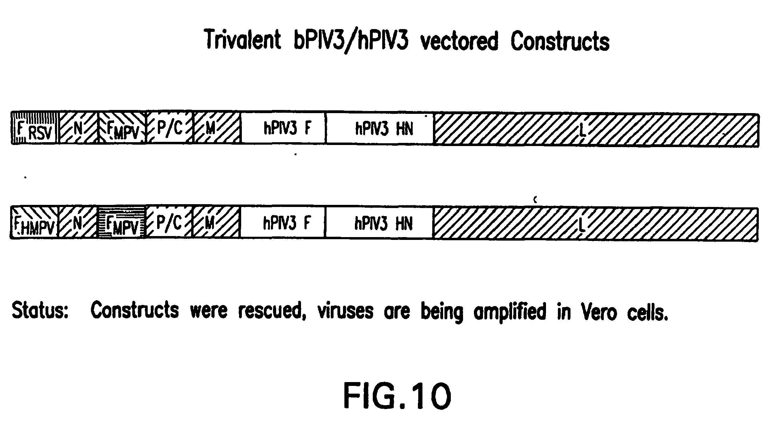

Figure 10. Constructs of trivalent b/h PIV3 vectored RSV F and hMPV F. Two virus genomes, each comprising a chimeric b/h PIV3 vector and a first heterologous sequences derived from a metapneumovirus F gene and a second heterologous sequence derived from respiratory syncytial virus F gene, are shown here. Virus with either of the constructs has been amplified in Vero cells. The engineered virus as described can be used as a trivalent vaccine against the parainfluenza virus infection, metapneumovirus infection and the respiratory syncytial virus infection.

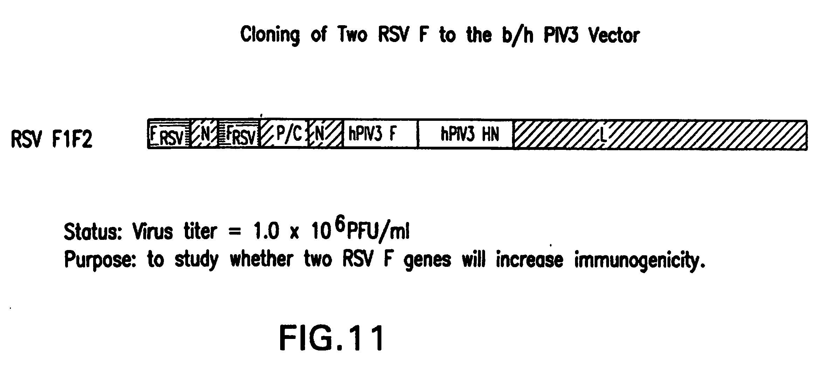

Figure 11. A construct harboring two RSV F genes. This construct can be used to determine virus growth kinetics, for RSV F protein production, and replication and immunogenicity in hamsters.

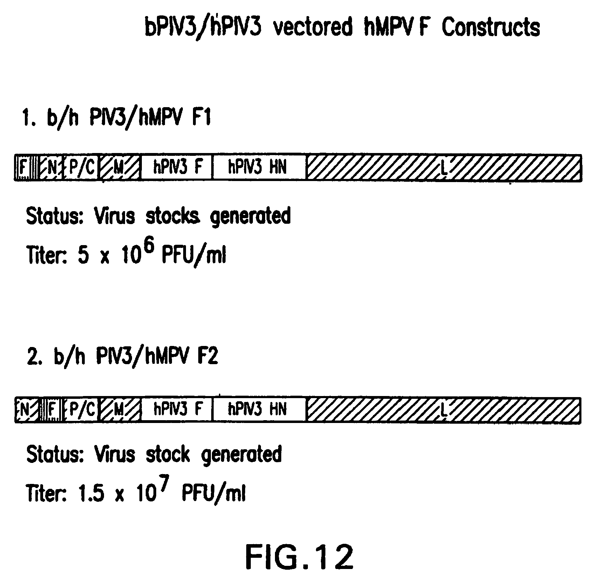

Figure 12. The chimeric b/h PIV3 vectored hMPV F constructs. The F gene of human metapneumovirus (hMPV) was inserted in position 1 or position 2 of the b/h PIV3 genome. The hMPV F gene cassette harbored the bPIV3 N-P intergenic region.

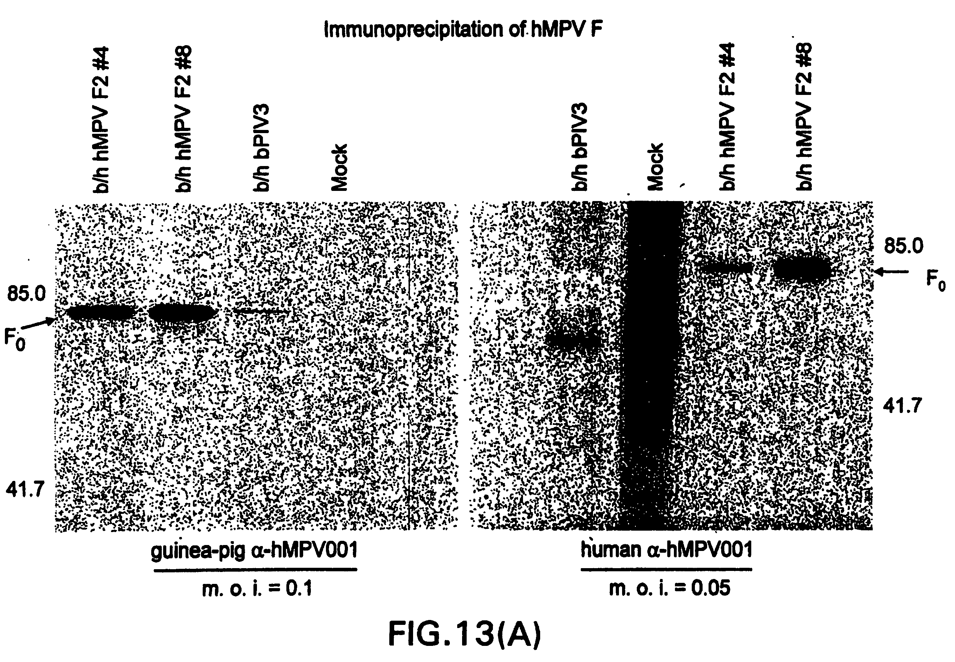

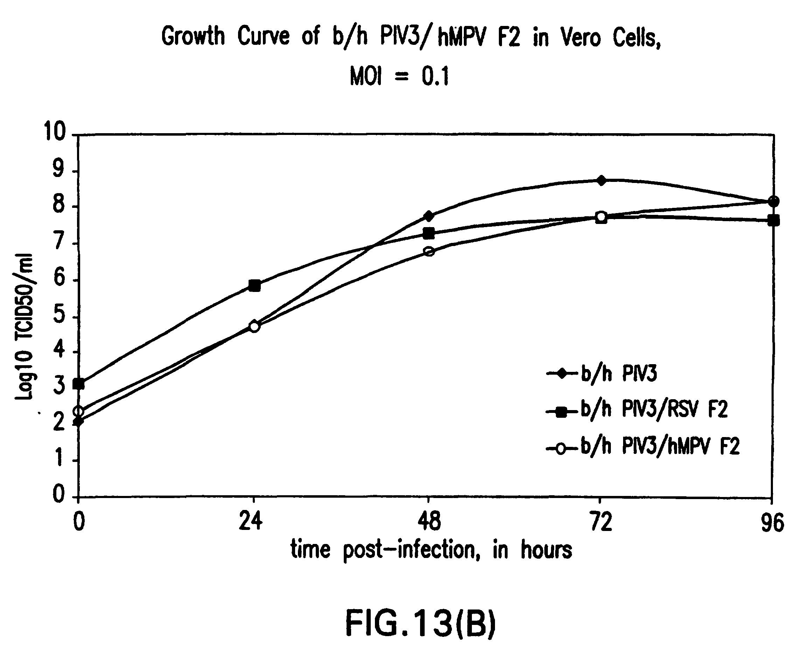

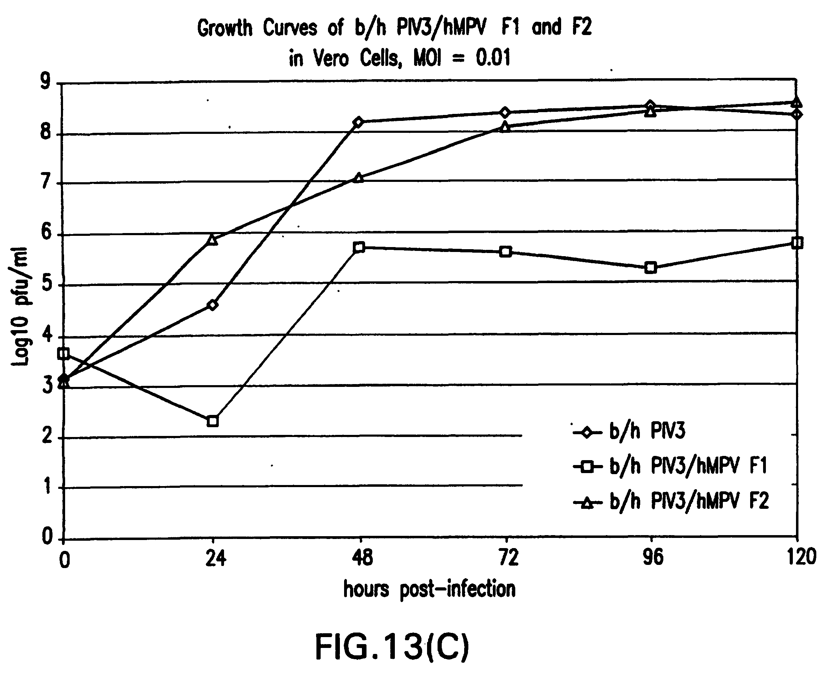

Figure 13. Immunoprecipitation and replication assays of b/h PIV3 vectored hMPV F gene (at position 2). (A) shows the immunoprecipitation of hMPV F protein using guinea pig or human anti-hMPV antiserum. A specific band migrating at approximately 80 kDa was observed in the lysates of b/h PIV3 vectored hMPV F2. This size corresponds to the F precursor protein, Fₒ. Non-specific bands of different sizes were also observed in the b/h PIV3 and mock control lanes. (B) shows growth curves that were performed to determine the kinetics of virus replication of b/h PIV3/hMPV F2 and compare it to those observed for b/h PIV3 and b/h PIV3/RSV F2 in Vero cells at an MOI of 0.1. (C) is growth curves that were performed to determine the kinetics of virus replication of b/h PIV3/hMPV F1 and compare it to those observed for b/h PIV3/hMPV F2 and b/h PIV3 in Vero cells at an MOI of 0.01.

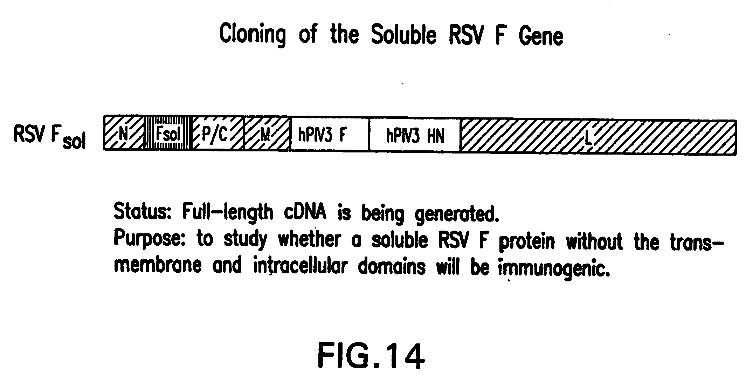

Figure 14. A chimeric b/h PIV3 vectored soluble RSV F gene construct. This construct comprises a single copy of the soluble RSV F gene, a version of the RSV F gene lacking the transmembrane and cytosolic domains. The advantage of this construct would be the inability of the soluble RSV F to be incorporated into the virion genome.

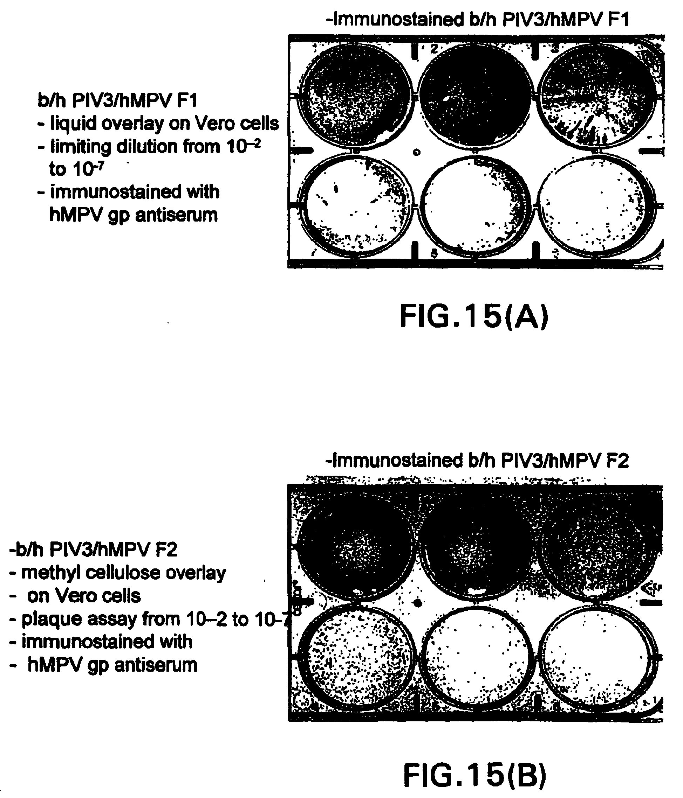

Figure 15. Immunostained b/h PIV3/hMPV F1 and b/h PIV3/hMPV F2. (A) the b/h PIV3/hMPV F1 virus were diluted and used to infect subconfluent Vero cells. Infected cells were overlayed with optiMEM media containing gentamycin and incubated at 35°C for 5 days. Cells were fixed and immunostained with guinea pig anti-hMPV sera. Expression of hMPV F is visualized by specific color development in the presence of the AEC substrate system. (B) the b/h PIV3/hMPV F2 virus were diluted and used to infect Vero cells. Infected cells were overlayed with 1% methyl cellulose in EMEM/L-15 medium (JRH Biosciences; Lenexa, KS). Cells were incubated, fixed and then immunostained with anti-hMPV guinea pig sera. The anti-hMPV guinea pig serum is specific for hMPV 001 protein.

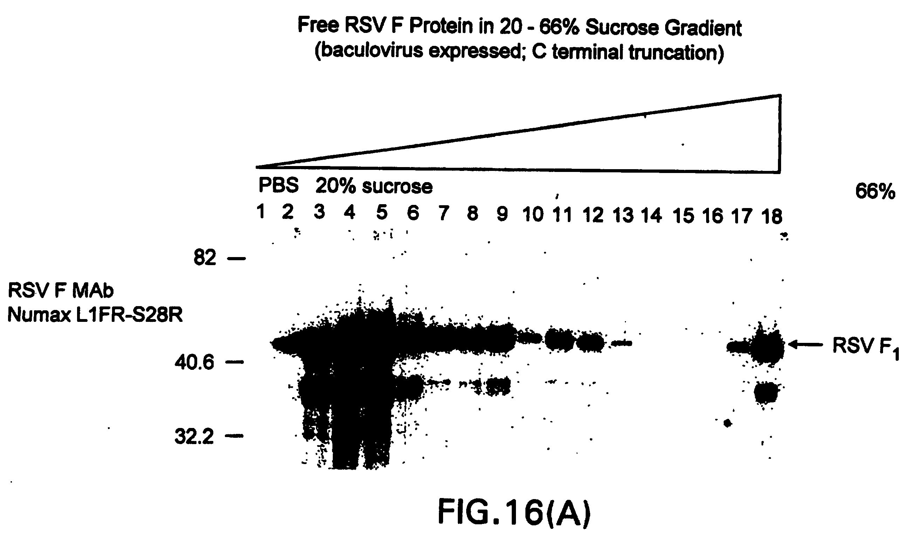

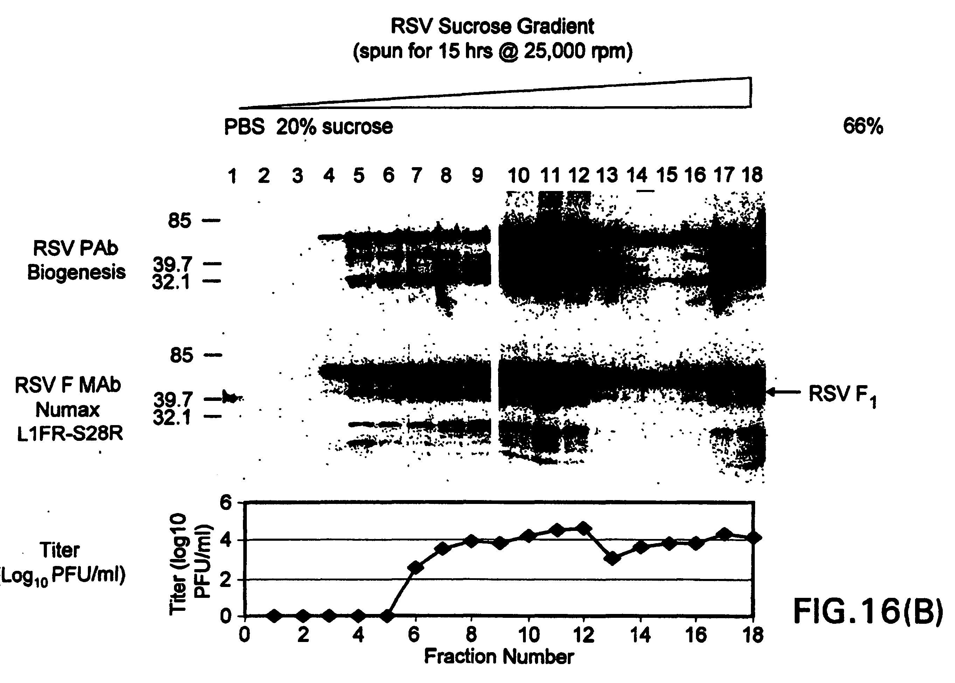

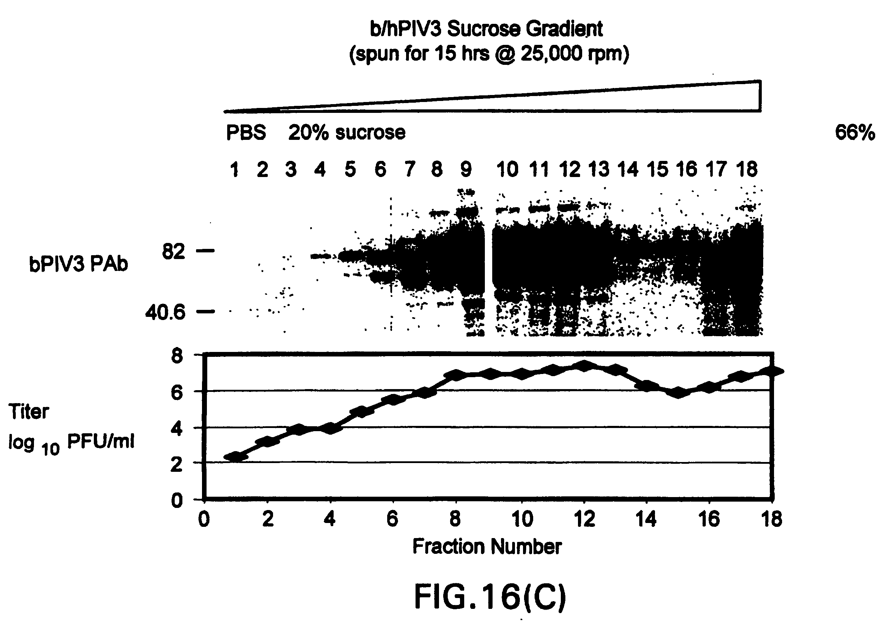

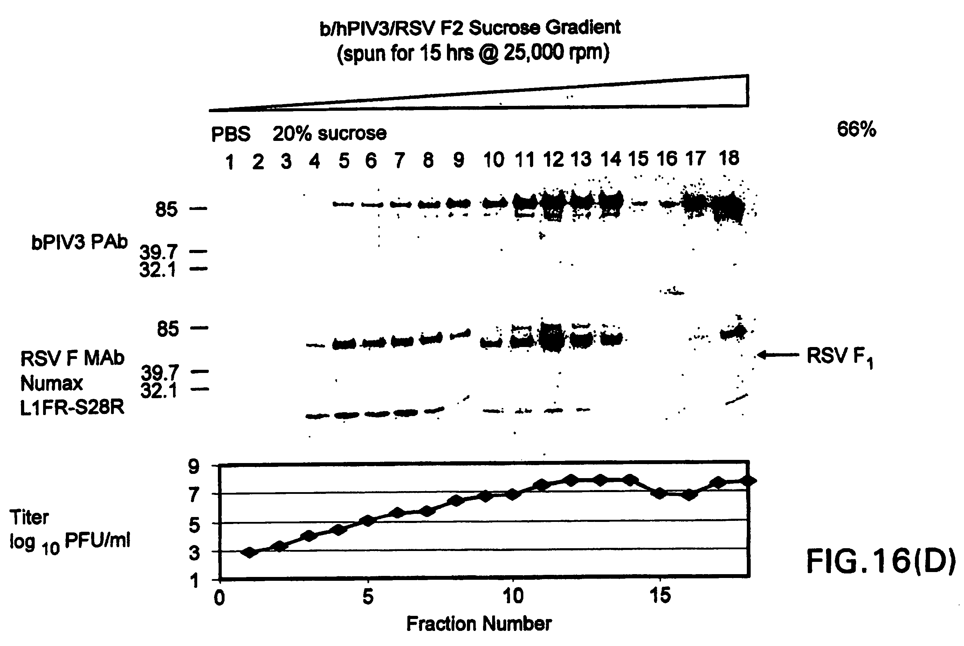

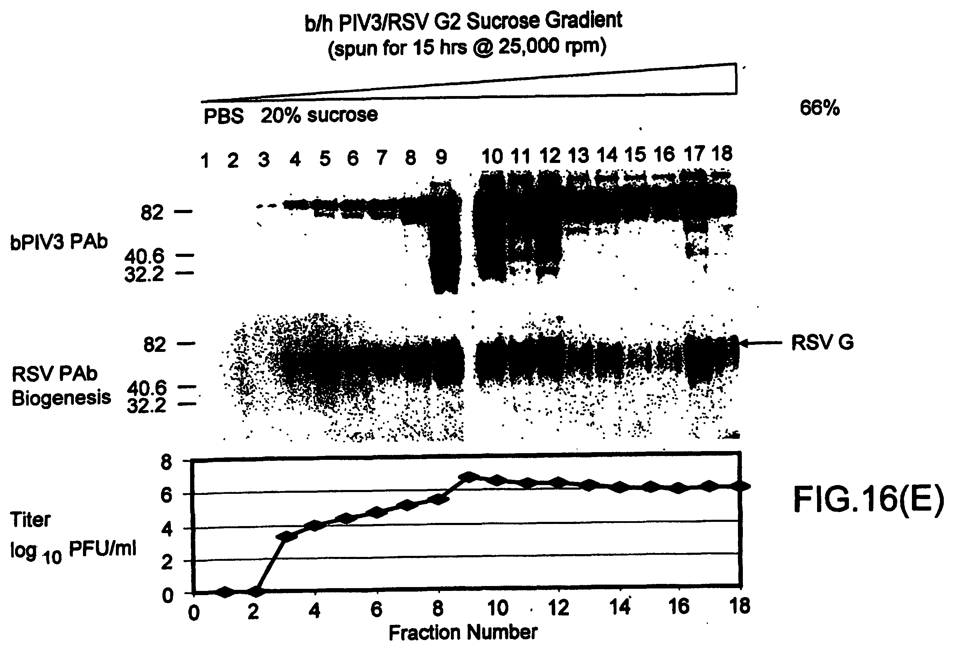

Figure 16. Virion fractionation of b/h PIV3 vectored RSV genes on sucrose gradients. These series experiments investigate whether the RSV proteins were incorporated into the b/h PIV3 virion. (A) shows control gradient of free RSV F (generated in baculovirus and C-terminally truncated). Majority of free RSV F was present in fractions 3, 4, 5, and 6. (B) shows that the biggest concentration of RSV virions was observed in fractions 10, 11 and 12. The RSV fractions were probed with RSV polyclonal antiserum as well as RSV F MAb. The fractions that contained the greatest amounts of RSV virions also showed the strongest signal for RSV F, suggesting that the RSV F protein co-migrated and associated with RSV virion. The last figure on (B) also shows that the fractions 10, 11 and 12 displayed the highest virus titer by plaque assay. (C) The b/h PIV3 virions may be more pleiomorphic and thus the spread of the peak fractions containing b/h PIV3 virions was more broad. (D) Sucrose gradient fractions of b/h PIV3/RSV F2 were analyzed with both a PIV polyclonal antiserum and an RSV F MAb. The fractions containing most of the virions were fractions 11, 12, 13 and 14, as shown by Western using the PIV3 antiserum. Correspondingly, these were also the fractions that displayed the highest amounts of RSV F protein. Some free RSV F was also present in fractions 5 and 6. Fractions 11, 12, 13 and 14 displayed the peak virus titers. (E) The fractions containing the most virions of b/h PIV3/RSV G2 (9, 10, 11 and 12) also showed the strongest signal for RSV G protein. Again, these were the fractions with the highest virus titers.

### 5. DESCRIPTION OF THE INVENTION

The present invention relates to recombinant parainfluenza cDNA and RNA constructs, including but not limited to, recombinant bovine and human PIV cDNA and RNA constructs, that may be used to express heterologous or non-native sequences.

In accordance with the present invention, a recombinant virus is one derived from a bovine parainfluenza virus or a human parainfluenza virus that is encoded by endogenous or native genomic sequences or non-native genomic sequences. In accordance with the invention, a non-native sequence is one that is different from the native or endogenous genomic sequence due to one or more mutations, including, but not limited to, point mutations, rearrangements, insertions, deletions, etc. to the genomic sequence that may or may not result in a phenotypic change.

In accordance with the present invention, a chimeric virus of the invention is a recombinant bPIV or hPIV which further comprises one or more heterologous nucleotide sequences. In accordance with the invention, a chimeric virus may be encoded by a nucleotide sequence in which heterologous nucleotide sequences have been added to the genome or in which nucleotide sequences have been replaced with heterologous nucleotide sequences. These recombinant and chimeric viruses and expression products may be used as vaccines suitable for administration to humans or animals. For example, the chimeric viruses of the invention may be used in vaccine formulations to confer protection against pneumovirus, respiratory syncytial virus, parainfluenza virus, or influenza virus infection.

In one embodiment, the invention relates to PIV cDNA and RNA constructs that are derived from human or bovine PIV variants and are engineered to express one, two, or three heterologous sequences, preferably heterologous genes encoding foreign antigens and other products from a variety of pathogens, cellular genes, tumor antigens, and viruses. In particular, the heterologous sequences are derived from morbillivrus or a negative strand RNA virus, including but not limited to, influenza virus, respiratory syncytial virus (RSV), mammalian metapneumovirus (*e.g.*, human metapneumovirus variants A1, A2, B1, and B2), and avian pneumovirus subgroups A, B, C and D. The mammalian MPVs can be a variant A1, A2, B1 or B2 mammalian MPV. However, the mammalian MPVs of the present invention may encompass additional variants of MPV yet to be identified, and are not limited to variants A1, A2, B1, or B2. In another embodiment of the invention, the heterologous sequences are non-native PIV sequences, including mutated PN sequences. In some embodiments, the heterologous sequences are derived from the same or from different viruses.

In a specific embodiment, the virus of the invention is a recombinant PIV comprising heterologous nucleotide sequences derived from human metapneumovirus or avian pneumovirus. The heterologous sequences to be inserted into the PIV genome include, but are not limited to, the sequences encoding the F, G and HN genes of human metapneumovirus variants A1, A2, B1 or B2, sequences encoding the F, G and HN genes of avian pneumovirus type A, B, C or D, and immunogenic and/or antigenic fragments thereof.

In certain embodiments, the heterologous nucleotide sequence is added to the viral genome. In alternative embodiments, the heterologous nucleotide sequence is exchanged for an endogenous nucleotide sequence. The heterologous nucleotide sequence may be added or inserted at various positions of the PIV genome, *e.g.*, at position 1, 2, 3, 4, 5, or 6. In a preferred embodiment, the heterologous nucleotide sequence is added or inserted at position 1. In another preferred embodiment, the heterologous nucleotide sequence is added or inserted at position 2. In even another preferred embodiment, the heterologous nucleotide sequence is added or inserted at position 3. Inserting or adding heterologous nucleotide sequences at the lower-numbered positions of the virus generally results in stronger expression of the heterologous nucleotide sequence compared to insertion at higher-numbered positions. This is due to a transcriptional gradient that occurs across the genome of the virus. However, virus replication efficiency must also be considered. For example, in the b/h PIV3 chimeric virus of the invention, insertion of a heterologous gene at position 1 delays replication kinetics *in vitro* and to a lesser degree also *in vivo (see* section 8, example 3 and Figure 5 as well as section 26, example 21). Therefore, inserting heterologous nucleotide sequences at lower-numbered positions is the preferred embodiment of the invention if strong expression of the heterologous nucleotide sequence is desired. Most preferably, a heterologous sequence is inserted at position 2 of a b/h PIV3 genome if strong expression of the heterologous sequence is desired. *(See* section 5.1.2. *infra* and section 8, example 3).

In some other embodiments, the recombinant or chimeric PIV genome is engineered such that the intergenic region between the end of the coding sequence of the heterologous gene and the start of the coding sequence of the downstream gene is altered. In yet some other embodiments, the virus of the invention comprises a recombinant or chimeric PIV genome engineered such that the heterologous nucleotide sequence is inserted at a position selected from the group consisting of positions 1, 2, 3, 4, 5, and 6, and the intergenic region between the heterologous nucleotide sequence and the next downstream gene is altered. Appropriate assays may be used to determine the best mode of insertion (*i.e.*, which position to insert, and the length of the intergenic region) to achieve appropriate levels of gene expression and viral growth characteristics. For detail, *see* Section 5.1.2., *infra.*

In certain embodiments, the chimeric virus of the invention contains two different heterologous nucleotide sequences. The different heterologous nucleotide sequences may be inserted at various positions of the PIV genome. In a preferred embodiment, one heterologous nucleotide sequence is inserted at position 1 and another heterologous nucleotide sequence is added or inserted at position 2 or 3. In other embodiments of the invention, additional heterologous nucleotide sequences are inserted at higher-numbered positions of the PIV genome. In accordance with the present invention, the position of the heterologous sequence refers to the order in which the sequences are transcribed from the viral genome, *e.g.*, a heterologous sequence at position 1 is the first gene sequence to be transcribed from the genome.

In certain embodiments of the invention, the heterologous nucleotide sequence to be inserted into the genome of the virus of the invention is derived from a negative strand RNA virus, including but not limited to, influenza virus, parainfluenza virus, respiratory syncytial virus, mammalian metapneumovirus, and avian pneumovirus. In a specific embodiment of the invention, the heterologous nucleotide sequence is derived from a human metapneumovirus. In another specific embodiment, the heterologous nucleotide sequence is derived from an avian pneumovirus. More specifically, the heterologous nucleotide sequence of the invention encodes a F, G or SH gene or a portion thereof of a human or avian metapneumovirus. In specific embodiments, a heterologous nucleotide sequences can be any one of SEQ ID NO:1 through SEQ ID NO:5, SEQ ID NO:14, and SEQ ID NO:15 (see Table 16). In certain specific embodiments, the nucleotide sequence encodes a protein of any one of SEQ ID NO:6 through SEQ ID NO:13, SEQ ID NO:16, and SEQ ID NO:17 (see Table 16). In certain specific embodiments, the nucleotide sequence encodes a protein of any one of SEQ ID NO: 314 through 389.

In specific embodiments of the invention, a heterologous nucleotide sequence of the invention is derived from a type A avian pneumovirus. In other specific embodiments of the invention, a heterologous nucleotide sequence of the invention is derived from a type B avian pneumovirus. In even other specific embodiments of the invention, a heterologous nucleotide sequence of the invention is derived from a type C avian pneumovirus. Phylogenetic analyses show that type A and type B are more closely related to each other than they are to type C (Seal, 2000, Animal Health Res. Rev. 1(1):67-72). Type A and type B are found in Europe whereas type C was first isolated in the U.S.

In another embodiment of the invention, the heterologous nucleotide sequence encodes a chimeric polypeptide, wherein the ectodomain contains antigenic sequences derived from a virus other than the strain of PIV from which the vector backbone is derived, and the trans membrane and luminal domains are derived from PIV sequences. The resulting chimeric virus would impart antigenicity of the negative strand RNA virus of choice and would have an attenuated phenotype.

In a specific embodiment of the invention, the heterologous nucleotide sequence encodes a chimeric F protein. Particularly, the ectodomain of the chimeric F protein is the ectodomain of a metapneumovirus, so that a human metapneumovirus or avian pneumovirus, and the transmembrane domain as well as the luminal domain are the transmembrane and luminal domains of a parainfluenza virus, such as a human or a bovine parainfluenza virus. While not bound by any theory, insertion of a chimeric F protein may further attenuate the virus in an intended host but retain the antigenicity of the F protein attributed by its ectodomain.

The chimeric viruses of the invention may be used in vaccine formulations to confer protection against various infections, including but not limited to, pneumovirus infection, respiratory syncytial virus infection, parainfluenza virus infection, influenza virus infection, or a combination thereof. The present invention provides vaccine preparations comprising chimeric PIV expressing one or more heterologous antigenic sequences, including bivalent and trivalent vaccines. The bivalent and trivalent vaccines of the invention may be administered in the form of one PIV vector expressing each heterologous antigenic sequences or two or more PIV vectors each encoding different heterologous antigenic sequences. Preferably, the heterologous antigenic sequences are derived from a negative strand RNA virus, including but not limited to, influenza virus, parainfluenza virus, respiratory syncytial virus (RSV), mammalian metapneumovirus (*e.g.*, human metapneumovirus) and avian pneumovirus. Thus, the chimeric virions of the present invention may be engineered to create, *e.g.*, anti-human influenza vaccine, anti-human parainfluenza vaccine, anti-human RSV vaccine, and anti-human metapneumovirus vaccine. Preferably, the vaccine preparation of the invention comprises attenuated chimeric viruses that are viable and infectious. The vaccine preparation of the invention can be administered alone or in combination with other vaccines or other prophylactic or therapeutic agents.

The present invention also relates to the use of viral vectors and chimeric viruses to formulate vaccines against a broad range of viruses and/or antigens including tumor antigens. The viral vectors and chimeric viruses of the present invention may be used to modulate a subject's immune system by stimulating a humoral immune response, a cellular immune response or by stimulating tolerance to an antigen. As used herein, a subject refers to a human, a primate, a horse, a cow, a sheep, a pig, a goat, a dog, a cat, a rodent and a member of avian species. When delivering tumor antigens, the invention may be used to treat subjects having disease amenable to immune response mediated rejection, such as non-solid tumors or solid tumors of small size. It is also contemplated that delivery of tumor antigens by the viral vectors and chimeric viruses described herein will be useful for treatment subsequent to removal of large solid tumors. The invention may also be used to treat subjects who are suspected of having cancer.

The invention may be divided into the following stages solely for the purpose of description and not by way of limitation: (a) construction of recombinant cDNA and RNA templates; (b) expression of heterologous gene products using recombinant cDNA and RNA templates; and ©) rescue of the heterologous genes in recombinant virus particles.

### 5.1. CONSTRUCTION OF THE RECOMBINANT cDNA AND RNA

The present invention encompasses recombinant or chimeric viruses encoded by viral vectors derived from the genomes of parainfluenza virus, including both bovine parainfluenza virus and mammalian parainfluenza virus. In accordance with the present invention, a recombinant virus is one derived from a bovine parainfluenza virus or a mammalian parainfluenza virus that is encoded by endogenous or native genomic sequences or non-native genomic sequences. In accordance with the invention, a non-native sequence is one that is different from the native or endogenous genomic sequence due to one or more mutations, including, but not limited to, point mutations, rearrangements, insertions, deletions etc. to the genomic sequence that may or may not result a phenotypic change. The recombinant viruses of the invention encompass those viruses encoded by viral vectors derived from the genomes of parainfluenza virus, including both bovine and mammalian parainfluenza virus, and may or may not, include nucleic acids that are non-native to the viral genome. In accordance with the present invention, a viral vector which is derived from the genome of a parainfluenza virus is one that contains a nucleic acid sequence that encodes at least a part of one ORF of a parainfluenza virus.

The present invention also encompasses recombinant viruses comprising a viral vector derived from a bovine and/or mammalian PIV genome which contains sequences which result in a virus having a phenotype more suitable for use in vaccine formulations, *e.g.*, attenuated phenotype or enhanced antigenicity. The mutations and modifications can be in coding regions, in intergenic regions and in the leader and trailer sequences of the virus.

In accordance with the present invention, the viral vectors of the invention are derived from the genome of a mammalian parainfluenza virus, in particular a human parainfluenza virus (hPIV). In particular embodiments of the invention, the viral vector is derived from the genome of a human parainfluenza virus type 3. In accordance with the present invention, these viral vectors may or may not include nucleic acids that are non-native to the viral genome.

In accordance with the present invention, the viral vectors of the inventions are derived from the genome of a bovine parainfluenza virus (bPIV). In particular embodiments of the invention, the viral vector is derived from the genome of bovine parainfluenza virus type 3. In accordance to the present invention, these viral vectors may or may include nucleic acids that are non-native to the viral genome.

In accordance with the invention, a chimeric virus is a recombinant bPIV or hPIV which further comprises a heterologous nucleotide sequence. In accordance with the invention, a chimeric virus may be encoded by a nucleotide sequence in which heterologous nucleotide sequence have been added to the genome or in which endogenous or native nucleotide sequence have been replaced with heterologous nucleotide sequence. In accordance with the invention, the chimeric viruses are encoded by the viral vectors of the invention which further comprise a heterologous nucleotide sequence. In accordance with the present invention, a chimeric virus is encoded by a viral vector that may or may not include nucleic acids that are non-native to the viral genome. In accordance with the invention, a chimeric virus is encoded by a viral vector to which heterologous nucleotide sequences have been added, inserted or substituted for native or non-native sequences.

A chimeric virus may be of particular use for the generation of recombinant vaccines protecting against two or more viruses (Tao et al., J. Virol. 72,2955-2961; Durbin et al., 2000, J.Virol. 74, 6821-6831; Skiadopoulos et al., 1998, J. Virol. 72, 1762-1768 (1998); Teng et al., 2000, J.Virol. 74, 9317-9321). For example, it can be envisaged that a hPN or bPIV virus vector expressing one or more proteins of another negative strand RNA virus, *e.g.*, MPV, or a RSV vector expressing one or more proteins of MPV will protect individuals vaccinated with such vector against both virus infections. A similar approach can be envisaged for other paramyxoviruses. Attenuated and replication-defective viruses may be of use for vaccination purposes with live vaccines as has been suggested for other viruses. (*See,* PCT WO 02/057302, at pp. 6 and 23, incorporated by reference herein).

In accordance with the present invention the heterologous to be incorporated into the viral vectors encoding the recombinant or chimeric viruses of the invention include sequences obtained or derived from different strains of metapneumovirus, strains of avian pneumovirus, and other negative strand RNA viruses, including, but not limited to, RSV, PIV, influenza virus and other viruses, including morbillivirus.

In certain embodiments of the invention, the chimeric or recombinant viruses of the invention are encoded by viral vectors derived from viral genomes wherein one or more sequences, intergenic regions, termini sequences, or portions or entire ORF have been substituted with a heterologous or non-native sequence. In certain embodiments of the invention, the chimeric viruses of the invention are encoded by viral vectors derived from viral genomes wherein one or more heterologous sequences have been added to the vector.

A specific embodiment of the present invention is a chimeric virus comprising a backbone encoded by nucleotide sequences derived from a parainfluenza virus genome. In a preferred embodiment, the PIV genome is derived from bovine PIV, such as the Kansas strain of bPIV3, or from human PIV. In a preferred embodiment, the PIV genome is derived from the Kansas strain of bPIV3, in which bovine parainfluenza virus nucleotide sequences have been substituted with heterologous sequences or in which heterologous sequences have been added to the complete bPIV genome. A further specific embodiment of the present invention is a chimeric virus comprising a backbone encoded by nucleotide sequences derived from human parainfluenza virus type 3 genome, in which human parainfluenza virus nucleotide sequences have been substituted with heterologous sequences or in which heterologous sequences have been added to the complete hPIV genome. An additional specific embodiment of the present invention is a chimeric virus comprising a backbone encoded by nucleotide sequences derived from bovine parainfluenza virus genome, such as the Kansas strain ofbPIV3, in which (a) the bovine parainfluenza virus F gene and HN gene have been substituted with the F gene and the HN gene of the human parainfluenza virus (bPIV/hPIV), and in which (b) heterologous sequences have been added to the complete bPIV genome.

The present invention also encompasses chimeric viruses comprising a backbone encoded by nucleotide sequences derived from the bPIV3, the hPIV3, or the bPIV/hPIV genome containing mutations or modifications, in addition to heterologous sequences, that result in a chimeric virus having a phenotype more suitable for use in vaccine formulations, *e.g.*, attenuated phenotype or enhanced antigenicity. In accordance with this particular embodiment of the invention, a heterologous sequence in the context of a bovine PIV3 backbone may be any sequence heterologous to bPIV3.

Another specific embodiment of the present invention is a chimeric virus comprising a backbone encoded by nucleotide sequences derived from human PIV 1, 2, or 3 in which hPIV nucleotide sequences have been substituted with heterologous sequences or in which heterologous sequences have been added to the complete hPIV genome, with the proviso that the resulting chimeric virus is not a chimeric hPIV3 in which the hemagglutinin-neuraminidase and fusion glycoproteins have been replaced by those of hPIV1. The present invention also encompasses chimeric viruses, comprising a backbone encoded by nucleotide sequences derived from a hPIV genome, containing mutations or modifications, in addition to heterologous sequences, that result in a chimeric virus having a phenotype more suitable for use in vaccine formulations, *e.g.*, attenuated phenotype or enhanced antigenicity.

Heterologous gene coding sequences flanked by the complement of the viral polymerase binding site/promoter, *e.g.*, the complement of 3'-PIV virus terminus of the present invention, or the complements of both the 3'- and 5'-PIV virus termini may be constructed using techniques known in the art. The resulting RNA templates may be of the negative-polarity and can contain appropriate terminal sequences that enable the viral RNA-synthesizing apparatus to recognize the template. Alternatively, positive-polarity RNA templates, that contain appropriate terminal sequences which enable the viral RNA-synthesizing apparatus to recognize the template, may also be used. Recombinant DNA molecules containing these hybrid sequences can be cloned and transcribed by a DNA-directed RNA polymerase, such as bacteriophage T7 polymerase, T3 polymerase, the SP6 polymerase or a eukaryotic polymerase such as polymerase I and the like, for the *in vitro* or *in vivo* production of recombinant RNA templates that possess the appropriate viral sequences and that allow for viral polymerase recognition and activity.

In one embodiment, the PIV vector of the invention expresses one, two, or three heterologous sequences, encoding antigenic polypeptides and peptides. In some embodiments, the heterologous sequences are derived from the same virus or from different viruses. In certain embodiments, more than one copy of the same heterologous nucleotide sequences are inserted in the genome of a bovine parainfluenza virus, human parainfluenza virus, or bPIV/hPIV chimeric vector. In a preferred embodiment, two copies of the same heterologous nucleotide sequences are inserted to the genome of the virus of the invention. In some embodiments, the heterologous nucleotide sequence is derived from a metapneumovirus, such as human metapneumovirus or an avian pneumovirus. In specific embodiments, the heterologous nucleotide sequence derived from a metapneumovirus is a F gene of the metapneumovirus. In other specific embodiments, the heterologous nucleotide sequence derived from a metapneumovirus is a G gene of the metapneumovirus. In some other embodiments, the heterologous nucleotide sequence is derived from a respiratory syncytial virus. In specific embodiments, the heterologous nucleotide sequence derived from respiratory syncytial virus is a F gene of the respiratory syncytial virus. In other specific embodiments, the heterologous nucleotide sequence derived from respiratory syncytial virus is a G gene of the respiratory syncytial virus. When one or more heterologous nucleotide sequences are inserted, the position of the insertion and the length of the intergenic region of each inserted copy can be manipulated and determined by different assays according to section 5.1.2. *infra.*

In certain embodiments, rescue of the chimeric virus or expression products may be achieved by reverse genetics in host cell systems where the host cells are transfected with chimeric cDNA or RNA constructs. The RNA templates of the present invention are prepared by transcription of appropriate DNA sequences with a DNA-directed RNA polymerase. The RNA templates of the present invention may be prepared either *in vitro* or *in vivo* by transcription of appropriate DNA sequences using a DNA-directed RNA polymerase such as bacteriophage T7 polymerase, T3 polymerase, the SP6 polymerase, or a eukaryotic polymerase such as polymerase I. In certain embodiments, the RNA templates of the present invention may be prepared either *in vitro* or *in vivo* by transcription of appropriate DNA sequences using a plasmid-based expression system as described in Hoffmann et al., 2000, Proc. Natl. Acad. Sci. USA 97:6108-6113 or the unidirectional RNA polymerase I-polymerase II transcription system as described in Hoffinann and Webster, 2000, J. Gen. Virol. 81:2843-2847. The resulting RNA templates of negative-polarity would contain appropriate terminal sequences that would enable the viral RNA-synthesizing apparatus to recognize the template. Alternatively, positive-polarity RNA templates that contain appropriate terminal sequences and enable the viral RNA-synthesizing apparatus to recognize the template may also be used. Expression from positive polarity RNA templates may be achieved by transfection of plasmids having promoters that are recognized by the DNA-dependent RNA polymerase. For example, plasmid DNA, encoding positive RNA templates under the control of a T7 promoter, can be used in combination with the vaccinia virus or fowlpox T7 system.

Bicistronic mRNAs can be constructed to permit internal initiation of translation of viral sequences and to allow for the expression of foreign protein coding sequences from the regular terminal initiation site, or vice versa. Alternatively, a foreign protein may be expressed from an internal transcriptional unit in which the transcriptional unit has an initiation site and polyadenylation site. In another embodiment, the foreign gene is inserted into a PIV gene such that the resulting expressed protein is a fusion protein.

In certain embodiments, the invention relates to trivalent vaccines comprising a virus of the invention. In specific embodiments, the virus used for a trivalent vaccine is a chimeric bovine parainfluenza type 3/human parainfluenza type3 virus containing a first heterologous nucleotide sequence derived from a metapneumovirus, such as human metapneumovirus or avian pneumovirus, and a second heterologous nucleotide sequence derived from respiratory syncytial virus. In an exemplary embodiment, such a trivalent vaccine would be specific to (a) the gene products of the F gene and the HN gene of the human parainfluenza virus; (b) the protein encoded by the heterologous nucleotide sequence derived from a metapneumovirus; and ©) the protein encoded by the heterologous nucleotide sequence derived from a respiratory syncytial virus. In a specific embodiment, the first heterologous nucleotide sequence is the F gene of the respiratory syncytial virus and is inserted in position 1, and the second heterologous nucleotide sequence is the F gene of the human metapneumovirus and is inserted in position 3. Many more combinations are encompassed by the present invention and some are shown by way of example in Table 1. For other combinations the F or G gene of an avian pneumovirus could be used. Further, nucleotide sequences encoding chimeric F proteins could be used (see *supra*). In some less preferred embodiments, the heterologous nucleotide sequence can be inserted at higher-numbered positions of the viral genome.

**Table 1. Exemplary arrangements of heterologous nucleotide sequences in the viruses used for trivalent vaccines.**

| Combination | Position 1 | Position 2 | Position 3 |
|---|---|---|---|
| 1 | F-gene of hMPV | F-gene of RSV | - |
| 2 | F-gene of RSV | F-gene of hMPV | - |
| 3 | - | F-gene of hMPV | F-gene of RSV |
| 4 | - | F-gene of RSV | F-gene of hMPV |
| 5 | F-gene of hMPV | - | F-gene of RSV |
| 6 | F-gene of RSV | - | F-gene of hMPV |
| 7 | G-gene of hMPV | G-gene of RSV | - |
| 8 | G-gene of RSV | G-gene of hMPV | - |
| 9 | - | G-gene of hMPV | G-gene of RSV |
| 10 | - | G-gene of RSV | G-gene of hMPV |
| 11 | G-gene of hMPV | - | G-gene of RSV |
| 12 | G-gene of RSV | - | G-gene of hMPV |
| 13 | F-gene of hMPV | G-gene of RSV | - |
| 14 | G-gene of RSV | F-gene of hMPV | - |
| 15 | - | F-gene of hMPV | G-gene of RSV |
| 16 | - | G-gene of RSV | F-gene of hMPV |
| 17 | F-gene of hMPV | - | G-gene of RSV |
| 18 | G-gene of RSV | - | F-gene of hMPV |
| 19 | G-gene of hMPV | F-gene of RSV | - |
| 20 | F-gene of RSV | G-gene of hMPV | - |
| 21 | - | G-gene of hMPV | F-gene of RSV |
| 22 | - | F-gene of RSV | G-gene of hMPV |
| 23 | G-gene of hMPV | - | F-gene of RSV |
| 24 | F-gene of RSV | - | G-gene of hMPV |

In some other embodiments, the intergenic region between a heterologous sequence and the start of the coding sequence of the downstream gene can be altered. For example, each gene listed on Table 1 may have a desirable length of the intergenic region. In an examplary embodiment, a trivalent vaccine comprises a b/h PIV3 vector with a F gene of respiratory syncytial virus inserted at position 1, an altered intergenic region of 177 nucleotides (originally 75 nucleotides to the downstream N gene start codon AUG), and a F gene of human metapneumovirus inserted at position 3 with its natural intergenic region. Many more combinations are encompassed by the present invention, as each insertion of a heterologous nucleotide sequence may be manipulated according to section 5.1.2., *infra.*

In a broader embodiment, the expression products and chimeric virions of the present invention may be engineered to create vaccines against a broad range of pathogens, including viral antigens, tumor antigens and auto antigens involved in autoimmune disorders. One way to achieve this goal involves modifying existing PIV genes to contain foreign sequences in their respective external domains. Where the heterologous sequences are epitopes or antigens of pathogens, these chimeric viruses may be used to induce a protective immune response against the disease agent from which these determinants are derived.

One approach for constructing these hybrid molecules is to insert the heterologous nucleotide sequence into a DNA complement of a PIV genome, *e.g.*, a hPIV3, a bPIV, or a bPIV/hPIV, so that the heterologous sequence is flanked by the viral sequences required for viral polymerase activity; *i.e.*, the viral polymerase binding site/promoter, hereinafter referred to as the viral polymerase binding site, and a polyadenylation site. In a preferred embodiment, the heterologous coding sequence is flanked by the viral sequences that comprise the replication promoters of the 5' and 3' termini, the gene start and gene end sequences, and the packaging signals that are found in the 5' and/or the 3' termini. In an alternative approach, oligonucleotides encoding the viral polymerase binding site, *e.g.*, the complement of the 3'-terminus or both termini of the virus genomic segment can be ligated to the heterologous coding sequence to construct the hybrid molecule. The placement of a foreign gene or segment of a foreign gene within a target sequence was formerly dictated by the presence of appropriate restriction enzyme sites within the target sequence. However, recent advances in molecular biology have lessened this problem greatly. Restriction enzyme sites can readily be placed anywhere within a target sequence through the use of site-directed mutagenesis (*e.g.*, see, for example, the techniques described by Kunkel, 1985, Proc. Natl. Acad. Sci. U.S.A. 82;488). Variations in polymerase chain reaction (PCR) technology, described *infra,* also allow for the specific insertion of sequences (*i.e.*, restriction enzyme sites) and also allow for the facile construction of hybrid molecules. Alternatively, PCR reactions could be used to prepare recombinant templates without the need of cloning. For example, PCR reactions could be used to prepare double-stranded DNA molecules containing a DNA-directed RNA polymerase promoter (*e.g.*, bacteriophage T3, T7 or SP6) and the hybrid sequence containing the heterologous gene and the PIV polymerase binding site. RNA templates could then be transcribed directly from this recombinant DNA. In yet another embodiment, the recombinant RNA templates may be prepared by ligating RNAs specifying the negative polarity of the heterologous gene and the viral polymerase binding site using an RNA ligase.

In addition, one or more nucleotides can be added at the 3' end of the HN gene in the untranslated region to adhere to the "Rule of Six" which may be important in successful virus rescue. The "Rule of Six" applies to many paramyxoviruses and requires that the number of nucleotides of an RNA genome be a factor of six to be functional. The addition of nucleotides can be accomplished by techniques known in the art such as using a commercial mutagenesis kits like the QuikChange mutagenesis kit (Stratagene). After addition of the appropriate number of nucleotides, the correct DNA fragment, for example, a DNA fragment of the hPIV3 F and HN gene, can then be isolated upon digestion with the appropriate restriction enzyme and gel purification. Sequence requirements for viral polymerase activity and constructs that may be used in accordance with the invention are described in the subsections below.

Without being bound by theory, several parameters affect the rate of replication of the recombinant virus and the level of expression of the heterologous sequence. In particular, the position of the heterologous sequence in bPIV, hPIV, b/h PIV and the length of the intergenic region that flanks the heterologous sequence determine rate of replication and expression level of the heterologous sequence.

In certain embodiments, the leader and or trailer sequence of the virus are modified relative to the wild type virus. In certain more specific embodiments, the lengths of the leader and/or trailer are altered. In other embodiments, the sequence(s) of the leader and/or trailer are mutated relative to the wild type virus.

The production of a recombinant virus of the invention relies on the replication of a partial or full-length copy of the negative sense viral RNA (vRNA) genome or a complementary copy thereof (cRNA). This vRNA or cRNA can be isolated from infectious virus, produced upon in-vitro transcription, or produced in cells upon transfection of nucleic acids. Second, the production of recombinant negative strand virus relies on a functional polymerase complex. Typically, the polymerase complex of pneumoviruses consists of N, P, L and possibly M2 proteins, but is not necessarily limited thereto.

Polymerase complexes or components thereof can be isolated from virus particles, isolated from cells expressing one or more of the components, or produced upon transfection of specific expression vectors.

Infectious copies of MPV can be obtained when the above mentioned vRNA, cRNA, or vectors expressing these RNAs are replicated by the above mentioned polymerase complex 16 (Schnell et al., 1994, EMBO J 13: 4195-4203; Collins et al., 1995, PNAS 92: 11563-11567; Hoffmann et al., 2000, PNAS 97: 6108-6113; Bridgen et al., 1996, PNAS 93: 15400-15404; Palese et al., 1996, PNAS 93: 11354-11358; Peeters et al., 1999, J.Virol. 73: 5001-5009; Durbin et al., 1997, Virology 235: 323-332).

The invention provides a host cell comprising a nucleic acid or a vector according to the invention. Plasmid or viral vectors containing the polymerase components of PIV (presumably N, P, L and M2, but not necessarily limited thereto) are generated in prokaryotic cells for the expression of the components in relevant cell types (bacteria, insect cells, eukaryotic cells). Plasmid or viral vectors containing full-length or partial copies of the PIV genome will be generated in prokaryotic cells for the expression of viral nucleic acids *in vitro* or *in vivo.* The latter vectors may contain other viral sequences for the generation of chimeric viruses or chimeric virus proteins, may lack parts of the viral genome for the generation of replication defective virus, and may contain mutations, deletions or insertions for the generation of attenuated viruses.

Infectious copies of PIV (being wild type, attenuated, replication-defective or chimeric) can be produced upon co-expression of the polymerase components according to the state-of-the-art technologies described above.

In addition, eukaryotic cells, transiently or stably expressing one or more full-length or partial PIV proteins can be used. Such cells can be made by transfection (proteins or nucleic acid vectors), infection (viral vectors) or transduction (viral vectors) and may be useful for complementation of mentioned wild type, attenuated, replication-defective or chimeric viruses.

### ... HETEROLOGOUS GENE SEQUENCES TO BE INSERTED

The present invention encompass engineering recombinant bovine or human parainfluenza viruses to express one or more heterologous sequences, wherein the heterologous sequences encode gene products or fragments of gene products that are preferably antigenic and/or immunogenic. As used herein, the term "antigenic" refers to the ability of a molecule to bind antibody or MHC molecules. The term "immunogenic" refers to the ability of a molecule to generate immune response in a host.

In a preferred embodiment, the heterologous nucleotide sequence to be inserted is derived from a negative strand RNA virus, including but not limited to, influenza virus, parainfluenza virus, respiratory syncytial virus, mammalian metapneumovirus (*e.g.*, human metapneumovirus) and avian pneumovirus. In a preferred embodiment, the heterologous sequence to be inserted includes, but is not limited to, a sequence that encodes a F or HN gene of human PIV, a F gene of RSV, a HA gene of influenza virus type A, B, or C, a F gene of human MPV, a F gene of avian pneumovirus, or an immunogenic and/or antigenic fragment thereof.

In some embodiments, the heterologous nucleotide sequence to be inserted is derived from a human metapneumovirus and/or an avian pneumovirus. In certain embodiments, the heterologous nucleotide sequence to be inserted is derived from (a) a human metapneumovirus and a respiratory syncytial virus; and/or (b) an avian pneumovirus and a respiratory syncytial virus.

In certain preferred embodiments of the invention, the heterologous nucleotide sequence to be inserted is derived from a F gene from a human metapneumovirus and/or an avian pneumovirus. In certain embodiments, the F gene is derived from (a) a human metapneumovirus and a respiratory syncytial virus; and/or (b) an avian pneumovirus and a respiratory syncytial virus.

In certain embodiments of the invention, the heterologous nucleotide sequence to be inserted is a G gene derived from a human metapneumovirus and/or an avian pneumovirus. In certain embodiments, the G gene is derived from (a) a human metapneumovirus and a respiratory syncytial virus; and/or (b) an avian pneumovirus and a respiratory syncytial virus.

In certain embodiments, any combination of different F genes and/or different G genes derived from human metapneumovirus, avian pneumovirus, and respiratory syncytial virus can be inserted into the virus of the invention with the proviso that in all embodiments at least one heterologous sequence derived from either human metapneumovirus or avian pneumovirus is present in the recombinant parainfluenza virus of the invention.

In certain embodiments, the nucleotide sequence to be inserted is a nucleotide sequence encoding a F protein derived from a human metapneumovirus. In certain other embodiments, the nucleotide sequence to be inserted is a nucleotide sequence encoding a G protein derived from a human metapneumovirus. In yet other embodiments, the nucleotide sequence to be inserted is a nucleotide sequence encoding a F protein derived from an avian pneumovirus. In yet other embodiments, the nucleotide sequence to be inserted is a nucleotide sequence encoding a G protein derived from an avian pneumovirus. With the proviso that in all embodiments of the invention at least one heterologous nucleotide sequence is derived from a metapneumovirus, the heterologous nucleotide sequence to be inserted encodes a F protein or a G protein of a respiratory syncytial virus.

In certain embodiments, the nucleotide sequence to be inserted encodes a chimeric F protein or a chimeric G protein. A chimeric F protein comprises parts of F proteins from different viruses, such as a human metapneumovirus, avian pneumovirus and/or respiratory syncytial virus. A chimeric G protein comprises parts of G proteins from different viruses, such as a human metapneumovirus, avian pneumovirus and/or respiratory syncytial virus. In a specific embodiment, the F protein comprises an ectodomain of a F protein of a metapneumovirus, a transmembrane domain of a F protein of a parainfluenza virus, and luminal domain of a F protein of a parainfluenza virus. In certain embodiments, the nucleic acid to be inserted encodes a F protein, wherein the transmembrane domain of the F protein is deleted so that a soluble F protein is expressed.

In certain specific embodiments, the heterologous nucleotide sequence of the invention is any one of SEQ ID NO:1 through SEQ ID NO:5, SEQ ID NO:14, and SEQ ID NO:15 (see Table 16). In certain specific embodiments, the nucleotide sequence encodes a protein of any one of SEQ ID NO:6 through SEQ ID NO:13, SEQ ID NO:16, and SEQ ID NO:17 (see Table 16). In certain specific embodiments, the nucleotide sequence encodes a protein of any one of SEQ ID NO. 314 to 389.

For heterologous nucleotide sequences derived from respiratory syncytial virus see, *e.g.*, PCT/US98/20230, which is hereby incorporated by reference in its entirety.

In a preferred embodiment, heterologous gene sequences that can be expressed into the chimeric viruses of the invention include but are not limited to those encoding antigenic epitopes and glycoproteins of viruses, such as influenza glycoproteins, in particular hemagglutinin H5, H7, respiratory syncytial virus epitopes, New Castle Disease virus epitopes, Sendai virus and infectious Laryngotracheitis virus (ILV), that result in respiratory disease. In a most preferred embodiment, the heterologous nucleotide sequences are derived from a metapneumovirus, such as human metapneumovirus and/or avian pneumovirus. In yet another embodiment of the invention, heterologous gene sequences that can be engineered into the chimeric viruses of the invention include, but are not limited to, those encoding viral epitopes and glycoproteins of viruses, such as hepatitis B virus surface antigen, hepatitis A or C virus surface glycoproteins of Epstein Barr virus, glycoproteins of human papilloma virus, simian virus 5 or mumps virus, West Nile virus, Dengue virus, glycoproteins of herpesviruses, VPI of poliovirus, and sequences derived from a human immunodeficiency virus (HIV), preferably type 1 or type 2. In yet another embodiment, heterologous gene sequences that can be engineered into chimeric viruses of the invention include, but are not limited to, those encoding Marek's Disease virus (MDV) epitopes, epitopes of infectious Bursal Disease virus (IBDV), epitopes of Chicken Anemia virus, infectious laryngotracheitis virus (ILV), Avian Influenza virus (AIV), rabies, feline leukemia virus, canine distemper virus, vesicular stomatitis virus, and swinepox virus *(see* Fields et al. (ed.), 1991, FUNDAMENTAL VIROLOGY, Second Edition, Raven Press, New York, incorporated by reference herein in its entirety).

Other heterologous sequences of the present invention include those encoding antigens that are characteristic of autoimmune diseases. These antigens will typically be derived from the cell surface, cytoplasm, nucleus, mitochondria and the like of mammalian tissues, including antigens characteristic of diabetes mellitus, multiple sclerosis, systemic lupus erythematosus, rheumatoid arthritis, pernicious anemia, Addison's disease, scleroderma, autoimmune atrophic gastritis, juvenile diabetes, and discold lupus erythromatosus.

Antigens that are allergens generally include proteins or glycoproteins, including antigens derived from pollens, dust, molds, spores, dander, insects and foods. In addition, antigens that are characteristic of tumor antigens typically will be derived from the cell surface, cytoplasm, nucleus, organelles and the like of cells of tumor tissue. Examples include antigens characteristic of tumor proteins, including proteins encoded by mutated oncogenes; viral proteins associated with tumors; and glycoproteins. Tumors include, but are not limited to, those derived.from the types of cancer: lip, nasopharynx, pharynx and oral cavity, esophagus, stomach, colon, rectum, liver, gall bladder, pancreas, larynx, lung and bronchus, melanoma of skin, breast, cervix, uterine, ovary, bladder, kidney, uterus, brain and other parts of the nervous system, thyroid, prostate, testes, Hodgkin's disease, non-Hodgkin's lymphoma, multiple myeloma and leukemia.

In one specific embodiment of the invention, the heterologous sequences are derived from the genome of human immunodeficiency virus (HIV), preferably human immunodeficiency virus-1 or human immunodeficiency virus-2. In another embodiment of the invention, the heterologous coding sequences may be inserted within a PIV gene coding sequence such that a chimeric gene product, that contains the heterologous peptide sequence within the PIV viral protein, is expressed. In such an embodiment of the invention, the heterologous sequences may also be derived from the genome of a human immunodeficiency virus, preferably of human immunodeficiency virus-1 or human immunodeficiency virus-2.

In instances whereby the heterologous sequences are HIV-derived, such sequences may include, but are not limited to sequences derived from the env gene (*i.e.*, sequences encoding all or part of gp160, gp120, and/or gp41), the pol gene (*i.e.*, sequences encoding all or part of reverse transcriptase, endonuclease, protease, and/or integrase), the gag gene (*i.e*., sequences encoding all or part of p7, p6, p55, p17/18, p24/25) tat, rev, nef, vif, vpu, vpr, and/or vpx.

In another embodiment, heterologous gene sequences that can be engineered into the chimeric viruses include those that encode proteins with immunopotentiating activities. Examples of immunopotentiating proteins include, but are not limited to, cytokines, interferon type 1, gamma interferon, colony stimulating factors, and interleukin -1, -2, -4, -5, -6, -12.

In addition, other heterologous gene sequences that may be engineered into the chimeric viruses include those encoding antigens derived from bacteria such as bacterial surface glycoproteins, antigens derived from fungi, and antigens derived from a variety of other pathogens and parasites. Examples of heterologous gene sequences derived from bacterial pathogens include, but are not limited to, those encoding antigens derived from species of the following genera: *Salmonella, Shigella, Chlamydia, Helicobacter, Yersinia, Bordatella, Pseudomonas, Neisseria, Vibrio, Haemophilus, Mycoplasma, Streptomyces, Treponema, Coxiella, Ehrlichia, Brucella, Streptobacillus, Fusospirocheta, Spirillum, Ureaplasma, Spirochaeta, Mycoplasma, Actinomycetes, Borrelia, Bacteroides, Trichomoras, Branhamella, Pasteurella, Clostridium, Corynebacterium, Listeria, Bacillus, Erysipelothrix, Rhodococcus, Escherichia, Klebsiella, Pseudomanas, Enterobacter, Serratia, Staphylococcus, Streptococcus, Legionella, Mycobacterium, Proteus, Campylobacter, Enterococcus, Acinetobacter, Morganella, Moraxella, Citrobacter, Rickettsia, Rochlimeae,* as well as bacterial species such as: *P. aeruginosa; E. coli, P. cepacia, S. epidermis, E. faecalis, S. pneumonias, S. aureus, N. meningitidis, S. pyogenes, Pasteurella multocida, Treponema pallidum,* and *P. mirabilis.*

Examples of heterologous gene sequences derived from pathogenic fungi, include, but are not limited to, those encoding antigens derived from fungi such as *Cryptococcus neoformans; Blastomyces dermatitidis; Aiellomyces dermatitidis; Histoplasma capsulatum; Coccidioides immitis; Candida species, including C. albicans, C. tropicalis, C. parapsilosis, C. guilliermondii and C. krusei, Aspergillus species, including A. fumigatus, A. flavus and A. niger, Rhizopus species; Rhizomucor species; Cunninghammella species; Apophysomyces species, including A. saksenaea, A. mucor and A. absidia; Sporothrix schenckii, Paracoccidioides brasiliensis; Pseudallescheria boydii, Torulopsis glabrata; Trichophyton species, Microsporum species and Dermatophyres species,* as well as any other yeast or fungus now known or later identified to be pathogenic.

Finally, examples of heterologous gene sequences derived from parasites include, but are not limited to, those encoding antigens derived from members of the Apicomplexa phylum such as, for example, *Babesia, Toxoplasma, Plasmodium, Eimeria, Isospora, Atoxoplasma, Cystoisospora, Hammondia, Besniotia, Sarcocystis, Frenkelia, Haemoproteus, Leucocytozoon, Theileria, Perkinsus and Gregarina spp.; Pneumocystis carinii;* members of the Microspora phylum such as, for example, *Nosema, Enterocytozoon, Encephalitozoon, Septata, Mrazekia, Amblyospora, Ameson, Glugea, Pleistophora and Microsporidium spp.;* and members of the Ascetospora phylum such as, for example, *Haplosporidium spp.,* as well as species including *Plasmodium falciparum, P. vivax, P. ovale, P. malaria; Toxoplasma gondii; Leishmania mexicana, L. tropica, L. major, L. aethiopica, L. donovani, Trypanosoma cruzi, T. brucei, Schistosoma mansoni_{;}S. haematobium, S. japonium; Trichinella spiralis; Wuchereria bancrofti; Brugia malayli; Entamoeba histolytica; Enterobius vermiculoarus; Taenia solium, T. saginata, Trichomonas vaginatis, T. hominis, T. tenax; Giardia lamblia; Cryptosporidium parvum; Pneumocytis carinii, Babesia bovis, B. divergens, B. microti, Isospora belli, L hominis; Dientamoeba fragilis; Onchocerca volvulus; Ascaris lumbricoides; Necator americanis; Ancylostoma duodenale; Strongyloides stercoralis; Capillaria philippinensis; Angiostrongylus cantonensis; Hymenolepis nana; Diphyllobothrium latum; Echinococcus granulosus, E. multilocularis; Paragonimus westermani, P. caliensis; Chlonorchis sinensis; Opisthorchis felineas, G. Viverini, Fasciola hepatica, Sarcoptes scabiei, Pediculus humanus; Phthirlus pubis; and Dermatobia hominis,* as well as any other parasite now known or later identified to be pathogenic.

### 5.1.2. METAPNEUMOVIRAL SEQUENCES TO BE INSERTED

proteins of a mammalian MPV. The invention further relates to nucleic acid sequences encoding fusion proteins, wherein the fusion protein contains a protein of a mammalian MPV or a fragment thereof and one or more peptides or proteins that are not derived from mammalian MPV. In a specific embodiment, a fusion protein of the invention contains a protein of a mammalian MPV or a fragment thereof and a peptide tag, such as, but not limited to a polyhistidine tag. The invention further relates to fusion proteins, wherein the fusion protein contains a protein of a mammalian MPV or a fragment thereof and one or more peptides or proteins that are not derived from mammalian MPV. The invention also relates to derivatives of nucleic acids encoding a protein of a mammlian MPV. The invention also relates to derivatives of proteins of a mammalian MPV. A derivative can be, but is not limited to, mutant forms of the protein, such as, but not limited to, additions, deletions, truncations, substitutions, and inversions. A derivative can further be a chimeric form of the protein of the mammalian MPV, wherein at least one domain of the protein is derived from a different protein. A derivative can also be a form of a protein of a mammalian MPV that is covalently or non-covalently linked to another molecule, such as, *e.g.*, a drug.

The viral isolate termed NL/1/00 (also 00-1) is a mammalian MPV of variant Al and its genomic sequence is shown in SEQ ID NO:95. The viral isolate termed NL/17/00 is a mammalian MPV of variant A2 and its genomic sequence is shown in SEQ ID NO:96. The viral isolate termed NL/1/99 (also 99-1) is a mammalian MPV of variant B1 and its genomic sequence is shown in SEQ ID NO:94. The viral isolate termed NL/1/94 is a mammalian MPV of variant B2 and its genomic sequence is shown in SEQ ID NO:97. A list of sequences disclosed in the present application and the corresponding SEQ ID Nos is set forth in Table 16.

The protein of a mammalian MPV can be a an N protein, a P protein, a M protein, a F protein, a M2-1 protein or a M2-2 protein or a fragment thereof. A fragment of a protein of a mammlian MPV can be can be at least 25 amino acids, at least 50 amino acids, at least 75 amino acids, at least 100 amino acids, at least 125 amino acids, at least 150 amino acids, at least 175 amino acids, at least 200 amino acids, at least 225 amino acids, at least 250 amino acids, at least 275 amino acids, at least 300 amino acids, at least 325 amino acids, at least 350 amino acids, at least 375 amino acids, at least 400 amino acids, at least 425 amino acids, at least 450 amino acids, at least 475 amino acids, at least 500 amino acids, at least 750 amino acids, at least 1000 amino acids, at least 1250 amino acids, at least 1500 amino acids, at least 1750 amino acids, at least 2000 amino acids or at least 2250 amino acids in length. A fragment of a protein of a mammlian MPV can be can be at most 25 amino acids, at most 50 amino acids, at most 75 amino acids, at most 100 amino acids, at most 125 amino acids, at most 150 amino acids, at most 175 amino acids, at most 200 amino acids, at most 225 amino acids, at most 250 amino acids, at most 275 amino acids, at most 300 amino acids, at most 325 amino acids, at most 350 amino acids, at most 375 amino acids, at most 400 amino acids, at most 425 amino acids, at most 450 amino acids, at most 475 amino acids, at most 500 amino acids, at most 750 amino acids, at most 1000 amino acids, at most 1250 amino acids, at most 1500 amino acids, at most 1750 amino acids, at most 2000 amino acids or at most 2250 amino acids in length.

In certain embodiments of the invention, the protein of a mammalian MPV is a N protein, wherein the N protein is phylogenetically closer related to a N protein of a mammalian MPV, such as the N protein encoded by, *e.g.*, the viral genome of SEQ ID NO:94, SEQ ID NO:95, SEQ ID NO:96, or SEQ ID NO:97, (see also Table 16 for a description of the SEQ ID Nos) than it is related to the N protein of APV type C. In certain embodiments of the invention, the protein of a mammalian MPV is a P protein, wherein the P protein is phylogenetically closer related to a P protein of a mammalian MPV, such as the P protein encoded by, *e.g.,* the viral genome of SEQ ID NO:NO:94, SEQ ID NO:95, SEQ ID NO:90, or SEQ ID NO:97, than it is related to the N protein of APV type C. In certain embodiments of the invention, the protein of a mammalian MPV is a M protein, wherein the M protein is closer related to a M protein of a mammalian MPV, such as the M protein encoded by, *e.g.,* the viral genome of SEQ ID NO:94, SEQ ID NO:95, SEQ ID NO:96, or SEQ ID NO:97, than it is related to the M protein of APV type C. In certain embodiments of the invention, the protein of a mammalian MPV is a F protein, wherein the F protein is phylogenetically closer related to a F protein of a mammalian MPV, such as the F protein encoded by, *e.g.*, the viral genome of SEQ ID NO:94, SEQ ID NO:95, SEQ ID NO:96, or SEQ ID NO:97, than it is related to the F protein of APV type C. In certain embodiments of the invention, the protein of a mammalian MPV is a M2-1 protein, wherein the M2-1 protein is phylogenetically closer related to a M2-1 protein of a mammalian MPV, such as the M2-1 1 protein encoded by, *e.g.,* the viral genome of SEQ ID NO:94, SEQ ID NO:95, SEQ ID NO:96, or SEQ ID NO:97, than it is related to the M2-1 protein of APV type C. In certain embodiments of the invention, the protein of a mammalian MPV is a M2-2 protein, wherein the M2-2 protein is phylogenetically closer related to a M2-2 protein of a mammalian MPV, such as the M2-2 protein encoded by, *e.g.*, the viral genome of SEQ ID NO:94, SEQ ID NO:95, SEQ ID NO:96, or SEQ ID NO:97, than it is related to the M2-2 protein of APV type C. In certain embodiments of the invention, the protein of a mammalian MPV is a G protein, wherein the G protein is phylogenetically closer related to a G protein of a mammalian MPV, such as the G protein encoded by, *e.g.*, the viral genome of SEQ ID NO:94, SEQ ID NO:95, SEQ ID NO:96, or SEQ ID NO:97, than it is related to any protein of APV type C. In certain embodiments of the invention, the protein of a mammalian MPV is a SH protein, wherein the SH protein is phylogenetically closer related to a SH protein of a mammalian MPV, such as the SH protein encoded by, *e.g.*, the viral genome of SEQ ID NO:94, SEQ ID NO:95, SEQ ID NO:96, or SEQ ID NO:97, than it is related to any protein of APV type C. In certain embodiments of the invention, the protein of a mammalian MPV is a L protein, wherein the L protein is phylogenetically closer related to a L protein of a mammalian MPV, such as the SH protein encoded by, *e.g.*, the viral genome of SEQ ID NO:94, SEQ ID NO:95, SEQ ID NO:96, or SEQ ID NO:97, than it is related to any protein of APV type C.

In certain embodiments of the invention, the protein of a mammalian MPV is a N protein, wherein the N protein is at least 60%, at least 65%, at least 70%, at least 75%, at least 80%, at least 85%, at least 90%, at least 95%, at least 98%, at least 99%, or at least 99.5% identical to the amino acid sequence of a N protein encoded by the viral genome of SEQ ID NO:94, SEQ ID NO:95, SEQ ID NO:96, or SEQ ID NO:97 (the amino acid sequences of the respective N proteins are disclosed in SEQ ID NO:366-369; see also Table 16). In certain embodiments of the invention, the protein of a mammalian MPV is a N protein, wherein the P protein is at least 60%, at least 65%, at least 70%, at least 75%, at least 80%, at least 85%, at least 90%, at least 95%, at least 98%, at least 99%, or at least 99.5% identical to the amino acid sequence of a P protein encoded by the viral genome of SEQ ID NO:94, SEQ ID NO:95, SEQ ID NO:96, or SEQ ID NO:97 (the amino acid sequences of the respective P proteins are disclosed in SEQ ID NO:78-85; see also Table 16). In certain embodiments of the invention, the protein of a mammalian MPV is a M protein, wherein the M protein is at least 60%, at least 65%, at least 70%, at least 75%, at least 80%, at least 85%, at least 90%, at least 95%, at least 98%, at least 99%, or at least 99.5% identical to the amino acid sequence of a M protein encoded by the viral genome of SEQ ID NO:94, SEQ ID NO:95, SEQ ID NO:96, or SEQ ID NO:97 (the amino acid sequences of the respective M proteins are disclosed in SEQ ID NO:358-361; see also Table 16). In certain embodiments of the invention, the protein of a mammalian MPV is a F protein, wherein the F protein is at least 60%, at least 65%, at least 70%, at least 75%, at least 80%, at least 85%, at least 90%, at least 95%, at least 98%, at least 99%, or at least 99.5% identical to the amino acid sequence of a F protein encoded by the viral genome of SEQ ID NO:94, SEQ ID NO:95, SEQ ID NO:96, or SEQ ID NO:97 (the amino acid sequences of the respective F proteins are disclosed in SEQ ID NO:18-25; see also Table 16). In certain embodiments of the invention, the protein of a mammalian MPV is a M2-1 protein, wherein the M2-1 protein is at least 60%, at least 65%, at least 70%, at least 75%, at least 80%, at least 85%, at least 90%, at least 95%, at least 98%, at least 99%, or at least 99.5% identical to the amino acid sequence of a M2-1 protein encoded by the viral genome of SEQ ID NO:94, SEQ ID NO:95, SEQ ID NO:96, or SEQ ID NO:97 (the amino acid sequences of the respective M2-1 proteins are disclosed in SEQ ID NO:42-49; see also Table 16). In certain embodiments of the invention, the protein of a mammalian MPV is a M2-2 protein, wherein the M2-2 protein is at least 60%, at least 65%, at least 70%, at least 75%, at least 80%, at least 85%, at least 90%, at least 95%, at least 98%, at least 99%, or at least 99.5% identical to the amino acid sequence of a M2-2 protein encoded by the viral genome of SEQ ID NO:94, SEQ ID NO:95, SEQ ID NO:96, or SEQ ID NO:97 (the amino acid sequences of the respective M2-2 proteins are disclosed in SEQ ID NO:50-57; see also Table 16). In certain embodiments of the invention, the protein of a mammalian MPV is a G protein, wherein the G protein is at least 60%, at least 65%, at least 70%, at least 75%, at least 80%, at least 85%, at least 90%, at least 95%, at least 98%, at least 99%, or at least 99.5% identical to the amino acid sequence of a G protein encoded by the viral genome of SEQ ID NO:94, SEQ ID NO:95, SEQ ID NO:96, or SEQ ID NO:97 (the amino acid sequences of the respective G proteins are disclosed in SEQ ID NO:26-33; see also Table 16). In certain embodiments of the invention, the protein of a mammalian MPV is a SH protein, wherein the SH protein is at least 60%, at least 65%, at least 70%, at least 75%, at least 80%, at least 85%, at least 90%, at least 95%, at least 98%, at least 99%, or at least 99.5% identical to the amino acid sequence of a SH protein encoded by the viral genome of SEQ ID NO:94, SEQ ID NO:95, SEQ ID NO:96, or SEQ ID NO:97 (the amino acid sequences of the respective SH proteins are disclosed in SEQ ID NO:86-93; see also Table 16). In certain embodiments of the invention, the protein of a mammalian MPV is a L protein, wherein the L protein is at least 60%, at least 65%, at least 70%, at least 75%, at least 80%, at least 85%, at least 90%, at least 95%, at least 98%, at least 99%, or at least 99.5% identical to the amino acid sequence of a L protein encoded by the viral genome of SEQ ID NO:94, SEQ ID NO:95, SEQ ID NO:96, or SEQ ID NO:97 (the amino acid sequences of the respective L proteins are disclosed in SEQ ID NO:34-41; see also Table 16).

A fragment of a protein of mammalian MPV is at least 60%, at least 65%, at least 70%, at least 75%, at least 80%, at least 85%, at least 90%, at least 95%, at least 98%, at least 99%, or at least 99.5% identical to the homologous protein encoded by the virus of SEQ ID NO:94, SEQ ID NO:95, SEQ ID NO:96, or SEQ ID NO:97 over the portion of the protein that is homologous to the fragment. In a specific, illustrative embodiment, the invention provides a fragment of the F protein of a mammalian MPV that contains the ectodomain of the F protein and homologs thereof. The homolog of the fragment of the F protein that contains the ectodomain is at least 60%, at least 65%, at least 70%, at least 75%, at least 80%, at least 85%, at least 90%, at least 95%, at least 98%, at least 99%, or at least 99.5% identical to the corresponding fragment containing the ectodomain of the F protein encoded by a virus of SEQ ID NO:94, SEQ ID NO:95, SEQ ID NO:96, or SEQ ID NO:97 (the amino acid sequences of the respective F proteins are disclosed in SEQ ID NO:18-25; see also Table 16).

In certain embodiments, the invention provides a protein of a mammalian MPV of subgroup A and fragments thereof. The invention provides a N protein of a mammalian MPV of subgroup A, wherein the N protein is phylogenetically closer related to the N protein encoded by a virus of SEQ ID NO:95 or SEQ ID NO:96 than it is related to the N protein encoded by a virus encoded by SEQ ID NO:94 or SEQ ID NO:97. The invention provides a G protein of a mammalian MPV of subgroup A, wherein the G protein is phylogenetically closer related to the G protein encoded by a virus of SEQ ID NO:95 or SEQ ID NO:96 than it is related to the G protein encoded by a virus encoded by SEQ ID NO:94 or SEQ ID NO:97. The invention provides a P protein of a mammalian MPV of subgroup A, wherein the P protein is phylogenetically closer related to the P protein encoded by a virus of SEQ ID NO:95 or SEQ ID NO:96 than it is related to the P protein encoded by a virus encoded by SEQ ID NO:94 or SEQ ID NO:97. The invention provides a M protein of a mammalian MPV of subgroup A, wherein the M protein is phylogenetically closer related to the M protein encoded by a virus of SEQ ID NO:95 or SEQ ID NO:96 than it is related to the M protein encoded by a virus encoded by SEQ ID NO:94 or SEQ ID NO:97. The invention provides a N protein of a mammalian MPV of subgroup A, wherein the F protein is phylogenetically closer related to the F protein encoded by a virus of SEQ ID NO:95 or SEQ ID NO:96 than it is related to the F protein encoded by a virus encoded by SEQ ID NO:94 or SEQ ID NO:97. The invention provides a M2-1 protein of a mammalian MPV of subgroup A, wherein the M2-1 protein is phylogenetically closer related to the M2-1 protein encoded by a virus of SEQ ID NO:95 or SEQ ID NO:96 than it is related to the M2-1 protein encoded by a virus encoded by SEQ ID NO:94 or SEQ ID NO:97. The invention provides a M2-2 protein of a mammalian MPV of subgroup A, wherein the M2-2 protein is phylogenetically closer related to the M2-2 protein encoded by a virus of SEQ ID NO:95 or SEQ ID NO:96 than it is related to the M2-2 protein encoded by a virus encoded by SEQ ID NO:94 or SEQ ID NO:97. The invention provides a SH protein of a mammalian MPV of subgroup A, wherein the SH protein is phylogenetically closer related to the SH protein encoded by a virus of SEQ ID NO:95 or SEQ ID NO:96 than it is related to the SH protein encoded by a virus encoded by SEQ ID NO:94 or SEQ ID NO:97. The invention provides a L protein of a mammalian MPV of subgroup A, wherein the L protein is phylogenetically closer related to the L protein encoded by a virus of SEQ ID NO:95 or SEQ ID NO:96 than it is related to the L protein encoded by a virus encoded by SEQ ID NO:94 or SEQ ID NO:97.

In other embodiments, the invention provides a protein of a mammalian MPV of subgroup B or fragments thereof. The invention provides a N protein of a mammalian MPV of subgroup B, wherein the N protein is phylogenetically closer related to the N protein encoded by a virus of SEQ ID NO:94 or SEQ ID NO:97 than it is related to the N protein encoded by a virus encoded by SEQ ID NO:95 or SEQ ID NO:96. The invention provides a G protein of a mammalian MPV of subgroup A, wherein the G protein is phylogenetically closer related to the G protein encoded by a virus of SEQ ID NO:94 or SEQ ID NO:97 than it is related to the G protein encoded by a virus encoded by SEQ ID NO:95 or SEQ ID NO:96. The invention provides a P protein of a mammalian MPV of subgroup A, wherein the P protein is phylogenetically closer related to the P protein encoded by a virus of SEQ ID NO:94 or SEQ ID NO:97 than it is related to the P protein encoded by a virus encoded by SEQ ID NO:95 or SEQ ID NO:96. The invention provides a M protein of a mammalian MPV of subgroup A, wherein the M protein is phylogenetically closer related to the M protein encoded by a virus of SEQ ID NO:94 or SEQ ID NO:97 than it is related to the M protein encoded by a virus encoded by SEQ ID NO:95 or SEQ ID NO:96. The invention provides a N protein of a mammalian MPV of subgroup A, wherein the F protein is phylogenetically closer related to the F protein encoded by a virus of SEQ ID NO:94 or SEQ ID NO:97 than it is related to the F protein encoded by a virus encoded by SEQ ID NO:95 or SEQ ID NO:96. The invention provides a M2-1 protein of a mammalian MPV of subgroup A, wherein the M2-1 protein is phylogenetically closer related to the M2-1 protein encoded by a virus of SEQ ID NO:94 or SEQ ID NO:97 than it is related to the M2-1 protein encoded by a virus encoded by SEQ ID NO:95 or SEQ ID NO:96. The invention provides a M2-2 protein of a mammalian MPV of subgroup A, wherein the M2-2 protein is phylogenetically closer related to the M2-2 protein encoded by a virus of SEQ ID NO:94 or SEQ ID NO:97 than it is related to the M2-2 protein encoded by a virus encoded by SEQ ID NO:95 or SEQ ID NO:96. The invention provides a SH protein of a mammalian MPV of subgroup A, wherein the SH protein is phylogenetically closer related to the SH protein encoded by a virus of SEQ ID NO:94 or SEQ ID NO:97 than it is related to the SH protein encoded by a virus encoded by SEQ ID NO:95 or SEQ ID NO:96. The invention provides a L protein of a mammalian MPV of subgroup A, wherein the L protein is phylogenetically closer related to the L protein encoded by a virus of SEQ ID NO:94 or SEQ ID NO:97 than it is related to the L protein encoded by a virus encoded by SEQ ID NO:95 or SEQ ID NO:96.

The invention provides a G protein of a mammalian MPV variant B1, wherein the G protein of a mammalian MPV variant B1 is phylogenetically closer related to the G protein of the prototype of variant B1, isolate NL/1/99, than it is related to the G protein of the prototype of variant A1, isolate NL/1/00, the G protein of the prototype of A2, isolate NL/17/00, or the G protein of the prototype of B2, isolate NL/1/94. The invention provides a G protein of a mammalian MPV variant B1, wherein the amino acid sequence of the G protein is at least 66%, at least 70%, at least 75%, at least 80%, at least 85%, at least 90%, at least 95%, at least 98%, or at least 99% or at least 99.5% identical to the G protein of a mammalian MPV variant B1 as represented by the prototype NL/1/99 (SEQ ID NO:28). In a specific embodiment, the G protein of a mammalian MPV has the amino acid sequence of SEQ ID NO:119-153. The invention provides a N protein of a mammalian MPV variant B1, wherein the N protein of a mammalian MPV variant B1 is phylogenetically closer related to the N protein of the prototype of variant B1, isolate NL/1/99, than it is related to the N protein of the prototype of variant A1, isolate NL/1/00, the N protein of the prototype of A2, isolate NL/17/00, or the N protein of the prototype of B2, isolate NL/1/94. The invention provides a N protein of a mammalian MPV variant B1, wherein the amino acid sequence of the N proteint is at least 98.5% or at least 99% or at least 99.5% identical to the N protein of a mammalian MPV variant B1 as represented by the prototype NL/1/99 (SEQ ID NO:72). The invention provides a P protein of a mammalian MPV variant B1, wherein the P protein of a mammalian MPV variant B 1 is phylogenetically closer related to the P protein of the prototype of variant B1, isolate NL/1/99, than it is related to the P protein of the prototype of variant A1, isolate NL/1/00, the P protein of the prototype of A2, isolate NL/17/00, or the P protein of the prototype of B2, isolate NL/1/94. The invention provides a P protein of a mammalian MPV variant B1, wherein the amino acid sequence of the P protein is at least 96%, at least 98%, or at least 99% or at least 99.5% identical the P protein of a mammalian MPV variant B1 as represented by the prototype NL/1/99 (SEQ ID NO:80). The invention provides a M protein of a mammalian MPV variant B1, wherein the M protein of a mammalian MPV variant B1 is phylogenetically closer related to the M protein of the prototype of variant B1, isolate NL/1/99, than it is related to the M protein of the prototype of variant A1, isolate NL/1/00, the M protein of the prototype of A2, isolate NL/17/00, or the M protein of the prototype of B2, isolate NL/1/94. The invention provides a M protein of a mammalian MPV variant B1, wherein the amino acid sequence of the M protein is identical to the M protein of a mammalian MPV variant B1 as represented by the prototype NL/1/99 (SEQ ID NO:64). The invention provides a F protein of a mammalian MPV variant B1, wherein the F protein of a mammalian MPV variant B1 is phylogenetically closer related to the F protein of variant B1, isolate NL/1/99, than it is related to the F protein of variant A1, isolate NL/1/00, the F protein of prototype A2, isolate NL/17/00, or the F protein of the prototype of B2, isolate NL/1/94. The invention provides a F protein of mammalian MPV variant B1, wherein the amino acid sequence of the F protein is identical at least 99% identical, to the F protein of a mammalian MPV variant B1 as represented by the prototype NL/1/99 (SEQ ID NO:20). In a specific embodiment, the F protein of a mammalian MPV has the amino acid sequence of SEQ ID NO: 248-327. The invention provides a M2-1 protein of a mammalian MPV variant B1, wherein the M2-1 protein of a mammalian MPV variant B1 is phylogenetically closer related to the M2-1 protein of the prototype of variant B1, isolate NL/1/99, than it is related to the M2-1 protein of the prototype of variant A1, isolate NL/1/00, the M2-1 protein of the prototype of A2, isolate NL/17/00, or the M2-1 protein of the prototype of B2, isolate NL/1/94. The invention provides a M2-1 protein of a mammalian MPV variant B1, wherein the amino acid sequence of the M2-1 protein is at least 98% or at least 99% or at least 99.5% identical the M2-1 protein of a mammalian MPV variant B1 as represented by the prototype NL/1/99 (SEQ ID NO:44). The invention provides a M2-2 protein of a mammalian MPV variant B1, wherein the M2-2 protein of a mammalian MPV variant B 1 is phylogenetically closer related to the M2-2 protein of the prototype of variant B1, isolate NL/1/99, than it is related to the M2-2 protein of the prototype of variant A1, isolate NL/1/00, the M2-2 protein of the prototype of A2, isolate NL/17/00, or the M2-2 protein of the prototype of B2, isolate NL/1/94. The invention provides a M2-2 protein of a mammalian MPV variant B1, wherein the amino acid sequence of the M2-2 protein is at least 99%or at least 99.5% identical the M2-2 protein of a mammalian MPV variant B1 as represented by the prototype NL/1/99 (SEQ ID NO:52). The invention provides a SH protein of a mammalian MPV variant B1, wherein the SH protein of a mammalian MPV variant B1 is phylogenetically closer related to the SH protein of the prototype of variant B1, isolate NL/1/99, than it is related to the SH protein of the prototype of variant A1, isolate NL/1/00, the SH protein of the prototype of A2, isolate NL/17/00, or the SH protein of the prototype of B2, isolate NL/1/94. The invention provides a SH protein of a mammalian MPV variant B1, wherein the amino acid sequence of the SH protein is at least 83%, at least 85%, at least 90%, at least 95%, at least 98%, or at least 99% or at least 99.5% identical the SH protein of a mammalian MPV variant B1 as represented by the prototype NL/1/99 (SEQ ID NO:88). The invention provides a L protein of a mammalian MPV variant B1, wherein the L protein of a mammalian MPV variant B1 is phylogenetically closer related to the L protein of the prototype of variant B1, isolate NL/1/99, than it is related to the L protein of the prototype of variant A1, isolate NL/1/00, the L protein of the prototype of A2, isolate NL/17/00, or the L protein of the prototype of B2, isolate NL/1/94. The invention provides a L protein of a mammalian MPV variant B1, wherein the amino acid sequence of the L protein is at least 99% or at least 99.5% identical the L protein a mammalian MPV variant B1 as represented by the prototype NL/1/99 (SEQ ID NO:36).

The invention provides a G protein of a mammalian MPV variant A1, wherein the G protein of a mammalian MPV variant A1 is phylogenetically closer related to the G protein of the prototype of variant A1, isolate NL/1/00, than it is related to the G protein of the prototype of variant B1, isolate NL/1/99, the G protein of the prototype of A2, isolate NL/17/00, or the G protein of the prototype of B2, isolate NL/1/94. The invention provides a G protein of a mammalian MPV variant A1, wherein the amino acid sequence of the G protein is at least 66%, at least 70%, at least 75%, at least 80%, at least 85%, at least 90%, at least 95%, at least 98%, or at least 99% or at least 99.5% identical to the G protein of a mammalian MPV variant A1 as represented by the prototype NL/1/00 (SEQ ID NO:26). The invention provides a N protein of a mammalian MPV variant A1, wherein the N protein of a mammalian MPV variant A1 is phylogenetically closer related to the N protein of the prototype of variant A1, isolate NL/1/00, than it is related to the N protein of the prototype of variant B1, isolate NL/1/99, the N protein of the prototype of A2, isolate NL/17/00, or the N protein of the prototype of B2, isolate NL/1/94. The invention provides a N protein of a mammalian MPV variant A1, wherein the amino acid sequence of the N protein is at least 99.5% identical to the N protein of a mammalian MPV variant A1 as represented by the prototype NL/1/00 (SEQ ID NO:70). The invention provides a P protein of a mammalian MPV variant A1, wherein the P protein of a mammalian MPV variant A1 is phylogenetically closer related to the P protein of the prototype of variant A1, isolate NL/1/00, than it is related to the P protein of the prototype of variant B1, isolate NL/1/99, the P protein of the prototype of A2, isolate NL/17/00, or the P protein of the prototype of B2, isolate NL/1/94. The invention provides a P protein of a mammalian MPV variant A1, wherein the amino acid sequence of the P protein is at least 96%, at least 98%, or at least 99% or at least 99.5% identical to the P protein of a mammalian MPV variant A1 as represented by the prototype NL/1/00 (SEQ ID NO:78). The invention provides a M protein of a mammalian MPV variant A1, wherein the M protein of a mammalian MPV variant A1 is phylogenetically closer related to the M protein of the prototype of variant A1, isolate NL/1/00, than it is related to the M protein of the prototype of variant B1, isolate NL/1/99, the M protein of the prototype of A2, isolate NL/17/00, or the M protein of the prototype of B2, isolate NL/1/94. The invention provides a M protein of a mammalian MPV variant A1, wherein the amino acid sequence of the M protein is at least 99% or at least 99.5% identical to the M protein of a mammalian MPV variant A1 as represented by the prototype NL/1/00 (SEQ ID NO:62). The invention provides a F protein of a mammalian MPV variant A1, wherein the F protein of a mammalian MPV variant A1 is phylogenetically closer related to the F protein of the prototype of variant A1, isolate NL/1/00, than it is related to the F protein of the prototype of variant B1, isolate NL/1/99, the F protein of the prototype of A2, isolate NL/17/00, or the F protein of the prototype of B2, isolate NL/1/94. The invention provides a F protein of a mammalian MPV variant A1, wherein the amino acid sequence of the F protein is at least 98% or at least 99% or at least 99.5% identical to the F protein of a mammalian MPV variant A1 as represented by the prototype NL/1/00 (SEQ ID NO:18). The invention provides a M2-1 protein of a mammalian MPV variant A1, wherein the M2-1 protein of a mammalian MPV variant A1 is phylogenetically closer related to the M2-1 protein of the prototype of variant A1, isolate NL/1/00, than it is related to the M2-1 protein of the prototype of variant B1, isolate NL/1/99, the M2-1 protein of the prototype of A2, isolate NL/17/00, or the M2-1 protein of the prototype of B2, isolate NL/1/94. The invention provides a M2-1 protein of a mammalian MPV variant A1, wherein the amino acid sequence of the M2-1 protein is at least 99% or at least 99.5% identical to the M2-1 protein of a mammalian MPV variant A1 as represented by the prototype NL/1/00 (SEQ ID NO:42). The invention provides a M2-2 protein of a mammalian MPV variant A1, wherein the M2-2 protein of a mammalian MPV variant A1 is phylogenetically closer related to the M2-2 protein of the prototype of variant A1, isolate NL/1/00, than it is related to the M2-2 protein of the prototype of variant B1, isolate NL/1/99, the M2-2 protein of the prototype of A2, isolate NL/17/00, or the M2-2 protein of the prototype of B2, isolate NL/1/94. The invention provides a M2-2 protein of a mammalian MPV variant A1, wherein the amino acid sequence of the M2-2 protein is at least 96% or at least 99% or at least 99.5% identical to the M2-2 protein of a mammalian MPV variant A1 as represented by the prototype NL/1/00 (SEQ ID NO:50). The invention provides a SH protein of a mammalian MPV variant A1, wherein the SH protein of a mammalian MPV variant A1 is phylogenetically closer related to the SH protein of the prototype of variant A1, isolate NL/1/00, than it is related to the SH protein of the prototype of variant B1, isolate NL/1/99, the SH protein of the prototype of A2, isolate NL/17/00, or the SH protein of the prototype of B2, isolate NL/1/94. The invention provides a SH protein of a mammalian MPV variant A1, wherein the amino acid sequence of the SH protein is at least 84%, at least 90%, at least 95%, at least 98%, or at least 99% or at least 99.5% identical to the SH protein of a mammalian MPV variant A1 as represented by the prototype NL/1/00 (SEQ ID NO:86). The invention provides a L protein of a mammalian MPV variant A1, wherein the L protein of a mammalian MPV variant A1 is phylogenetically closer related to the L protein of the prototype of variant A1, isolate NL/1/00, than it is related to the L protein of the prototype of variant B1, isolate NL/1/99, the L protein of the prototype of A2, isolate NL/17/00, or the L protein of the prototype of B2, isolate NL/1/94. The invention provides a L protein of a mammalian MPV variant A1, wherein the amino acid sequence of the L protein is at least 99% or at least 99.5% identical to the L protein of a virus of a mammalian MPV variant A1 as represented by the prototype NL/1/00 (SEQ ID NO:34).

The invention provides a G protein of a mammalian MPV variant A2, wherein the G protein of a mammalian MPV variant A2 is phylogenetically closer related to the G protein of the prototype of variant A2, isolate NL/17/00, than it is related to the G protein of the prototype of variant B1, isolate NL/1/99, the G protein of the prototype of A1, isolate NL/1/00, or the G protein of the prototype of B2, isolate NL/1/94. The invention provides a G protein of a mammalian MPV variant A2, wherein the amino acid sequence of the G protein is at least 66%, at least 70%, at least 75%, at least 80%, at least 85%, at least 90%, at least 95%, at least 98%, at least 99% or at least 99.5% identical to the G protein of a mammalian MPV variant A2 as represented by the prototype NL/17/00 (SEQ ID NO:27). The invention provides a N protein of a mammalian MPV variant A2, wherein the N protein of a mammalian MPV variant A2 is phylogenetically closer related to the N protein of the prototype of variant A2, isolate NL/17/00, than it is related to the N protein of the prototype of variant B1, isolate NL/1/99, the N protein of the prototype of A1, isolate NL/1/00, or the N protein of the prototype of B2, isolate NL/1/94. The invention provides a N protein of a mammalian MPV variant A2, wherein the amino acid sequence of the N protein at least 99.5% identical to the N protein of a mammalian MPV variant A2 as represented by the prototype NL/17/00 (SEQ ID NO:71). The invention provides a P protein of a mammalian MPV variant A2, wherein the P protein of a mammalian MPV variant A2 is phylogenetically closer related to the P protein of the prototype of variant A2, isolate NL/17/00, than it is related to the P protein of the prototype of variant B1, isolate NL/1/99, the P protein of the prototype of A1, isolate NL/1/00, or the P protein of the prototype of B2, isolate NL/1/94. The invention provides a P protein of a mammalian MPV variant A2, wherein the amino acid sequence of the P protein is at least 96%, at least 98%, at least 99% or at least 99.5% identical to the P protein of a mammalian MPV variant A2 as represented by the prototype NL/17/00 (SEQ ID NO:79). The invention provides a M protein of a mammalian MPV variant A2, wherein the M protein of a mammalian MPV variant A2 is phylogenetically closer related to the M protein of the prototype of variant A2, isolate NL/17/00, than it is related to the M protein of the prototype of variant B1, isolate NL/1/99, the M protein of the prototype of A1, isolate NL/1/00, or the M protein of the prototype of B2, isolate NL/1/94. The invention provides a M protein of a mammalian MPV variant A2, wherein the the amino acid sequence of the M protein is at least 99%, or at least 99.5% identical to the M protein of a mammalian MPV variant A2 as represented by the prototype NL/17/00 (SEQ ID NO:63). The invention provides a F protein of a mammalian MPV variant A2, wherein the F protein of a mammalian MPV variant A2 is phylogenetically closer related to the F protein of the prototype of variant A2, isolate NL/17/00, than it is related to the F protein of the prototype of variant B1, isolate NL/1/99, the F protein of the prototype of A1, isolate NL/1/00, or the F protein of the prototype of B2, isolate NL/1/94. The invention provides a F protein of a mammalian MPV variant A2, wherein the amino acid sequence of the F protein is at least 98%, at least 99% or at least 99.5% identical to the F protein of a mammalian MPV variant A2 as represented by the prototype NL/17/00 (SEQ ID NO:19). The invention provides a M2-1 protein of a mammalian MPV variant A2, wherein the M2-1 protein of a mammalian MPV variant A2 is phylogenetically closer related to the M2-1 protein of the prototype of variant A2, isolate NL/17/00, than it is related to the M2-1 protein of the prototype of variant B1, isolate NL/1/99, the M2-1 protein of the prototype of A1, isolate NL/1/00, or the M2-1 protein of the prototype of B2, isolate NL/1/94. The invention provides a M2-1 protein of a mammalian MPV variant A2, wherein the amino acid sequence of the M2-1 protein is at least 99%, or at least 99.5% identical to the M2-1 protein of a mammalian MPV variant A2 as represented by the prototype NL/17/00 (SEQ ID NO:43). The invention provides a M2-2 protein of a mammalian MPV variant A2, wherein the M2-2 protein of a mammalian MPV variant A2 is phylogenetically closer related to the M2-2 protein of the prototype of variant A2, isolate NL/17/00, than it is related to the M2-2 protein of the prototype of variant B1, isolate NL/1/99, the M2-2 protein of the prototype of A1, isolate NL/1/00, or the M2-2 protein of the prototype of B2, isolate NL/1/94. The invention provides a M2-2 protein of a mammalian MPV variant A2, wherein the amino acid sequence of the M2-2 protein is at least 96%, at least 98%, at least 99% or at least 99.5% identical to the M2-2 protein of a mammalian MPV variant A2 as represented by the prototype NL/17/00 (SEQ ID NO:51). The invention provides a SH protein of a mammalian MPV variant A2, wherein the SH protein of a mammalian MPV variant A2 is phylogenetically closer related to the SH protein of the prototype of variant A2, isolate NL/17/00, than it is related to the SH protein of the prototype of variant B1, isolate NL/1/99, the SH protein of the prototype of A1, isolate NL/1/00, or the SH protein of the prototype of B2, isolate NL/1/94. The invention provides a SH protein of a mammalian MPV variant A2, wherein the amino acid sequence of the SH protein is at least 84%, at least 85%, at least 90%, at least 95%, at least 98%, at least 99% or at least 99.5% identical to the SH protein of a mammalian MPV variant A2 as represented by the prototype NL/17/00 (SEQ ID NO:87). The invention provides a L protein of a mammalian MPV variant A2, wherein the L protein of a mammalian MPV variant A2 is phylogenetically closer related to the L protein of the prototype of variant A2, isolate NL/17/00, than it is related to the L protein of the prototype of variant B1, isolate NL/1/99, the L protein of the prototype of A1, isolate NL/1/00, or the L protein of the prototype of B2, isolate NL/1/94. The invention provides a L protein of a mammalian MPV variant A2, wherein the amino acid sequence of the L protein is at least 99% or at least 99.5% identical to the L protein of a mammalian MPV variant A2 as represented by the prototype NL/17/00 (SEQ ID NO:35).

The invention provides a G protein of a mammalian MPV variant B2, wherein the G protein of a mammalian MPV variant B2 is phylogenetically closer related to the G protein of the prototype of variant B2, isolate NL/1/94, than it is related to the G protein of the prototype of variant B1, isolate NL/1/99, the G protein of the prototype of A1, isolate NL/1/00, or the G protein of the prototype of A2, isolate NL/17/00. The invention provides a G protein of a mammalian MPV variant B2, wherein the amino acid sequence of the G protein is at least 66%, at least 70%, at least 75%, at least 80%, at least 85%, at least 90%, at least 95%, at least 98%, or at least 99% or at least 99.5% identical to the G protein of a mammalian MPV variant B2 as represented by the prototype NL/1/94 (SEQ ID NO:29). The invention provides a N protein of a mammalian MPV variant B2, wherein the N protein of a mammalian MPV variant B2 is phylogenetically closer related to the N protein of the prototype of variant B2, isolate NL/1/94, than it is related to the N protein of the prototype of variant B1, isolate NL/1/99, the N protein of the prototype of A1, isolate NL/1/00, or the N protein of the prototype of A2, isolate NL/17/00. The invention provides a N protein of a mammalian MPV variant B2, wherein the amino acid sequence of the N protein is at least 99% or at least 99.5% identical to the N protein of a mammalian MPV variant B2 as represented by the prototype NL/1/94 (SEQ ID NO:73). The invention provides a P protein of a mammalian MPV variant B2, wherein the P protein of a mammalian MPV variant B2 is phylogenetically closer related to the P protein of the prototype of variant B2, isolate NL/1/94, than it is related to the P protein of the prototype of variant B1, isolate NL/1/99, the P protein of the prototype of A1, isolate NL/1/00, or the P protein of the prototype of A2, isolate NL/17/00. The invention provides a P protein of a mammalian MPV variant B2, wherein the amino acid sequence of the P protein is at least 96%, at least 98%, or at least 99% or at least 99.5% identical to the P protein of a mammalian MPV variant B2 as represented by the prototype NL/1/94 (SEQ ID NO:81). The invention provides a M protein of a mammalian MPV variant B2, wherein the M protein of a mammalian MPV variant B2 is phylogenetically closer related to the M protein of the prototype of variant B2, isolate NL/1/94, than it is related to the M protein of the prototype of variant B1, isolate NL/1/99, the M protein of the prototype of A1, isolate NL/1/00, or the M protein of the prototype of A2, isolate NL/17/00. The invention provides a M protein of a mammalian MPV variant B2, wherein the amino acid sequence of its M protein is identical to the M protein of a mammalian MPV variant B2 as represented by the prototype NL/1/94 (SEQ ID NO:65). The invention provides a F protein of a mammalian MPV variant B2, wherein the F protein of a mammalian MPV variant B2 is phylogenetically closer related to the F protein of the prototype of variant B2, isolate NL/1/94, than it is related to the F protein of the prototype of variant B1, isolate NL/1/99, the F protein of the prototype of A1, isolate NL/1/00, or the F protein of the prototype of A2, isolate NL/17/00. The invention provides a F protein of a mammalian MPV variant B2, wherein the amino acid sequence of the F protein is at least 99% or at least 99.5% identical to the F protein of a mammalian MPV variant B2 as represented by the prototype NL/1/94 (SEQ ID NO:21). The invention provides a M2-1 protein of a mammalian MPV variant B2, wherein the M2-1 protein of a mammalian MPV variant B2 is phylogenetically closer related to the M2-1 protein of the prototype of variant B2, isolate NL/1/94, than it is related to the M2-1 protein of the prototype of variant B1, isolate NL/1/99, the M2-1 protein of the prototype of A1, isolate NL/1/00, or the M2-1 protein of the prototype of A2, isolate NL/17/00. The invention provides a M2-1 protein of a mammalian MPV variant B2, wherein the amino acid sequence of the M2-1 protein is at least 98% or at least 99% or at least 99.5% identical to the M2-1 protein of a mammalian MPV variant B2 as represented by the prototype NL/1/94 (SEQ ID NO:45). The invention provides a M2-2 protein of a mammalian MPV variant B2, wherein the M2-2 protein of a mammalian MPV variant B2 is phylogenetically closer related to the M2-2 protein of the prototype of variant B2, isolate NL/1/94, than it is related to the M2-2 protein of the prototype of variant B1, isolate NL/1/99, the M2-2 protein of the prototype of A1, isolate NL/1/00, or the M2-2 protein of the prototype of A2, isolate NL/17/00. The invention provides a M2-2 protein of a mammalian MPV variant B2, wherein the amino acid sequence is at least 99% or at least 99.5% identical to the M2-2 protein of a mammalian MPV variant B2 as represented by the prototype NL/1/94 (SEQ ID NO:53). The invention provides a SH protein of a mammalian MPV variant B2, wherein the SH protein of a mammalian MPV variant B2 is phylogenetically closer related to the SH protein of the prototype of variant B2, isolate NL/1/94, than it is related to the SH protein of the prototype of variant B1, isolate NL/1/99, the SH protein of the prototype of A1, isolate NL/1/00, or the SH protein of the prototype of A2, isolate NL/17/00. The invention provides a SH protein of a mammalian MPV variant B2, wherein the amino acid sequence of the SH protein is at least 84%, at least 85%, at least 90%, at least 95%, at least 98%, or at least 99% or at least 99.5% identical to the SH protein of a mammalian MPV variant B2 as represented by the prototype NL/1/94 (SEQ ID NO:89). The invention provides a L protein of a mammalian MPV variant B2, wherein the L protein of a mammalian MPV variant B2 is phylogenetically closer related to the L protein of the prototype of variant B2, isolate NL/1/94, than it is related to the L protein of the prototype of variant B1, isolate NL/1/99, the L protein of the prototype of A1, isolate NL/1/00, or the L protein of the prototype of A2, isolate NL/17/00. The invention provides a L protein of a mammalian MPV variant B2, wherein the and/or if the amino acid sequence of the L protein is at least 99% or at least 99.5% identical to the L protein of a mammalian MPV variant B2 as represented by the prototype NL/1/94 (SEQ ID NO:37).

In certain embodiments, the percentage of sequence identity is based on an alignment of the full length proteins. In other embodiments, the percentage of sequence identity is based on an alignment of contiguous amino acid sequences of the proteins, wherein the amino acid sequences can be 25 amino acids, 50 amino acids, 75 amino acids, 100 amino acids, 125 amino acids, 150 amino acids, 175 amino acids, 200 amino acids, 225 amino acids, 250 amino acids, 275 amino acids, 300 amino acids, 325 amino acids, 350 amino acids, 375 amino acids, 400 amino acids, 425 amino acids, 450 amino acids, 475 amino acids, 500 amino acids, 750 amino acids, 1000 amino acids, 1250 amino acids, 1500 amino acids, 1750 amino acids, 2000 amino acids or 2250 amino acids in length.

The invention further provides nucleic acid sequences derived from a mammalian MPV. The invention also provides derivatives of nucleic acid sequences derived from a mammalian MPV. In certain specific embodiments the nucleic acids are modified.

In certain embodiments, a nucleic acid of the invention encodes a G protein, a N protein, a P protein, a M protein, a F protein, a M2-1 protein, a M2-2 protein, a SH protein, or a L protein of a mammalian MPV as defined above. In certain embodiments, a nucleic acid of the invention encodes a G protein, a N protein, a P protein, a M protein, a F protein, a M2-1 protein, a M2-2 protein, a SH protein, or a L protein of subgroup A of a mammalian MPV as defined above. In a specific embodiment, the G gene of a mammalian MPV has the nucleotide sequence of SEQ ID NO:98-132. In a specific embodiment, the F gene of a mammalian MPV has the nucleotide sequence of SEQ ID NO: 168-247. In certain embodiments, a nucleic acid of the invention encodes a G protein, a N protein, a P protein, a M protein, a F protein, a M2-1 protein, a M2-2 protein, a SH protein, or a L protein of subgroup B of a mammalian MPV as defined above. In certain embodiments, a nucleic acid of the invention encodes a G protein, a N protein, a P protein, a M protein, a F protein, a M2-1 protein, a M2-2 protein, a SH protein, or a L protein of variant A1 of a mammalian MPV as defined above. In certain embodiments, a nucleic acid of the invention encodes a G protein, a N protein, a P protein, a M protein, a F protein, a M2-1 protein, a M2-2 protein, a SH protein, or a L protein of variant A2 of a mammalian MPV as defined above. In certain embodiments, a nucleic acid of the invention encodes a G protein, a N protein, a P protein, a M protein, a F protein, a M2-1 protein, a M2-2 protein, a SH protein, or a L protein of variant B1 of a mammalian MPV as defined above. In certain embodiments, a nucleic acid of the invention encodes a G protein, a N protein, a P protein, a M protein, a F protein, a M2-1 protein, a M2-2 protein, a SH protein, or a L protein of variant B2 of a mammalian MPV as defined above.

In certain embodiments, the invention provides a nucleotide sequence that is at least 50%, at least 55%, at least 60%, at least 65%, at least 70%, at least 75%, at least 75%, at least 80%, at least 85%, at least 90%, at least 95%, at least 98%, at least 99%, or at least 99.5% identical to the nucleotide sequence of SEQ ID NO:94, SEQ ID NO:95, SEQ ID NO:96, or SEQ ID NO:97. In certain embodiments, the nucleic acid sequence of the invention, is at least 50%, at least 55%, at least 60%, at least 65%, at least 70%, at least 75%, at least 75%, at least 80%, at least 85%, at least 90%, at least 95%, at least 98%, at least 99%, or at least 99.5% identical to a fragment of the nucleotide sequence of SEQ ID NO:94, SEQ ID NO:95, SEQ ID NO:96, or SEQ ID NO:97, wherein the fragment is at least 25 nucleotides, at least 50 nucleotides, at least 75 nucleotides, at least 100 nucleotides, at least 150 nucleotides, at least 200 nucleotides, at least 250 nucleotides, at least 300 nucleotides, at least 400 nucleotides, at least 500 nucleotides, at least 750 nucleotides, at least 1,000 nucleotides, at least 1,250 nucleotides, at least 1,500 nucleotides, at least 1,750 nucleotides, at least 2,000 nucleotides, at least 2,00 nucleotides, at least 3,000 nucleotides, at least 4,000 nucleotides, at least 5,000 nucleotides, at least 7,500 nucleotides, at least 10,000 nucleotides, at least 12,500 nucleotides, or at least 15,000 nucleotides in length. In a specific embodiment, the nucleic acid sequence of the invention is at least 50%, at least 55%, at least 60%, at least 65%, at least 70%, at least 75%, at least 75%, at least 80%, at least 85%, at least 90%, at least 95%, at least 98%, at least 99%, or at least 99.5% or 100% identical to one of the nucleotide sequences of SEQ ID NO:98-132; SEQ ID NO:168-247; SEQ ID NO:22-25; SEQ ID NO:30-33; SEQ ID NO:38-41; SEQ ID NO:46-49; SEQ ID NO:54-57; SEQ ID NO:58-61; SEQ ID NO:66-69; SEQ ID NO:74-77; SEQ ID NO:82-85; or SEQ ID NO:90-93.

In specific embodiments of the invention, a nucleic acid sequence of the invention is capable of hybridizing under low stringency, medium stringency or high stringency conditions to one of the nucleic acid sequences of SEQ ID NO:94, SEQ ID NO:95, SEQ ID NO:96, or SEQ ID NO:97. In specific embodiments of the invention, a nucleic acid sequence of the invention is capable of hybridizing under low stringency, medium stringency or high stringency conditions to one of the nucleic acid sequences of SEQ ID NO:98-132; SEQ ID NO:168-247; SEQ ID NO:22-25; SEQ ID NO:30-33; SEQ ID NO:38-41; SEQ ID NO:46-49; SEQ ID NO:54-57; SEQ ID NO:58-61; SEQ ID NO:66-69; SEQ ID NO:74-77; SEQ ID NO:82-85; or SEQ ID NO:90-93. In certain embodiments, a nucleic acid hybridizes over a length of at least 25 nucleotides, at least 50 nucleotides, at least 75 nucleotides, at least 100 nucleotides, at least 150 nucleotides, at least 200 nucleotides, at least 250 nucleotides, at least 300 nucleotides, at least 400 nucleotides, at least 500 nucleotides, at least 750 nucleotides, at least 1,000 nucleotides, at least 1,250 nucleotides, at least 1,500 nucleotides, at least 1,750 nucleotides, at least 2,000 nucleotides, at least 2,00 nucleotides, at least 3,000 nucleotides, at least 4,000 nucleotides, at least 5,000 nucleotides, at least 7,500 nucleotides, at least 10,000 nucleotides, at least 12,500 nucleotides, or at least 15,000 nucleotides with the nucleotide sequence of SEQ ID NO:94, SEQ ID NO:95, SEQ ID NO:96, or SEQ ID NO:97.

The invention further provides antibodies and antigen-binding fragments that bind specifically to a protein of a mammalian MPV. An antibody of the invention binds specifically to a G protein, a N protein, a P protein, a M protein, a F protein, a M2-1 protein, a M2-2 protein, a SH protein, or a L protein of a mammalian MPV. In specific embodiments, the antibody is a human antibody or a humanized antibody. In certain embodiments, an antibody of the invention binds specifically to a G protein, a N protein, a P protein, a M protein, a F protein, a M2-1 protein, a M2-2 protein, a SH protein, or a L protein of a virus of subgroup A of a mammalian MPV. In certain other embodiments, an antibody of the invention binds specifically to a G protein, a N protein, a P protein, a M protein, a F protein, a M2-1 protein, a M2-2 protein, a SH protein, or a L protein of a virus of subgroup B of a mammalian MPV. In certain, more specific, embodiments, an antibody of the invention binds specifically to a G protein, a N protein, a P protein, a M protein, a F protein, a M2-1 protein, a M2-2 protein, a SH protein, or a L protein of a virus of variant A1 of a mammalian MPV. In other embodiments, the antibody of the invention binds specifically to a G protein, a N protein, a P protein, a M protein, a F protein, a M2-1 protein, a M2-2 protein, a SH protein, or a L protein of a virus of subgroup A2 of a mammalian MPV. In certain embodiments, an antibody of the invention binds specifically to a G protein, a N protein, a P protein, a M protein, a F protein, a M2-1 protein, a M2-2 protein, a SH protein, or a L protein of a virus of subgroup B1 of a mammalian MPV. In certain other embodiments, an antibody of the invention binds specifically to a G protein, a N protein, a P protein, a M protein, a F protein, a M2-1 protein, a M2-2 protein, a SH protein, or a L protein of a virus of subgroup B2 of a mammalian MPV.

### 5.1.3. INSERTION OF THE HETEROLOGOUS GENE SEQUENCE

Insertion of a foreign gene sequence into a viral vector of the invention can be accomplished by either a complete replacement of a viral coding region with a heterologous sequence, or by a partial replacement of the same, or by adding the heterologous nucleotide sequence to the viral genome. Complete replacement would probably best be accomplished through the use of PCR-directed mutagenesis. Briefly, PCR-primer A would contain, from the 5' to 3' end: a unique restriction enzyme site, such as a class IIS restriction enzyme site (*i.e.,* a "shifter" enzyme; that recognizes a specific sequence but cleaves the DNA either upstream or downstream of that sequence); a stretch of nucleotides complementary to a region of the PIV gene; and a stretch of nucleotides complementary to the carboxy-terminus coding portion of the heterologous sequence. PCR-primer B would contain from the 5' to 3' end: a unique restriction enzyme site; a stretch of nucleotides complementary to a PIV gene; and a stretch of nucleotides corresponding to the 5' coding portion of the foreign gene. After a PCR reaction using these primers with a cloned copy of the foreign gene, the product may be excised and cloned using the unique restriction sites. Digestion with the class IIS enzyme and transcription with the purified phage polymerase would generate an RNA molecule containing the exact untranslated ends of the PIV gene with a foreign gene insertion. In an alternate embodiment, PCR-primed reactions could be used to prepare double-stranded DNA containing the bacteriophage promoter sequence, and the hybrid gene sequence so that RNA templates can be transcribed directly without cloning.

A heterologous nucleotide sequence can be added or inserted at various positions of the virus of the invention. In one embodiment, the heterologous nucleotide sequence is added or inserted at position 1. In another embodiment, the heterologous nucleotide sequence is added or inserted at position 2. In another embodiment, the heterologous nucleotide sequence is added or inserted at position 3. In another embodiment, the heterologous nucleotide sequence is added or inserted at position 4. In another embodiment, the heterologous nucleotide sequence is added or inserted at position 5. In yet another embodiment, the heterologous nucleotide sequence is added or inserted at position 6. As used herein, the term "position" refers to the position of the heterologous nucleotide sequence on the viral genome to be transcribed, *e.g.,* position 1 means that it is the first gene to be transcribed, and position 2 means that it is the second gene to be transcribed. Inserting heterologous nucleotide sequences at the lower-numbered positions of the virus generally results in stronger expression of the heterologous nucleotide sequence compared to insertion at higher-numbered positions due to a transcriptional gradient that occurs across the genome of the virus. However, the transcriptional gradient also yields specific ratios of viral mRNAs. Insertion of foreign genes will perturb these ratios and result in the synthesis of different amounts of viral proteins that may influence virus replication. Thus, both the transcriptional gradient and the replication kinetics must be considered when choosing an insertion site. For example, insertion of heterologous nucleotide sequence at position 2 of the b/h PIV3 vector results in the best replication rate and expression level of the heterologous gene. Inserting heterologous nucleotide sequences at lower-numbered positions is the preferred embodiment of the invention if strong expression of the heterologous nucleotide sequence is desired. In a preferred embodiment, the heterologous sequence is added or inserted at position 1, 2 or 3.

When inserting a heterologous nucleotide sequence into the virus of the invention, the intergenic region between the end of the coding sequence of the heterologous gene and the start of the coding sequence of the downstream gene can be altered to achieve a desired effect. As used herein, the term "intergenic region" refers to nucleotide sequence between the stop signal of one gene and the start codon (*e.g*., AUG) of the coding sequence of the next downstream open reading frame. An intergenic region may comprise a non-coding region of a gene, *i.e*., between the transcription start site and the start of the coding sequence (AUG) of the gene. This non-coding region occurs naturally in bPIV3 mRNAs and other viral genes, which is illustrated as non-limiting examples in Table 2:

**Table 2: Lengths of Non-coding Regions for bPIV3 mRNAs**

| ... CTT [Gene Start] | ........................................................... | AUG ... |
|---|---|---|
| N | 45 nucleotides | |
| P | 68 nucleotides | |
| M | 21 nucleotides | |
| F | 201 nucleotides | |
| HN | 62 nucleotides | |
| L | 12 nucleotides | |
| b/h RSV F1 | 10 nucleotides | |
| b/h RSV F2 | 86 nucleotides | |
| b/h RSV F1 NP-P | 83 nucleotides | |

In various embodiments, the intergenic region between the heterologous nucleotide sequence and the downstream gene can be engineered, independently from each other, to be at least 10 nt in length, at least 20 nt in length, at least 30 nt in length, at least 50 nt in length, at least 75 nt in length, at least 100 nt in length, at least 125 nt in length, at least 150 nt in length, at least 175 nt in length or at least 200 nt in length. In certain embodiments, the intergenic region between the heterologous nucleotide sequence and the downstream gene can be engineered, independently from each other, to be at most 10 nt in length, at most 20 nt in length, at most 30 nt in length, at most 50 nt in length, at most 75 nt in length, at most 100 nt in length, at most 125 nt in length, at most 150 nt in length, at most 175 nt in length or at most 200 nt in length. In various embodiments, the non-coding region of a desired gene in a virus genome can also be engineered, independently from each other, to be at least 10 nt in length, at least 20 nt in length, at least 30 nt in length, at least 50 nt in length, at least 75 nt in length, at least 100 nt in length, at least 125 nt in length, at least 150 nt in length, at least 175 nt in length or at least 200 nt in length. In certain embodiments, the non-coding region of a desired gene in a virus genome can also be engineered, independently from each other, to be at most 10 nt in length, at most 20 nt in length, at most 30 nt in length, at most 50 nt in length, at most 75 nt in length, at most 100 nt in length, at most 125 nt in length, at most 150 nt in length, at most 175 nt in length or at most 200 nt in length.

When inserting a heterologous nucleotide sequence, the positional effect and the intergenic region manipulation can be used in combination to achieve a desirable effect. For example, the heterologous nucleotide sequence can be added or inserted at a position selected from the group consisting of position 1, 2, 3, 4, 5, and 6, and the intergenic region between the heterologous nucleotide sequence and the next downstream gene can be altered (*see* Table 3). In an examplary embodiment, hRSV F gene is inserted at position 1of a b/h PIV3 vector, and the intergenic region between F gene and N gene (*i.e.,* the next downstream gene of F) is altered to 177 nucleotides. Many more combinations are encompassed by the present invention and some are shown by way of example in Table 3.

**Table 3. Examples of mode of insertion of heterologous nucleotide sequences**

| | Position 1 | Position 2 | Position 3 | Position 4 | Position 5 | Position 6 |
|---|---|---|---|---|---|---|
| IGR^{a} | 10-20 | 10-20 | 10-20 | 10-20 | 10-20 | 10-20 |
| IGR | 21-40 | 21-40 | 21-40 | 21-40 | 21-40 | 21-40 |
| IGR | 41-60 | 41-60 | 41-60 | 41-60 | 41-60 | 41-60 |
| IGR | 61-80 | 61-80 | 61-80 | 61-80 | 61-80 | 61-80 |
| IGR | 81-100 | 81-100 | 81-100 | 81-100 | 81-100 | 81-100 |
| IGR | 101-120 | 101-120 | 101-120 | 101-120 | 101-120 | 101-120 |
| IGR | 121-140 | 121-140 | 121-140 | 121-140 | 121-140 | 121-140 |
| IGR | 141-160 | 141-160 | 141-160 | 141-160 | 141-160 | 141-160 |
| IGR | 161-180 | 161-180 | 161-180 | 161-180 | 161-180 | 161-180 |
| IGR | 181-200 | 181-200 | 181-200 | 181-200 | 181-200 | 181-200 |
| IGR | 201-220 | 201-220 | 201-220 | 201-220 | 201-220 | 201-220 |
| IGR | 221-240 | 221-240 | 221-240 | 221-240 | 221-240 | 221-240 |
| IGR | 241-260 | 241-260 | 241-260 | 241-260 | 241-260 | 241-260 |
| IGR | 261-280 | 261-280 | 261-280 | 261-280 | 261-280 | 261-280 |
| IGR | 281-300 | 281-300 | 281-300 | 281-300 | 281-300 | 281-300 |

| | | | | | | |
|---|---|---|---|---|---|---|
| ^{a} Intergenic Region, measured in nucleotide. | | | | | | |

Depending on the purpose (*e.g*., to have strong immunogenicity) of the inserted heterologous nucleotide sequence, the position of the insertion and the length of the intergenic region of the inserted heterologous nucleotide sequence can be determined by various indexes including, but not limited to, replication kinetics and protein or mRNA expression levels, measured by following non-limiting examples of assays: plaque assay, fluorescent-focus assay, infectious center assay, transformation assay, endpoint dilution assay, efficiency of plating, electron microscopy, hemagglutination, measurement of viral enzyme activity, viral neutralization, hemagglutination inhibition, complement fixation, immunostaining, immunoprecipitation and immunoblotting, enzyme-linked immunosorbent assay, nucleic acid detection (*e.g*., Southern blot analysis, Northern blot analysis, Western blot analysis), growth curve, employment of a reporter gene (*e.g.,* using a reporter gene, such as Green Fluorescence Protein (GFP) or enhanced Green Fluorescence Protein (eGFP), integrated to the viral genome the same fashion as the interested heterologous gene to observe the protein expression), or a combination thereof. Procedures of performing these assays are well known in the art (*see, e.g.,* Flint et al., PRINCIPLES OF VIROLOGY, MOLECULAR BIOLOGY, PATHOGENESIS, AND CONTROL, 2000, ASM Press pp 25 - 56, the entire text is incorporated herein by reference), and non-limiting examples are given in the Example sections, *infra.*

For example, expression levels can be determined by infecting cells in culture with a virus of the invention and subsequently measuring the level of protein expression by, *e.g*., Western blot analysis or ELISA using antibodies specific to the gene product of the heterologous sequence, or measuring the level of RNA expression by, *e.g*., Northern blot analysis using probes specific to the heterologous sequence. Similarly, expression levels of the heterologous sequence can be determined by infecting an animal model and measuring the level of protein expressed from the heterologous sequence of the recombinant virus of the invention in the animal model. The protein level can be measured by obtaining a tissue sample from the infected animal and then subjecting the tissue sample to Western blot analysis or ELISA, using antibodies specific to the gene product of the heterologous sequence. Further, if an animal model is used, the titer of antibodies produced by the animal against the gene product of the heterologous sequence can be determined by any technique known to the skilled artisan, including but not limited to, ELISA.

As the heterologous sequences can be homologous to a nucleotide sequence in the genome of the virus, care should be taken that the probes and the antibodies are indeed specific to the heterologous sequence or its gene product.

In certain specific embodiments, expression levels of F-protein of RSV or hMPV from chimeric b/h PIV3 RSV or b/h PIV3 hMPV or b/h PIV3 RSV F and hMPV F can be determined by any technique known to the skilled artisan. Expression levels of the F-protein can be determined by infecting cells in a culture with the chimeric virus of the invention and measuring the level of protein expression by, *e.g*., Western blot analysis or ELISA using antibodies specific to the F-protein and/or the G-protein of hMPV, or measuring the level of RNA expression by, *e.g*., Northern blot analysis using probes specific to the F-gene and/or the G-gene of human metapneumovirus. Similarly, expression levels of the heterologous sequence can be determined using an animal model by infecting an animal and measuring the level of F-protein and/or G-protein in the animal model. The protein level can be measured by obtaining a tissue sample from the infected animal and then subjecting the tissue sample to Western blot analysis or ELISA using antibodies specific to F-protein and/or G-protein of the heterologous sequence. Further, if an animal model is used, the titer of antibodies produced by the animal against F-protein and/or G-protein can be determined by any technique known to the skilled artisan, including but not limited to, ELISA.

The rate of replication of a recombinant virus of the invention can be determined by any technique known to the skilled artisan.

In certain embodiments, to facilitate the identification of the optimal position of the heterologous sequence in the viral genome and the optimal length of the intergenic region, the heterologous sequence encodes a reporter gene. Once the optimal parameters are determined, the reporter gene is replaced by a heterologous nucleotide sequence encoding an antigen of choice. Any reporter gene known to the skilled artisan can be used with the methods of the invention. For more detail, see section 5.5.

The rate of replication of the recombinant virus can be determined by any standard technique known to the skilled artisan. The rate of replication is represented by the growth rate of the virus and can be determined by plotting the viral titer over the time post infection. The viral titer can be measured by any technique known to the skilled artisan. In certain embodiments, a suspension containing the virus is incubated with cells that are susceptible to infection by the virus. Cell types that can be used with the methods of the invention include, but are not limited to, Vero cells, LLC-MK-2 cells, Hep-2 cells, LF 1043 (HEL) cells, MRC-5 cells, WI-38 cells, 293 T cells, QT 6 cells, QT 35 cells, chicken embryo fibroblast (CEF), or tMK cells. Subsequent to the incubation of the virus with the cells, the number of infected cells is determined. In certain specific embodiments, the virus comprises a reporter gene. Thus, the number of cells expressing the reporter gene is representative of the number of infected cells. In a specific embodiment, the virus comprises a heterologous nucleotide sequence encoding for eGFP, and the number of cells expressing eGFP, *i.e.,* the number of cells infected with the virus, is determined using FACS.

In certain embodiments, the replication rate of the recombinant virus of the invention is at most 20 % of the replication rate of the wild type virus from which the recombinant virus is derived under the same conditions. The same conditions refer to the same initial titer of virus, the same strain of cells, the same incubation temperature, growth medium, number of cells and other test conditions that may affect the replication rate. For example, the replication rate of b/h PIV3 with RSV's F gene in position 1 is at most 20 % of the replication rate of bPIV3.

In certain embodiments, the replication rate of the recombinant virus of the invention is at most 5 %, at most 10 %, at most 20 %, at most 30 %, at most 40 %, at most 50 %, at most 75 %, at most 80 %, at most 90 % of the replication rate of the wild type virus from which the recombinant virus is derived under the same conditions. In certain embodiments, the replication rate of the recombinant virus of the invention is at least 5 %, at least 10 %, at least 20 %, at least 30 %, at least 40 %, at least 50 %, at least 75 %, at least 80 %, at least 90 % of the replication rate of the wild type virus from which the recombinant virus is derived under the same conditions. In certain embodiments, the replication rate of the recombinant virus of the invention is between 5 % and 20 %, between 10 % and 40 %, between 25 % and 50 %, between 40 % and 75 %, between 50 % and 80 %, or between 75 % and 90 % of the replication rate of the wild type virus from which the recombinant virus is derived under the same conditions.

In certain embodiments, the expression level of the heterologous sequence in the recombinant virus of the invention is at most 20 % of the expression level of the F-protein of the wild type virus from which the recombinant virus is derived under the same conditions. The same conditions refer to the same initial titer of virus, the same strain of cells, the same incubation temperature, growth medium, number of cells and other test conditions that may affect the replication rate. For example, the expression level of the heterologous sequence of the F-protein of MPV in position 1 of bPIV3 is at most 20 % of the expression level of the bovine F-protein of bPIV3.

In certain embodiments, the expression level of the heterologous sequence in the recombinant virus of the invention is at most 5 %, at most 10 %, at most 20 %, at most 30 %, at most 40 %, at most 50 %, at most 75 %, at most 80 %, at most 90 % of the expression level of the F-protein of the wild type virus from which the recombinant virus is derived under the same conditions. In certain embodiments, the expression level of the heterologous sequence in the recombinant virus of the invention is at least 5 %, at least 10 %, at least 20 %, at least 30 %, at least 40 %, at least 50 %, at least 75 %, at least 80 %, at least 90 % of the expression level of the F-protein of the wild type virus from which the recombinant virus is derived under the same conditions. In certain embodiments, the expression level of the heterologous sequence in the recombinant virus of the invention is between 5 % and 20 %, between 10 % and 40 %, between 25 % and 50 %, between 40 % and 75 %, between 50 % and 80 %, or between 75 % and 90 % of the expression level of the F-protein of the wild type virus from which the recombinant virus is derived under the same conditions.

### 5.1.4. INSERTION OF THE HETEROLOGOUS GENE SEQUENCE INTO THE HN GENE

The protein responsible for the hemagglutinin and neuraminidase activities of PIV are coded for by a single gene, HN. The HN protein is a major surface glycoprotein of the virus. For a variety of viruses, such as parainfluenza, the hemagglutinin and neuraminidase proteins have been shown to contain a number of antigenic sites. Consequently, this protein is a potential target for the humoral immune response after infection. Therefore, substitution of antigenic sites of HN with a portion of a foreign protein may provide for a vigorous humoral response against this foreign peptide. If a sequence is inserted within the HN molecule, and it is expressed on the outside surface of the HN, it will be immunogenic. For example, a peptide derived from gp160 ofHIV could replace an antigenic site of the HN protein, resulting in a humoral immune response to both gp160 and the HN protein. In a different approach, the foreign peptide sequence may be inserted within the antigenic site without deleting any viral sequences. Expression products of such constructs may be useful in vaccines against the foreign antigen, and may indeed circumvent a problem discussed earlier, that of propagation of the recombinant virus in the vaccinated host. An intact HN molecule with a substitution only in antigenic sites may allow for HN function and thus allow for the construction of a viable virus. Therefore, this virus can be grown without the need for additional helper functions. The virus may also be attenuated in other ways to avoid any danger of accidental escape.

Other hybrid constructions may be made to express proteins on the cell surface or enable them to be released from the cell. As a surface glycoprotein, HN has an amino-terminal cleavable signal sequence necessary for transport to the cell surface, and a carboxy-terminal sequence necessary for membrane anchoring. In order to express an intact foreign protein on the cell surface, it may be necessary to use these HN signals to create a hybrid protein. In this case, the fusion protein may be expressed as a separate fusion protein from an additional internal promoter. Alternatively, if only the transport signals are present and the membrane anchoring domain is absent, the protein may be secreted out of the cell.

### 5.1.5. CONSTRUCTION OF BICISTRONIC RNA

Bicistronic mRNA could be constructed to permit internal initiation of translation of viral sequences and allow for the expression of foreign protein coding sequences from the regular terminal initiation site. Alternatively, a bicistronic mRNA sequence may be constructed wherein the viral sequence is translated from the regular terminal open reading frame, while the foreign sequence is initiated from an internal site. Certain internal ribosome entry site (IRES) sequences may be utilized. The IRES sequences that are chosen should be short enough to avoid interference with parainfluenza packaging limitations. Thus, it is preferable that the IRES chosen for such a bicistronic approach be no more than 500 nucleotides in length, with less than 250 nucleotides being of ideal length. In a specific embodiment, the IRES is derived from a picornavirus and does not include any additional picornaviral sequences. Preferred IRES elements include, but are not limited to, the mammalian BiP IRES and the hepatitis C virus IRES.

Alternatively, a foreign protein may be expressed from a new internal transcriptional unit in which the transcriptional unit has an initiation site and polyadenylation site. In another embodiment, the foreign gene is inserted into a PIV gene such that the resulting expressed protein is a fusion protein.

### 5.2. EXPRESSION OF HETEROLOGOUS GENE PRODUCTS USING RECOMBINANT cDNA AND RNA TEMPLATES

The recombinant templates prepared as described above can be used in a variety of ways to express the heterologous gene products in appropriate host cells or to create chimeric viruses that express the heterologous gene products. In one embodiment, the recombinant cDNA can be used to transfect appropriate host cells and the resulting RNA may direct the expression of the heterologous gene product at high levels. Host cell systems which provide for high levels of expression include continuous cell lines that supply viral functions such as cell lines superinfected with PIV, cell lines engineered to complement PIV functions, etc.

In an alternate embodiment of the invention, the recombinant templates may be used to transfect cell lines that express a viral polymerase protein in order to achieve expression of the heterologous gene product. To this end, transformed cell lines that express a polymerase protein such as the L protein may be utilized as appropriate host cells. Host cells may be similarly engineered to provide other viral functions or additional functions such as HN, NP or N.

In another embodiment, a helper virus may provide the RNA polymerase protein utilized by the cells in order to achieve expression of the heterologous gene product. In yet another embodiment, cells may be transfected with vectors encoding viral proteins such as the N or NP, P, M2-1 and L proteins.

Different technique may be used to detect the expression of heterologous gene products (*see, e.g.,* Flint et al., PRINCIPLES OF VIROLOGY, MOLECULAR BIOLOGY, PATHOGENESIS, AND CONTROL, 2000, ASM Press pp 25 - 56, the entire text is incorporated herein by reference). In an examplary assay, cells infected with the virus are permeabilized with methanol or acetone and incubated with an antibody raised against the heterologous gene products. A second antibody that recognizes the first antibody is then added. This second antibody is usually conjugated to an indicator so that the expression of heterologous gene products may be visualized or detected.

### 5.3. RESCUE OF RECOMBINANT VIRUS PARTICLES

In order to prepare chimeric virus, modified cDNAs, virus RNAs, or RNA coding for the PIV genome and/or foreign proteins in the plus or minus sense may be used to transfect cells that provide viral proteins and functions required for replication and rescue. Alternatively, cells may be transfected with helper virus before, during, or after transfection by the DNA or RNA molecule coding for the PIV genome and/or foreign proteins. The synthetic recombinant plasmid PIV DNAs and RNAs can be replicated and rescued into infectious virus particles by any number of techniques known in the art, as described in U.S. Patent No. 5,166,057 issued November 24, 1992; in U.S. Patent No. 5,854,037 issued December 29, 1998; in European Patent Publication EP 0702085A1, published February 20, 1996; in U.S. Patent Application Serial No. 09/152,845; in International Patent Publications PCT WO97/12032 published April 3, 1997; WO96/34625 published November 7, 1996; in European Patent Publication EP-A780475; WO 99/02657 published January 21, 1999; WO 98/53078 published November 26, 1998; WO 98/02530 published January 22, 1998; WO 99/15672 published April 1, 1999; WO 98/13501 published April 2, 1998; WO 97/06270 published February 20, 1997; and EPO 780 47SA1 published June 25, 1997, each of which is incorporated by reference herein in its entirety.

In one embodiment of the present invention, synthetic recombinant viral RNAs that contain the non-coding regions of the negative strand virus RNA essential for the recognition by viral polymerases and for packaging signals necessary to generate a mature virion, may be prepared. There are a number of different approaches that may be used to apply the reverse genetics approach to rescue negative strand RNA viruses. First, the recombinant RNAs are synthesized from a recombinant DNA template and reconstituted *in vitro* with purified viral polymerase complex to form recombinant ribonucleoproteins (RNPs) that can be used to transfect cells. In another approach, a more efficient transfection is achieved if the viral polymerase proteins are present during transcription of the synthetic RNAs either *in vitro* or *in vivo.* With this approach the synthetic RNAs may be transcribed from cDNA plasmids that are either co-transcribed *in vitro* with cDNA plasmids encoding the polymerase proteins, or transcribed *in vivo* in the presence of polymerase proteins, *i.e.,* in cells which transiently or constitutively express the polymerase proteins.

In additional approaches described herein, the production of infectious chimeric virus may be replicated in host cell systems that express a PIV viral polymerase protein (*e.g*., in virus/host cell expression systems; transformed cell lines engineered to express a polymerase protein, etc.), so that infectious chimeric viruses are rescued. In this instance, helper virus need not be utilized since this function is provided by the viral polymerase proteins expressed.

In accordance with the present invention, any technique known to those of skill in the art may be used to achieve replication and rescue of recombinant and chimeric viruses. One approach involves supplying viral proteins and functions required for replication *in vitro* prior to transfecting host cells. In such an embodiment, viral proteins may be supplied in the form of wild type virus, helper virus, purified viral proteins or recombinantly expressed viral proteins. The viral proteins may be supplied prior to, during or post transcription of the synthetic cDNAs or RNAs encoding the chimeric virus. The entire mixture may be used to transfect host cells. In another approach, viral proteins and functions required for replication may be supplied prior to or during transcription of the synthetic cDNAs or RNAs encoding the chimeric virus. In such an embodiment, viral proteins and functions required for replication are supplied in the form of wild type virus, helper virus, viral extracts, synthetic cDNAs or RNAs that express the viral proteins are introduced into the host cell via infection or transfection. This infection/transfection takes place prior to or simultaneous to the introduction of the synthetic cDNAs or RNAs encoding the chimeric virus.

In a particularly desirable approach, cells engineered to express all viral genes of the recombinant or chimeric virus of the invention may result in the production of infectious chimeric virus that contain the desired genotype; thus eliminating the need for a selection system. Theoretically, one can replace any one of the six genes or part of any one of the six genes encoding structural proteins of PIV with a foreign sequence. However, a necessary part of this equation is the ability to propagate the defective virus (defective because a normal viral gene product is missing or altered). A number of possible approaches are available to circumvent this problem. In one approach, a virus having a mutant protein can be grown in cell lines that are constructed to constitutively express the wild type version of the same protein. By this way, the cell line complements the mutation in the virus. Similar techniques may be used to construct transformed cell lines that constitutively express any of the PIV genes. These cell lines which are made to express the viral protein may be used to complement the defect in the recombinant virus and thereby propagate it. Alternatively, certain natural host range systems may be available to propagate recombinant virus.

In yet another embodiment, viral proteins and functions required for replication may be supplied as genetic material in the form of synthetic cDNAs or RNAs so that they are co-transcribed with the synthetic cDNAs or RNAs encoding the chimeric virus. In a particularly desirable approach, plasmids that express the chimeric virus and the viral polymerase and/or other viral functions are co-transfected into host cells. For example, plasmids encoding the genomic or antigenomic PIV RNA, either wild type or modified, may be co-transfected into host cells with plasmids encoding the PIV viral polymerase proteins NP or N, P, M2-1 or L. Alternatively, rescue of chimeric b/h PIV3 virus may be accomplished by the use of Modified Vaccinia Virus Ankara (MVA) encoding T7 RNA polymerase, or a combination of MVA and plasmids encoding the polymerase proteins (N, P, and L). For example, MVA-T7 or Fowl Pox-T7 can be infected into Vero cells, LLC-MK-2 cells, Hep-2 cells, LF 1043 (HEL) cells, tMK cells, LLC-MK2, HUT 292, FRHL-2 (rhesus), FCL-1 (green monkey), WI-38 (human), MRC-5 (human) cells, 293 T cells, QT 6 cells, QT 35 cells and CEF cells. After infection with MVA-T7 or Fow Pox-T7, a full length antigenomic b/h PIV3 cDNA may be transfected into the HeLa or Vero cells together with the NP, P, M2-1 and L encoding expression plasmids. Alternatively, the polymerase may be provided by plasmid transfection. The cells and cell supernatant can subsequently be harvested and subjected to a single freeze-thaw cycle. The resulting cell lysate may then be used to infect a fresh HeLa or Vero cell monolayer in the presence of 1-beta-D-arabinofuranosylcytosine (ara C), a replication inhibitor of vaccinia virus, to generate a virus stock. The supernatant and cells from these plates can then be harvested, freeze-thawed once, and the presence of bPIV3 virus particles detected by immunostaining of virus plaques using PIV3-specific antiserum.

Another approach to propagating the recombinant virus involves co-cultivation with wild-type virus. This could be done by simply taking recombinant virus and co-infecting cells with this and another wild-type virus (preferably a vaccine strain). The wild-type virus should complement for the defective virus gene product and allow growth of both the wild-type and recombinant virus. Alternatively, a helper virus may be used to support propagation of the recombinant virus.

In another approach, synthetic templates may be replicated in cells co-infected with recombinant viruses that express the PIV virus polymerase protein. In fact, this method may be used to rescue recombinant infectious virus in accordance with the invention. To this end, the PIV polymerase protein may be expressed in any expression vector/host cell system, including but not limited to viral expression vectors (*e.g.,* vaccinia virus, adenovirus, baculovirus, etc.) or cell lines that express a polymerase protein (*e.g*., see Krystal et al., 1986, Proc. Natl. Acad. Sci. USA 83: 2709-2713). Moreover, infection of host cells expressing all six PIV proteins may result in the production of infectious chimeric virus particles. It should be noted that it may be possible to construct a recombinant virus without altering virus viability. These altered viruses would then be growth competent and would not need helper functions to replicate.

### 5.4. ATTENUATION OF RECOMBINANT VIRUSES

The recombinant viruses of the invention can be further genetically engineered to exhibit an attenuated phenotype. In particular, the recombinant viruses of the invention exhibit an attenuated phenotype in a subject to which the virus is administered as a vaccine. Attenuation can be achieved by any method known to a skilled artisan. Without being bound by theory, the attenuated phenotype of the recombinant virus can be caused, *e.g*., by using a virus that naturally does not replicate well in an intended host (*e.g*., using a bovine PIV3 vector in human), by reduced replication of the viral genome, by reduced ability of the virus to infect a host cell, or by reduced ability of the viral proteins to assemble to an infectious viral particle relative to the wild type strain of the virus. The viability of certain sequences of the virus, such as the leader and the trailer sequence can be tested using a minigenome assay (*see* section 5.5.1).

The attenuated phenotypes of a recombinant virus of the invention can be tested by any method known to the artisan (see, *e.g.,* section 5.5). A candidate virus can, for example, be tested for its ability to infect a host or for the rate of replication in a cell culture system. In certain embodiments, a mini-genome system is used to test the attenuated virus when the gene that is altered is N, P, L, M2 or a combination thereof. In certain embodiments, growth curves at different temperatures are used to test the attenuated phenotype of the virus. For example, an attenuated virus is able to grow at 35°C, but not at 39°C or 40°C. In certain embodiments, different cell lines can be used to evaluate the attenuated phenotype of the virus. For example, an attenuated virus may only be able to grow in monkey cell lines but not the human cell lines, or the achievable virus titers in different cell lines are different for the attenuated virus. In certain embodiments, viral replication in the respiratory tract of a small animal model, including but not limited to, hamsters, cotton rats, mice and guinea pigs, is used to evaluate the attenuated phenotypes of the virus. In other embodiments, the immune response induced by the virus, including but not limited to, the antibody titers (*e.g*., assayed by plaque reduction neutralization assay or ELISA) is used to evaluate the attenuated phenotypes of the virus. In a specific embodiment, the plaque reduction neutralization assay or ELISA is carried out at a low dose. In certain embodiments, the ability of the recombinant virus to elicit pathological symptoms in an animal model can be tested. A reduced ability of the virus to elicit pathological symptoms in an animal model system is indicative of its attenuated phenotype. In a specific embodiment, the candidate viruses are tested in a monkey model for nasal infection, indicated by mucous production.

The viruses of the invention can be attenuated such that one or more of the functional characteristics of the virus are impaired. In certain embodiments, attenuation is measured in comparison to the wild type strain of the virus from which the attenuated virus is derived. In other embodiments, attenuation is determined by comparing the growth of an attenuated virus in different host systems. Thus, for a non-limiting example, a bovine PIV3 is said to be attenuated when grown in a human host if the growth of the bovine PIV3 in the human host is reduced compared to the growth of the bovine PIV3 in a bovine host.

In certain embodiments, the attenuated virus of the invention is capable of infecting a host, is capable of replicating in a host such that infectious viral particles are produced. In comparison to the wild type strain, however, the attenuated strain grows to lower titers or grows more slowly. Any technique known to the skilled artisan can be used to determine the growth curve of the attenuated virus and compare it to the growth curve of the wild type virus. For exemplary methods see Example section, *infra.* In a specific embodiment, the attenuated virus grows to a titer of less than 10⁵ pfu/ml, of less than 10⁴ pfu/ml, of less than 10³ pfu/ml, or of less than 10² pfu/ml in Vero cells under conditions as described.

In certain embodiments, the attenuated virus of the invention (*e.g*., a chimeric PIV3) cannot replicate in human cells as well as the wild type virus (*e.g*., wild type PIV3) does. However, the attenuated virus can replicate well in a cell line that lack interferon functions, such as Vero cells.

In other embodiments, the attenuated virus of the invention is capable of infecting a host, of replicating in the host, and of causing proteins of the virus of the invention to be inserted into the cytoplasmic membrane, but the attenuated virus does not cause the host to produce new infectious viral particles. In certain embodiments, the attenuated virus infects the host, replicates in the host, and causes viral proteins to be inserted in the cytoplasmic membrane of the host with the same efficiency as the wild type mammalian virus. In other embodiments, the ability of the attenuated virus to cause viral proteins to be inserted into the cytoplasmic membrane into the host cell is reduced compared to the wild type virus. In certain embodiments, the ability of the attenuated mammalian virus to replicate in the host is reduced compared to the wild type virus. Any technique known to the skilled artisan can be used to determine whether a virus is capable of infecting a mammalian cell, of replicating within the host, and of causing viral proteins to be inserted into the cytoplasmic membrane of the host. For illustrative methods see section 5.5.

In certain embodiments, the attenuated virus of the invention is capable of infecting a host. In contrast to a wild type PIV, however, the attenuated PIV cannot be replicated in the host. In a specific embodiment, the attenuated virus can infect a host and can cause the host to insert viral proteins in its cytoplasmic membranes, but the attenuated virus is incapable of being replicated in the host. Any method known to the skilled artisan can be used to test whether the attenuated virus has infected the host and has caused the host to insert viral proteins in its cytoplasmic membranes.

In certain embodiments, the ability of the attenuated mammalian virus to infect a host is reduced compared to the ability of the wild type virus to infect the same host. Any technique known to the skilled artisan can be used to determine whether a virus is capable of infecting a host. For illustrative methods see section 5.5.

In certain embodiments, mutations (*e.g*., missense mutations) are introduced into the genome of the virus to generated a virus with an attenuated phenotype. Mutations (*e.g*., missense mutations) can be introduced into the N-gene, the P-gene, the F-gene, the M2-gene, the M2-1-gene, the M2-2-gene, the SH-gene, the G-gene or the L-gene of the recombinant virus. Mutations can be additions, substitutions, deletions, or combinations thereof. In specific embodiments, a single amino acid deletion mutation for the N, P, L or M2 proteins are introduced, which can be screened for functionality in the mini-genome assay system and be evaluated for predicted functionality in the virus. In more specific embodiments, the missense mutation is a cold-sensitive mutation. In other embodiments, the missense mutation is a heat-sensitive mutation. In one embodiment, major phosphorylation sites of P protein of the virus is removed. In another embodiment, a mutation or mutations are introduced into the L gene of the virus to generate a temperature sensitive strain. In yet another embodiment, the cleavage site of the F gene is mutated in such a way that cleavage does not occur or occurs at very low efficiency.

In other embodiments, deletions are introduced into the genome of the recombinant virus. In more specific embodiments, a deletion can be introduced into the N-gene, the P-gene, the F-gene, the M2-gene, the M2-1-gene, the M2-2-gene, the SH-gene, the G-gene or the L-gene of the recombinant virus. In specific embodiments, the deletion is in the M2-gene of the recombinant virus of the present invention. In other specific embodiments, the deletion is in the SH-gene of the recombinant virus of the present invention. In yet another specific embodiment, both the M2-gene and the SH-gene are deleted.

In certain embodiments, the intergenic region of the recombinant virus is altered. In one embodiment, the length of the intergenic region is altered. See Section 5.1.2. for illustrative examples. In another embodiment, the intergenic regions are shuffled from 5' to 3' end of the viral genome.

In other embodiments, the genome position of a gene or genes of the recombinant virus is changed. In one embodiment, the F or G gene is moved to the 3' end of the genome. In another embodiment, the N gene is moved to the 5' end of the genome.

In certain embodiments, attenuation of the virus is achieved by replacing a gene of the wild type virus with a gene of a virus of a different species. In illustrative embodiments, the N-gene, the P-gene, the F-gene, the M2-gene, the M2-1-gene, the M2-2-gene, the SH-gene, the HN-gene or the L-gene of bPIV3 is replaced with the N-gene, the P-gene, the F-gene, the M2-gene, the M2-1-gene, the M2-2-gene, the SH-gene, the HN-gene or the L-gene, respectively, of hPIV3. In other illustrative embodiments, the N-gene, the P-gene, the F-gene, the M2-gene, the M2-1-gene, the M2-2-gene, the SH-gene, the HN-gene or the L-gene of hPIV3 is replaced with the N-gene, the P-gene, the F-gene, the M2-gene, the M2-1-gene, the M2-2-gene, the SH-gene, the HN-gene or the L-gene, respectively, of bPIV3. In a preferred embodiment, attenuation of the virus is achieved by replacing one or more polymerase associated genes (*e.g*., N, P, L or M2) with genes of a virus of a different species.

In certain embodiments, attenuation of the virus is achieved by replacing or deleting one or more specific domains of a protein of the wild type virus with domains derived from the corresponding protein of a virus of a different species. In an illustrative embodiment, the ectodomain of a F protein of bPIV3 is replaced with an ectodomain of a F protein of a metapneumovirus. In a preferred embodiment, one or more specific domains of L, N, or P protein are replaced with domains derived from corresponding proteins of a virus of a different species. In another illustrative embodiment, the transmembrane domain of the F protein is deleted so that a soluble F protein is expressed.

In certain embodiments of the invention, the leader and/or trailer sequence of the recombinant virus of the invention can be modified to achieve an attenuated phenotype. In certain, more specific embodiments, the leader and/or trailer sequence is reduced in length relative to the wild type virus by at least 1 nucleotide, at least 2 nucleotides, at least 3 nucleotides, at least 4 nucleotides, at least 5 nucleotides or at least 6 nucleotides. In certain other, more specific embodiments, the sequence of the leader and/or trailer of the recombinant virus is mutated. In a specific embodiment, the leader and the trailer sequence are 100% complementary to each other. In other embodiments, 1 nucleotide, 2 nucleotides, 3 nucleotides, 4 nucleotides, 5 nucleotides, 6 nucleotides, 7 nucleotides, 8 nucleotides, 9 nucleotides, or 10 nucleotides are not complementary to each other where the remaining nucleotides of the leader and the trailer sequences are complementary to each other. In certain embodiments, the non-complementary nucleotides are identical to each other. In certain other embodiments, the non-complementary nucleotides are different from each other. In other embodiments, if the non-complementary nucleotide in the trailer is purine, the corresponding nucleotide in the leader sequence is also a purine. In other embodiments, if the non-complementary nucleotide in the trailer is pyrimidine, the corresponding nucleotide in the leader sequence is also a purine.

When a live attenuated vaccine is used, its safety must also be considered. The vaccine must not cause disease. Any techniques known in the art that can make a vaccine safe may be used in the present invention. In addition to attenuation techniques, other techniques may be used. One non-limiting example is to use a soluble heterologous gene that cannot be incorporated into the virion membrane. For example, a single copy of the soluble RSV F gene, a version of the RSV gene lacking the transmembrane and cytosolic domains, can be used. Since it cannot be incorporated into the virion membrane, the virus tropism is not expected to change.

Various assays can be used to test the safety of a vaccine. *See* section 5.5., *infra.* Particularly, sucrose gradients and neutralization assays can be used. A sucrose gradient assay can be used to determine whether a heterologous protein is inserted in a virion. If the heterologous protein is inserted in the virion, the virion should be tested for its ability to cause symptoms even if the parental strain does not cause symptoms. Without bound by theory, if the heterologous protein is incorporated in the virion, the virus may have acquired new, possibly pathological, properties.

### 5.5. MEASUREMENT OF VIRAL TITER, EXPRESSION OF ANTIGENIC SEQUENCES, IMMUNOGENICITY AND OTHER CHARACTERISTICS OF CHIMERIC VIRUSES

A number of assays may be employed in accordance with the present invention in order to determine the rate of growth of a chimeric or recombinant virus in a cell culture system, an animal model system or in a subject. A number of assays may also be employed in accordance with the present invention in order to determine the requirements of the chimeric and recombinant viruses to achieve infection, replication and packaging of virions.

The assays described herein may be used to assay viral titre over time to determine the growth characteristics of the virus. In a specific embodiment, the viral titre is determined by obtaining a sample from the infected cells or the infected subject, preparing a serial dilution of the sample and infecting a monolayer of cells that are susceptible to infection with the virus at a dilution of the virus that allows for the emergence of single plaques. The plaques can then be counted and the viral titre express as plaque forming units per milliliter of sample. In a specific embodiment of the invention, the growth rate of a virus of the invention in a subject is estimated by the titer of antibodies against the virus in the subject. Without being bound by theory, the antibody titer in the subject reflects not only the viral titer in the subject but also the antigenicity. If the antigenicity of the virus is constant, the increase of the antibody titer in the subject can be used to determine the growth curve of the virus in the subject. In a preferred embodiment, the growth rate of the virus in animals or humans is best tested by sampling biological fluids of a host at multiple time points post-infection and measuring viral titer.

The expression of heterologous gene sequence in a cell culture system or in a subject can be determined by any technique known to the skilled artisan. In certain embodiments, the expression of the heterologous gene is measured by quantifying the level of the transcript. The level of the transcript can be measured by Northern blot analysis or by RT-PCR using probes or primers, respectively, that are specific for the transcript. The transcript can be distinguished from the genome of the virus because the virus is in the antisense orientation whereas the transcript is in the sense orientation. In certain embodiments, the expression of the heterologous gene is measured by quantifying the level of the protein product of the heterologous gene. The level of the protein can be measured by Western blot analysis using antibodies that are specific to the protein.

In a specific embodiment, the heterologous gene is tagged with a peptide tag. The peptide tag can be detected using antibodies against the peptide tag. The level of peptide tag detected is representative for the level of protein expressed from the heterologous gene. Alternatively, the protein expressed from the heterologous gene can be isolated by virtue of the peptide tag. The amount of the purified protein correlates with the expression level of the heterologous gene. Such peptide tags and methods for the isolation of proteins fused to such a peptide tag are well known in the art. A variety of peptide tags known in the art may be used in the modification of the heterologous gene, such as, but not limited to, the immunoglobulin constant regions, polyhistidine sequence (Petty, 1996, Metal-chelate affinity chromatography, in Current Protocols in Molecular Biology, volume 1-3 (1994-1998). Ed. by Ausubel, F.M., Brent, R., Kunston, R.E., Moore, D.D., Seidman, J.G., Smith, J.A. and Struhl, K. Published by John Wiley and sons, Inc., USA, Greene Publish. Assoc. & Wiley Interscience), glutathione S-transferase (GST; Smith, 1993, Methods Mol. Cell Bio. 4:220-229), the *E. coli* maltose binding protein (Guan et al., 1987, Gene 67:21-30), various cellulose binding domains (U.S. patent 5,496,934; 5,202,247; 5,137,819; Tomme et al., 1994, Protein Eng. 7:117-123), and the FLAG epitope (Short Protocols in Molecular Biology, 1999, Ed. Ausubel et al., John Wiley & Sons, Inc., Unit 10.11) etc. Other peptide tags are recognized by specific binding partners and thus facilitate isolation by affinity binding to the binding partner, which is preferably immobilized and/or on a solid support. As will be appreciated by those skilled in the art, many methods can be used to obtain the coding region of the above-mentioned peptide tags, including but not limited to, DNA cloning, DNA amplification, and synthetic methods. Some of the peptide tags and reagents for their detection and isolation are available commercially.

Samples from a subject can be obtained by any method known to the skilled artisan. In certain embodiments, the sample consists of nasal aspirate, throat swab, sputum or broncho-alveolar lavage.

### 5.5.1. MINIGENOME CONSTRUCTS

Minireplicon constructs can be generated to contain an antisense reporter gene. Any reporter gene known to the skilled artisan can be used with the invention. In a specific embodiment, the reporter gene is CAT. In certain embodiments, the reporter gene can be flanked by the negative-sense bPIV or hPIV leader linked to the hepatitis delta ribozyme (Hep-d Ribo) and T7 polymerase termination (T-T7) signals, and the bPIV or hPIV trailer sequence preceded by the T7 RNA polymerase promoter.

In certain embodiments, the plasmid encoding the minireplicon is transfected into a host cell. The host cell expresses T7 RNA polymerase, the N gene, the P gene, the L gene, and the M2.1 gene. In certain embodiments, the host cell is transfected with plasmids encoding T7 RNA polymerase, the N gene, the P gene, the L gene, and the M2.1 gene. In other embodiments, the plasmid encoding the minireplicon is transfected into a host cell and the host cell is infected with a helper virus.

The expression level of the reporter gene and/or its activity can be assayed by any method known to the skilled artisan, such as, but not limited to, the methods described in section 5.5.6.

In certain, more specific, embodiments, the minireplicon comprises the following elements, in the order listed: T7 RNA Polymerase or RNA polymerase I, leader sequence, gene start, GFP, trailer sequence, Hepatitis delta ribozyme sequence or RNA polymerase I termination sequence. If T7 is used as RNA polymerase, Hepatitis delta ribozyme sequence should be used as termination sequence. If RNA polymerase I is used, RNA polymerase I termination sequence may be used as a termination signal. Dependent on the rescue system, the sequence of the minireplicon can be in the sense or antisense orientation. In certain embodiments, the leader sequence can be modified relative to the wild type leader sequence of the virus of the invention. The leader sequence can optionally be preceded by an AC. The T7 promoter sequence can be with or without a G-doublet or triplet, where the G-doublet or triplet provides for increased transcription.

In a specific embodiment, a cell is infected with a virus of the invention at T0. 24 hours later, at T24, the cell is transfected with a minireplicon construct. 48 hours after T0 and 72 hours after T0, the cells are tested for the expression of the reporter gene. If a fluorescent reporter gene product is used (*e.g*., GFP), the expression of the reporter gene can be tested using FACS.

In another embodiment, a cell is transfected with six plasmids at T=0 hours. Cells are then harvested at T=40 hours and T=60 hours and analyzed for CAT or GFP expression.

In another specific embodiment, a cell is infected with MVA-T7 at T0. 1 hour later, at T1, the cell is transfected with a minireplicon construct. 24 hours after T0, the cell is infected with a virus of the invention. 72 hours after T0, the cells are tested for the expression of the reporter gene. If a fluorescent reporter gene product is used (*e.g*., GFP), the expression of the reporter gene can be tested using FACS.

### 5.5.2. MEASUREMENT OF INCIDENCE OF INFECTION RATE

The incidence of infection can be determined by any method well-known in the art, including but not limited to, the testing of clinical samples (*e.g*., nasal swabs) for the presence of an infection, *e.g*., hMPV, RSV, hPIV, or bPIV/hPIV components can be detected by immunofluorescence assay (IFA) using an anti-hMPV-antigen antibody, an anti-RSV-antigen antibody, an anti-hPIV-antigen antibody, and/or an antibody that is specific to the gene product of the heterologous nucleotide sequence, respectively.

In certain embodiments, samples containing intact cells can be directly processed, whereas isolates without intact cells should first be cultured on a permissive cell line (*e.g*. HEp-2 cells). In an illustrative embodiment, cultured cell suspensions are cleared by centrifugation at, *e.g.,* 300xg for 5 minutes at room temperature, followed by a PBS, pH 7.4 (Ca++ and Mg++ free) wash under the same conditions. Cell pellets are resuspended in a small volume of PBS for analysis. Primary clinical isolates containing intact cells are mixed with PBS and centrifuged at 300xg for 5 minutes at room temperature. Mucus is removed from the interface with a sterile pipette tip and cell pellets are washed once more with PBS under the same conditions. Pellets are then resuspended in a small volume of PBS for analysis. Five to ten microliters of each cell suspension are spotted per 5 mm well on acetone washed 12-well HTC supercured glass slides and allowed to air dry. Slides are fixed in cold (-20°C) acetone for 10 minutes. Reactions are blocked by adding PBS - 1% BSA to each well followed by a 10 minute incubation at room temperature. Slides are washed three times in PBS - 0.1% Tween-20 and air dried. Ten microliters of each primary antibody reagent diluted to 250 ng/ml in blocking buffer is spotted per well and reactions are incubated in a humidified 37°C environment for 30 minutes. Slides are then washed extensively in three changes of PBS - 0.1 % Tween-20 and air dried. Ten microliters of appropriate secondary conjugated antibody reagent diluted to 250 ng/ml in blocking buffer are spotted per respective well and reactions are incubated in a humidified 37°C environment for an additional 30 minutes. Slides are then washed in three changes of PBS - 0.1% Tween-20. Five microliters of PBS-50% glycerol-10 mM Tris pH 8.0-1 mM EDTA are spotted per reaction well and slides are mounted with cover slips. Each reaction well is subsequently analyzed by fluorescence microscopy at 200X power using a B-2A filter (EX 450-490 nm). Positive reactions are scored against an autofluorescent background obtained from unstained cells or cells stained with secondary reagent alone. RSV positive reactions are characterized by bright fluorescence punctuated with small inclusions in the cytoplasm of infected cells.

### 5.5.3. MEASUREMENT OF SERUM TITER

Antibody serum titer can be determined by any method well-known in the art, for example, but not limited to, the amount of antibody or antibody fragment in serum samples can be quantitated by a sandwich ELISA. Briefly, the ELISA consists of coating microtiter plates overnight at 4°C with an antibody that recognizes the antibody or antibody fragment in the serum. The plates are then blocked for approximately 30 minutes at room temperature with PBS-Tween-0.5% BSA. Standard curves are constructed using purified antibody or antibody fragment diluted in PBS-BSA-BSA, and samples are diluted in PBS-BSA. The samples and standards are added to duplicate wells of the assay plate and are incubated for approximately 1 hour at room temperature. Next, the non-bound antibody is washed away with PBS-TWEEN and the bound antibody is treated with a labeled secondary antibody (e.g., horseradish peroxidase conjugated goat-anti-human IgG) for approximately 1 hour at room temperature. Binding of the labeled antibody is detected by adding a chromogenic substrate specific for the label and measuring the rate of substrate turnover, e.g., by a spectrophotometer. The concentration of antibody or antibody fragment levels in the serum is determined by comparison of the rate of substrate turnover for the samples to the rate of substrate turnover for the standard curve.

### 5.5.4. CHALLENGE STUDIES

This assay is used to determine the ability of the recombinant viruses of the invention and of the vaccines of the invention to prevent lower respiratory tract viral infection in an animal model system, including but not limited to, cotton rats, Syrian Golden hamsters, and Balb/c mice. The recombinant virus and/or the vaccine can be administered by intravenous (IV) route, by intramuscular (IM) route or by intranasal route (IN). The recombinant virus and/or the vaccine can be administered by any technique well-known to the skilled artisan. This assay is also used to correlate the serum concentration of antibodies with a reduction in lung titer of the virus to which the antibodies bind.

On day 0, groups of animals, including but not limited to, cotton rats (*Sigmodon hispidis,* average weight 100 g) and hamsters (*e.g*., Syrian Golden hamsters) are inoculated with the recombinant virus or the vaccine of interest or BSA by intramuscular injection, by intravenous injection, or by intranasal route. Prior to, concurrently with, or subsequent to administration of the recombinant virus or the vaccine of the invention, the animals are infected with wild type virus wherein the wild type virus is the virus against which the vaccine was generated. In certain embodiments, the animals are infected with the wild type virus at least 1 day, at least 2 days, at least 3 days, at least 4 days, at least 5 days, at least 6 days, at least 1 week, at least 2 weeks, at least 3 weeks or at least 4 weeks subsequent to the administration of the recombinant virus and/or the vaccine of the invention. In a preferred embodiment, the animals are infected with the wild type virus 21 days subsequent to the administration of the recombinant virus and/or the vaccine of the invention. In another preferred embodiment, the animals are infected with the wild type virus 28 days subsequent to the administration of the recombinant virus and/or the vaccine of the invention.

After the infection, the animals are sacrificed, and their nasal turbinate tissue and/or lung tissue are harvested and virus titers are determined by appropriate assays, *e.g*., plaque assay and TCID₅₀ assay. Bovine serum albumin (BSA) 10 mg/kg can be used as a negative control. Antibody concentrations in the serum at the time of challenge can be determined using a sandwich ELISA.

### 5.5.5. CLINICAL TRIALS

Vaccines of the invention or fragments thereof that have been tested in *in vitro* assays and animal models may be further evaluated for safety, tolerance, immunogenicity, infectivity and pharmacokinetics in groups of normal healthy human volunteers, including all age groups. In a preferred embodiment, the healthy human volunteers are infants at about 6 weeks of age or older, children and adults. The volunteers are administered intranasally, intramuscularly, intravenously or by a pulmonary delivery system in a single dose of a recombinant virus of the invention and/or a vaccine of the invention. Multiple doses of virus and/or vaccine of the invention may be required in seronegative children 6 to 60 months of age. Multiple doses of virus and/or vaccine of the invention may also be required in the first six months of life to stimulate local and systemic immunity and to overcome neutralization by maternal antibody. In a preferred embodiment, a primary dosing regimen at 2, 4, and 6 months of age and a booster dose at the beginning of the second year of life are used. A recombinant virus of the invention and/or a vaccine of the invention can be administered alone or concurrently with pediatric vaccines recommended at the corresponding ages.

In a preferred embodiment, double-blind randomized, placebo-controlled clinical trials are used. In a specific embodiment, a computer generated randomization schedule is used. For example, each subject in the study will be enrolled as a single unit and assigned a unique case number. Multiple subjects within a single family will be treated as individuals for the purpose of enrollment. Parent/guardian, subjects, and investigators will remain blinded to which treatment group subjects have been assigned for the duration of the study. Serologic and virologic studies will be performed by laboratory personnel blinded to treatment group assignment. However, it is expected that isolation of the vaccine virus from nasal wash fluid obtained after vaccination will identify likely vaccinees to the virology laboratory staff. The serologic and virologic staff are separate and the serology group will be prevented from acquiring any knowledge of the culture results.

Each volunteer is preferably monitored for at least 12 hours prior to receiving the recombinant virus of the invention and/or a vaccine of the invention, and each volunteer will be monitored for at least fifteen minutes after receiving the dose at a clinical site. Then volunteers are monitored as outpatients on days 1-14, 21, 28, 35, 42, 49, and 56 postdose. In a preferred embodiment, the volunteers are monitored for the first month after each vaccination as outpatients. All vaccine related serious adverse events will be reported for the entire duration of the trial. A serious adverse event is defined as an event that 1) results in death, 2) is immediately life threatening, 3) results in permanent or substantial disability, 4) results in or prolongs an existing in-patient hospitalization, 5) results in a congenital anomaly, 6) is a cancer, or 7) is the result of an overdose of the study vaccine. Serious adverse events that are not vaccine related will be reported beginning on the day of the first vaccination (Day 0) and continue for 30 days following the last vaccination. Non-vaccine related serious adverse events will not be reported for 5 to 8 months after the 30 day reporting period following the last vaccination. A dose of vaccine/placebo will not be given if a child has a vaccine-related serous adverse event following the previous dose. Any adverse event that is not considered vaccine related, but which is of concern, will be discussed by the clinical study monitor and the medical monitor before the decision to give another dose is made.

Blood samples are collected via an indwelling catheter or direct venipuncture (*e.g*., by using 10 ml red-top Vacutainer tubes) at the following intervals: (1) prior to administering the dose of the recombinant virus of the invention and/or a vaccine of the invention; (2) during the administration of the dose of the recombinant virus of the invention and/or a vaccine of the invention; (3) 5 minutes, 10 minutes, 15 minutes, 20 minutes, 30 minutes, 1 hour, 2 hours, 4 hours, 8 hours, 12 hours, 24 hours, and 48 hours after administering the dose of the recombinant virus of the invention and/or a vaccine of the invention; and (4) 3 days, 7 days 14 days, 21 days, 28 days, 35 days, 42 days, 49 days, and 56 days after administering the dose of the recombinant virus of the invention and/or a vaccine of the invention. In a specific embodiment, a total of 5 blood draws (3-5 ml each) are obtained, each just prior to the first, third and booster doses and approximately one month following the third dose and booster dose of administration of the vaccine or placebo. Samples are allowed to clot at room temperature and the serum is collected after centrifugation.

Sera are tested for strain-specific serum hemaglutination inhibition (HAI) antibody levels against the virus of the invention. Other indicators of immunogenicity such as IgG, IgA, or neutralizing antibodies are also tested. Serum antibody responses to one or more of the other vaccines given concurrently may be measured. The amount of antibodies generated against the recombinant virus of the invention and/or a vaccine of the invention in the samples from the patients can be quantitated by ELISA.

The concentration of antibody levels in the serum of volunteers are corrected by subtracting the predose serum level (background level) from the serum levels at each collection interval after administration of the dose of recombinant virus of the invention and/or a vaccine of the invention. For each volunteer the pharmacokinetic parameters are computed according to the model-independent approach (Gibaldi et al., eds., 1982, Pharmacokinetics, 2nd edition, Marcel Dekker, New York) from the corrected serum antibody or antibody fragment concentrations.

Nasal washes obtained approximately 2, 3, 4, 5, 6, 7 or 8 days after each doses of vaccine/placebo will be cultured to detect shedding of the vaccine virus of the invention. In a preferred embodiment, nasal washes obtained 7 days after each doses of vaccine/placebo are cultured. A nasopharyngeal swab, a throat swab, or a nasal wash is also used to determine the presence of other viruses in volunteers with medically attended febrile illness (rectal temperature greater than or equal to 102° F ) and/or croup, bronchiolitis, or pneumonia at any time during the study. Samples are shipped on dry ice to designated site for study. Assays for isolation and quantitation of the vaccine virus of the invention and immunostaining assays using MAb to identify the vaccine virus of the invention are used (examples of such assays are given in the Example sections, *infra*)*.* Nasal wash specimens may be tested for other viruses and immune responses including IgG, IgA, and neutralizing antibody.

### 5.5.6. REPORTER GENES

In certain embodiments, assays for measurement of reporter gene expression in tissue culture or in animal models can be used with the methods of the invention. The nucleotide sequence of the reporter gene is cloned into the virus, such as bPIV, HPIV, or b/hPIV3, wherein (i) the position of the reporter gene is changed and (ii) the length of the intergenic regions flanking the reporter gene are varied. Different combinations are tested to determine the optimal rate of expression of the reporter gene and the optimal replication rate of the virus comprising the reporter gene.

In certain embodiments, minigenome constructs are generated to include a reporter gene. The construction of minigenome constructs is described in section 5.5.1.

The abundance of the reporter gene product can be determined by any technique known to the skilled artisan. Such techniques include, but are not limited to, Northern blot analysis or Western blot analysis using probes or antibodies, respectively, that are specific to the reporter gene.

In certain embodiments, the reporter gene emits a fluorescent signal that can be detected in a FACS. FACS can be used to detect cells in which the reporter gene is expressed.

Techniques for practicing the specific aspect of this invention will employ, unless otherwise indicated, conventional techniques of molecular biology, microbiology, and recombinant DNA manipulation and production, which are routinely practiced by one of skill in the art. See, *e.g*., Sambrook, 1989, Molecular Cloning, A Laboratory Manual, Second Edition; DNA Cloning, Volumes I and II (Glover, Ed. 1985); and Transcription and Translation (Hames & Higgins, Eds. 1984).

The biochemical activity of the reporter gene product represents the expression level of the reporter gene. The total level of reporter gene activity depends also on the replication rate of the recombinant virus of the invention. Thus, to determine the true expression level of the reporter gene from the recombinant virus, the total expression level should be divided by the titer of the recombinant virus in the cell culture or the animal model.

Reporter genes that can be used with the methods of invention include, but are not limited to, the genes listed in the Table 4 below:

**Table 4: Reporter genes and the biochemical properties of the respective reporter gene products**

| Reporter Gene | Protein Activity & Measurement |
|---|---|
| CAT (chloramphenicol acetyltransferase) | Transfers radioactive acetyl groups to chloramphenicol or detection by thin layer chromatography and autoradiography |
| GAL (b-galactosidase) | Hydrolyzes colorless galactosides to yield colored products. |
| GUS (b-glucuronidase) | Hydrolyzes colorless glucuronides to yield colored products. |
| LUC (luciferase) | Oxidizes luciferin, emitting photons |
| GFP (green fluorescent protein) | fluorescent protein without substrate |
| SEAP (secreted alkaline phosphatase) | luminescence reaction with suitable substrates or with substrates that generate chromophores |
| HRP (horseradish peroxidase) | in the presence of hydrogen oxide, oxidation of 3,3',5,5'-tetramethylbenzidine to form a colored complex |
| AP (alkaline phosphatase) | luminescence reaction with suitable substrates or with substrates that generate chromophores |

The abundance of the reporter gene can be measured by, *inter alia,* Western blot analysis or Northern blot analysis or any other technique used for the quantification of transcription of a nucleotide sequence, the abundance of its mRNA its protein (see Short Protocols in Molecular Biology, Ausubel et al. (editors), John Wiley & Sons, Inc., 4th edition, 1999). In certain embodiments, the activity of the reporter gene product is measured as a readout of reporter gene expression from the recombinant virus. For the quantification of the activity of the reporter gene product, biochemical characteristics of the reporter gene product can be investigated (see Table 1). The methods for measuring the biochemical activity of the reporter gene products are well-known to the skilled artisan. A more detailed description of illustrative reporter genes that can be used with the methods of the invention is set forth below.

### LUCIFERASE

Luciferases are enzymes that emit light in the presence of oxygen and a substrate (luciferin) and which have been used for real-time, low-light imaging of gene expression in cell cultures, individual cells, whole organisms, and transgenic organisms (reviewed by Greer & Szalay, 2002, Luminescence 17(1):43-74).

As used herein, the term "luciferase" as used in relation to the invention is intended to embrace all luciferases, or recombinant enzymes derived from luciferases that have luciferase activity. The luciferase genes from fireflies have been well characterized, for example, from the *Photinus* and *Luciola* species (see, *e.g*., International Patent Publication No. WO 95/25798 for *Photinus pyralis,* European Patent Application No. EP 0 524 448 for *Luciola cruciata* and *Luciola lateralis,* and Devine et al., 1993, Biochim. Biophys. Acta 1173(2):121-132 for *Luciola mingrelica.* Other eucaryotic luciferase genes include, but are not limited to, the sea panzy (*Renilla reniformis,* see, *e.g.,* Lorenz et al., 1991, Proc Natl Acad Sci U S A 88(10):4438-4442), and the glow worm (*Lampyris noctiluca,* see *e.g.,* Sula-Newby et al., 1996, Biochem J. 313:761-767). Bacterial luciferin-luciferase systems include, but are not limited to, the bacterial lux genes of terrestrial *Photorhabdus luminescens* (see, *e.g.,* Manukhov et al., 2000, Genetika 36(3):322-30) and marine bacteria *Vibrio fischeri* and *Vibrio harveyi* (see, *e.g*., Miyamoto et al., 1988, J Biol Chem. 263(26):13393-9, and Cohn et al., 1983, Proc Natl Acad Sci USA., 80(1):120-3, respectively). The luciferases encompassed by the present invention also includes the mutant luciferases described in U.S. Patent No. 6,265,177 to Squirrell et al*.,* which is hereby incorporated by reference in its entirety.

### GREEN FLUORESCENT PROTEIN

Green fluorescent protein ("GFP") is a 238 amino acid protein with amino acids 65 to 67 involved in the formation of the chromophore that does not require additional substrates or cofactors to fluoresce (see, *e.g*., Prasher et al., 1992, Gene 111:229-233; Yang et al., 1996, Nature Biotechnol. 14:1252-1256; and Cody et al., 1993, Biochemistry 32:1212-1218).

As used herein, the term "green fluorescent protein" or "GFP" as used in relation to the invention is intended to embrace all GFPs (including the various forms of GFPs that exhibit colors other than green), or recombinant enzymes derived from GFPs that have GFP activity. The native gene for GFP was cloned from the bioluminescent jellyfish *Aequorea victoria* (see, *e.g.,* Morin et al., 1972, J. Cell Physiol. 77:313-318). Wild type GFP has a major excitation peak at 395 nm and a minor excitation peak at 470 nm. The absorption peak at 470 nm allows the monitoring of GFP levels using standard fluorescein isothiocyanate (FITC) filter sets. Mutants of the GFP gene have been found useful to enhance expression and to modify excitation and fluorescence. For example, mutant GFPs with alanine, glycine, isoleucine, or threonine substituted for serine at position 65 result in mutant GFPs with shifts in excitation maxima and greater fluorescence than wild type protein when excited at 488 nm (see, *e.g.,* Heim et al., 1995, Nature 373:663-664); U.S. Patent No. 5,625,048; Delagrave et al., 1995, Biotechnology 13:151-154; Cormack et al., 1996, Gene 173:33-38; and Cramer et al., 1996, Nature Biotechnol. 14:315-319). The ability to excite GFP at 488 nm permits the use of GFP with standard fluorescence activated cell sorting ("FACS") equipment. In another embodiment, GFPs are isolated from organisms other than the jellyfish, such as, but not limited to, the sea pansy, *Renilla reriformis.*

EGFP is a red-shifted variant of wild-type GFP (3-5) which has been optimized for brighter fluorescence and higher expression in mammalian cells. (Excitation maximum = 488 nm; emission maximum = 507 nm.) EGFP encodes the GFPmutl variant which contains the double-amino-acid substitution of Phe-64 to Leu and Ser-65 to Thr. The coding sequence of the EGFP gene contains more than 190 silent base changes which correspond to human codon-usage preferences.

### BETA GALACTOSIDASE

Beta galactosidase ("β-gal") is an enzyme that catalyzes the hydrolysis of b-galactosides, including lactose, and the galactoside analogs o-nitrophenyl-β-D-galactopyranoside ("ONPG") and chlorophenol red-b-D-galactopyranoside ("CPRG") (see, *e.g*., Nielsen et al., 1983 Proc Natl Acad Sci USA 80(17):5198-5202; Eustice et al., 1991, Biotechniques 11:739-742; and Henderson et al., 1986, Clin. Chem. 32:1637-1641). The β-gal gene functions well as a reporter gene because the protein product is extremely stable, resistant to proteolytic degradation in cellular lysates, and easily assayed. When ONPG is used as the substrate, β-gal activity can be quantitated with a spectrophotometer or a microplate reader.

As used herein, the term "beta galactosidase" or "β-gal" as used in relation to the invention is intended to embrace all b-gals, including *lacZ* gene products, or recombinant enzymes derived from b-gals which have b-gal activity. The b-gal gene functions well as a reporter gene because the protein product is extremely stable, resistant to proteolytic degradation in cellular lysates, and easily assayed. In an embodiment where ONPG is the substrate, b-gal activity can be quantitated with a spectrophotometer or microplate reader to determine the amount of ONPG converted at 420 nm. In an embodiment when CPRG is the substrate, b-gal activity can be quantitated with a spectrophotometer or microplate reader to determine the amount of CPRG converted at 570 to 595 nm.

### CHLORAMPHENICOL ACETYLTRANSFERASE

Chloramphenicol acetyl transferase ("CAT") is commonly used as a reporter gene in mammalian cell systems because mammalian cells do not have detectable levels of CAT activity. The assay for CAT involves incubating cellular extracts with radiolabeled chloramphenicol and appropriate co-factors, separating the starting materials from the product by, for example, thin layer chromatography ("TLC"), followed by scintillation counting (see, *e.g.,* U.S. Patent No. 5,726,041, which is hereby incorporated by reference in its entirety).

As used herein, the term "chloramphenicol acetyltransferase" or "CAT" as used in relation to the invention is intended to embrace all CATs, or recombinant enzymes derived from CAT which have CAT activity. While it is preferable that a reporter system which does not require cell processing, radioisotopes, and chromatographic separations would be more amenable to high through-put screening, CAT as a reporter gene may be preferable in situations when stability of the reporter gene is important. For example, the CAT reporter protein has an *in vivo* half life of about 50 hours, which is advantageous when an accumulative versus a dynamic change type of result is desired.

### SECRETED ALKALINE PHOSPHATASE

The secreted alkaline phosphatase ("SEAP") enzyme is a truncated form of alkaline phosphatase, in which the cleavage of the transmembrane domain of the protein allows it to be secreted from the cells into the surrounding media.

As used herein, the term "secreted alkaline phosphatase" or "SEAP" as used in relation to the invention is intended to embrace all SEAP or recombinant enzymes derived from SEAP which have alkaline phosphatase activity. SEAP activity can be detected by a variety of methods including, but not limited to, measurement of catalysis of a fluorescent substrate, immunoprecipitation, HPLC, and radiometric detection. The luminescent method is preferred due to its increased sensitivity over calorimetric detection methods. The advantages of using SEAP is that a cell lysis step is not required since the SEAP protein is secreted out of the cell, which facilitates the automation of sampling and assay procedures. A cell-based assay using SEAP for use in cell-based assessment of inhibitors of the Hepatitis C virus protease is described in U.S. Patent No. 6,280,940 to Potts et al*.* which is hereby incorporated by reference in its entirety.

### 5.5.7. CELL CULTURE SYSTEMS, EMBRYONATED EGGS, AND ANIMAL MODELS

Cell culture systems known in the art can be used to propagate or test activities of the viruses of the present invention. (*See e.g.,* Flint et al., PRINCIPLES OF VIROLOGY, MOLECULAR BIOLOGY, PATHOGENESIS, AND CONTROL, 2000, ASM Press pp25-29, the entire text is incorporated herein by reference). Examples of such cell culture systems include, but are not limited to, primary cell culture that are prepared from animal tissues (*e.g*., cell cultures derived from monkey kidney, human embryonic amnion, kidney, and foreskin, and chicken or mouse embryos); diploid cell strains that consist of a homogeneous population of a single type and can divide up to 100 times before dying (*e.g.,* cell culture derived from human embryos, such as the WI-38 strain derived from human embryonic lung); and continuous cell lines consist of a single cell type that can be propagated indefinitely in culture (*e.g*., HEp-2 cells, Hela cells, Vero cells, L and 3T3 cells, and BHK-21 cells).

Viruses of the invention can also be propagated in embryonated chicken eggs. At 5 to 14 days after fertilization, a hole is drilled in the shell and virus is injected into the site appropriate for its replication.

Any animal models known in the art can be used in the present invention to accomplish various purposes, such as to determine the effectiveness and safeness of vaccines of the invention. Examples of such animal models include, but are not limited to, cotton rats (*Sigmodon hispidis*), hamsters, mice, monkeys, and chimpanzees. In a preferred embodiment, Syrian Golden hamsters are used.

### 5.5.8. NEUTRALIZATION ASSAY

Neutralization assays can be carried out to address the important safety issue of whether the heterologous surface glycoproteins are incorporated into the virion which may result in an altered virus tropism phenotype. As used herein, the term "tropism" refers to the affinity of a virus for a particular cell type. Tropism is usually determined by the presence of cell receptors on specific cells which allow a virus to enter that and only that particular cell type. A neutralization assay is performed by using either MAbs of the heterologous surface glycoprotein (non-limiting example is the F protein of a negative strand RNA virus) or polyclonal antiserum comprising antibodies against the heterologous surface glycoprotein. Different dilution of the antibodies are tested to see whether the chimeric virus of the invention can be neutralized . The heterologous surface glycoprotein should not be present on the virion surface in an amount sufficient to result in antibody binding and neutralization.

### 5.5.9. SUCROSE GRADIENT ASSAY

The question of whether the heterologous proteins are incorporated into the virion can be further investigated by use of a biochemical assay. Infected cell lysates can be fractionated in 20 - 60% sucrose gradients, various fractions are collected and analyzed for the presence and distribution of heterologous proteins and the vector proteins by Western blot. The fractions and the virus proteins can also be assayed for peak virus titers by plaque assay. Examples of sucrose gradient assay are given in section 23, *infra.* When the heterologous proteins are associated with the virion, they will co-migrate with the virion.

### 5.6. VACCINE FORMULATIONS USING THE CHIMERIC VIRUSES

The invention encompasses vaccine formulations comprising the engineered negative strand RNA virus of the present invention. The recombinant PIV viruses of the present invention may be used as a vehicle to express foreign epitopes that induce a protective response to any of a variety of pathogens. In a specific embodiment, the invention encompasses the use of recombinant bPIV viruses or attenuated hPIV that have been modified in vaccine formulations to confer protection against hPIV infection.

The vaccine preparations of the invention encompass multivalent vaccines, including bivalent and trivalent vaccine preparations. The bivalent and trivalent vaccines of the invention may be administered in the form of one PIV vector expressing each heterologous antigenic sequence or two or more PIV vectors each encoding different heterologous antigenic sequences. For example, a first chimeric PIV expressing one or more heterologous antigenic sequences can be administered in combination with a second chimeric PIV expressing one or more heterologous antigenic sequences, wherein the heterologous antigenic sequences in the second chimeric PIV are different from the heterologous antigenic sequences in the first chimeric PIV. The heterologous antigenic sequences in the first and the second chimeric PIV can be derived from the same virus but encode different proteins, or derived from different viruses. In a preferred embodiment, the heterologous antigenic sequences in the first chimeric PIV are derived from respiratory syncytial virus, and the heterologous antigenic sequences in the second chimeric PIV are derived from human metapneumovirus. In another preferred embodiment, the heterologous antigenic sequences in the first chimeric PIV are derived from respiratory syncytial virus, and the heterologous antigenic sequences in the second chimeric PIV are derived from avian pneumovirus.

In certain preferred embodiments, the vaccine formulation of the invention is used to protect against infections caused by a negative strand RNA virus, including but not limited to, influenza virus, parainfluenza virus, respiratory syncytial virus, and mammalian metapneumovirus (*e.g*., human metapneumovirus). More specifically, the vaccine formulation of the invention is used to protect against infections by a human metapneumovirus and/or an avian pneumovirus. In certain embodiments, the vaccine formulation of the invention is used to protect against infections by (a) a human metapneumovirus and a respiratory syncytial virus; and/or (b) an avian pneumovirus and a respiratory syncytial virus.

In a preferred embodiment, the invention provides a proteinaceous molecule or metapneumovirus-specific viral protein or functional fragment thereof encoded by a nucleic acid according to the invention. Useful proteinaceous molecules are for example derived from any of the genes or genomic fragments derivable from a virus according to the invention. Particularly useful are the F, SH and/or G protein or antigenic fragments thereof for inclusion as antigen or subunit immunogen, but inactivated whole virus can also be used. Particularly useful are also those proteinaceous substances that are encoded by recombinant nucleic acid fragments that are identified for phylogenetic analyses, of course preferred are those that are within the preferred bounds and metes of ORFs useful in phylogenetic analyses, in particular for eliciting MPV specific antibody or T cell responses, whether in vivo (e.g. for protective purposes or for providing diagnostic antibodies) or in vitro (e.g. by phage display technology or another technique useful for generating synthetic antibodies).

A pharmaceutical composition comprising a virus, a nucleic acid, a proteinaceous molecule or fragment thereof, an antigen and/or an antibody according to the invention can for example be used in a method for the treatment or prevention of a MPV infection and/or a respiratory illness comprising providing an individual with a pharmaceutical composition according to the invention. This is most useful when said individual is a human, specifically when said human is below 5 years of age, since such infants and young children are most likely to be infected by a human MPV as provided herein. Generally, in the acute phase patients will suffer from upper respiratory symptoms predisposing for other respiratory and other diseases. Also lower respiratory illnesses may occur, predisposing for more and other serious conditions. The compositions of the invention can be used for the treatment of immuno-compromised individuals including cancer patients, transplant recipients and the elderly.

The invention also provides methods to obtain an antiviral agent useful in the treatment of respiratory tract illness comprising establishing a cell culture or experimental animal comprising a virus according to the invention, treating said culture or animal with an candidate antiviral agent, and determining the effect of said agent on said virus or its infection of said culture or animal. The invention also provides use of an antiviral agent according to the invention for the preparation of a pharmaceutical composition, in particular for the preparation of a pharmaceutical composition for the treatment of respiratory tract illness, specifically when caused by an MPV infection or related disease, and provides a pharmaceutical composition comprising an antiviral agent according to the invention, useful in a method for the treatment or prevention of an MPV infection or respiratory illness, said method comprising providing an individual with such a pharmaceutical composition.

In certain embodiments of the invention, the vaccine of the invention comprises mammalian metapneumovirus. In certain, more specific embodiments, the mammalian metapneumovirus is a human metapneumovirus. In a preferred embodiment, the mammalian metapneumovirus to be used in a vaccine formulation has an attenuated phenotype. For methods to achieve an attenuated phenotype, see section 5.4.

The invention provides vaccine formulations for the prevention and treatment of infections with PIV, RSV, APV, and/or hMPV. In certain embodiments, the vaccine of the invention comprises recombinant and chimeric viruses of the invention. In certain embodiments, the virus is attenuated.

In a specific embodiment, the vaccine comprises APV and the vaccine is used for the prevention and treatment for hMPV infections in humans. Without being bound by theory, because of the high degree of homology of the F protein of APV with the F protein of hMPV, infection with APV will result in the production of antibodies in the host that will cross-react with hMPV and protect the host from infection with hMPV and related diseases.

In another specific embodiment, the vaccine comprises hMPV and the vaccine is used for the prevention and treatment for APV infection in birds, such as, but not limited to, in turkeys. Without being bound by theory, because of the high degree of homology of the F protein of APV with the F protein of HMPV, infection with hMPV will result in the production of antibodies in the host that will cross-react with APV and protect the host from infection with APV and related diseases.

In certain embodiments, the vaccine formulation of the invention is used to protect against infections by (a) a human metapneumovirus and a human parainfluenza virus; and/or (b) an avian pneumovirus and a human parainfluenza virus and related diseases.

In certain embodiments, the vaccine formulation of the invention is used to protect against infections by (a) a human metapneumovirus, a respiratory syncytial virus, and a human parainfluenza virus; and/or (b) an avian pneumovirus, a respiratory syncytial virus, and a human parainfluenza virus and related diseases.

In certain embodiments, the vaccine formulation of the invention is used to protect against infections by a human metapneumovirus, a respiratory syncytial virus, and a human parainfluenza virus. In certain other embodiments, the vaccine formulation of the invention is used to protect against infections by an avian pneumovirus, a respiratory syncytial virus, and a human parainfluenza virus, and related diseases.

Due to the high degree of homology among the F proteins of different viral species, for exemplary amino acid sequence comparisons see Figure 1, the vaccine formulations of the invention can be used for protection from viruses different from the one from which the heterologous nucleotide sequence encoding the F protein was derived. In a specific exemplary embodiment, a vaccine formulation contains a virus comprising a heterologous nucleotide sequence derived from an avian pneumovirus type A, and the vaccine formulation is used to protect from infection by avian pneumovirus type A and avian pneumovirus type B. In another specific exemplary embodiment, a vaccine formulation contains a virus comprising a heterologous nucleotide sequence derived from an avian pneumovirus subgroup C, and the vaccine formulation is used to protect from infection by avian pneumovirus subgroup C and avian pneumovirus subgroup D.

The invention encompasses vaccine formulations to be administered to humans and animals that are useful to protect against PIV, hMPV, APV (including APV C and APV D), influenza, RSV, Sendai virus, mumps, laryngotracheitis virus, simianvirus 5, human papillomavirus, as well as other viruses, pathogens and related diseases. The invention further encompasses vaccine formulations to be administered to humans and animals that are useful to protect against human metapneumovirus infections, avian pneumovirus infections, and related diseases.

In one embodiment, the invention encompasses vaccine formulations that are useful against domestic animal disease causing agents including rabies virus, feline leukemia virus (FLV) and canine distemper virus. In yet another embodiment, the invention encompasses vaccine formulations that are useful to protect livestock against vesicular stomatitis virus, rabies virus, rinderpest virus, swinepox virus, and further, to protect wild animals against rabies virus.

Attenuated viruses generated by the reverse genetics approach can be used in the vaccine and pharmaceutical formulations described herein. Reverse genetics techniques can also be used to engineer additional mutations to other viral genes important for vaccine production. For example, mutations in the 5' non-coding region may affect mRNA translation, mutations in capsid proteins are believed to influence viral assembly, and temperature-sensitive and cold-adapted mutants are often less pathogenic than the parental virus. (*see, e.g.,* Flint et al., PRINCIPLES OF VIROLOGY, MOLECULAR BIOLOGY, PATHOGENESIS, AND CONTROL, 2000, ASM Press pp 670 - 683, the entire text is incorporated herein by reference). The epitopes of useful vaccine strain variants can be engineered into the attenuated virus. Alternatively, completely foreign epitopes, including antigens derived from other viral or non-viral pathogens can be engineered into the attenuated strain. For example, antigens of non-related viruses such as HIV (gp 160, gp 120, gp41), parasite antigens (*e.g*., malaria), bacterial or fungal antigens, or tumor antigens can be engineered into the attenuated strain. Alternatively, epitopes which alter the tropism of the virus *in* vivo can be engineered into the chimeric attenuated viruses of the invention.

Virtually any heterologous gene sequence may be constructed into the chimeric viruses of the invention for use in vaccines. Preferably, moieties and peptides that act as biological response modifiers are constructed into the chimeric viruses of the invention for use in vaccines. Preferably, epitopes that induce a protective immune response to any of a variety of pathogens, or antigens that bind neutralizing antibodies may be expressed by or as part of the chimeric viruses. For example, heterologous gene sequences that can be constructed into the chimeric viruses of the invention include, but are not limited to influenza and parainfluenza hemagglutinin neuraminidase and fusion glycoproteins such as the HN and F genes of human PN3. In yet another embodiment, heterologous gene sequences that can be engineered into the chimeric viruses include those that encode proteins with immunomodulating activities. Examples of immunomodulating proteins include, but are not limited to, cytokines, interferon type 1, gamma interferon, colony stimulating factors, interleukin -1, -2, -4, -5, -6, -12, and antagonists of these agents.

In addition, heterologous gene sequences that can be constructed into the chimeric viruses of the invention for use in vaccines include but are not limited to sequences derived from a human immunodeficiency virus (HIV), preferably type 1 or type 2. In a preferred embodiment, an immunogenic HIV-derived peptide that may be the source of an antigen may be constructed into a chimeric PIV that may then be used to elicit a vertebrate immune response. Such HIV-derived peptides may include, but are not limited to, sequences derived from the env gene (*i.e.,* sequences encoding all or part of gp160, gp120, and/or gp41), the pol gene (*i.e*., sequences encoding all or part of reverse transcriptase, endonuclease, protease, and/or integrase), the gag gene (*i.e*., sequences encoding all or part of p7, p6, p55, p17/18, p24/25), tat, rev, nef, vif, vpu, vpr, and/or vpx.

Other heterologous sequences may be derived from hepatitis B virus surface antigen (HBsAg); hepatitis A or C virus surface antigens, the glycoproteins of Epstein Barr virus; the glycoproteins of human papillomavirus; the glycoproteins of respiratory syncytial virus, parainfluenza virus, Sendai virus, simianvirus 5 or mumps virus; the glycoproteins of influenza virus; the glycoproteins of herpesviruses; VP 1 of poliovirus; antigenic determinants of non-viral pathogens such as bacteria and parasites, to name but a few. In another embodiment, all or portions of immunoglobulin genes may be expressed. For example, variable regions of anti-idiotypic immunoglobulins that mimic such epitopes may be constructed into the chimeric viruses of the invention.

Other heterologous sequences may be derived from tumor antigens, and the resulting chimeric viruses can be used to generate an immune response against the tumor cells leading to tumor regression *in vivo.* These vaccines may be used in combination with other therapeutic regimens, including but not limited to, chemotherapy, radiation therapy, surgery, bone marrow transplantation, etc. for the treatment of tumors. In accordance with the present invention, recombinant viruses may be engineered to express tumor-associated antigens (TAAs), including but not limited to, human tumor antigens recognized by T cells (Robbins and Kawakami, 1996, Curr. Opin. Immunol. 8:628-636, incorporated herein by reference in its entirety), melanocyte lineage proteins, including gp100, MART-1/MelanA, TRP-1 (gp75), tyrosinase; Tumor-specific widely shared antigens, MAGE-1, MAGE-3, BAGE, GAGE-1, GAGE-1, N-acetylglucosaminyltransferase-V, p15; Tumor-specific mutated antigens, β-catenin, MUM-1, CDK4; Nonmelanoma antigens for breast, ovarian, cervical and pancreatic carcinoma, HER-2/neu, human papillomavirus -E6, -E7, MUC-1.

In even other embodiments, a heterologous nucleotide sequence is derived from a metapneumovirus, such as human metapneumovirus and/or avian pneumovirus. In even other embodiments, the virus of the invention contains two different heterologous nucleotide sequences wherein one is derived from a metapneumovirus, such as human metapneumovirus and/or avian pneumovirus, and the other one is derived from a respiratory syncytial virus. The heterologous nucleotide sequence encodes a F protein or a G protein of the respective virus. In a specific embodiment, a heterologous nucleotide sequences encodes a chimeric F protein, wherein the chimeric F protein contains the ectodomain of a F protein of a metapneumovirus and the transmembrane domain as well as the luminal domain of a F protein of a parainfluenza virus.

Either a live recombinant viral vaccine or an inactivated recombinant viral vaccine can be formulated. A live vaccine may be preferred because multiplication in the host leads to a prolonged stimulus of similar kind and magnitude to that occurring in natural infections, and therefore, confers substantial, long-lasting immunity. Production of such live recombinant virus vaccine formulations may be accomplished using conventional methods involving propagation of the virus in cell culture or in the allantois of the chick embryo followed by purification. Additionally, as bPIV has been demonstrated to be non-pathogenic in humans, this virus is highly suited for use as a live vaccine.

In this regard, the use of genetically engineered PIV (vectors) for vaccine purposes may desire the presence of attenuation characteristics in these strains. The introduction of appropriate mutations (*e.g.,* deletions) into the templates used for transfection may provide the novel viruses with attenuation characteristics. For example, specific missense mutations that are associated with temperature sensitivity or cold adaption can be made into deletion mutations. These mutations should be more stable than the point mutations associated with cold or temperature sensitive mutants and reversion frequencies should be extremely low.

Alternatively, chimeric viruses with "suicide" characteristics may be constructed. Such viruses would go through only one or a few rounds of replication within the host. When used as a vaccine, the recombinant virus would go through limited replication cycle(s) and induce a sufficient level of immune response but it would not go further in the human host and cause disease. Recombinant viruses lacking one or more of the PIV genes or possessing mutated PIV genes would not be able to undergo successive rounds of replication. Defective viruses can be produced in cell lines which permanently express such a gene(s). Viruses lacking an essential gene(s) would be replicated in these cell lines, however, when administered to the human host, they would not be able to complete a round of replication. Such preparations may transcribe and translate --in this abortive cycle -- a sufficient number of genes to induce an immune response. Alternatively, larger quantities of the strains could be administered, so that these preparations serve as inactivated (killed) virus vaccines. For inactivated vaccines, it is preferred that the heterologous gene product be expressed as a viral component, so that the gene product is associated with the virion. The advantage of such preparations is that they contain native proteins and do not undergo inactivation by treatment with formalin or other agents used in the manufacturing of killed virus vaccines. Alternatively, mutated PIV made from cDNA may be highly attenuated so that it replicates for only a few rounds.

In certain embodiments, the vaccine of the invention comprises an attenuated virus. Without being bound by theory, the attenuated virus can be effective as a vaccine even if the attenuated virus is incapable of causing a cell to generate new infectious viral particles because the viral proteins are inserted in the cytoplasmic membrane of the host thus stimulating an immune response.

In another embodiment of this aspect of the invention, inactivated vaccine formulations may be prepared using conventional techniques to "kill" the chimeric viruses. Inactivated vaccines are "dead" in the sense that their infectivity has been destroyed. Ideally, the infectivity of the virus is destroyed without affecting its immunogenicity. In order to prepare inactivated vaccines, the chimeric virus may be grown in cell culture or in the allantois of the chick embryo, purified by zonal ultracentrifugation, inactivated by formaldehyde or β-propiolactone, and pooled. The resulting vaccine is usually inoculated intramuscularly.

Inactivated viruses may be formulated with a suitable adjuvant in order to enhance the immunological response. Such adjuvants may include but are not limited to mineral gels, *e.g*., aluminum hydroxide; surface active substances such as lysolecithin, pluronic polyols, polyanions; peptides; oil emulsions; and potentially useful human adjuvants such as BCG, Corynebacterium parvum, ISCOMS, and virosomes.

Many methods may be used to introduce the vaccine formulations described above, these include but are not limited to oral, intradermal, intramuscular, intraperitoneal, intravenous, subcutaneous, percutaneous, and intranasal and inhalation routes. It may be preferable to introduce the chimeric virus vaccine formulation via the natural route of infection of the pathogen for which the vaccine is designed.

In certain embodiments, the invention relates to immunogenic compositions. The immunogenic compositions comprise a chimeric PIV. In certain embodiments, the immunogenic composition comprises an attenuated chimeric PIV. In certain embodiments, the immunogenic composition further comprises a pharmaceutically acceptable carrier.

Various techniques may be used to evaluate the effectiveness and safeness of a vaccine according to the present invention. An effective vaccine is a vaccine that protects vaccinated individuals from illness due to pathogens, by invoking proper innate, cellular, and humoral responses with minimal side effect. The vaccine must not cause disease. Any techniques that are able to measure the replication of the virus and the immune response of the vaccinated subject may be used to evaluate the vaccine. Non-limiting examples are given in the Example sections, *infra.*

### 5.6.1. DOSAGE REGIMENS AND ADMINISTRATION OF THE VACCINES OR IMMUNOGENIC PREPARATIONS OF THE INVENTION

The present invention provides vaccines and immunogenic preparations comprising chimeric PIV expressing one or more heterologous or non-native antigenic sequences. The vaccines or immunogenic preparations of the invention encompass single or multivalent vaccines, including bivalent and trivalent vaccines. The vaccines or immunogenic formulations of the invention are useful in providing protections against various viral infections. Particularly, the vaccines or immunogenic formulations of the invention provide protection against respiratory tract infections in a host.

A recombinant virus and/or a vaccine or immunogenic formulation of the invention can be administered alone or in combination with other vaccines. Preferably, a vaccine or immunogenic formulation of the invention is administered in combination with other vaccines or immunogenic formulations that provide protection against respiratory tract diseases, such as but not limited to, respiratory syncytial virus vaccines, influenza vaccines, measles vaccines, mumps vaccines, rubella vaccines, pneumococcal vaccines, rickettsia vaccines, staphylococcus vaccines, whooping cough vaccines or vaccines against respiratory tract cancers. In a preferred embodiment, the virus and/or vaccine of the invention is administered concurrently with pediatric vaccines recommended at the corresponding ages. For example, at two, four or six months of age, the virus and/or vaccine of the invention can be administered concurrently with DtaP (IM), Hib (IM), Polio (IPV or OPV) and Hepatitis B (IM). At twelve or fifteen months of age, the virus and/or vaccine of the invention can be administered concurrently with Hib (IM), Polio (IPV or OPV), MMRII^{Ⓡ} (SubQ); Varivax^{®} (SubQ), and hepatitis B (IM). The vaccines that can be used with the methods of invention are reviewed in various publications, *e.g*., The Jordan Report 2000, Division of Microbiology and Infectious Diseases, National Institute of Allergy and Infectious Diseases, National Institutes of Health, United States, the content of which is incorporated herein by reference in its entirety.

A vaccine or immunogenic formulation of the invention may be administered to a subject *per se* or in the form of a pharmaceutical or therapeutic composition. Pharmaceutical compositions comprising an adjuvant and an immunogenic antigen of the invention (*e.g.,* a virus, a chimeric virus, a mutated virus) may be manufactured by means of conventional mixing, dissolving, granulating, dragee-making, levigating, emulsifying, encapsulating, entrapping or lyophilizing processes. Pharmaceutical compositions may be formulated in conventional manner using one or more physiologically acceptable carriers, diluents, excipients or auxiliaries which facilitate processing of the immunogenic antigen of the invention into preparations which can be used pharmaceutically. Proper formulation is, or amongst others, dependent upon the route of administration chosen.

When a vaccine or immunogenic composition of the invention comprises adjuvants or is administered together with one or more adjuvants, the adjuvants that can be used include, but are not limited to, mineral salt adjuvants or mineral salt gel adjuvants, particulate adjuvants, microparticulate adjuvants, mucosal adjuvants, and immunostimulatory adjuvants. Examples of adjuvants include, but are not limited to, aluminum hydroxide, aluminum phosphate gel, Freund's Complete Adjuvant, Freund's Incomplete Adjuvant, squalene or squalane oil-in-water adjuvant formulations, biodegradable and biocompatible polyesters, polymerized liposomes, triterpenoid glycosides or saponins (*e.g*., QuilA and QS-21, also sold under the trademark STIMULON, ISCOPREP), N-acetyl-muramyl-L-threonyl-D-isoglutamine (Threonyl-MDP, sold under the trademark TERMURTIDE), LPS, monophosphoryl Lipid A (3D-MLAsold under the trademark MPL).

The subject to which the vaccine or an immunogenic composition of the invention is administered is preferably a mammal, most preferably a human, but can also be a non-human animal, including but not limited to, primates, cows, horses, sheep, pigs, fowl (*e.g.,* chickens, turkeys), goats, cats, dogs, hamsters, mice and rodents.

Many methods may be used to introduce the vaccine or the immunogenic composition of the invention, including but not limited to, oral, intradermal, intramuscular, intraperitoneal, intravenous, subcutaneous, percutaneous, intranasal and inhalation routes, and via scarification (scratching through the top layers of skin, *e.g*., using a bifurcated needle).

For topical administration, the vaccine or immunogenic preparations of the invention may be formulated as solutions, gels, ointments, creams, suspensions, etc. as are well-known in the art.

For administration intranasally or by inhalation, the preparation for use according to the present invention can be conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g*., gelatin for use in an inhaler or insufflator may be formulated containing a powder mix of the compound and a suitable powder base such as lactose or starch.

For injection, the vaccine or immunogenic preparations may be formulated in aqueous solutions, preferably in physiologically compatible buffers such as Hanks's solution, Ringer's solution, or physiological saline buffer. The solution may contain formulatory agents such as suspending, stabilizing and/or dispersing agents. Alternatively, the proteins may be in powder form for constitution with a suitable vehicle, *e.g*., sterile pyrogen-free water, before use.

Determination of an effective amount of the vaccine or immunogenic formulation for administration is well within the capabilities of those skilled in the art, especially in light of the detailed disclosure provided herein.

An effective dose can be estimated initially from *in vitro* assays. For example, a dose can be formulated in animal models to achieve an induction of an immunity response using techniques that are well known in the art. One having ordinary skill in the art could readily optimize administration to all animal species based on results described herein. Dosage amount and interval may be adjusted individually. For example, when used as an immunogenic composition, a suitable dose is an amount of the composition that when administered as described above, is capable of eliciting an antibody response. When used as a vaccine, the vaccine or immunogenic formulations of the invention may be administered in about 1 to 3 doses for a 1-36 week period. Preferably, 1 or 2 doses are administered, at intervals of about 2 weeks to about 4 months, and booster vaccinations may be given periodically thereafter. Alternate protocols may be appropriate for individual animals. A suitable dose is an amount of the vaccine formulation that, when administered as described above, is capable of raising an immunity response in an immunized animal sufficient to protect the animal from an infection for at least 4 to 12 months. In general, the amount of the antigen present in a dose ranges from about 1 pg to about 100 mg per kg of host, typically from about 10 pg to about 1 mg, and preferably from about 100 pg to about 1 µg. Suitable dose range will vary with the route of injection and the size of the patient, but will typically range from about 0.1 mL to about 5 mL.

In a specific embodiment, the viruses and/or vaccines of the invention are administered at a starting single dose of at least 10³ TCID₅₀, at least 10⁴ TCID₅₀, at least 10⁵ TCID₅₀, at least 10⁶ TCID₅₀. In another specific embodiment, the virus and/or vaccines of the invention are administered at multiple doses. In a preferred embodiment, a primary dosing regimen at 2, 4, and 6 months of age and a booster dose at the beginning of the second year of life are used. More preferably, each dose of at least 10⁵ TCID₅₀, or at least 10⁶ TCID₅₀ is given in a multiple dosing regimen. The replication rate of a virus can be used as an index to adjust the dosage of a vaccine in a clinical trial. For example, assays to test the replication rate of a virus (*e.g*., a growth curve, *see* Section 5.5. for available assays) can be used to compare the replication rate of the viruses and/or vaccines of the invention to that of the bPIV3, which was demonstrated in previous studies (*see* Clements et al., J. Clin. Microbiol. 29:1175-82 (1991); Karron et al., J. Infect. Dis. 171:1107-14 (1995); Karron et al., Ped. Inf. Dis. J. 5:650-654 (1996). These studies showed that a bovine PIV3 vaccine is generally safe and well tolerated by healthy human volunteers, including adults, children 6-60 months of age, and infants 2-6 months of age. In these studies, subjects have received at least a single dose of bPIV3 vaccine from 10³ TCID₅₀ to 10⁶ TCID₅₀. Twelve children received two doses of 10⁵ TCID₅₀ PIV3 vaccine instead of one dose without untoward effects.). A comparable replication rate as to bPIV3 suggests that a comparable dosage may be used in a clinical trial. A lower replication rate compared to that of bPIV3 suggests that a higher dosage can be used.

### 5.6.1.1. CHALLENGE STUDIES

This assay is used to determine the ability of the recombinant viruses of the invention and of the vaccines of the invention to prevent lower respiratory tract viral infection in an animal model system, such as, but not limited to, cotton rats or hamsters. The recombinant virus and/or the vaccine can be administered by intravenous (IV) route, by intramuscular (IM) route or by intranasal route (IN). The recombinant virus and/or the vaccine can be administered by any technique well-known to the skilled artisan. This assay is also used to correlate the serum concentration of antibodies with a reduction in lung titer of the virus to which the antibodies bind.

On day 0, groups of animals, such as, but not limited to, cotton rats (Sigmodon hispidis, average weight 100 g) cynomolgous macacques (average weight 2.0 kg) are administered the recombinant or chimeric virus or the vaccine of interest or BSA by intramuscular injection, by intravenous injection, or by intranasal route. Prior to, concurrently with, or subsequent to administration of the recombinant virus or the vaccine of the invention, the animals are infected with wild type virus wherein the wild type virus is the virus against which the vaccine was generated. In certain embodiments, the animals are infected with the wild type virus at least 1 day, at least 2 days, at least 3 days, at least 4 days, at least 5 days, at least 6 days, 1 week or 1 or more months subsequent to the administration of the recombinant virus and/or the vaccine of the invention.

After the infection, cotton rats are sacrificed, and their lung tissue is harvested and pulmonary virus titers are determined by plaque titration. Bovine serum albumin (BSA) 10 mg/kg is used as a negative control. Antibody concentrations in the serum at the time of challenge are determined using a sandwich ELISA. Similarly, in macacques, virus titers in nasal and lung lavages can be measured.

### 5.6.1.2. TARGET POPULATIONS

In certain embodiments of the invention, the target population for the therapeutic and diagnostic methods of the invention is defined by age. In certain embodiments, the target population for the therapeutic and/or diagnostic methods of the invention is characterized by a disease or disorder in addition to a respiratory tract infection.

In a specific embodiment, the target population encompasses young children, below 2 years of age. In a more specific embodiment, the children below the age of 2 years do not suffer from illnesses other than respiratory tract infection.

In other embodiments, the target population encompasses patients above 5 years of age. In a more specific embodiment, the patients above the age of 5 years suffer from an additional disease or disorder including cystic fibrosis, leukaemia, and non-Hodgkin lymphoma, or recently received bone marrow or kidney transplantation.

In a specific embodiment of the invention, the target population encompasses subjects in which the hMPV infection is associated with immunosuppression of the hosts. In a specific embodiment, the subject is an immunocompromised individual.

In certain embodiments, the target population for the methods of the invention encompasses the elderly.

In a specific embodiment, the subject to be treated with the methods of the invention was infected with hMPV in the winter months.

### 5.6.1.3. CLINICAL TRIALS

Vaccines of the invention or fragments thereof tested in *in vitro* assays and animal models may be further evaluated for safety, tolerance and pharmacokinetics in groups of normal healthy adult volunteers. The volunteers are administered intramuscularly, intravenously or by a pulmonary delivery system a single dose of a recombinant virus of the invention and/or a vaccine of the invention. Each volunteer is monitored at least 24 hours prior to receiving the single dose of the recombinant virus of the invention and/or a vaccine of the invention and each volunteer will be monitored for at least 48 hours after receiving the dose at a clinical site. Then volunteers are monitored as outpatients on days 3, 7, 14, 21, 28, 35, 42, 49, and 56 postdose.

Blood samples are collected via an indwelling catheter or direct venipuncture using 10 ml red-top Vacutainer tubes at the following intervals: (1) prior to administering the dose of the recombinant virus of the invention and/or a vaccine of the invention; (2) during the administration of the dose of the recombinant virus of the invention and/or a vaccine of the invention; (3) 5 minutes, 10 minutes, 15 minutes, 20 minutes, 30 minutes, 1 hour, 2 hours, 4 hours, 8 hours, 12 hours, 24 hours, and 48 hours after administering the dose of the recombinant virus of the invention and/or a vaccine of the invention; and (4) 3 days, 7 days 14 days, 21 days, 28 days, 35 days, 42 days, 49 days, and 56 days after administering the dose of the recombinant virus of the invention and/or a vaccine of the invention. Samples are allowed to clot at room temperature and serum will be collected after centrifugation.

The amount of antibodies generated against the recombinant virus of the invention and/or a vaccine of the invention in the samples from the patients can be quantitated by ELISA. T-cell immunity (cytotoxic and helper responses) in PBMC and lung and nasal lavages can also be monitored.

The concentration of antibody levels in the serum of volunteers are corrected by subtracting the predose serum level (background level) from the serum levels at each collection interval after administration of the dose of recombinant virus of the invention and/or a vaccine of the invention. For each volunteer the pharmacokinetic parameters are computed according to the model-independent approach (Gibaldi et al., eds., 1982, Pharmacokinetics, 2nd edition, Marcel Dekker, New York) from the corrected serum antibody or antibody fragment concentrations.

The following examples are illustrative, but not limiting, of the present invention. Cells and Viruses used in the examples are maintained as follows: the RSV A2 strain and the bovine parainfluenza type 3/human parainfluenza type 3 vectored RSV viruses (bPIV3/hPIV3/RSV viruses) were grown in Vero cells in Opti-MEM (Gibco/BRL) in the presence of gentamycin. The modified vaccinia virus Ankara (MVA-T7) or fowl-pox-T7 (FP-T7) which expressed the phage T7 RNA polymerase were grown in chicken embryonic kidney cells (SPAFAS). Vero, HeLa and Hep-2 cells were maintained in MEM (JRH Biosciences) supplemented with 10% fetal bovine serum (FBS), 2 mM L-glutamine, non-essential amino acids, and antibiotics.

### 6. EXAMPLE 1: CONSTRUCTION AND CLONING OF CHIMEERIC BOVINE PARAINFLUENZA 3 / HUMAN PARAINFLUENZA 3 cDNA

In order to substitute the F and HN genes of bPIV3 with those of hPIV3, additional restriction enzyme sites were introduced into the infectious bPIV3 cDNA. Using site-directed mutagenesis, a unique Nhe I site was introduced at nucleotide position 5041 and a Sal I site was introduced at nt 8529 of the bPIV3 cDNA. The modified full-length bPIV3 cDNA was treated with Nhe I and Sal I restriction enzymes and a -14 kb DNA fragment encompassing all of the viral bPIV3 sequences except the F and HN genes, was isolated by gel purification.

To obtain the hPIV3 F and HN gene sequences, a 10 cm dish of confluent Vero cells was infected with a strain of hPIV3 (hPIV3/Tex/12084/1983). After 3 days of incubation at 37°C, the cells were harvested and total RNA was isolated using RNA STAT-LS 50 (Tel-Test Inc.). Viral cDNA was generated by reverse transcription using a hPIV3 specific oligo annealing at position 4828 of the hPIV3 genome. The hPIV3 F and HN genes were amplified by PCR (polymerase chain reaction) using Taq polymerase. The PCR product was cloned into the pT/A TOPO cloning vector (Invitrogen) and from two clones (#11 and #14) the hPIV3 F and HN genes were sequenced. Sequence analysis revealed that for clone #11, the F gene was correct, but the HN gene contained aberrant sequences; for clone #14, the HN gene was correct, but the F gene contained aberrant stop codons. Thus, a plasmid, containing functional hPIV3 F and HN genes, was constructed by combining the correct F gene of #11 with the correct HN gene of #14 in the following manner. Both hPIV3 plasmids (#11 and #14) were digested with Nhe1 and EcoR1. A 1.6 kb fragment harboring the correct F gene was isolated from clone #11 and a 8.5 kb fragment containing the correct HN gene and plasmid sequences, was isolated from clone #14. The two fragments were ligated to produce the intact hPIV3 F and HN genes-containing plasmid. The correct sequence was confirmed by DNA sequence analysis. Finally, a single nucleotide was added at the 3' end of the HN gene in the untranslated region to satisfy the "Rule of Six." The addition of the single nucleotide was accomplished by using the QuikChange mutagenesis kit (Stratagene) and was confirmed by DNA sequencing. The correct hPIV3 F and HN gene DNA fragment was then isolated by digestion with Nhe 1 and Sal 1 and a 3.5 kb DNA fragment was gel purified.

The full-length b/h PIV3 chimeric cDNA was constructed by ligating the 14.5 kb DNA fragment harboring bPIV3 sequences described above and the 3.5 kb DNA fragment containing the hPIV3 F and HN genes (see Figure 3). The full-length chimeric plasmid DNA was confirmed by extensive restriction enzyme mapping. In addition, the M/F and HN/L gene junctions of the chimeric construct were confirmed by DNA sequencing to both contain bPIV3 and hPIV3 sequences as well as a Nhe 1 and a Sal 1 restriction enzyme site, respectively.

### 7. EXAMPLE 2 : CONSTRUCTION AND CLONING OF CHIMERIC BOVINE PARAINFLUENZA 3/HUMAN PARAINFLUENZA 3 VECTORED RESPIRATORY SYNCYTIAL VIRUS F OR G cDNAs

In order to determine the effects of RSV antigen insertions in position 1 or 2 of the b/h PIV3 genome on virus replication, respiratory syncytial virus (RSV) F and G genes were cloned into different positions of the chimeric bovine parainfluenza 3/human parainfluenza 3 vector (b/h PIV3 vector). *See* Figure 4.

In order to insert foreign genes into the bovine/human (b/h) PIV3 cDNA, AvrII restriction enzyme sites were introduced in the b/h PIV3 cDNA plasmid (Haller *et al.,* 2000; 2001, this is the same construct as in Example 6) by site-directed mutagenesis using the QuickChange kit (Stratagene). One AvrII site was introduced at nucleotide (nt) 104 in the b/h PIV3 genome altering four nucleotides using the following oligo 5'GAA ATC CTA AGA CCC TAG GCA TGT TGA GTC3' and its complement. This restriction enzyme site was used to insert the RSV genes in the first (most 3') position of the viral genome. Another AvrII site was introduced in the N-P intergenic region at nt 1774 changing two nucleotides using the following oligo 5'CCACAACTCAATCAACCTAGGATTCATGGAAGACAATG 3' and its complement. This restriction site was used to insert the RSV genes in the second position between the N and P genes of b/h PIV3 (Figure 4). Full-length b/h PIV3 cDNAs harboring the AvrII sites at nts 104 and 1774 were tested for functionality by recovering viruses by reverse genetics.

Construction of RSV G cassette (N-P gene stop/start): A DNA fragment was generated that contained the bPIV3 N-P intergenic region as well as the 3' end sequences of the RSV G gene, using the b/h PIV3 cDNA as PCR template. This fragment was generated by PCR using the following oligos: 5'CCCAACACACCACGCCAGTAGTCACAA AGAGATGACCACTATCAC3' and 5'CCCAAGCTTCCTAGGTGAATCTTTG GTTGATTGAGTTGTGG3'. This fragment was then used to carry out overlapping PCR to add the bPIV3 N-P intergenic region to the RSV G gene. For the second PCR reaction, a plasmid containing the RSV G and F gene was used as a DNA template, the oligo 5'CAGCGGATCCTAGGGGAGAAAAGTGTCGAAGAAAAATGTCC3' and an oligo generated from the short PCR fragment above were used as primers. The resulting PCR fragment containing the RSV G gene linked to the bPIV3 N-P intergenic region and flanking AvrII restriction enzyme sites, was cloned into pGEM3. The RSV G gene was sequenced to confirm the presence of an intact open reading frame and the predicted amino acid sequences. The DNA fragments harboring the RSV G gene were inserted into the first or second position using the AvrII restriction enzyme sites into a subclone harboring only the first 5200 nucleotides of the bPIV3 (1-5 bPIV3) genome that was linearized with AvrII. As used herein and other Examples, 1-5 bPIV3 refers to the nucleotide 1 to 5196 (or 5200) of bovine PIV3 genome. There is a BstBl site at this location.

Construction of RSV F cassette (N-P gene start/stop): The RSV F gene fragment was isolated by PCR from a full-length bPIV3/RSV F+G cDNA plasmid using oligos that added AvrII sites at the 5' and 3' end of the RSV F gene, and introduced into the 1-5 bPIV3 plasmid harboring the AvrII site at nt 1774, which was linearized with AvrII. The bPIV3 N-P intergenic region was isolated by PCR using 1-5 bPIV3/RSV G2 as a template. The oligo 5'GACGCGTCGACCACAAAGAGATGACCACTATCACC 3' and an oligo annealing in the bPIV3 F gene were used to generate a PCR fragment containing the bPIV3 N-P intergenic region, AvrII site, and bPIV3 sequences up to nt 5200. The PCR fragment was digested with SalI and NheI, and added to the 1-5 bPIV3 plasmid harboring the RSV F gene in position 2, which was treated with SalI and NheI. To introduce the RSV F gene containing the N-P intergenic region into position 1, the 1.8 kb RSV F cassette was excised using AvrII, and ligated into 1-5 bPIV3 containing the AvrII site at nt 104, which was linearized with AvrII.

Construction of the RSV F cassette with a short intergenic region (N stop/N start):

The generation of the RSV F gene with the short N-N intergenic region was accomplished by performing a PCR reaction using 1-5 bPIV3/RSV F2 as a template, the oligo 5'GCGCGTCGACCAAGTAAGAAAAACTTAGGATTAAAGAACCCTAGGACTGTA3', and an oligo annealing upstream of the 5' end of the RSV F gene encompassing the AvrII restriction enzyme site. The PCR product containing the RSV F gene and the short N-N intergenic region, was digested with AvrII and introduced into 1-5 bPIV3 nt 104 which was linearized with AvrII.

After confirming proper orientation by restriction enzyme mapping, the plasmids harboring the RSV genes in the first position were digested with SphI and BssHII and 4 kb (1-5 bPIV3/RSV G1) or 4.8 kb (1-5 bPIV3/RSV F1) DNA fragments were isolated. In a second cloning step, the remainder of the b/h PIV3 genome was added as a SphI-BssHII 15.1 kb DNA fragment, yielding full-length cDNAs. The bPIV3 subclones, harboring the RSV genes in the second position, were cut with SphI and NheI, and 5.8 kb (bPIV3/RSV G2) and a 6.5 kb (bPIV3/RSV F2) DNA fragments were isolated. In a second cloning step, the rest of the b/h PIV3 genome was added as an NheI-SphI DNA fragment of 14 kb in size. The full-length chimeric b/h PIV3/RSV plasmids were propagated in STBL-2 cells (Gibco/BRL) that provided high yields of full-length virus cDNA plasmids.

### 8. EXAMPLE 3: BOVINE PARAINFLUENZA 3/HUMAN PARAINFLUENZA 3 VECTORED RESPIRATORY SYNCYTIAL VIRUS F OR G DISPLAYED A POSITIONAL EFFECT WITH REGARDS TO mRNA PRODUCTION AND PROTEIN EXPRESSION AS WELL AS VIRUS REPLICATION IN VITRO

Three experiments were performed to confirm the effective expression of the RSV F or G gene in the constructs of Example 2, and to determine positional effects of gene insertions in the PIV3 genome.

First, in order to demonstrate RSV protein expression by the chimeric viruses, a Western blot of chimeric virus-infected cell lysates was carried out and probed with RSV-specific antisera. See Figure 5A. Western blots were performed as follows: Chimeric viruses were used to infect (70-80%) subconfluent Vero cells at a MOI of 0.1 or 1.0. Forty-eight hours post infection the media overlay was removed and infected monolayers were washed once with 1 ml of PBS. The cells were subsequently lysed in 400 ml of Laemmli buffer (Bio-Rad) containing 0.05% b-Mercaptoethanol (Sigma). 15 ml of each sample was separated on 12% Tris-HCl Ready Gel (Bio-Rad) and transferred to nylon membranes using a semi-dry transfer cell (Bio-Rad). Nylon membranes were rinsed in PBS [pH 7.6] containing 0.5% (v/v) Tween-20 (Sigma) (PBST) and blocked with PBST containing 5% (w/v) dry milk (PBST-M) for 20-30 minutes at room temperature. Membranes were incubated with either a mixture of RSV F monoclonal antibodies (WHO 1269,1200, 1153, 1112, 1243, 1107) at a 1:1000 dilution in PBST-M or RSV G 10181 polyclonal antibody (Orbigen) at a 1:2000 dilution in PBST-M for 1 hour at room temperature. Following four washes with PBST, the membranes were incubated with a secondary horseradish peroxidase-conjugated goat anti-mouse antibody (Dako) at a 1:2000 dilution in PBST-M for 1 hour at room temperature. Membranes were washed 4 times with PBST and developed using a chemiluminescence substrate (Amersham Pharmacia) and exposed to Biomax Light Film (Kodak) for visualization of protein bands.

Consistent with the reduced replication efficiency of b/h/RSV F1*N-N in Vero cells (Figure 5C, *see* below), the amount of RSV F₁ detected at 48 hours post infection was about 10 times less than that present in b/h PIV3/RSV F2 or wild-type RSV A2 infected cells (compare lanes 2, 3, and 4, Figure 5A). A 50 kDa band representing the F₁ fragment was detected in cells infected with all chimeric viruses as well as wild-type RSV. However, there was greater accumulation of a 20 kDa F fragment in infected cell lysates of chimeric viruses compared to wild-type RSV. When b/h PIV3/RSV F1*N-N infections were repeated at a higher MOI of 1.0 (Figure 5A, lanel), the F₁ fragment in b/h PIV3/RSV F1 infected cells accumulated to wild-type RSV levels at 48 hours post-infection. The relative amount of the 50 kDa and 20 kDa F₁ fragments in b/h PIV3/RSV F1 or b/h PIV3/RSV F2 infected cells was approximately 1:5. No F₀ was detected in cells infected with chimeric viruses indicating that the F₀ precursors were efficiently processed during b/h PIV3/RSV F1 and b/h PIV3/RSV F2 infections as was also observed in wild-type RSV infections.

The relative expression of RSV G in b/h PIV3/RSV G1, b/h PIV3/RSV G2 and wild-type RSV infected cells is shown in Figure 5A. Both the immature and glycosylated forms of RSV G that migrated at approximately 50 kDa and 90 kDa, respectively, were detected. b/h PIV3/RSV G1 infected cells showed levels of RSV G expression similar to that seen in wild-type RSV infected cells (lanes 1 and 3, Figure 5A). However, in b/h PIV3/RSV G2 infected cells, the accumulation of RSV G was about 2-3 times more than that present in wild-type RSV infected cells (lanes 2 and 3, Figure 5A). Collectively, these data showed that the chimeric b/h PIV3/RSV efficiently expressed the RSV proteins in either position 1 or 2. However, the viruses harboring the RSV genes in position 2 expressed higher levels of RSV proteins.

Next, Northern blot analysis showed that the mRNA transcription correlated with the result of the protein expression demonstrated by the Western blot, *see* Figure 5B. Northern blot was performed as follows: total cellular RNA was prepared from virus-infected cells using Trizol LS (Life Technologies). The RNA was further purified by one phenolchloroform extraction and precipitated with ethanol. RNA pellets were resuspended in diethyl pyrocarbonate-treated water and stored at -80°C. Equal amounts of total RNA were separated on 1% agarose gels containing 1% formaldehyde and transferred to nylon membranes (Amersham Pharmacia Biotech) using a Turboblotter apparatus (Schleicher & Schuell). The blots were hybridized with digoxigenin (DIG)-UTP-labeled riboprobes synthesized by in vitro transcription using a DIG RNA labeling kit (Roche Molecular Biochemicals). Hybridization was carried out at 68°C for 12 h in Express Hyb solution (Clontech). The blots were washed at 68°C twice with 2X SSC (1X SSC contained 0.015 M NaCl with 0.015 M sodium citrate)-0.1 % sodium dodecyl sulfate (SDS) followed by one wash with 0.5X SSC-0.1% SDS and a final wash with 0.1X SSC-0.1%SDS. Signals from the hybridized probes were detected by using a DIG-Luminescent detection kit (Roche Molecular Biochemicals) and visualized by exposure to BioMax ML film (Kodak).

Northern analysis of b/h PIV3/RSV F1*N-N, b/h PIV3/RSV F2, b/h PIV3/RSV G1 and b/h PIV3/RSV G2 showed that the viral mRNA levels for RSV F or RSV G correlated well with the RSV protein levels observed (Figure 5B). The lowest levels of RSV F mRNAs were observed for b/h PIV3/RSV F1*N-N which also displayed the least amount of RSV F protein produced. b/h PIV3/RSV G1 produced less RSV G mRNAs resulting in lower RSV G protein levels than was observed for b/h PIV3/RSV G2.

Finally, growth of different virus (with RSV F or G gene at either position 1 or position 2) correlates with the results of the protein expression and the RNA transcription. The growth curve showed in Figure 5C was obtained as follows: Vero cells were grown to 90% confluence and infected at an MOI of 0.01 or 0.1 with b/h PIV3, b/h PIV3 RSV F1, b/h PIV3 RSV G1, b/h PIV3 RSV F2, and b/h PIV3 RSV G2. The infected monolayers were incubated at 37°C. At 0, 24, 48, 72, 96 and 120 hours post-infection, cells and media were harvested together and stored at -70°C. Virus titers for each time point harvest were determined by TCID₅₀ or plaque assays in Vero cells. TCID₅₀ assays were inspected visually for CPE following incubation at 37°C for 6 days, while plaque assays were immunostained with RSV polyclonal antisera for quantification after 5 days of incubation.

At an MOI of 0.01 in Vero cells, the chimeric viruses harboring the RSV G or F genes in the first position (b/h PIV3 RSV G1 and b/h PIV3 RSV F1*N-N) replicated at a slower rate, yielded lower peak titers, and exhibited a greater lag phase than the viruses that contained the RSV genes in the second position. Peak titers of b/h PIV3/RSV F1*N-N and b/h PIV3/RSV G1 at 96 hours post-infection were 10^{6.7} and 10^{5.5} TCID₅₀/ml, respectively (Figure 5C). In contrast, peak titers of b/h PIV3/RSV F2 and b/h PN3/RSV G2 were 10^{8.0} and 10^{7.4} at 72 and 96 hours post-infection, respectively (Figure 5C). The b/h PIV3 control virus displayed peak titers of 10^{8.0} TCID50/ml, respectively (Figure 5C). The b/h PIV3/RSV F2 yielded 1.3 log₁₀ higher titers than b/h PIV3/RSV F1*N-N. The b/h PIV3/RSV G2 replicated to 1.9 log₁₀ higher titers than b/h PIV3/RSV G1. The results indicated that the chimeric viruses harboring the RSV genes in the first position were delayed in onset for replication *in vitro* compared to chimeric viruses containing the RSV genes in the second position.

To determine whether higher titers of b/h PIV3/RSV F1*N-N and b/h PIV3/RSV G1 could be achieved at all, the growth curves were repeated at a higher MOI of 0.1. At an MOI of 0.1, the peak titers of b/h PIV3/RSV F1*N-N and b/h PIV3/RSV G1 increased by 0.5 to 1.3 log₁₀ (data not shown). The lag phases of these viruses were reduced and peak titers were achieved earlier during the growth cycle.

### 9. EXAMPLE 4: POSITIONAL EFFECT OF eGFP INSERTIONS IN THE BOVINE PARAINFLUENZA 3/HUMAN PARAINFLUENZA 3 GENOME ON VIRUS REPLICATION

The effect of gene insertions into the bovine/human PIV3 vector backbone was assessed systematically by introducing the eGFP gene sequentially between all genes of PIV3 and observing the effect on virus replication and eGFP expression (Figure 6). This type of assay investigates the importance of the transcriptional gradient observed for paramyxoviruses that yields specific ratios of viral mRNAs. Insertion of foreign genes will perturb these ratios and result in the synthesis of different amounts of viral proteins which may influence virus replication. The eGFP gene was chosen for this assay since it will not be incorporated into the virion membrane, and therefore should not interfere with viral processes such as packaging, budding, entry, etc. The eGFP gene was inserted into four positions of the b/h PIV3 genome, three of which were characterized for eGFP expression and virus replication. The eGFP gene cassette was linked to the bPIV3 N-P intergenic region. b/h GFP1 harbored the eGFP gene cassette in the 3' most proximal position of the b/h PIV3 genome. b/h PIV3/GFP2 contained the eGFP gene cassette between the N and P genes of the b/h PIV3 genome. b/h PIV3/GFP3 was located between P and M, and b/h PIV3/GFP4 had the eGFP gene between M and F of b/h PIV3 (Figure 6).

Construction of the eGFP gene cassette: the template of the eGFP gene is commercially available, *e.g*., it can be purchased from BD Biosciences (pIRES2-EGFP) or Clontech (pEGFP-N1). *See* Hoffmann et al., Virology 267:310-317 (2000). The eGFP gene was isolated by PCR and the bPIV3 N-P intergenic region was added by employing the overlapping PCR method, using the following oligos: 5'ATTCCTAGGATGGTGAGCAAG GGCG3', 5'GGACGAGCTGTACAAGTAAAAAAATAGCACCTAATCATG3', and 5'CTACCTAGGTGAATCTTTGGTTG3'. The eGFP cassette was inserted into pCR2.1, sequenced, and adherence to the rule-of-six was confirmed. Then the eGFP cassette was digested with AvrII, gel purified, and inserted into positions 1, 2, 3, and 4 of b/h PIV3 as described below.

Generation of full-length cDNAs harboring the eGFP gene in positions 1 and 2: the eGFP gene cassette was inserted into the 1-5 bPIV3 plasmids which contained bPIV3 sequences from nts 1 - 5200 and an AvrII restriction enzyme site either at nt 104 (position 1) or nt 1774 (position 2). After confirming proper orientation by restriction enzyme mapping, the plasmid harboring the eGFP gene in the first position was digested with SphI and BssHII and 4 kb (1-5 eGFP1) DNA fragments were isolated. Next, the rest of the b/h PIV3 genome was added as a SphI-BssHII 15.1 kb DNA fragment, yielding full-length cDNAs. For generation of full-length cDNA comprising the eGFP in position 2, the bPIV3 subclones harboring the eGFP genes in the second position were cut with SphI and NheI, and 5.8 kb (1-5 eGFP2) DNA fragments were isolated. Next, the rest of the b/h PIV3 genome was added as an NheI-SphI DNA fragment of 14 kb in size. The full-length chimeric b/h PIV3/eGFP plasmids were propagated in STBL-2 cells (Gibco/BRL) that provided high yields of full-length virus cDNA plasmids.

Generation of full-length cDNAs harboring the eGFP gene in positions 3 and 4: in order to insert the eGFP cassette into position 3 of the b/h PIV3 genome, an AvrII restriction enzyme site was introduced at nt 3730 in the P-M intergenic region of a subclone containing nts 1 - 5200 of bPIV3, altering two nucleotides. The following oligo and its complement were used in a QuickChange PCR reaction to introduce the AvrII site: 5'GGACTAATCAATCCTAGGAAACAATGAGCATCACC3'. The eGFP cassette was digested with AvrII and ligated into the AvrII linearized 1-5 bPIV3 subclone harboring the AvrII site at nt 3730. A 5.5 kb DNA fragment from SphI to NheI was isolated from the GFP containing subclone and introduced into the b/h PIV3 cDNA digested with SphI and NheI to produce a full-length plasmid. In order to add the eGFP gene cassette into position 4 of the b/h PIV3 genome, a subclone containing b/h PIV3 sequences from nts 1- 8500 was generated. This subclone was linearized with NheI (nt 5042), and the eGFP cassette containing compatible AvrII ends was inserted. Then the subclone harboring the eGFP cassette was digested with SphI and XhoI and a 7.1 kb DNA fragment was isolated. The b/h PIV3 plasmid was treated with SphI and XhoI and a 11 kb fragment was produced. These two DNA fragments were ligated to generate b/h PIV3/GFP4.

The amount of eGFP produced by b/h PIV3/GFP1, 2, and 3 was assessed in two ways. First, the amount of green cells produced upon infecting Vero cells with b/h PIV3 GFP1, 2, and 3 at MOIs of 0.1 and 0.01 for 20 hours, was determined using a fluorescent microscope (Figure 7A). b/h PIV3/GFP3 produced strikingly fewer green cells than b/h PIV3/GFP1 or 2.

Secondly, western analysis was performed on infected cells and the blots were probed with a GFP MAb as well as a PIV3 PAb. The initial observation that b/h PIV3/GFP3 produced dramatically less eGFP protein, was confirmed (Figure 7B). b/h PIV3 GFP1 and GFP2 produced similar amounts of eGFP protein. The western blots methods controlled for same volume loading by probing with a PIV3 antibody (Figure 7B). Interestingly, all three viruses showed similar amounts of PIV3 proteins (the HN protein is the most prominent band) produced. These results suggested that b/h PIV3/GFP3 transcribed less GFP mRNAs in position 3 as compared to positions 1 and 2. This data confirmed the presence of a transcriptional gradient of viral mRNAs in paramyxoviruses. The level of production of the PIV3 HN protein was not affected by the eGFP gene insertions (Figure 7B).

In order to determine whether the GFP gene insertions had an effect on the kinetics of virus replication of b/h PIV3/GFP1, 2, and 3, multicycle growth curves in Vero cells were carried out (Figure 7C). The growth curves showed that b/h PIV3/GFP1 had a delayed onset of virus replication at 24 and 48 hours post-infection than b/h PIV3/GFP2 or GFP3. However, the final peak titers obtained were similar for all three viruses. The kinetics of replication for b/h PIV3/GFP2 and GFP3 were nearly identical (Figure 7C). Interestingly, the altered ratios of viral mRNAs did not appear to effect virus replication significantly.

### 10. EXAMPLE 5: CONSTRUCTION AND CLONING OF CHIMERIC BOVINE PARAINFLUENZA 3/HUMAN PARAINFLUENZA 3 VECTORED RESPIRATORY SYNCYTIAL VIRUS F WITH DIFFERENT INTERGENIC REGIONS

Three different constructs were used to determine the effect of intergenic region (nucleotides between each mRNA, *e.g*., nucleotides between the F gene and the N gene) on protein expression and viral replication. See Figure 8. The first construct was b/h PIV3 vectored RSV F1* N-N in position 1, which had a shorter bPIV N gene stop/N gene start sequence (RSV F* N-N in Figure 4); the second construct was b/h PIV3 vectored RSV F at position 1 (RSV F2 in Figure 4); and the last one was b/h PIV3 vectored RSV at position 1 (RSV F1 in Figure 4). All three constructs were generated according to the cloning strategies described in section 7, Example 2.

The most dramatic difference between the two cassettes is the distance between the N gene start sequence and the N translation start codon in b/h PIV3/RSV F1*N-N which was only 10 nts long. In contrast, this distance is 86 nts long in b/h PIV3/RSV F2. The other difference is the use of the N gene start sequence in b/h PIV3/RSV F1*N-N rather than the P gene start sequence as was done in b/h PIV3/RSV F2. In order to determine whether the distance between the transcription gene start and the translation start of a viral transcription unit has an effect on virus replication, the b/h PIV3/RSV F1 construct was generated that contained the identical RSV F gene cassette as was used for b/h PIV3/RSV F2.

### 11. EXAMPLE 6: THE LENGTH AND/OR NATURE OF THE INTERGENIC REGION DOWNSTREAM OF THE RESPIRATORY SYNCYTIAL VIRUS GENE HAS AN EFFECT ON VIRUS REPLICATION

The three constructs in Example 5 were used in the following experiments to determine the effects of the intergenic region on viral protein expression and viral replication. *See* Figure 9.

First, RSV F protein expression for b/h PIV3/RSV F1, b/h PIV3/RSV F1*N-N, and b/h PIV3/RSV F2 was compared at 24 and 48 hrs post-infection at an MOI of 0.1 in Vero cells using Western blots. Western blots were performed as follows: Chimeric viruses were used to infect (70-80%) subconfluent Vero cells at a MOI of 0.1. Twenty-four hours and forty-eight hours post infection the media overlay was removed and infected monolayers were washed once with 1 ml of PBS. The cells were subsequently lysed in 400 ml of Laemmli buffer (Bio-Rad) containing 0.05% b-Mercaptoethanol (Sigma). 15 ml of each sample was separated on 12% Tris-HCl Ready Gel (Bio-Rad) and transferred to nylon membranes using a semi-dry transfer cell (Bio-Rad). Nylon membranes were rinsed in PBS (pH 7.6) containing 0.5% (v/v) Tween-20 (Sigma) (PBST) and blocked with PBST containing 5% (w/v) dry milk (PBST-M) for 20-30 minutes at room temperature. Membranes were incubated with either a mixture of RSV F monoclonal antibodies (WHO 1269,1200, 1153, 1112, 1243, 1107) at a 1:1000 dilution in PBST-M in PBST-M for 1 hour at room temperature. Following 4 washes with PBST, the membranes were incubated with a secondary horseradish peroxidase-conjugated goat anti-mouse antibody (Dako) at a 1:2000 dilution in PBST-M for 1 hour at room temperature. Membranes were washed 4 times with PBST and developed using a chemiluminescence substrate (Amersham Pharmacia) and exposed to Biomax Light Film (Kodak) for visualization of protein bands.

b/h PIV3/RSV F1 expressed RSV F₁ protein levels at 24 and 48 hrs post-infection close to the levels observed for b/h PIV3/RSV F2 but much higher than those of b/h PIV3/RSV F1*N-N. Therefore, the spacing between the gene start element and the translation start codon may be critical for virus replication. The N gene start sequences were changed to P gene start sequences, however this change only incurred the alteration of a single nucleotide. Either of these factors may be responsible for rescuing the RSV F protein expression phenotype.

Next, multicycle growth curves were carried out to compare the kinetics of virus replication of b/h PIV3/RSV F1, b/h PIV3/RSV F1*N-N, and b/h PIV3/RSV F2 in Vero cells at an MOI of 0.1 (*see* Figure 9B), which was performed as follows: Vero cells were grown to 90% confluence and infected at an MOI of 0.1 with b/h PIV3, b/h PIV3/RSV F1*N-N, b/h PIV3/RSV F1, and b/h PIV3/RSV F2. The infected monolayers were incubated at 37°C. At 0, 24, 48, 72, and 96 hours post-infection, cells and media were harvested together and stored at -70°C. Virus titers for each time point harvest were determined by plaque assays in Vero cells. The plaque assays were immunostained with RSV polyclonal antisera for quantification after 5 days of incubation.

As was shown on Figure 9B, the onset of replication of b/h PIV3/RSV F1*N-N was delayed and peak titers were lower than those of b/h PIV3/RSV F2. In contrast, b/h PIV3/RSV F1 displayed a growth curve that was nearly identical to that observed for b/h PIV3/RSV F2.

### 12. EXAMPLE 7: CLONING OF TRIVALENT BOVINE PARAINFLUENZA 3/HUMAN PARAINFLUENZA 3 VECTORED CONSTRUCTS

The following examples relate to the generation of trivalent vaccines that harbor the surface glycoproteins (F and HN) of hPIV3, RSV F, and hMPV F to protect children from disease caused by RSV, hMPV and hPIV3 using a single live attenuated virus vaccine. These trivalent viruses were recovered by reverse genetics.

The construction of two virus genomes, each comprising a chimeric b/h PIV3 backbone with two additional heterologous sequence insertions, wherein one heterologous nucleotide sequence is derived from a metapneumovirus F gene and another heterologous nucleotide sequence is derived from a respiratory syncytial virus F gene, were done as follows (*see* Figure 10): plasmids b/h PIV3/RSV F2 or b/h PIV3/hMPV F2 was digested with SphI and NheI, and a 6.5 kb fragment was isolated. The full-length cDNA for b/h PIV3 RSV F1 or b/h PIV3/hMPV F1 was digested with SphI and NheI and a 14.8 kb DNA fragment was isolated and ligated with the 6.5 kb DNA fragment derived from plasmid b/h PIV3/RSV F2 or b/h PIV3/hMPV F2 to generate full-length viral cDNAs.

Virus with the above described constructs has been amplified in Vero cells. The engineered virus as described herein can be used as a trivalent vaccine against the parainfluenza virus infection, metapneumovirus infection, and the respiratory syncytial virus infection.

### 13. EXAMPLE 8: CLONING OF TWO RESPIRATORY SYNCYTIAL VIRUS F TO THE BOVINE PARAINFLUENZA 3/HUMAN PARAINFLUENZA 3 VECTOR

Chimeric viruses that carry two copies of the RSV F gene were designed in order to determine whether more RSV protein produced by the chimeric virus will result in an improved immunogenicity. This virus was rescued by reverse genetics, biologically cloned and amplified in Vero cells to yield a virus stock with a titer of 1 x 10⁶ pfu/ml. This virus, b/h PIV3/RSV F1F2, can be used to assess for virus growth kinetics, for RSV F protein production, and for replication and immunogenicity in hamsters.

The constructs were generated in the following manner (*see* Figure 11): the 1-5 RSV F2 plasmid was digested with SphI and NheI, and a 6.5 kb fragment was isolated. The full-length cDNA for b/h PIV3 RSV F1 was digested with SphI and NheI and a 14.8 kb DNA fragment was isolated and ligated with the 6.5 kb DNA fragment derived from 1-5 bPIV3/RSV F2 to generate full-length viral cDNAs.

### 14. EXAMPLE 9: CONSTRUCTION AND CLONING OF BOVINE PARAINFLUENZA 3/HUMAN PARAINFLUENZA 3 VECTORED HUMAN METAPNEUMOVIRUS F cDNA

The F gene of human metapneumovirus (hMPV) was inserted in positions 1 and 2 of the b/h PIV3 genome (Figure 12). The hMPV F gene cassette harbored the bPIV3 N-P intergenic region. The hMPV F gene plasmid (pRF515) was used, and a single nucleotide mutation in the hMPV F gene was corrected (*i.e.,* nucleotide 3352 was corrected from C to T (wild type)), generating pRF515-M4. The bPIV3 N-P intergenic region was added at the 3' end of the hMPV F gene using overlapping PCR. For hMPV F, the overlapping PCR oligo was 5'GGCTTCATACCACATAATTAGAAAAATAGCA CCTAATCATGTTCTTACAATGGTCGACC 3'. During this cloning step, oligos were used at the 5' end (5' GCAGCCTAGGCCGCAATAACAATGTCTTGGAAAGTGGTG ATC 3') and at the 3' end of the hMPV F gene cassette (5' CTACCTAGGTGAATCTT TGGT TG 3') in the PCR reaction that contained AvrII restriction enzyme sites. The hMPV F gene cassette was adjusted to conform to the rule of six using QuickChange mutagenesis kit and the following oligos (5'CCTAGGCCGCAATAGACAATGT CTTGG 3', 5'CCAAGACATT GTCTATTGCGGCCTAGG 3'). Full-length b/h PIV3/hMPV F1 (position 1) and F2 (position 2) cDNA plasmids were generated in the same fashion as described in section 9, Example 4, *supra,* for b/h PIV3/eGFP1 and eGFP2.

### 15. EXAMPLE 10: IMMUNOPRECIPITATION AND REPLICATION ASSAYS OF BOVINE PARAINFLUENZA 3/HUMAN PARAINFLUENZA 3 VECTORED HUMAN METAPNEUMOVIRUS F

To confirm that the F protein was expressed in the b/h PIV3 vectored human metapneumovirus F at position 2 (hMPV F2), guinea pig or human antiserum were used to immunoprecipitate the hMPV F protein (*see* Figure 13A). For immunoprecipitation of the hMPV F protein expressed by b/h PIV3, Vero cells were infected with b/h PIV3 or b/h PIV3/hMPV F2 at an MOI of 0.1 or 0.05. Twenty-four hours post-infection, the cells were washed once with DME without cysteine and minus methionine (ICN) and incubated in the same media for 30 min. The media was removed and 0.5 ml DME lacking cysteine and methionine containing 100 µCi of [³⁵S]-Pro-Mix (Amersham) was added to the cells. The infected cells were incubated in the presence of ³⁵S-isotopes for 5 hours at 37°C. Media was removed and the infected cells were lysed in 0.3 M RIPA buffer containing protease inhibitors. The cell lysate was incubated with hMPV guinea pig or human polyclonal antisera and bound to IgG-agarose (Sigma). After washing three times with 0.5 M RIPA buffer, the samples were fractionated on a 10% protein gel. The gel was dried and exposed to X-ray film.

The expression of HMPV F protein by b/h PIV3/hMPV F2 was shown by immunoprecipitation using the gp and human anti-hMPV antisera (Figure 13A). Interestingly, a specific band migrating at approximately 80 kDa was observed in the lysates of b/h PIV3/hMPV F2. This size corresponded to the F precursor protein, F₀. Non-specific bands of different sizes were also observed in the b/h PIV3 and mock control lanes (Figure 13). This data suggested that the b/h PIV3/hMPV F2 expressed the hMPV F protein. However, the hMPV antibody reagents available are limited and these antisera interact only with the precursor of the hMPV F protein. It could also be possible that the cleaved F1 is unstable and thus not easily visualized using this method.

Growth curves were performed to determine the kinetics of virus replication of b/h PIV3/hMPV F2 and compare them to those observed for b/h PIV3 and b/h PIV3/RSV F2 in Vero cells at an MOI of 0.1 (Figure 13B). The data showed that b/h PIV3/hMPV F2 displayed a delayed onset of replication at 24 hours post-infection compared to b/h PIV3/RSV F2. However, at 48 hours post-infection and beyond, a difference in replication was no longer observed.

Growth curves were also performed to determine the kinetics of viral replication of b/h PIV3/hMPV F1 and compare them to those observed for b/h PIV3/hMPV F2 and b/h PIV3 in Vero cells at an MOI of 0.01 (Figure 13C). The data showed that b/h PIV/hMPV F1 had a delayed onset of replication and yields lower peak titers compared to b/h PIV3/hMPV F2 or b/h PIV3. The plaque size of b/h hMPV F1 is also smaller compared to b/h hMPV F2.

The chimeric viruses, b/h PIV3/hMPV F1 and F2 were also assessed for their ability to infect and replicate in Syrian Golden hamsters (Table 5). The results showed that b/h PIV3/hMPV F1 and F2 replicated in the nasal turbinates and lungs of hamsters to levels observed for b/h PIV3. Even hMPV replicated to titers of 5.3 and 3.6 log₁₀ TCID₅₀/g tissue in the upper and lower respiratory tracts of hamsters. These data showed that b/h PIV3/hMPV F1 and F2 could efficiently infect and replicate in the respiratory tract of hamsters, demonstrating thereby that hamsters represent a suitable small animal model to determine immunogenicity of hMPV as well as utilize this animal model to evaluate hMPV vaccine candidates.

**Table 5**

| **Replication of b/h PIV3 Expressing the hMPV F Protein in Positions 1 or 2 in Hamsters** | | |
|---|---|---|
| Virus ^{a} | Mean virus titer on day 4 post-infection (log₁₀TCID₅₀/g tissue ± S.E.) ^{b} | |
| | Nasal turbinates | Lungs |
| b/h PIV3 | 4.8 ± 0.2 | 5.6 ± 0.6 |
| b/h hMPV F1 | 5.3 ± 0.5 | 5.7 ± 0.4 |
| b/h hMPV F2 | 5.7 ± 0.5 | 4.6 ± 0.3 |
| hMPV | 5.3 ± 0.1 | 3.6 ± 0.3 |

| | | |
|---|---|---|
| ^{a} Groups of six hamster were inoculated intranasally with 1x 10⁶ pfu of indicated virus. ^{b} Standard error Note: TCID₅₀ assays were read for CPE on Day 10. | | |

### 16. EXAMPLE 11: CLONING OF THE SOLUBLE RESPIRATORY SYNCYTIAL VIRUS F GENE CONSTRUCT

A construct containing a single copy of the soluble RSV F gene, a version of the RSV F gene lacking the transmembrane and cytosolic domains, was also generated (Figure 14). This construct can be used to test for immunogenicity. Its advantage would be the inability of the soluble RSV F to be incorporated into the virion membrane. Therefore this virus may be viewed as a safer chimeric virus since its virus tropism is not expected to change. The cDNA plasmid for b/h PIV3/sol RSV F can be rescued by reverse genetics.

The plasmid 1-5/RSV F2 (described previously) was used as a DNA template for PCR. The oligo RSV f.2 (5'GCTGTAACAGAATTGCAGTTGC 3') (which anneals at nt 5946 of RSV) and the oligo 5'CGTGGTCGACCATTGTAAGAACATGATTAGGTGCTAT TTTTATTTAATTTGTGGTGGATTTACCGGC3' were employed to remove the transmembrane and cytoplasmic domains of RSV F, deleting 150 nucleotides. The resulting PCR fragment was digested with HpaI and SalI and introduced into 1-5 RSV F2 treated with HpaI and SalI to yield 1-5 bPIV3/sol RSV F. This plasmid was digested with SphI and NheI and the resulting fragment was introduced into a b/h PIV3 cDNA digested with SphI and NheI to generate a full-length cDNA.

### 17. EXAMPLE 12: EXPRESSION OF HUMAN METAPNEUMOVIRUS F IN CELLS INFECTED WITH BOVINE PARAINFLUENZA 3/HUMAN PARAINFLUENZA 3 VECTORED HUMAN METAPNEUMOVIRUS F

The b/h 104 hMPV F virus stocks were serially diluted 10 fold and used to infect subconfluent Vero cells. Infected cells were overlayed with optiMEM media containing gentamycin and incubated at 35°C for 5 days. Cells were fixed with 100% methanol and immunostained with 1:1000 dilution of anti-hMPV001 guinea pig sera followed by 1:1000 dilution of anti-guinea pig HRP conjugated antibodies. Expression of HMPV F is visualized by specific color development in the presence of the AEC substrate system (DAKO corporation). See Figure 15A.

The b/h NP-P hMPV F virus stocks were serially diluted 10 fold and used to infect subconfluent Vero cells. Infected cells were overlayed with 1% methyl cellulose in EMEM/L-15 medium (JRH Biosciences; Lenexa, KS) supplemented with 1x L15/MEM media containing penicillin/streptomycin, L-glutamine and fetal bovine serum. Infected cells were incubated at 35°C for 5 days, fixed with 100% methanol and immunostained with 1:1000 dilution of anti-hMPV001 guinea pig sera followed by 1:1000 dilution of anti-guinea pig HRP conjugated antibodies. (See Figure 15B). The anti hMPV001 guinea pig serum is specific for hMPV001 proteins and do not bind to b/h PIV3 proteins.

### 18. EXAMPLE 13: RESCUE OF CHIMERIC BOVINE PARAINFLUENZA TYPE 3 / HUMAN PARAINFLUENZA TYPE 3 VIRUS IN HELA CELLS AND VERO CELLS

Rescue of the chimeric b/h PIV3 virus was done using a similar procedure as for bPIV3 rescue. Rescue of b/h PIV3 chimeric virus by reverse genetics was carried out in HeLa cells using LipofecTACE (Gibco/BRL). The 80% confluent HeLa cells, Hep-2 cells, or Vero cells were infected with MVA at an MOI of 4. One hour post-infection, the full-length anti-genomic b/h PIV3 cDNA (4 µg) was transfected into the infected HeLa or Vero cells together with the NP (0.4 µg), P (0.4 µg), and L/pCITE (0.2 µg) expression plasmids. Forty hours post-transfection, the cells and the cell supernatant were harvested (P0) and subjected to a single freeze-thaw cycle. The resulting cell lysate was then used to infect a fresh Vero cell monolayer in the presence of 1-beta-D-arabinofuranosylcytosine (ara C), a replication inhibitor of vaccinia virus, to generate a P1 virus stock. The supernatant and cells from these plates were harvested, freeze-thawed once and the presence of bPIV3 virus particles was assayed for by immunostaining of virus plaques using PIV3-specific antiserum. The cell lysates of the P1 harvest resulted in complete CPE of the Vero cell monolayers and immunostaining indicated the presence of an extensive virus infection.

### 19. EXAMPLE 14: RESCUE OF BOVINE PARAINFLUENZA TYPE 3/HUMAN PARAINFLUENZA TYPE 3 VECTORED HUMAN METAPNEUMOVIRUS F VIRUSES

The b/h PIV3 viruses expressing hMPV F at position one (b/h 104 hMPV F) or position two (b/h NP-P hMPV F) were obtained as follows. HEp-2 or Vero cells at 80-90% confluency in 6 well dishes were infected with Fowlpox-T7 at a multiplicity of infection (m.o.i) of 0.1 to 0.3. Following infection with Fowlpox-T7, cells were washed once with PBS and transfected with the following amounts of plasmid DNA: full length b/h 104 hMPV F or b/h NP-P hMPV F cDNA 2.0 µg, pCite N 0.4 µg, pCite P 0.4 µg, pCite L 0.2 µg. (The pCite plasmids have a T7 promoter followed by the IRES element derived from the encephalomyocarditis virus (EMCV)). Transfection was performed in the presence of Lipofectamine 2000 (Invitrogen) according to manufacturer's instruction. The transfection reaction was incubated at 33°C for 5 to 12 hours following which the media containing lipofectamine 2000 was replaced with 2 ml of fresh OptiMEM containing gentamicin. The transfected cells were further incubated at 33°C for two days. Cells were stabilized with SPG and lyzed by one freeze-thaw cycle at -80°C. The crude cell lysate was used to infect a new Vero monolayer in order to amplify rescued viruses.

### 20. EXAMPLE 15: RESCUE OF BOVINE PARAINFLUENZA TYPE 3/HUMAN PARAINFLUENZA TYPE 3 VECTORED RESPIRATORY SYNCYTIAL VIRUS GENES BY REVERSE GENETICS

Infectious virus was recovered by reverse genetics in HeLa or HEp-2 cells using transfection methods described previously (see Example 13). Briefly, HEp-2 or Vero cells at 80-90% confluency in 6 well tissue culture dishes were infected with FP-T7 or MVA-T7 at a multiplicity of infection (m.o.i.) of 0.1 - 0.3 or 1 - 5 respectively. Following infection with FP-T7 or MVA-T7, cells were washed once with PBS and transfected with the following amounts of plasmid DNA (2.0µg full-length b/h PIV3 RSV F or G cDNA, 0.4µg pCITE/N, 0.4µg pCITE/P, 0.2µg pCITE/L). Transfections were performed in the presence of Lipofectamine2000 (Invitrogen) according to manufacturer's instruction. The transfection reactions were incubated at 33°C for 5 to 12 hours following which the media containing Lipofectamine 2000 was replaced with 2 ml of fresh OptiMEM containing gentamicin. The transfected cells were incubated further at 33°C for two days. Cells were stabilized with SPG and lysed with one freeze-thaw cycle at -80°C. The crude cell lysate was used to infect a new Vero cell monolayer in order to amplify rescued viruses. The chimeric viruses were purified by limiting dilutions in Vero cells and high titer virus stocks of 10⁶ - 10⁸ PFU/ml were generated. The RSV genes of the chimeric viruses were isolated by RT-PCR and the sequences were confirmed. Expression of the RSV proteins was confirmed by immunostaining of infected Vero cell monolayers with RSV goat polyclonal antiserum (Biogenesis).

### 21. EXAMPLE 16: CONFIRMATION OF CHIMERIC BOVINE PARAINFLUENZA TYPE 3 / HUMAN PARAINFLUENZA TYPE 3 VIRUS RESCUE BY RT-PCR

To ascertain that the rescued virus is chimeric in nature, i.e. the virus contains hPIV3 F and HN gene sequences in a bPIV3 backbone, the viral RNA genome was analyzed further by RT-PCR. Vero cells, infected with the P1 virus stock of three independently derived isolates of b/h PIV3 were harvested and total RNA was isolated. The viral RNA was amplified using an oligo that anneals at position 4757 of bPIV3. A viral region from nt 5255 to 6255 was amplified by PCR. The resulting 1 kb PCR fragment should contain hPIV3 sequences. This was confirmed by digestion with enzymes (Sac1 and Bgl II) specific for hPIV3 and that do not cut in the complementary region of bPIV3 (see Figure 2). As expected, Sac1 and Bgl II cut the PCR fragment into smaller fragments confirming that the isolated sequences are derived from hPIV3 (see lanes 3, 5, 7). In addition, a region in the polymerase L gene from nt 9075 to nt 10469 was amplified by PCR. This region should contain bPIV3 sequences. Again the resulting 1.4 kb PCR fragment was digested using enzyme specific for bPIV3 (Pvull and BamH1) that do not cut in the equivalent region of hPIV3 (Figure 3). The 1.4 kb fragment was indeed digested by both Pvull and BamH1 confirming that the polymerase gene is bPIV3 in origin (see lanes 3, 4, 6, 7, 9 and 10 of Figure 3). In summary, the RT-PCR analysis shows that the rescued b/h PIV3 virus is chimeric in nature. It contains hPIV3 F and HN genes in a bPIV3 genetic backbone.

### 22. EXAMPLE 17: GENETIC STABILITY OF BOVINE PARAINFLUENZA TYPE 3/HUMAN PARAINFLUENZA TYPE 3 VECTORED RESPIRATORY SYNCYTIAL VIRUS GENES

In order to demonstrate that the b/h PIV3/RSV chimeric viruses are genetically stable and maintain the introduced RSV gene cassettes, infected cell lysates were serially blind passaged ten times in Vero cells. Sub-confluent Vero cells in T25 flasks were infected with b/h PIV3/RSV at an MOI of 0.1 and incubated for 4 days at 33°C or until CPE was visible. At the end of the incubation period the infected cells and media were harvested, frozen and thawed two times, and the resulting cell lysate was used to infect a new T25 flask of Vero cells. This cycle was repeated ten times. All cell lysates from P1 to P10 were analyzed by plaque assay and immunostaining with RSV polyclonal antisera for expression of RSV proteins and virus titers. At passage 10, the RSV gene cassettes were isolated by RT-PCR and the RSV gene sequences were verified by DNA sequence analysis (to identify possible nucleotide alterations). All of the isolates maintained the RSV gene cassettes and RSV protein expression for the 10 passages analyzed (data not shown).

### 23. EXAMPLE 18: VIRION FRACTIONATION OF BOVINE PARAINFLUENZA TYPE 3/HUMAN PARAINFLUENZA TYPE 3 VECTORED RESPIRATORY SYNCYTIAL VIRUS GENES ON SUCROSE GRADIENTS

The question of whether the RSV proteins were incorporated into the b/h PIV3 virion was investigated further by use of a biochemical assay. Vero cells were inoculated with each of the chimeric b/h PIV3/RSV viruses at an MOI of 0.1. When maximum CPE was visible, the infected monolayers were frozen, thawed, and spun for 10 minutes at 2000 rpm. The clarified supernatants were spun through a 20% sucrose cushion at 100,000 x g for 90 minutes. The pellet was then resuspended in PBS and layered gently on top of a 20-66% sucrose gradient. The gradients were spun at 100,000 x g for 20 hours to achieve equilibrium. Eighteen 2 ml fractions were harvested starting from the top of the gradient. 0.4 ml of each fraction was removed for virus titer determination. Each fraction was resuspended in 2 volumes of 20% PBS and concentrated by spinning at 100,000 x g for 1 hour. The pellet was then resuspended in 0.05 ml Laemmli buffer (Biorad) and analyzed for RSV and PIV3 proteins by Western blot, using an RSV F MAb (NuMax L1FR-S28R), RSV (Biogenesis) and bPIV3 (VMRD) polyclonal antisera. C-terminally truncated RSV F protein expressed in baculovirus that was purified to homogeneity, was also analyzed on a sucrose gradients.

The fractions were also analyzed for peak virus titers by plaque assay. Control gradients of free RSV F (generated in baculovirus and C-terminally truncated), RSV A2, and b/h PIV3 were carried out initially. The majority of free RSV F was present in fractions 3, 4, 5, and 6 in the top portion of the gradient (Figure 16A). The biggest concentration of RSV virions was observed in fractions 10, 11 and 12 (Figure 16B). The RSV fractions were probed with RSV polyclonal antiserum as well as with RSV F MAb. The fractions that contained the greatest amounts of RSV virions also showed the strongest signal for RSV F, suggesting that the RSV F protein co-migrated and associated with RSV virions (Figure 16B). These fractions also displayed the highest virus titers (Figure 16B). The b/h PIV3 virions may be more pleiomorphic and thus the spread of the peak fractions containing b/h PIV3 virions was more broad. b/h PIV3 virions were present in fractions 9,10, 11,12, and 13 (Figure 16C). Again the fractions harboring the most amounts of virions, also displayed the highest virus titers by plaque assay (Figure 16C). Sucrose gradient fractions of b/h PIV3/RSV F2 were analyzed with both a PIV3 polyclonal antiserum and an RSV F MAb (Figure 16D). The fractions containing most of the virions were fractions 11, 12, 13, and 14 as was shown by western using the PIV3 antiserum. Correspondingly, these were also the fractions that displayed the highest amounts of RSV F protein. However, some free RSV F was also present in fractions 5 and 6. Fractions 11, 12, 13 and 14 displayed the peak virus titers (Figure 16D). Similarly, the fractions containing the most virions of b/h PIV3/RSV G2 (fractions 9, 10, 11, and 12) also showed the strongest signal for RSV G protein (Figure 16E). Again these were the fractions with the highest virus titers (Figure 16E). Collectively these data suggested that the majority of the RSV F and G proteins co-migrated and associated with the b/h PIV3 virions. However, some free RSV proteins were also present in the top fractions of the gradients.

### 24. EXAMPLE 19: THE CHIMERIC BOVINE PARAINFLUENZA TYPE 3/HUMAN PARAINFLUENZA TYPE 3 VECTORED RESPIRATORY SYNCYTIAL VIRUS (RSV) COULD NOT BE NEUTRALIZED WITH RSV ANTISERA

In order to address the important safety question of whether the RSV surface glycoproteins incorporated into the b/h PIV3 virion resulted in an altered virus tropism phenotype, neutralization assays were carried out (Tables 6 and 7). RSV F MAbs (WHO 1200 MAb) neutralized 50% of wildtype RSV A2 at a 1:2000 dilution (Table 6). In contrast, even a dilution of 1:25 did not neutralize any of the chimeric b/h PIV3/RSV. Similarly, a dilution of 1:400 of the polyclonal RSV antiserum (Biogenesis) neutralized 50% of RSV A2, but even a dilution of 1:15.6 did not neutralize b/hPIV3 RSV (Table 6).

**Table 6**

| **The b/h PIV3 RSV Chimeric Viruses are not Neutralized by RSV Antibodies** | | |
|---|---|---|
| Virus used in neutralization | Reciprocal 50% neutralizing antibody dilution | |
| assay | RSV F MAb | RSV Ab |
| RSV | 2000 | 400.0 |
| b/h PIV3 | <25 | <15.6 |
| b/h RSV F1*N-N | <25 | <15.6 |
| b/h RSV F2 | <25 | <15.6 |
| b/h RSV G1 | ND | <15.6 |
| b/h RSV G2 | ND | <15.6 |

hPIV3 F MAb C191/9 neutralized 50% of b/h PIV3 as well as the b/h PIV3/RSV at a dilution of 1:500 (Table 7). An hPIV3 HN MAb 68/2 neutralized b/h PIV3 at a dilution of 1:16,000, and the b/h PIV3/RSV at a dilution of 1:32,000 (Table 7).

**Table 7**

| **The b/h PIV3 RSV Chimeric Viruses are Neutralized by hPIV3 Mabs** | | |
|---|---|---|
| Virus used in neutralization | Reciprocal 50% neutralizing antibody dilution | |
| assay | hPIV3 F MAb | hPIV3 HN MAb |
| RSV | 62.5 | <500 |
| b/h PIV3 | 500 | 16000 |
| b/h RSV F1*N-N | 500 | 32000 |
| b/h RSV F2 | 500 | 32000 |
| b/h RSV G1 | ND^{d} | 32000 |
| b/h RSV G2 | ND | 32000 |

| | | |
|---|---|---|
| ^{d} not determined. | | |

### 25. EXAMPLE 20: THE CHIMERIC BOVINE PIV DEMONSTRATE ATTENUATED PHENOTYPES AND ELICIT STRONG PROTECTIVE RESPONSES WHEN ADMINISTERED IN VIVO

Five week old Syrian Golden hamsters were infected with 5 x 10⁵ pfu of wildtype bPIV3, recombinant bPIV3, hPIV3, human/bovine PIV3, and placebo. The five different animal groups were kept separate in micro-isolator cages. Four days post-infection, the animals were sacrificed. The nasal turbinates and lungs of the animals were homogenized and stored at -80°C. Virus present in the tissues was determined by TCID₅₀ assays in MDBK cells at 37°C. Virus infection was confirmed by hemabsorption with guinea pig red blood cells. Table 8 shows the replication titers of the different PIV3 strains in hamsters in the lungs and nasal turbinates. Note that recombinant bPIV3 and the b/h PIV3 chimeric viruses are attenuated in the lungs of the hamsters:

**Table 8**

| **Replication of PIV3 Viruses in Syrian Golden Hamsters in the Nasal Turbinates and Lungs.** | | |
|---|---|---|
| Replication of bPIV3, r-bPIV3, r-bPIV3(1), hPIV3 and Bovine/Human | | |
| PIV3(1) in the Upper and Lower Respiratory Tract of Hamsters | | |
| | Mean virus titer on day 4 postinfection (log₁₀ TCID₅₀/g tissue = S. E.) ^{b} | |
| Virus^{a} | Nasal turbinates | Lungs |
| bPIV3 | 5.3 ± 0.3 | 5.3 ± 0.2 |
| r-bPIV 3 | 5.0 ± 0.3 | 3.5 ± 0.2 |
| r-bPIV3(1) | 5.5 ± 0.2 | 5.4 ± 0.2 |
| hPIV3 | 4.9 ± 0.2 | 5.4 ± 0.2 |
| Bovine/human PIV3(1) | 4.9 ± 0.2 | 4.5 ± 0.2 |

| | | |
|---|---|---|
| ^{a} Groups of four hamsters were inoculated intranasally with 5 x 10⁵ PFU of indicated virus. ^{b} Standard error. | | |

Furthermore, serum samples collected from the hamsters prior to infection and at day 21 post-infection were analyzed in a hemagglutination inhibition assay. The serum samples were treated with receptor destroying enzyme (RDE, DENKA Seiken Co.) and non-specific agglutinins were removed by incubation with guinea pig red blood cells for 1 hour on ice. Wildtype bPIV3 and hPIV3 were added to two-fold serially diluted hamster serum samples. Finally, guinea pig red blood cells (0.5%) were added, and hemagglutination was allowed to occur at room temperature. Table 9 shows the antibody response generated in the hamsters upon being infected with the different PIV3 strains. Note that the b/h PIV3 chimeric virus generates as strong an antibody response against hPIV3 as does wild type hPIV3, far exceeding the response generated by the recombinant or wildtype bPIV3:

**Table 9**

| **Hemaglutination Inhibition Assay Using Serum from Hamsters Infected with Different PIV3 Viruses.** | | |
|---|---|---|
| Virus Used for Inoculation of the Hamsters | Hamster Serum Titers for | |
| | wt bPIV3 | HPIV3 |
| Recombinant bPIV3 | 1:16 | 1:16 |
| Wt bPIV3 | 1:16 | 1:8 |
| Wt hPIV3 | 1:4 | 1:128 |
| b/h PIV3 chimeric virus | 1:8 | 1:128 |
| Placebo | <1:4 | <1.4 |

These results demonstrate the properties of b/h PIV3 chimeric viruses of the present invention which make these recombinants suitable for use in vaccine formulations. Not only do the b/h PIV3 chimeric viruses demonstrate an attenuated phenotype when administered in vivo, but they also generate as strong an antibody response as the wildtype hPIV3. Thus, because the chimeric viruses of the present invention have a unique combination of having an attenuated phenotype and eliciting as strong an immune response as a wildtype hPIV, these chimeric viruses have the characteristics necessary for successful use in humans to inhibit and/or protect against infection with PIV.

### 26. EXAMPLE 21 : REPLICATION OF BOVINE PARAINFLUENZA 3/HUMAN PARAINFLUENZA 3 VECTORED RESPIRATORY SYNCYTIAL VIRUS G OR F PROTEIN IN THE UPPER AND LOWER RESPIRATORY TRACT OF HAMSTERS

Five week old Syrian Golden hamsters (six animals per group) were infected intranasally with 1 x 10⁶ pfu or 1 x 10⁴ PFU of b/h PIV3, b/h PIV3/RSV, RSV A2, or placebo medium in a 100 ml volume. The different groups were maintained separately in micro-isolator cages. Four days post-infection, the nasal turbinates and lungs of the animals were harvested, homogenized and stored at -70°C. The titers of virus present in the tissues were determined by TCID₅₀ assays in Vero cells. For the challenge assays, the animals were inoculated on day 28 intranasally with 1 x 10⁶ pfu/ml of hPIV3 or RSV A2. Four days post-challenge, the nasal turbinates and lungs of the animals were isolated and assayed for challenge virus replication by plaque assays on Vero cells that were immunostained for quantification. Table 10 shows the replication titers of the different strains in hamsters in the lungs and nasal turbinates.

**Table 10**

| **Replication of bovine/human PIV3 Expressing the RSV G or F proteins in the Upper and Lower Respiratory Tract of Hamsters.** | | |
|---|---|---|
| | Mean virus titer on day 4 postinfection (1og₁₀ TCID₅₀/g tissue = S. E.)^{b} | |
| Virus^{a} | Nasal turbinates | Lungs |
| b/h PIV3 | 4.8 ± 0.4 | 4.4 ± 0.3 |
| RSV A2 | 3.4 ± 0.5 | 3.3 ± 0.5 |
| b/h RSV G1 | 4.2 ± 0.7 | 2.9 ± 0.7 |
| b/h RSV F1 | 3.9 ± 0.4 | 2.7 ± 0.2 |
| b/h RSV F1 N-P | 4.6 ± 0.4 | 3.5 ± 0.2 |
| b/h RSV G2 | 4.2 ± 0.9 | 4.3 ± 0.2 |
| b/h RSV F2 | 4.6 ± 0.6 | 4.4 ± 0.5 |

| | | |
|---|---|---|
| ^{a} Groups of four hamsters were inoculated intranaselly with 5 x 10⁶ PFU of indicated virus. ^{b} Standard error. | | |

Syrian Golden hamsters represent a suitable small animal model to evaluate replication and immunogenicity of recombinant bPIV3 and hPIV3 genetically engineered viruses. It was expected that the introduction of the RSV antigens would not alter the ability of the chimeric b/h PIV3 to replicate in hamsters. The results showed that all of the chimeric viruses replicated to levels similar to those of b/h PIV3 in the nasal turbinates of hamsters (Table 10). In contrast, the chimeric viruses harboring the RSV genes in the first position displayed 1 - 1.5 log₁₀ reduced titers in the lungs of hamsters compared to b/h PIV3 (Table 10). The chimeric viruses containing the RSV genes in the second position replicated to similar titers observed for b/h PIV3 in the lower respiratory tract of hamsters (Table 10).

### 27. EXAMPLE 22: BOVINE PARAINFLUENZA 3/HUMAN PARAINFLUENZA 3 VECTORED RESPIRATORY SYNCYTIAL VIRUS IMMUNIZED HAMSTERS WERE PROTECTED UPON CHALLENGE WITH HUMAN PARAINFLUENZA 3 AND RESPIRATORY SYNCYTIAL VIRUS A2

On Day 28 post-immunization, the hamsters were challenged with 1 x 10⁶ PFU of either RSV A2 or hPIV3 to evaluate the immunogenicity induced by the b/h PIV3/RSV. Animals that received the b/h PIV3/RSV were protected completely from RSV as well as hPIV3 (Table 11). Only the animals that were administered the placebo medium displayed high titers of challenge virus in the lower and upper respiratory tracts. This assay also showed that animals immunized with RSV, were not protected from challenge with hPIV3. Similarly, animals vaccinated with hPIV3 displayed high titers of the RSV challenge virus (Table 11).

**Table 11**

| **b/h PIV3/RSV Immunized Hamsters were Protected Upon Challenge with hPIV3 and RSV A2** | | | | |
|---|---|---|---|---|
| Challenge | hPIV3 | | RSV A2 | |
| Virus: | Mean Virus Titer on Day 4 Post-challenge (log₁₀TCID₅₀/₉ tissue ± S.E.)^{b,c} | | Mean Virus Titer on Day 4 Post-challenge (log₁₀ pfu/g tissue ± S.E.)^{b} | |
| Immunizing Virus ^{a} | Nasal turbinates | Lungs | Nasal Turbinates | Lungs |
| b/h PIV3 | <1.2 ± 0.0 | <1.0 ± 0.1 | ND | ND |
| b/h RSV G1 | <1.2 ± 0.1 | <1.1 ± 0.1 | < 1.0 ± 0.3 | < 0.7 ± 0.1 |
| b/h RSV F1 | <1.2 ± 0.2 | <1.0 ± 0.0 | < 1.1 ± 0.5 | < 0.6 ± 0.0 |
| b/h RSV F1 NP-P | <1.0 ± 0.0 | <1.0 ± 0.0 | < 0.8 ± 0.1 | < 0.5 ± 0.0 |
| b/h RSV G2 | <1.2 ± 0.2 | <1.1 ± 0.2 | < 0.8 ± 0.1 | < 0.8 ± 0.3 |
| b/h RSV F2 | <1.2 ± 0.1 | <1.0 ± 0.1 | < 1.3 ± 0.6 | < 1.6 ± 1.0 |
| RSV A2 | 4.5 ± 0.6 | 4.8 ± 0.6 | < 0.6 ± 0.2 | < 0.6 ± 0.1 |
| Placebo | 4.4 ± 0.1 | 4.1 ± 0.1 | 3.6 ± 0.8 | 3.1 ± 0.7 |

| | | | | |
|---|---|---|---|---|
| ^{a} Virus used to immunize groups of six hamsters on day 0. ^{b} On day 28, the hamsters were challenged with 10⁶ pfu of hPIV3 or RSV A2. Four days post-challenge, the lungs and nasal turbinates of the animals were harvested. ^{C} Standard error. | | | | |

### 28. EXAMPLE 23: VACCINATION OF HAMSTERS WITH BOVINE PARAINFLUENZA 3/HUMAN PARAINFLUENZA 3 VECTORED RESPIRATORY SYNCYTIAL VIRUS INDUCES SERUM HAI AND NEUTRALIZING ANTIBODIES

Prior to the challenge, serum samples were obtained on Day 28 from the immunized animals. The hamster sera were analyzed for the presence or RSV neutralizing antibodies using a 50% plaque reduction assay, and for PIV3 HAI serum antibodies by carrying out hemaggluination inhibition (HAI) assays (Table 8). 50% plaque reduction assay (neutralization assay) was carried out as follows: the hamster sera were two-fold serially diluted, and incubated with 100 PFU of RSV A2 for one hour. Then the virus-serum mixtures were transferred to Vero cell monolayers and overlaid with methylcellulose. After 5 days of incubation at 35°C, the monolayers were immunostained using RSV polyclonal antiserum for quantification. Hemagglutination-inhibition (HAI) assays were performed by incubating serial two-fold dilutions of Day 28 hamster sera at 25°C for 30 min with hPIV3 in V-bottom 96-well plates. Subsequently, guinea pig erythrocytes were added to each well, incubation was continued for an additional 90 min, and the presence or absence of hemagglutination in each well was recorded.

The results showed that the viruses expressing the RSV F protein displayed RSV neutralizing antibody titers nearly as high as those observed with serum obtained from animals vaccinated with wildtype RSV (Table 12). In contrast, the viruses expressing the RSV G protein showed much lower levels of RSV neutralizing antibodies (Table 12). All of the chimeric b/h PIV3/RSV hamster sera showed levels of HAI serum antibodies that were close to the levels observed for b/h PIV3 (Table 12). The results showed that the chimeric b/h PIV3 can infect and replicate efficiently in hamsters and elicit a protective immune response.

**Table 12**

| **Vaccination of Hamsters with b/h PIV3/RSV Induces Serum HAI and Neutralizing Antibodies** | | |
|---|---|---|
| Virus^{a} | Neutralizing antibody response to RSV ^{b,c} (mean reciprocal log₂ ± SE) | HAI antibody response to hPIV3 ^{c} (mean reciprocal log₂ ± SE) |
| RSV | 7.9 ± 1.00 | ND |
| b/h RSV F1*N-N | 7.8 ± 0.85 | 6.6 ± 0.5 |
| b/h RSV F1 | 5.5 ± 0.53 | 5.5 ± 0.5 |
| b/h RSV G1 | 3.4 ± 0.50 | 6.6 ± 0.7 |
| b/h RSV F2 | 6.9 ± 0.65 | 6.7 ± 0.8 |
| b/h RSV G2 | 3.4 ± 0.50 | 5.2 ± 0.4 |
| b/h PIV3 | ND | 7.2 ± 0.5 |

| | | |
|---|---|---|
| ^{a} Viruses used to immunize hamsters. ^{b} The neutralizing antibody titers were determined by a 50% plaque reduction assay. ^{c} The neutralizing antibody titers of hamster pre-serum were < 1.0 and the HAI antibody titers were < 4.0. | | |

### 29. EXAMPLE 24: VACCINATION OF HAMSTERS WITH LOW DOSE OF BOVINE PARAINFLUENZA 3/HUMAN PARAINFLUENZA 3 VECTORED RESPIRATORY SYNCYTIAL VIRUS PROTECTED HAMSTERS FROM CHALLENGE WITH RESPIRATORY SYNCYTIAL VIRUS A2, AND INDUCES SERUM HAI AND NEUTRALIZING ANTIBODIES

In order to identify the best vaccine candidate, low dose virus with different constructs (see Example 2) were used to immunize hamsters. The results of the challenging experiments are summarized in Table 13.

**Table 13**

| **b/h PIV3/RSV-Low Dose Immunized Hamsters are Protected From Challenge with RSV A2** | | | | |
|---|---|---|---|---|
| | Replication | | Challenge with RSV A2 | |
| | Mean Virus Titer on Day 4 Post-vaccination (log₁₀TCID₅₀/g tissue ± S.E.)^{b,c} | | Mean Virus Titer on Day 4 Post-challenge (log₁₀ pfu/g tissue ± S.E.)^{b} | |
| Immunizing Virus^{a} | Nasal turbinates | Lungs | Nasal Turbinates | Lungs |
| b/h PIV3 | 4.9 ± 0.5 | 4.8 ± 1.0 | ND | ND |
| b/h RSV G1 | 3.0 ± 0.8 | 3.1 ± 0.5 | < 0.9 ± 0.5 | < 0.7 ± 0.4 |
| b/h RSV F1*N-N | 3.4 ± 0.1 | 3.5 ± 0.1 | < 1.4 ± 0.7 | < 0.5 ± 0.0 |
| b/h RSV G2 | 4.1 ± 0.6 | 3.8 ± 0.4 | < 0.8 ± 0.0 | < 0.5 ± 0.1 |
| b/h RSV F2 | 5.2 ± 0.6 | 3.9 ± 0.4 | < 0.7 ± 0.1 | < 0.5 ± 0.1 |
| RSV A2 | 2.8 ± 0.3 | 2.7 ± 0.6 | < 0.8 ± 0.1 | < 0.5 ± 0.0 |
| Placebo | ND^{d} | ND | 3.0 ± 0.8 | 3.2 ± 0.9 |

| | | | | |
|---|---|---|---|---|
| ^{a} Virus used to immunize groups of six hamsters on day 0 with a low dose of 10⁴ PFU/ml. ^{b} On day 28, the hamsters were challenged with 10⁶ pfu of RSV A2. Four days post-challenge, the lungs and nasal turbinates of the animals were harvested. ^{c} Standard error. ^{d} not determined. | | | | |

Next, the neutralizing antibody titers were determined by a 50% plaque reduction assay (neutralization assay). Neutralization assays were performed for b/h PIV3, b/h PIV3/RSV chimeric viruses or RSV using Vero cells. Serial two-fold dilutions of RSV polyclonal antiserum (Biogenesis; Poole, England), an RSV F MAb (WHO 1200 MAb) obtained from MedImmune or hPIV3 F (C191/9) and HN (68/2) MAbs, were incubated with approximately 100 PFU of either b/h PIV3, b/h PIV3/RSV chimeric viruses or RSV in 0.5 ml OptiMEM at RT for 60 min. Following the incubation, virus-serum mixtures were transferred to Vero cell monolayers, incubated at 35°C for 1 hour, overlaid with 1% methyl cellulose in EMEM/L-15 medium (JRH Biosciences; Lenexa, KS) and incubated at 35°C. Six days post-inoculation, the infected cell monolayers were immunostained. Neutralization titers were expressed as the reciprocal of the highest serum dilution that inhibited 50% of viral plaques. Neutralization assays were also carried out for serum obtained on Day 28 post-infection from hamsters immunized with b/h PIV3, b/h PIV3/RSV chimeric viruses, or RSV A2. The hamster sera were two-fold serially diluted, and incubated with 100 PFU of RSV A2 for one hour. Then the virus-serum mixtures were transferred to Vero cell monolayers and overlaid with methylcellulose. After 5 days of incubation at 35°C the monolayers were immunostained using RSV polyclonal antiserum for quantification. The neutralizing antibody titers of hamster pre-serum were < 1.0 and the HAI antibody titers were < 4.0. Hemagglutination-inhibition (HAI) assays were performed by incubating serial two-fold dilutions of Day 28 hamster sera at 25°C for 30 min with hPIV3 in V-bottom 96-well plates. Subsequently, guinea pig erythrocytes were added to each well, incubation was continued for an additional 90 min, and the presence or absence of hemagglutination in each well was recorded. Table 14 summarizes the results:

**Table 14**

| **Vaccination of Hamsters with Lower Doses of b/h PIV3/RSV Induces Serum HAI and Neutralizing Antibodies** | | |
|---|---|---|
| Virus^{a} | Neutralizing antibody response to RSV ^{b,c} (mean reciprocal log₂ ± SE) | HAI antibody response to hPIV3^{c} (mean reciprocal log₂ ± SE) |
| RSV | 6.5 ± 0.7 | ND |
| b/h RSV F1*N-N | 2.5 ± 0.7 | 5.7 ± 0.6 |
| b/h RSV G1 | 2.0 ± 0.0 | 6.0 ± 0.0 |
| b/h RSV F2 | 3.8 ± 1.5 | 6.7 ± 0.6 |
| b/h RSV G2 | 3.8 ± 1.3 | 5.5 ± 0.6 |
| b/h PIV3 | ND | 6.5 ± 0.7 |

| | | |
|---|---|---|
| ^{a} Viruses used to immunize hamsters at a low dose of 10⁴ pfu/ml. ^{b} The neutralizing antibody titers were determined by a 50% plaque reduction assay. ^{c} The neutralizing antibody titers of hamster pre-serum were < 1.0 and the HAI antibody titers were < 4.0. | | |

The restricted replication phenotype of the chimeric viruses possessing RSV genes in the first position was exacerbated when the inoculation dose was reduced to 1 x 10⁴ PFU per animal. b/h PIV3/RSV F1 and G1 replicated in the upper respiratory tracts of hamsters to titers that were reduced by 1.0 - 2.0 log₁₀ compared to those of b/h PIV3 (Table 13). In contrast, b/h PIV3/RSV with the RSV genes in position 2, replicated in the upper respiratory tract to levels observed for b/h PIV3. Replication in the lungs of hamsters was also more restricted for the b/h PIV3/RSV harboring RSV genes in the first position (Table 13). In contrast, b/h PIV3/RSV F2 still replicated to high titers of 10^{5.2} and 10^{3.9} in the nasal turbinates and lungs, respectively (Table 13). The vaccinated hamsters were challenged on Day 28 with 1 x 10⁶ pfu of RSV A2 (Table 13). Despite the low levels of replication observed in the respiratory tracts of hamsters, the animals were protected in both the lower and upper respiratory tract from challenge with RSV (Table 13). The degree of protection was as good as was observed for animals vaccinated with wt RSV. Only the animals that received placebo medium showed high virus titers in the nasal turbinates and lungs (Table 13). Serum was collected from the immunized hamsters on Day 28, and analyzed for the presence of RSV neutralizing and PIV3 HAI serum antibodies (Table 14). An approximately 50% drop in RSV neutralizing antibody titers was observed in sera obtained from hamsters immunized with b/h PIV3/RSV as compared to the titers observed for wt RSV sera (Table 14). But the sera obtained from animals that had received b/h PIV3 harboring the RSV genes in position 2, still displayed higher RSV neutralization antibody titers than was observed for sera from b/h PIV3/RSV with the RSV genes in position 1. The PIV3 HAI serum antibody titers were also slightly reduced compared to the b/h PIV3 sera (Table 14).

### 30. EXAMPLE 25: BOVINE PARAINFLUENZA 3/HUMAN PARAINFLUENZA 3 VECTORED HUMAN METAPNEUMOVIRUS F IMMUNIZED HAMSTERS WERE PROTECTED UPON CHALLENGE WITH HUMAN PARAINFLUENZA VIRUS 3 OR HUMAN METAPNEUMOVIRUS NL/001

Five groups of Syrian Golden Hamsters (each group had six hamsters) were immunized with b/h PIV3, b/h hMPV F1, b/h hMPV F2, hMPV or placebo repectively. The five different animal groups were kept separate in micro-isolator cages. On Day 28 post-immunization, the hamsters were challenged with 1 x 10⁶ PFU of either hPIV3 or hMPV (NL/001 strain) to evaluate the immunogenicity induced by the b/h PIV3/hMPV F. Four days post-challenge, the animals were sacrificed. The nasal turbinates and lungs of the animals were homogenized and stored at -80°C. Virus present in the tissues was determined by TCID₅₀ assays in MDBK cells at 37°C. Virus infection was confirmed by hemabsorption with guinea pig red blood cells. Table 15 shows the replication titers of the PIV3 strain and the MPV strain in hamsters in the lungs and nasal turbinates.

**Table 15**

| **b/h PIV3/hMPV F-Immunized Hamsters were Protected Upon Challenge with hPIV3 or hMPV/NL/001** | | | | |
|---|---|---|---|---|
| Challenge virus: | hPIV3 | | hMPV | |
| | Mean virus titer on day 4 post-challenge (log₁₀TCID₅₀/g tissue ± S.E)^{b} | | Mean virus titer on day 4 post-challenge (log₁₀ PFU/g tissue ± S.E)^{b} | |
| Immunizing virus^{a} | Nasal Turbinates | Lungs | Nasal Turbinates | Lungs |
| b/h PIV3 | < 1.3 ± 0.2 | < 1.1 ± 0.1 | ND | |
| b/h hMPV F1 | < 1.3 ± 0.1 | < 1.1 ± 0.1 | 3.5 ± 0.8 | < 0.5 ± 0.2 |
| b/h hMPV F2 | < 1.2 ± 0.1 | < 1.2 ± 0.1 | < 0.9 ± 0.4 | < 0.5 ± 0.1 |
| hMPV | ND | | < 0.8 ± 0.3 | < 0.4 ± 0.0 |
| Placebo | 4.3 ± 0.3 | 4.5 ± 0.5 | 6.0 ± 0.3 | 4.5 ± 1.3 |

| | | | | |
|---|---|---|---|---|
| ^{a} Virus used to immunize groups of six hamsters on day 0. ^{b} On day 28, the hamsters were challenged with 10⁶ pfu of hPIV3 or hMPV. Four days post-challenge, the lungs and nasal turbinates of the animals were harvested. ND = not determined. | | | | |

The results showed that animals that received the b/h PIV3/hMPV F2 (F at position two) were protected completely from hMPV as well as hPIV3 (Table 15). However, b/h PIV3/hMPV F1 (F at position one) only reduced the titers of infected hMPV in the upper respiratory tract (e.g., nasal turbinates) by 2.5 logs, while it provided complete protection in the lower respiratory tract (e.g., the lung) from both hMPV and hPIV3 infection (Table 15). The animals that were administered the placebo medium displayed high titers of challenge virus in the lower and upper respiratory tracts (Table 15).

The present invention is not to be limited in scope by the specific described embodiments that are intended as single illustrations of individual aspects of the invention, and any constructs, viruses or enzymes that are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claims.

Various publications are cited herein, the disclosures of which are incorporated by reference in their entireties.

**Table 16**

| **LEGEND FOR SEQUENCE LISTING** | |
|---|---|
| SEQ ID NO:1 and | Human metapneumovirus isolate 00-1 matrix protein (M) fusion protein (F) genes |
| | |
| SEQ ID NO:2 | Avian pneumovirus fusion protein gene, partial cds |
| | |
| SEQ ID NO:3 | Avian pneumovirus isolate 1b fusion protein mRNA, complete cds |
| | |
| SEQ ID NO:4 | Turkey rhinotracheitis virus gene for fusion protein (F1 and F2 subunits), complete cds |
| | |
| SEQ ID NO:5 | Avian pneumovirus matrix protein (M) gene, partial cds and Avian pneumovirus fusion glycoprotein (F) gene, complete cds |
| | |
| SEQ ID NO:6 | paramyxovirus F protein hRSV B |
| | |
| SEQ ID NO:7 | paramyxovirus F protein hRSV A2 |
| | |
| SEQ ID NO:8 | human metapneumovirus 01-71 (partial sequence) |
| | |
| SEQ ID NO:9 | Human metapneumovirus isolate 00-1 matrix protein(M) and fusion protein (F) genes |
| | |
| SEQ ID NO:10 | Avian pneumovirus fusion protein gene, partial cds |
| | |
| SEQ ID NO:11 | Avian pneumovirus isolate 1b fusion protein mRNA, complete cds |
| | |
| SEQ ID NO:12 | Turkey rhinotracheitis virus gene for fusion protein (F1 and F2 subunits), complete cds |
| | |
| SEQ ID NO:13 | Avian pneumovirus fusion glycoprotein (F) gene, complete cds |
| | |
| SEQ ID NO:14 | Turkey rhinotracheitis virus (strain CVL14/1)attachment protien (G) mRNA, complete cds |
| | |
| SEQ ID NO:15 | Turkey rhinotracheitis virus (strain 6574) attachment protein (G), complete cds |
| | |
| SEQ ID NO:16 | Turkey rhinotracheitis virus (strain CVL14/1) attachment protein (G) mRNA, complete cds |
| | |
| SEQ ID NO:17 | Turkey rhinotracheitis virus (strain 6574) attachment protein (G), complete cds |
| | |
| SEQ ID NO:18 | F protein sequence for HMPV isolate NL/1/00 |
| | |
| SEQ ID NO:19 | F protein sequence for HMPV isolate NL/17/00 |
| | |
| SEQ ID NO:20 | F protein sequence for FIMPV isolate NL/1/99 |
| | |
| SEQ ID NO:21 | F protein sequence for HMPV isolate NL/1/94 |
| | |
| SEQ ID NO:22 | F-gene sequence for HMPV isolate NL/1/00 |
| | |
| SEQ ID NO:23 | F-gene sequence for HMPV isolate NL/17/00 |
| | |
| SEQ ID NO:24 | F-gene sequence for HMPV isolate NL/1/99 |
| | |
| SEQ ID NO:25 | F-gene sequence for HMPV isolate NL/1/94 |
| | |
| SEQ ID NO:26 | G protein sequence for HMPV isolate NL/1/00 |
| | |
| SEQ ID NO:27 | G protein sequence for HMPV isolate NL/17/00 |
| | |
| SEQ ID NO:28 | G protein sequence for HMPV isolate NL/1/99 |
| | |
| SEQ ID NO:29 | G protein sequence for HMPV isolate NL/1/94 |
| | |
| SEQ ID NO:30 | G-gene sequence for HMPV isolate NL/1/00 |
| | |
| SEQ ID NO:31 | G-gene sequence for HMPV isolate NL/17/00 |
| | |
| SEQ ID NO:32 | G-gene sequence for HMPV isolate NL/1/99 |
| | |
| SEQ ID NO:33 | G-gene sequence for HMPV isolate NL/1/94 |
| | |
| SEQ ID NO:34 | L protein sequence for HMPV isolate NL/1/00 |
| | |
| SEQ ID NO:35 | L protein sequence for HMPV isolate NL/17/00 |
| | |
| SEQ ID NO:36 | L protein sequence for HMPV isolate NL/1/99 |
| | |
| SEQ ID NO:37 | L protein sequence for HMPV isolate NL/1/94 |
| | |
| SEQ ID NO:38 | L-gene sequence for HMPV isolate NL/1/00 |
| | |
| SEQ ID NO:39 | L-gene sequence for HMPV isolate NL/17/00 |
| | |
| SEQ ID NO:40 | L-gene sequence for HMPV isolate NL/1/99 |
| | |
| SEQ ID NO:41 | L-gene sequence for HMPV isolate NL/1/94 |
| | |
| SEQ ID NO:42 | M2-1 protein sequence for HMPV isolate NL/1/00 |
| | |
| SEQ ID NO:43 | M2-1 protein sequence for HMPV isolate NL/17/00 |
| | |
| SEQ ID NO:44 | M2-1 protein sequence for HMPV isolate NL/1/99 |
| | |
| SEQ ID NO:45 | M2-1 protein sequence for HMPV isolate NL/1/94 |
| | |
| SEQ ID NO:46 | M2-1 gene sequence for HMPV isolate NL/1/00 |
| | |
| SEQ ID NO:47 | M2-1 gene sequence for HMPV isolate NL/17/00 |
| | |
| SEQ ID NO:48 | M2-1 gene sequence for HMPV isolate NL/1/99 |
| | |
| SEQ ID NO:49 | M2-1 gene sequence for HMPV isolate NL/1/94 |
| | |
| SEQ ID NO:50 | M2-2 protein sequence for HMPV isolate NL/1/00 |
| | |
| SEQ ID NO:51 | M2-2 protein sequence for HMPV isolate NL/17/00 |
| | |
| SEQ ID NO:52 | M2-2 protein sequence for HMPV isolate NL/1/99 |
| | |
| SEQ ID NO:53 | M2-2 protein sequence for HMPV isolate NL/1/94 |
| | |
| SEQ ID NO:54 | M2-2 gene sequence for HMPV isolate NL/1/00 |
| | |
| SEQ ID NO:55 | M2-2 gene sequence for HMPV isolate NL/17/00 |
| | |
| SEQ ID NO:56 | M2-2 gene sequence for HMPV isolate NL/1/99 |
| | |
| SEQ ID NO:57 | M2-2 gene sequence for HMPV isolate NL/1/94 |
| | |
| SEQ ID NO:58 | M2 gene sequence for HMPV isolate NL/1/00 |
| | |
| SEQ ID NO:59 | M2 gene sequence for HMPV isolate NL/17/00 |
| | |
| SEQ ID NO:60 | M2 gene sequence for HMPV isolate NL/1/99 |
| | |
| SEQ ID NO:61 | M2 gene sequence for HMPV isolate NL/1/94 |
| | |
| SEQ ID NO:62 | M protein sequence for HMPV isolate NL/1/00 |
| | |
| SEQ ID NO:63 | M protein sequence for HMPV isolate NL/17/00 |
| | |
| SEQ ID NO:64 | M protein sequence for HMPV isolate NL/1/99 |
| | |
| SEQ ID NO:65 | M protein sequence for HMPV isolate NL/1/94 |
| | |
| SEQ ID NO:66 | M gene sequence for HMPV isolate NL/1/00 |
| | |
| SEQ ID NO:67 | M gene sequence for HMPV isolate NL/17/00 |
| | |
| SEQ ID NO:68 | M gene sequence for HMPV isolate NL/1/99 |
| | |
| SEQ ID NO:69 | M gene sequence for HMPV isolate NL/1/94 |
| | |
| SEQ ID NO:70 | N protein sequence for HMPV isolate NL/1/00 |
| | |
| SEQ ID NO:71 | N protein sequence for HMPV isolate NL/17/00 |
| | |
| SEQ ID NO:72 | N protein sequence for HMPV isolate NL/1/99 |
| | |
| SEQ ID NO:73 | N protein sequence for HMPV isolate NL/1/94 |
| | |
| SEQ ID NO:74 | N gene sequence for HMPV isolate NL/1/00 |
| | |
| SEQ ID NO:75 | N gene sequence for HMPV isolate NL/17/00 |
| | |
| SEQ ID NO:76 | N gene sequence for HMPV isolate NL/1/99 |
| | |
| SEQ ID NO:77 | N gene sequence for HMPV isolate NL/1/94 |
| | |
| SEQ ID NO:78 | P protein sequence for HMPV isolate NL/1/00 |
| | |
| SEQ ID NO:79 | P protein sequence for HMPV isolate NL/17/00 |
| | |
| SEQ ID NO:80 | P protein sequence for HMPV isolate NL/1/99 |
| | |
| SEQ ID NO:81 | P protein sequence for HMPV isolate NL/1/94 |
| | |
| SEQ ID NO:82 | P gene sequence for HMPV isolate NL/1/00 |
| | |
| SEQ ID NO:83 | P gene sequence for HMPV isolate NL/17/00 |
| | |
| SEQ ID NO:84 | P gene sequence for HMPV isolate NL/1/99 |
| | |
| SEQ ID NO:85 | P gene sequence for HMPV isolate NL/1/94 |
| | |
| SEQ ID NO:86 | SH protein sequence for HMPV isolate NL/1/00 |
| | |
| SEQ ID NO:87 | SH protein sequence for HMPV isolate NL/17/00 |
| | |
| SEQ ID NO:88 | SH protein sequence for HMPV isolate NL/1/99 |
| | |
| SEQ ID NO:89 | SH protein sequence for HMPV isolate NL/1/94 |
| | |
| SEQ ID NO:90 | SH gene sequence for HMPV isolate NL/1/00 |
| | |
| SEQ ID NO:91 | SH gene sequence for HMPV isolate NL/17/00 |
| | |
| SEQ ID NO:92 | SH gene sequence for HMPV isolate NL/1/99 |
| | |
| SEQ ID NO:93 | SH gene sequence for HMPV isolate NL/1/94 |
| | |
| SEQ ID NO:94 | isolate NL/1/99 (99-1) HMPV (Human Metapneumovirus) cDNA sequence |
| | |
| SEQ ID NO:95 | isolate NL/1/00 (00-1) HMPV cDNA sequence |
| | |
| SEQ ID NO:96 | isolate NL/17/00 HMPV cDNA sequence |
| | |
| SEQ ID NO:97 | isolate NL/1/94 HMPV cDNA sequence |
| | |
| SEQ ID NO:98 | G-gene coding sequence for isolate NL/1/00 (A1) |
| | |
| SEQ ID NO:99 | G-gene coding sequence for isolate BR/2/01 (A1) |
| | |
| SEQ ID NO:100 | G-gene coding sequence for isolate FL/4/01 (A1) |
| | |
| SEQ ID NO:101 | G-gene coding sequence for isolate FL/3/01 (A1) |
| | |
| SEQ ID NO:102 | G-gene coding sequence for isolate FL/8/01 (A1) |
| | |
| SEQ ID NO:103 | G-gene coding sequence for isolate FL/10/01 (A1) |
| | |
| SEQ ID NO:104 | G-gene coding sequence for isolate NL/10/01 (A1) |
| | |
| SEQ ID NO:105 | G-gene coding sequence for isolate NL/2/02 (A1) |
| | |
| SEQ ID NO:106 | G-gene coding sequence for isolate NL/17/00 (A2) |
| | |
| SEQ ID NO:107 | G-gene coding sequence for isolate NL/1/81 (A2) |
| | |
| SEQ ID NO:108 | G-gene coding sequence for isolate NL/1/93 (A2) |
| | |
| SEQ ID NO:109 | G-gene coding sequence for isolate NL/2/93 (A2) |
| | |
| SEQ ID NO:110 | G-gene coding sequence for isolate NL/3/93 (A2) |
| | |
| SEQ ID NO:111 | G-gene coding sequence for isolate NL/1/95 (A2) |
| | |
| SEQ ID NO:112 | G-gene coding sequence for isolate NL/2/96 (A2) |
| | |
| SEQ ID NO:113 | G-gene coding sequence for isolate NL/3/96 (A2) |
| | |
| SEQ ID NO:114 | G-gene coding sequence for isolate NL/22/01 (A2) |
| | |
| SEQ ID NO:115 | G-gene coding sequence for isolate NL/24/01 (A2) |
| | |
| SEQ ID NO:116 | G-gene coding sequence for isolate NL/23/01 (A2) |
| | |
| SEQ ID NO:117 | G-gene coding sequence for isolate NL/29/01 (A2) |
| | |
| SEQ ID NO:118 | G-gene coding sequence for isolate NL/3/02 (A2) |
| | |
| SEQ ID NO:119 | G-gene coding sequence for isolate NL/1/99 (B1) |
| | |
| SEQ ID NO:120 | G-gene coding sequence for isolate NL/11/00 (B1) |
| | |
| SEQ ID NO:121 | G-gene coding sequence for isolate NL/12/00 (B1) |
| | |
| SEQ ID NO:122 | G-gene coding sequence for isolate NL/5/01 (B1) |
| | |
| SEQ ID NO:123 | G-gene coding sequence for isolate NL/9/01 (B1) |
| | |
| SEQ ID NO:124 | G-gene coding sequence for isolate NL/21/01 (B1) |
| | |
| SEQ ID NO:125 | G-gene coding sequence for isolate NL/1/94 (B2) |
| | |
| SEQ ID NO:126 | G-gene coding sequence for isolate NL/1/82 (B2) |
| | |
| SEQ ID NO:127 | G-gene coding sequence for isolate NL/1/96 (B2) |
| | |
| SEQ ID NO:128 | G-gene coding sequence for isolate NL/6/97 (B2) |
| | |
| SEQ ID NO:129 | G-gene coding sequence for isolate NL/9/00 (B2) |
| | |
| SEQ ID NO:130 | G-gene coding sequence for isolate NL/3/01 (B2) |
| | |
| SEQ ID NO:131 | G-gene coding sequence for isolate NL/4/01 (B2) |
| | |
| SEQ ID NO:132 | G-gene coding sequence for isolate UK/5/01 (B2) |
| | |
| SEQ ID NO:133 | G-protein sequence for isolate NL/1/00 (A1) |
| | |
| SEQ ID NO:134 | G-protein sequence for isolate BR/2/01 (A1) |
| | |
| SEQ ID NO:135 | G-protein sequence for isolate FL/4/01 (A1) |
| | |
| SEQ ID NO:136 | G-protein sequence for isolate FL/3/01 (A1) |
| | |
| SEQ ID NO:137 | G-protein sequence for isolate FL/8/01 (A1) |
| | |
| SEQ ID NO:138 | G-protein sequence for isolate FL/10/01 (A1) |
| | |
| SEQ ID NO:139 | G-protein sequence for isolate NL/10/01 (A1) |
| | |
| SEQ ID NO:140 | G-protein sequence for isolate NL/2/02 (A1) |
| | |
| SEQ ID NO:141 | G-protein sequence for isolate NL/17/00 (A2) |
| | |
| SEQ ID NO:142 | G-protein sequence for isolate NL/1/81 (A2) |
| | |
| SEQ ID NO:143 | G-protein sequence for isolate NL/1/93 (A2) |
| | |
| SEQ ID NO:144 | G-protein sequence for isolate NL/2/93 (A2) |
| | |
| SEQ ID NO:145 | G-protein sequence for isolate NL/3/93 (A2) |
| | |
| SEQ ID NO:146 | G-protein sequence for isolate NL/1/95 (A2) |
| | |
| SEQ ID NO:147 | G-protein sequence for isolate NL/2/96 (A2) |
| | |
| SEQ ID NO:148 | G-protein sequence for isolate NL/3/96 (A2) |
| | |
| SEQ ID NO:149 | G-protein sequence for isolate NL/22/01 (A2) |
| | |
| SEQ ID NO:150 | G-protein sequence for isolate NL/24/01 (A2) |
| | |
| SEQ ID NO:151 | G-protein sequence for isolate NL/23/01 (A2) |
| | |
| SEQ ID NO:152 | G-protein sequence for isolate NL/29/01 (A2) |
| | |
| SEQ ID NO:153 | G-protein sequence for isolate NL/3/02 (A2) |
| | |
| SEQ ID NO:154 | G-protein sequence for isolate NL/1/99 (B1) |
| | |
| SEQ ID NO:155 | G-protein sequence for isolate NL/11/00 (B1) |
| | |
| SEQ ID NO:156 | G-protein sequence for isolate NL/12/00 (B1) |
| | |
| SEQ ID NO:157 | G-protein sequence for isolate NL/5/01 (B1) |
| | |
| SEQ ID NO:158 | G-protein sequence for isolate NL/9/01 (B1) |
| | |
| SEQ ID NO:159 | G-protein sequence for isolate NL/21/01 (B1) |
| | |
| SEQ ID NO:160 | G-protein sequence for isolate NL/1/94 (B2) |
| | |
| SEQ ID NO:161 | G-protein sequence for isolate NL/1/82 (B2) |
| | |
| SEQ ID NO:162 | G-protein sequence for isolate NL/1/96 (B2) |
| | |
| SEQ ID NO:163 | G-protein sequence for isolate NL/6/97 (B2) |
| | |
| SEQ ID NO:164 | G-protein sequence for isolate NL/9/00 (B2) |
| | |
| SEQ ID NO:165 | G-protein sequence for isolate NL/3/01 (B2) |
| | |
| SEQ ID NO:166 | G-protein sequence for isolate NL/4/01 (B2) |
| | |
| SEQ ID NO:167 | G-protein sequence for isolate NL/5/01 (B2) |
| | |
| SEQ ID NO:168 | F-gene coding sequence for isolate NL/1/00 |
| | |
| SEQ ID NO:169 | F-gene coding sequence for isolate UK/1/00 |
| | |
| SEQ ID NO:170 | F-gene coding sequence for isolate NL/2/00 |
| | |
| SEQ ID NO:171 | F-gene coding sequence for isolate NL/13/00 |
| | |
| SEQ ID NO:172 | F-gene coding sequence for isolate NL/14/00 |
| | |
| SEQ ID NO:173 | F-gene coding sequence for isolate FL/3/01 |
| | |
| SEQ ID NO:174 | F-gene coding sequence for isolate FL/4/01 |
| | |
| SEQ ID NO:175 | F-gene coding sequence for isolate FL/8/01 |
| | |
| SEQ ID NO:176 | F-gene coding sequence for isolate UK/1/01 |
| | |
| SEQ ID NO:177 | F-gene coding sequence for isolate UK/7/01 |
| | |
| SEQ ID NO:178 | F-gene coding sequence for isolate FL/10/01 |
| | |
| SEQ ID NO:179 | F-gene coding sequence for isolate NL/6/01 |
| | |
| SEQ ID NO:180 | F-gene coding sequence for isolate NL/8/01 |
| | |
| SEQ ID NO:181 | F-gene coding sequence for isolate NL/10/01 |
| | |
| SEQ ID NO:182 | F-gene coding sequence for isolate NL/14/01 |
| | |
| SEQ ID NO:183 | F-gene coding sequence for isolate NL/20/01 |
| | |
| SEQ ID NO:184 | F-gene coding sequence for isolate NL/25/01 |
| | |
| SEQ ID NO:185 | F-gene coding sequence for isolate NL/26/01 |
| | |
| SEQ ID NO:186 | F-gene coding sequence for isolate NL/28/01 |
| | |
| SEQ ID NO:187 | F-gene coding sequence for isolate NL/30/01 |
| | |
| SEQ ID NO:188 | F-gene coding sequence for isolate BR/2/01 |
| | |
| SEQ ID NO:189 | F-gene coding sequence for isolate BR/3/01 |
| | |
| SEQ ID NO:190 | F-gene coding sequence for isolate NL/2/02 |
| | |
| SEQ ID NO:191 | F-gene coding sequence for isolate NL/4/02 |
| | |
| SEQ ID NO:192 | F-gene coding sequence for isolate NL/5/02 |
| | |
| SEQ ID NO:193 | F-gene coding sequence for isolate NL/6/02 |
| | |
| SEQ ID NO:194 | F-gene coding sequence for isolate NL/7/02 |
| | |
| SEQ ID NO:195 | F-gene coding sequence for isolate NL/9/02 |
| | |
| SEQ ID NO:196 | F-gene coding sequence for isolate FL/1/02 |
| | |
| SEQ ID NO:197 | F-gene coding sequence for isolate NL/1/81 |
| | |
| SEQ ID NO:198 | F-gene coding sequence for isolate NL/1/93 |
| | |
| SEQ ID NO:199 | F-gene coding sequence for isolate NL/2/93 |
| | |
| SEQ ID NO:200 | F-gene coding sequence for isolate NL/4/93 |
| | |
| SEQ ID NO:201 | F-gene coding sequence for isolate NL/1/95 |
| | |
| SEQ ID NO:202 | F-gene coding sequence for isolate NL/2/96 |
| | |
| SEQ ID NO:203 | F-gene coding sequence for isolate NL/3/96 |
| | |
| SEQ ID NO:204 | F-gene coding sequence for isolate NL/1/98 |
| | |
| SEQ ID NO:205 | F-gene coding sequence for isolate NL/17/00 |
| | |
| SEQ ID NO:206 | F-gene coding sequence for isolate NL/22/01 |
| | |
| SEQ ID NO:207 | F-gene coding sequence for isolate NL/29/01 |
| | |
| SEQ ID NO:208 | F-gene coding sequence for isolate NL/23/01 |
| | |
| SEQ ID NO:209 | F-gene coding sequence for isolate NL/17/01 |
| | |
| SEQ ID NO:210 | F-gene coding sequence for isolate NL/24/01 |
| | |
| SEQ ID NO:211 | F-gene coding sequence for isolate NL/3/02 |
| | |
| SEQ ID NO:212 | F-gene coding sequence for isolate NL/3/98 |
| | |
| SEQ ID NO:213 | F-gene coding sequence for isolate NL/1/99 |
| | |
| SEQ ID NO:214 | F-gene coding sequence for isolate NL/2/99 |
| | |
| SEQ ID NO:215 | F-gene coding sequence for isolate NL/3/99 |
| | |
| SEQ ID NO:216 | F-gene coding sequence for isolate NL/11/00 |
| | |
| SEQ ID NO:217 | F-gene coding sequence for isolate NL/12/00 |
| | |
| SEQ ID NO:218 | F-gene coding sequence for isolate NL/1/01 |
| | |
| SEQ ID NO:219 | F-gene coding sequence for isolate NL/5/01 |
| | |
| SEQ ID NO:220 | F-gene coding sequence for isolate NL/9/01 |
| | |
| SEQ ID NO:221 | F-gene coding sequence for isolate NL/19/01 |
| | |
| SEQ ID NO:222 | F-gene coding sequence for isolate NL/21/01 |
| | |
| SEQ ID NO:223 | F-gene coding sequence for isolate UK/11/01 |
| | |
| SEQ ID NO:224 | F-gene coding sequence for isolate FL/1/01 |
| | |
| SEQ ID NO:225 | F-gene coding sequence for isolate FL/2/01 |
| | |
| SEQ ID NO:226 | F-gene coding sequence for isolate FL/5/01 |
| | |
| SEQ ID NO:227 | F-gene coding sequence for isolate FL/7/01 |
| | |
| SEQ ID NO:228 | F-gene coding sequence for isolate FL/9/01 |
| | |
| SEQ ID NO:229 | F-gene coding sequence for isolate UK/10/01 |
| | |
| SEQ ID NO:230 | F-gene coding sequence for isolate NL/1/02 |
| | |
| SEQ ID NO:231 | F-gene coding sequence for isolate NL/1/94 |
| | |
| SEQ ID NO:232 | F-gene coding sequence for isolate NL/1/96 |
| | |
| SEQ ID NO:233 | F-gene coding sequence for isolate NL/6/97 |
| | |
| SEQ ID NO:234 | F-gene coding sequence for isolate NL/7/00 |
| | |
| SEQ ID NO:235 | F-gene coding sequence for isolate NL/9/00 |
| | |
| SEQ ID NO:236 | F-gene coding sequence for isolate NL/19/00 |
| | |
| SEQ ID NO:237 | F-gene coding sequence for isolate NL/28/00 |
| | |
| SEQ ID NO:238 | F-gene coding sequence for isolate NL/3/01 |
| | |
| SEQ ID NO:239 | F-gene coding sequence for isolate NL/4/01 |
| | |
| SEQ ID NO:240 | F-gene coding sequence for isolate NL/11/01 |
| | |
| SEQ ID NO:241 | F-gene coding sequence for isolate NL/15/01 |
| | |
| SEQ ID NO:242 | F-gene coding sequence for isolate NL/18/01 |
| | |
| SEQ ID NO:243 | F-gene coding sequence for isolate FL/6/01 |
| | |
| SEQ ID NO:244 | F-gene coding sequence for isolate UK/5/01 |
| | |
| SEQ ID NO:245 | F-gene coding sequence for isolate UK/8/01 |
| | |
| SEQ ID NO:246 | F-gene coding sequence for isolate NL/12/02 |
| | |
| SEQ ID NO:247 | F-gene coding sequence for isolate HK/1/02 |
| | |
| SEQ ID NO:248 | F-protein sequence for isolate NL/1/00 |
| | |
| SEQ ID NO:249 | F-protein sequence for isolate UK/1/00 |
| | |
| SEQ ID NO:250 | F-protein sequence for isolate NL/2/00 |
| | |
| SEQ ID NO:251 | F-protein sequence for isolate NL/13/00 |
| | |
| SEQ ID NO:252 | F-protein sequence for isolate NL/14/00 |
| | |
| SEQ ID NO:253 | F-protein sequence for isolate FL/3/01 |
| | |
| SEQ ID NO:254 | F-protein sequence for isolate FL/4/01 |
| | |
| SEQ ID NO:255 | F-protein sequence for isolate FL/8/01 |
| | |
| SEQ ID NO:256 | F-protein sequence for isolate UK/1/01 |
| | |
| SEQ ID NO:257 | F-protein sequence for isolate UK/7/01 |
| | |
| SEQ ID NO:258 | F-protein sequence for isolate FL/10/01 |
| | |
| SEQ ID NO:259 | F-protein sequence for isolate NL/6/01 |
| | |
| SEQ ID NO:260 | F-protein sequence for isolate NL/8/01 |
| | |
| SEQ ID NO:261 | F-protein sequence for isolate NL/10/01 |
| | |
| SEQ ID NO:262 | F-protein sequence for isolate NL/14/01 |
| | |
| SEQ ID NO:263 | F-protein sequence for isolate NL/20/01 |
| | |
| SEQ ID NO:264 | F-protein sequence for isolate NL/25/01 |
| | |
| SEQ ID NO:265 | F-protein sequence for isolate NL/26/01 |
| | |
| SEQ ID NO:266 | F-protein sequence for isolate NL/28/01 |
| | |
| SEQ ID NO:267 | F-protein sequence for isolate NL/30/01 |
| | |
| SEQ ID NO:268 | F-protein sequence for isolate BR/2/01 |
| | |
| SEQ ID NO:269 | F-protein sequence for isolate BR/3/01 |
| | |
| SEQ ID NO:270 | F-protein sequence for isolate NL/2/02 |
| | |
| SEQ ID NO:271 | F-protein sequence for isolate NL/4/02 |
| | |
| SEQ ID NO:272 | F-protein sequence for isolate NL/5/02 |
| | |
| SEQ ID NO:273 | F-protein sequence for isolate NL/6/02 |
| | |
| SEQ ID NO:274 | F-protein sequence for isolate NL/7/02 |
| | |
| SEQ ID NO:275 | F-protein sequence for isolate NL/9/02 |
| | |
| SEQ ID NO:276 | F-protein sequence for isolate FL/1/02 |
| | |
| SEQ ID NO:277 | F-protein sequence for isolate NL/1/81 |
| | |
| SEQ ID NO:278 | F-protein sequence for isolate NL/1/93 |
| | |
| SEQ ID NO:279 | F-protein sequence for isolate NL/2/93 |
| | |
| SEQ ID NO:280 | F-protein sequence for isolate NL/4/93 |
| | |
| SEQ ID NO:281 | F-protein sequence for isolate NL/1/95 |
| | |
| SEQ ID NO:282 | F-protein sequence for isolate NL/2/96 |
| | |
| SEQ ID NO:283 | F-protein sequence for isolate NL/3/96 |
| | |
| SEQ ID NO:284 | F-protein sequence for isolate NL/1/98 |
| | |
| SEQ ID NO:285 | F-protein sequence for isolate NL/17/00 |
| | |
| SEQ ID NO:286 | F-protein sequence for isolate NL/22/01 |
| | |
| SEQ ID NO:287 | F-protein sequence for isolate NL/29/01 |
| | |
| SEQ ID NO:288 | F-protein sequence for isolate NL/23/01 |
| | |
| SEQ ID NO:289 | F-protein sequence for isolate NL/17/01 |
| | |
| SEQ ID NO:290 | F-protein sequence for isolate NL/24/01 |
| | |
| SEQ ID NO:291 | F-protein sequence for isolate NL/3/02 |
| | |
| SEQ ID NO:292 | F-protein sequence for isolate NL/3/98 |
| | |
| SEQ ID NO:293 | F-protein sequence for isolate NL/1/99 |
| | |
| SEQ ID NO:294 | F-protein sequence for isolate NL/2/99 |
| | |
| SEQ ID NO:295 | F-protein sequence for isolate NL/3/99 |
| | |
| SEQ ID NO:296 | F-protein sequence for isolate NL/11/00 |
| | |
| SEQ ID NO:297 | F-protein sequence for isolate NL/12/00 |
| | |
| SEQ ID NO:298 | F-protein sequence for isolate NL/1/01 |
| | |
| SEQ ID NO:299 | F-protein sequence for isolate NL/5/01 |
| | |
| SEQ ID NO:300 | F-protein sequence for isolate NL/9/01 |
| | |
| SEQ ID NO:301 | F-protein sequence for isolate NL/19/01 |
| | |
| SEQ ID NO:302 | F-protein sequence for isolate NL/21/01 |
| | |
| SEQ ID NO:303 | F-protein sequence for isolate UK/11/01 |
| | |
| SEQ ID NO:304 | F-protein sequence for isolate FL/1/01 |
| | |
| SEQ ID NO:305 | F-protein sequence for isolate FL/2/01 |
| | |
| SEQ ID NO:306 | F-protein sequence for isolate FL/5/01 |
| | |
| SEQ ID NO:307 | F-protein sequence for isolate FL/7/01 |
| | |
| SEQ ID NO:308 | F-protein sequence for isolate FL/9/01 |
| | |
| SEQ ID NO:309 | F-protein sequence for isolate UK/10/01 |
| | |
| SEQ ID NO:310 | F-protein sequence for isolate NL/1/02 |
| | |
| SEQ ID NO:311 | F-protein sequence for isolate NL/1/94 |
| | |
| SEQ ID NO:312 | F-protein sequence for isolate NL/1/96 |
| | |
| SEQ ID NO:313 | F-protein sequence for isolate NL/6/97 |
| | |
| SEQ ID NO:314 | F-protein sequence for isolate NL/7/00 |
| | |
| SEQ ID NO:315 | F-protein sequence for isolate NL/9/00 |
| | |
| SEQ ID NO:316 | F-protein sequence for isolate NL/19/00 |
| | |
| SEQ ID NO:317 | F-protein sequence for isolate NL/28/00 |
| | |
| SEQ ID NO:318 | F-protein sequence for isolate NL/3/01 |
| | |
| SEQ ID NO:319 | F-protein sequence for isolate NL/4/01 |
| | |
| SEQ ID NO:320 | F-protein sequence for isolate NL/11/01 |
| | |
| SEQ ID NO:321 | F-protein sequence for isolate NL/15/01 |
| | |
| SEQ ID NO:322 | F-protein sequence for isolate NL/18/01 |
| | |
| SEQ ID NO:323 | F-protein sequence for isolate FL/6/01 |
| | |
| SEQ ID NO:324 | F-protein sequence for isolate UK/5/01 |
| | |
| SEQ ID NO:325 | F-protein sequence for isolate UK/8/01 |
| | |
| SEQ ID NO:326 | F-protein sequence for isolate NL/12/02 |
| | |
| SEQ ID NO:327 | F-protein sequence for isolate HK/1/02 |

The present invertion will now be further defined by the following numbered clauses
1. A chimeric bovine parainfluenza virus type 3 comprising a heterologous nucleotide sequence encoding a metapneumovirus polypeptide.
2. A chimeric human parainfluenza virus type 3 comprising a heterologous nucleotide sequence encoding a metapneumovirus polypeptide.
3. A chimeric bovine parainfluenza virus type 3/human parainfluenza virus type 3 comprising a heterologous nucleotide sequence encoding a metapneumovirus polypeptide.
4. The virus of clause 1, 2, or 3, wherein said virus comprises heterologous nucleotide sequences derived from the same gene of a metapneumovirus.
5. The virus of clause 1, 2, or 3, wherein said virus comprises heterologous nucleotide sequences derived from at least two different genes of a metapneumovirus.
6. The virus of clause 1, 2, or 3, wherein one or more of the open reading frames in the genome of the virus have been replaced by an ORF which encodes one or more of (i) an avian pneumovirus (APV) F protein; (ii) an APV G protein; (iii) an APV SH protein; (iv) an APV N protein; (v) an APV P protein; (vi) an APV M2 protein; (vii) an APV M2-1 protein; (viii) an APV M2-2 protein; or (ix) an APV L protein.
7. The virus of clause 1, 2, or 3, wherein one or more of the open reading frames in the genome of virus have been replaced by an ORF which encodes one or more of (i) a mammalian metapneumovirus (MPV) F protein; (ii) a mammalian MPV G protein; (iii) a mammalian MPV SH protein; (iv) a mammalian MPV N protein; (v) a mammalian MPV P protein; (vi) a mammalian MPV M2 protein; (vii) a mammalian MPV M2-1 protein; (viii) a mammalian MPV M2-2 protein; or (ix) a mammalian MPV L protein.
8. The virus of clause 6, wherein the avian pneumovirus is APV type A, APV type B, APV type C, or APV type D.
9. The virus of clause 7, wherein the mammalian MPV is variant A1, variant A2, variant B1, or variant B2.
10. The virus of clause 1, 2, or 3, wherein said heterologous nucleotide sequence is added to the complete genome of said virus.
11. The virus of clause 1, 2, or 3, wherein said nucleotide sequence is added at a nucleotide position of the parainfluenza virus genome of 104, 1774, and 3724.
12. The virus of clause 1, 2, or 3, wherein a nucleotide sequence of said parainfluenza virus has been substituted by said heterologous nucleotide sequence.
13. The virus of clause 1, 2, or 3, wherein the heterologous sequences are inserted into the genome of said parainfluenza virus, wherein the genome has been deleted of one or more genes.
14. The virus of clause 1, wherein said bovine parainfluenza virus is a Kansas-strain bovine parainfluenza virus type 3.
15. The virus of clause 1,2, or 3, wherein said heterologous nucleotide sequence is derived from the nucleotide sequences encoding a F protein, a G protein or a fragment thereof.
16. The virus of clause 15, wherein said F protein comprises an ectodomain of a F protein of a metapneumovirus, a transmembrane domain of a F protein of a parainfluenza virus, and luminal domain of a F protein of a parainfluenza virus.
17. The virus of clause 1, 2, or 3, wherein said nucleotide sequence is SEQ ID Nos:1 to 5, 14, or 15.
18. The virus of clause 1, 2, or 3, wherein said nucleotide sequence encodes a protein of SEQ ID Nos: 6 to 13, 16, or 17.
19. The virus of clause 1, 2, or 3, wherein said virus further comprises a heterologous nucleotide sequence derived from a respiratory syncytial virus or a mutated form of respiratory syncytial virus.
20. The virus of clause 19, wherein the respiratory syncytial virus is a respiratory syncytial virus type A, a respiratory syncytial virus type B, a bovine respiratory syncytial virus, or an ovine respiratory syncytial virus.
21. The virus of clause 19, wherein said sequence is derived from the nucleotide sequences encoding a F protein, a G protein or a fragment thereof of said respiratory syncytial virus.
22. The chimeric parainfluenza virus of clause 1, 2, or 3, wherein the genome of said virus contains mutations or modifications, in addition to said heterologous nucleotide sequences, which result in a chimeric virus having a phenotype more suitable for use in vaccine formulations such as an attenuated phenotype or a phenotype with enhanced antigenicity.
23. The chimeric parainfluenza virus of clause 1, 2, or 3, wherein the intergenic region of said heterologous nucleotide sequence is altered.
24. The chimeric parainfluenza virus of clause 1, 2, or 3, wherein said heterologous nucleotide sequence is added at a position of the parainfluenza virus genome selected from the group consisting of position 1, 2, 3, 4, 5, and 6, and wherein the intergenic region of said heterologous nucleotide sequence is altered.
25. A recombinant DNA or RNA molecule encoding the genome of the virus of clause 1, 2, or 3.
26. A recombinant DNA or RNA molecule encoding the genome of the virus of clause 18.
27. A vaccine formulation comprising the chimeric virus of clause 1, 2, or 3, and a pharmaceutically acceptable excipient.
28. A vaccine formulation comprising the chimeric virus of clause 18 and a pharmaceutically acceptable excipient.
29. The vaccine formulation of clause 27, wherein said chimeric virus comprises a genomic modification or mutation which results in an attenuated phenotype or enhanced antigenicity.
30. The vaccine formulation of clause 29, wherein said modification or mutation is derived from a naturally occurring mutant.
31. The vaccine formulation of clause 27, wherein said vaccine is used to modulate the immune response of humans, primates, horses, cows, sheep, pigs, goats, dogs, cats, avian species or rodents.
32. The vaccine formulation of clause 30, wherein the vaccine is used to modulate the immune response of a human infant or a child.
33. An immunogenic formulation comprising the chimeric virus of clause 1, 2, or 3, and a pharmaceutically acceptable excipient.
34. An immunogenic formulation comprising the chimeric virus of clause 18 and a pharmaceutically acceptable excipient.
35. The immunogenic formulation of clause 33, wherein said chimeric virus comprises a genomic modification or mutation which results in an attenuated phenotype or enhanced antigenicity.
36. The immunogenic formulation of clause 35, wherein said modification or mutation is derived from a naturally occurring mutant.
37. The vaccine formulation of clause 33, wherein said vaccine is used to modulate the immune response of humans, primates, horses, cows, sheep, pigs, goats, dogs, cats, avian species or rodents.
38. The vaccine formulation of clause 36, wherein the vaccine is used to modulate the immune response of a human infant or a child.
39. A method for treating or preventing a respiratory tract infection in a mammal, said method comprising administering a vaccine of clause 27.
40. The method of clause 39, wherein the respiratory tract infection is a MPV infection.
41. The method of clause 39, wherein the respiratory tract infection is an infection with MPV, RSV, hPIV or a combination thereof.
42. The method of clause 39, wherein the subject is a human.
43. The method of clause 42, wherein the human subject is less than 5 years of age.
44. The method of clause 42, wherein the human subject is less than 2 years of age.
45. The method of clause 42, wherein the human subject suffers from a disease or a condition in addition to the respiratory tract infection.
46. The method of clause 45, wherein the disease or a condition is of cystic fibrosis, leukemia, non-Hodgkin lymphoma, asthma, and bone marrow transplantation and kidney transplantation.
47. The human subject of clause 42, wherein the human subject is an immunocompromised individual.
48. The human subject of clause 42, wherein the human subject is an elderly.

## Claims

1. A chimeric PIV virus comprising a backbone encoded by nucleotide sequences derived from bovine parainfluenza virus type 3 genome (bPIV3) in which (a) the bovine parainfluenza virus F gene and HN gene have been substituted with the F gene and HN gene of the human parainfluenza virus type 3 (hPIV3) and b) in which a heterologous sequence from RSV has been added to complete the chimeric virus genome wherein the heterologous RSV sequence is the F gene of RSV and this is inserted between the first and second open reading frames of the bPIV3 genome.

2. The chimeric PIV virus according to claim 1 wherein the F gene of RSV is inserted in the intergenic region between the first and second open reading frames of the bPIV3 genome.

3. The chimeric PIV virus according to claims 1 or 2, wherein the F gene of RSV is inserted at position 1774.

4. The chimeric PIV virus according to any preceding claim wherein the bPIV3 is the Kansas strain.

5. The chimeric PIV virus according to any preceding claim wherein the hPIV3 is the strain hPIV3/Tex/12084/1983.

6. The chimeric PIV virus according to any preceding claim wherein the RSV F gene encodes a protein comprising the amino acid sequence as shown in SEQ ID NO:7.
